# EUROPEAN PATENT APPLICATION

(11) **EP 4 840 444 A1**
(43) Date of publication of application: **18.11.2026**
(21) Application number: 25741658.6
(22) Date of filing: 20.01.2025
(51) Int. Cl.: A61K 9/51, A61K 48/00, A61K 31/7088, A61K 31/7105, A61K 31/713, A61K 47/18, A61K 47/22, A61K 47/26, A61K 47/24, A61K 47/28

(54) **USE OF BLANK LIPID NANOPARTICLES IN PREPARATION OF IN-VIVO DELIVERY PRODUCT**

(30) Priority: 19.01.2024 CN 202410079585
(71) Applicant: Scindy Pharmaceutical Co., Ltd, Suzhou, Jiangsu 215124 (CN)
(72) Inventor: CHEN, Xiaobao, Suzhou, Jiangsu 215124 (CN)
(74) Representative: Elzaburu S.L.P.
(86) International application number: PCT/CN2025/073412
(87) International publication number: WO 2025/153097

(57) **Abstract**

The use of blank lipid nanoparticles in the preparation of an in-vivo delivery product, comprising the step of mixing the blank lipid nanoparticles with a biologically active substance in a solvent to obtain a blank lipid nanoparticle-based composition. The blank lipid nanoparticles consist of an ionizable lipid, a phospholipid, a steroid or a derivative thereof, and a polyethylene glycol-lipid conjugate. According to the blank lipid nanoparticles, the dosage of the biologically active substance can be flexibly adjusted according to the requirements of a user, and the blank lipid nanoparticles can be administered via multiple routes such as intravenous injection, intramuscular injection, intraperitoneal injection, and subcutaneous injection, all of which can achieve an ideal delivery effect.

## Description

### CROSS-REFERENCE TO RELATED APPLICATIONS

This application claims priority to Chinese Patent Application No. 202410079585.4, filed on January 19, 2024, the entire contents of which are incorporated herein by reference.

### TECHNICAL FIELD

The present application belongs to the technical field of biomedicine, and specifically relates to the use of blank lipid nanoparticles in the preparation of an in-vivo delivery product.

### BACKGROUND

Gene editing therapy holds the potential to cure genetic diseases, yet gene-editing drugs need to be delivered safely and efficiently to the corresponding target organs and tissues in the body. Gene editing drugs may exist in the form of DNA, mRNA, protein, or ribonucleoprotein (RNP). Successful intracellular delivery of such macromolecular drugs requires overcoming multiple physiological barriers: 1) preventing dissociation between the drug and carrier or drug degradation before the drug enters cells; 2) targeting specific cells; 3) penetrating cell membranes to enter the interior of cells; 4) releasing the drug in specific organelles. Therefore, how to safely and efficiently introduce gene editing drugs into corresponding target cells in vivo poses a major challenge.

Existing in-vivo delivery technologies for gene editing drugs still suffer from numerous drawbacks. For instance, the most widely adopted delivery technology mediated by viral vectors typically uses adeno-associated viral delivery vectors or lentiviral vectors to deliver drugs. Adeno-associated viral delivery vectors have good safety and biocompatibility, and are capable of delivering drugs to tissues including eyes, livers, brains, cardiac muscles, skeletal muscles and the like. Naturally-occurring and laboratory-synthesized AAV capsid serotypes of different types can achieve different tissue targeting property. Nevertheless, they have limited packaging capacity (only 5 kb of DNA) and carry potential safety risks. Lentiviral vectors can accommodate DNA drugs up to 10 kb, which is sufficient to encapsulate known gene editing drugs within a single vector, yet they bear a risk of potential genome integration. In summary, viral vectors are plagued by multiple drawbacks including vector immunogenicity, long-term expression of gene editing drugs, off-target gene editing, as well as possibility of genome integration, manufacturing costs and dose-limiting toxicity, potential safety hazards such as integration genotoxicity, induction of immune responses in host cells and other similar hidden dangers, which restrict their uses.

Delivery technologies mediated by cationic polymers such as polyethyleneimine and the like also suffer from issues including low transfection efficiency, high toxicity and the like. Although numerous in-vivo delivery methods for gene editing drugs are currently available, most of them feature high costs, potential safety risks, high toxicity or low transfection efficiency. Accordingly, the current low-efficiency in-vivo delivery of nucleic acids stands as one of the bottlenecks restricting the further development of gene therapy and gene immunotherapy technologies. Therefore, there is an urgent demand for an in-vivo transfection reagent with high transfection efficiency and low toxicity to meet current requirements.

Lipid nanoparticle (LNP) delivery has the following advantages in the field of delivering gene editing drugs: 1) delivery of gene editing drugs by LNPs enables transient expression. Compared with the long-term expression of gene editing drugs induced by viral vectors, LNPs can minimize the possibility of off-target effects to the greatest extent; 2) LNPs have much lower immunogenicity than viral vectors, allow repeated administration under certain conditions, possess good safety and biocompatibility, and can deliver drug dosages sufficient to achieve effective gene editing levels; 3) the large-scale manufacturing processes for LNPs have currently matured, which provides a foundation for clinical trials that use LNPs to deliver in-vivo gene editing drugs. Patents CN116832051A and CN116969850A have conducted researches in this field. Nevertheless, in the prior art, delivering gene editing drugs by lipid nanoparticles (LNP) generally requires a special mixing process; otherwise, a good delivery effect cannot be achieved.

Based on the above, the present application provides the use of the blank lipid nanoparticles in the preparation of an in-vivo delivery product; simple mixing of a biologically active substance and the blank lipid nanoparticles under a solution state can achieve good safe and effective delivery of the biologically active substance.

### SUMMARY

In view of the problems in the prior art, the present application provides the use of blank lipid nanoparticles in the preparation of an in-vivo delivery product. The use of the blank lipid nanoparticles in the preparation of an in-vivo delivery product comprises the step of mixing the blank lipid nanoparticles with a biologically active substance in a solvent to obtain a blank lipid nanoparticle-based composition. The blank lipid nanoparticles consist of an ionizable lipid, a phospholipid, cholesterol, and a polyethylene glycol-lipid conjugate. By using the blank lipid nanoparticles in the present application, the dosage of the biologically active substance can be flexibly adjusted according to the requirements of a user, and administration can be carried out via multiple routes to achieve an ideal delivery effect.

To achieve the above objects, in a first aspect, the present application provides the use of blank lipid nanoparticles in the preparation of an in-vivo delivery product, comprising the step of mixing the blank lipid nanoparticles with a biologically active substance in a solvent to obtain a blank lipid nanoparticle-based composition. The blank lipid nanoparticles consist of 10-60 mol% of a first ionizable lipid, 0-30 mol% of a second ionizable lipid, 5-30 mol% of a phospholipid, 15-70 mol% of a steroid or a derivative thereof, and 0-5 mol% of a polyethylene glycol-lipid conjugate.

In a preferred embodiment, the product is a delivery system or a medicament.

In a preferred embodiment, the use further comprises the step of introducing the blank lipid nanoparticle-based composition into an animal body.

In a preferred embodiment, routes for introducing the blank lipid nanoparticle-based composition into an animal body comprise oral administration, intranasal administration, intravenous administration, intraperitoneal administration, intramuscular administration, intra-articular administration, intralesional administration, intratracheal administration, subcutaneous administration or intradermal administration.

In a preferred embodiment, the animal is a mammal or a non-mammal.

In a preferred embodiment, the biologically active substance comprises: nucleic acids, proteins, polypeptides and small-molecule active substances; the nucleic acids are DNA and/or RNA.

In a preferred embodiment, the nucleic acid comprises at least one of siRNA, miRNA, saRNA, sgRNA, dsRNA, shRNA, smRNA, ssRNA, mRNA, circRNA, snRNA, crRNA, IncRNA, snoRNA, piRNA, pDNA, ssDNA, circular or linear DNA, DNA minicircles and msDNA.

In a preferred embodiment, the amount of the nucleic acid accounts for 0.5%-50% of the total amount of the blank lipid nanoparticles and the nucleic acid; in the blank lipid nanoparticle-based composition, the concentration of the biologically active substance ranges from 50-5000 ng/µl.

In a preferred embodiment, the first ionizable lipid is selected from a compound having a general formula (1), or a pharmaceutically acceptable salt, stereoisomer and tautomer thereof; 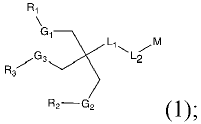
wherein R₁, R₂ and R₃ are each independently H, linear or branched C₅₋₄₀ alkyl, linear or branched C₅₋₄₀ alkenyl, linear or branched C₅₋₄₀ alkynyl, 3- to 6-membered saturated or partially unsaturated cycloalkyl having 1-3 side chains, or 6- to 10-membered aromatic group having 1-3 side chains; wherein the side chains are each independently selected from linear or branched C₁₀₋₃₀ alkyl, linear or branched C₁₀₋₃₀ alkenyl, and linear or branched C₁₀₋₃₀ alkynyl, provided that at most one of R₁, R₂ and R₃ is H;
M is selected from -NR₄R₅, 3- to 6-membered saturated or partially unsaturated heterocyclic group containing at least one nitrogen atom, and 6- to 10-membered heteroaromatic group containing at least one nitrogen atom; wherein the heterocyclic group and the heteroaromatic group are unsubstituted or substituted with one or more -OH, carboxy, amino, oxo or halogen;
R₄ and R₅ are each independently H, linear or branched C₁₋₆ alkyl, linear or branched C₂₋₆ alkenyl, or linear or branched C₂₋₆ alkynyl; wherein the linear or branched C₁₋₆ alkyl, linear or branched C₂₋₆ alkenyl, or linear or branched C₂₋₆ alkynyl is unsubstituted or substituted with one or more -OH, carboxy, aminoamide group, amidino, guanidino, or halogen;
G₁, G₂ and G₃ are each independently -O-, -S-, -NR₆-, -S-S-, -C(=O)-, -C(=S)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₆-, -NR₆-S(=O)₂-, -P(=O)(OR₆)O-, -OP(=O)(OR₆)- or -OP(=O)(OR₆)O-; wherein each R₆ and R₁₃ are each independently selected from H, hydroxy, linear or branched C₁₋₃₀ alkyl or cycloalkyl, and linear or branched C₂₋₃₀ alkenyl;
L₁ is selected from -X₁- or -(CR₇R₈)m-X₁-; wherein each X₁ is independently selected from -O-, -S-, -NR₁₄-, -S-S-, -C(=O)-, -C(=S)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₄-, -NR₁₄-S(=O)₂-, -P(=O)(OR₁₄)O-, -OP(=O)(OR₁₄)- or -OP(=O)(OR₁₄)O-; wherein m is an integer of 2-6, R₇ and R₈ are each independently H, hydroxy, halogen, linear or branched C₁₋₆ alkyl or cycloalkyl, linear or branched C₂₋₆ alkenyl, and each R₁₄ and R₁₅ are each independently selected from H, linear or branched C₁₋₃₀ alkyl or cycloalkyl, linear or branched C₂₋₃₀ alkenyl;
L₂ is -(CR₉R₁₀)ₙ₋ or -(CR₉R₁₀)ₙ-X₂-(CR₁₁R₁₂)ₖ-; wherein X₂ is selected from -O-, -S-, -NR₁₆-, -S-S-, -C(=O)-, -C(=S)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₆-, -NR₁₆-S(=O)₂-, -P(=O)(OR₁₆)O-, -OP(=O)(OR₁₆)- or -OP(=O)(OR₁₆)O-; n is an integer of 1-6; k is an integer of 1-6; R₉, R₁₀, R₁₁ and R₁₂ are each independently H, hydroxy, halogen, linear or branched C₁₋₆ alkyl or cycloalkyl, linear or branched C₂₋₆ alkenyl, and each R₁₆ and R₁₇ are each independently selected from H, linear or branched C₁₋₃₀ alkyl or cycloalkyl, linear or branched C₂₋₃₀ alkenyl;
wherein the alkyl, cycloalkyl and alkenyl in R₄ to R₁₇ are unsubstituted or substituted with one or more groups selected from hydroxy, mercapto, amino, substituted amino or halogen;
the salts exclude quaternary ammonium salts.

In a preferred embodiment, R₁, R₂ and R₃ are each independently the following groups: 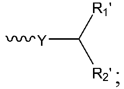 wherein Y is absent, or linear or branched C₁₋₃₀ alkyl or cycloalkyl, linear or branched C₂₋₂₀ alkenyl, linear or branched C₂₋₂₀ alkynyl; R₁' and R₂' are each independently H, linear or branched C₁₋₃₀ alkyl, linear or branched C₂₋₃₀ alkenyl, linear or branched C₂₋₃₀ alkynyl, and the total carbon chain length of Y, R₁' and R₂' is 8-40.

In a preferred embodiment, R₁, R₂ and R₃ are each independently selected from the following groups: 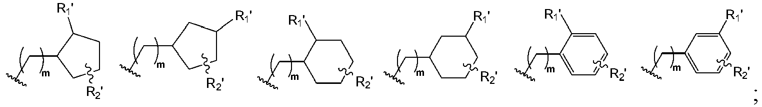 wherein R₁' and R₂' are each independently H, linear or branched C₁₋₃₀ alkyl, linear or branched C₂₋₃₀ alkenyl, linear or branched C₂₋₃₀ alkynyl, and the total carbon chain length of R₁' and R₂' is 8-30.

In a preferred embodiment, R₁, R₂ and R₃ are each independently selected from any one of the following groups:

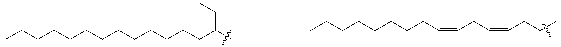

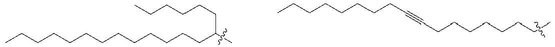

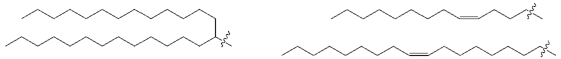

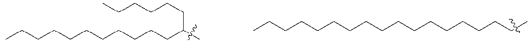

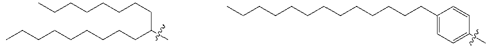

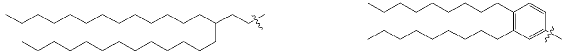

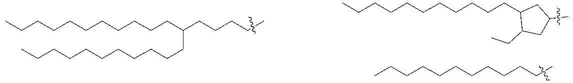

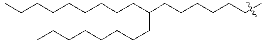

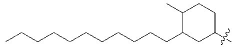

In a preferred embodiment, G₁, G₂ and G₃ are each independently -O-, -S-, -NR₆-, -S-S-, -C(=O)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -P(=O)(OR₆)O-, -OP(=O)(OR₆)- or -OP(=O)(OR₆)O-.

In a preferred embodiment, L₁ is selected from -(CR₇R₈)ₘ-X₁-, wherein X₁ is selected from -O-, -S-, -NR₁₄-, -S-S-, -C(=O)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -P(=O)(OR₁₄)O-, -OP(=O)(OR₁₄)- or -OP(=O)(OR₁₄)O-.

In a preferred embodiment, L₂ is -(CR₉R₁₀)ₙ-X₂-(CR₁₁R₁₂)ₖ-, wherein X₂ is selected from -O-, -S-, -NR₁₆-, -S-S-, -C(=O)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -P(=O)(OR₁₆)O-, -OP(=O)(OR₁₆)- or -OP(=O)(OR₁₆)O-.

In a preferred embodiment, M is selected from the following structures: 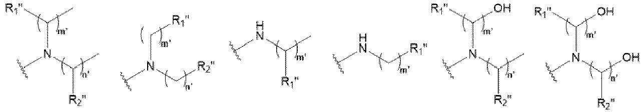 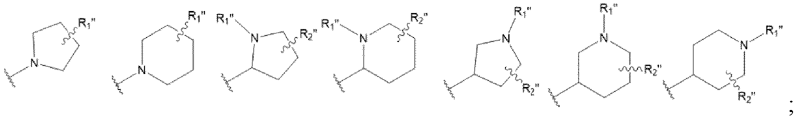 wherein m' and n' are each independently an integer of 0-6, R1" and R2" are each independently H, C₁₋₆ alkyl, C₂₋₆ alkenyl, guanidino, amidino, amido, aliphatic amino, 3- to 10-membered nitrogen-containing heterocycles; the nitrogen-containing heterocycles are selected from pyrrole, imidazole, pyridine, pyrazole, triazole, oxazole, isoxazole, thiophene, isothiazole, pyridazine, pyrazine, piperazine, indole, benzimidazole, carbazole, quinoline, isoquinoline, purine and pyrimidine, and tautomeric forms thereof, which are unsubstituted or optionally substituted with one or more organic groups selected from hydroxy, mercapto, amino, substituted amino, halogen, C₁₋₆ alkyl, C₂₋₆ alkenyl, C₂₋₆ alkynyl and C₆₋₁₄ aryl.

In a preferred embodiment, the compound of formula (1) is selected from the compound shown in formula (1A):

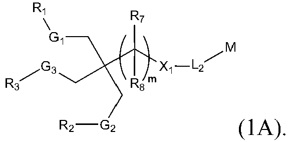

In a preferred embodiment, the compound of formula (1) is selected from the compound shown in formula (1B):

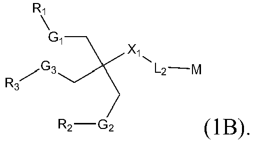

In a preferred embodiment, the compound of formula (1) is selected from the compound shown in formula (1C):

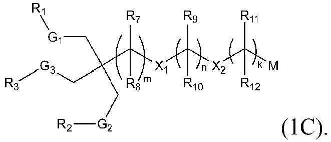

In a preferred embodiment, the compound of formula (1) is selected from the compound shown in formula (1D):

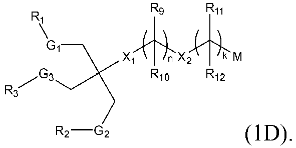

In a preferred embodiment, the compound of formula (1) is selected from the compound shown in formula (1E):

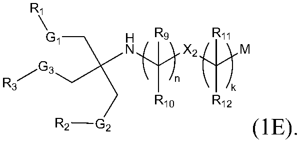

In a preferred embodiment, the compound of formula (1) is selected from the compound shown in formula (1F):

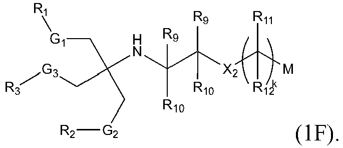

In a preferred embodiment, the compound of formula (1) is selected from the compound shown in formula (1G):

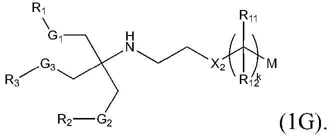

In a preferred embodiment, the compound of formula (1) is selected from the compound shown in formula (1H):

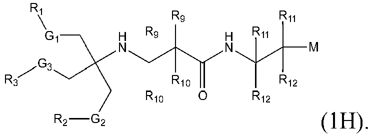

In a preferred embodiment, M is selected from any one of the following groups:

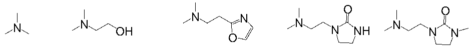

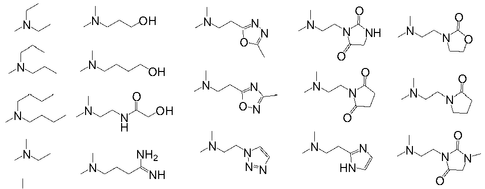

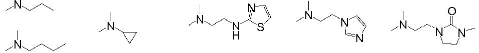

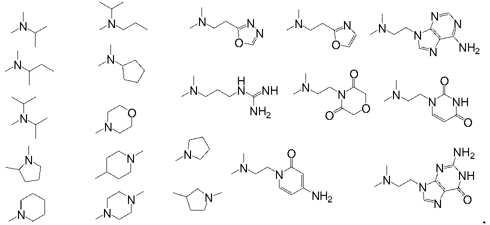

In a preferred embodiment, the compound of formula (1) is selected from the compound shown in formula (1I):

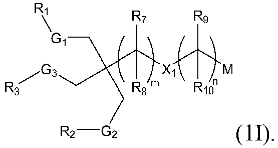

In a preferred embodiment, the compound of formula (1) is selected from the compound shown in formula (1J):

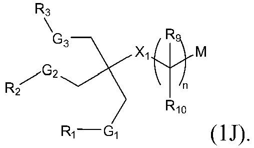

In a preferred embodiment, the compound of formula (1) is selected from the compound shown in formula (1K):

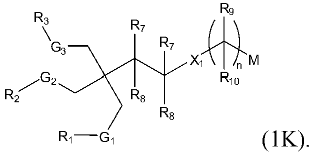

In a preferred embodiment, Y is absent, and the compound of formula (1) is selected from the compound shown in formula (1L):

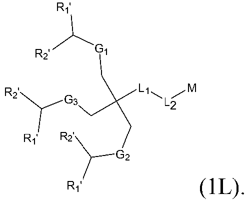

Wherein R₁' and R₂' are each independently selected from H, linear or branched C₁₋₃₀ alkyl, linear or branched C₂₋₃₀ alkenyl, linear or branched C₂₋₃₀ alkynyl, and the total carbon chain length of R₁' and R₂' is 8-40.

In a preferred embodiment, the compound of formula (1) is selected from the compound shown in formula (1M):

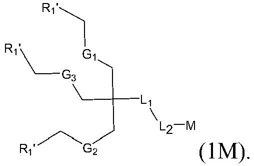

The compound of formula (1) is selected from:

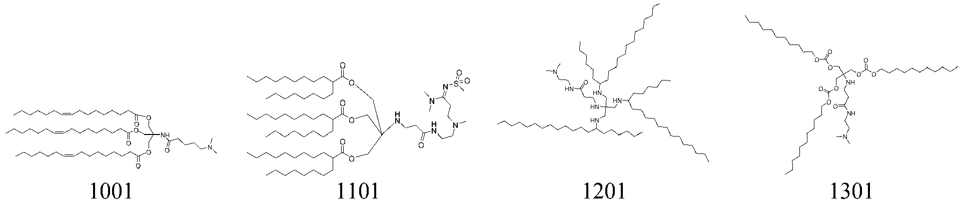

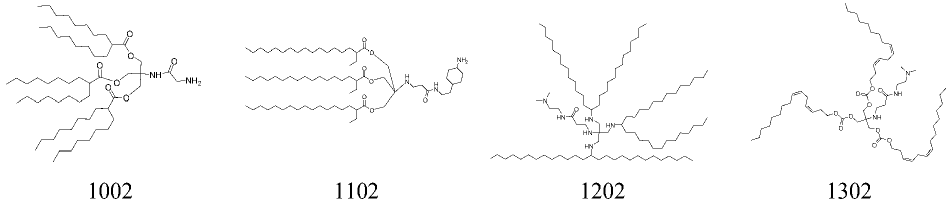

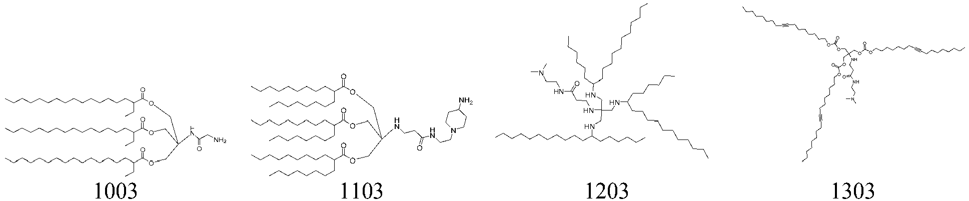

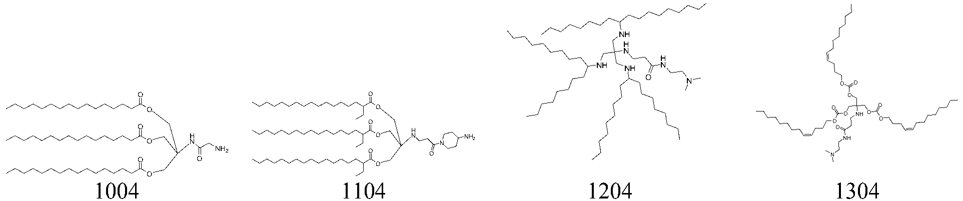

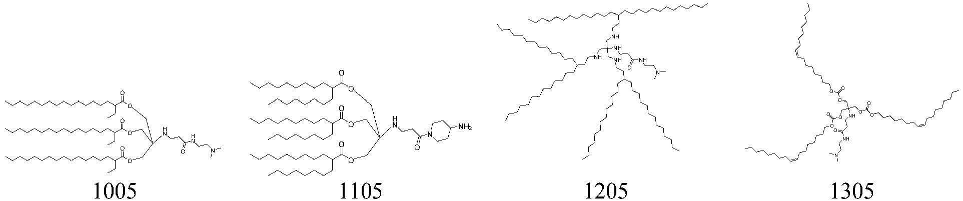

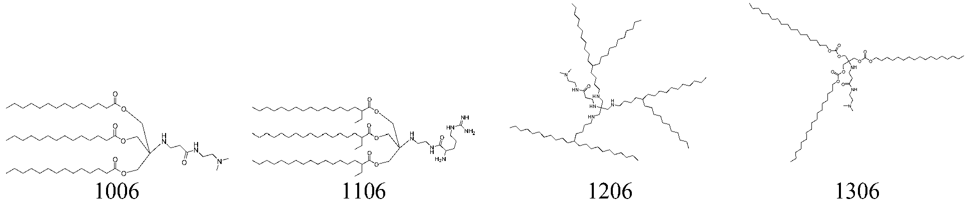

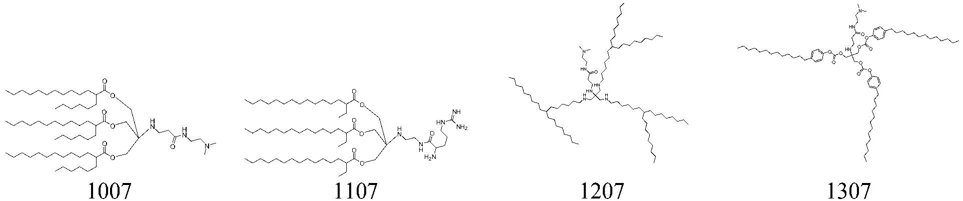

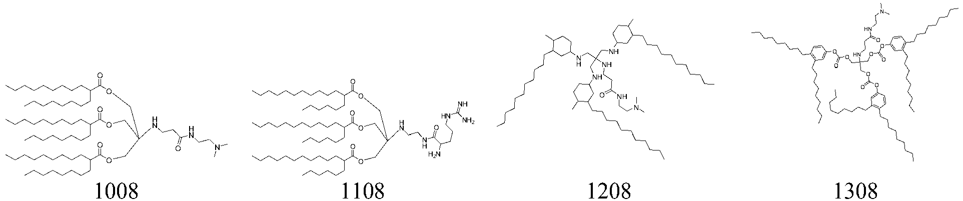

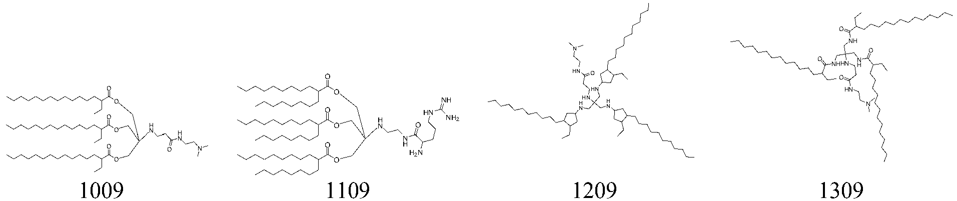

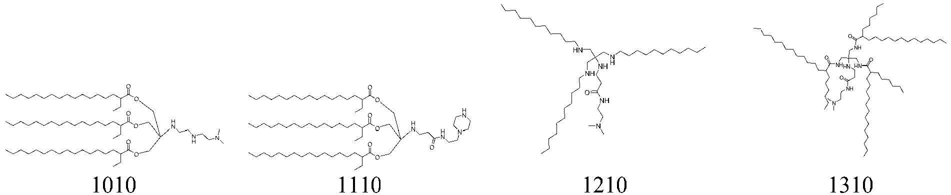

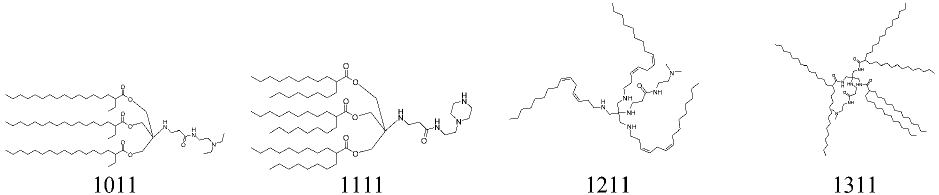

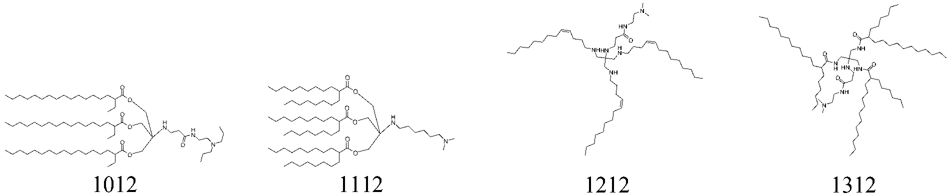

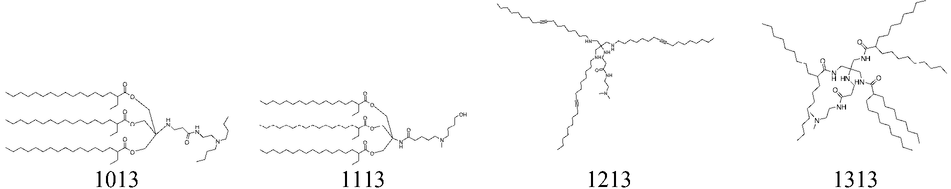

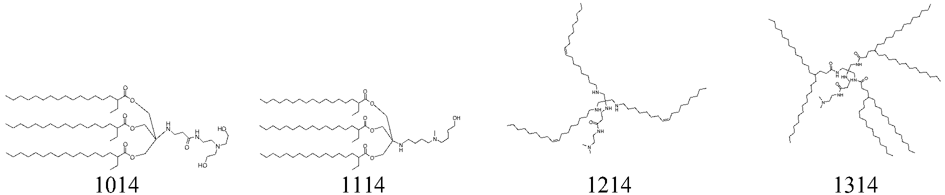

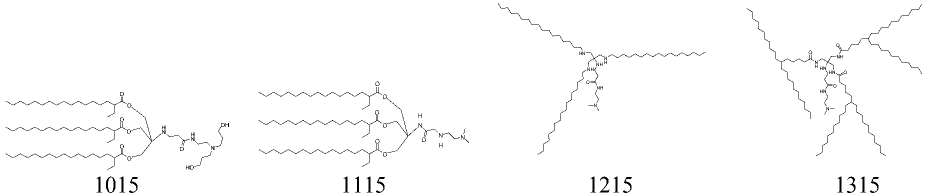

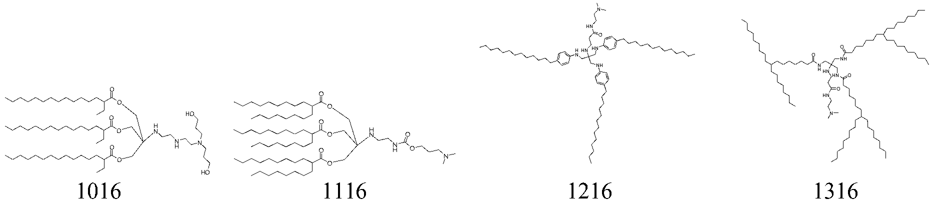

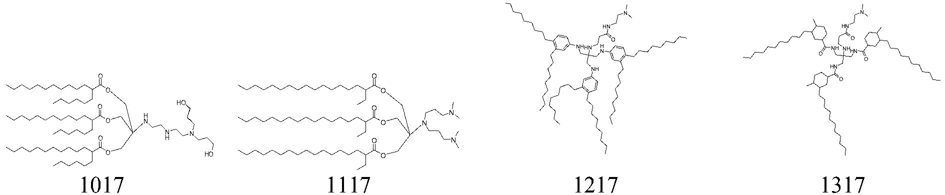

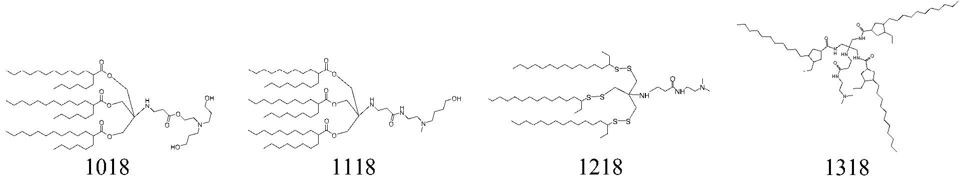

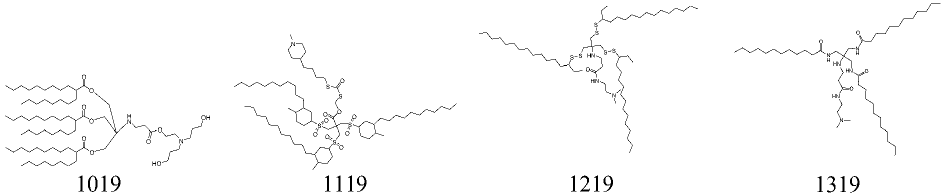

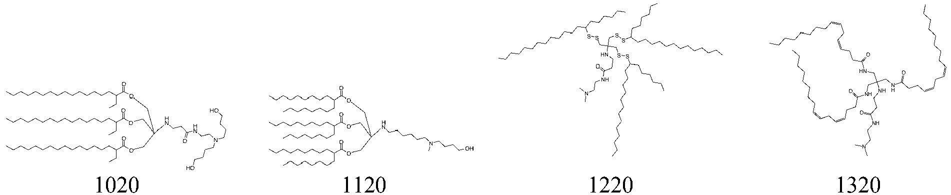

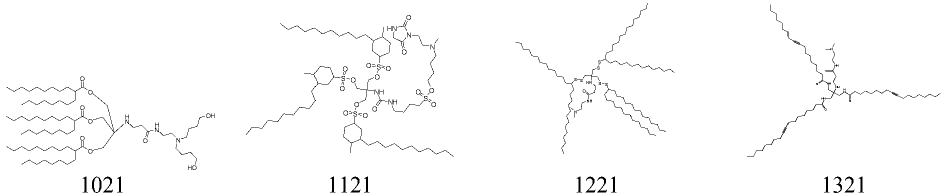

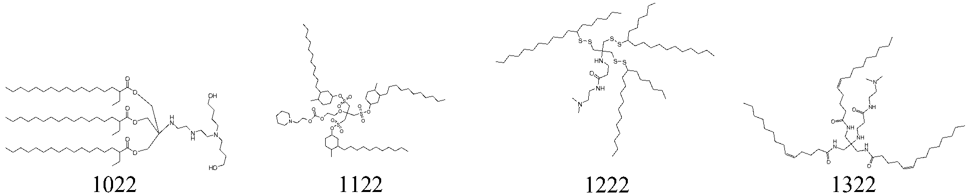

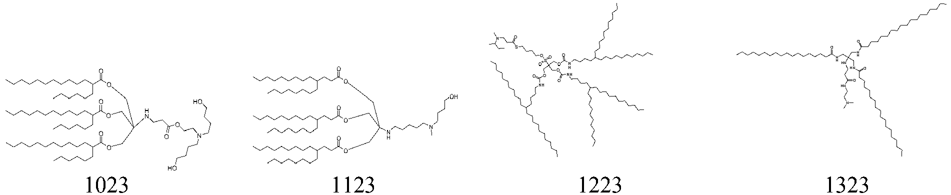

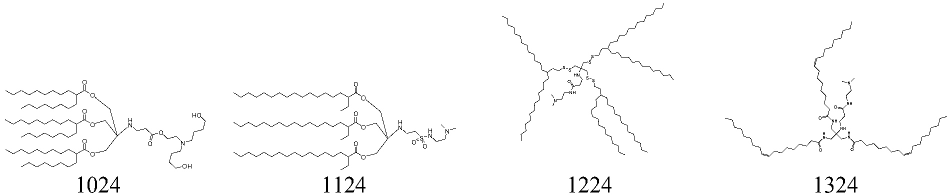

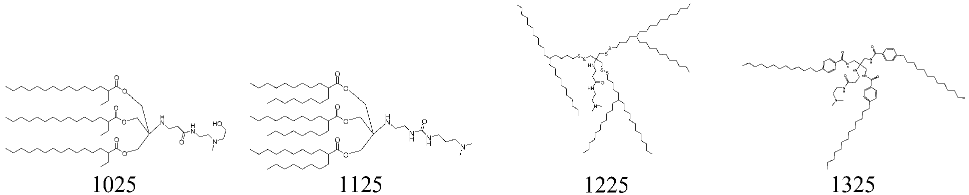

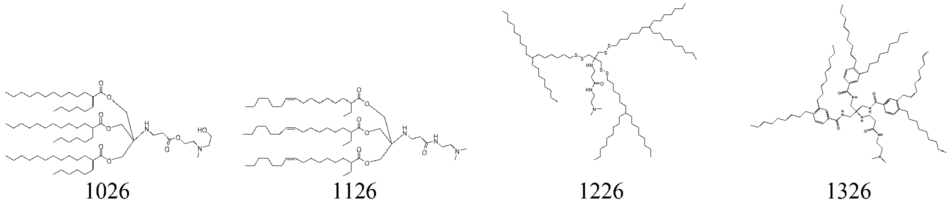

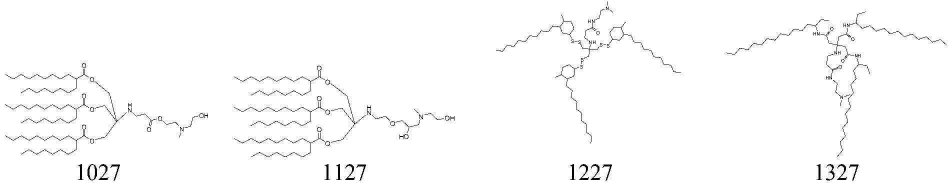

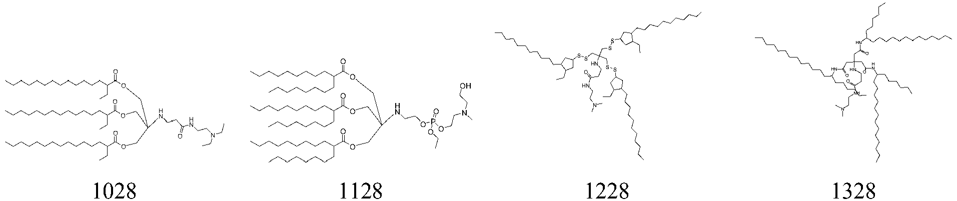

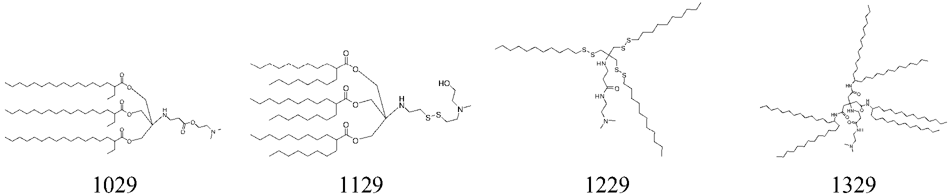

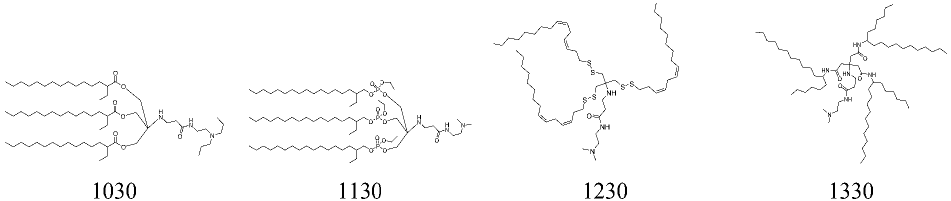

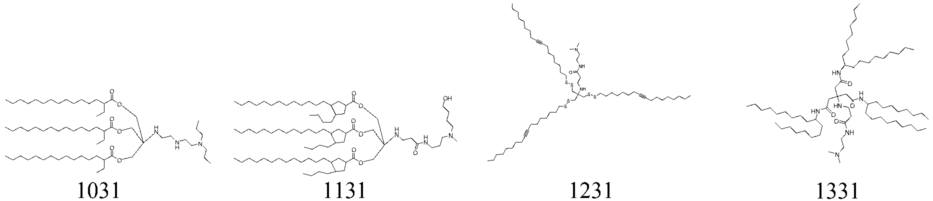

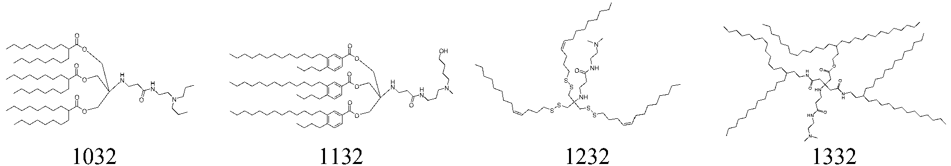

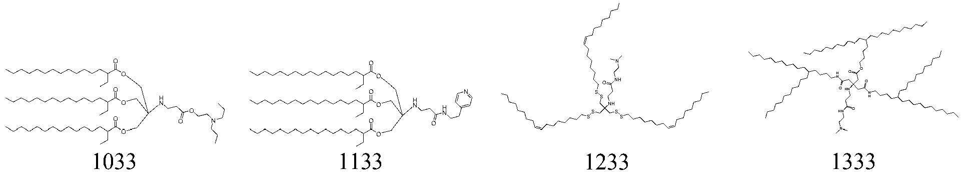

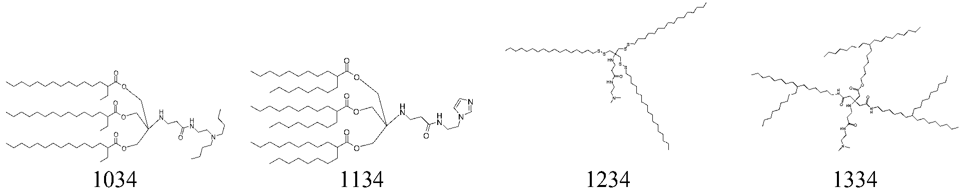

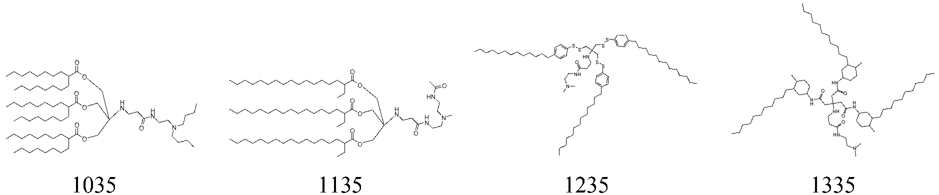

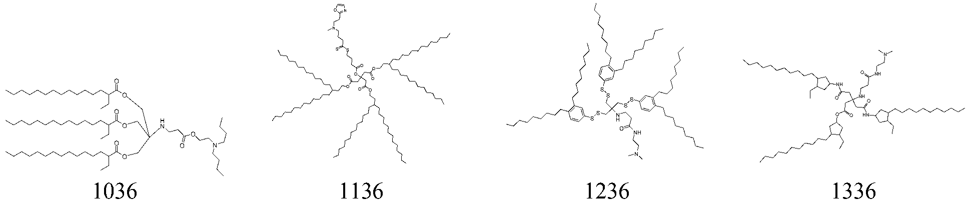

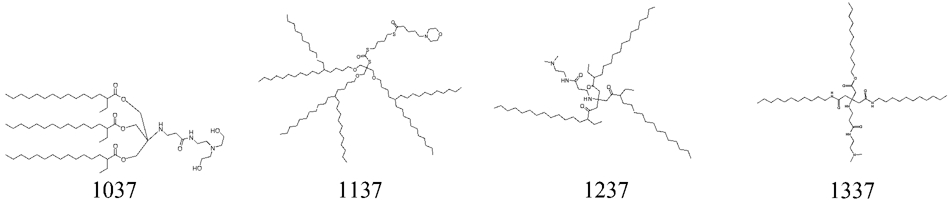

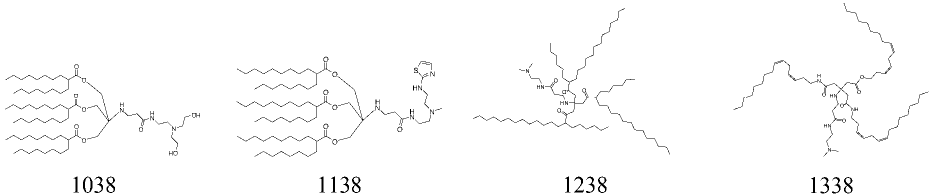

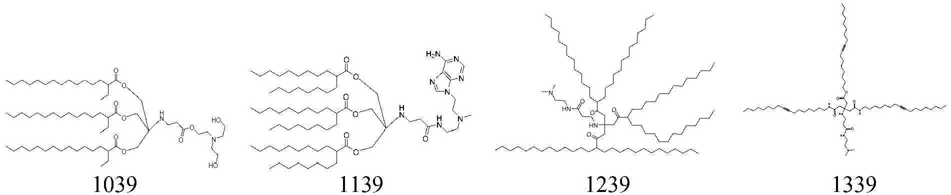

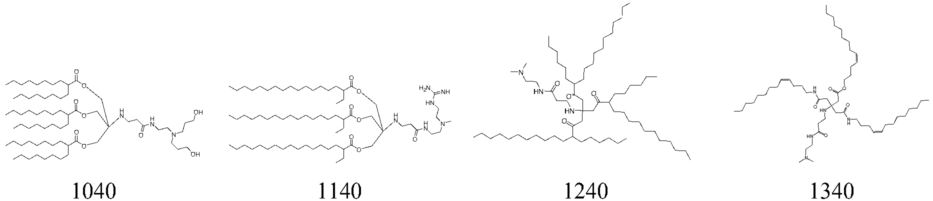

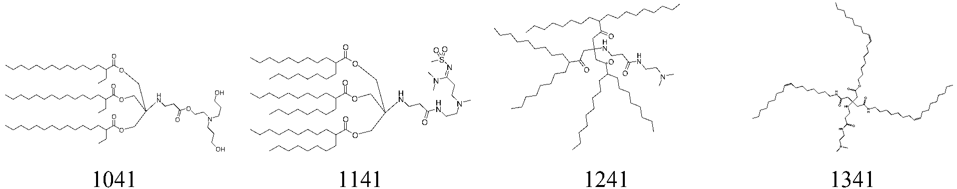

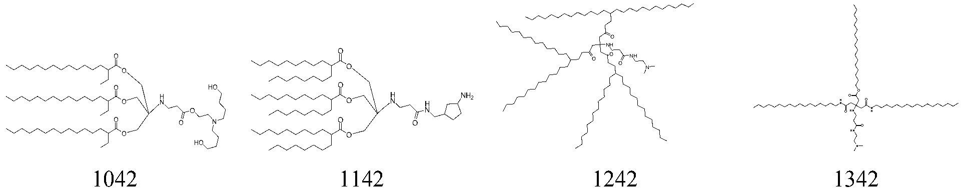

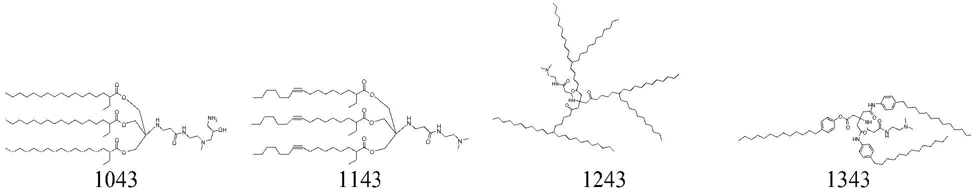

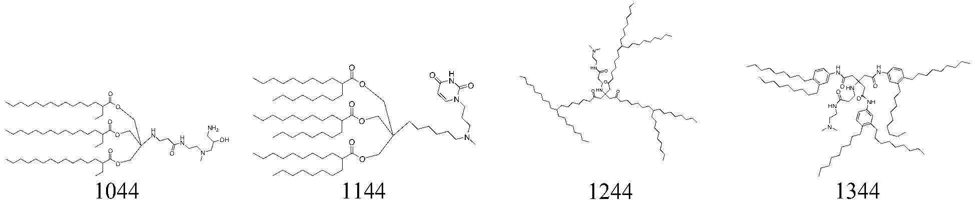

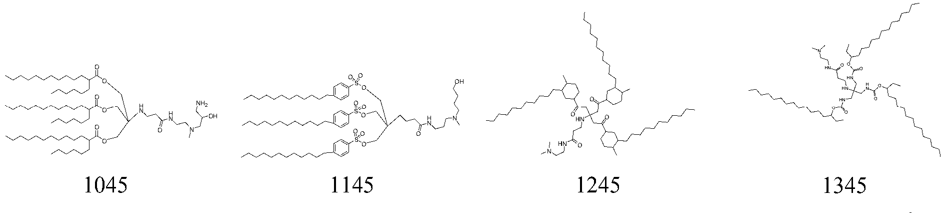

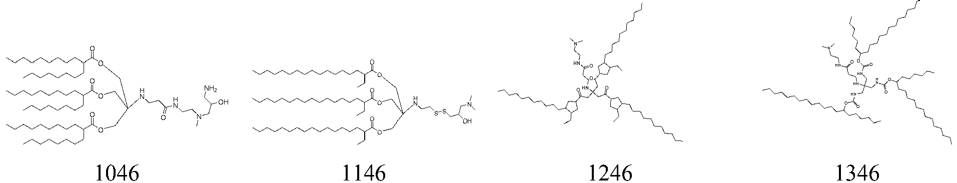

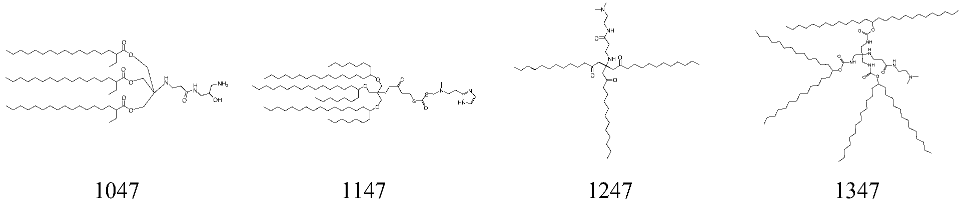

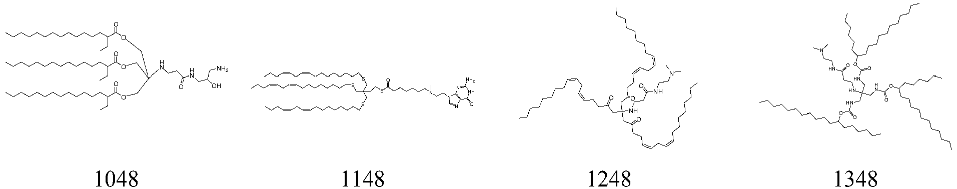

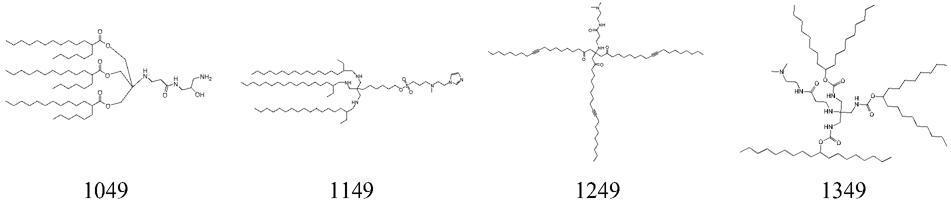

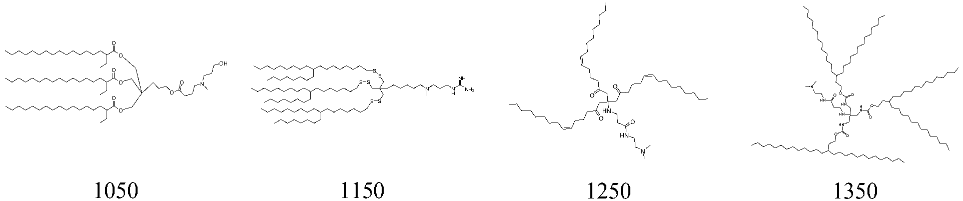

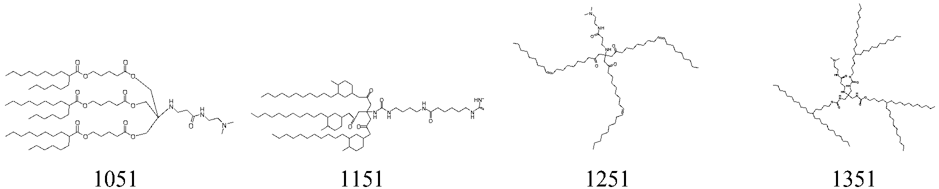

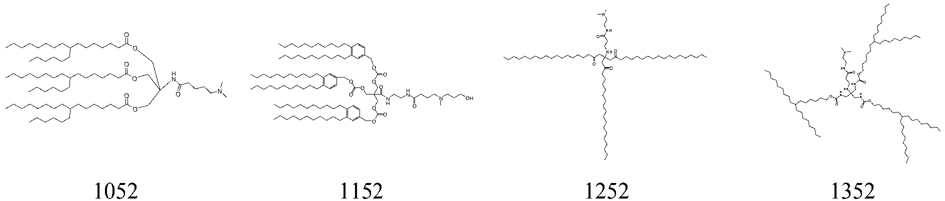

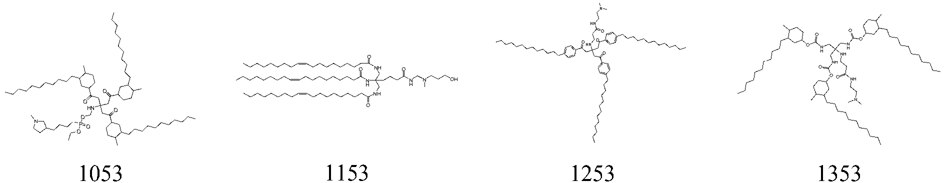

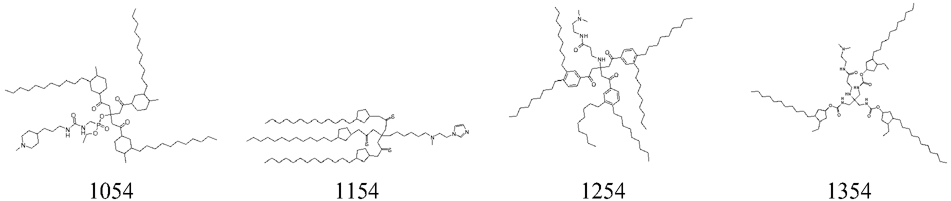

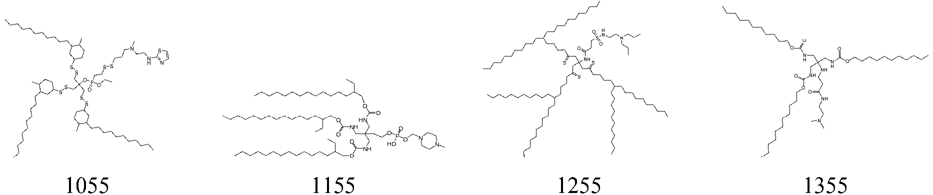

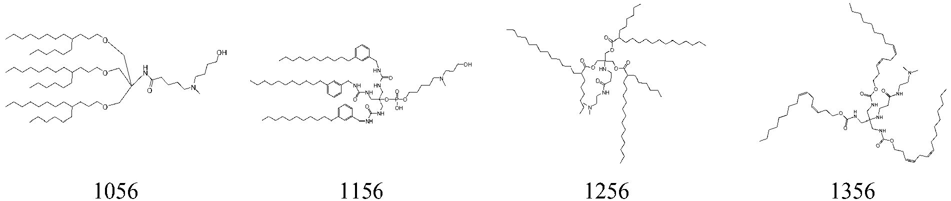

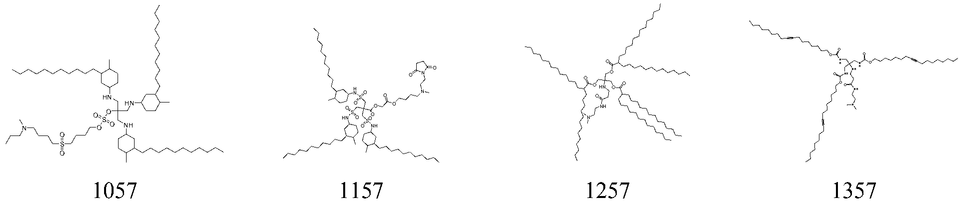

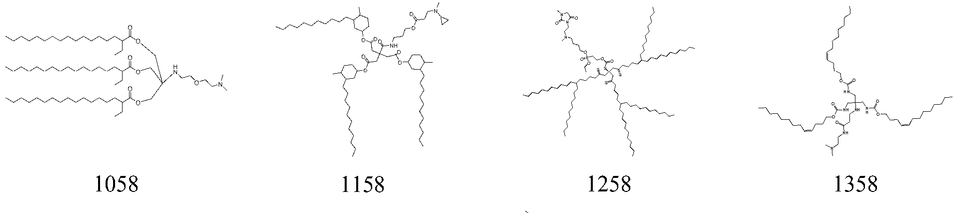

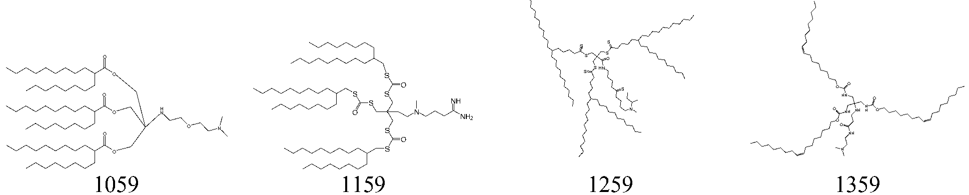

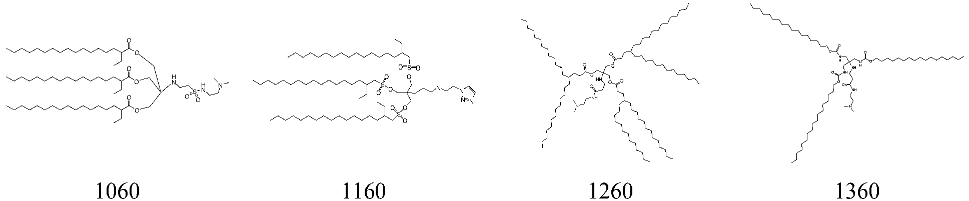

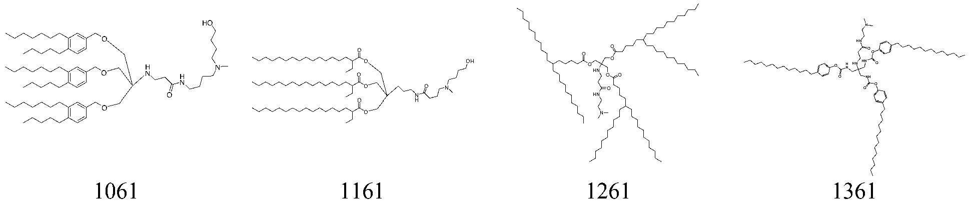

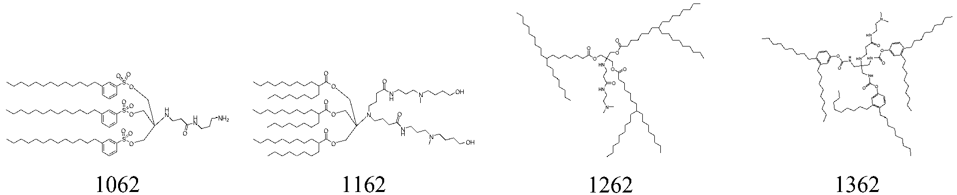

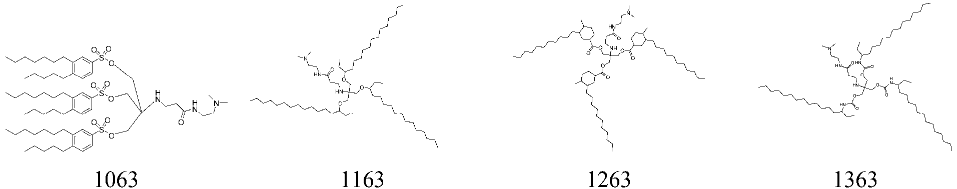

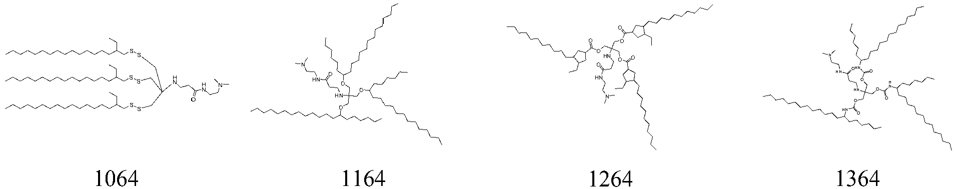

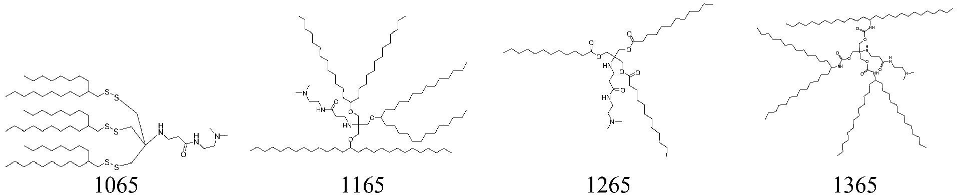

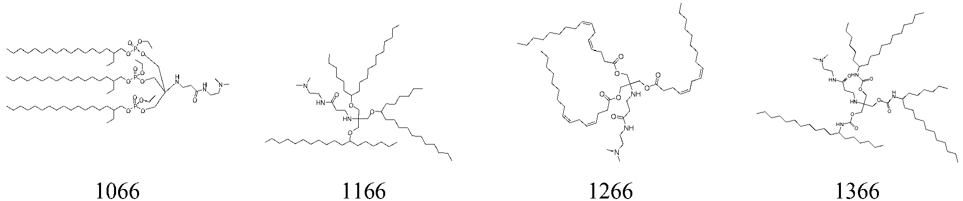

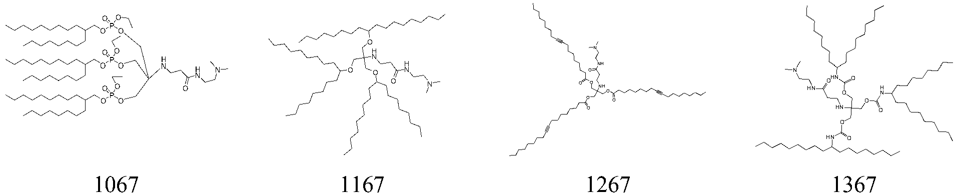

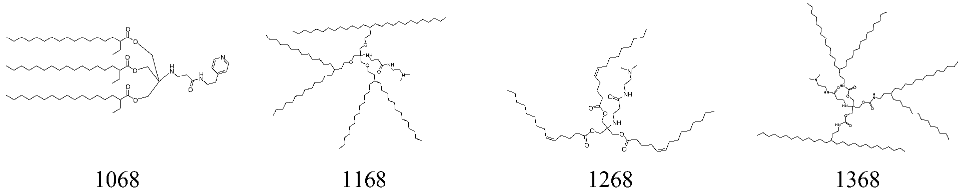

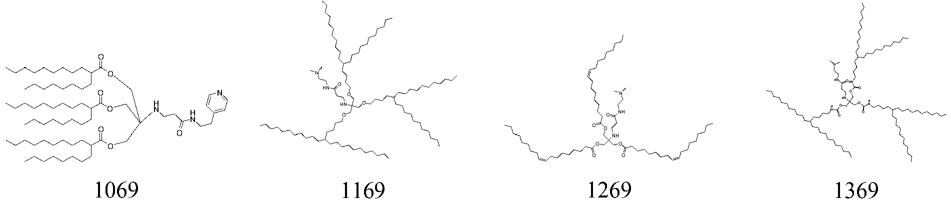

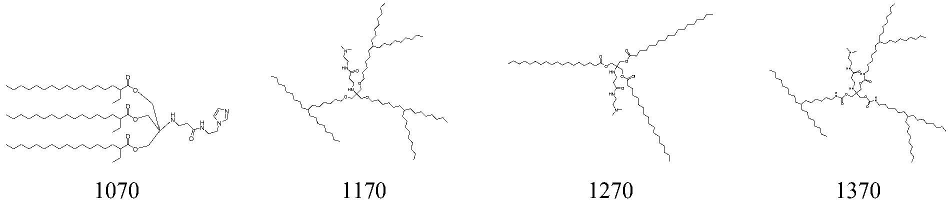

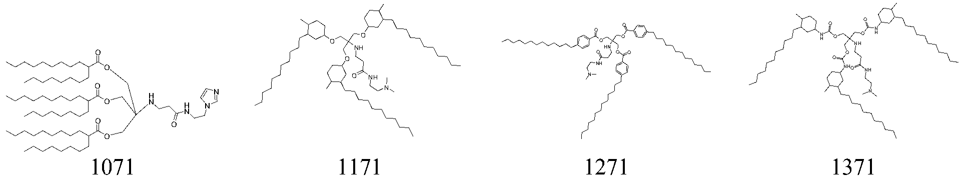

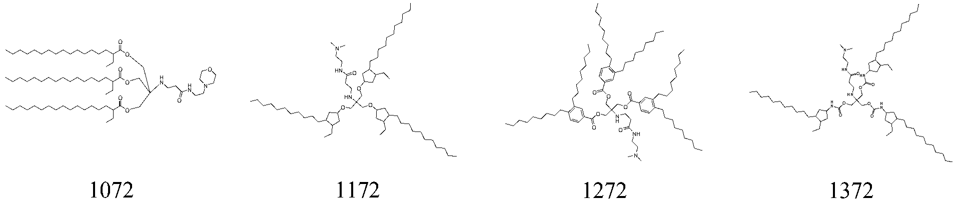

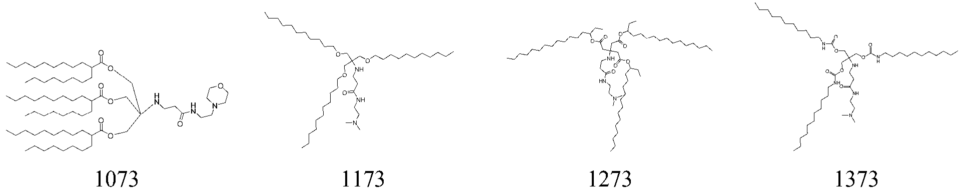

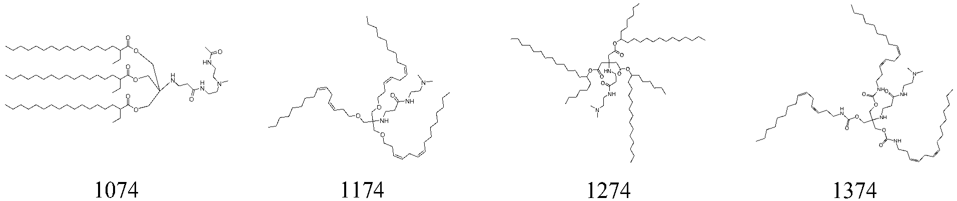

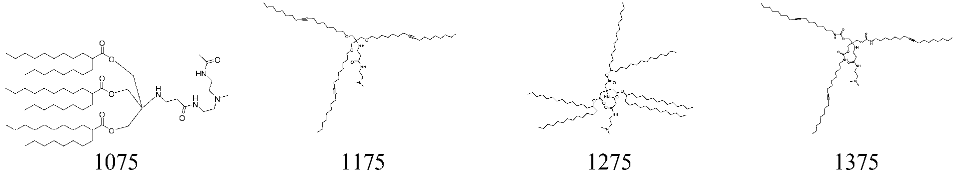

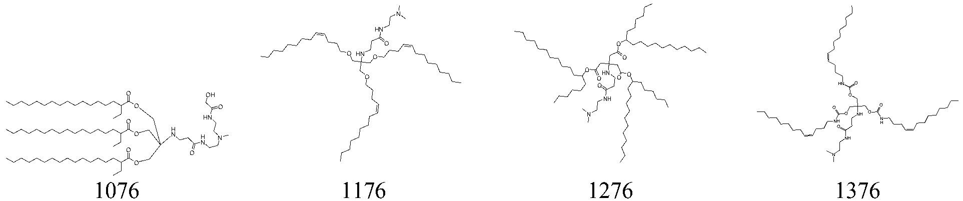

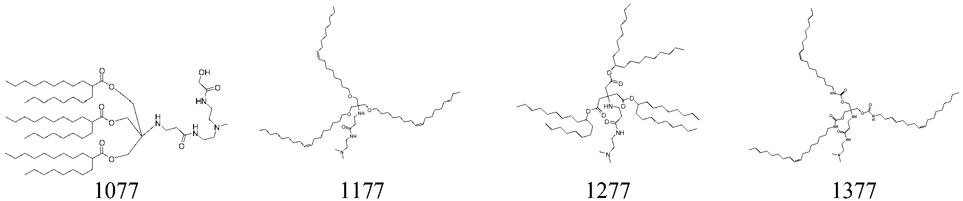

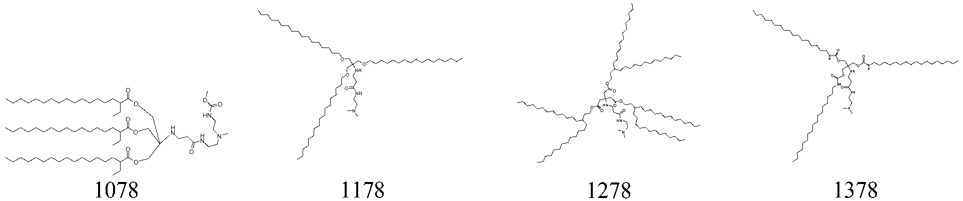

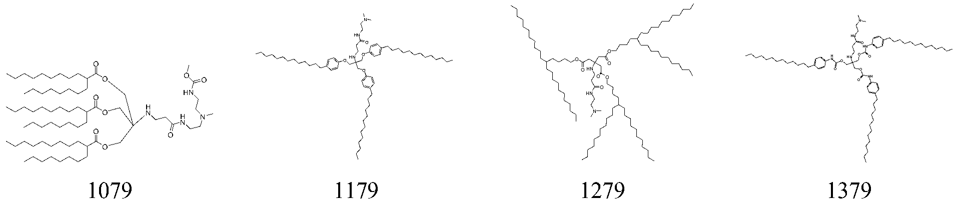

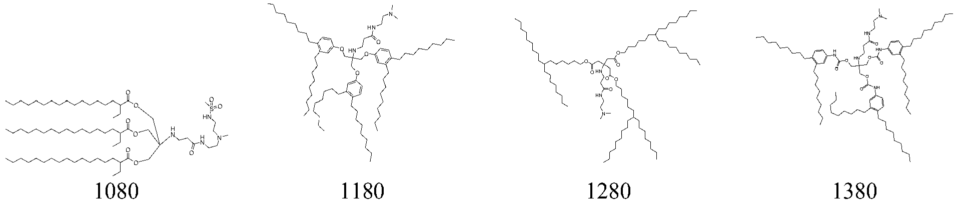

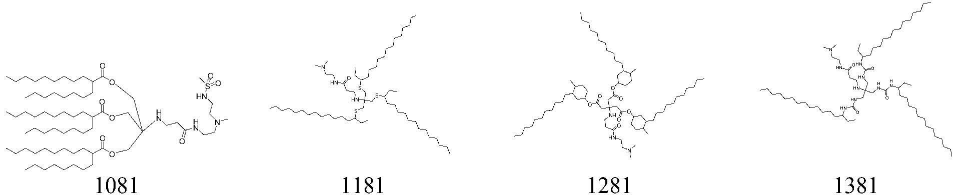

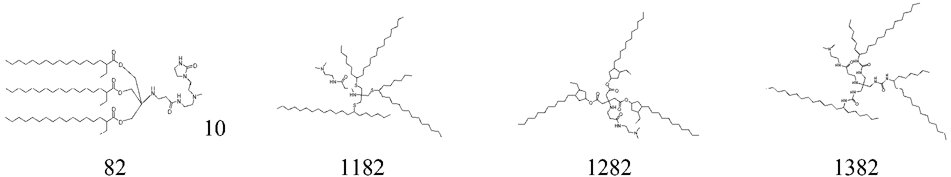

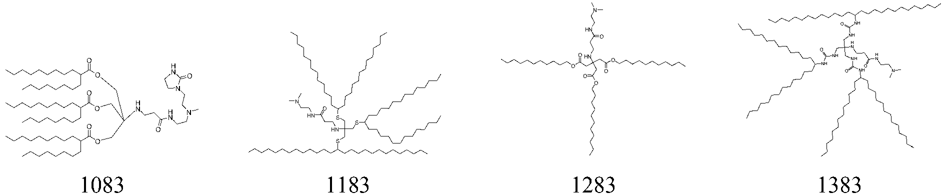

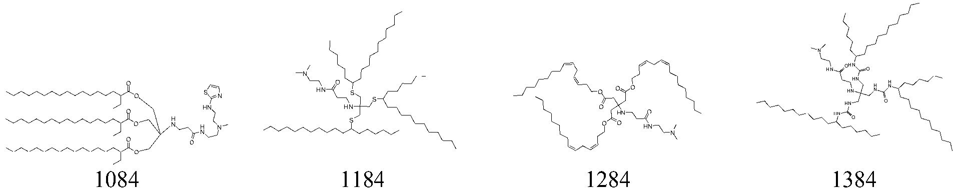

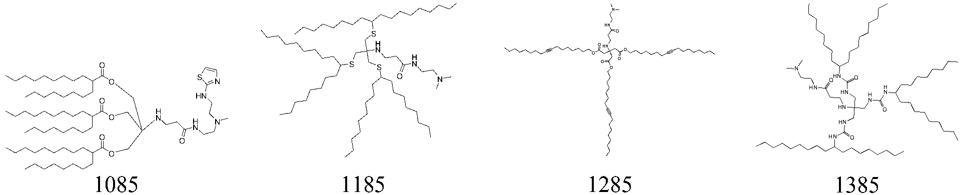

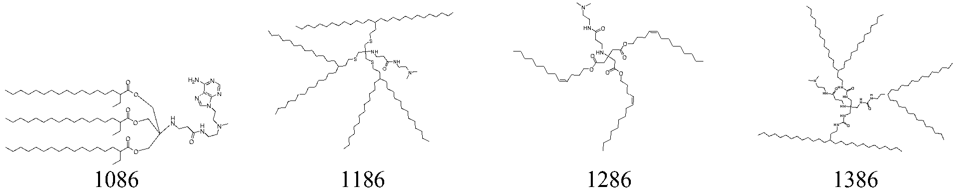

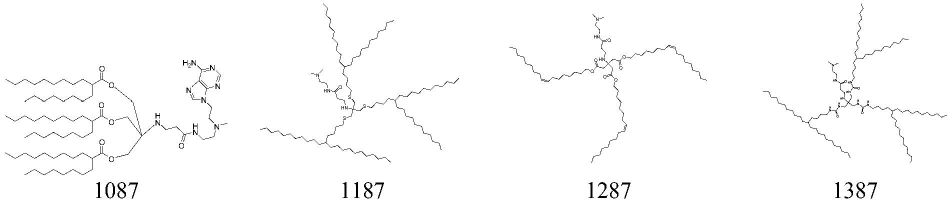

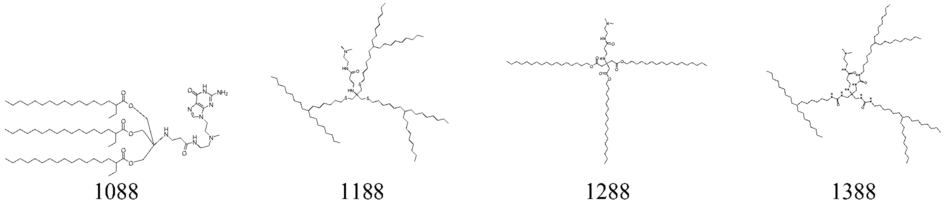

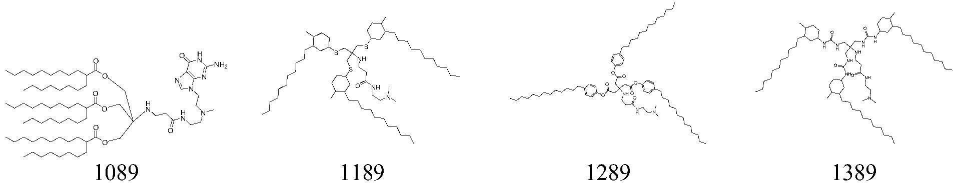

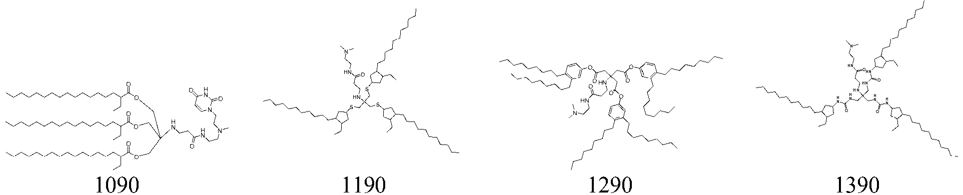

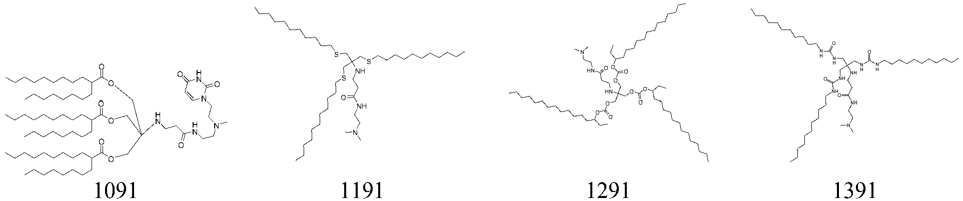

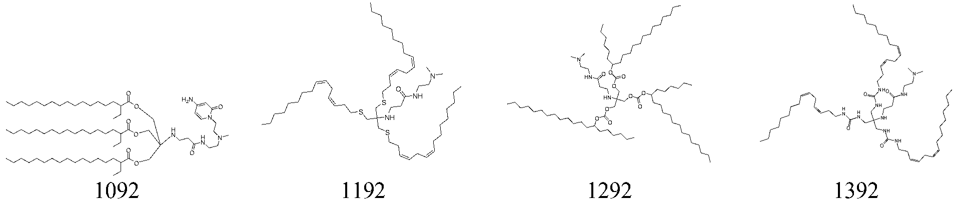

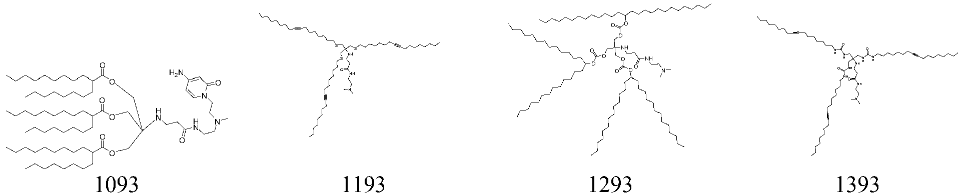

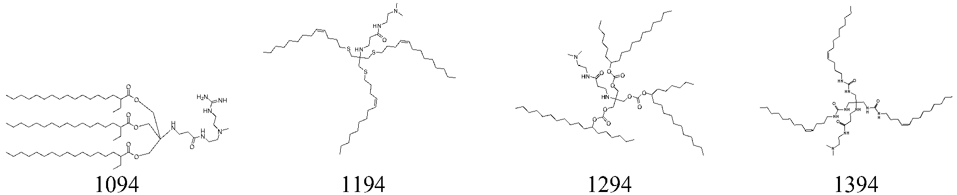

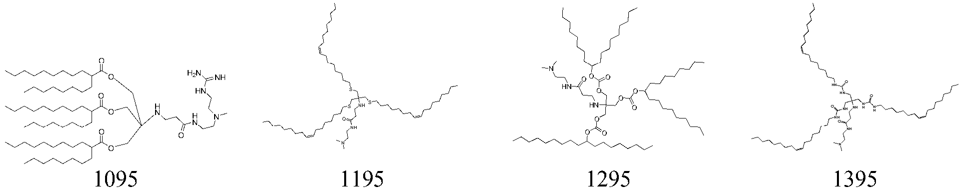

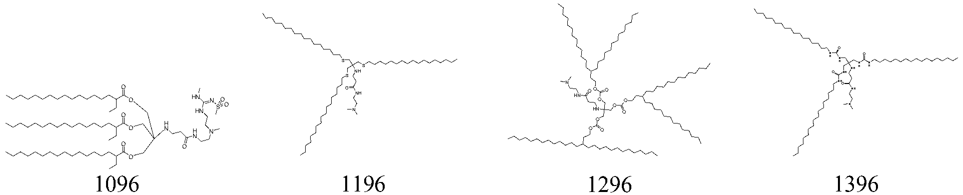

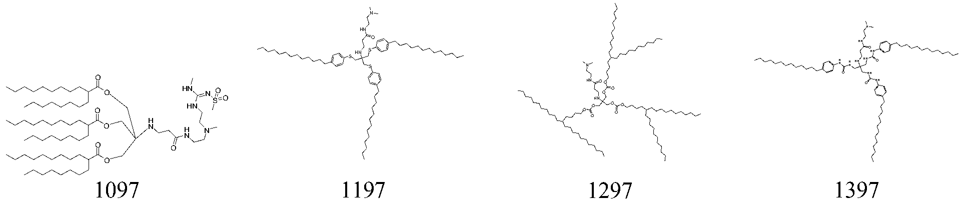

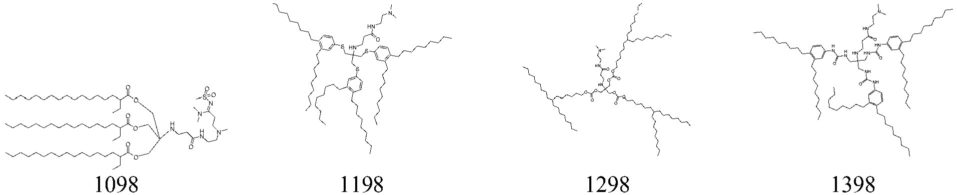

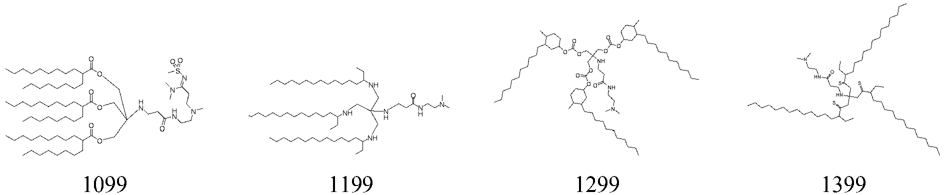

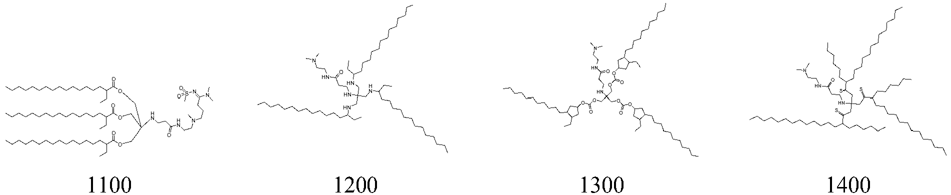

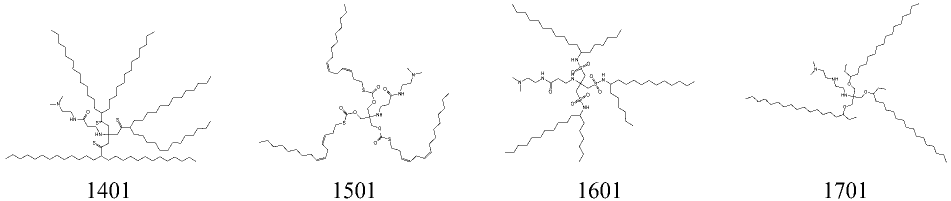

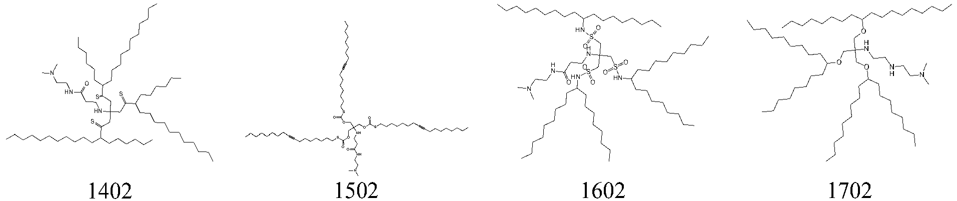

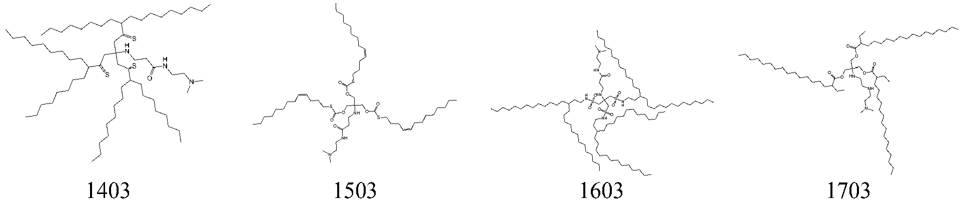

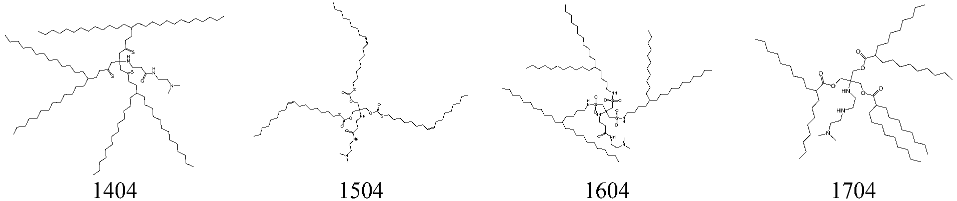

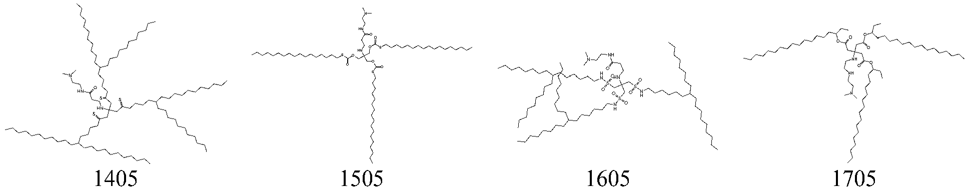

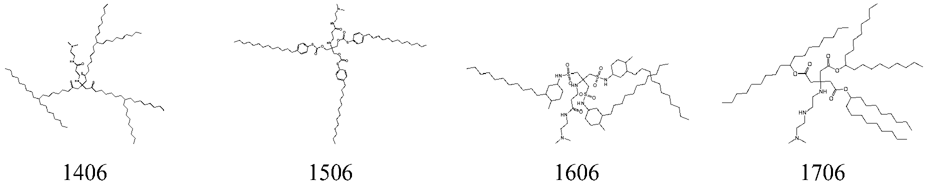

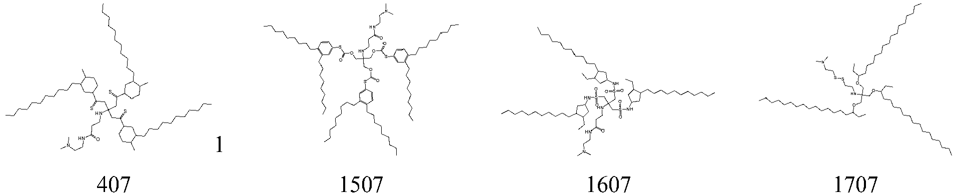

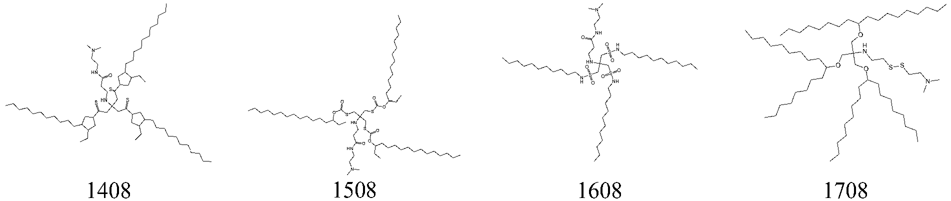

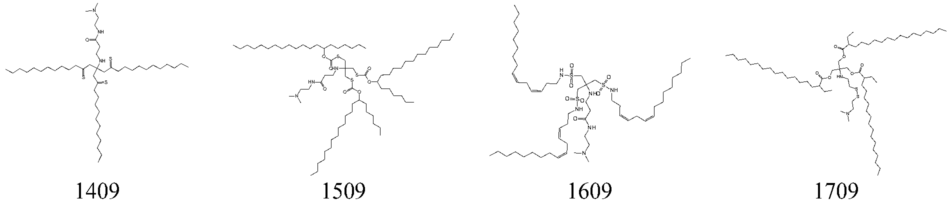

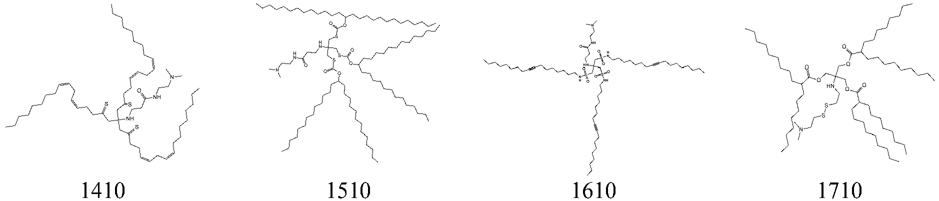

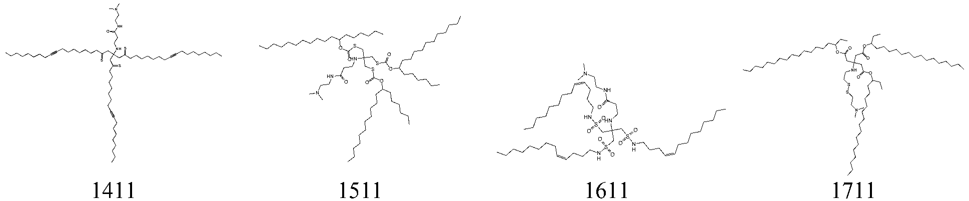

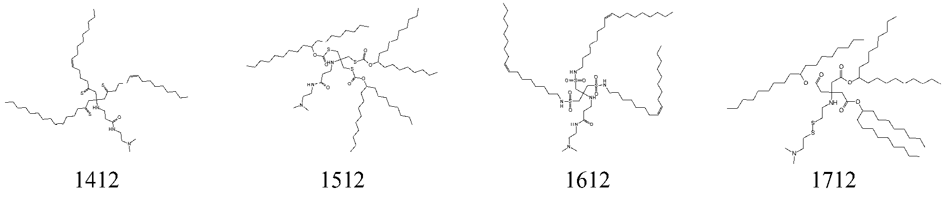

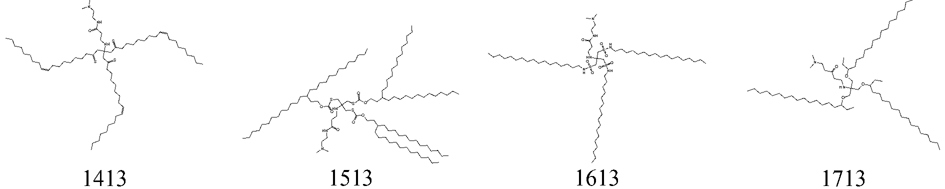

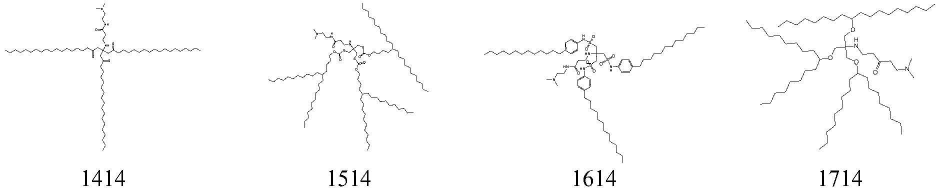

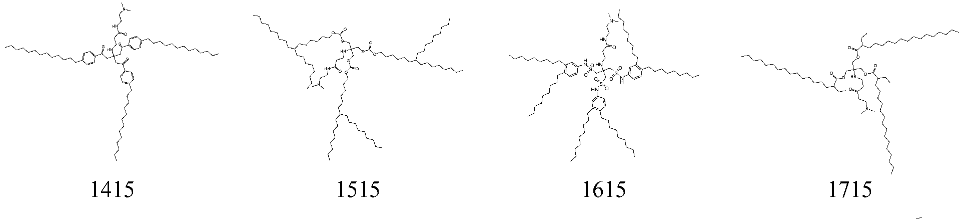

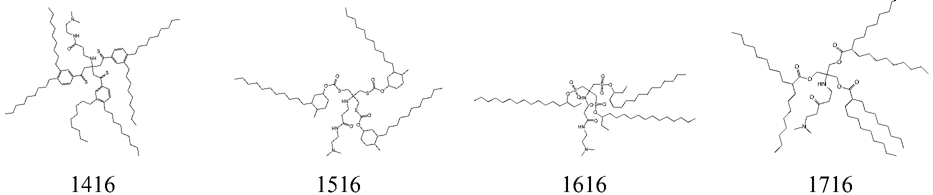

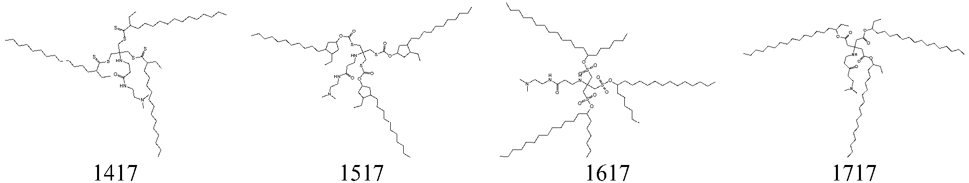

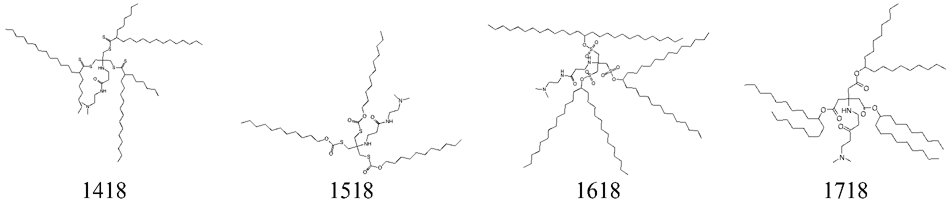

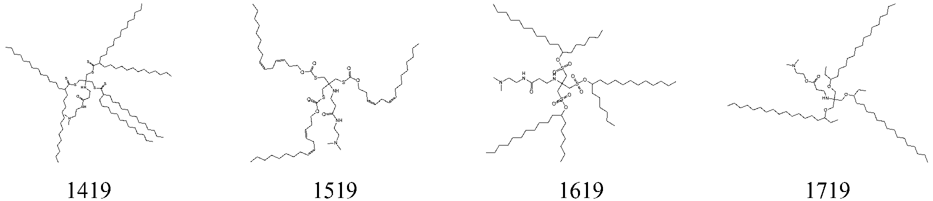

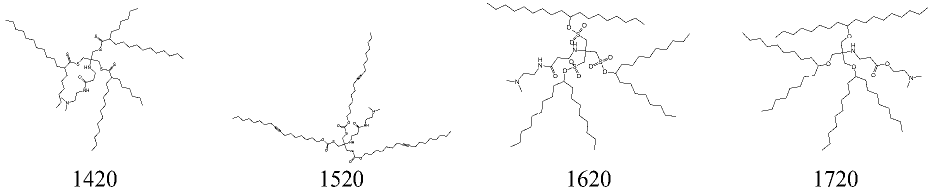

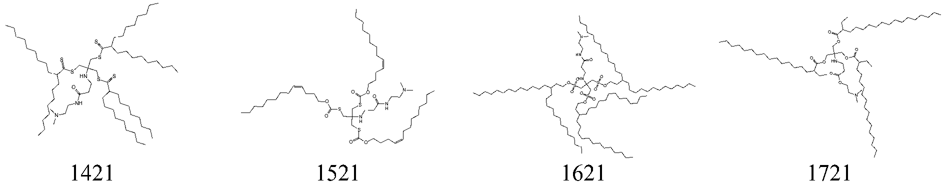

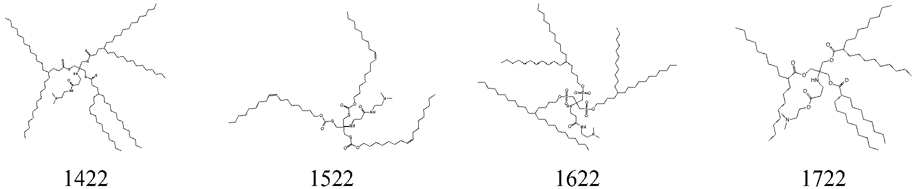

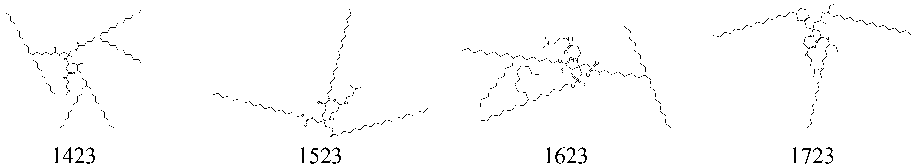

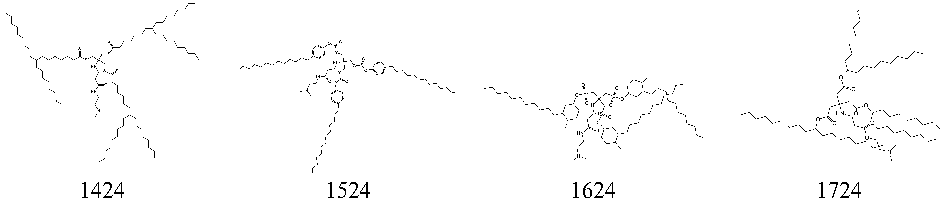

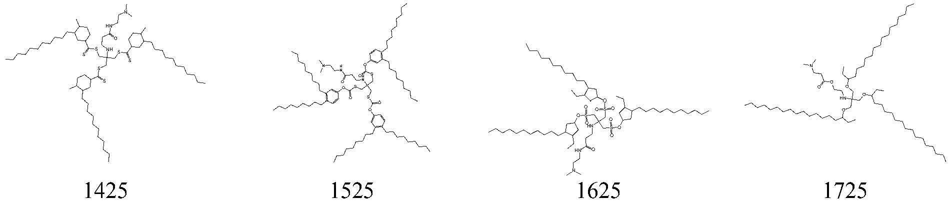

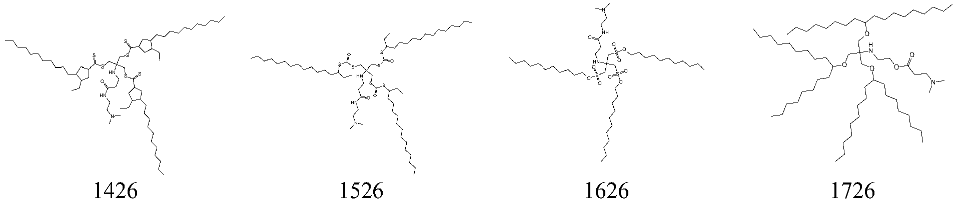

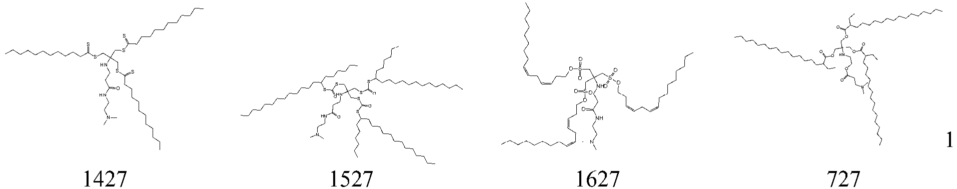

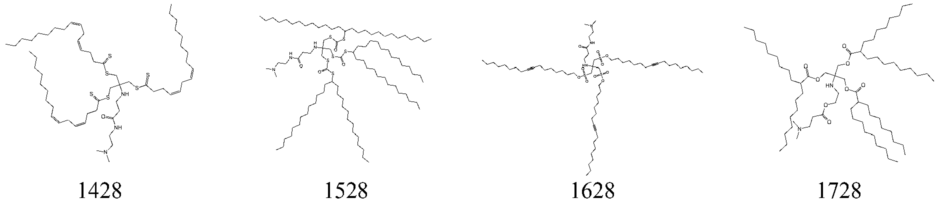

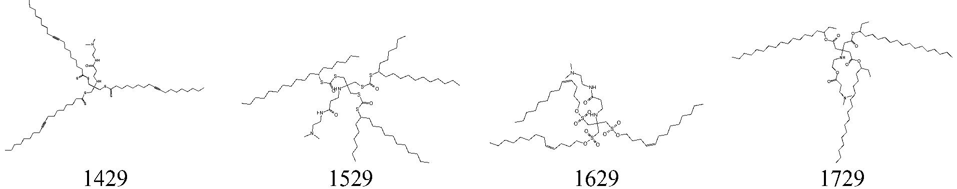

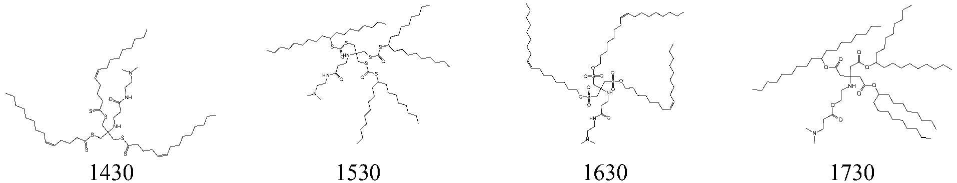

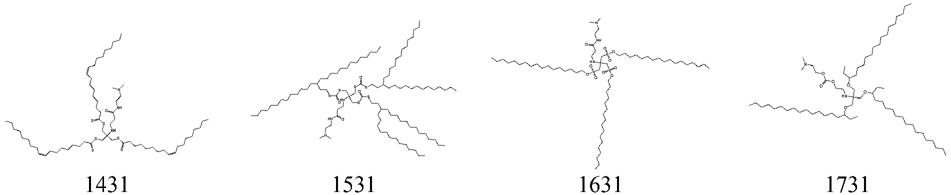

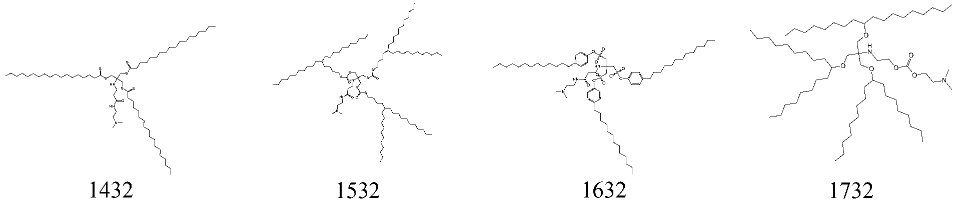

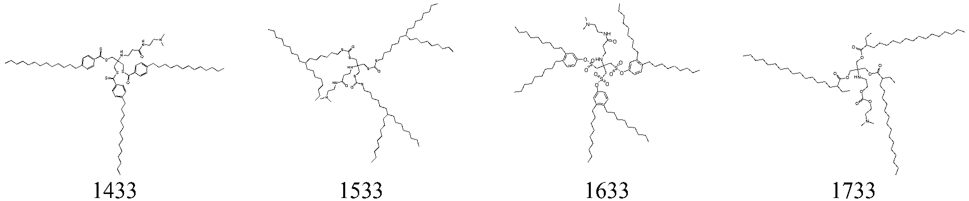

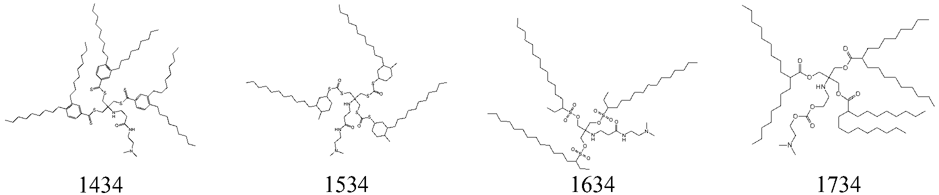

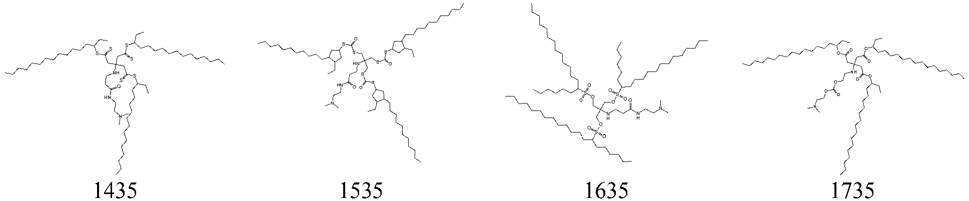

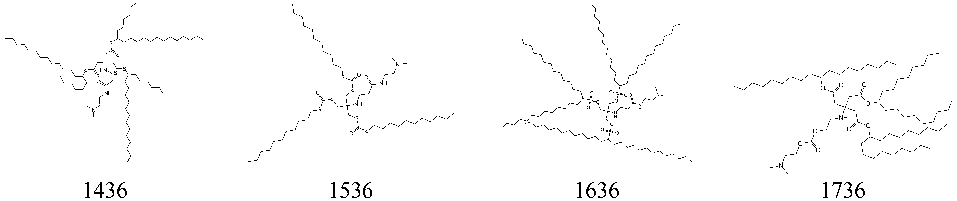

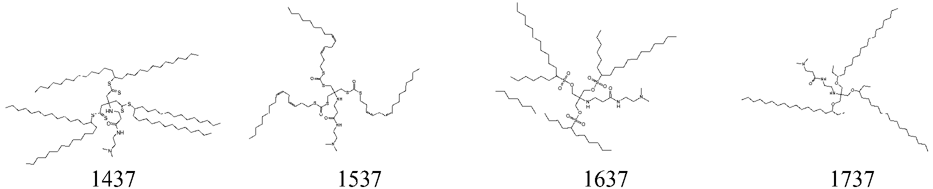

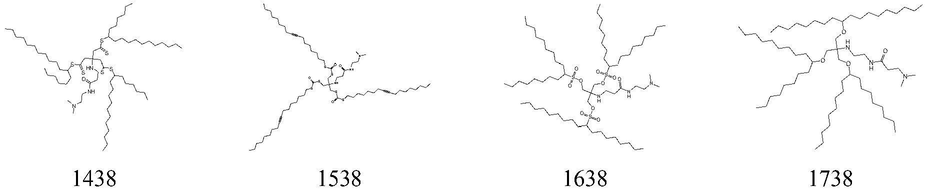

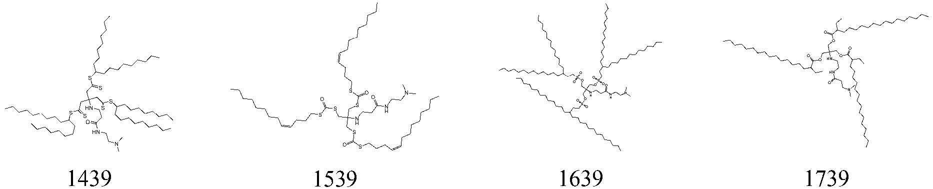

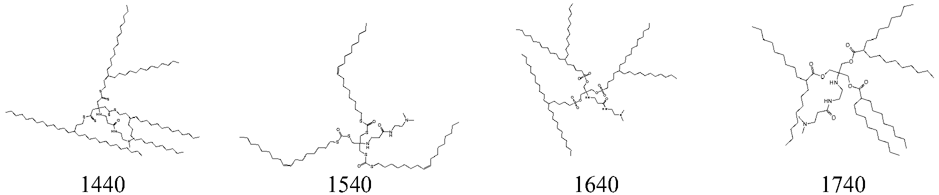

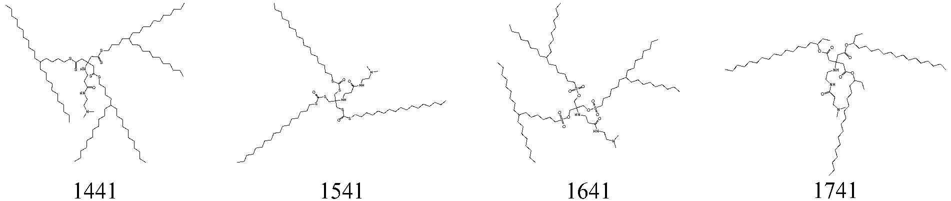

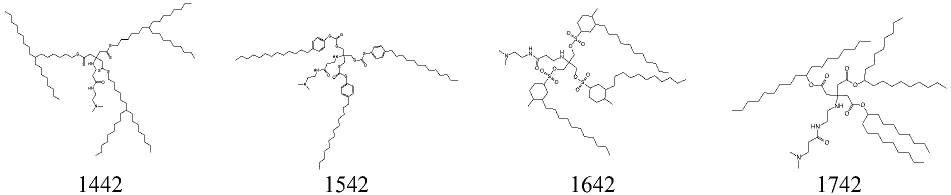

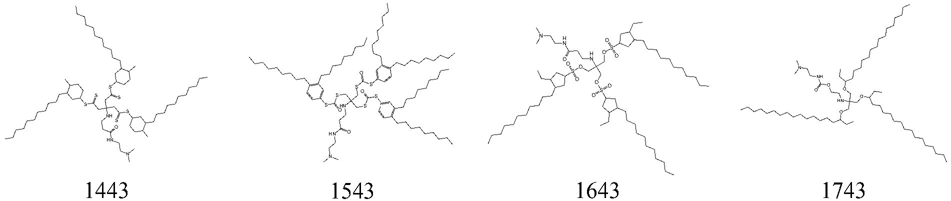

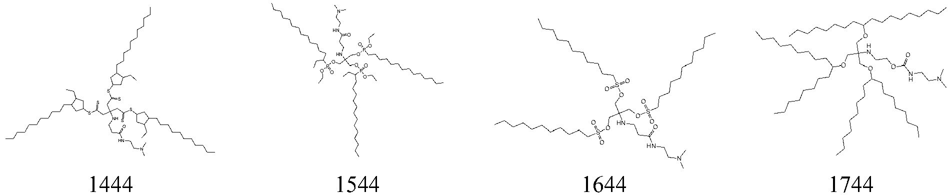

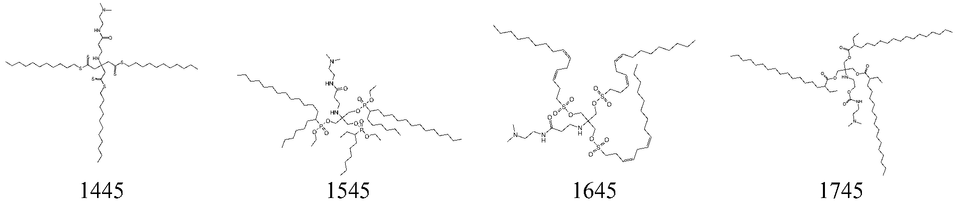

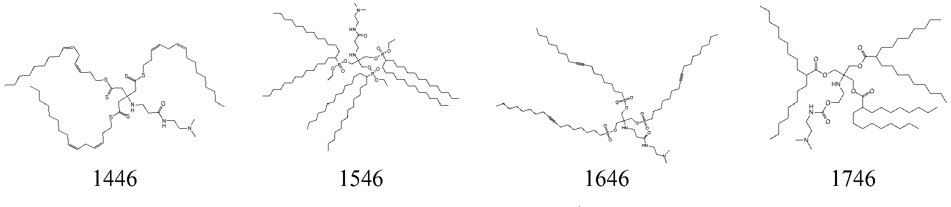

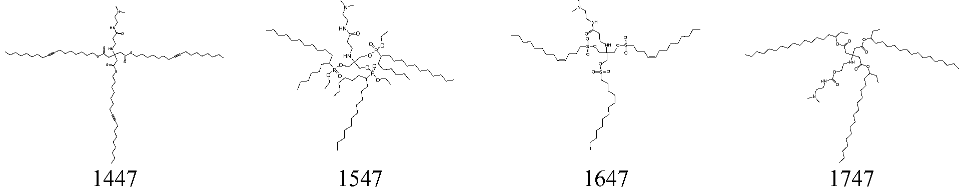

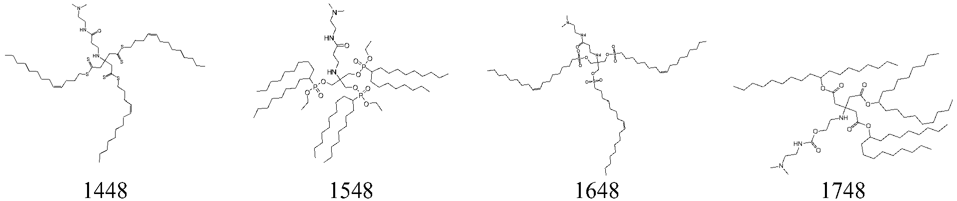

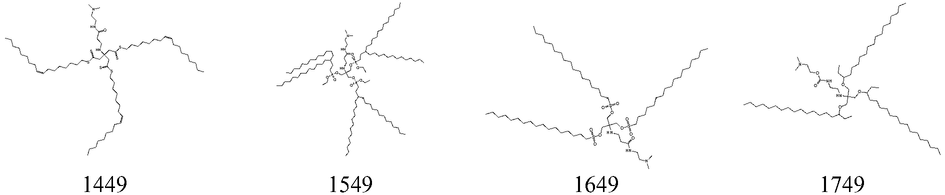

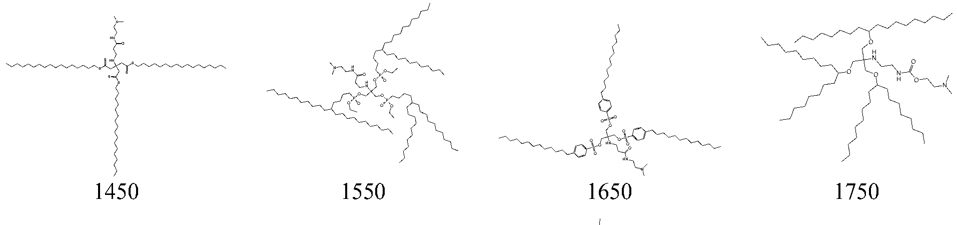

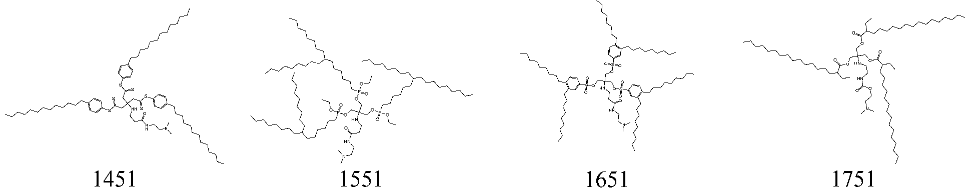

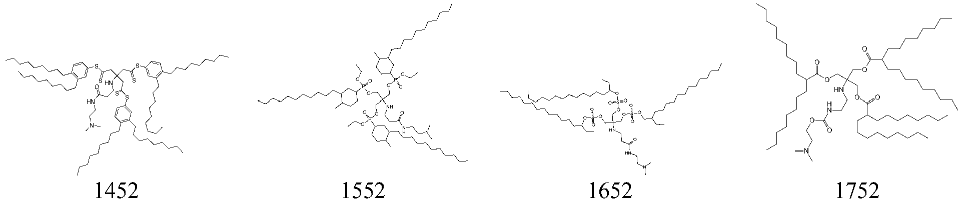

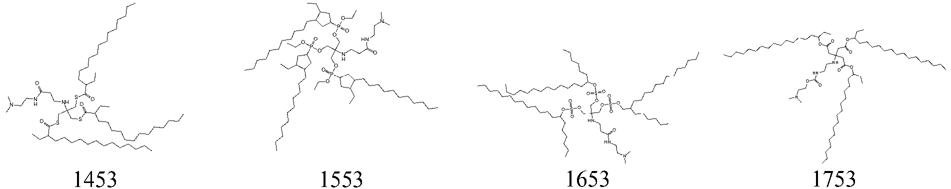

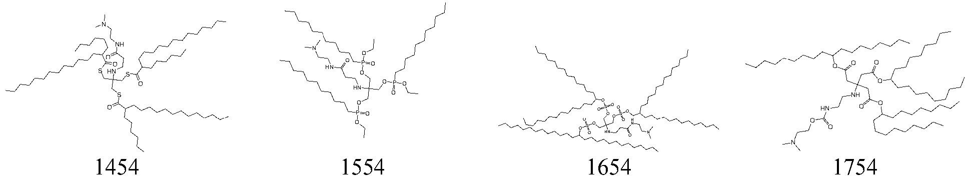

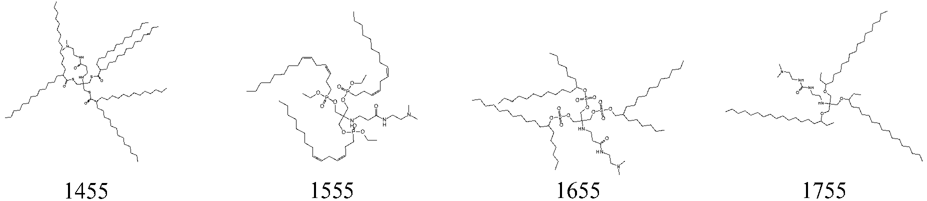

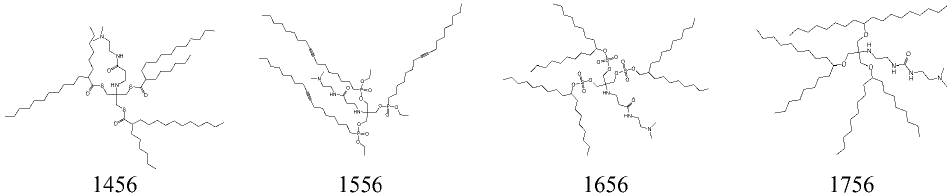

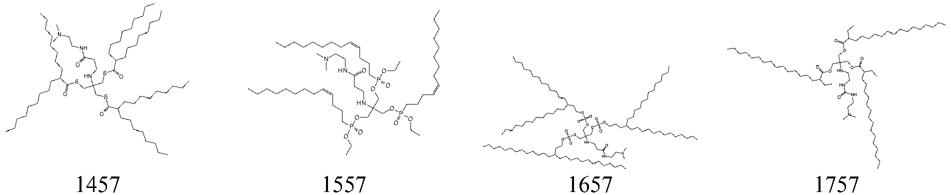

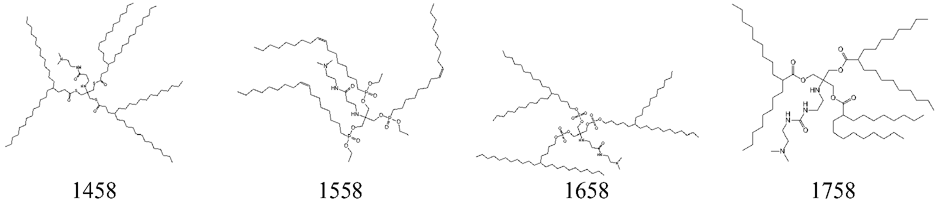

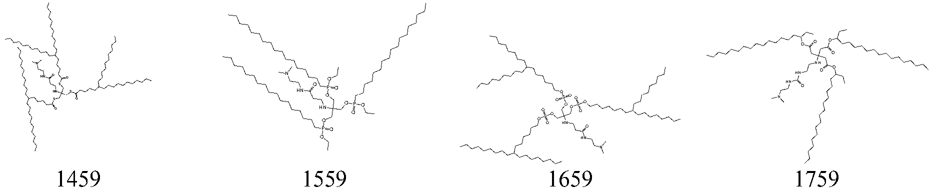

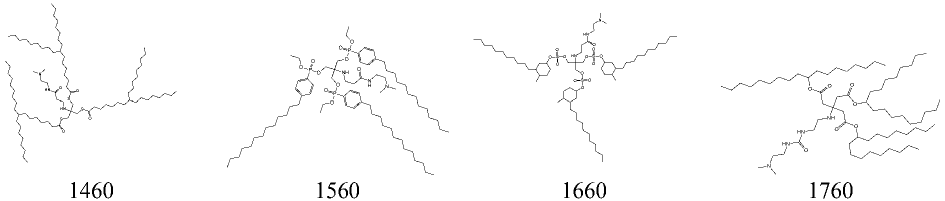

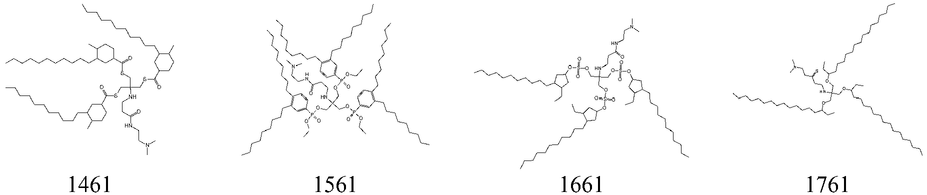

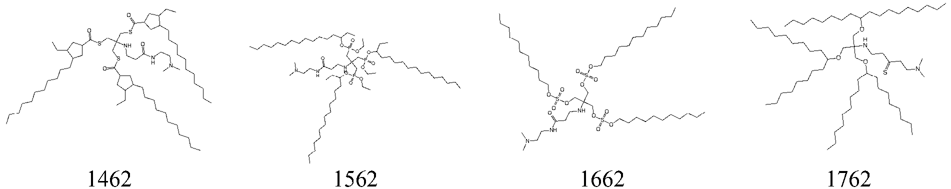

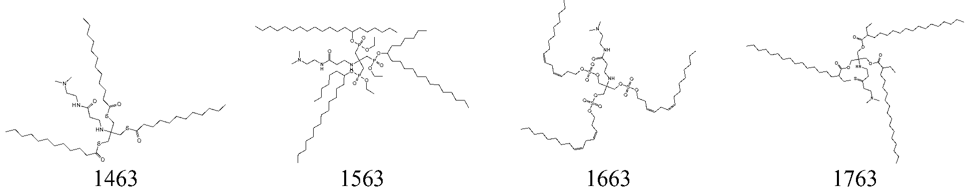

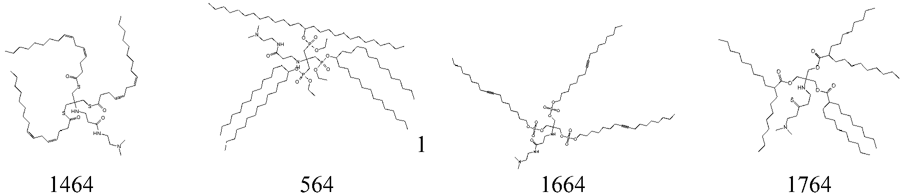

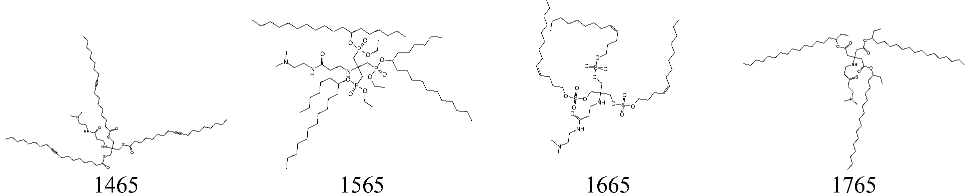

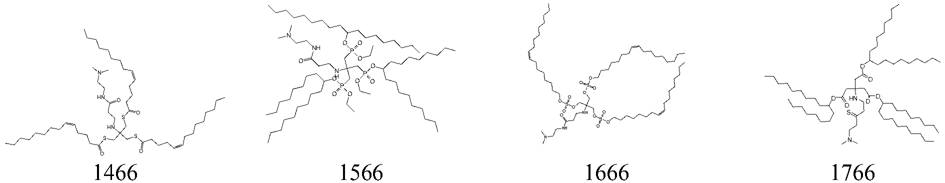

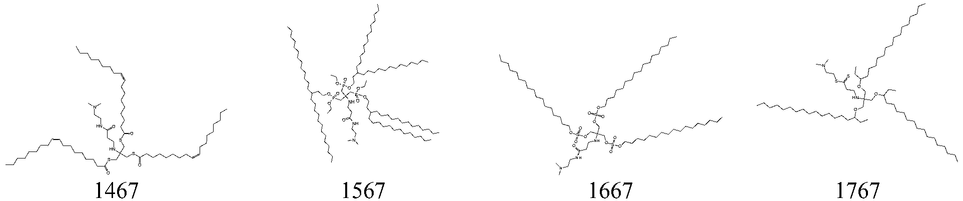

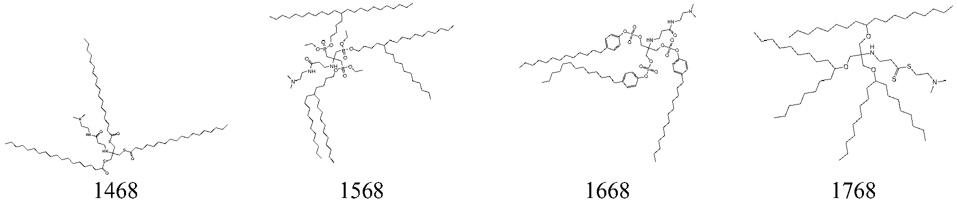

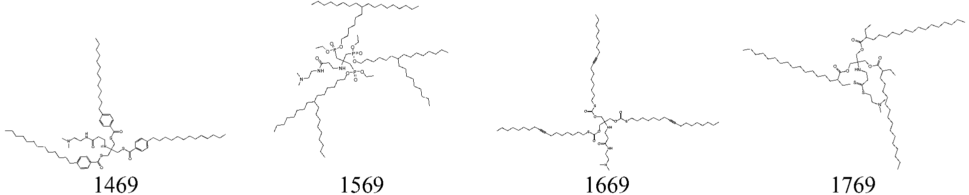

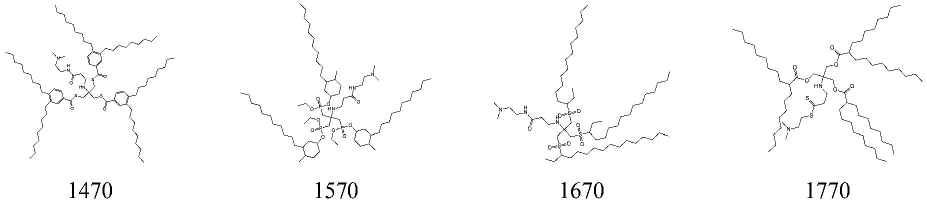

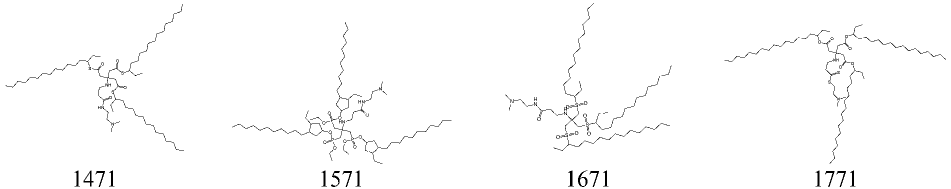

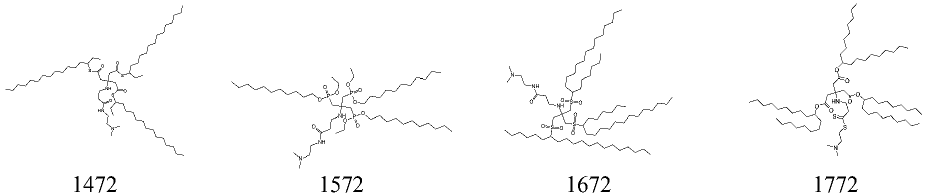

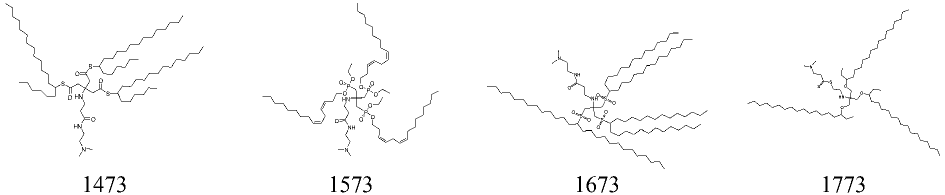

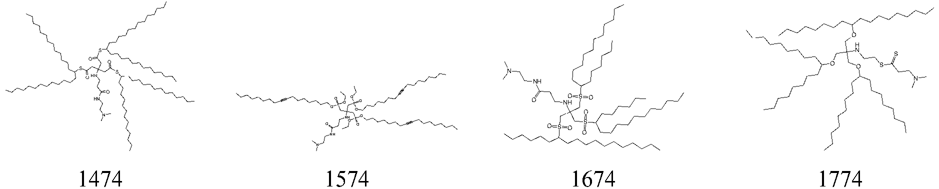

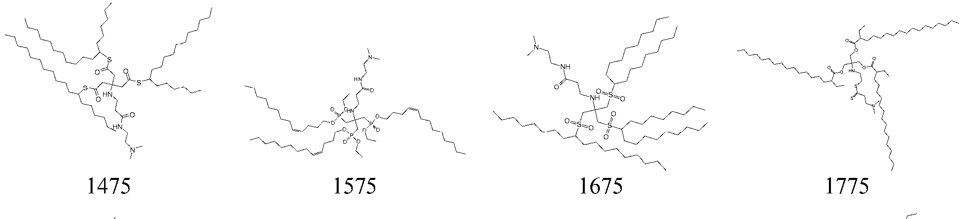

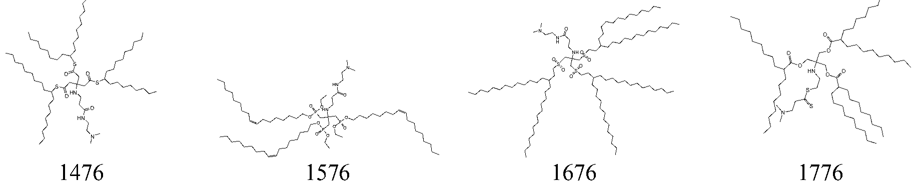

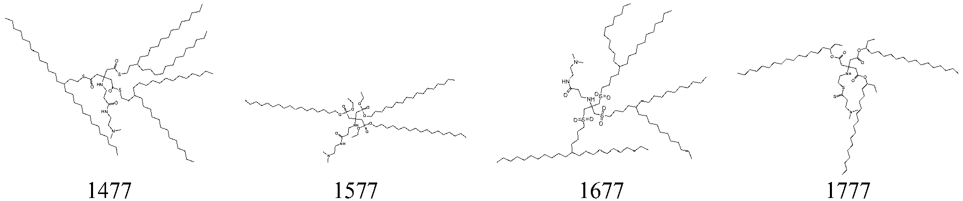

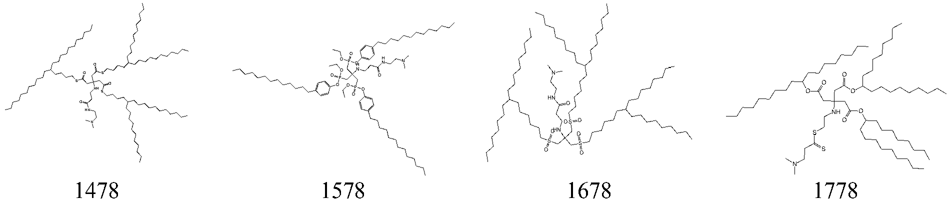

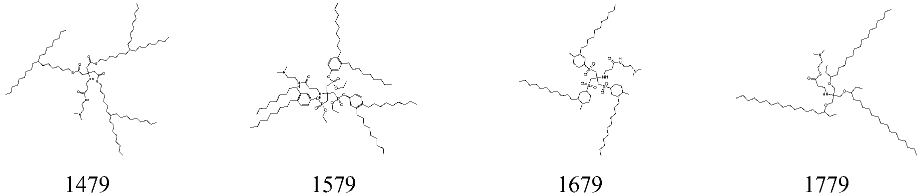

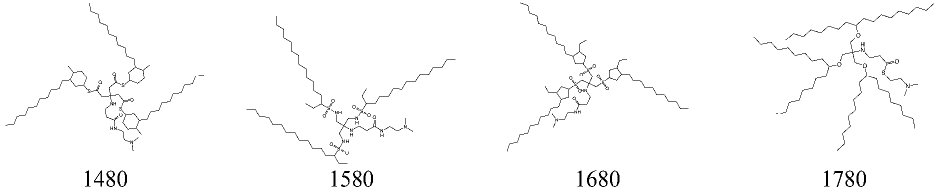

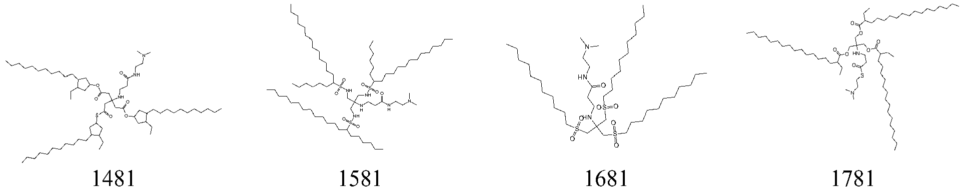

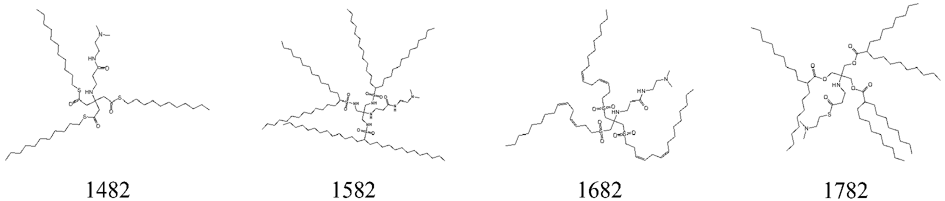

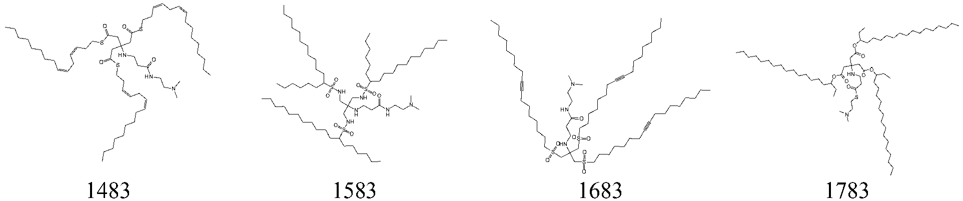

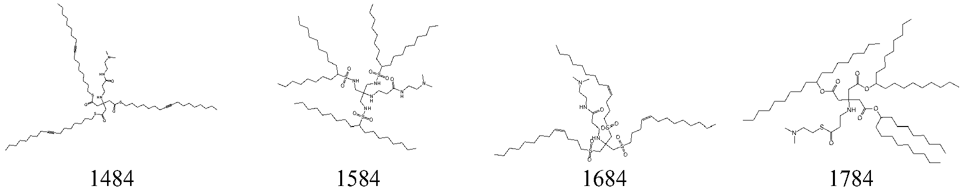

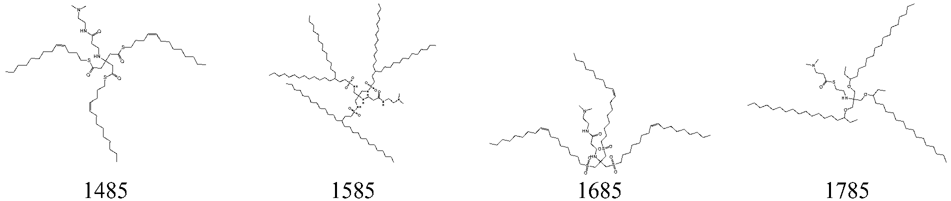

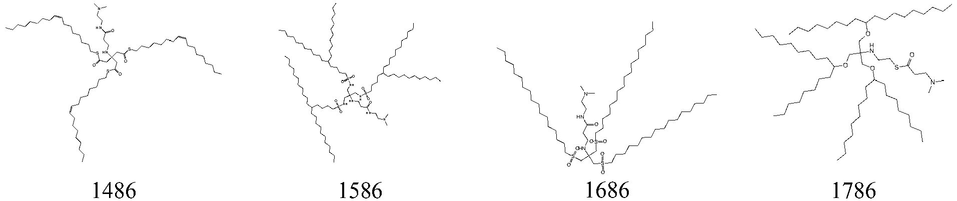

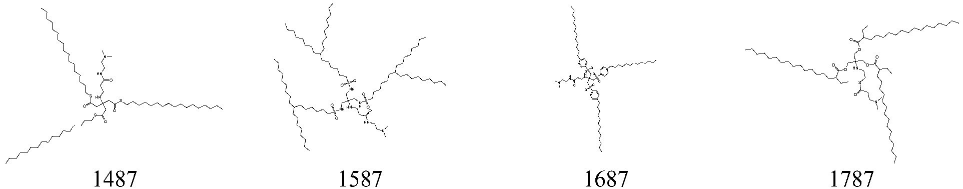

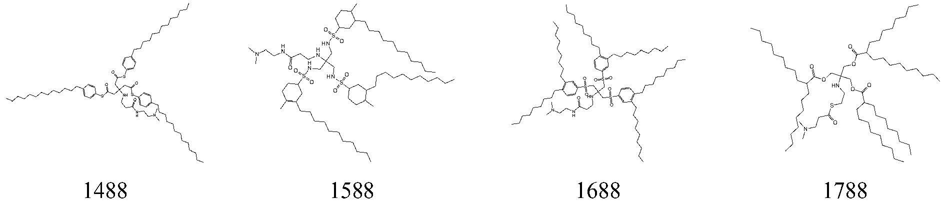

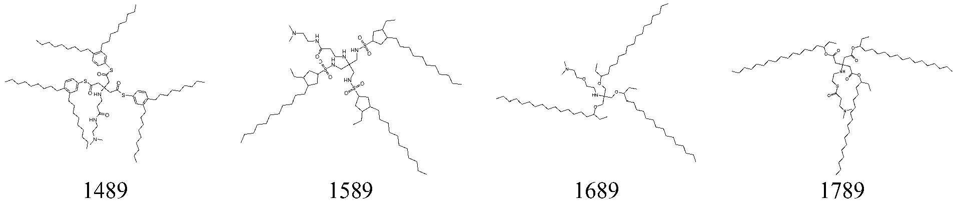

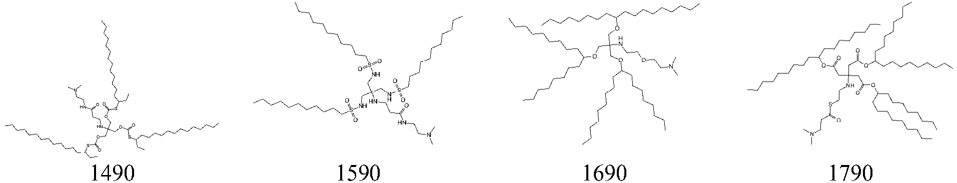

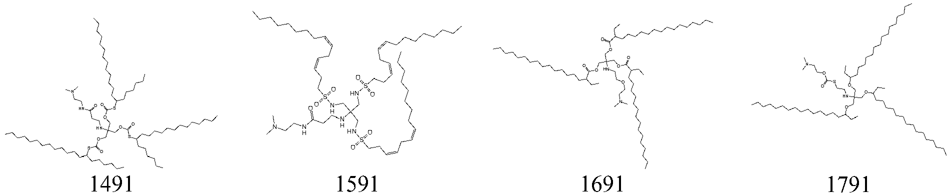

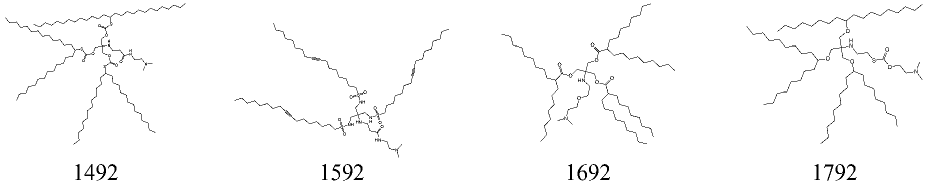

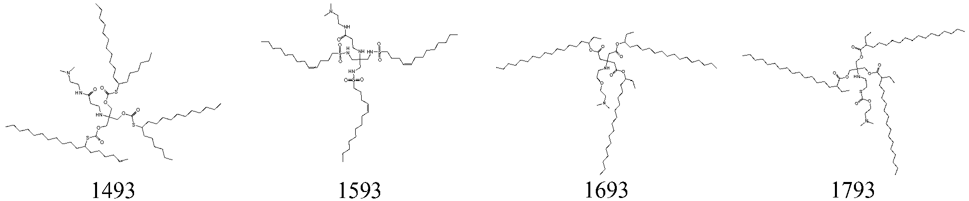

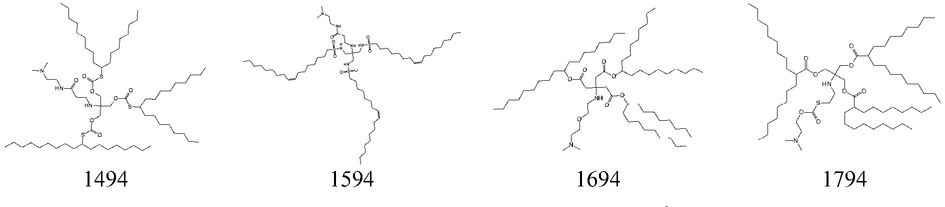

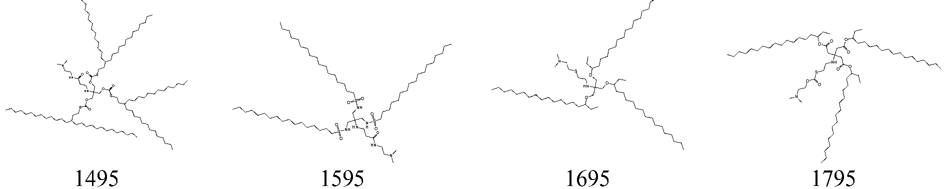

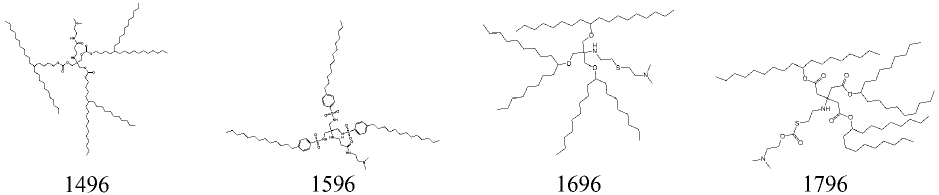

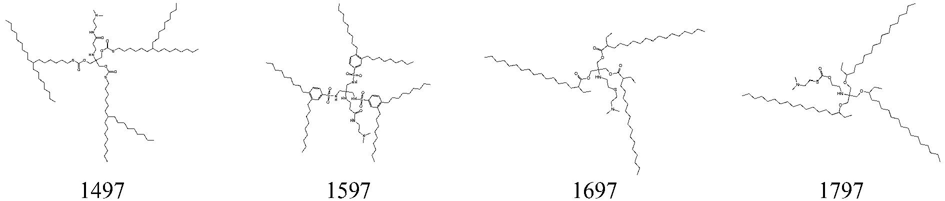

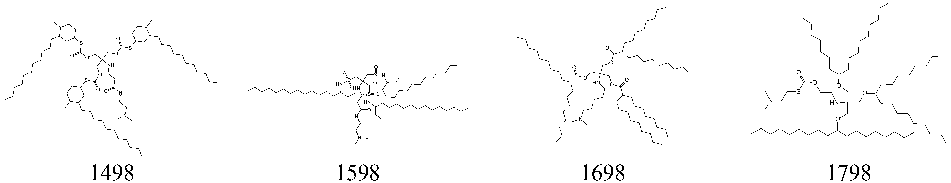

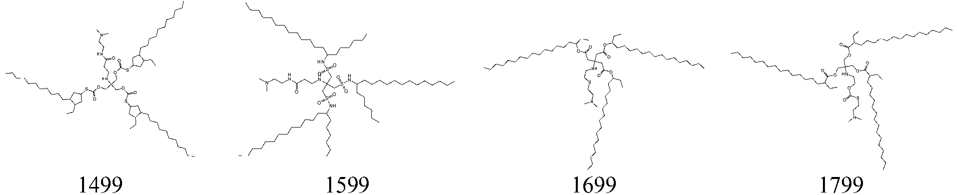

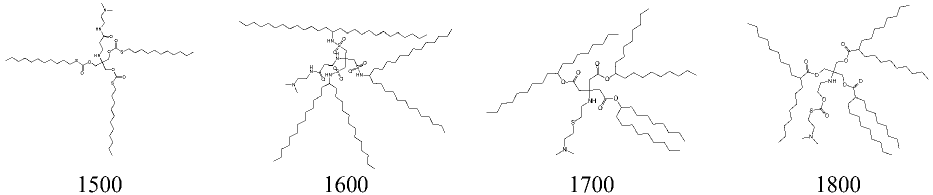

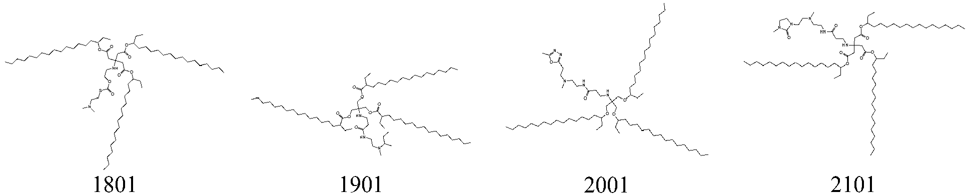

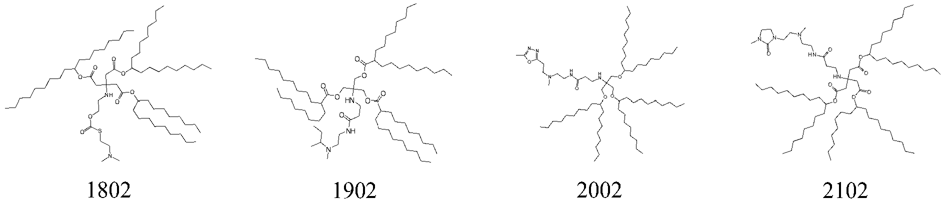

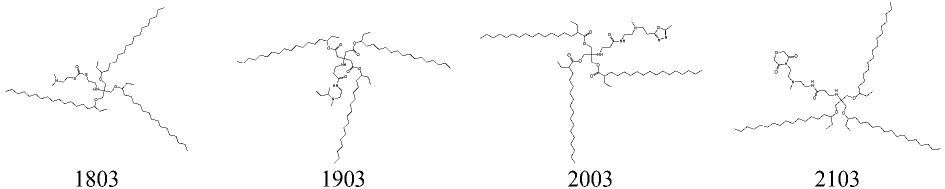

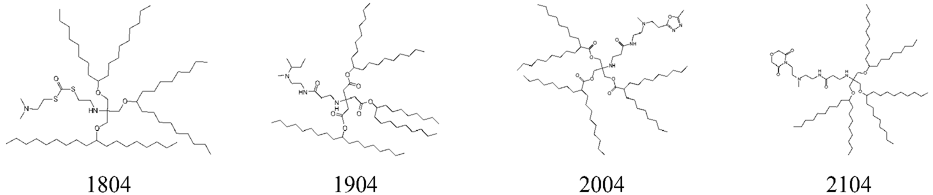

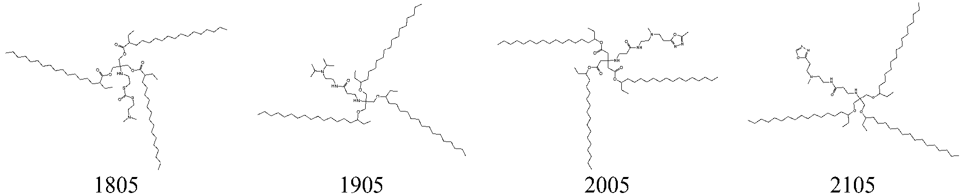

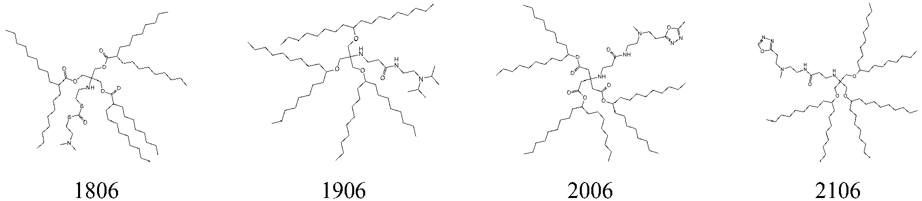

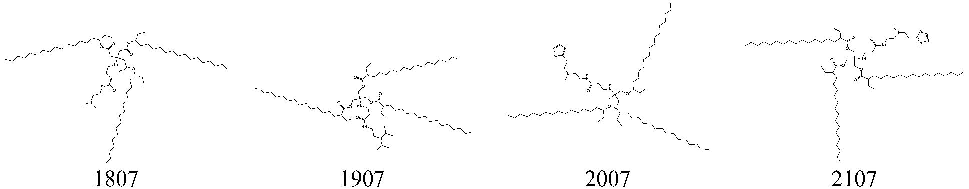

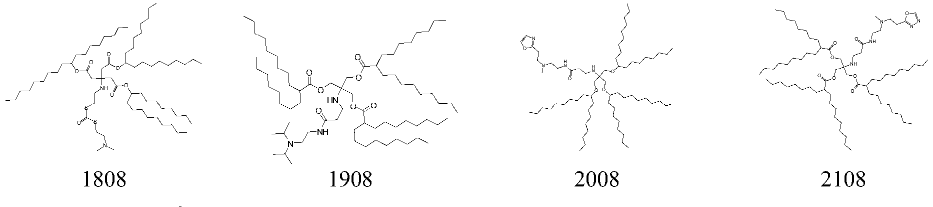

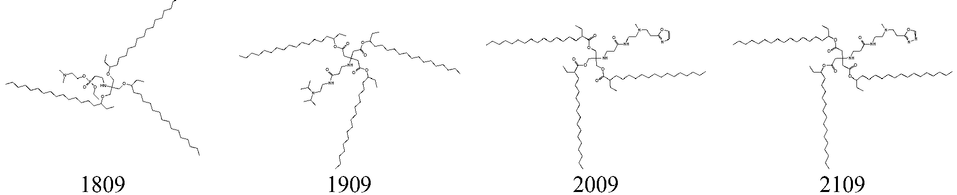

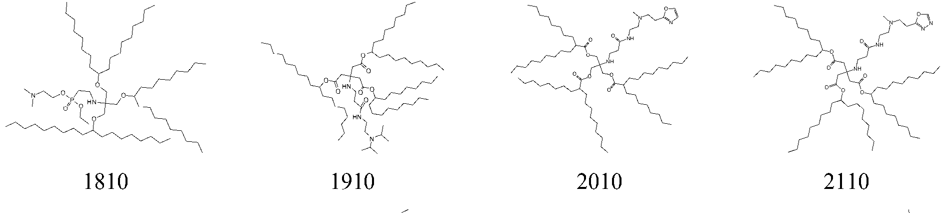

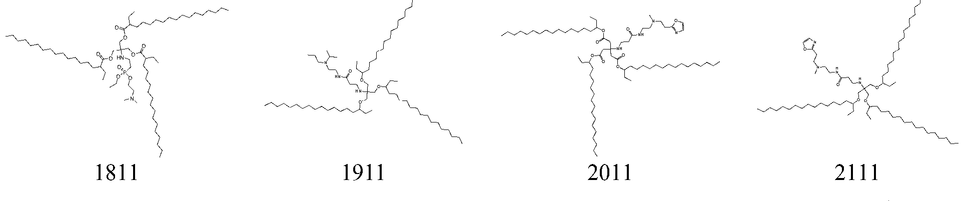

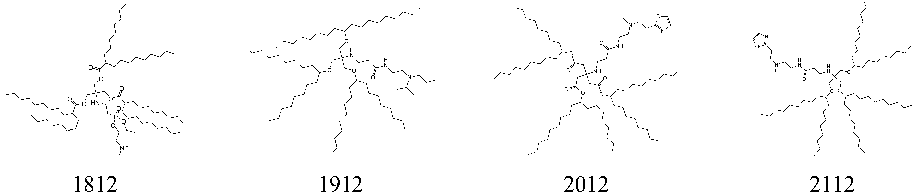

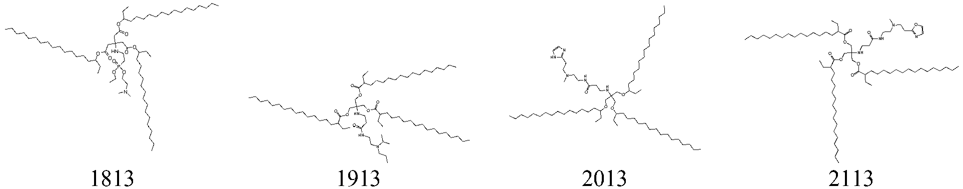

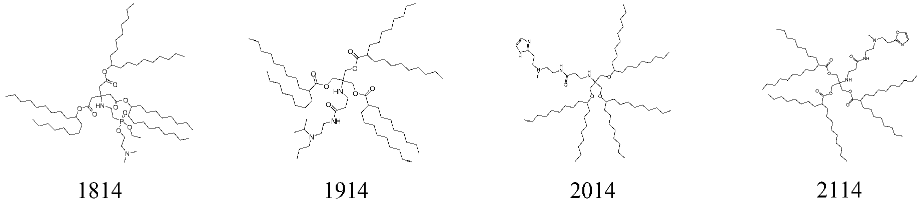

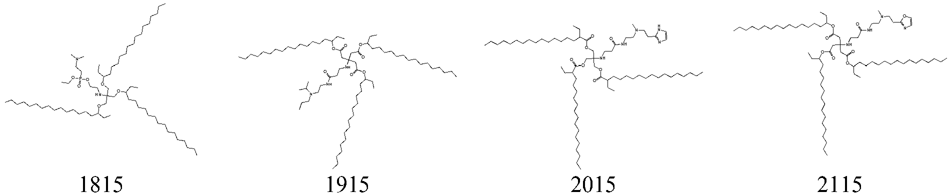

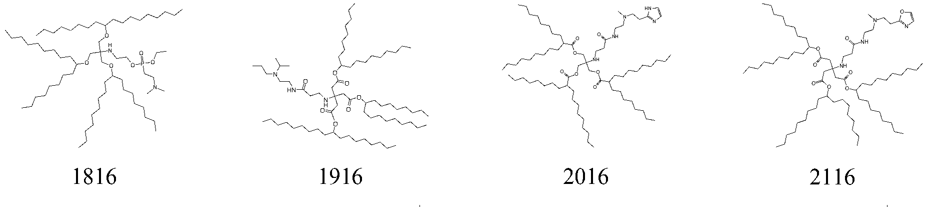

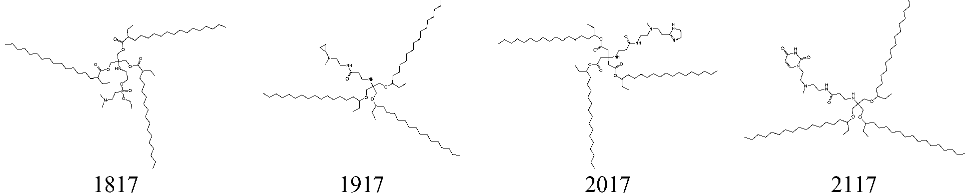

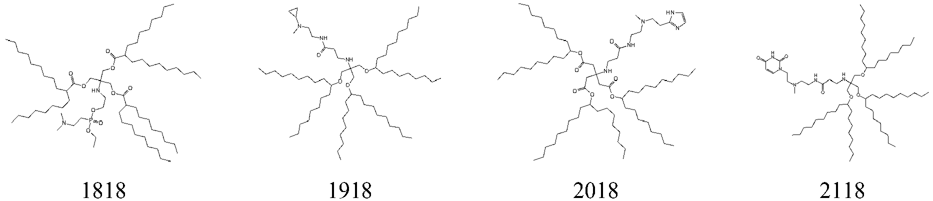

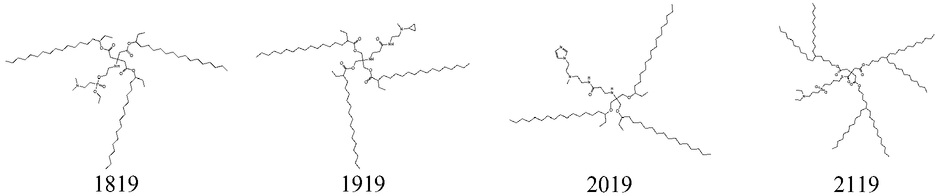

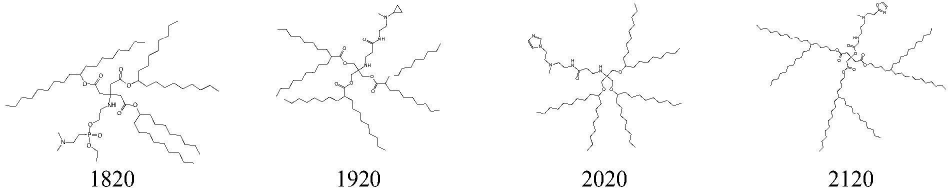

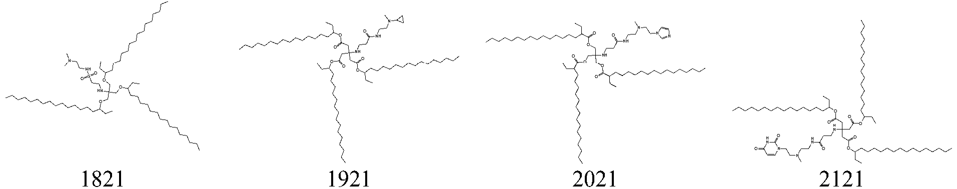

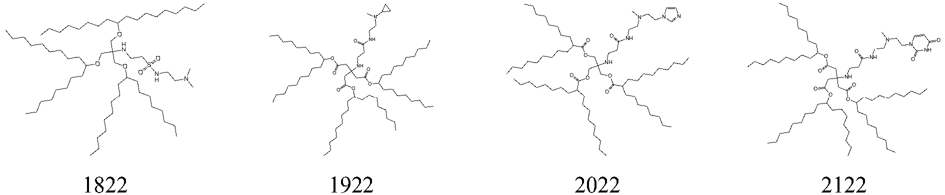

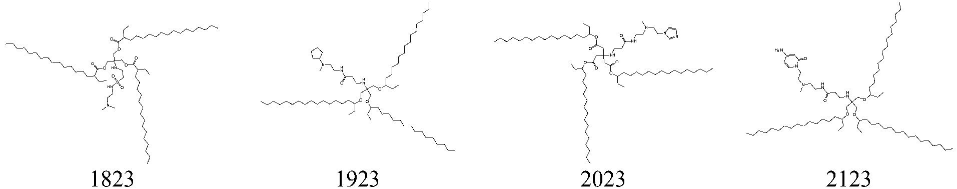

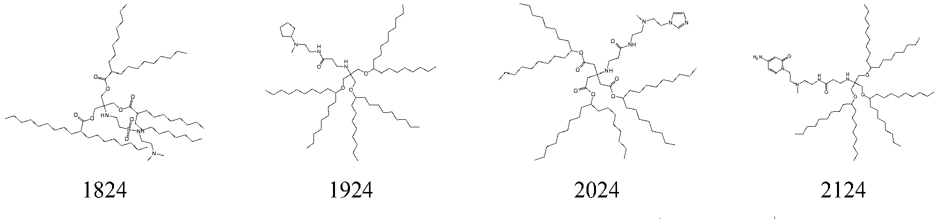

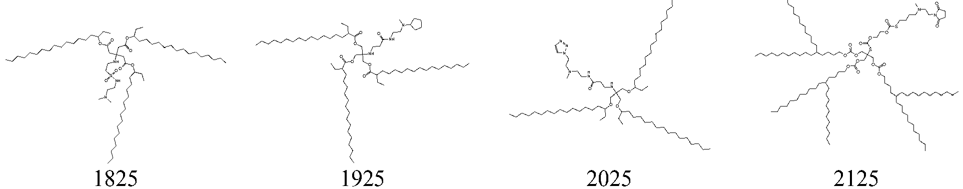

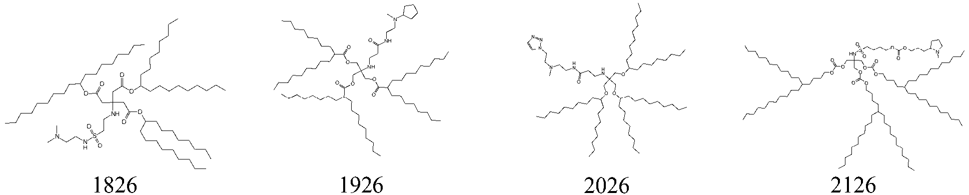

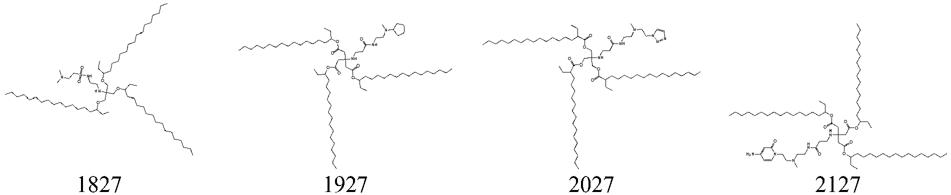

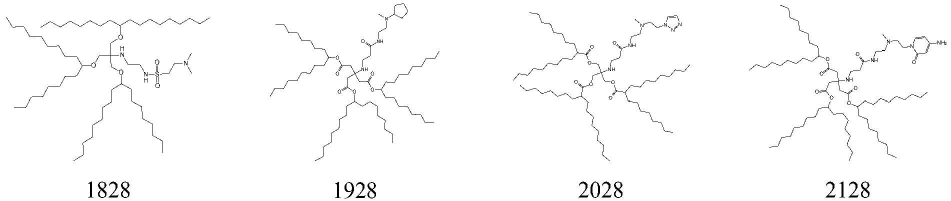

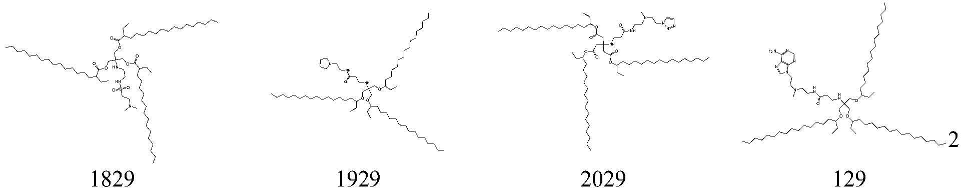

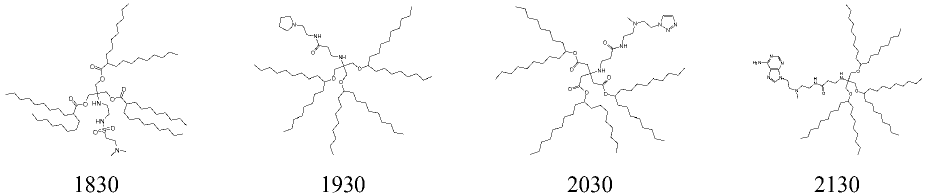

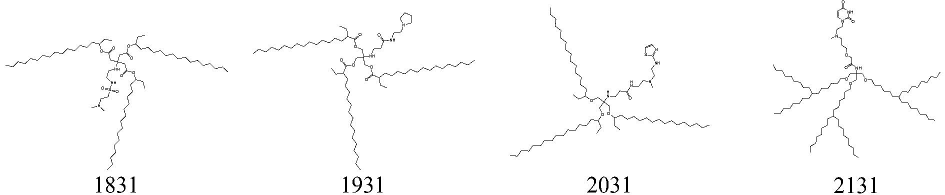

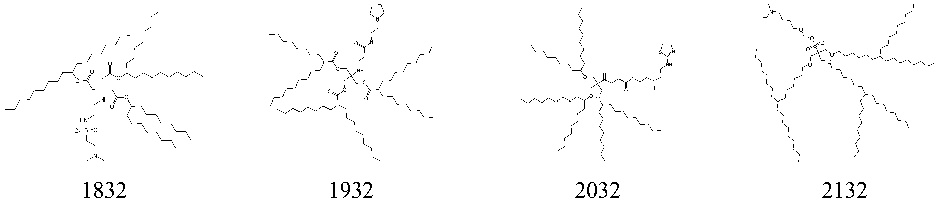

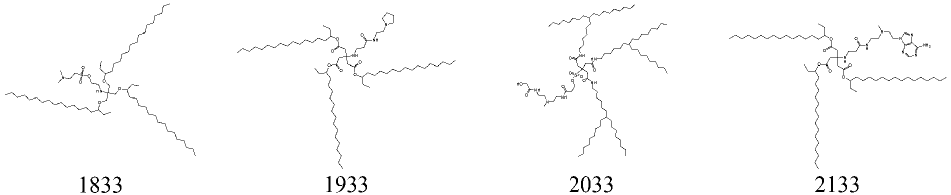

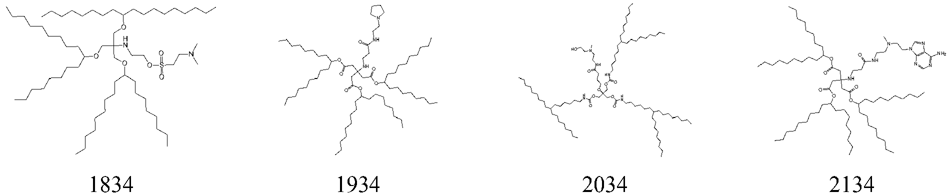

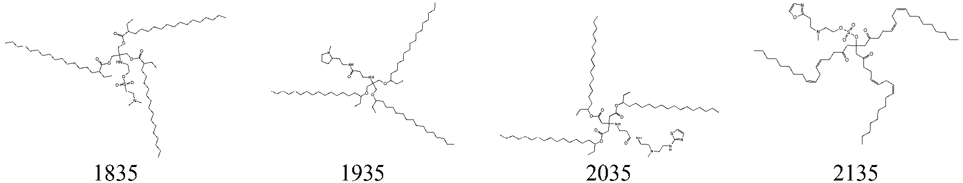

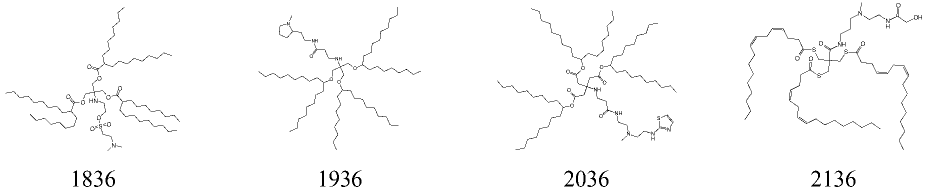

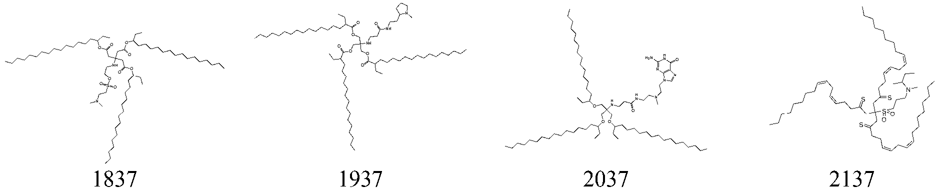

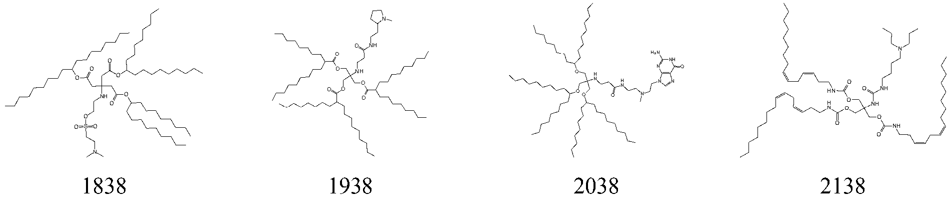

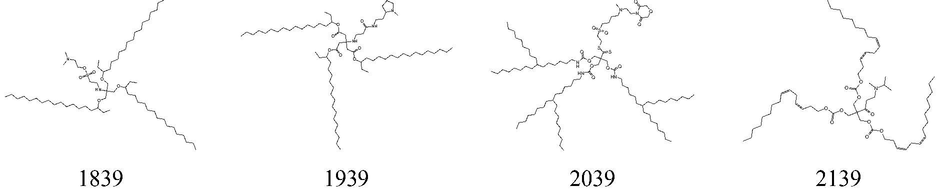

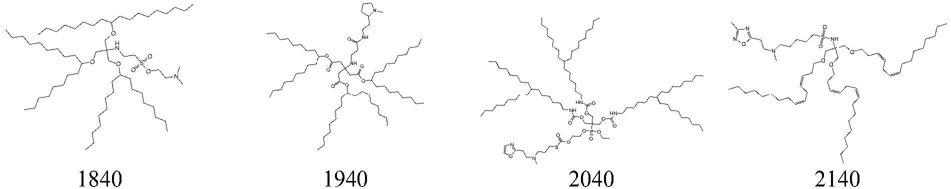

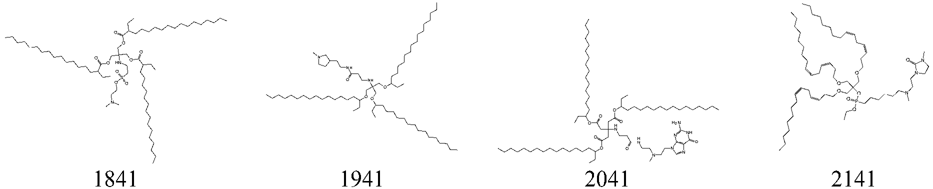

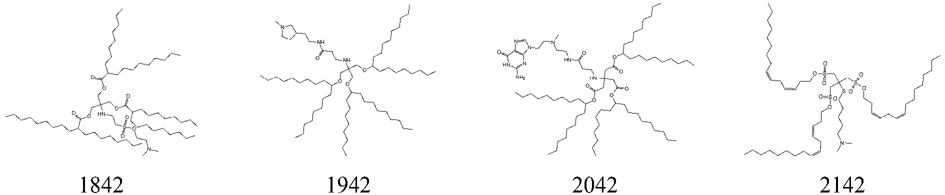

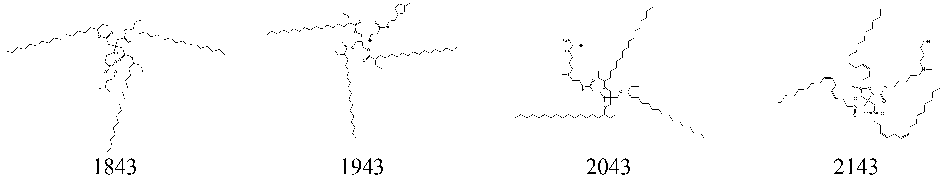

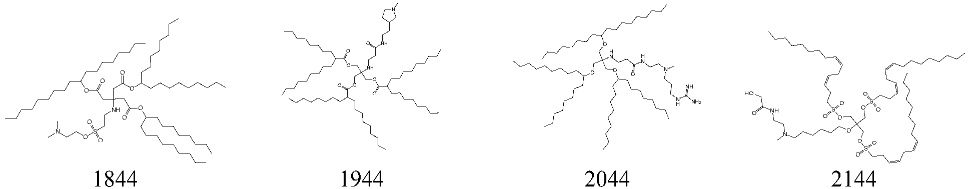

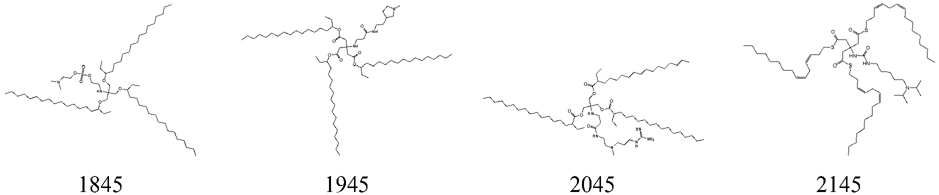

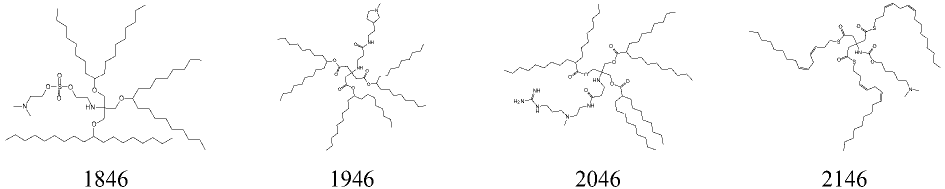

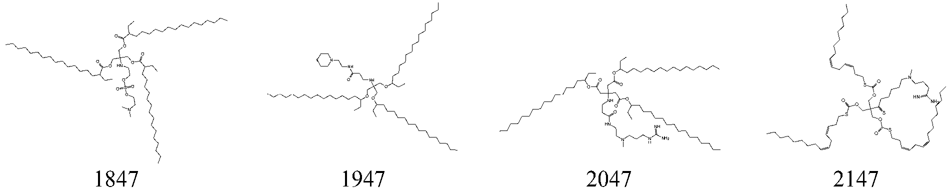

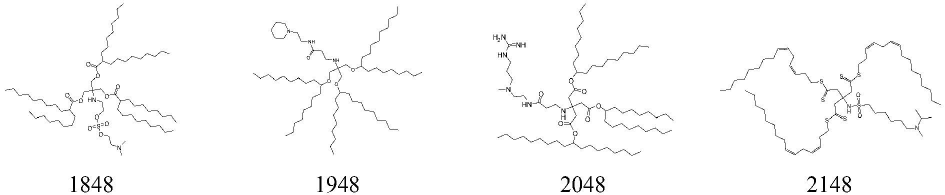

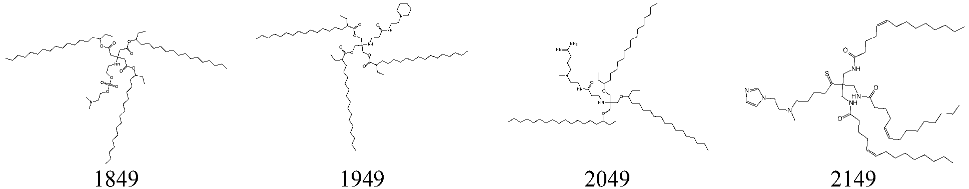

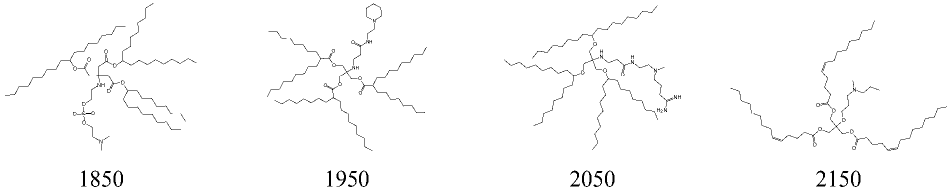

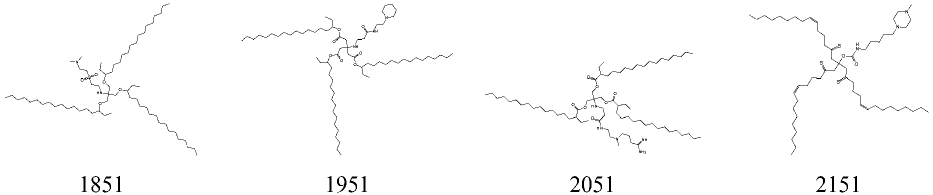

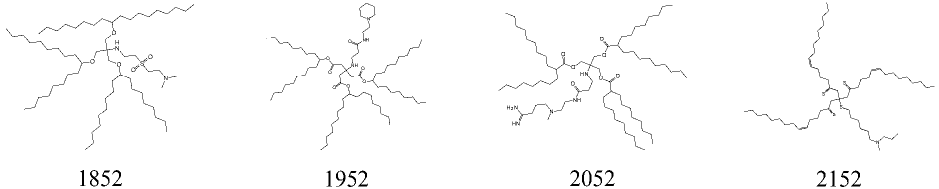

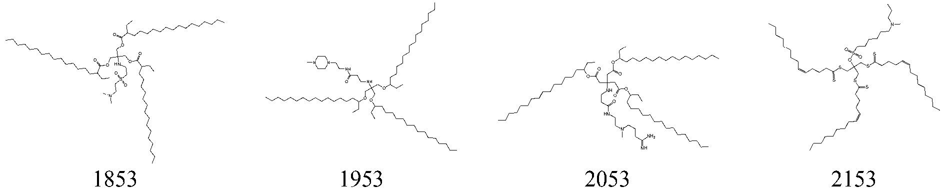

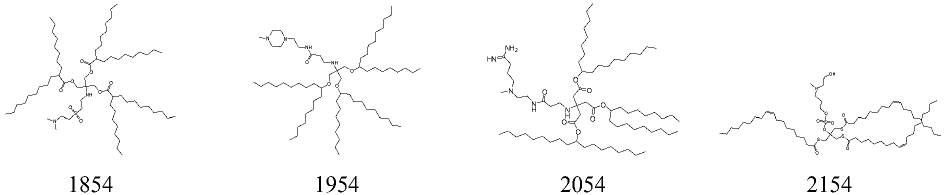

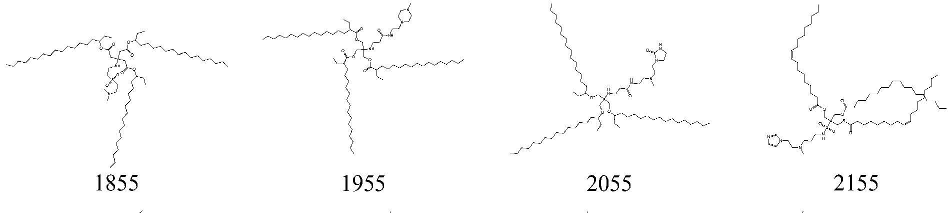

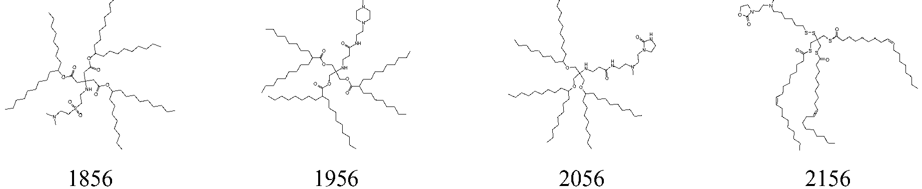

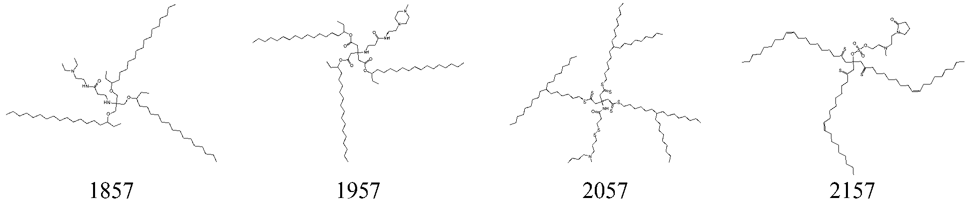

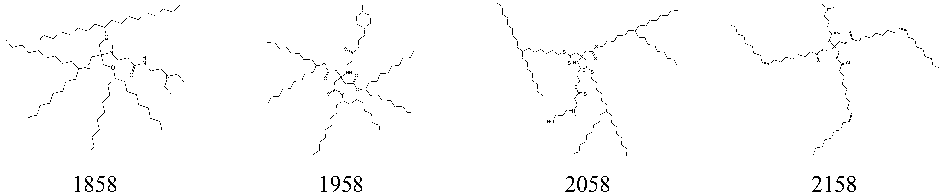

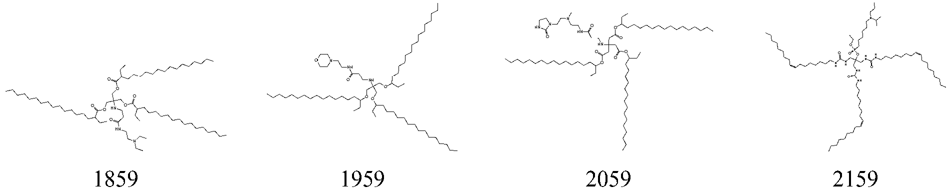

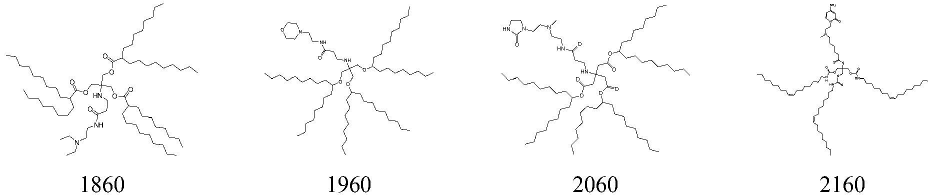

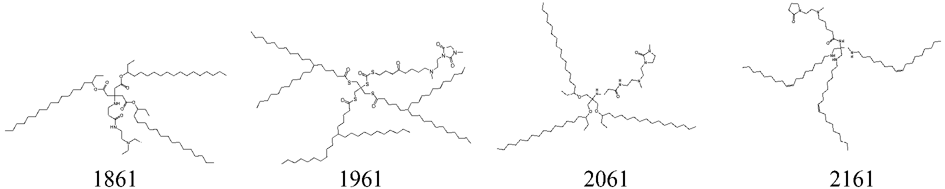

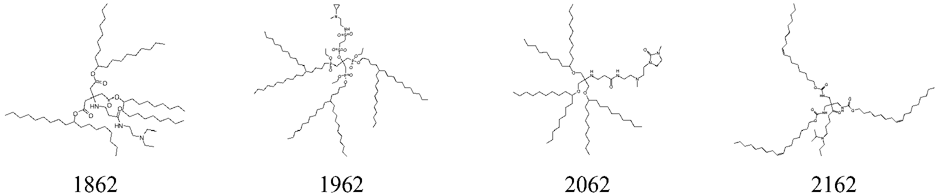

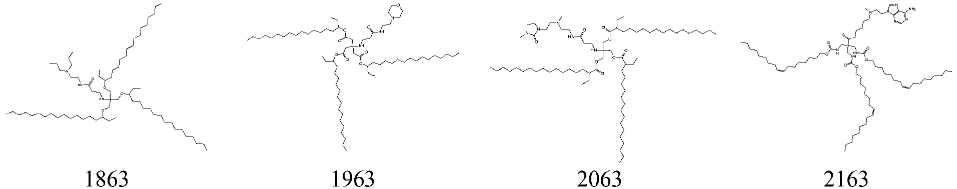

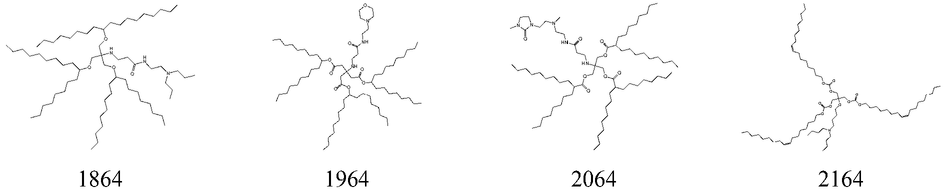

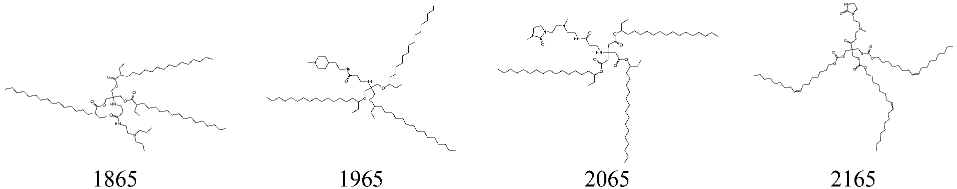

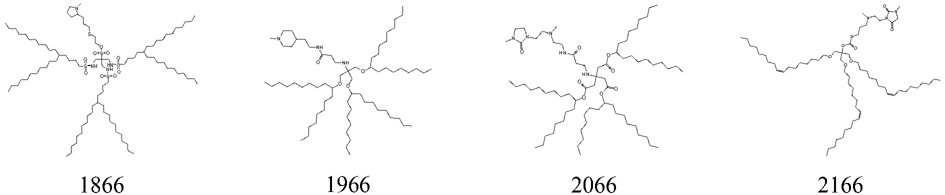

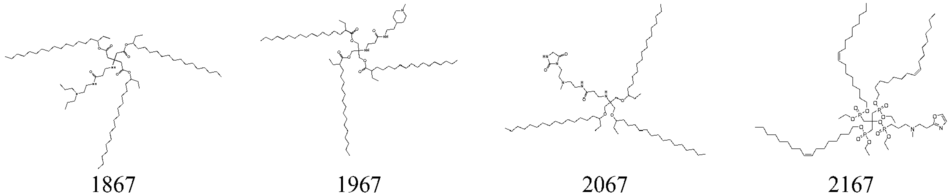

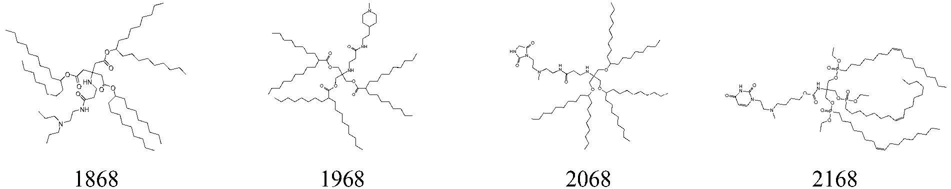

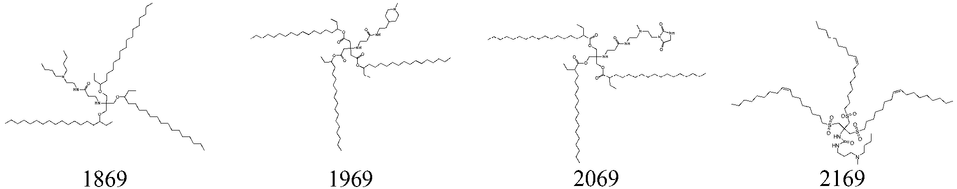

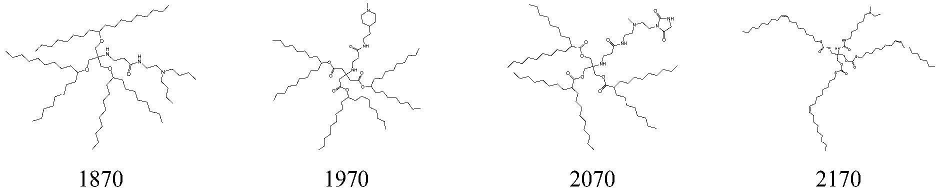

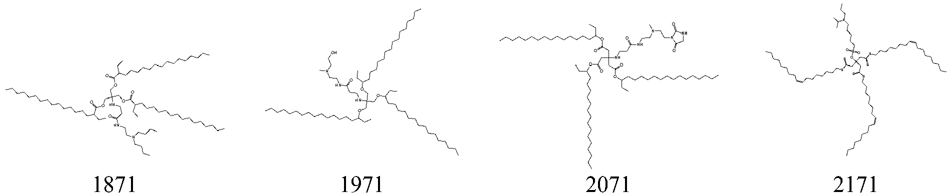

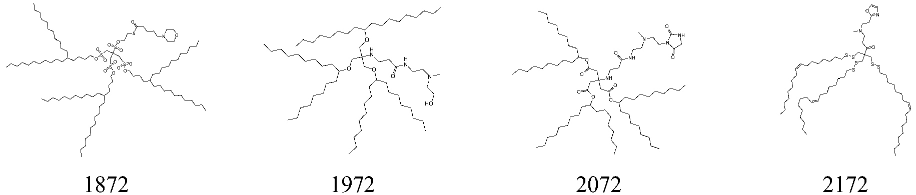

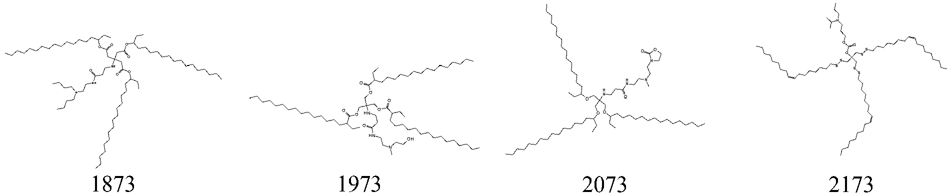

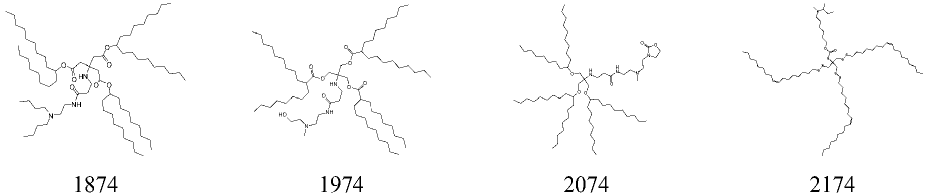

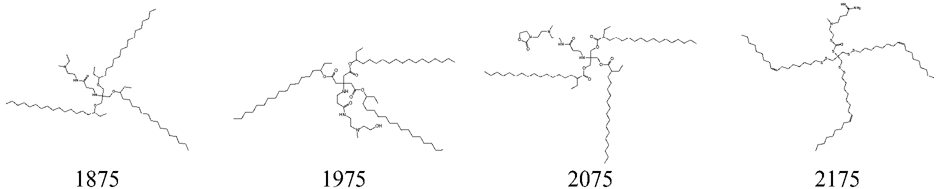

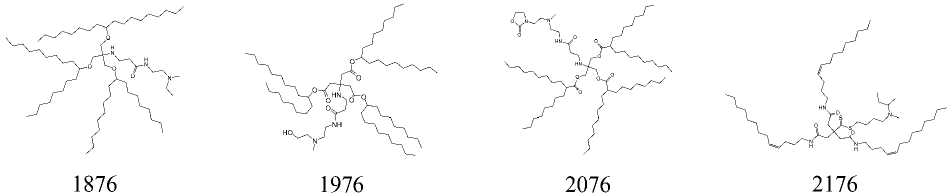

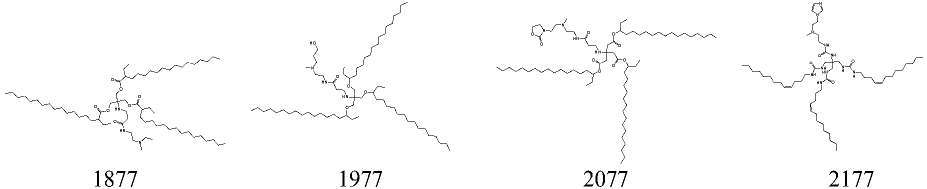

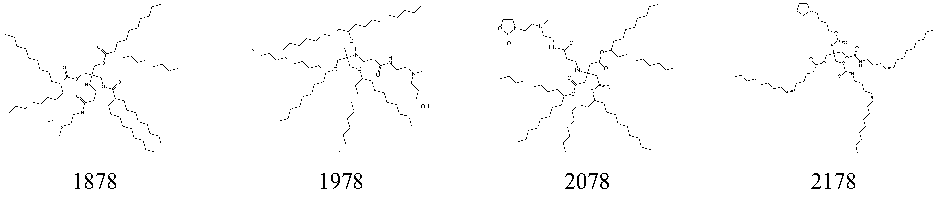

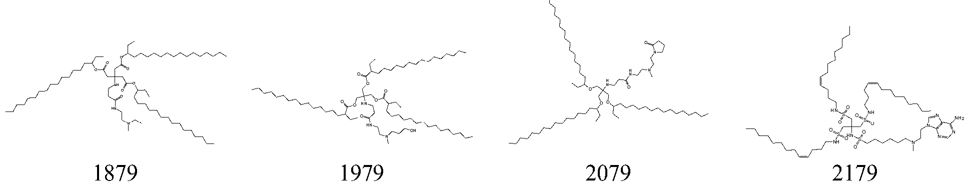

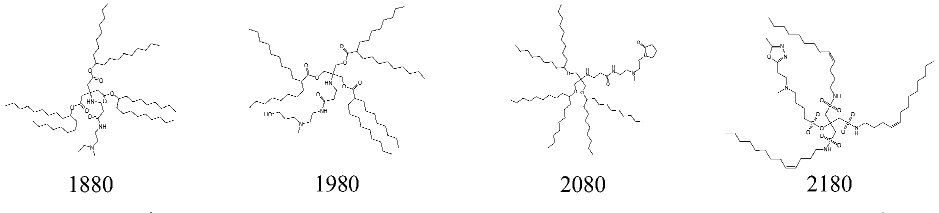

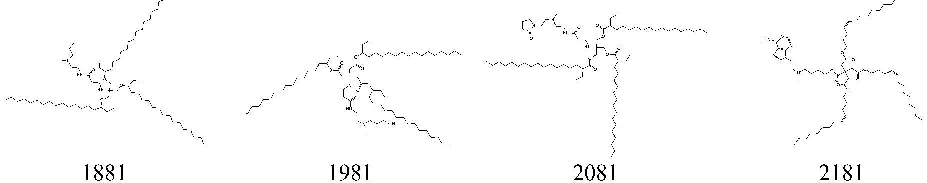

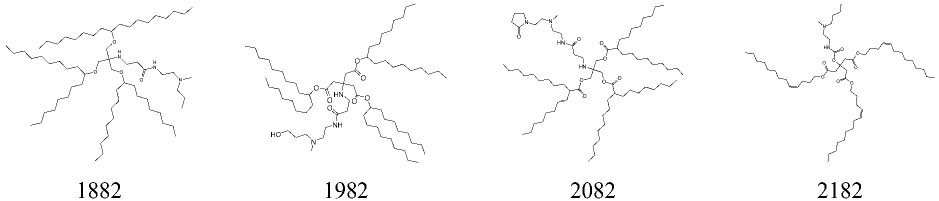

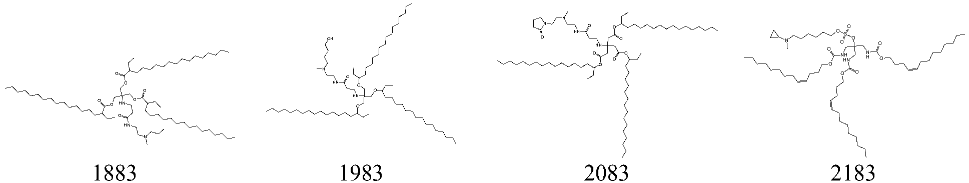

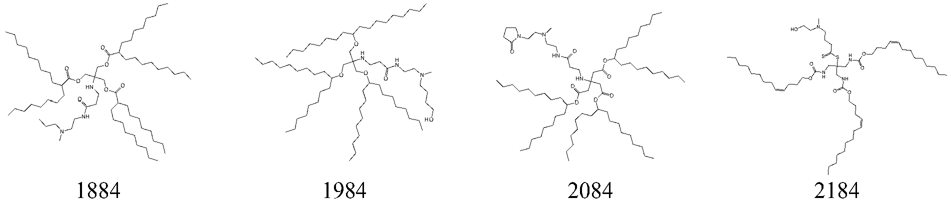

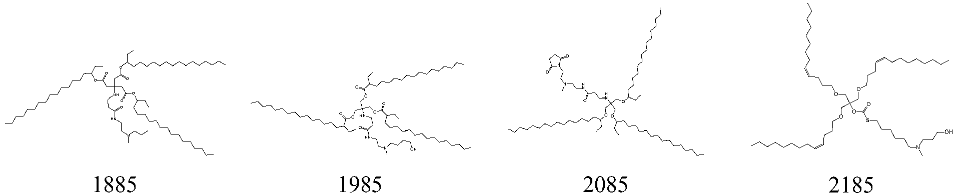

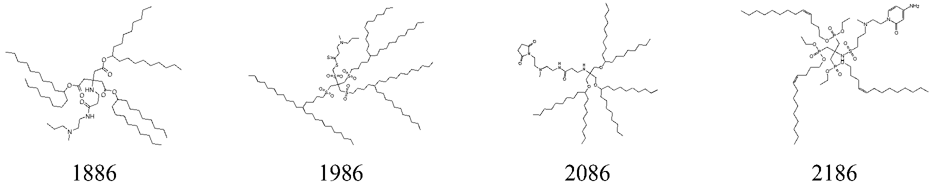

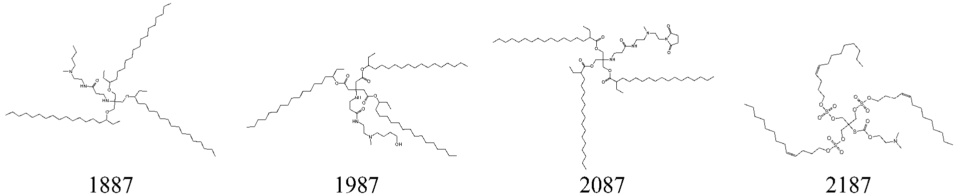

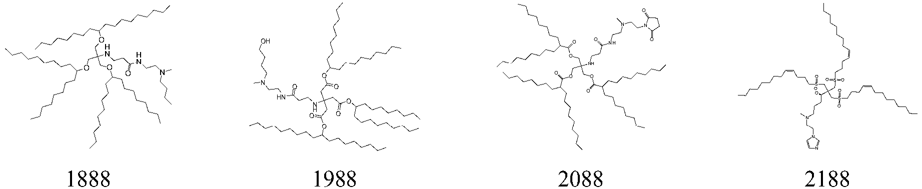

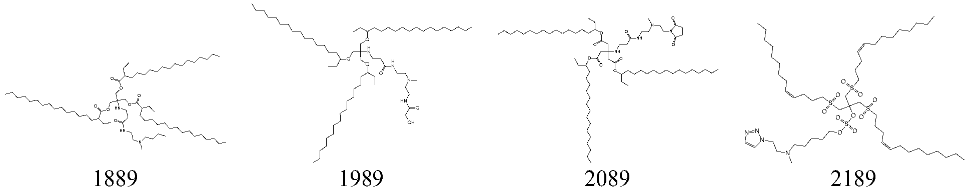

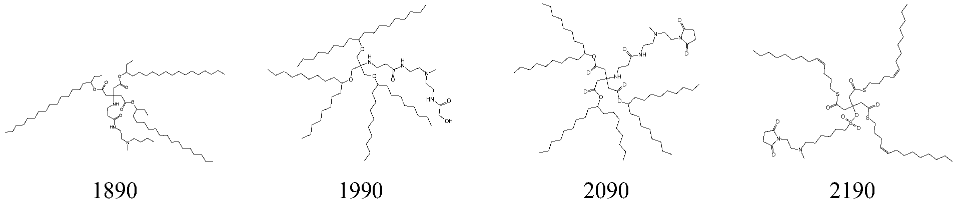

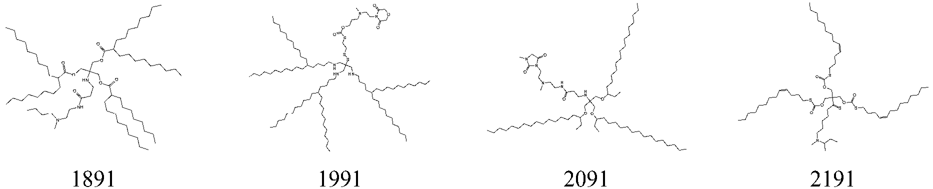

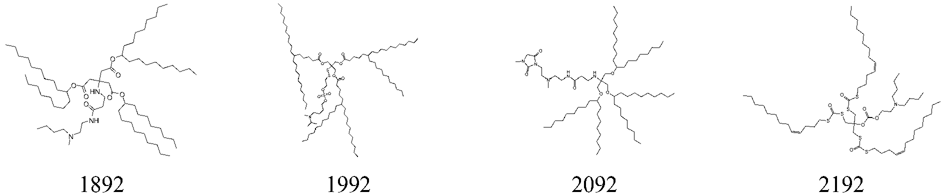

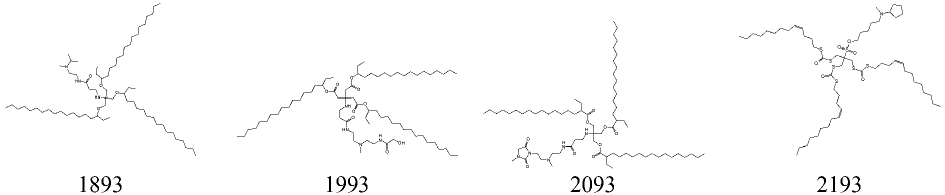

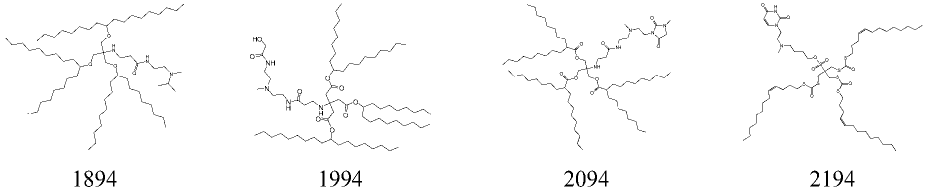

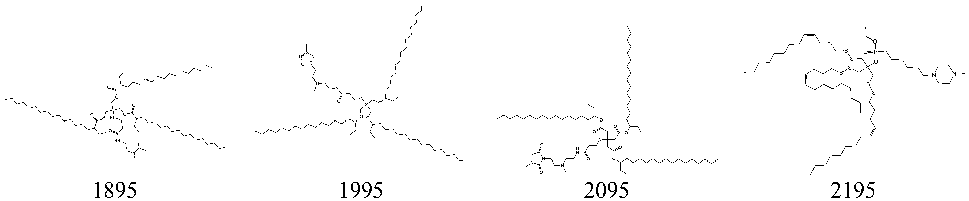

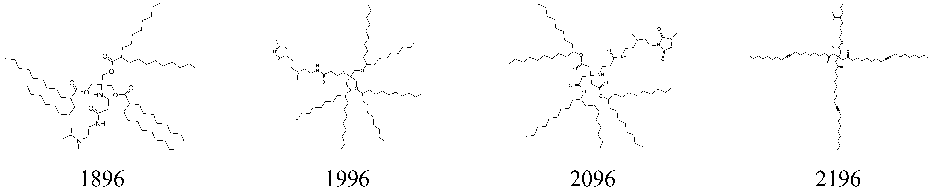

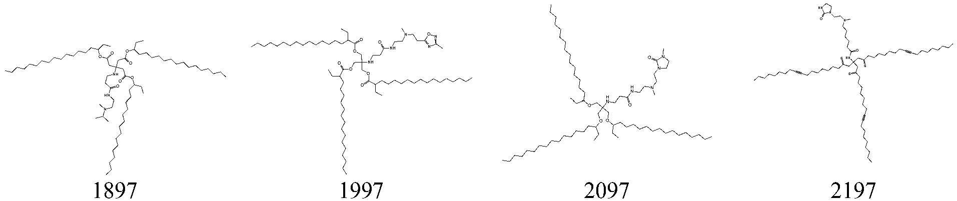

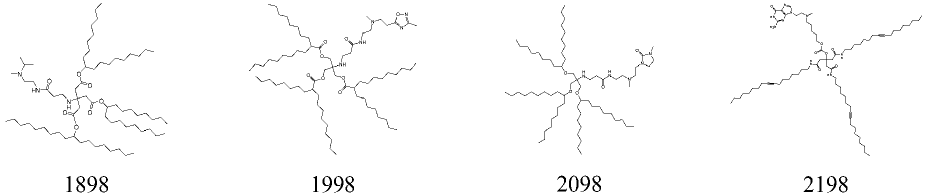

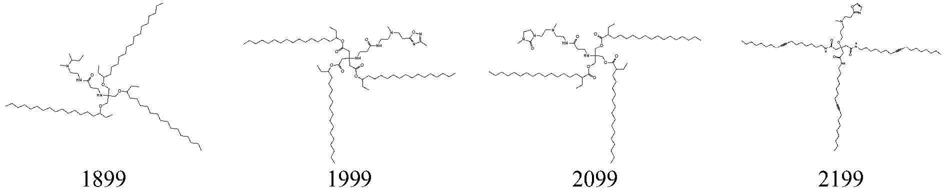

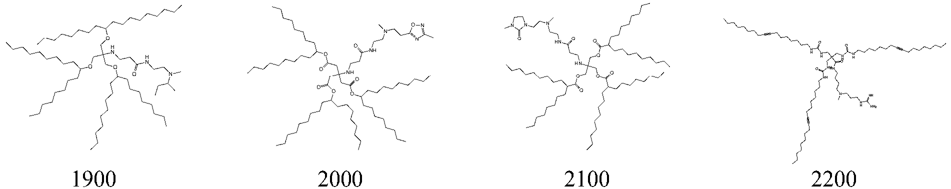

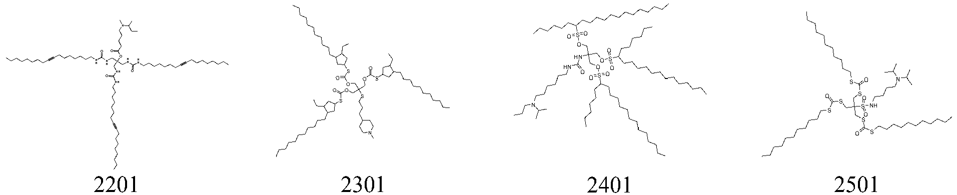

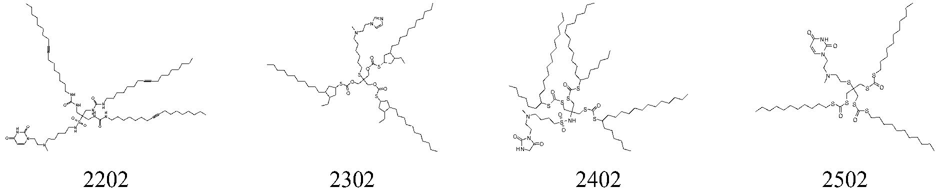

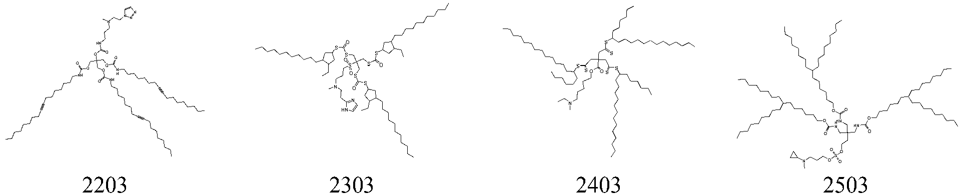

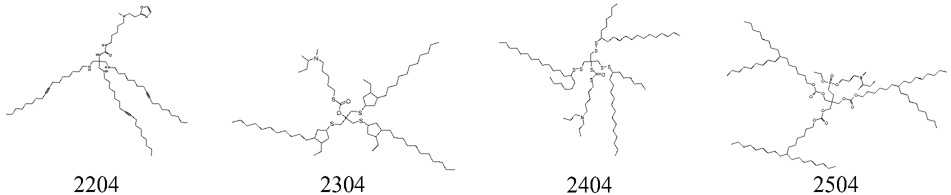

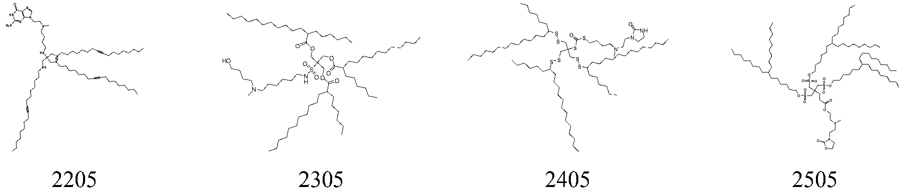

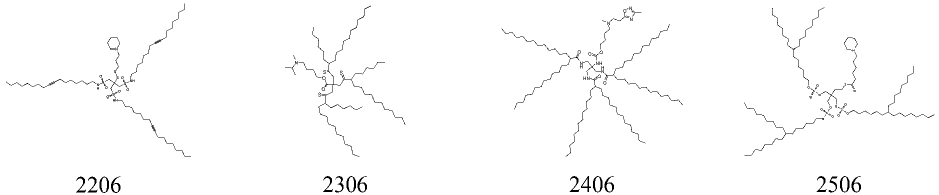

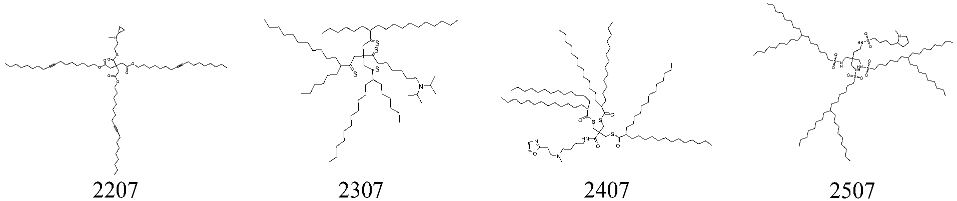

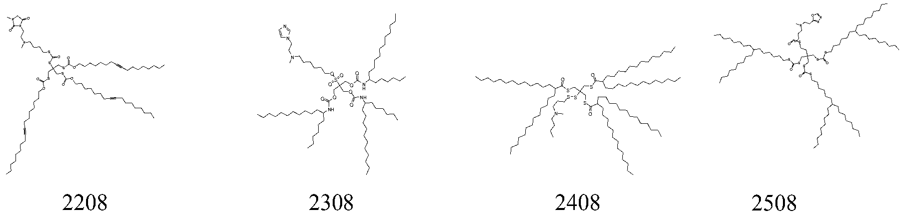

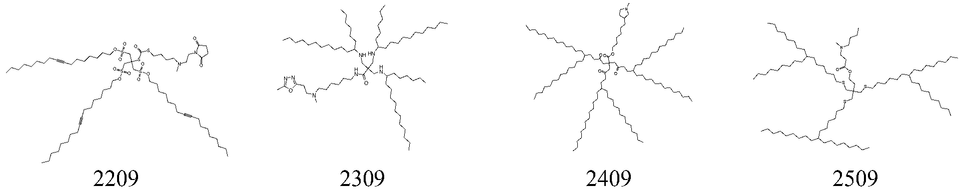

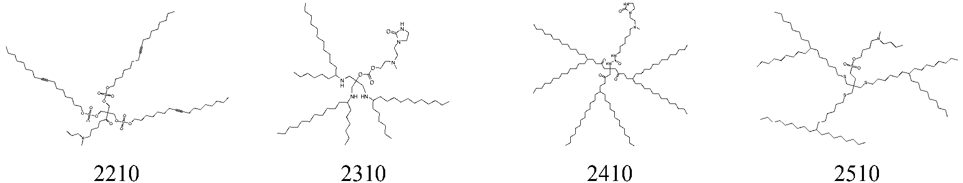

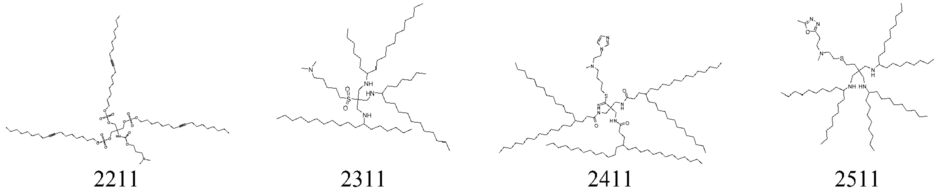

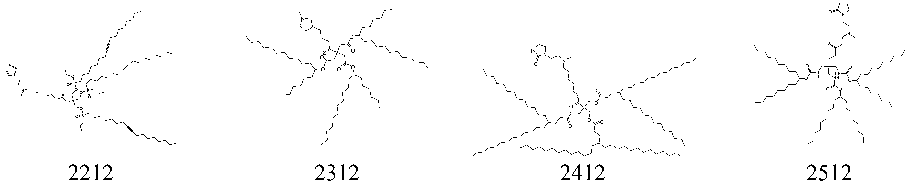

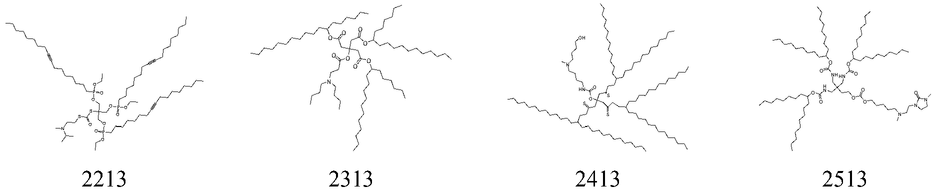

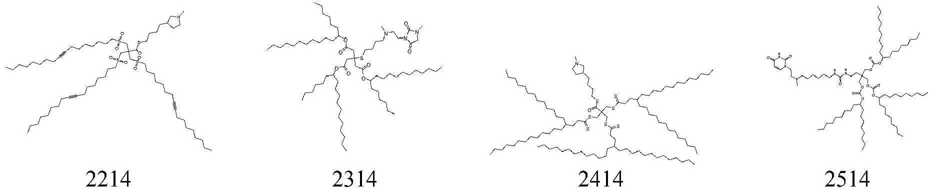

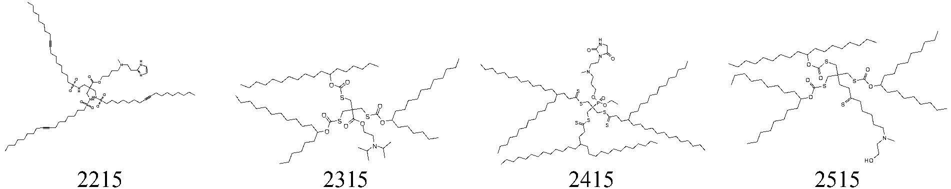

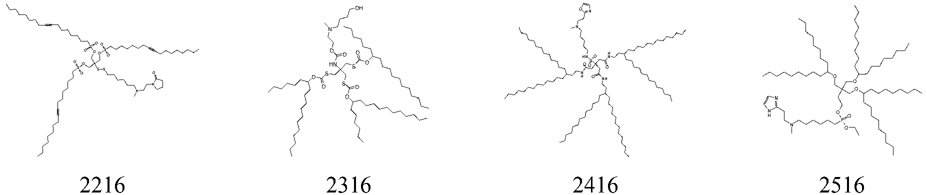

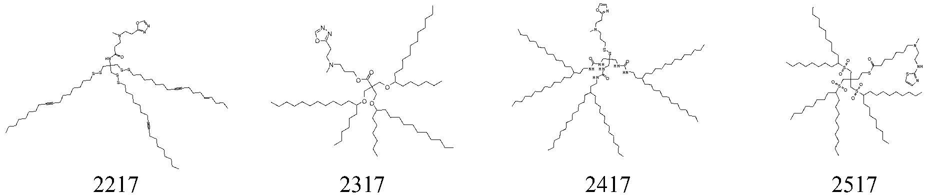

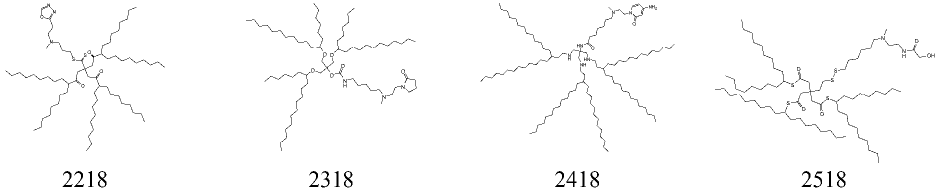

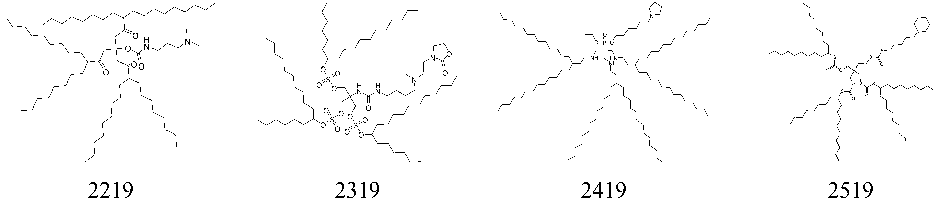

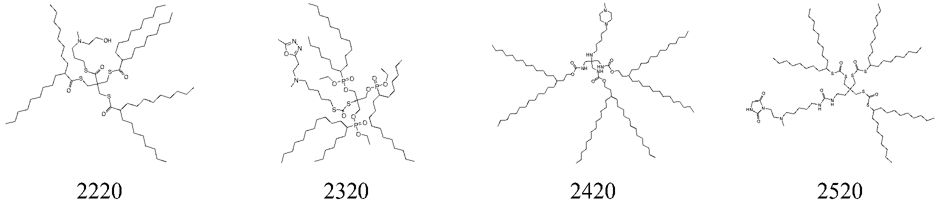

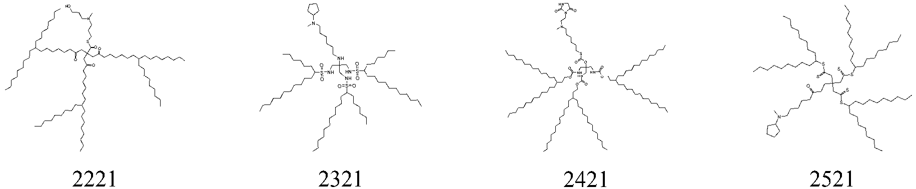

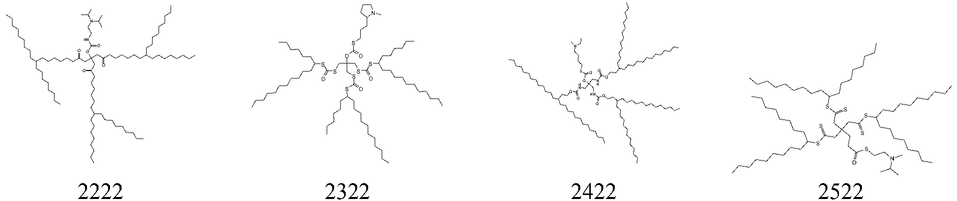

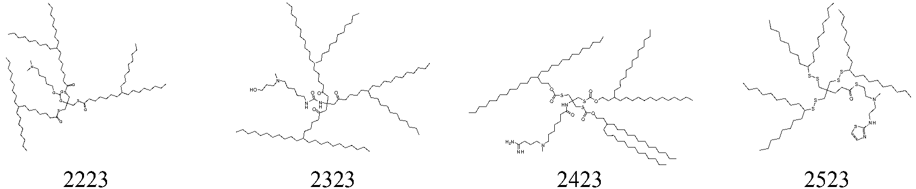

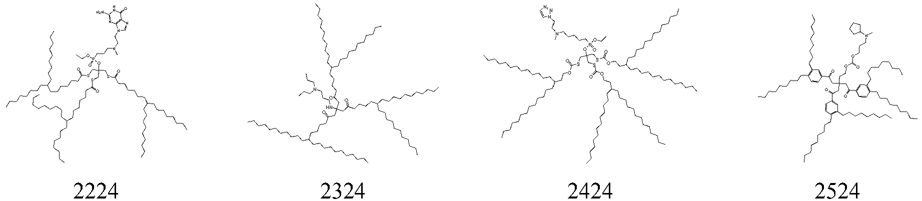

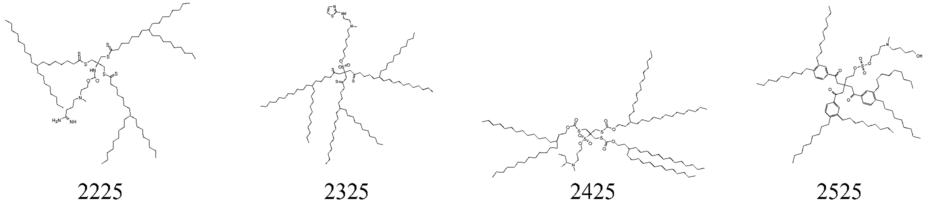

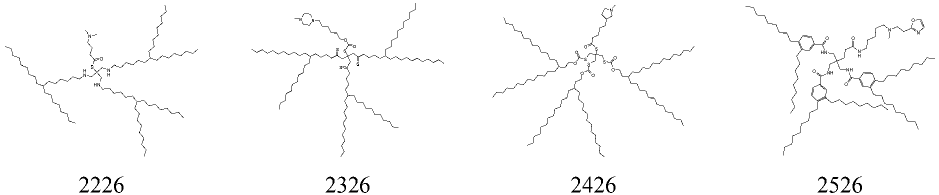

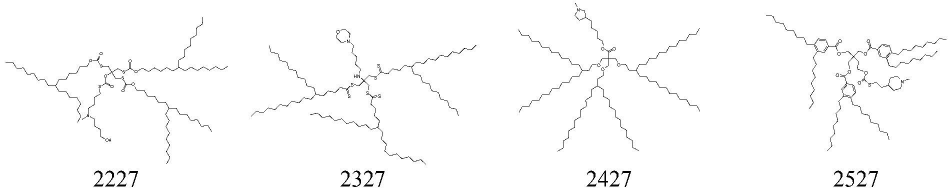

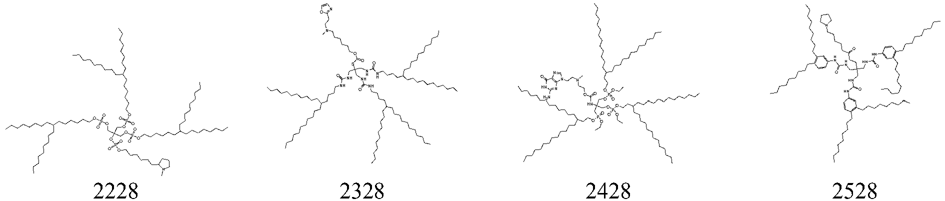

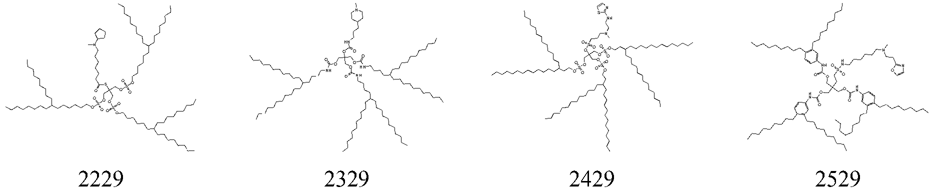

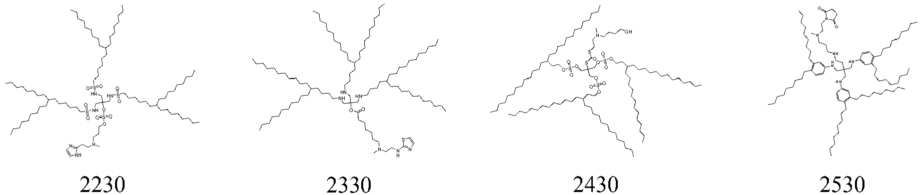

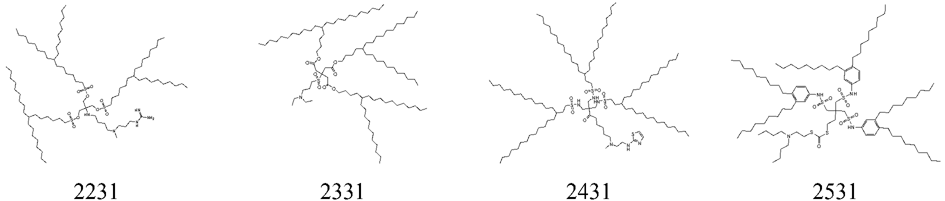

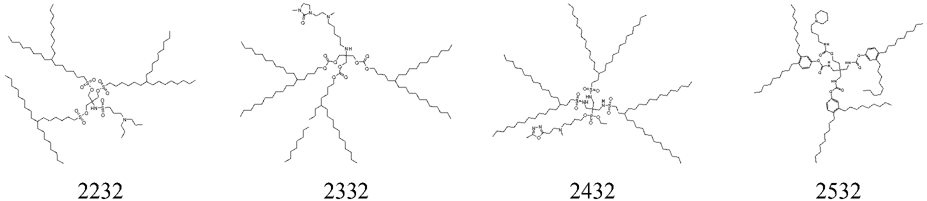

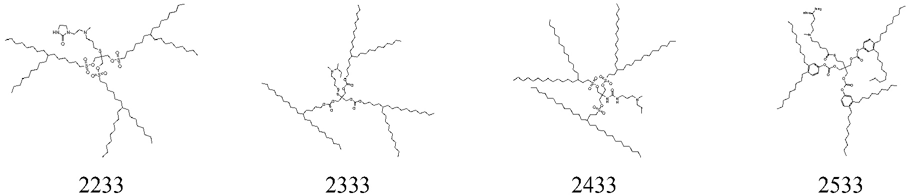

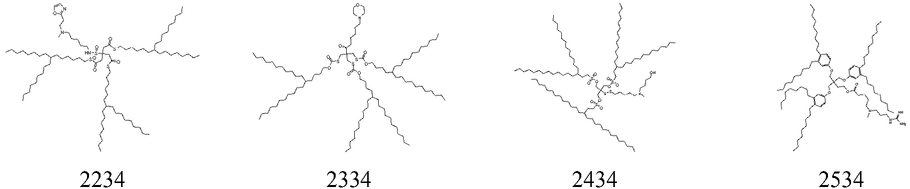

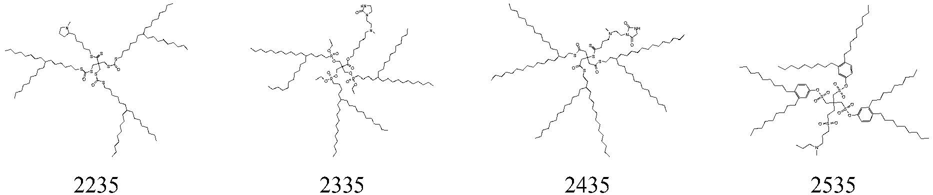

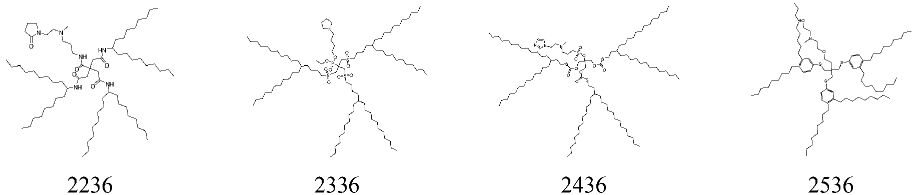

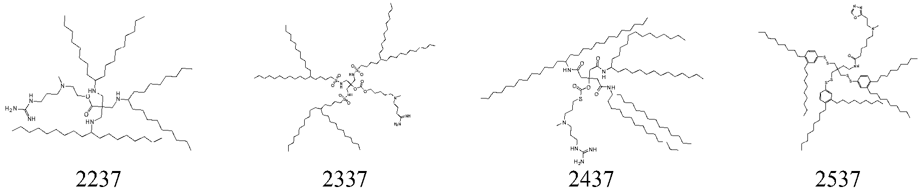

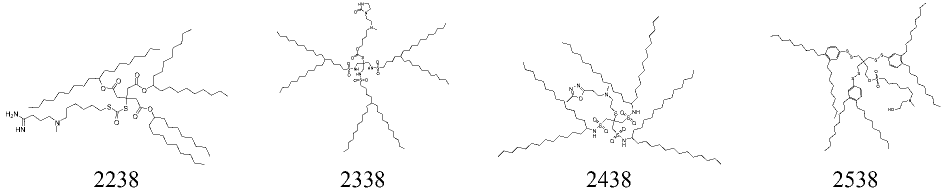

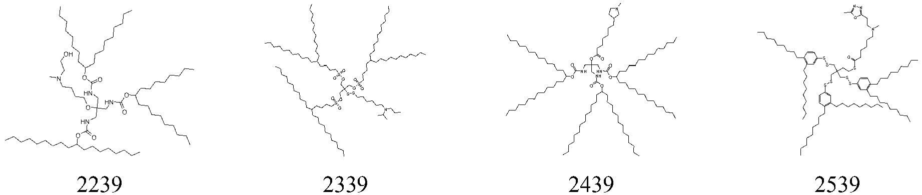

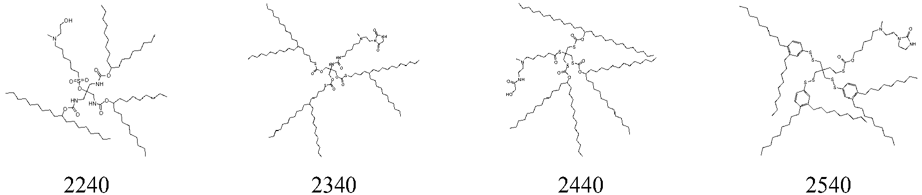

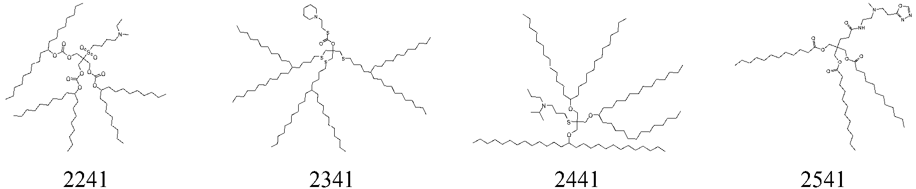

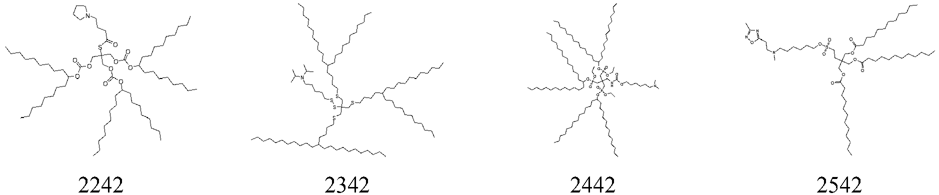

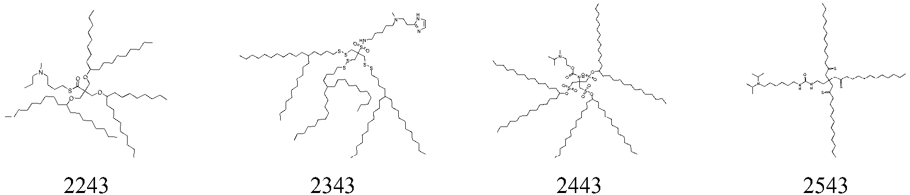

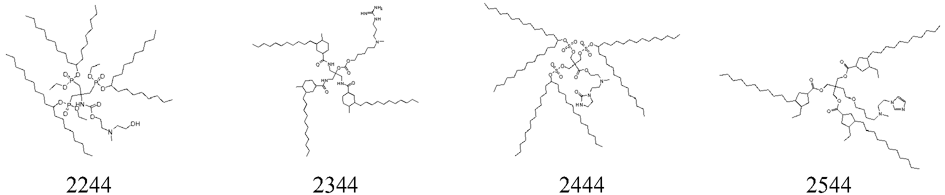

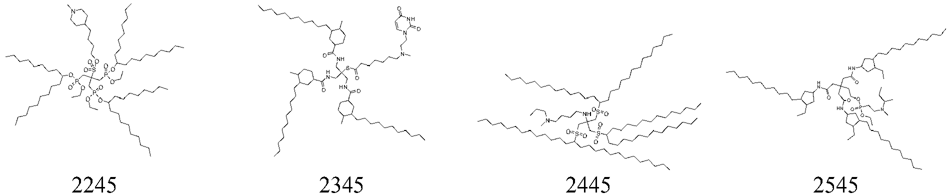

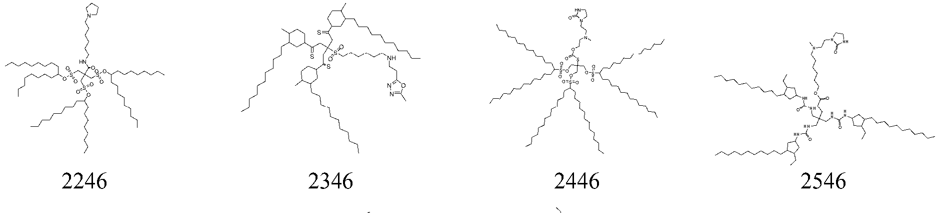

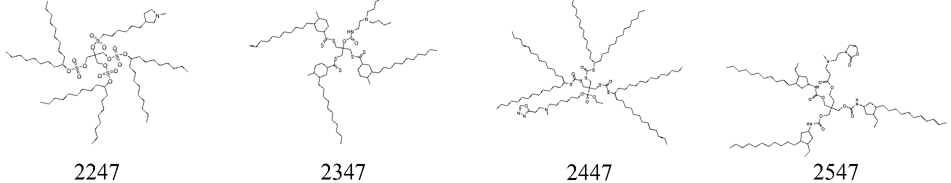

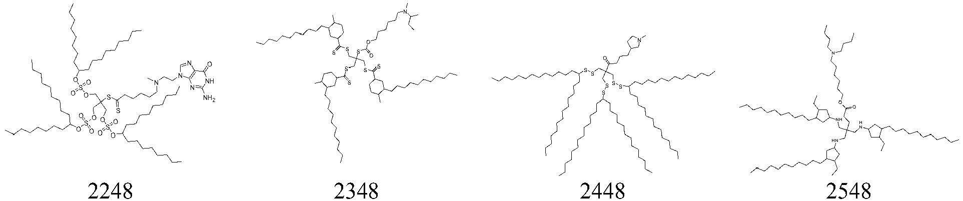

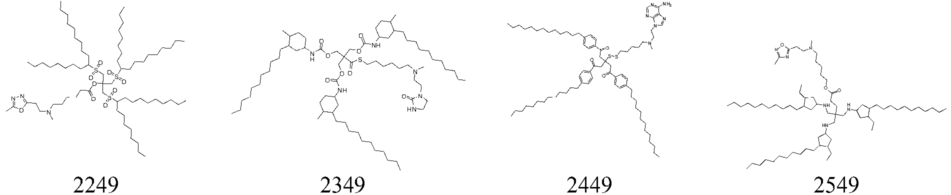

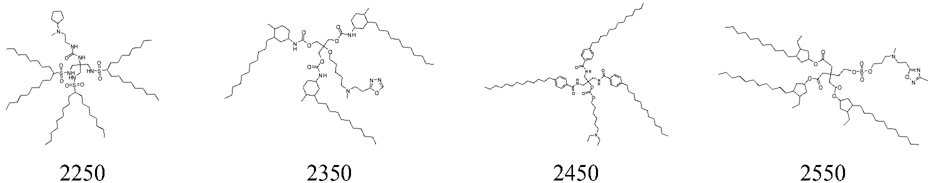

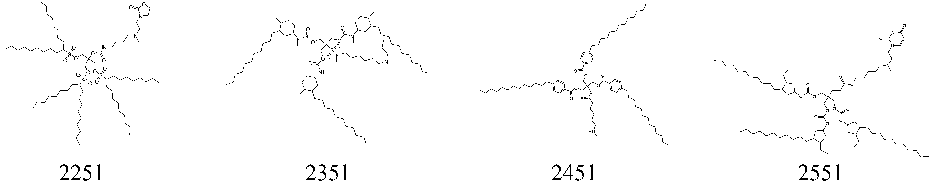

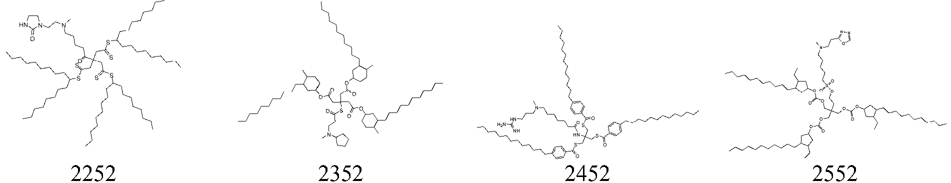

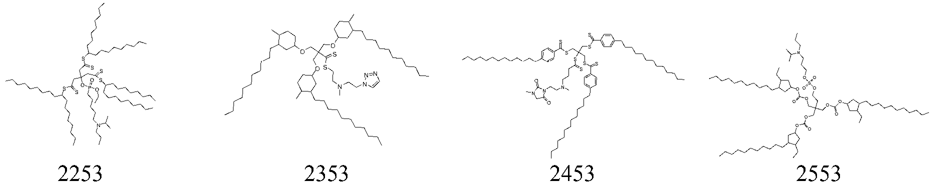

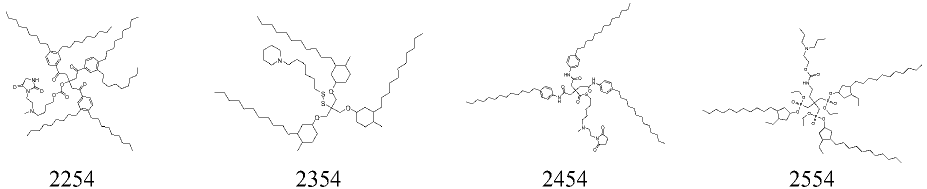

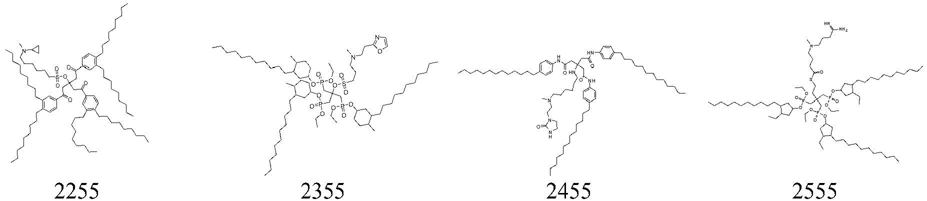

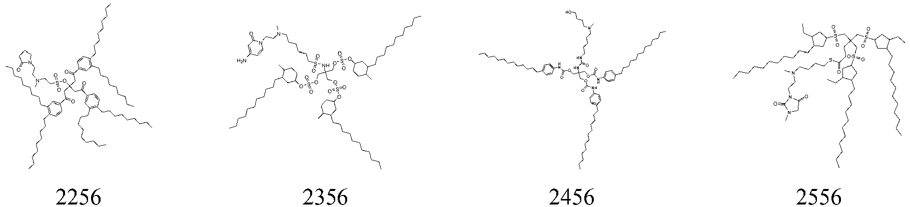

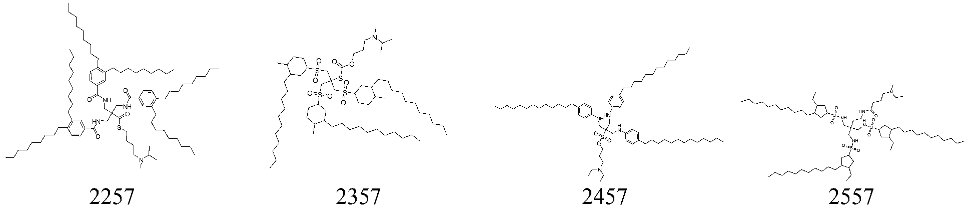

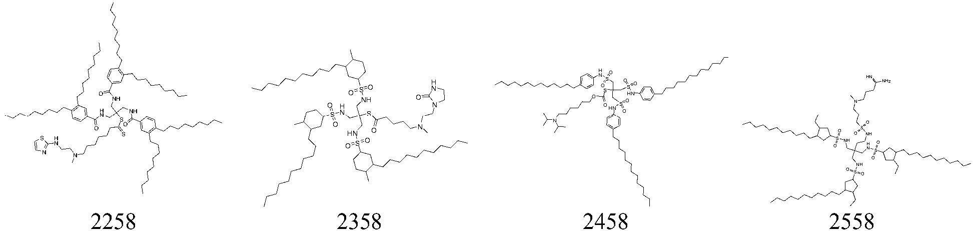

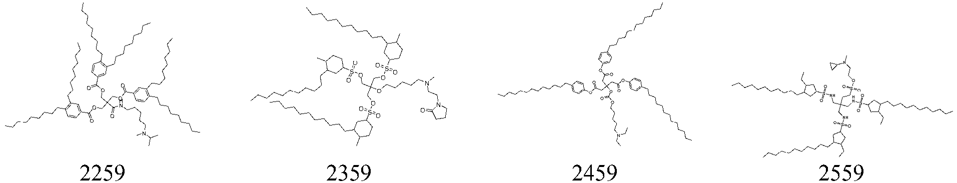

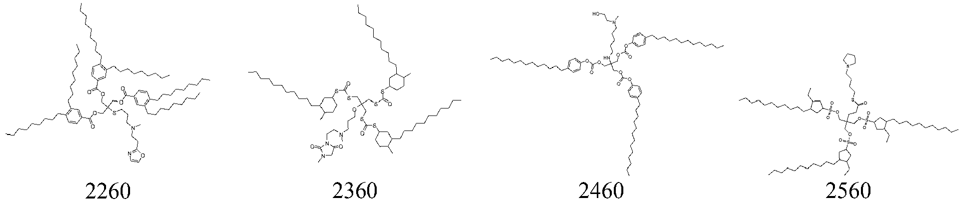

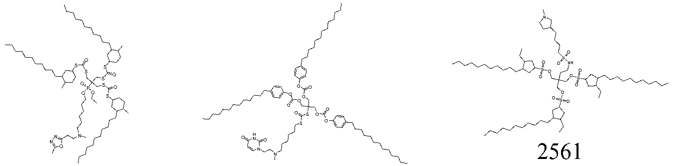

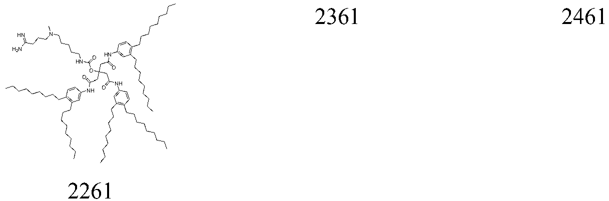

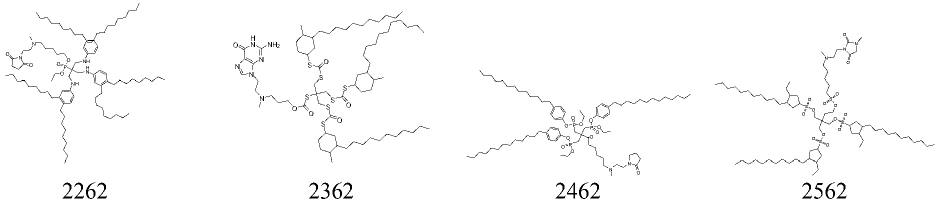

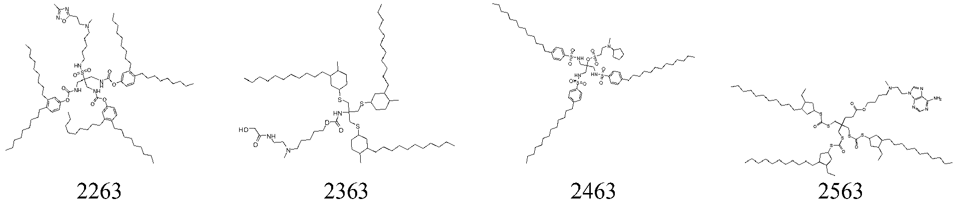

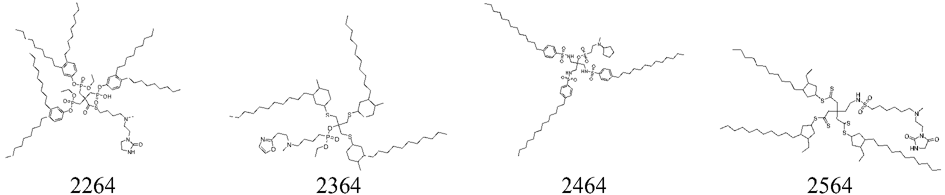

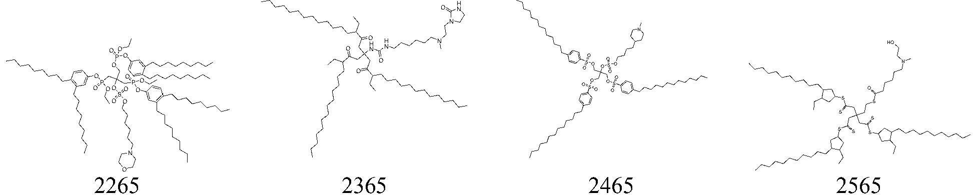

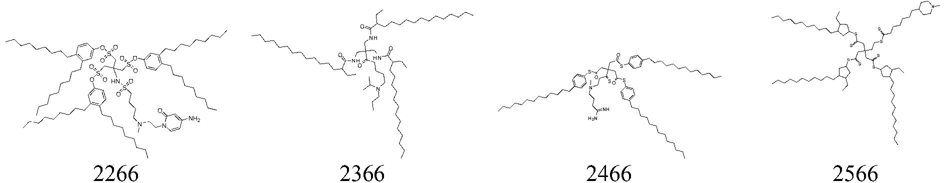

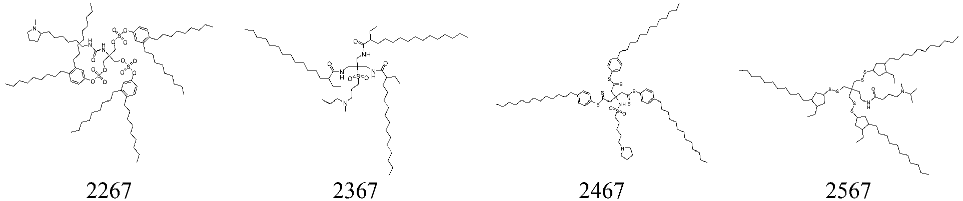

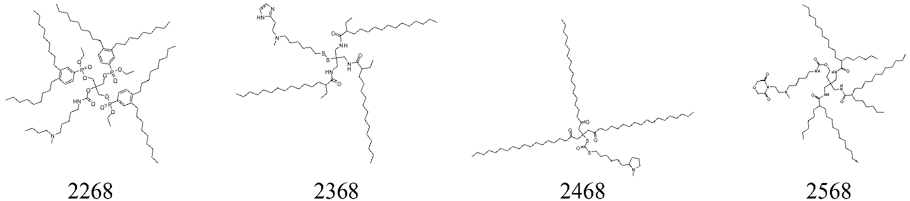

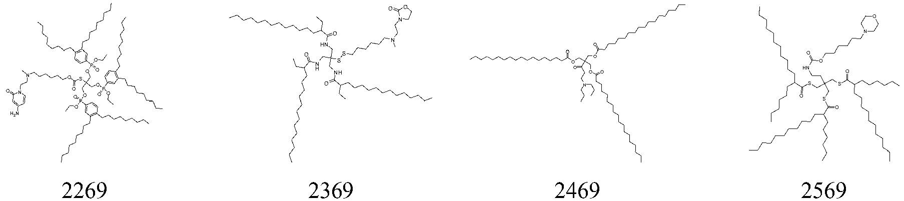

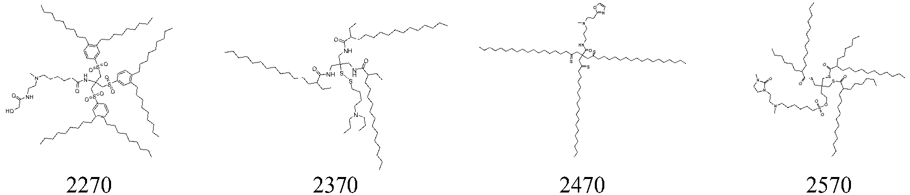

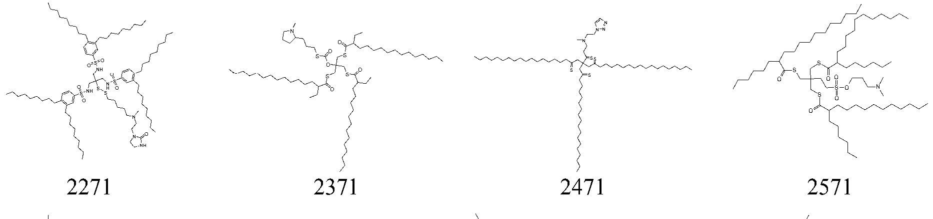

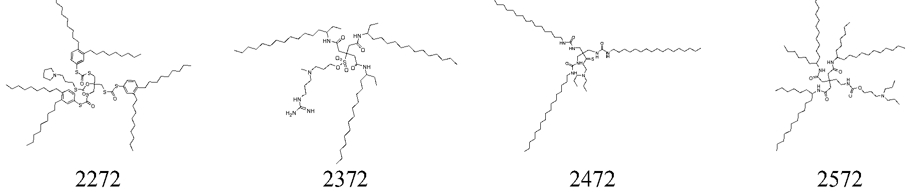

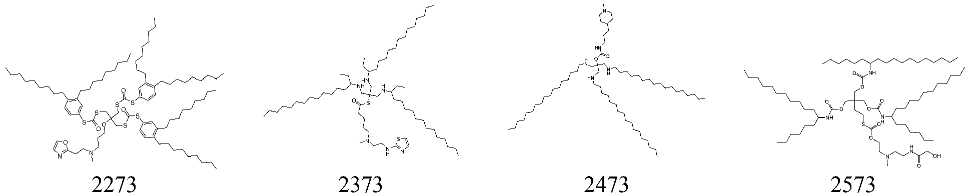

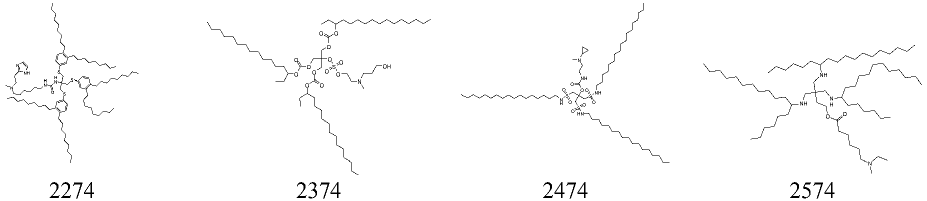

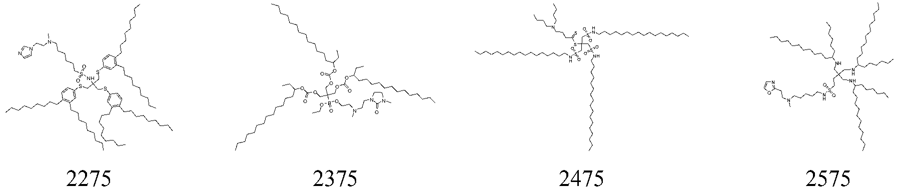

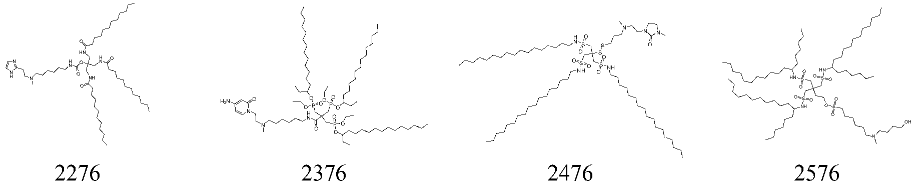

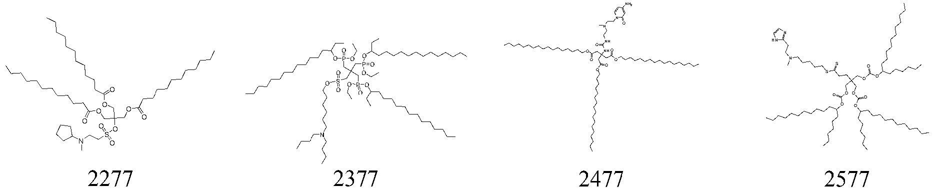

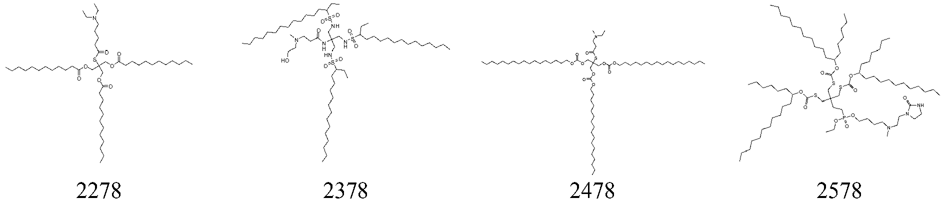

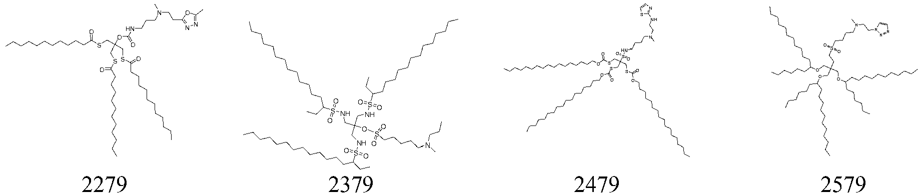

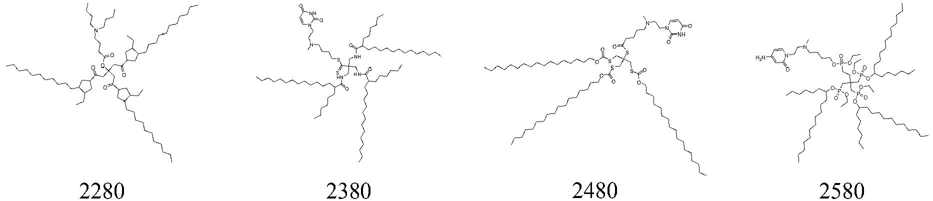

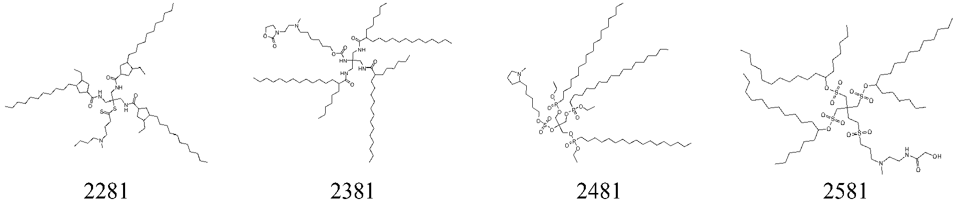

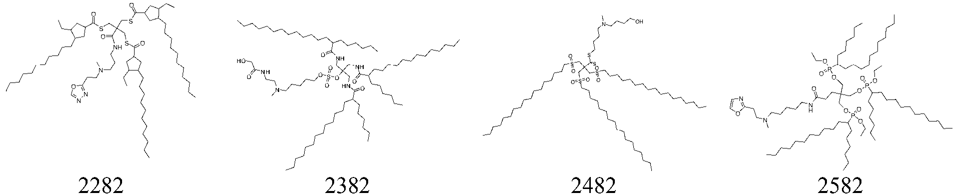

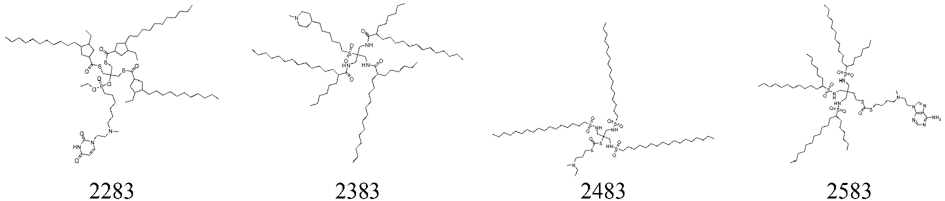

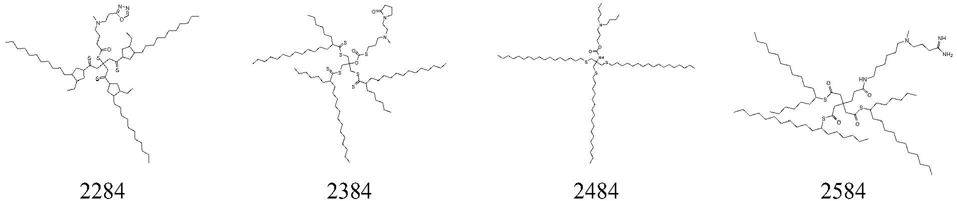

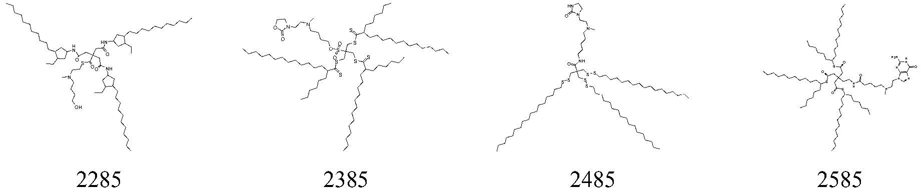

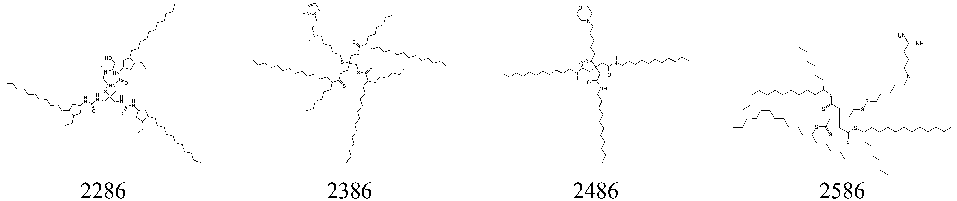

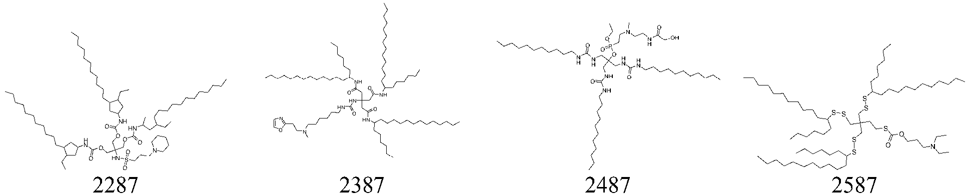

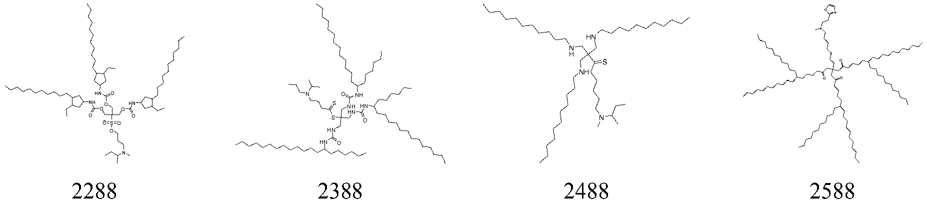

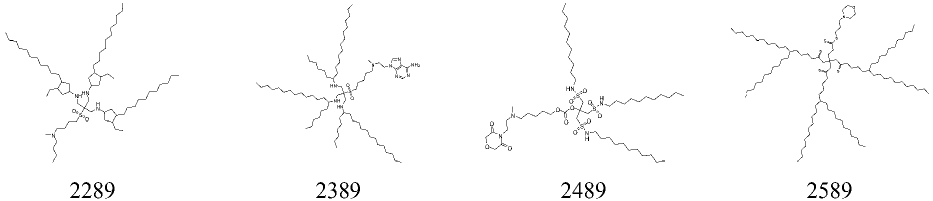

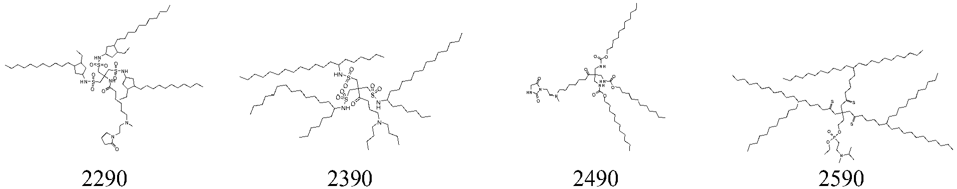

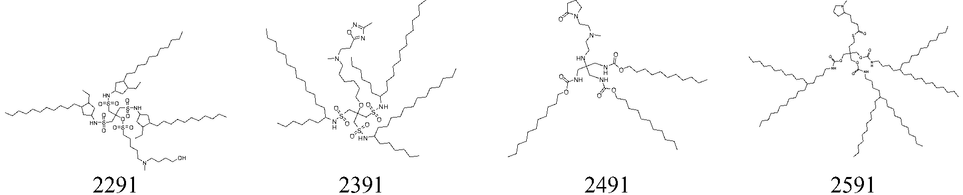

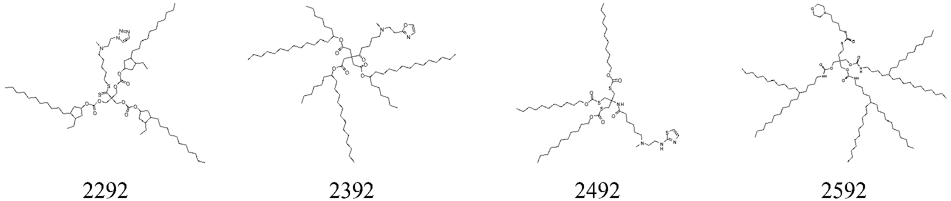

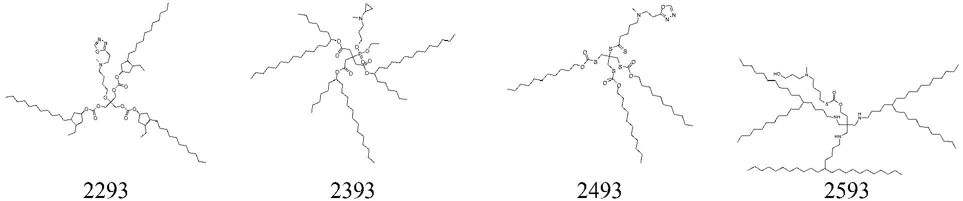

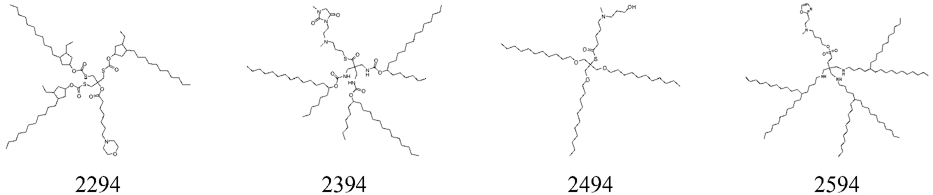

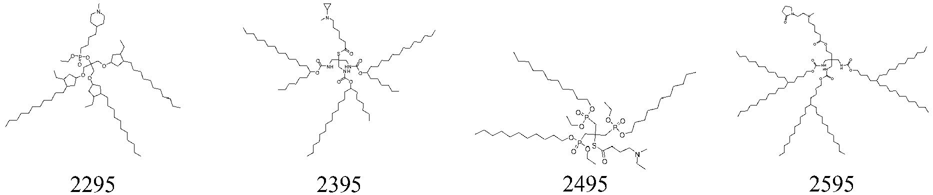

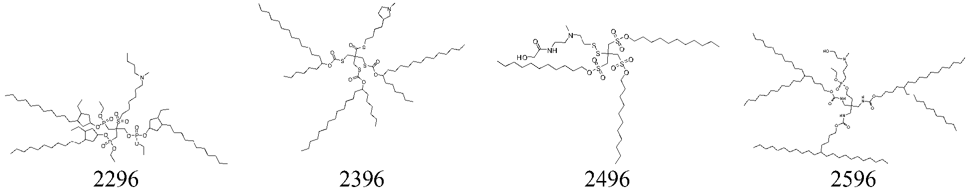

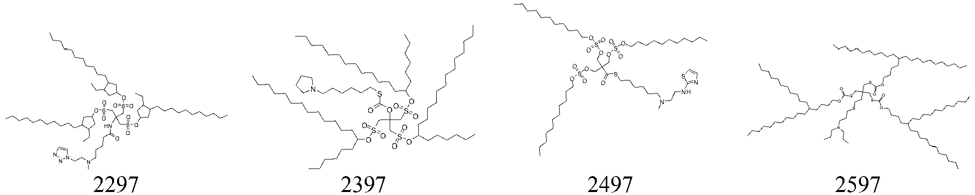

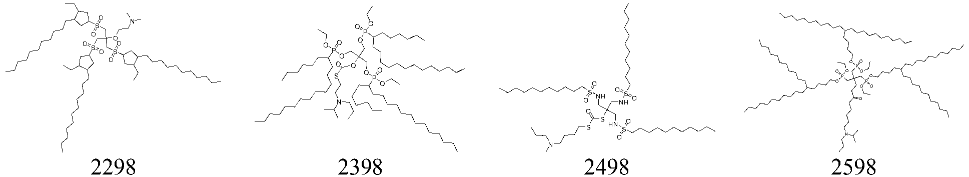

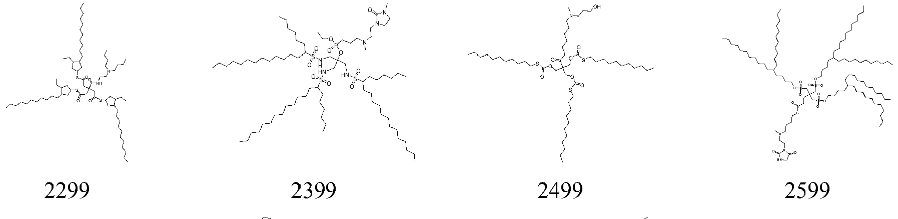

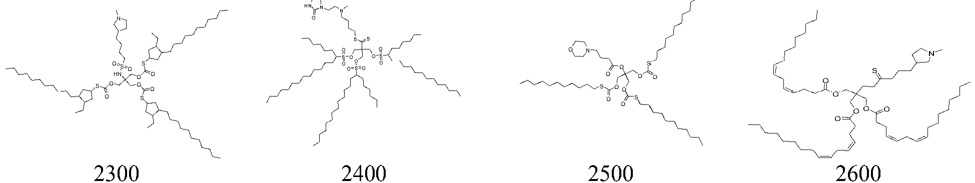

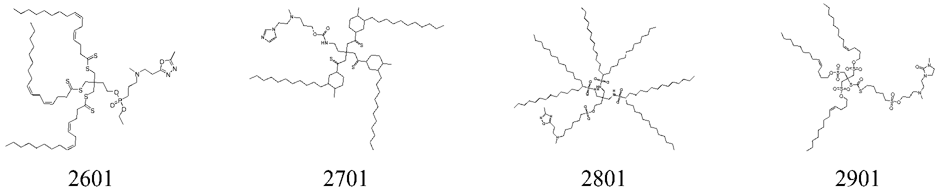

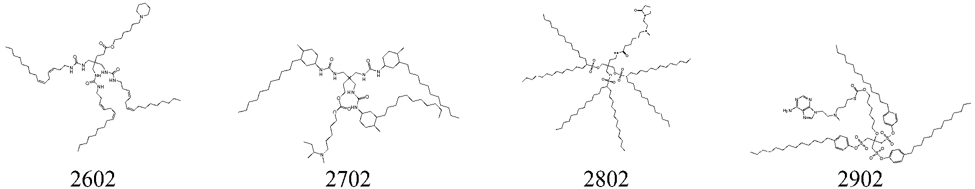

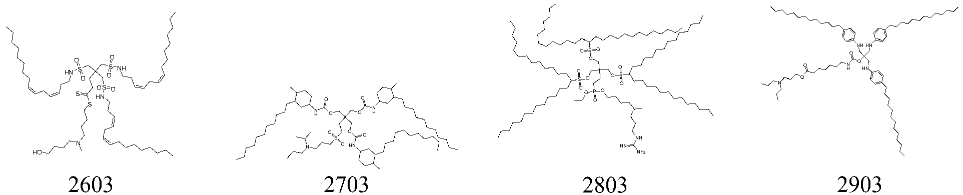

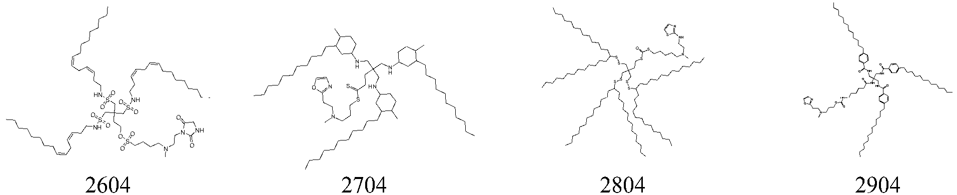

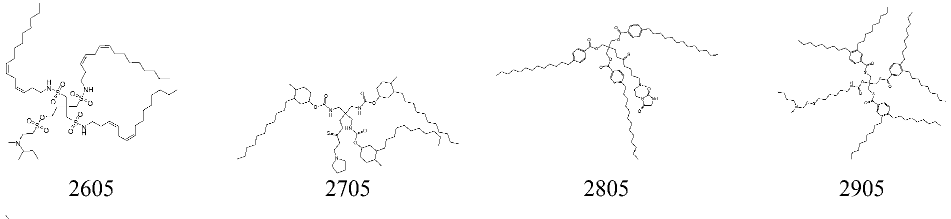

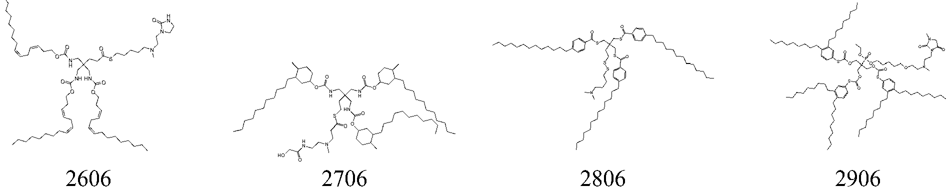

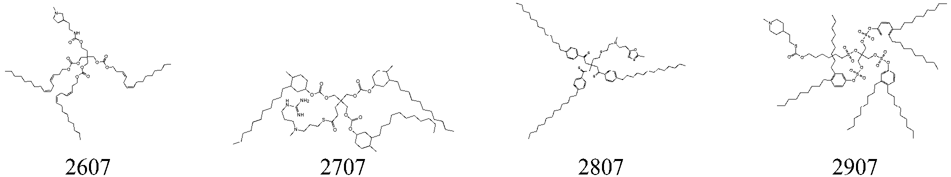

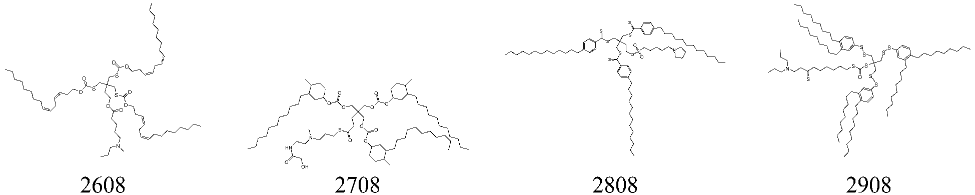

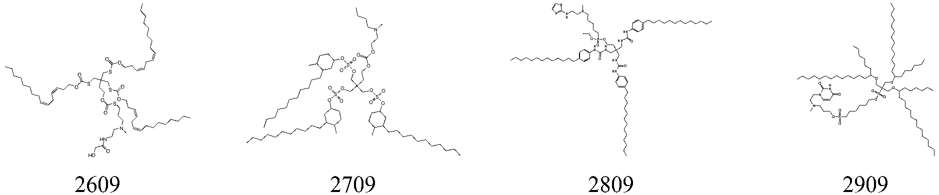

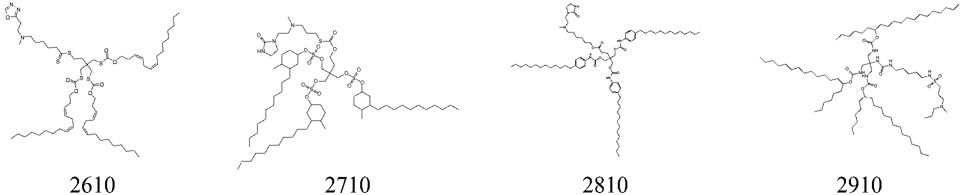

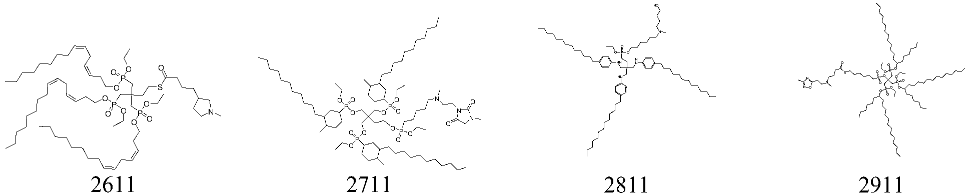

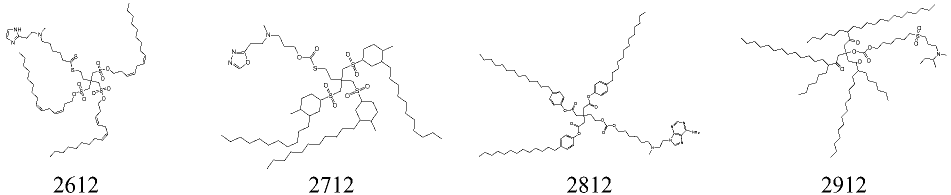

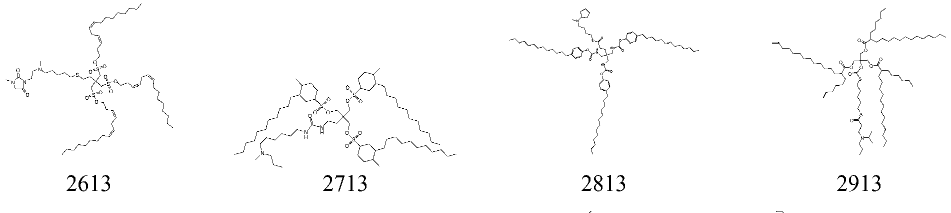

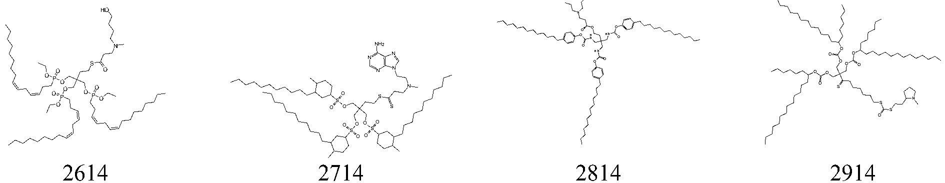

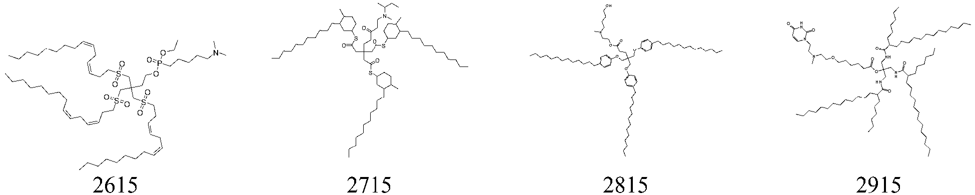

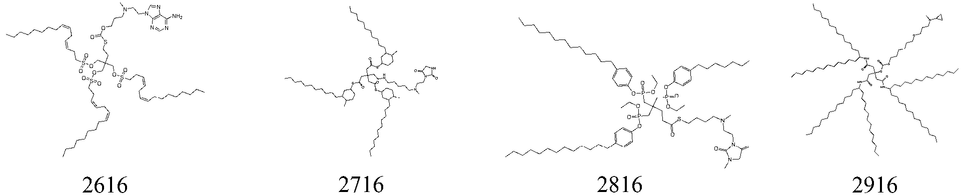

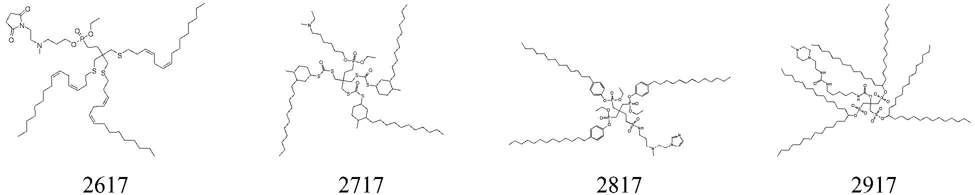

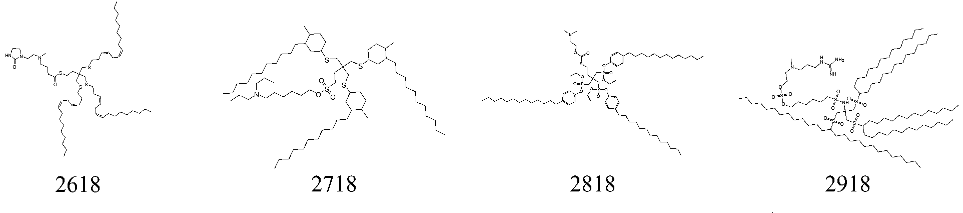

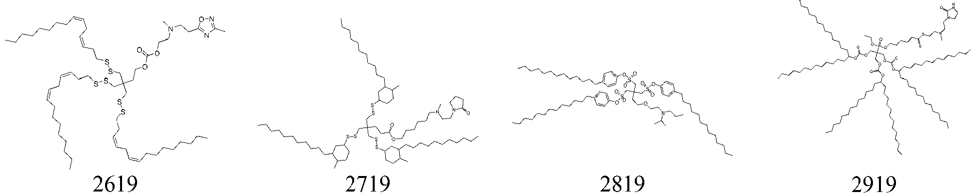

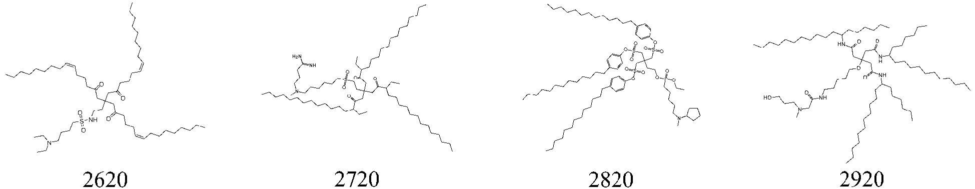

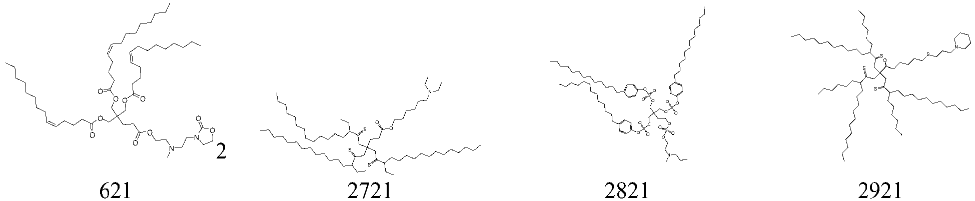

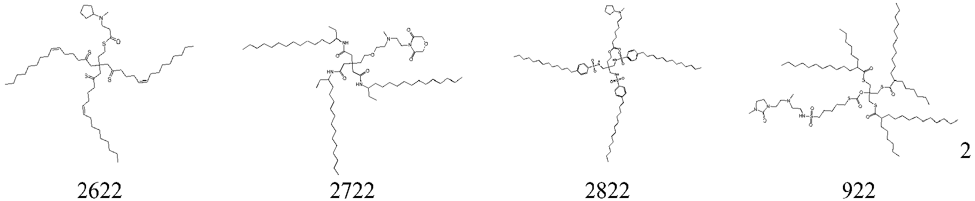

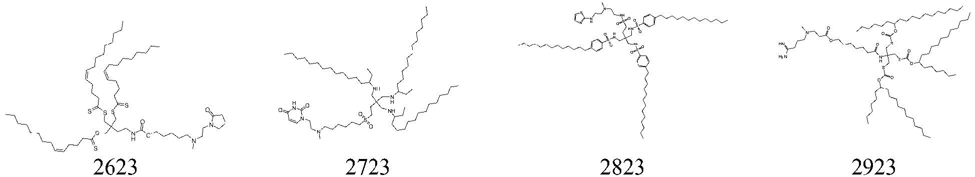

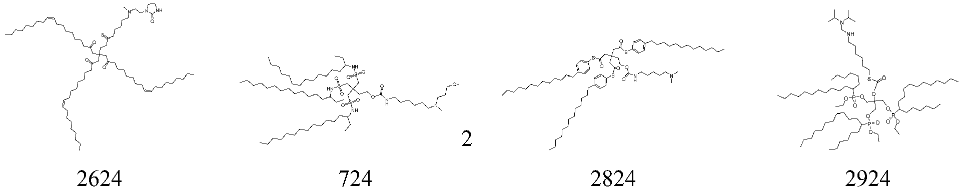

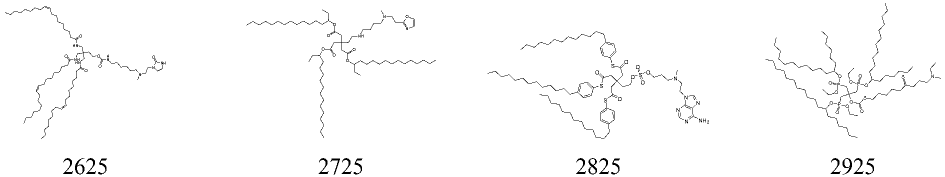

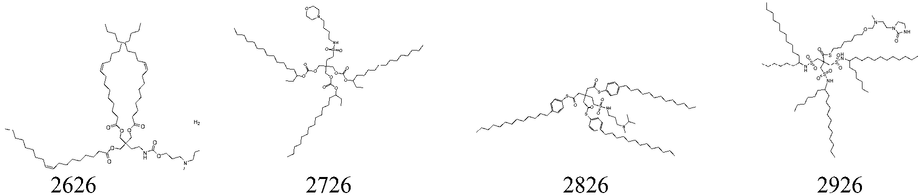

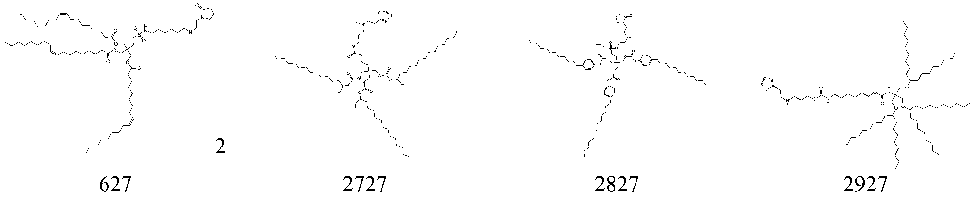

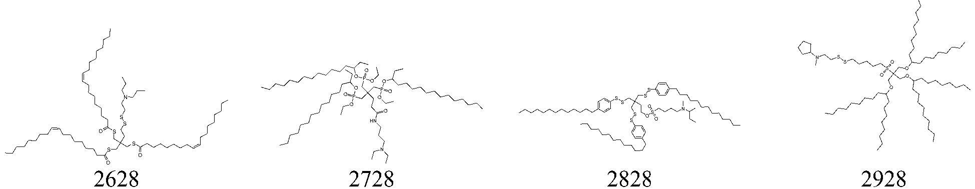

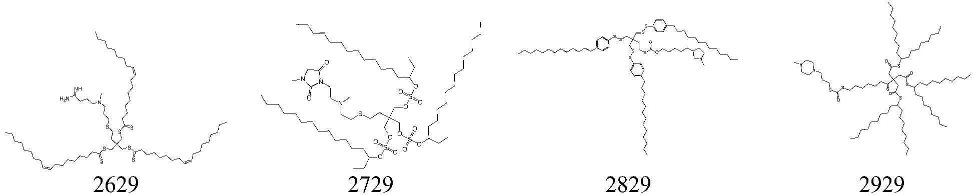

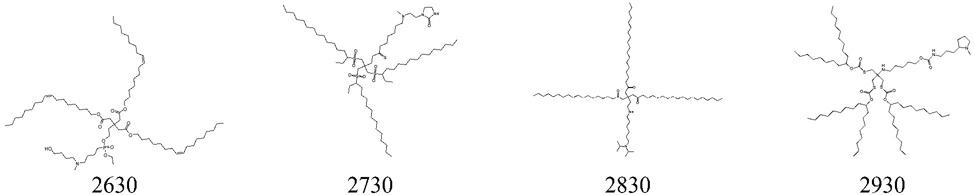

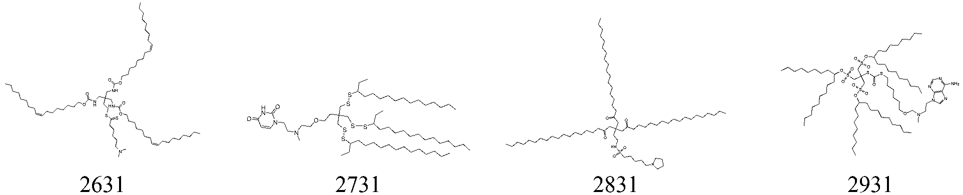

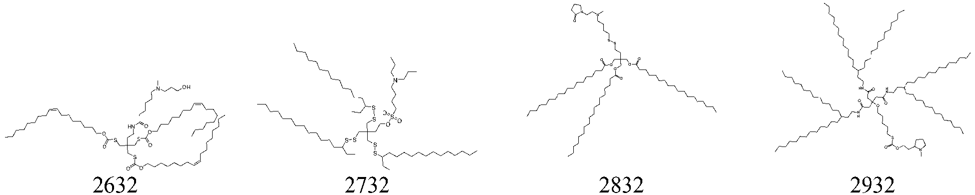

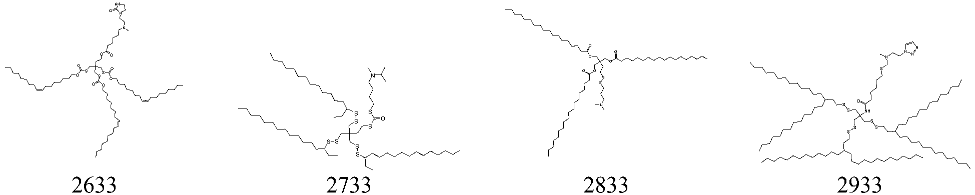

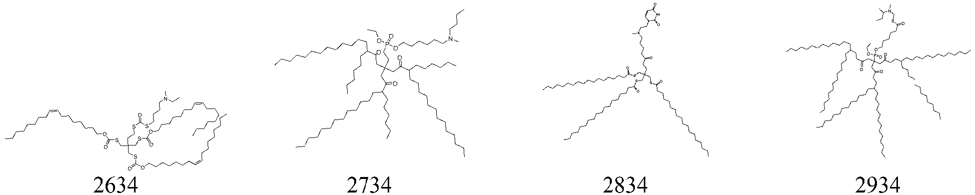

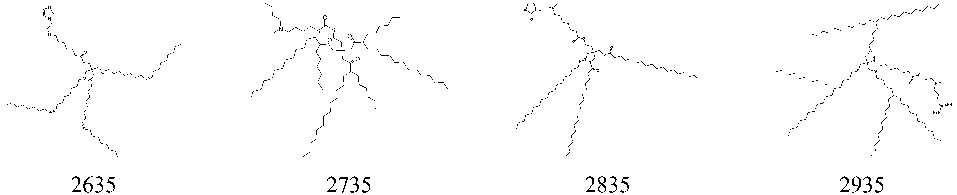

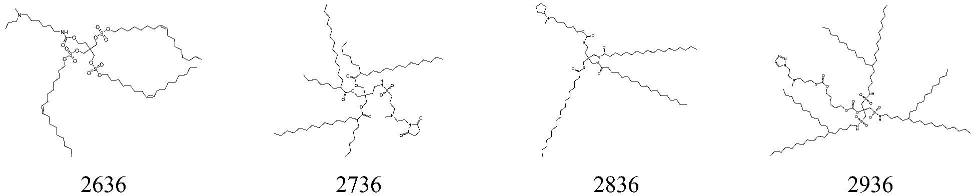

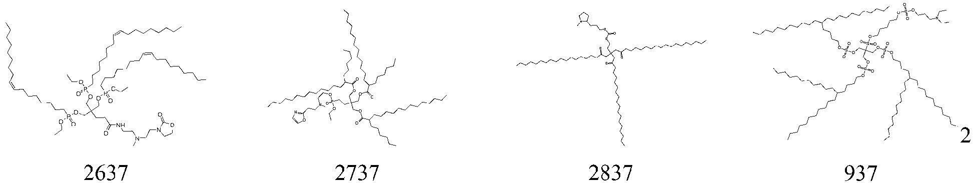

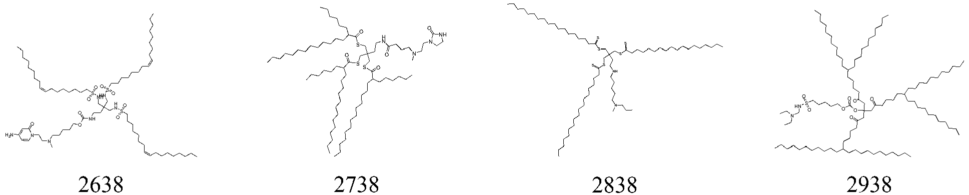

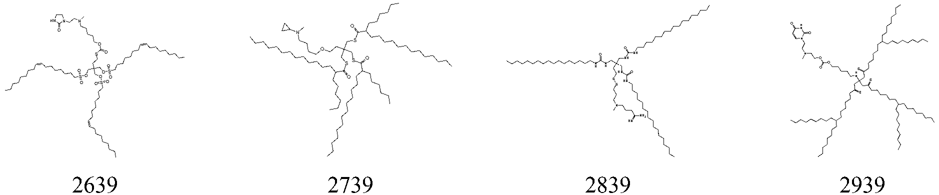

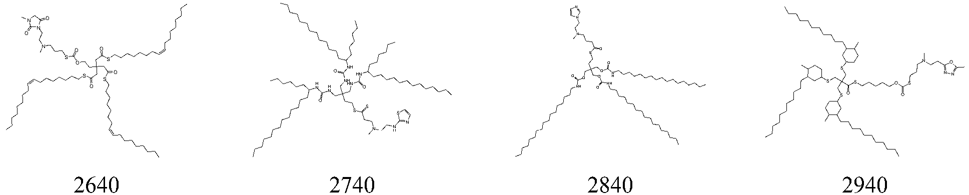

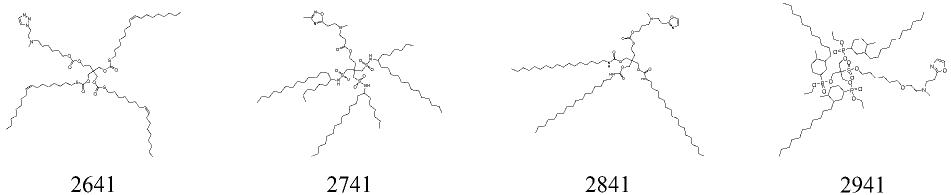

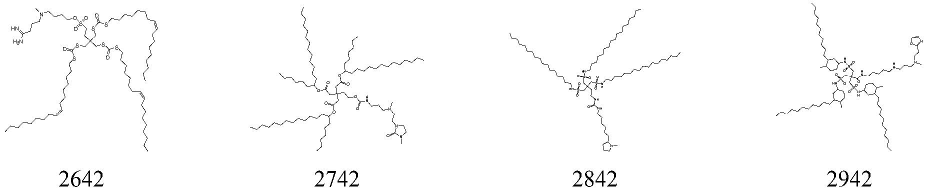

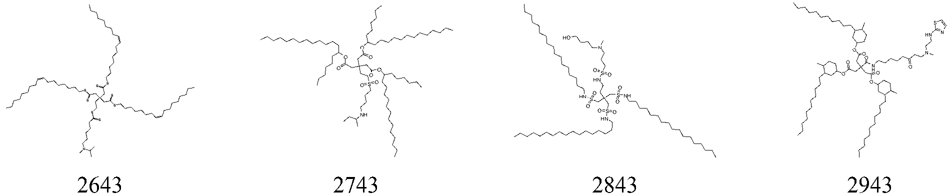

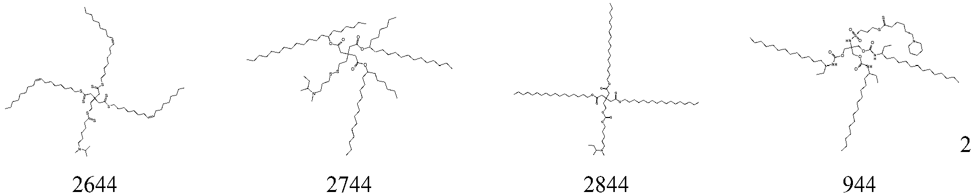

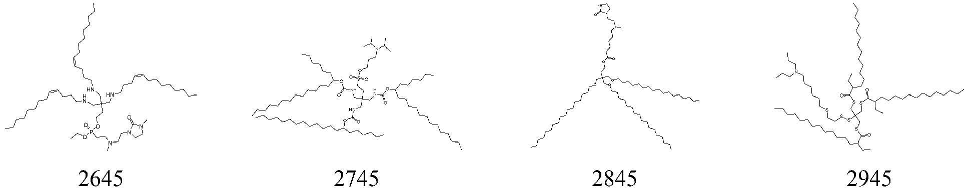

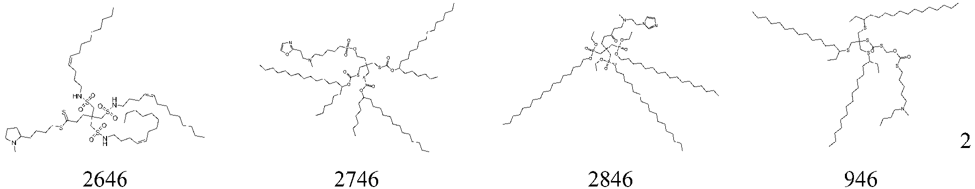

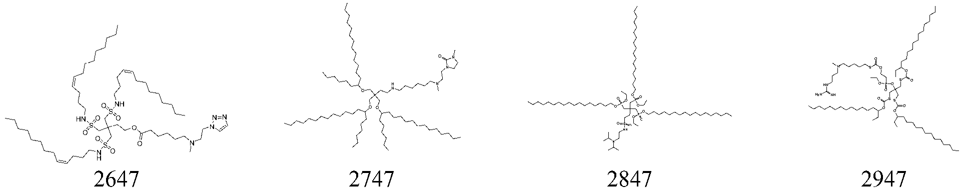

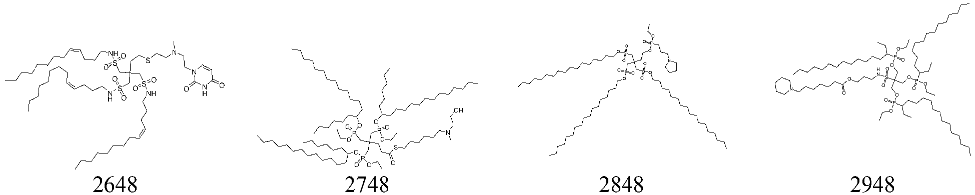

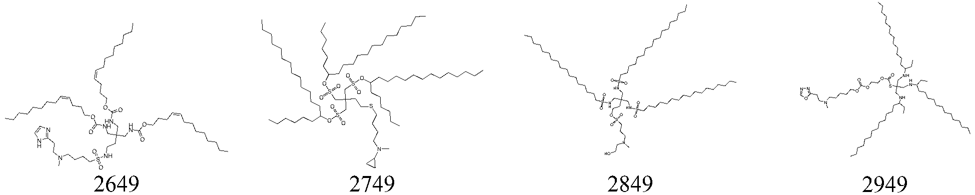

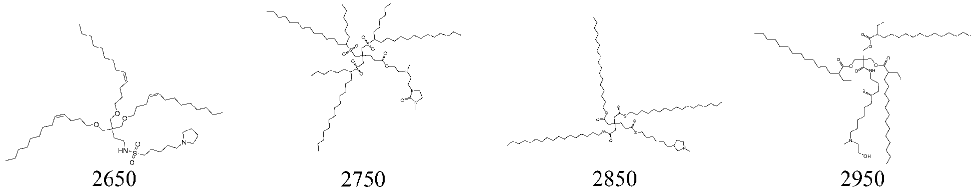

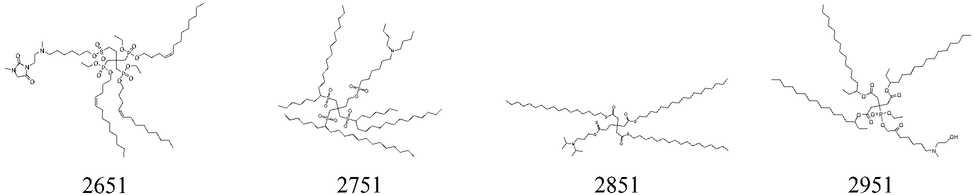

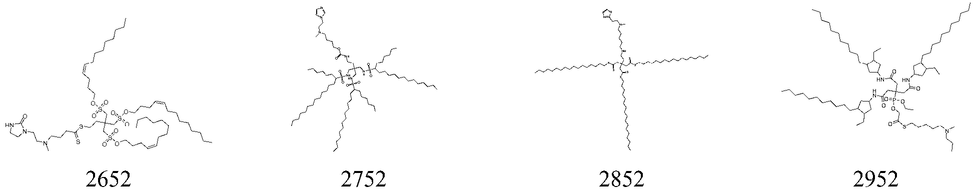

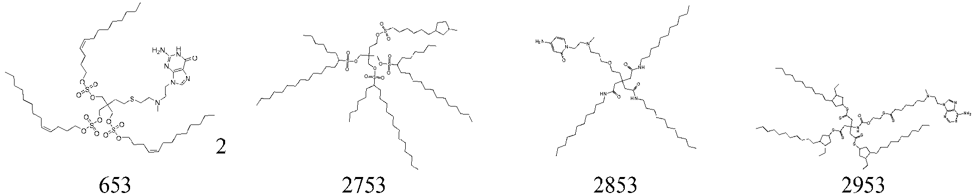

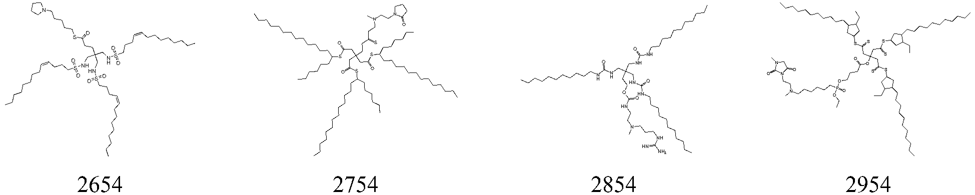

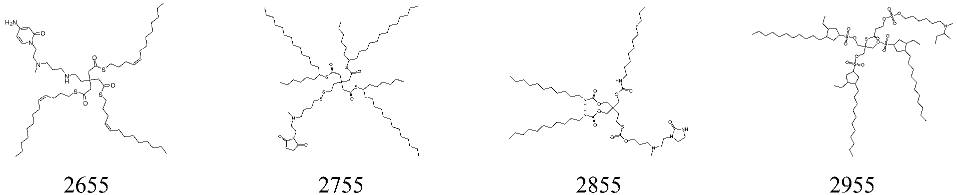

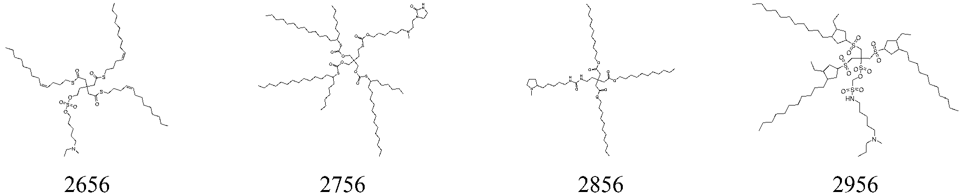

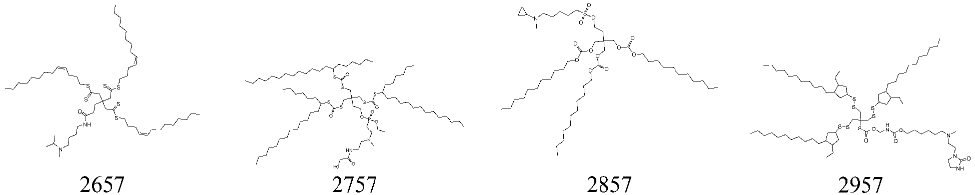

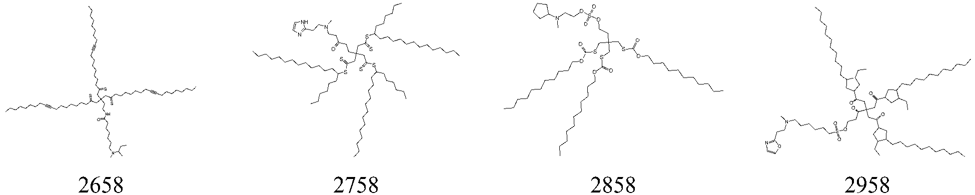

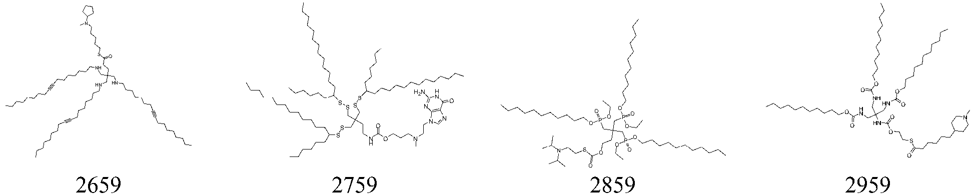

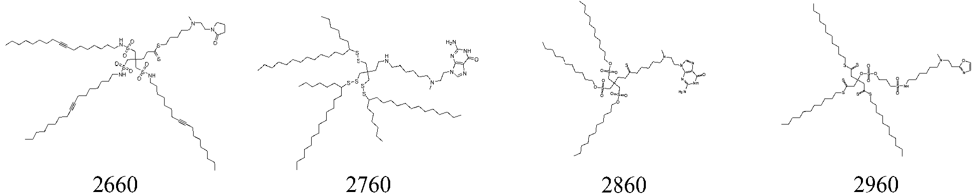

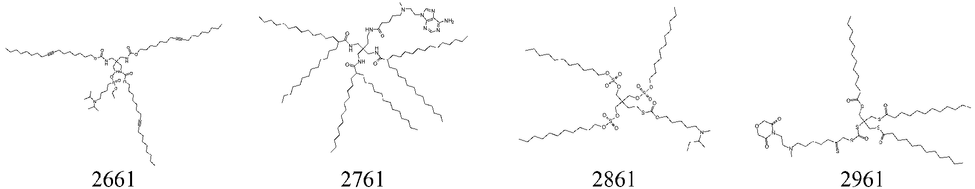

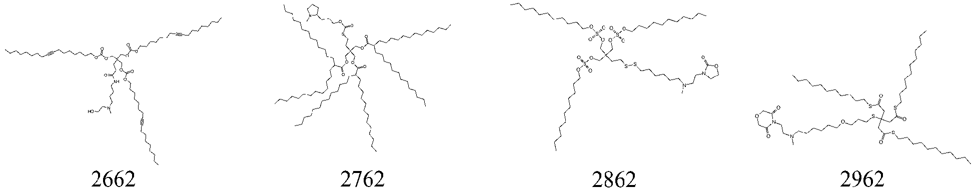

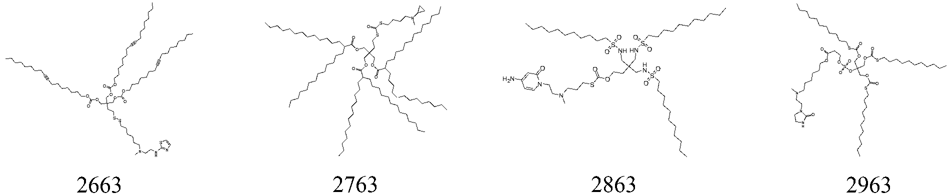

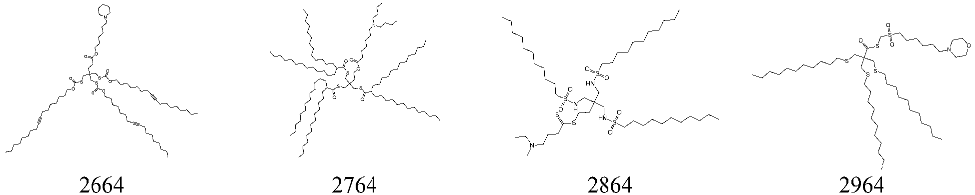

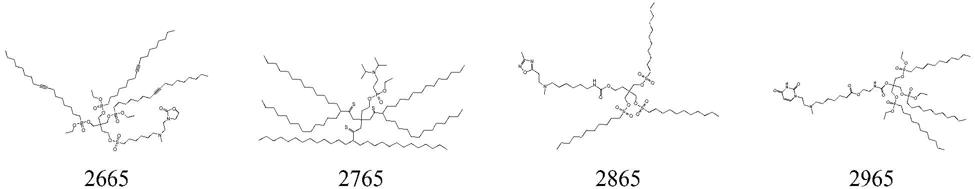

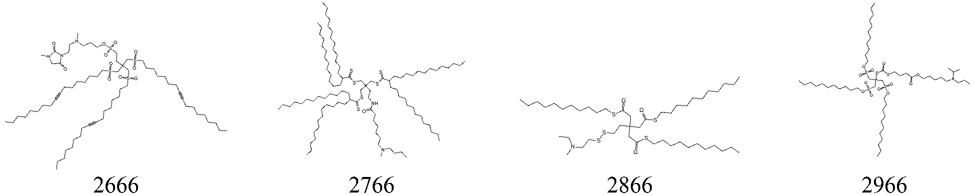

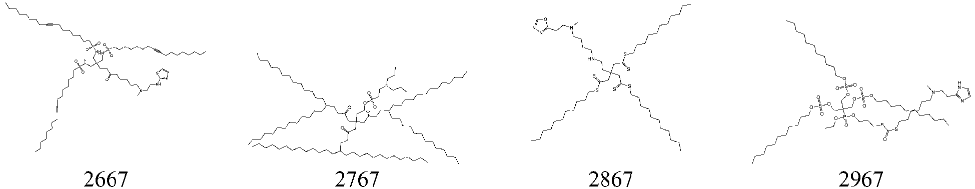

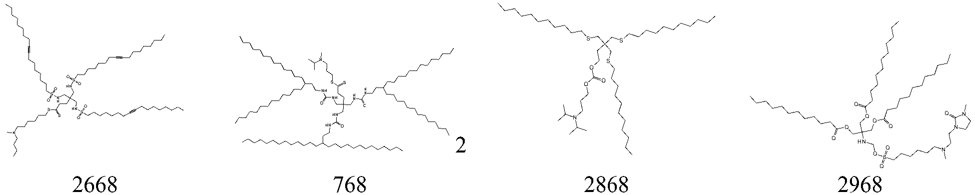

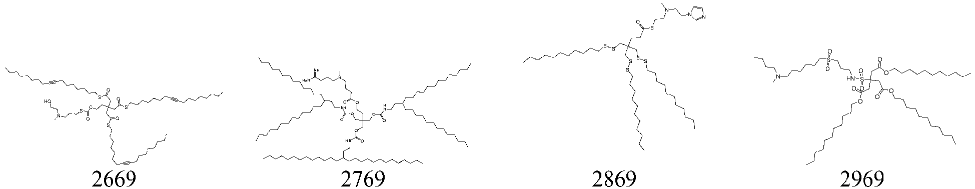

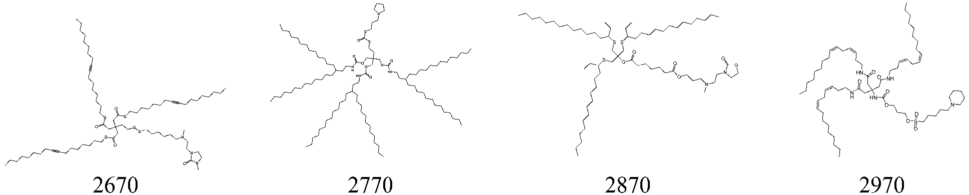

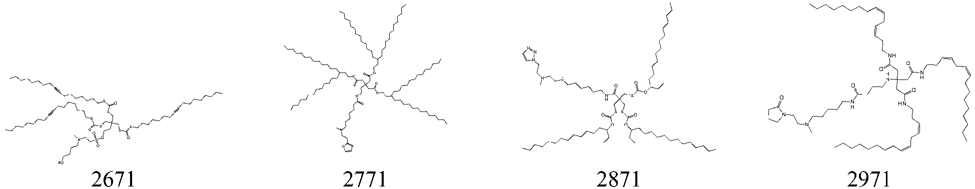

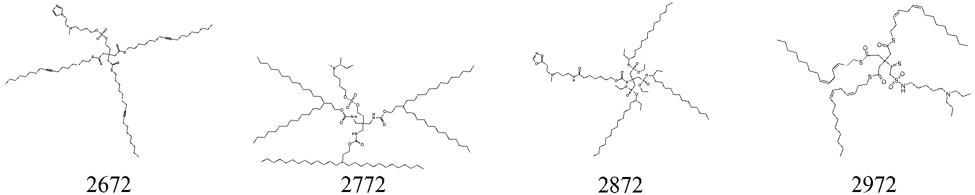

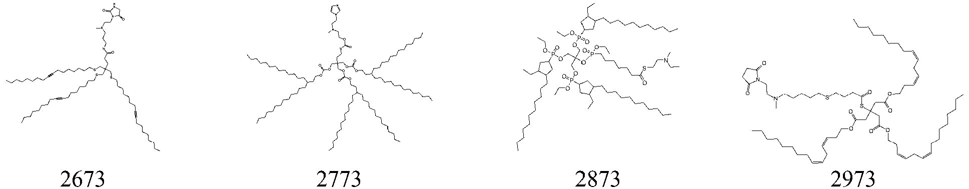

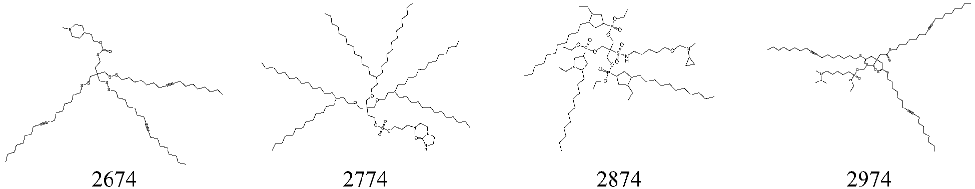

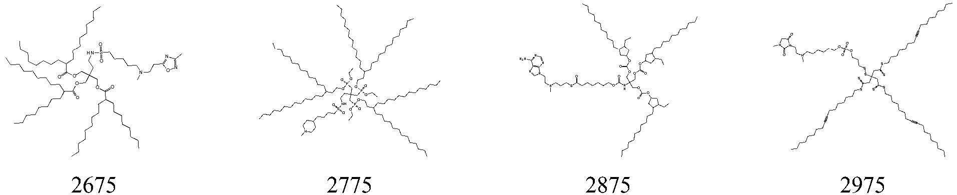

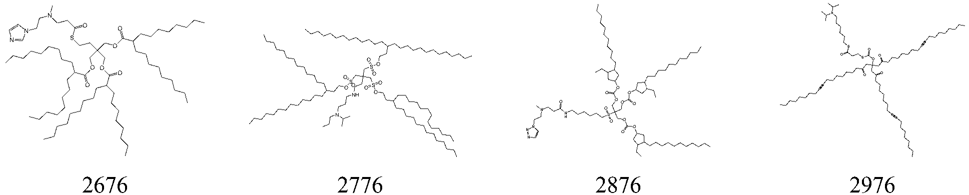

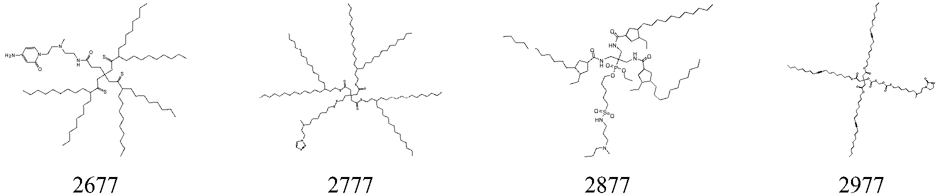

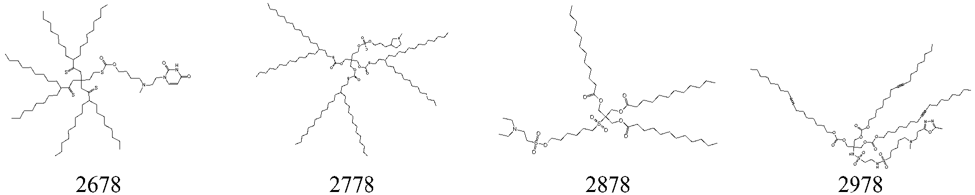

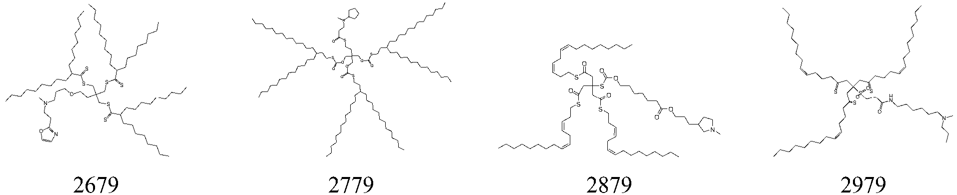

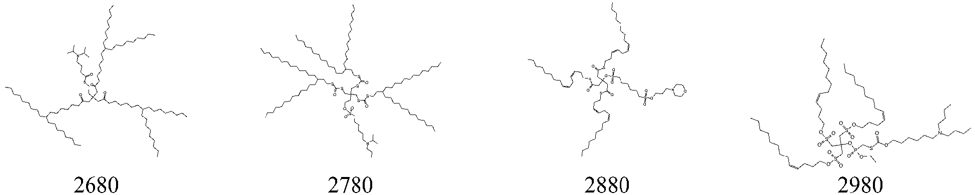

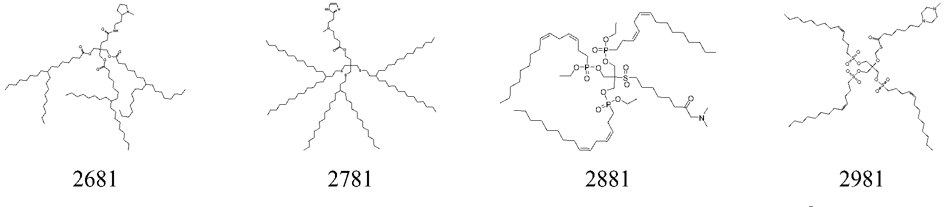

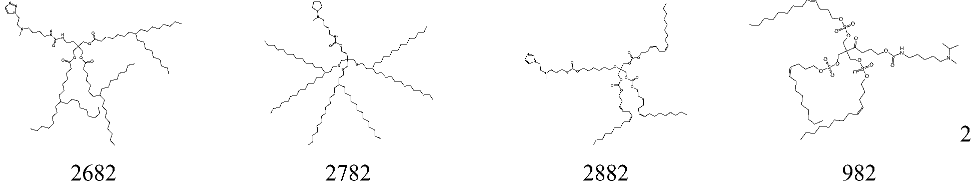

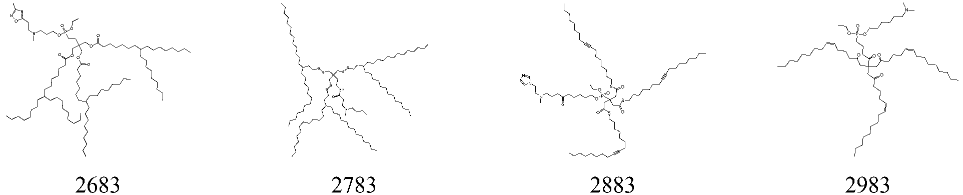

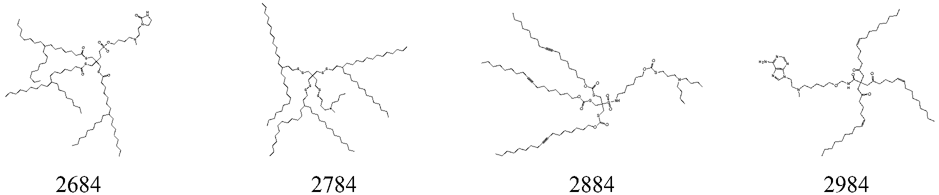

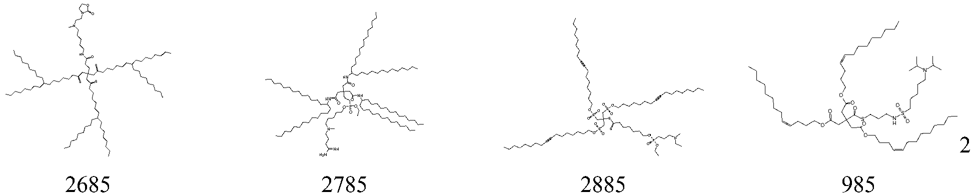

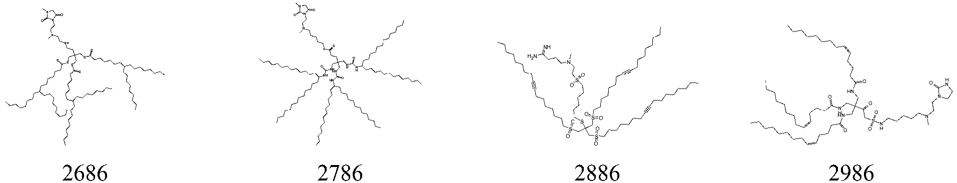

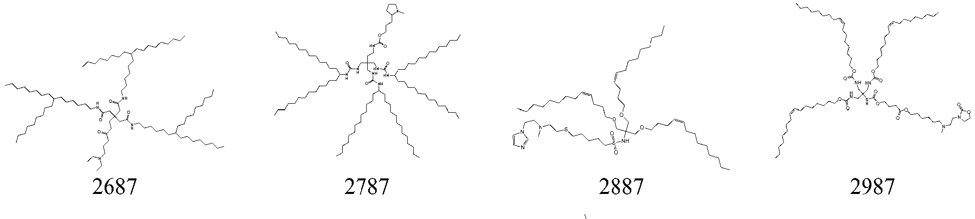

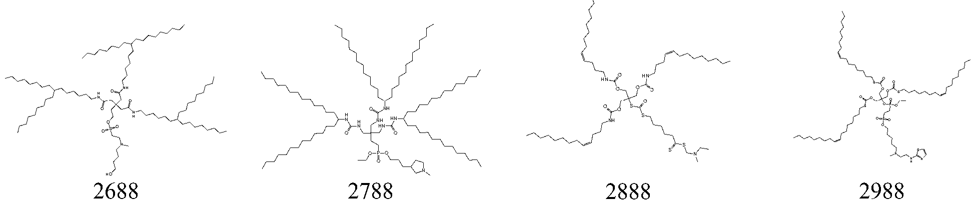

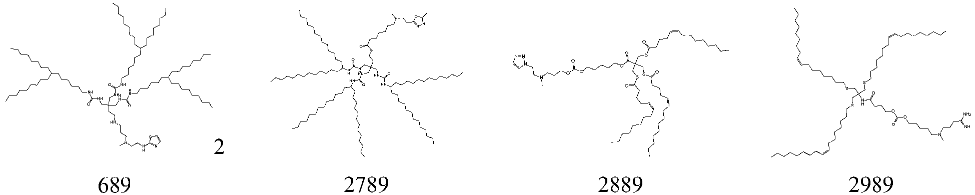

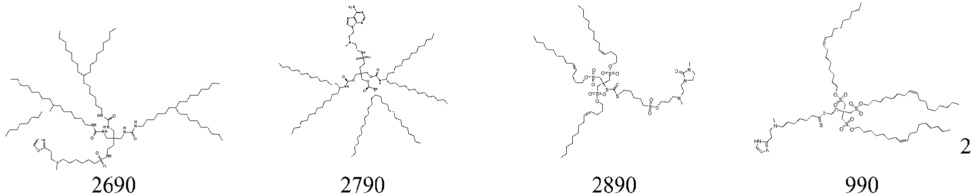

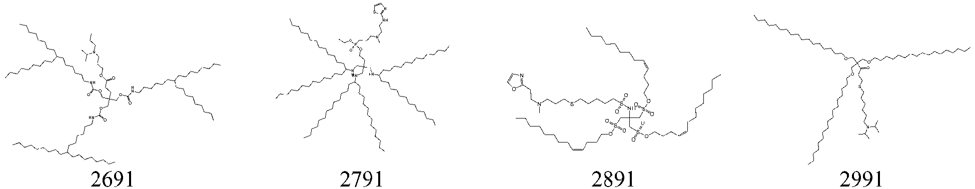

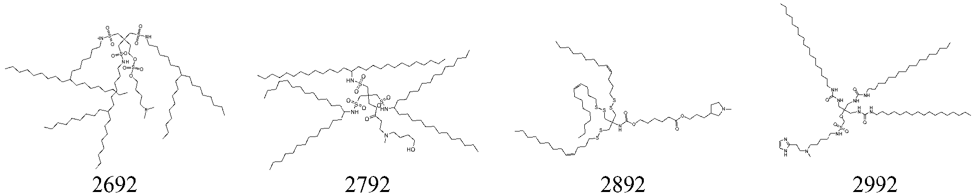

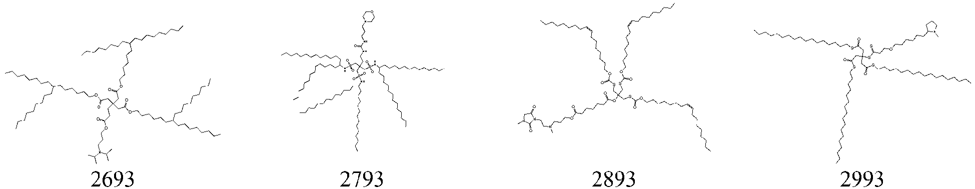

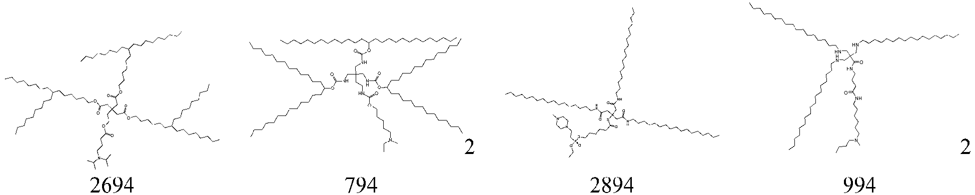

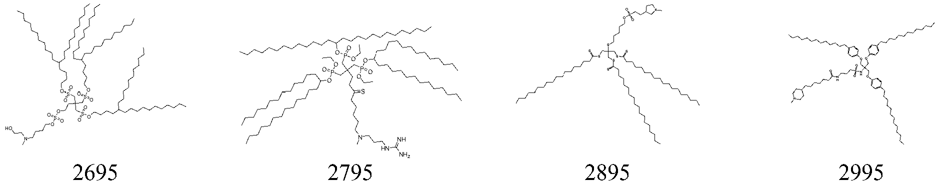

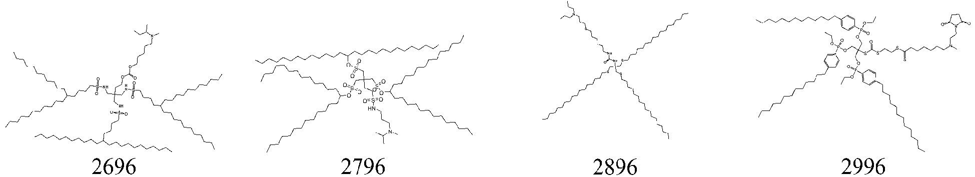

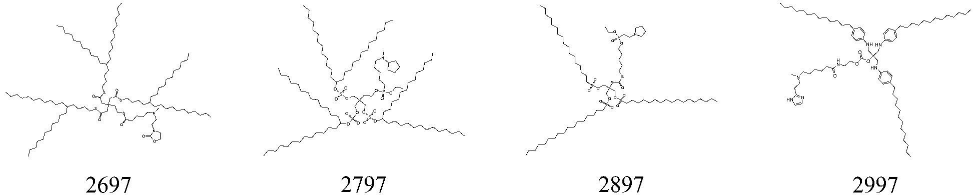

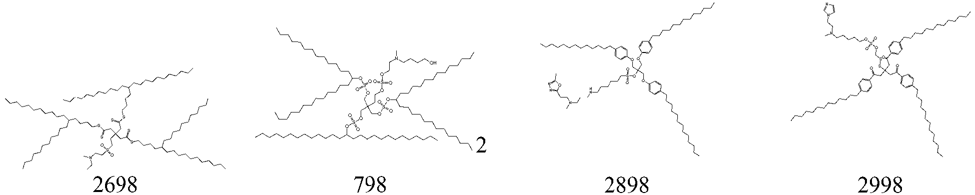

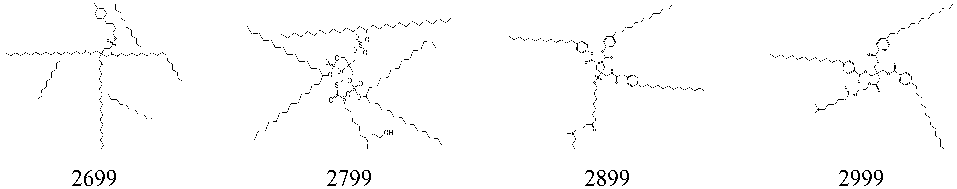

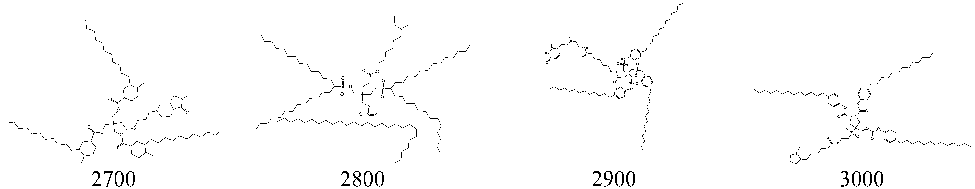

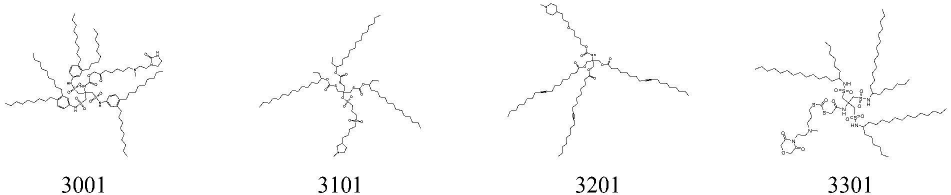

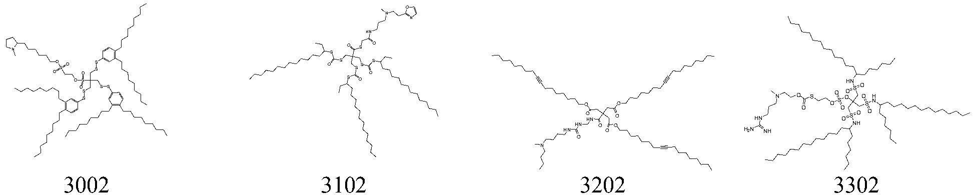

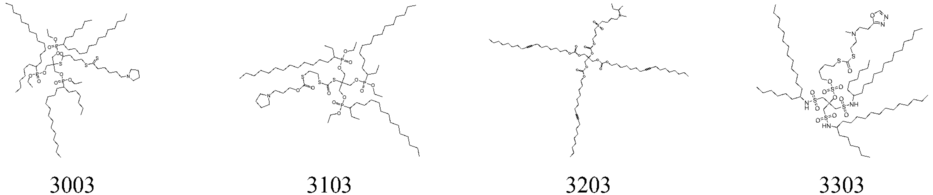

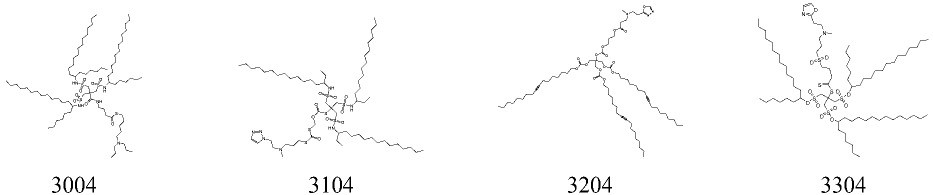

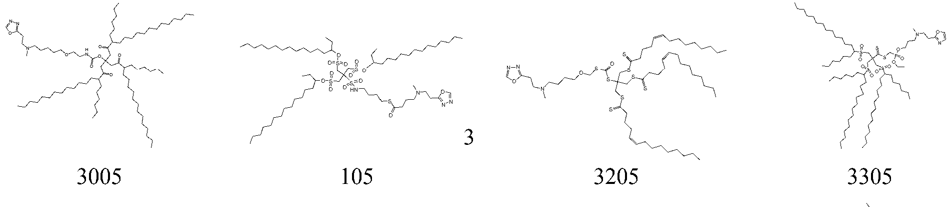

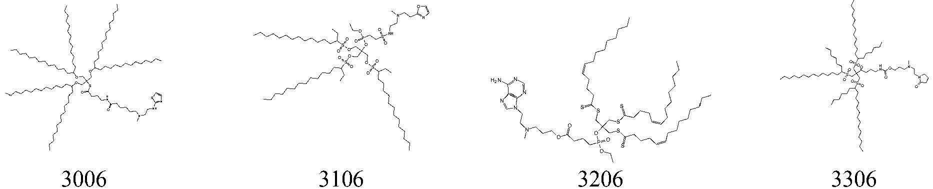

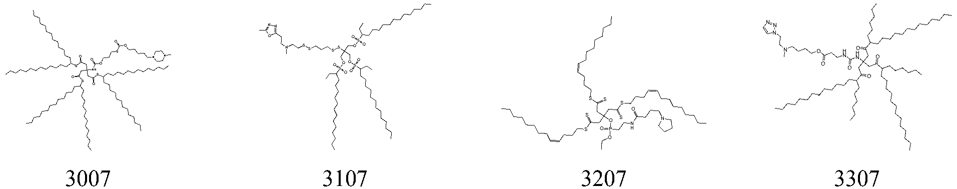

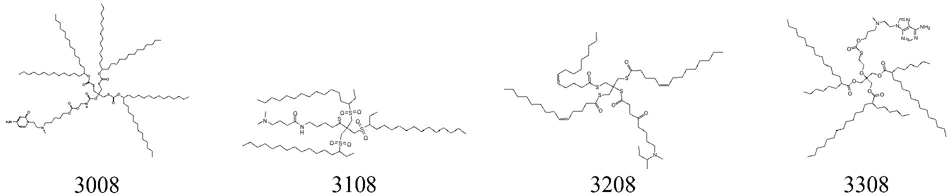

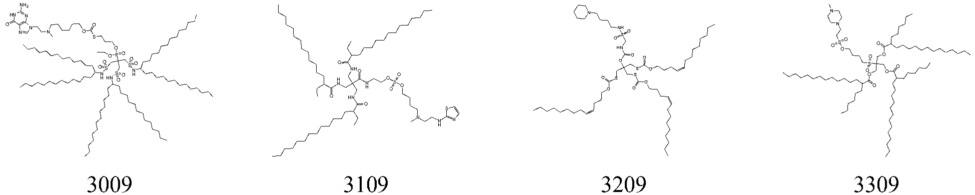

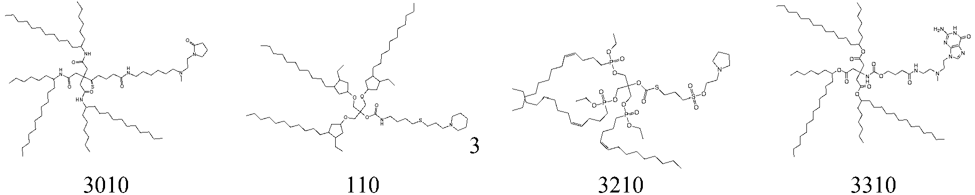

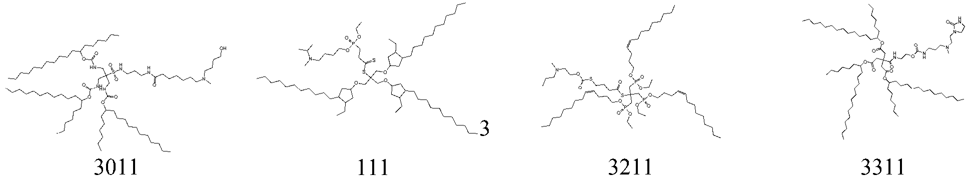

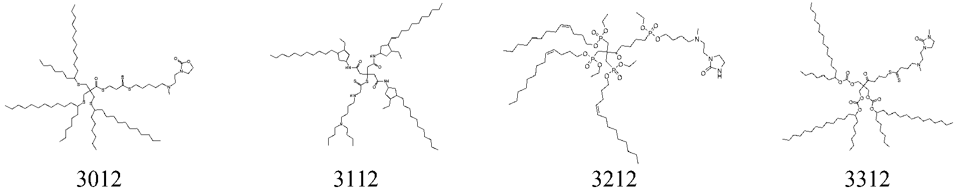

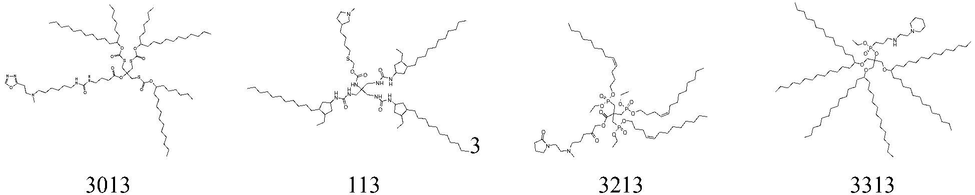

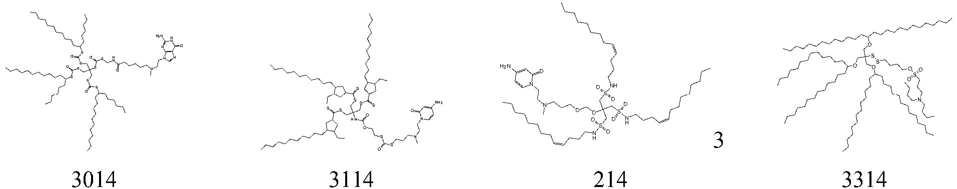

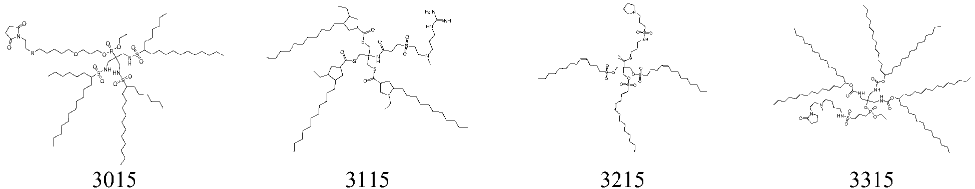

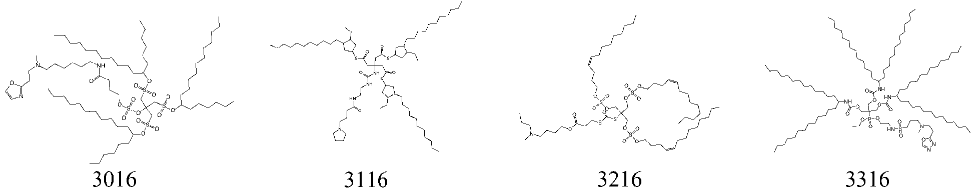

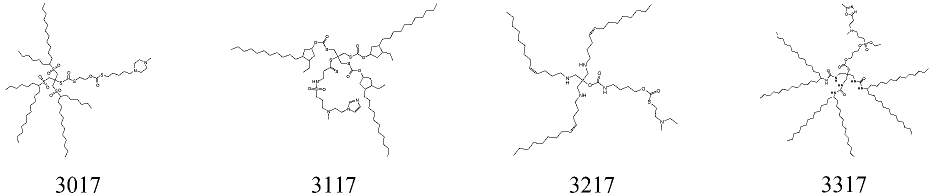

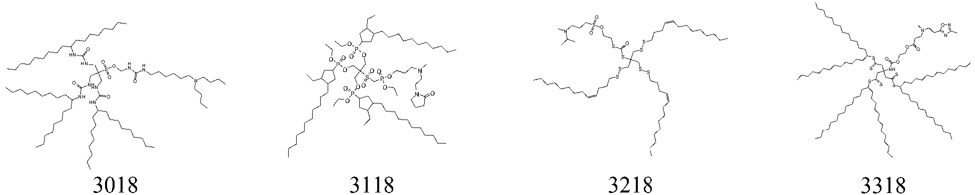

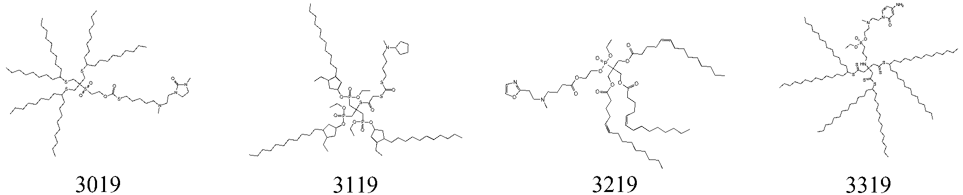

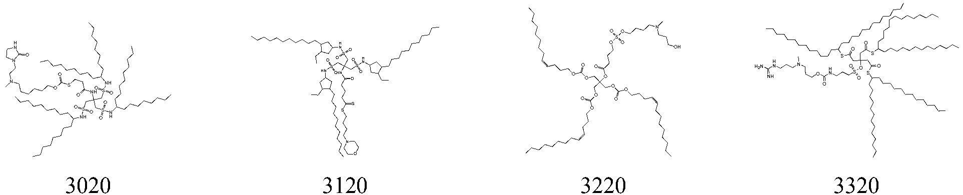

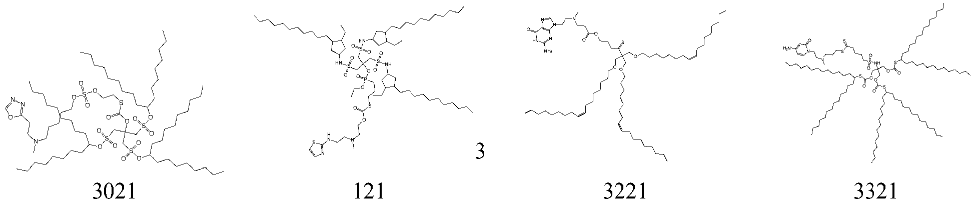

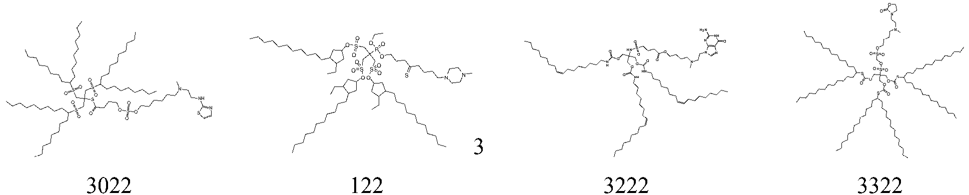

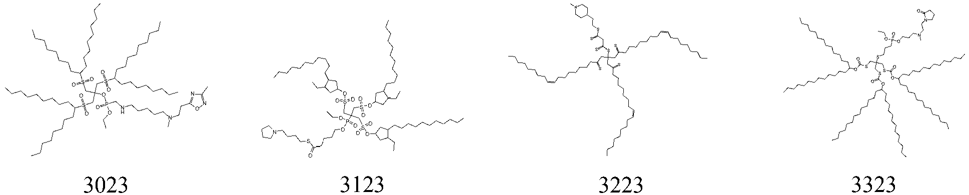

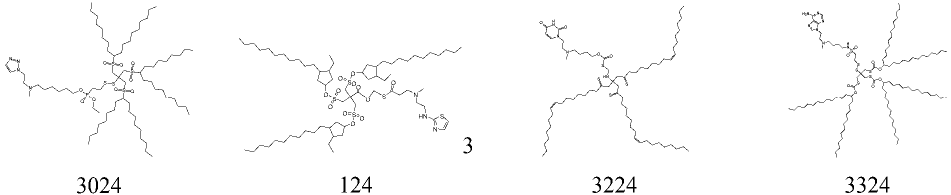

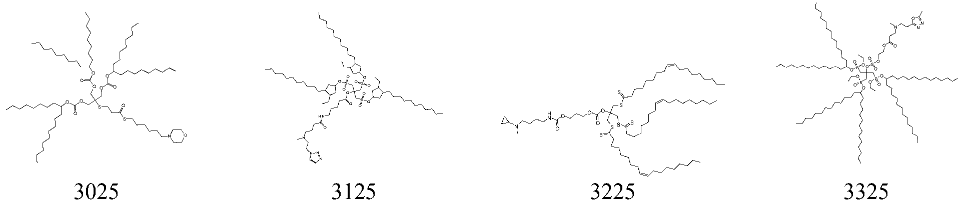

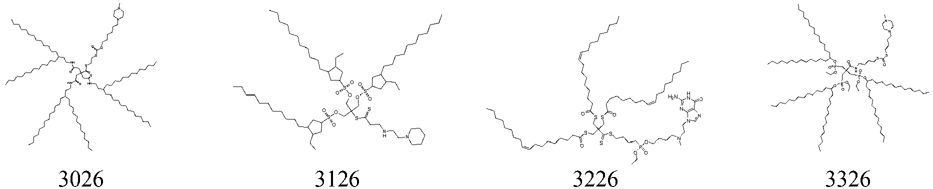

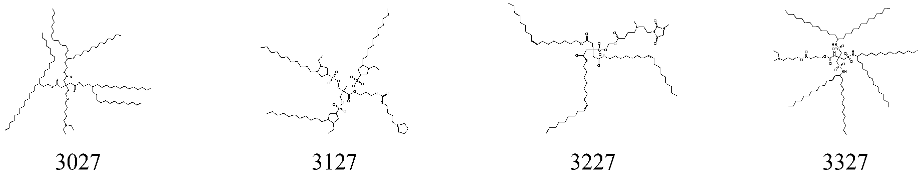

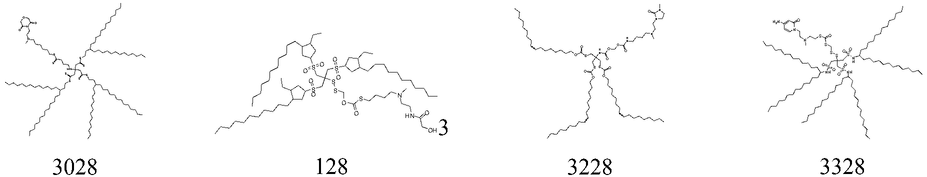

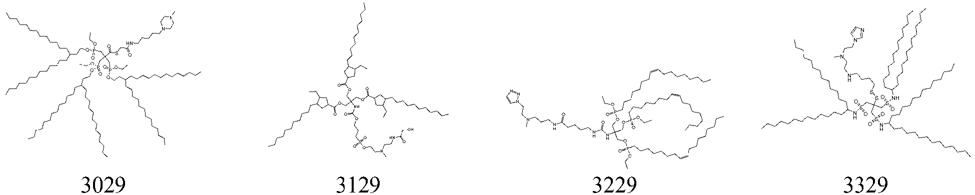

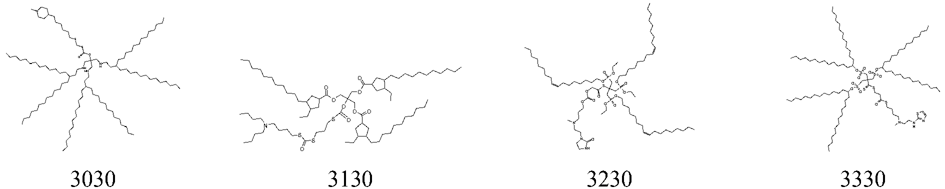

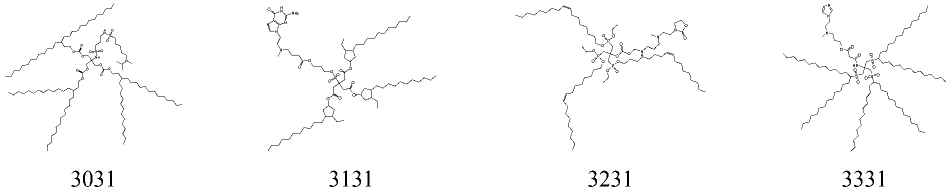

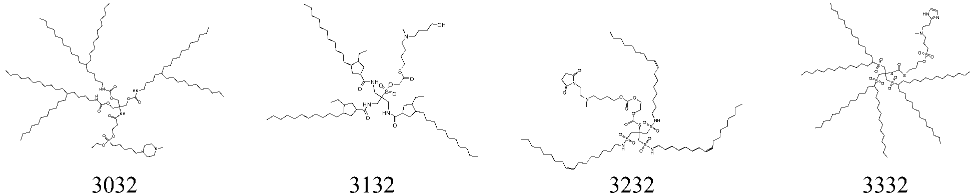

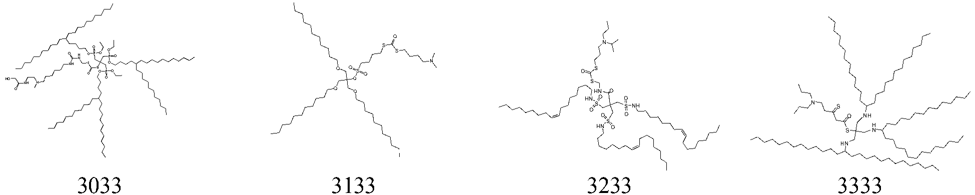

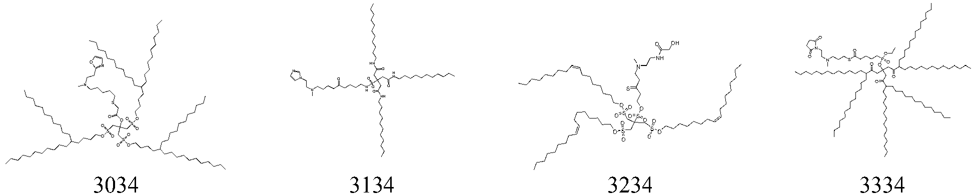

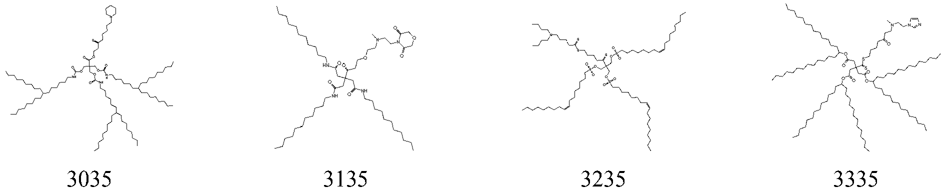

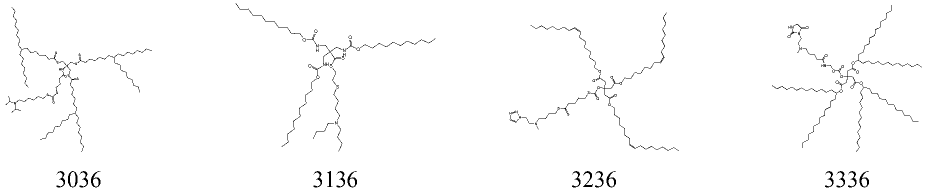

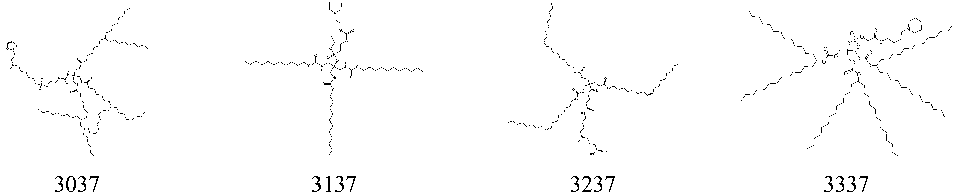

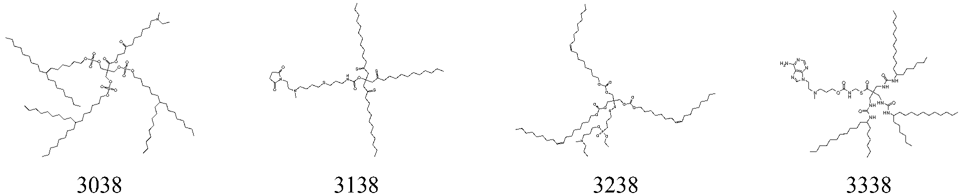

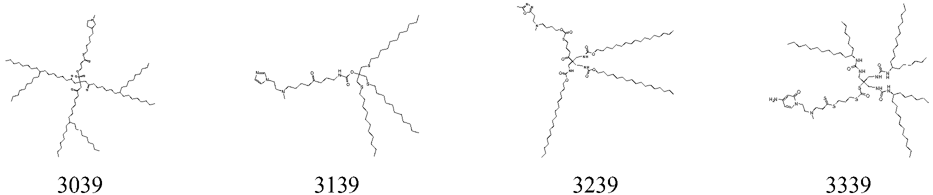

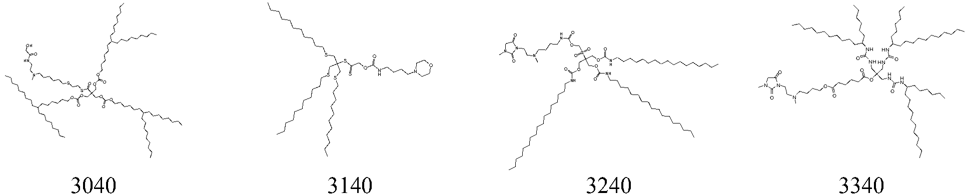

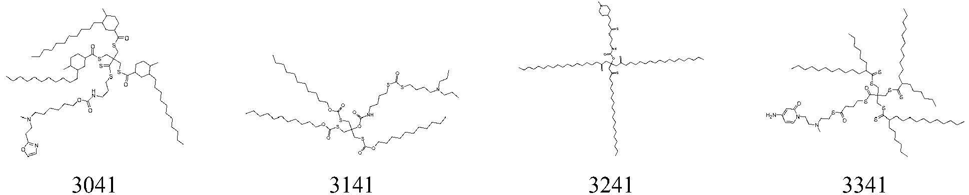

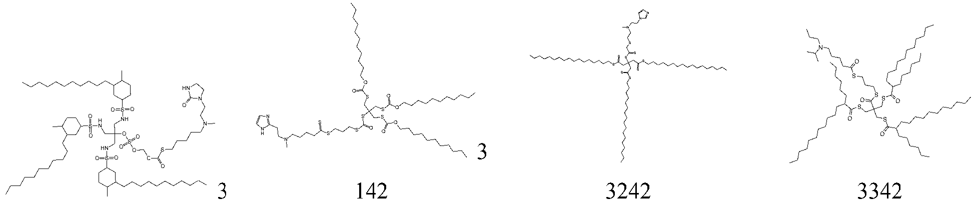

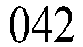

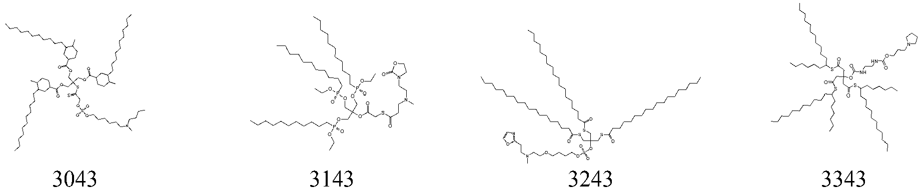

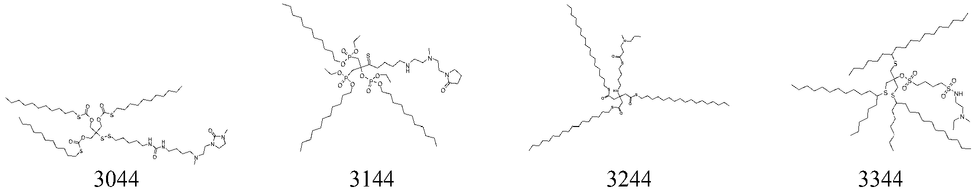

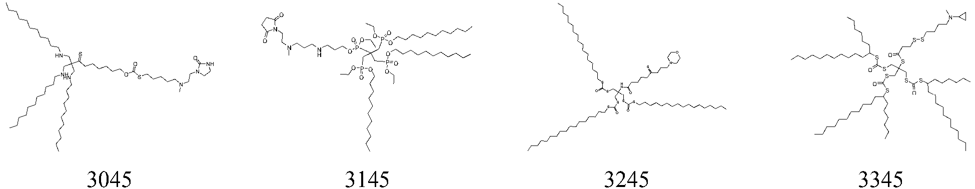

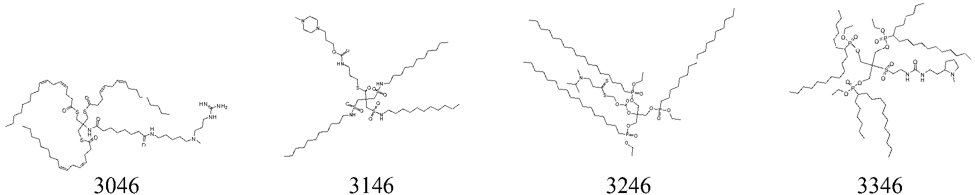

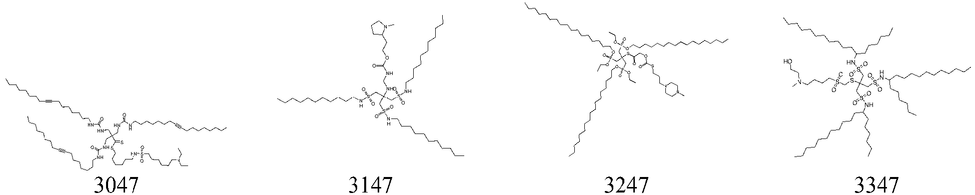

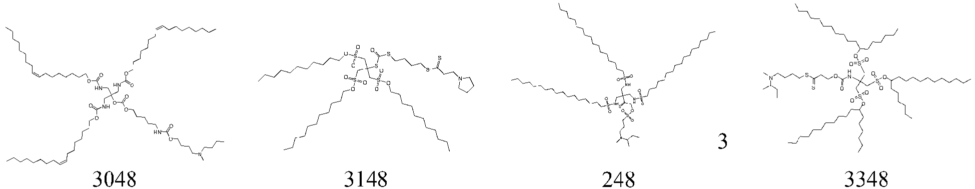

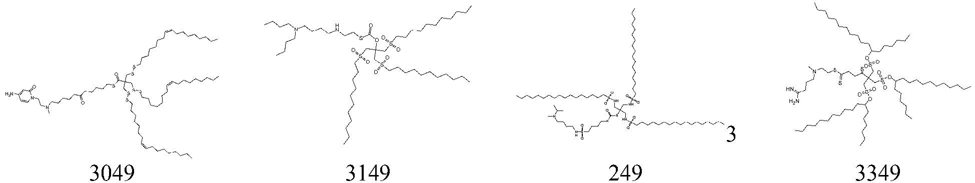

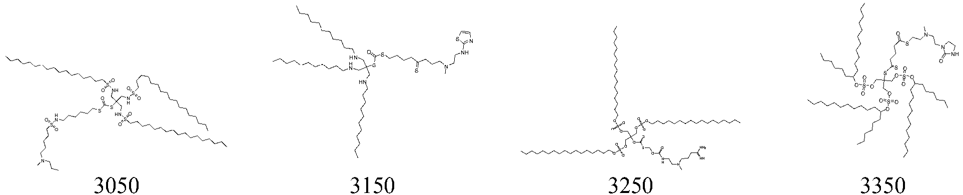

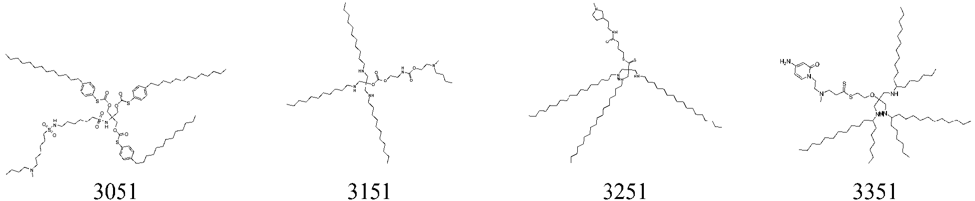

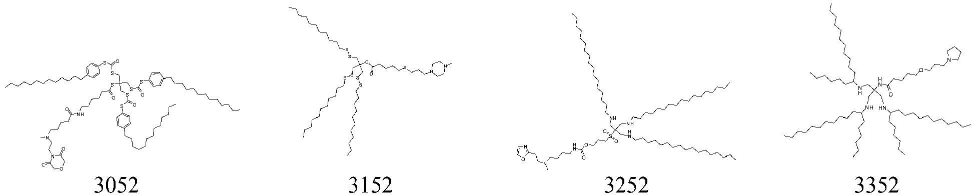

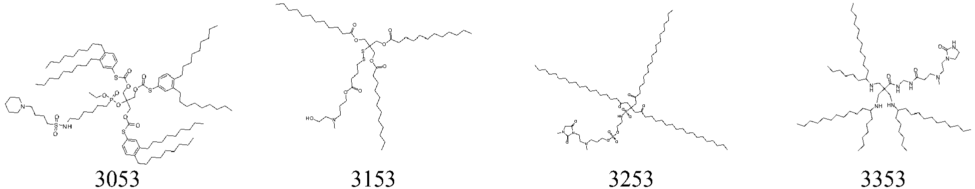

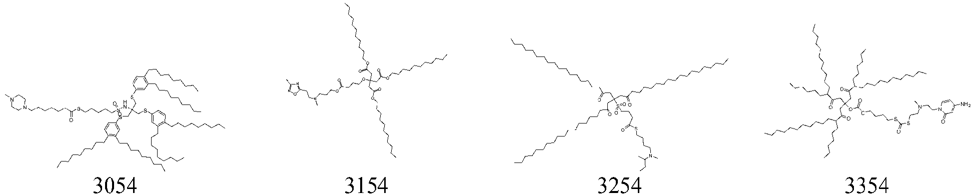

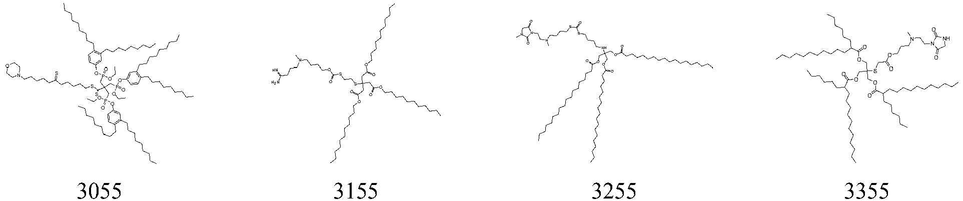

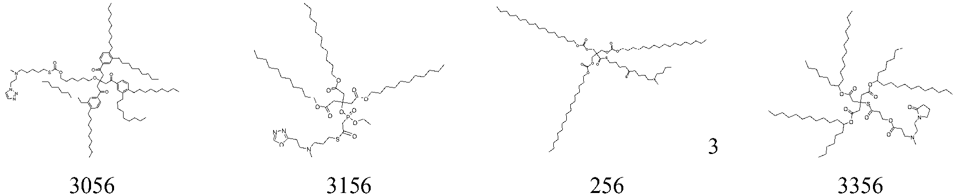

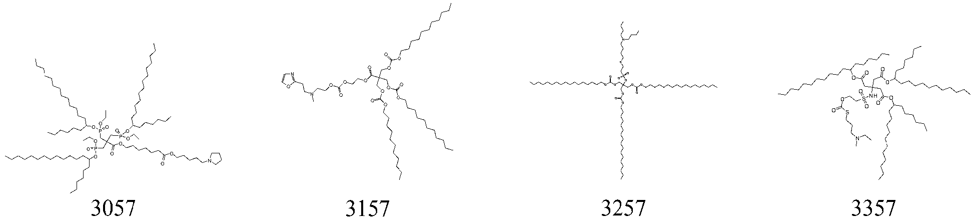

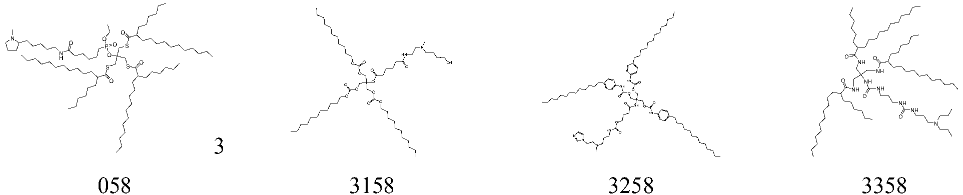

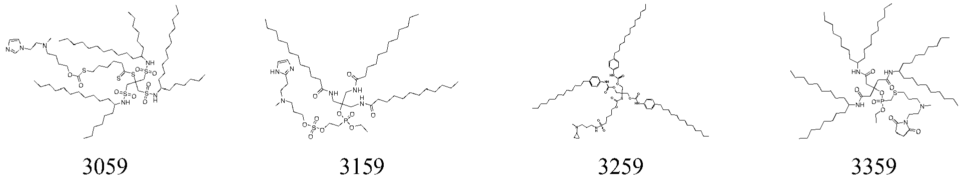

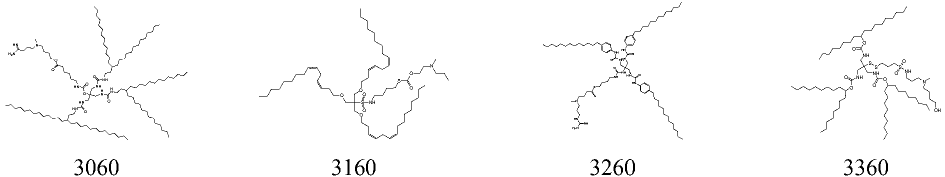

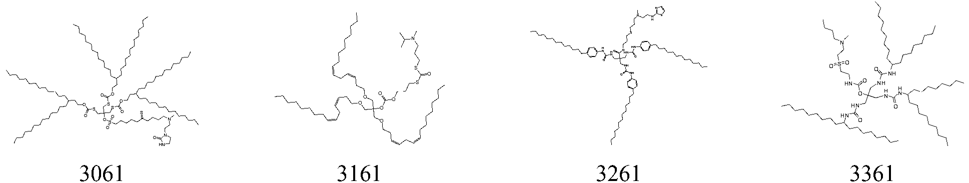

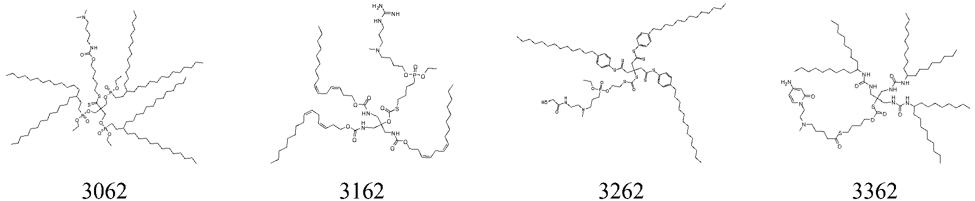

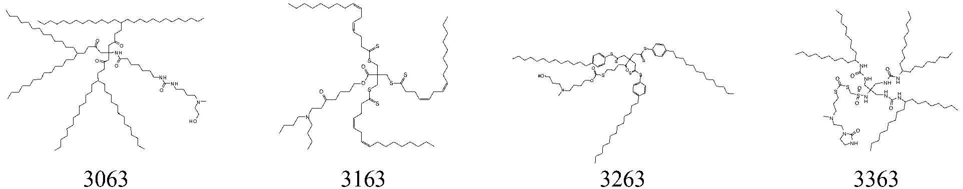

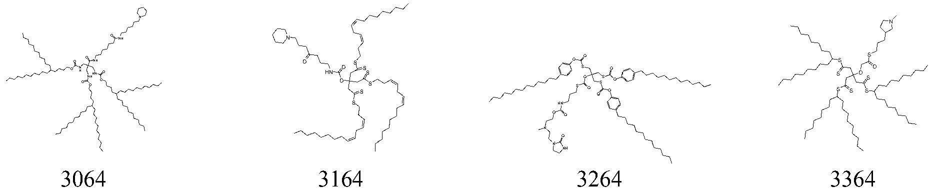

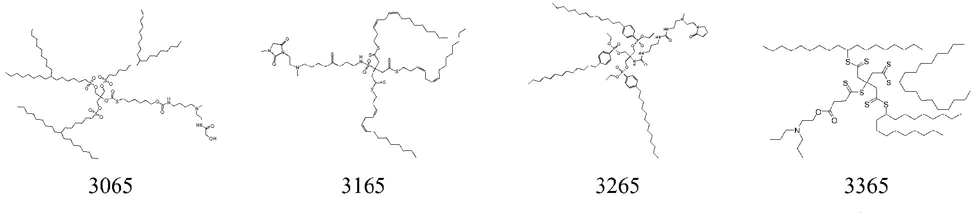

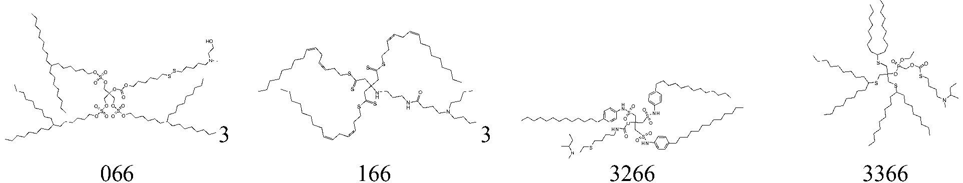

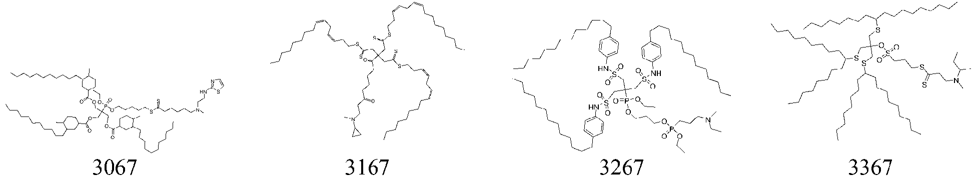

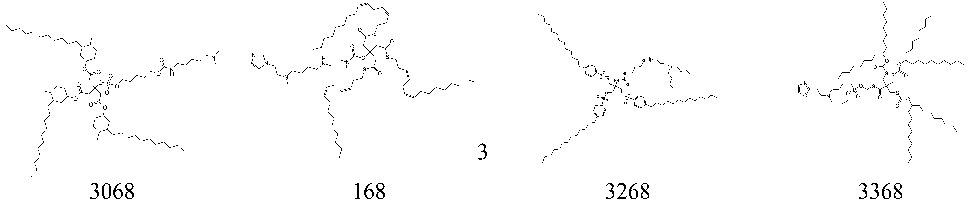

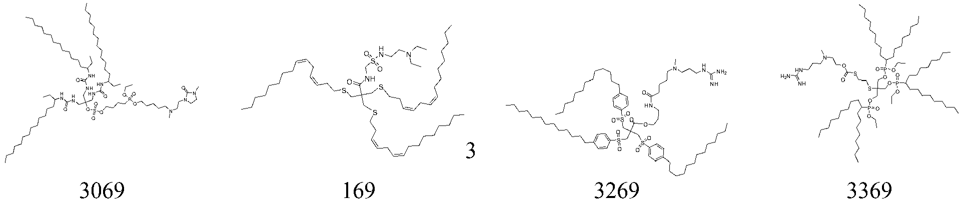

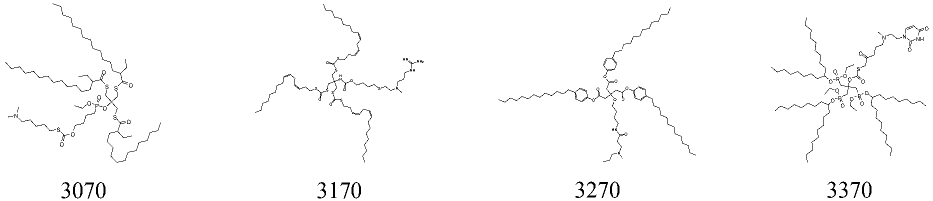

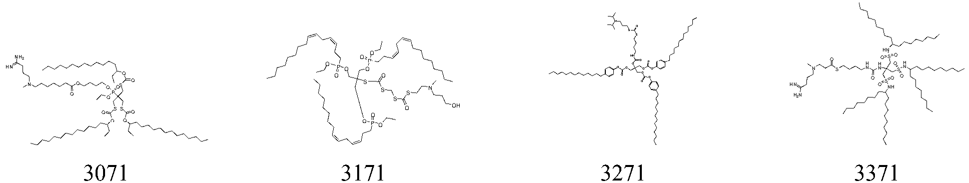

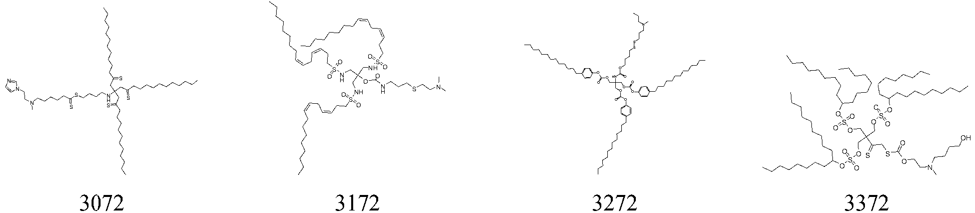

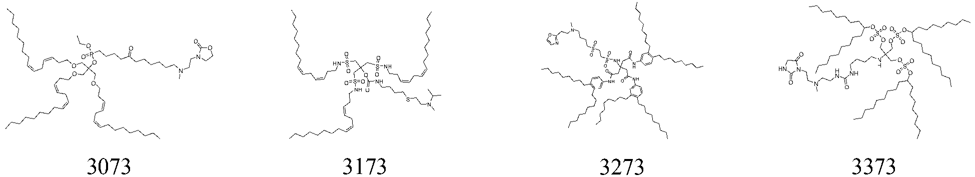

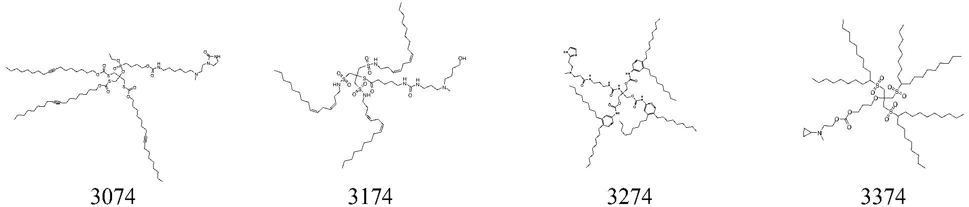

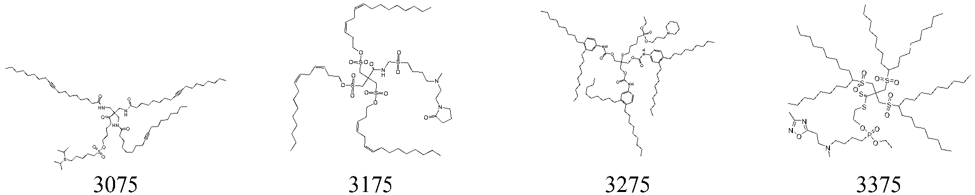

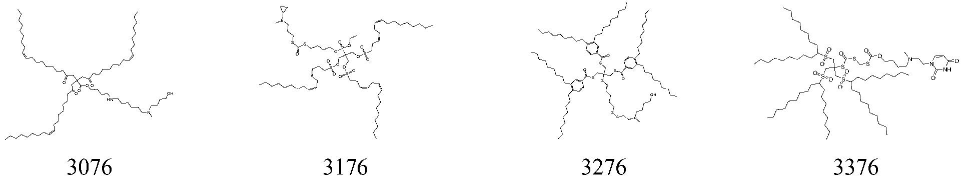

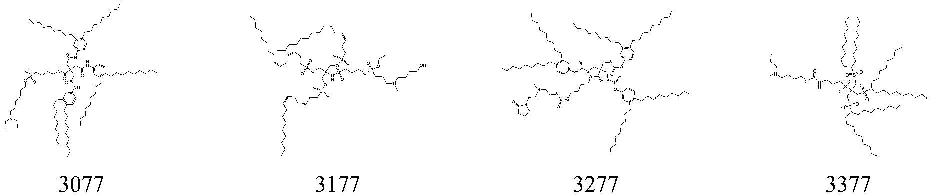

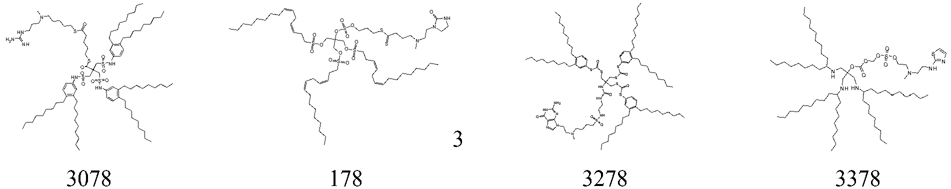

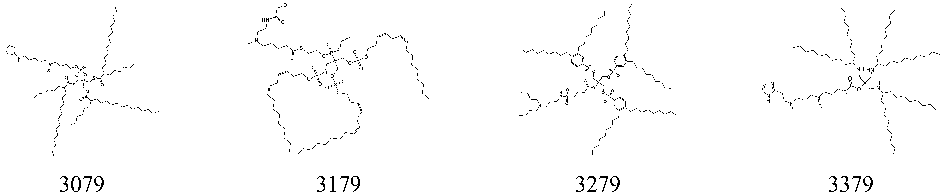

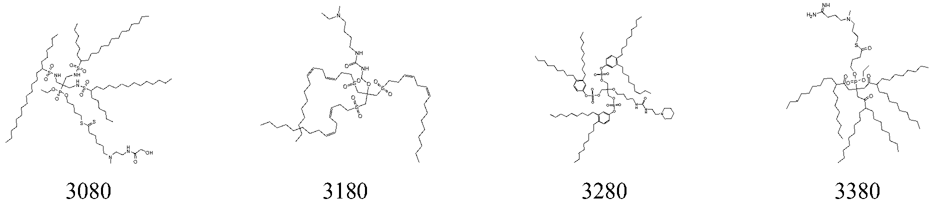

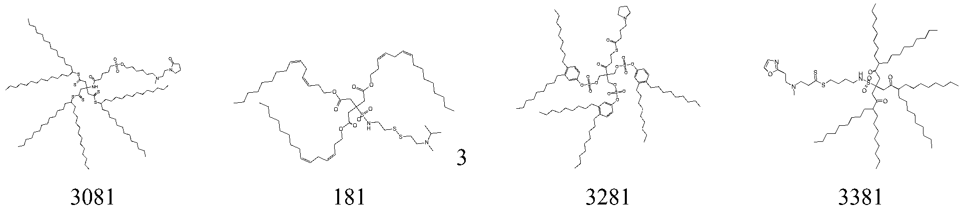

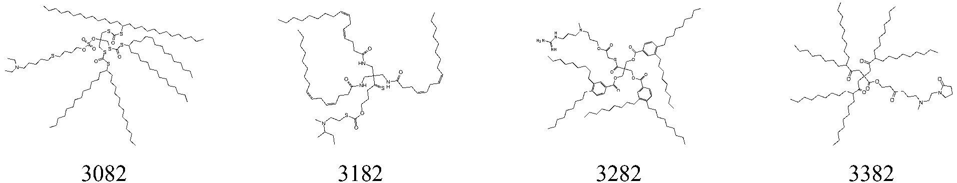

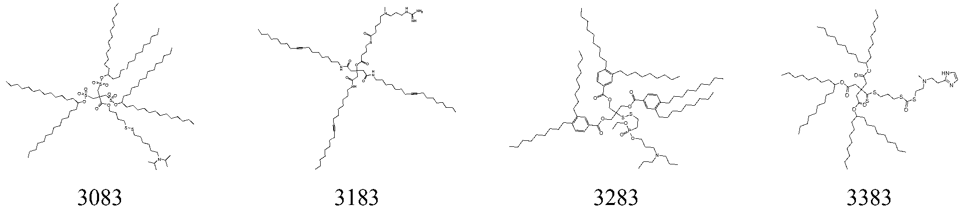

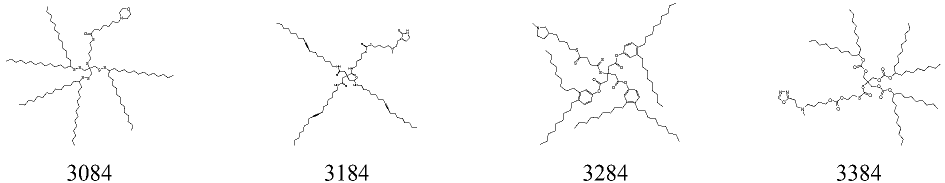

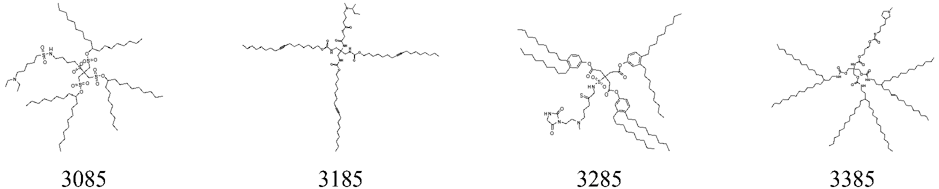

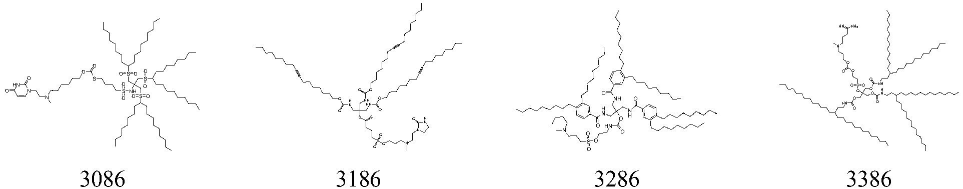

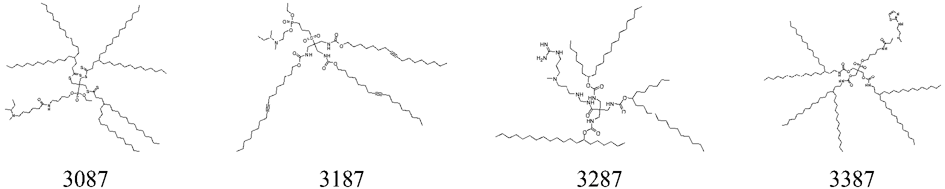

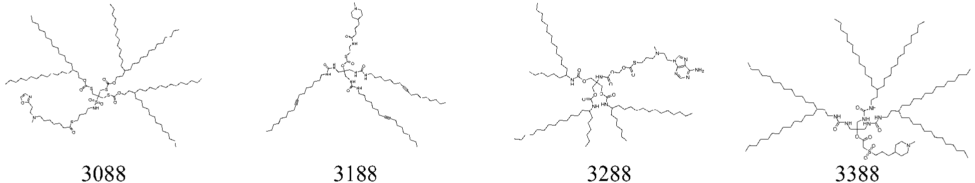

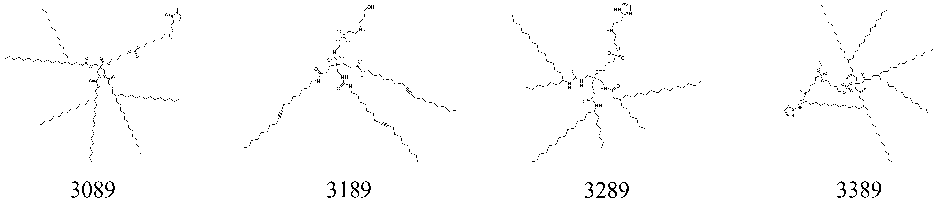

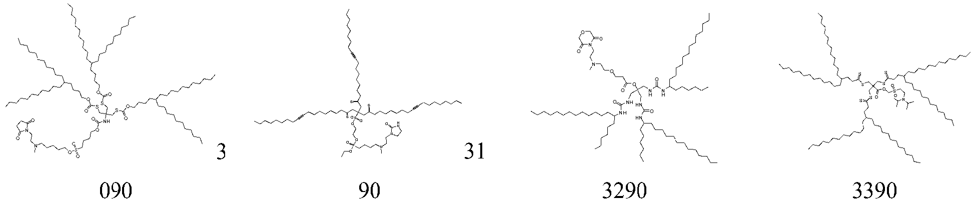

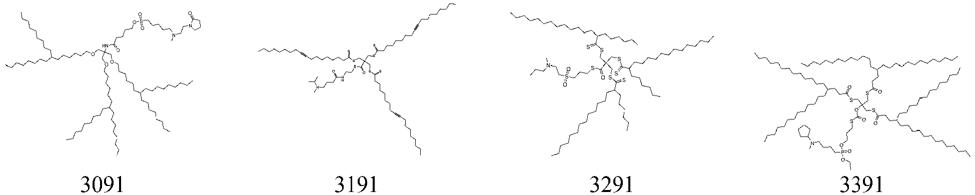

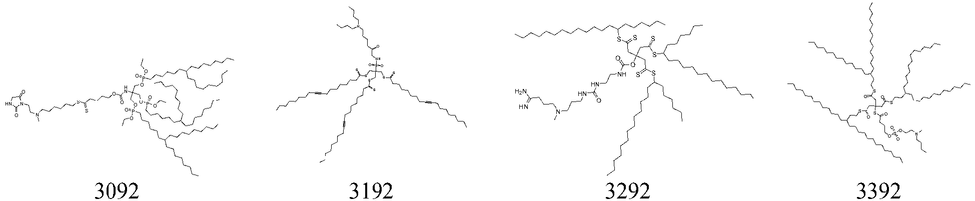

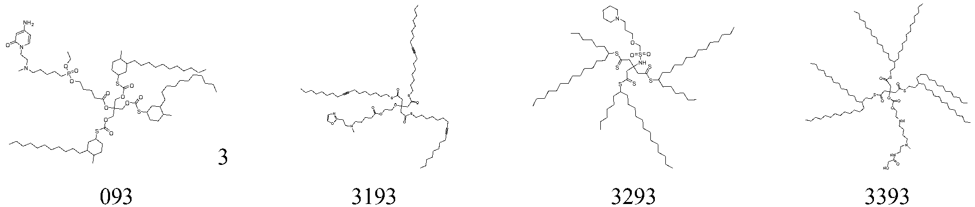

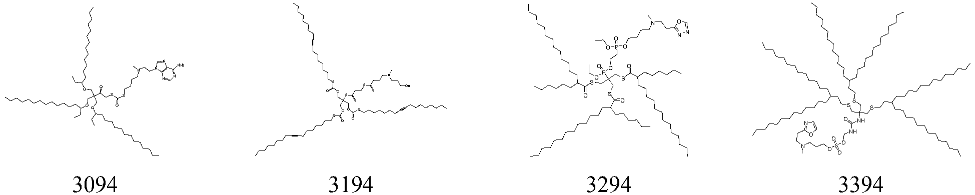

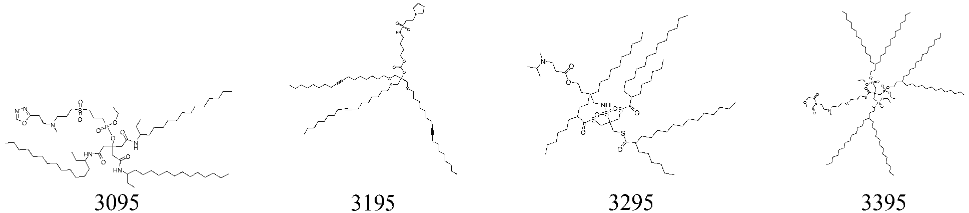

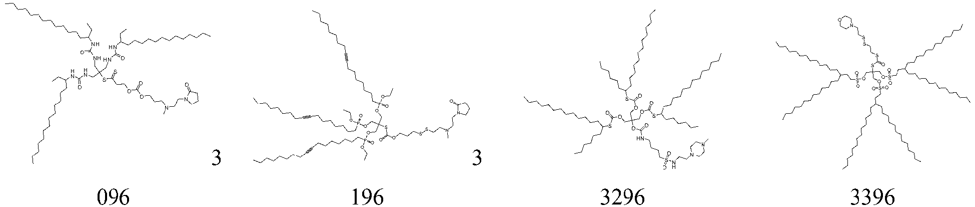

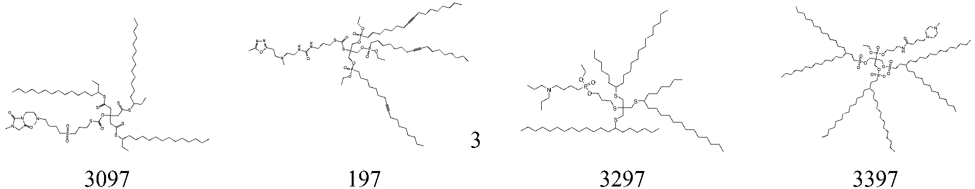

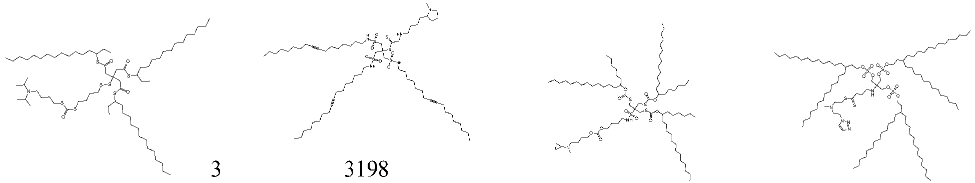

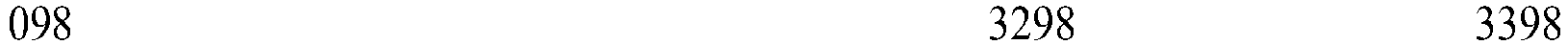

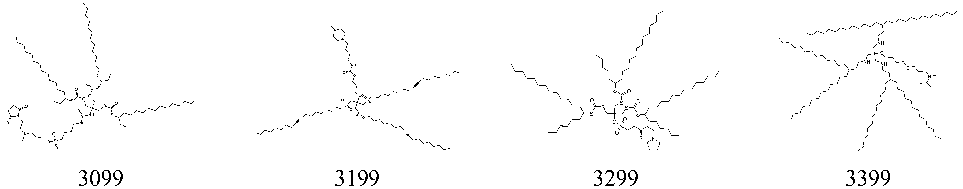

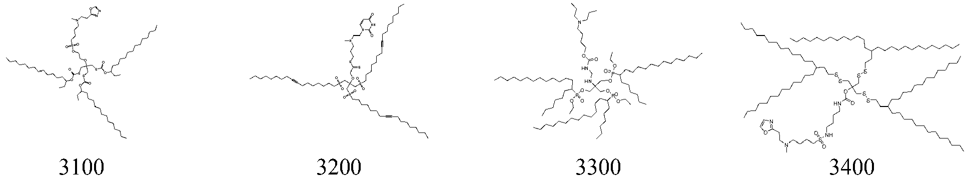

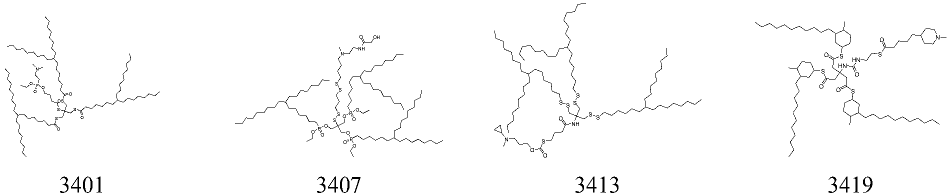

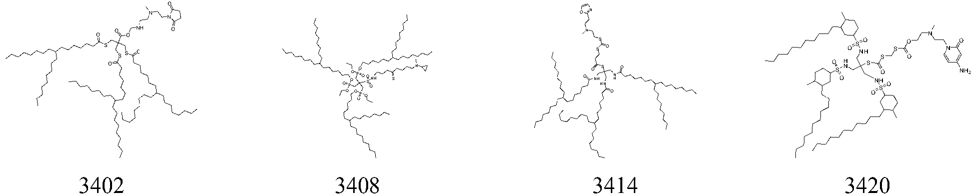

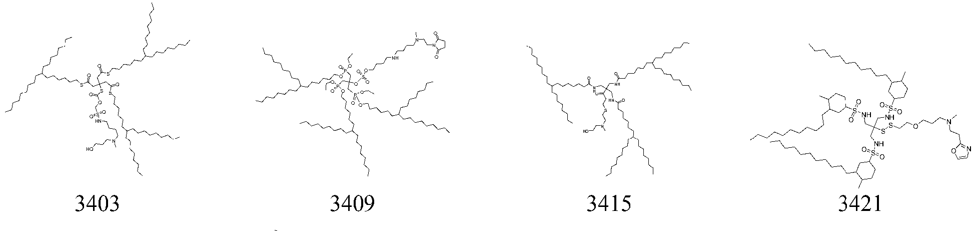

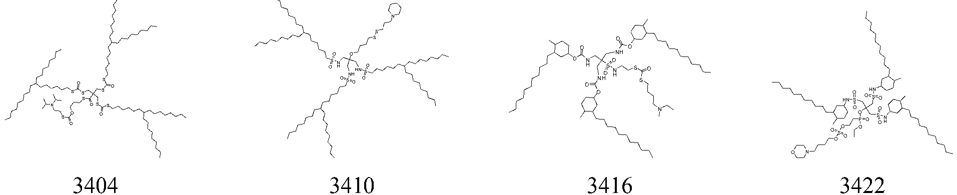

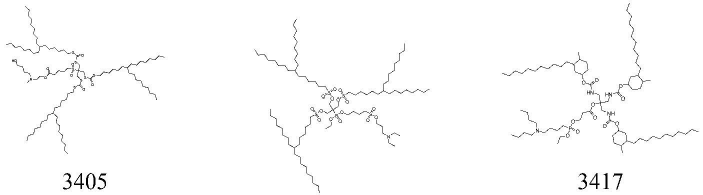

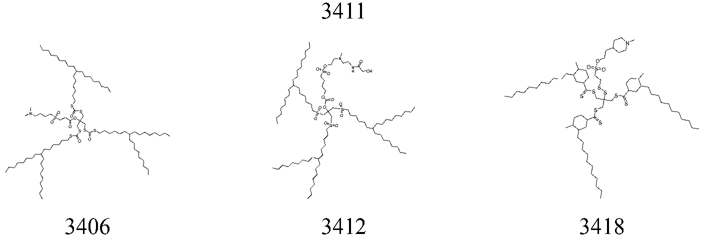

In a preferred embodiment, the preparation method of the first ionizable lipid comprises a step of reacting a compound of formula (II) with a compound of formula (III): 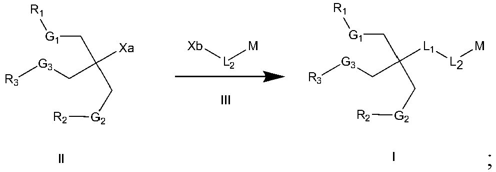 wherein Xa and Xb are groups containing leaving groups or nucleophilic groups, and L₁ is formed by a nucleophilic reaction or a condensation reaction between Xa and Xb.

In a preferred embodiment, the preparation method of the first ionizable lipid comprises a step of reacting a compound of formula (IV) with a compound of formula (V): 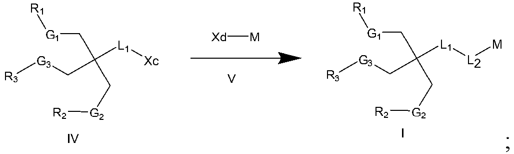 wherein Xc and Xd are groups containing leaving groups or nucleophilic groups, and L₂ is formed by a nucleophilic reaction or a condensation reaction between Xc and Xd.

In a preferred embodiment, the preparation method of the first ionizable lipid comprises a step of reacting a compound of formula (VI): 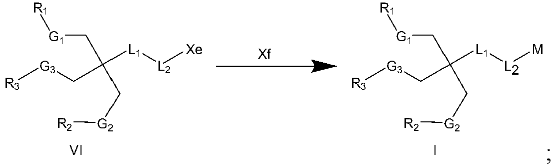 wherein Xe is a group containing leaving groups or nucleophilic groups, Xf is a compound containing leaving groups or nucleophilic groups, and M is formed by a nucleophilic reaction or a condensation reaction between Xe and Xf.

In a preferred embodiment, the preparation method of the first ionizable lipid comprises a step of sequentially reacting a compound of formula (VII) with a compound of formula (VIII), a compound of formula (IX) and a compound of formula (X): 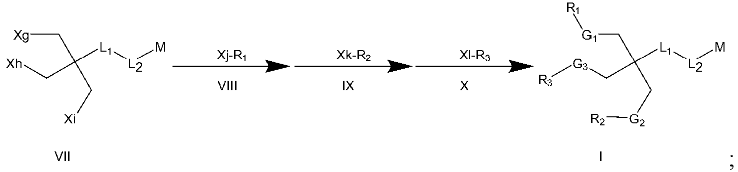 wherein Xg, Xh, Xi, Xj, Xk and X₁ are groups containing leaving groups or nucleophilic groups, and G₁ is formed by a nucleophilic reaction or a condensation reaction between Xg and Xj, G₂ is formed by a nucleophilic reaction or a condensation reaction between Xh and Xk, G₃ is formed by a nucleophilic reaction or a condensation reaction between Xi and Xl.

In a preferred embodiment, the preparation method of the first ionizable lipid comprises a step of reacting a compound of formula (XI) with a compound of formula (XII): 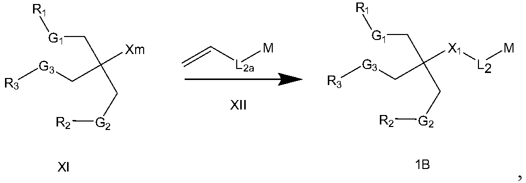 or a step of reacting a compound of formula (XIII) with a compound of formula (XII): 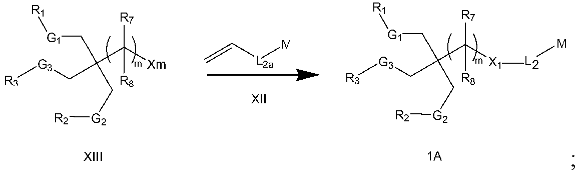 wherein Xm is a group containing nucleophilic groups, and X₁-L₂ is formed by an addition reaction between Xm and C=C-L₂ₐ of the compound of formula (XII).

In a preferred embodiment, R₁, R₂ and R₃ are each independently the following groups: 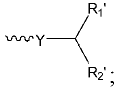
wherein Y, R₁' and R₂' have the same meanings as defined above;
wherein the method further comprises a step of forming the tail chains R₁, R₂ and R₃: 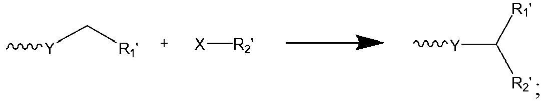
wherein X is a leaving group.

In a preferred embodiment, the second ionizable lipid is a compound of formula (2), or a salt, stereoisomer or tautomer thereof: 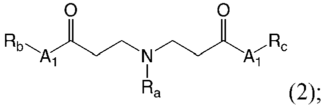
wherein A₁ is NH or O;
Ra is selected from C₆-C₂₄ alkyl, C₆-C₂₄ alkenyl, C₆-C₂₄ cycloalkyl, C₆-C₂₄ alcohol and short-chain C₆-C₂₄ polyoxyethylene; the C₆-C₂₄ alkyl, C₆-C₂₄ alkenyl, C₆-C₂₄ cycloalkyl, C₆-C₂₄ alcohol and short-chain C₆-C₂₄ polyoxyethylene each have a linear or branched structure; R_{b} and R_{c} are each independently selected from C₁-C₁₂ alkyl, C₂-C₁₂ alkenyl, C₂-C₁₂ alkynyl, C₃-C₁₂ cycloalkyl, C₆-C₁₂ aryl, C₁-C₁₂ alkyl alcohol, C₁-C₁₂ heterocyclyl and alkylamine;
the alkylamine is 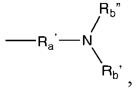 wherein Ra' is C₁-C₁₂ alkyl, and R_{b}' and R_{b}" are each independently selected from H, C₁-C₆ alkylamine, 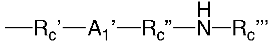 or 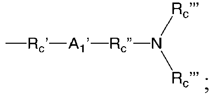 Rc" is selected from C₁-C₆ alkyl unsubstituted or substituted with amino groups, and Rc‴ is H, 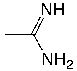 or -R_{c}'-A₁'-R_{c}"-NH₂;
provided that, when A₁' is -CO-NH-, -NH-CO- or -CO-O-, R_{c}' is C₁-C₆ alkyl; when A₁' is -CO-, R_{c}' is absent.

In a preferred embodiment, R_{b} and R_{c} are each independently selected from C₁-C₁₂ alkyl alcohol and alkylamine.

In a preferred embodiment, provided that: when Rₐ is C₆-C₂₄ alkenyl, A₁ is NH, and R_{b} and R_{c} are each independently selected from C₁-C₁₂ alkyl alcohol;
when Rₐ is a branched C₆-C₂₄ alkyl, A₁ is NH, and R_{b} and R_{c} are each independently selected from C₁-C₁₂ alkyl alcohol and alkylamine;
when Rₐ is a linear C₆-C₂₄ alkyl, A₁ is NH or O, and R_{b} and R_{c} are each independently selected from alkylamine;
when Rₐ is a linear C₆-C₂₄ alkyl, A₁ is O, and R_{b} and R_{c} are each independently selected from C₁-C₁₂ alkyl alcohol;
when Rₐ is a short-chain C₆-C₂₄ polyoxyethylene, A₁ is NH or O, and R_{b} and R_{c} are each independently selected from alkylamine;
or when Rₐ is a C₆-C₂₄ alkyl alcohol, A₁ is NH or O, and R_{b} and R_{c} are each independently selected from alkylamine.

In a preferred embodiment, Rₐ is selected from the following compound structures:

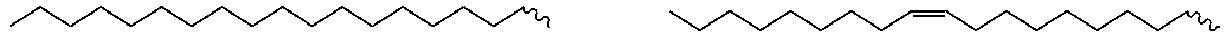

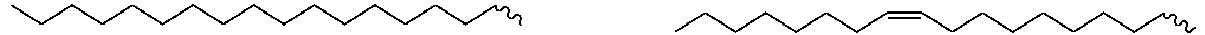

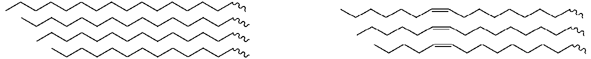

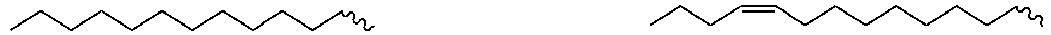

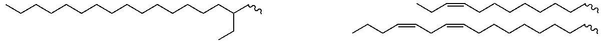

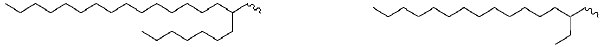

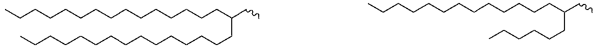

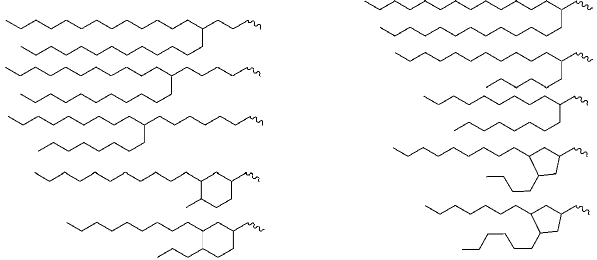

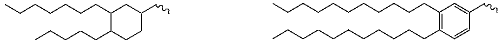

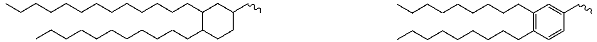

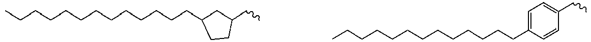

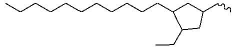

In a preferred embodiment, R_{b} and R_{c} are selected from the following compound structures:

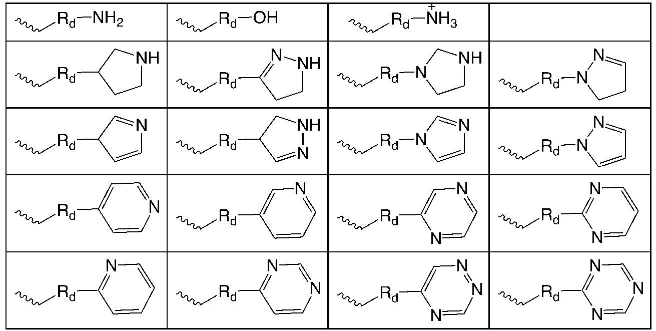

R_{d} is selected from C₁-C₆ alkane or cycloalkane.

In a preferred embodiment, the compound of formula (2) is selected from at least one of the following compounds:

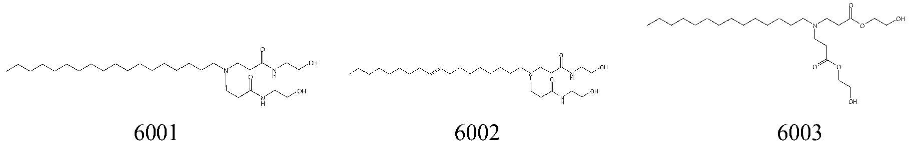

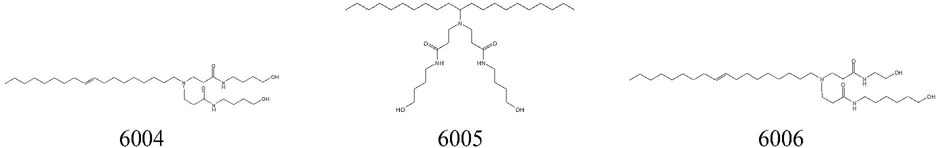

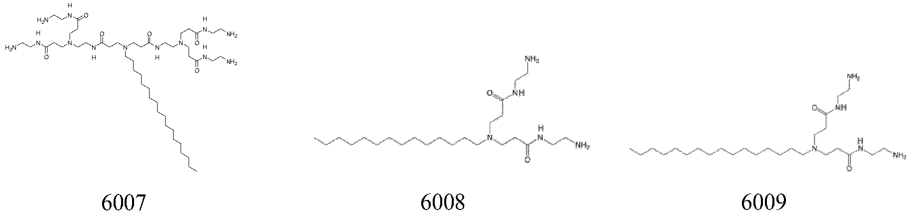

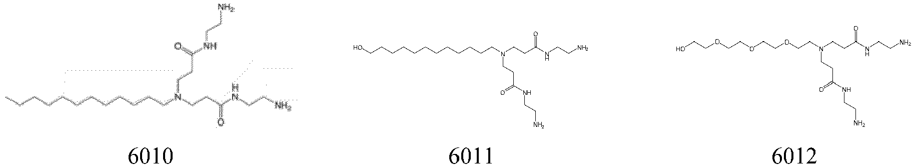

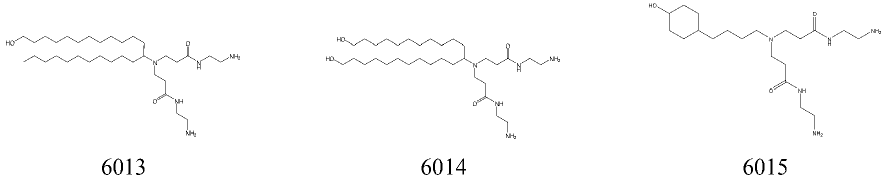

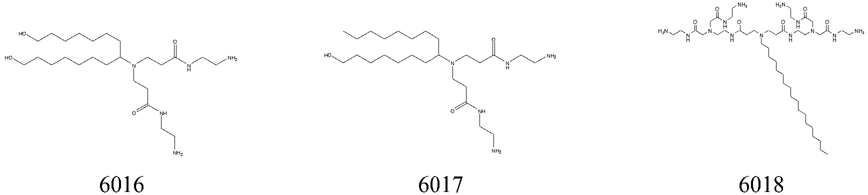

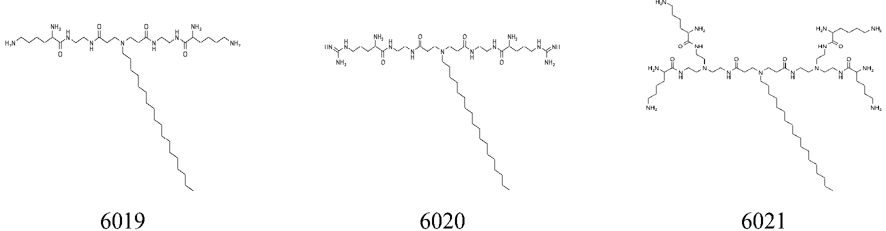

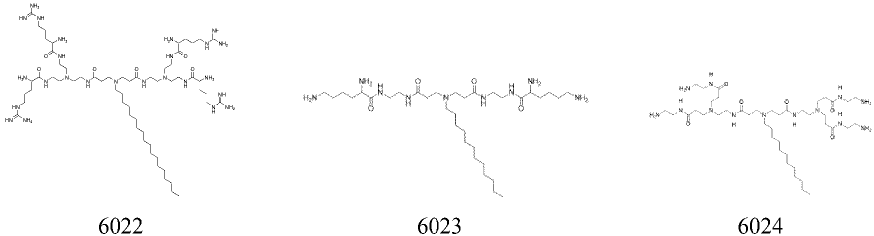

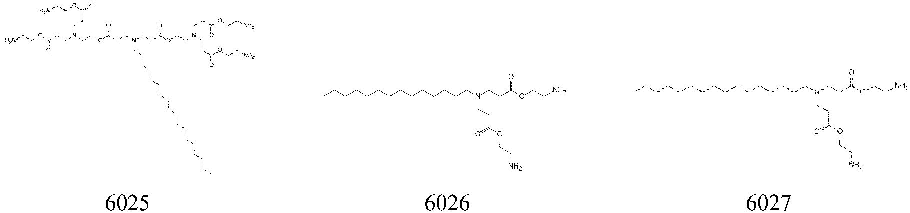

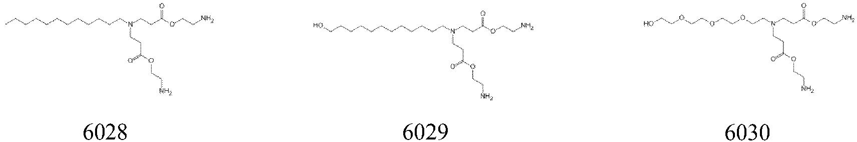

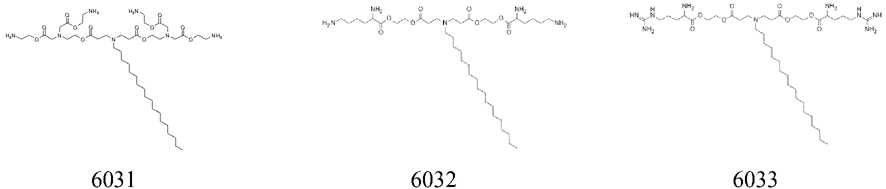

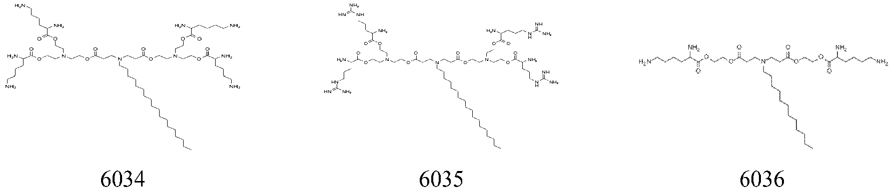

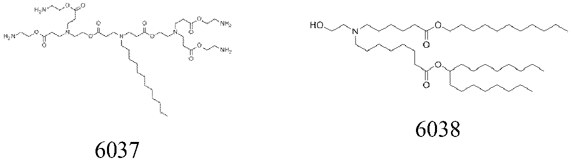

In a preferred embodiment, the preparation method of the second ionizable lipid comprises the following reaction steps:
reacting Ra"-NH₂ ① with an α,β-unsaturated carbonyl compound ② to form the ionizable lipid compound③: 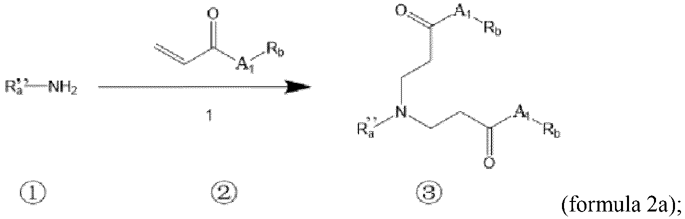
wherein, Ra"-NH₂ is Rₐ-NH₂ or 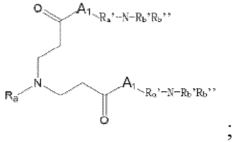
R_{b}' and R_{b}" are both H, or both C₁-C₆ alkylamine, or both -R_{c}'-A₁'-R_{c}"-NH₂.

In a preferred embodiment, the preparation method of the second ionizable lipid comprises:
1) reacting Rₐ"-NH₂① with an α,β-unsaturated carbonyl compound④ to form compound⑤;
2) reacting compound⑤ with a nucleophilic reagent⑥ to form ionizable lipid compound③; 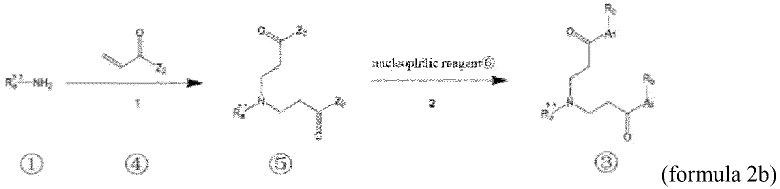
wherein the nucleophilic reagent⑥ is R_{b}-NH₂ or R_{b}-OH; Ra"-NH₂ is selected from Rₐ-NH₂ or 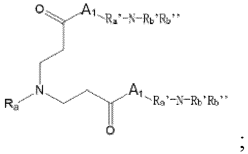 R_{b}' and R_{b}" are both H, or both C₁-C₆ alkylamine, or both -R_{c}'-A₁'-R_{c}"-NH₂ ; Z₂ is a leaving group, and Z₂ reacts with NH₂ to form A₁.

In a preferred embodiment, the preparation method of the second ionizable lipid comprises:
1) reacting Rₐ"-NH₂① with an α,β-unsaturated carbonyl compound② to form compound⑦;
2) reacting compound⑦ with an α,β-unsaturated carbonyl compound⑧ to form compound⑨;
3) reacting compound⑨ with a nucleophilic reagent⑩ to form ionizable lipid compound ⑪; 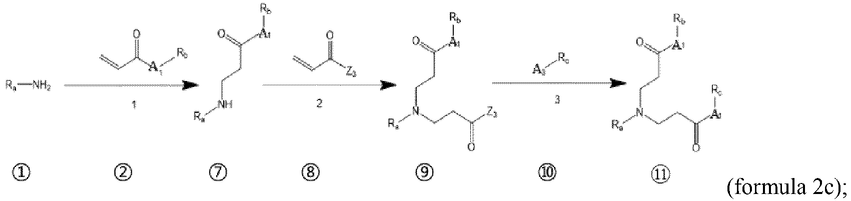
wherein Ra"-NH₂ is Rₐ-NH₂, Z₃ is a leaving group, and A₃ reacts with Z₃ to form A₁.

In a preferred embodiment, in formula 2a to formula 2c, at least one nucleophilic reagent undergoes a Michael addition reaction with the β-carbon atom of at least one α,β-unsaturated carbonyl compound to form a double-branched ionizable lipid compound containing carbon-carbon bonds, carbon-oxygen bonds, carbon-nitrogen bonds, carbon-sulfur bonds or carbon-selenium bonds.

In a preferred embodiment, formula 2a to formula 2b further include a step of carrying out iterative reactions according to step 1 in formula 2a or steps 1 to 2 in formula 2b, using the compound of formula (2) bearing terminal amino groups obtained from the reactions of formula 2a to formula 2b as a starting material;
the compound of formula (2) bearing terminal amino groups is 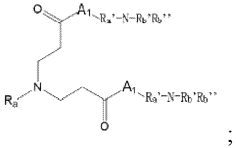
wherein R_{b}' and R_{b}" are both H, or both C₁-C₆ alkylamine, or both -R_{c}'-A₁'-R_{c}"-NH₂.

In a preferred embodiment, formula 2a to formula 2c further comprise a step of reacting with 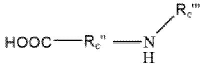 by using the compound of formula (2) bearing terminal amino groups obtained from the reactions of formula 2a to formula 2c as a starting material;
the compound of formula (2) bearing terminal amino groups is 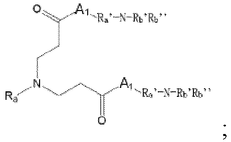
wherein R_{b}' and R_{b}" are both H, or both C₁-C₆ alkylamine, or both -R_{c}'-A₁'-R_{c}"-NH₂.

The leaving group described above refers to a moiety that departs in a nucleophilic reaction or a condensation reaction, including but not limited to: H, OH, H₂O, halogen (e.g., F, Cl, Br and I), cyanate anions, inorganic acids (e.g., nitric acid, sulfuric acid, phosphoric acid), carboxylic acids (e.g., acetic acid, trifluoroacetic acid, benzoic acid, etc.), sulfonic acids (e.g., methanesulfonic acid, trifluoromethanesulfonic acid, benzenesulfonic acid, p-toluenesulfonic acid and p-nitrobenzenesulfonic acid, etc.), carbon dioxide (CO₂), nitrogen gas (N₂), imidazole, alkoxy groups (R-O-), amino groups (-NHR, wherein R is an alkyl or aryl group with one hydrogen removed), phenoxy groups, tertiary carbocations (e.g., tert-butyl carbocation), carbocations stabilized by unsaturated systems or heteroatoms, or various protecting groups described above.

The nucleophilic group described above refers to a molecule or ion capable of donating electron pairs to form new chemical bonds in chemical reactions. Common nucleophilic groups include: hydroxide anions (HO⁻), ammonia (NH₃), hydroxylamine (NH₂OH), hydrazine (NH₂-NH₂), substituted hydrazines, nucleophilic halogen (e.g., Cl⁻, Br⁻ or I⁻), hydride anions (H⁻), azide anions (N₃⁻), cyanide anions (CN⁻), alcohols or alkoxide anions (e.g., alcohols with the hydroxyl hydrogen removed), amino groups (including primary amines, secondary amines and tertiary amines) or amide anions, carbanions (e.g., carbanions in organometallic reagents such as Grignard reagents, organolithium reagents, Gilman reagents, etc.), mercapto groups or thiolate anions, thioethers, enols or enolate anions, vinyl ethers, enamines, carboxylic acids or carboxylate anions, alkyl or aryl phosphines (e.g., triphenylphosphine), aromatic heterocycles bearing lone electron pairs (e.g., pyridine), and the like.

In a preferred embodiment, starting materials used during the reaction further contain protecting groups, and the reaction steps include steps of protection and/or deprotection.

In a preferred embodiment, the blank lipid nanoparticle-based in-vivo delivery method further comprises 0-60 mol% of other ionizable lipids, wherein the other ionizable lipids are selected from at least one of the following compounds:

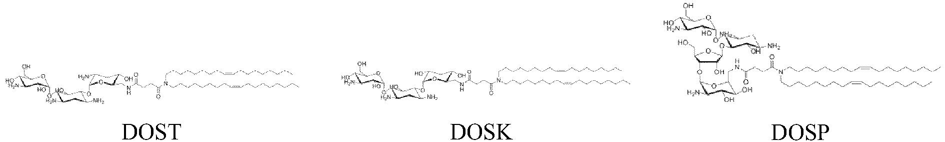

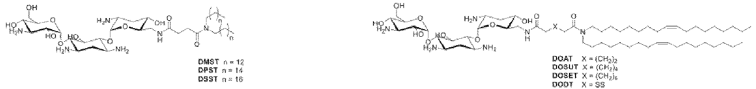

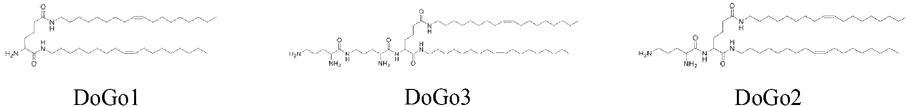

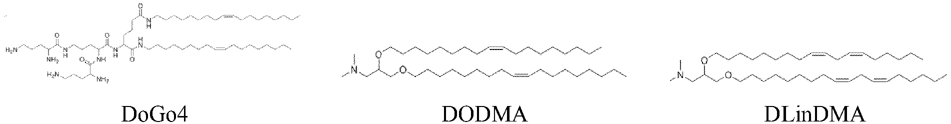

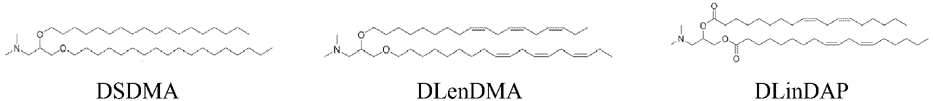

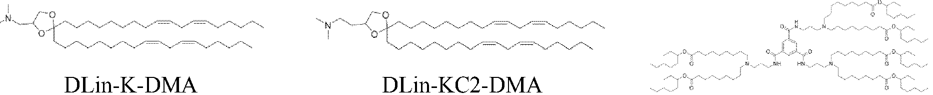

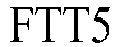

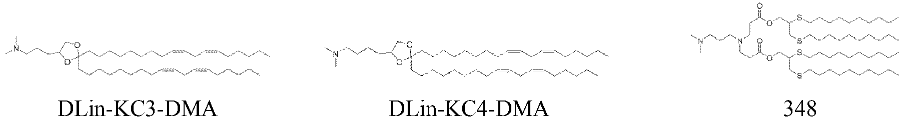

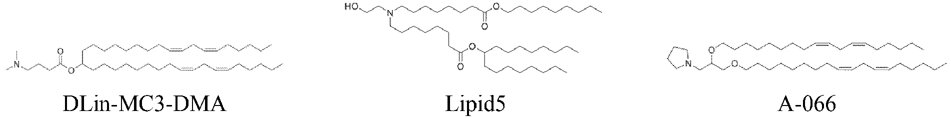

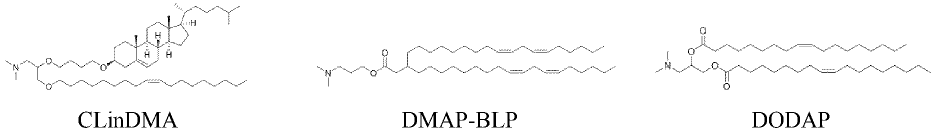

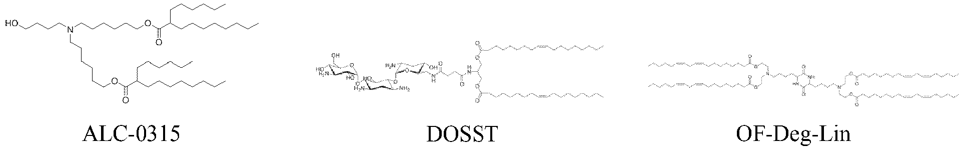

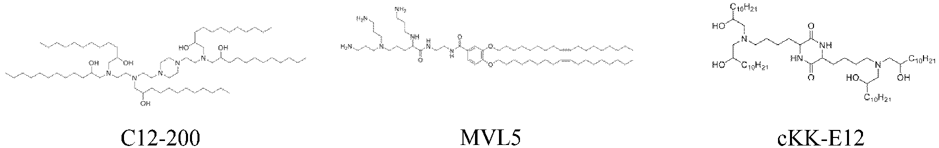

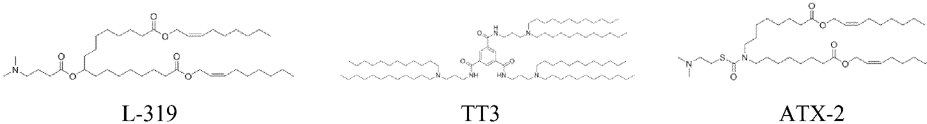

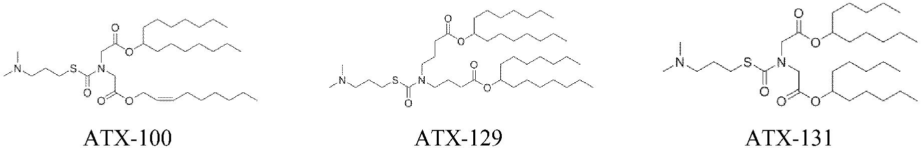

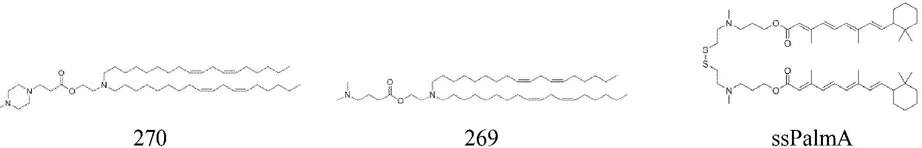

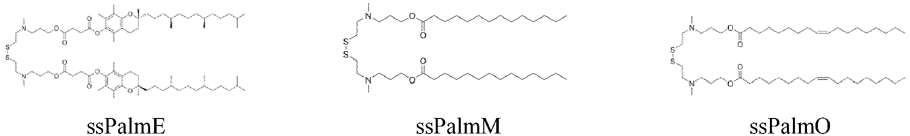

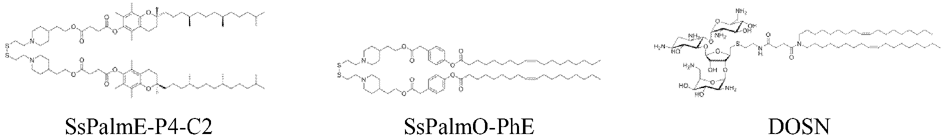

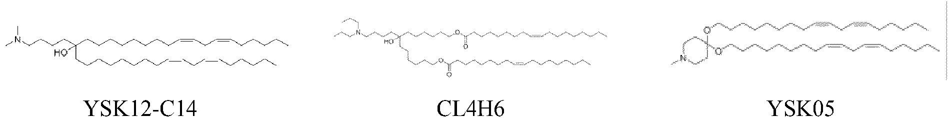

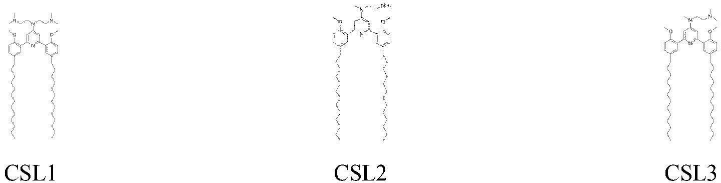

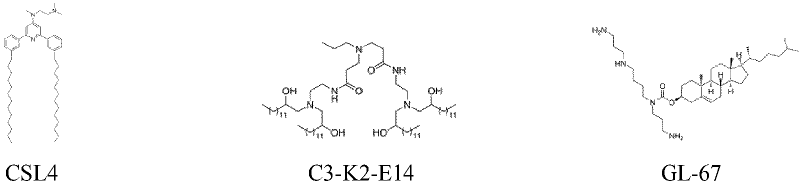

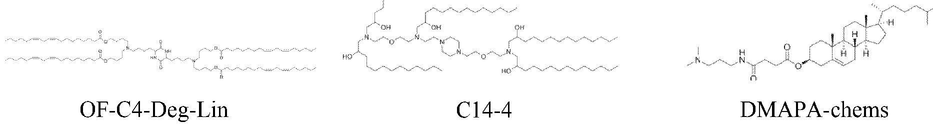

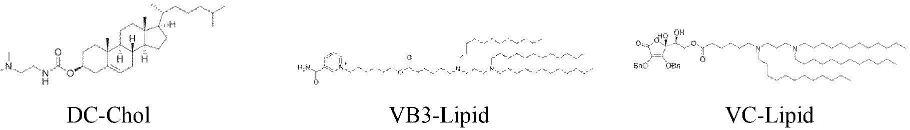

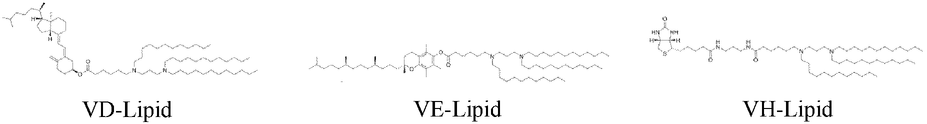

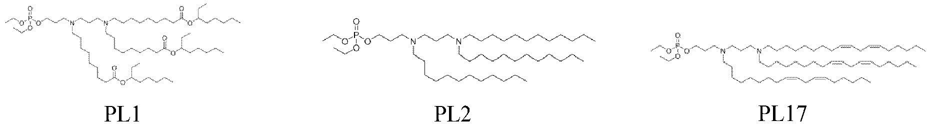

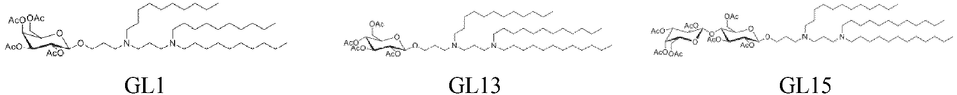

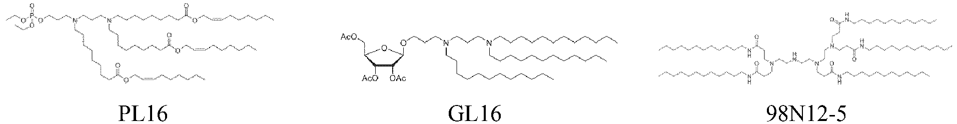

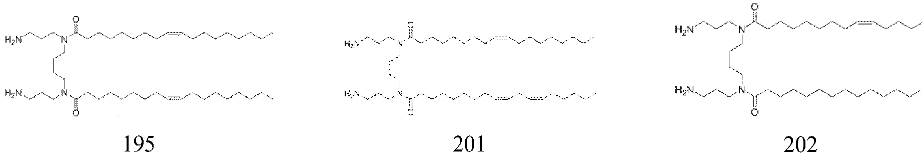

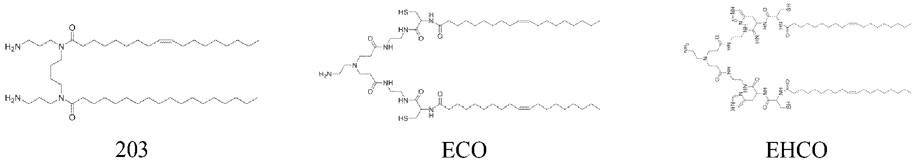

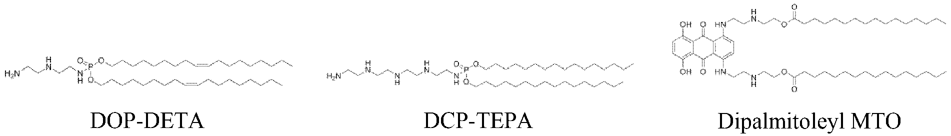

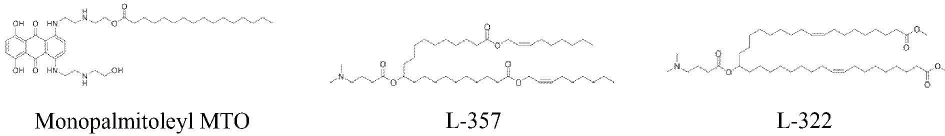

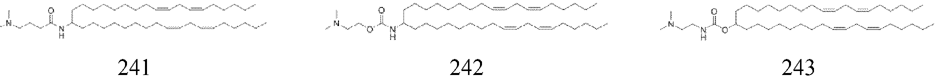

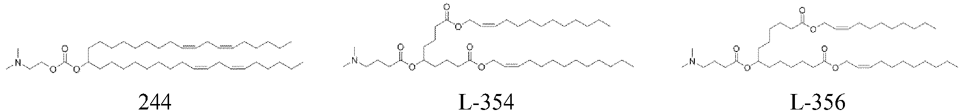

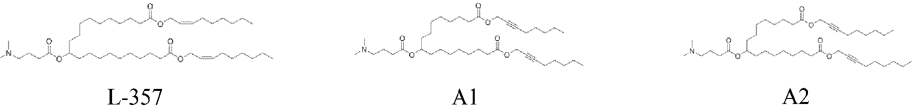

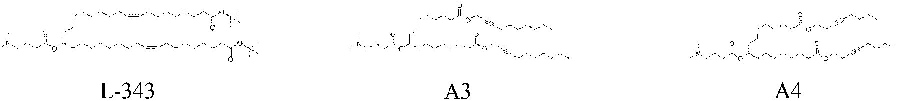

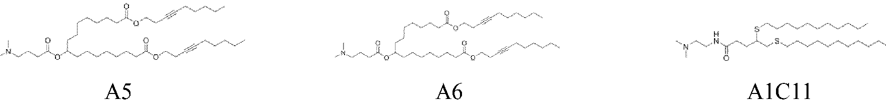

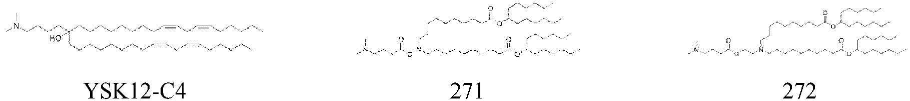

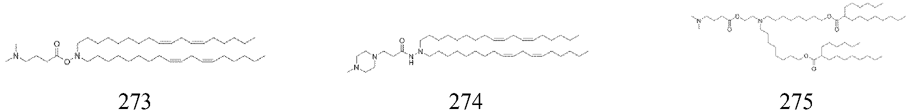

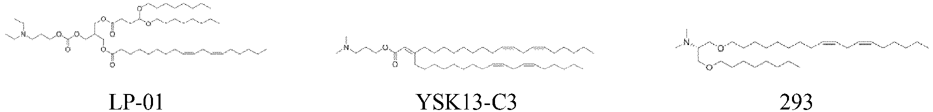

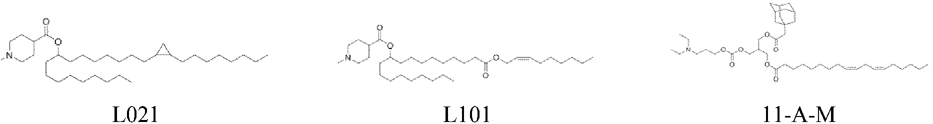

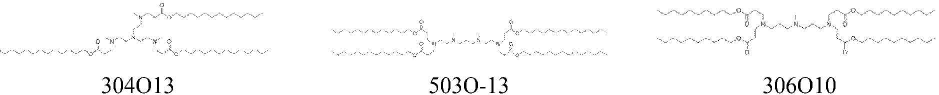

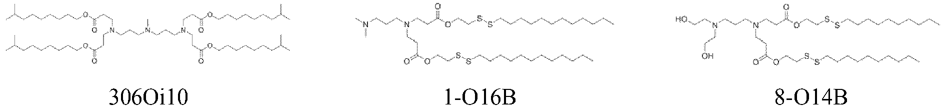

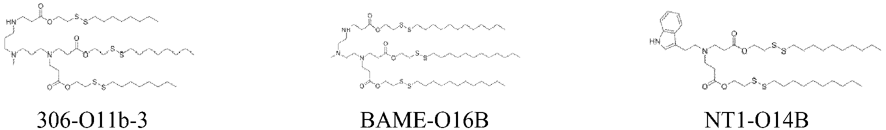

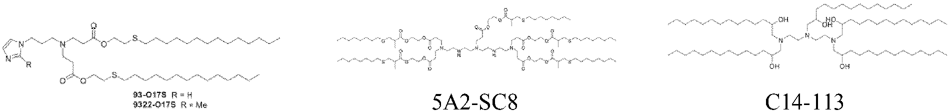

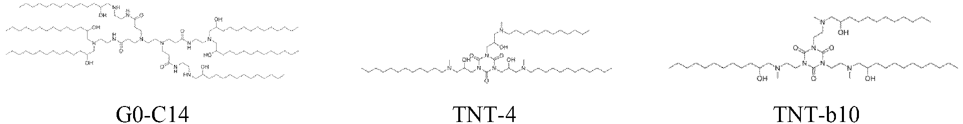

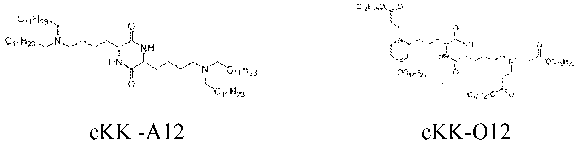

In a preferred embodiment, the phospholipid comprises at least one of 1,2-dilinoleoyl-sn-glycero-3-phosphocholine (DLPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-3-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-docosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-docosahexaenoyl-sn-glycero-3-phosphoethanolamine, 1,2-dioleoyl-sn-glycero-3-phospho-rac-(1-glycerol) sodium salt (DOPG), dipalmitoylphosphatidylglycerol (DPPG), palmitoyloleoylphosphatidylethanolamine (POPE), distearoylphosphatidylethanolamine (DSPE), dipalmitoylphosphatidylethanolamine (DPPE), dimyristoylphosphoethanolamine (DMPE), 1-stearoyl-2-oleoyl-phosphatidylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoylphosphatidylcholine, lysophosphatidylcholine and lysophosphatidylethanolamine (LPE).

In a preferred embodiment, the steroid or a derivative thereof comprises at least one of cholesterol, cholesteryl stearate, coprostanol, sitosterol, ergosterol, campesterol, stigmasterol, brassicasterol, fucosterol, tomatine, ursolic acid and α-tocopherol.

In a preferred embodiment, the polyethylene glycol-lipid conjugate comprises at least one of PEG-modified phosphatidylethanolamine, PEG-modified phosphatidic acid, PEG-modified ceramide, PEG-modified dialkylamine, PEG-modified diacylglycerol and PEG-modified dialkylglycerol.

In a preferred embodiment, the polyethylene glycol-lipid conjugate comprises at least one of PEG-distearoxypropyl (PEG-DSA), PEG-c-DOMG, PEG-DPPC, polyethylene glycol dimethacrylate (PEG-DMA), 1,2-dimyristoyl-rac-glycero-3-methoxypolyethylene glycol (PEG-DMG), dipalmitoylglycerol-polyethylene glycol (DPG-PEG), 1,2-distearoyl-rac-glycero-3-methoxypolyethylene glycol (DSG-PEG), 2-[(polyethylene glycol)-2000]-N,N-ditetradecylacetamide (ALC-0159), dipalmitoylphosphatidylethanolamine-polyethylene glycol (DPPE-PEG), distearoylphosphatidylethanolamine-polyethylene glycol (PEG-DSPE), dilauroylphosphatidylethanolamine-polyethylene glycol (PEG-DLPE) and dimyristoylphosphatidylethanolamine-polyethylene glycol (PEG-DMPE) lipids.

In a preferred embodiment, in the blank lipid nanoparticle-based composition, the biologically active substance is encapsulated inside the blank lipid nanoparticles and/or adsorbed onto the surface of the blank lipid nanoparticles to form a complex.

In a preferred embodiment, the solvent is selected from at least one of water, aqueous organic solvent solutions and buffered salt solutions.

In a preferred embodiment, the buffered salt solution has a pH value of 1-9, a concentration of the buffered salt is 0.1-200 mM, and the aqueous organic solvent solution is an alcohol solution with a volume concentration of less than 50%.

In a preferred embodiment, the alcohol is ethanol.

In a preferred embodiment, the buffered salt solution is selected from at least one of citrate solution, acetate solution, tartrate solution, phosphate solution, carbonate solution, Tris-HCl solution and sodium chloride solution.

In a preferred embodiment, the blank lipid nanoparticle-based composition is further added with at least one of sugar, glycerol, DMSO, salt, antibiotics and surfactants.

In a second aspect, the present application provides an in-vitro gene delivery method, comprising the step of mixing the blank lipid nanoparticles with a biologically active substance in a solvent to obtain a blank lipid nanoparticle-based composition; wherein the blank lipid nanoparticle consists of the following components: 10-60 mol% first ionizable lipid, 0-30 mol% second ionizable lipid, 5-30 mol% phospholipid, 15-70 mol% cholesterol, and 0-10 mol% polyethylene glycol-lipid conjugate.

Compared with the prior art, the present application has the following beneficial effects:
1. the use of the blank lipid nanoparticles in the preparation of an in-vivo delivery product, comprising the step of mixing the blank lipid nanoparticles with a biologically active substance in a solvent to obtain a blank lipid nanoparticle-based composition; the blank lipid nanoparticles consist of an ionizable lipid, a phospholipid, a cholesterol and a polyethylene glycol-lipid conjugate.
2. according to the blank lipid nanoparticles used in the present application, the dosage of the biologically active substance can be flexibly adjusted according to the requirements of a user, and the blank lipid nanoparticles can be administered via multiple routes, all of which can achieve an ideal delivery effect.
3. the preparation process of the present application is simple, and the preparation can be completed without special equipment.

### BRIEF DESCRIPTION OF THE DRAWINGS

FIG. 1 shows whole-body in vivo images of mice at 3 h and 6 h after intravenous administration of the complex of blank lipid nanoparticles and luciferase mRNA prepared in Example 5;
FIG. 2 shows abdominal and lateral in vivo images of mice at 3 h and 6 h after intramuscular injection of the complex of blank lipid nanoparticles and luciferase mRNA prepared in Example 10;
FIG. 3 shows in vivo images of mice at 3 h and 6 h following intraperitoneal and subcutaneous administration of the complex of blank lipid nanoparticles and luciferase mRNA prepared in Example 16;

### DETAILED DESCRIPTION OF THE EMBODIMENTS

The synthetic processes of the present application can tolerate a wide range of functional groups, thereby allowing the use of various substituted starting materials. Such processes generally obtain the desired final compound at or near the end of the synthesis route; however, under certain circumstances, the compound may need to be further converted into a pharmaceutically acceptable salt thereof. The compounds of the present application may be prepared in a variety of ways by using standard synthetic methods and procedures known to those skilled in the art or apparent to the skilled person based on the teachings herein, using commercially available starting materials, compounds known from literature, or easily prepared intermediates. Standard synthetic methods and procedures for preparing organic molecules, as well as functional group interconversions and manipulations, are available from relevant scientific literature or standard textbooks in the field. The following description of synthetic approaches is intended to illustrate, rather than limit, the general procedures for preparing the compounds of the present application.

The compounds of the present application having the formulas described herein may be prepared from commercially available starting materials or starting materials preparable via literature procedures in accordance with the processes illustrated in the corresponding general synthetic schemes. Variables in each general synthetic scheme (e.g., R₁, R₂, R₃, and the like) are as defined herein. Those of ordinary skill in the art will recognize that the sequence of certain steps in the reaction procedures and synthetic schemes described herein may be altered, such as the introduction and removal of protecting groups.

In the reaction schemes described herein, a variety of stereoisomers can be produced. Where no specific stereoisomer is indicated, this shall be construed to include all possible stereoisomers yielded by the reaction. Those of ordinary skill in the art will recognize that the reactions may be optimized to favor formation of one isomer, or new schemes may be designed to yield a single isomer. If a mixture is obtained, the isomers may be separated using techniques such as preparative thin-layer chromatography, preparative HPLC, preparative chiral HPLC, preparative SFC, and the like.

### (1) Preparation Example 1: Synthesis of the First Ionizable Lipid

General synthetic routes for the first ionizable lipid are set forth in General Synthetic Routes 1 to 5.

### General Synthetic Route 1

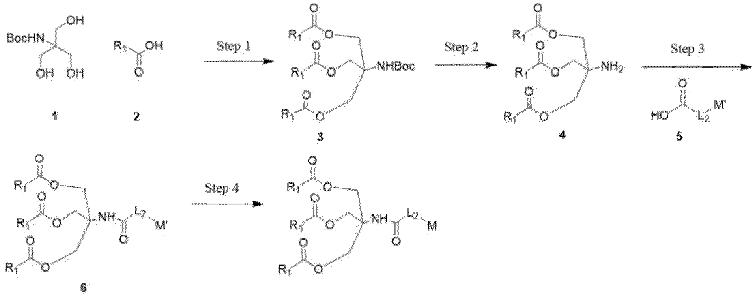

wherein M' is M or M bearing a protecting group.

As illustrated in General Synthetic Route 1 above, Compound 3 is formed by a condensation reaction between Boc-aminotris(hydroxymethyl)methane and an acid (Compound 2). Step 1 can be carried out in an organic solvent (e.g., dichloromethane (DCM)) in the presence of, for example, 1-ethyl-(3-dimethylaminopropyl) carbodiimide hydrochloride (EDCl) and 4-dimethylaminopyridine (DMAP). Step 1 can be carried out at room temperature for 24 hours.

Subsequently, Compound 4 is formed by removal of the Boc protecting group from Compound 3. Step 2 can be carried out in an organic solvent (e.g., DCM) under catalysis by an acid (e.g., trifluoroacetic acid).

Then, Compound 6 is formed by a condensation reaction between Compound 4 and Compound 5. Step 3 can be carried out in an organic solvent (e.g., DCM or DMF) under catalysis by EDCl and DMAP, or dicyclohexylcarbodiimide (DCC).

If the M' moiety of Compound 6 contains the protecting group as described above, then the protecting group is removed and the target lipid compound is obtained. Step 4 can be carried out under reaction conditions for removing the selected protecting group.

### General Synthetic Route 2

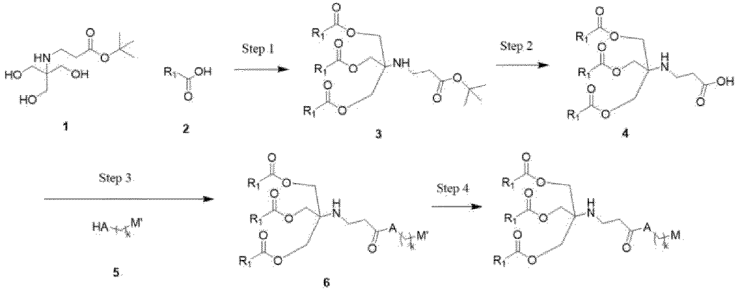

wherein M' is M or M containing a protecting group; A is O, NH or S.

As illustrated in General Synthetic Route 2 above, Compound 3 is formed by a condensation reaction between Compound 1 and Compound 2. Step 1 can be carried out in an organic solvent (e.g., DCM) in the presence of EDCl and DMAP.

Subsequently, Compound 4 is formed by removal of the Boc protecting group from Compound 3. Step 2 can be carried out in an organic solvent (e.g., DCM) in the presence of an acid (e.g., trifluoroacetic acid) and a cation scavenger (e.g., triisopropylsilane (TiPS)).

Then, Compound 6 is formed by a condensation reaction between Compound 4 and Compound 5. Step 3 can be carried out in an organic solvent (e.g., DCM or DMF) in the presence of EDCl and DMAP, or DCC.

If the M' moiety of Compound 6 contains the protecting group as described above, then the protecting group is removed and the target lipid compound is obtained. Step 4 can be carried out under reaction conditions for removal of the selected protecting group.

### General Synthetic Route 3

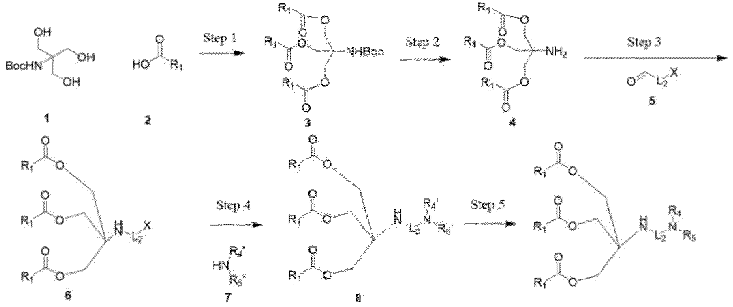

wherein X is a halogen such as Cl, Br or I; R₄' is R₄ or R₄ containing a protecting group; R₅' is R₅ or R₅ containing a protecting group.

As illustrated in General Synthetic Route 3 above, Compound 3 is formed by a condensation reaction between Boc-aminotris(hydroxymethyl)methane and Compound 2. Step 1 can be carried out in an organic solvent (e.g., DCM) in the presence of, for example, EDCl and DMAP. Step 1 can be carried out at room temperature for 24 hours.

Subsequently, Compound 4 is formed by removal of the Boc protecting group from Compound 3. Step 2 can be carried out in an organic solvent (e.g., DCM) under catalysis by an acid (such as trifluoroacetic acid).

Then, Compound 6 is formed by condensation and reduction reactions between Compound 4 and a halogen-substituted aldehyde (Compound 5). Step 3 can be carried out in an organic solvent (e.g., dichloroethane (DCE)) in the presence of a reducing agent (e.g., sodium triacetoxyborohydride (NaBH(OAc)₃)).

Then, Compound 8 is formed by a nucleophilic reaction between Compound 6 and an amine (Compound 7). Step 4 can be carried out in an organic solvent (e.g., DMF) in the presence of a base (such as a non-nucleophilic organic base (e.g., triethylamine, iPr2EtN) or an inorganic base (e.g., K₂CO₃)) and a catalyst (KI or NaI).

If the R₄' and/or R₅' moieties of Compound 8 contain the protecting group as described above, then the protecting group is removed and the target lipid compound is obtained. Step 5 can be carried out under reaction conditions for removal of the selected protecting group.

### General Synthetic Route 4

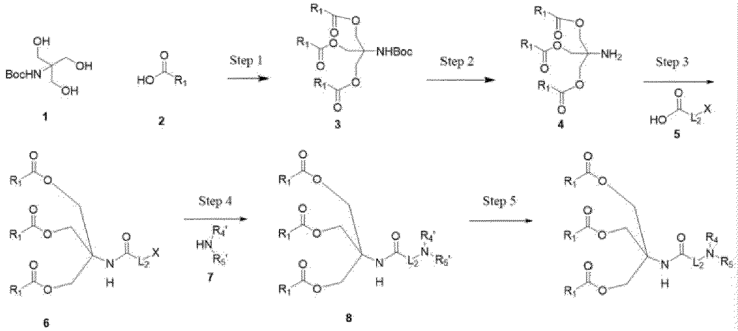

wherein X is a halogen such as Cl, Br or I; R₄' is R₄ or R₄ containing a protecting group; R₅' is R₅ or R₅ containing a protecting group.

As illustrated in General Synthetic Route 4 above, Compound 3 is formed by a condensation reaction between Boc-aminotris(hydroxymethyl)methane and Compound 2. Step 1 can be carried out in an organic solvent (e.g., DCM) in the presence of, for example, EDCl and DMAP. Step 1 can be carried out at room temperature for 24 hours.

Subsequently, Compound 4 is formed by removal of the Boc protecting group from Compound 3. Step 2 can be carried out in an organic solvent (e.g., DCM) under catalysis by an acid (such as trifluoroacetic acid).

Then, Compound 6 is formed by a condensation reaction between Compound 4 and a halogen-substituted Compound 5. Step 3 can be carried out in an organic solvent (e.g., DCM or DMF) under catalysis by EDCl and DMAP, or DCC.

Then, Compound 8 is formed by a nucleophilic reaction between Compound 6 and an amine (Compound 7). Step 4 can be carried out in an organic solvent (e.g., DMF) in the presence of a base (such as a non-nucleophilic organic base (e.g., triethylamine, iPr2EtN) or an inorganic base (e.g., K₂CO₃)) and a catalyst (KI or NaI).

If the R₄' and/or R₅' groups of Compound 8 contain the protecting group as described above, then the protecting group is removed and the target lipid compound is obtained. Step 5 can be carried out under reaction conditions for removal of the selected protecting group.

### General Synthetic Route 5

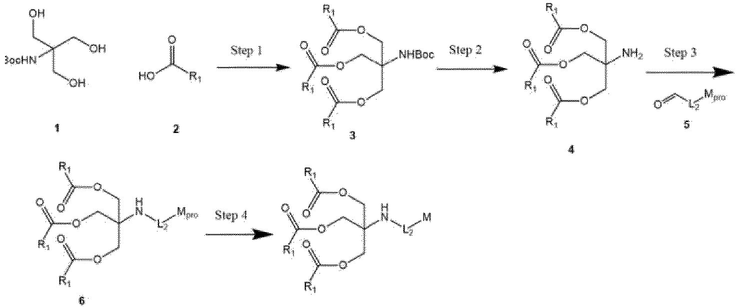

wherein Mₚᵣₒ is M or M containing a protecting group.

As illustrated in General Synthetic Route 5 above, Compound 3 is formed by a condensation reaction between Boc-aminotris(hydroxymethyl)methane and Compound 2. Step 1 can be carried out in an organic solvent (e.g., DCM) in the presence of, for example, EDCl and DMAP. Step 1 can be carried out at room temperature for 24 hours.

Subsequently, Compound 4 is formed by removal of the Boc protecting group from Compound 3. Step 2 can be carried out in an organic solvent (e.g., DCM) under catalysis by an acid (such as trifluoroacetic acid).

Then, Compound 6 is formed by condensation and reduction reactions between Compound 4 and Compound 5. Step 3 can be carried out in an organic solvent (e.g., dichloroethane (DCE)) in the presence of a reducing agent (e.g., sodium triacetyl borohydride (NaBH(OAc)₃)).

If the Mₚᵣₒ group of Compound 6 contains the protecting group as described above, then the protecting group is removed and the target lipid compound is obtained. Step 4 can be carried out under reaction conditions for removal of the selected protecting group.

Furthermore, it should also be understood that any specific embodiment of the present application falling within the scope of the prior art may be explicitly excluded from any one or more claims. Such embodiments may be excluded even if such exclusion is not explicitly set forth herein, as they are deemed known to those of ordinary skill in the art.

All cited sources, such as references, publications, databases, database entries and techniques cited herein, are incorporated into the present application by reference, even where not explicitly stated upon citation. In the event of any inconsistency between the statements in the cited sources and those of the present application, the statements of the present application shall prevail.

### (1) Synthesis of compounds according to formula (1), (1A), (1B), (1C), (1D), (1E), (1F), (1G), (1H), (1I), (1J), (1K), (1L) or (1M)

### A. General Considerations

It is worth noting that all starting materials used in the present application are common commercially available products, and no specific limitation is imposed on their sources.

The synthetic routes described below can be used to synthesize Compounds 1001 to 3422 of the present application.

The following abbreviations are adopted herein:
THF: tetrahydrofuran
MeCN: acetonitrile
MeOH: methanol
PE: petroleum ether
EA: ethyl acetate
DMF: N,N-dimethylformamide
EDCl: 1-ethyl-(3-dimethylaminopropyl)carbodiimide hydrochloride
LAH: lithium aluminum hydride
DCM: dichloromethane
DMAP: 4-dimethylaminopyridine
LDA: lithium diisopropylamide
rt: room temperature
DCE: 1,2-dichloroethane
n-BuLi: n-butyllithium
i-Pr2EtN: N,N-diisopropylethylamine
B. Synthesis of Intermediates

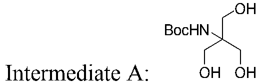

Intermediate A is obtained via the following synthetic route:

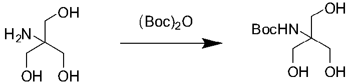

Tris(hydroxymethyl)aminomethane (50.0 g) and di-tert-butyl dicarbonate (Boc₂O) (99.1 g) were dissolved in a mixed solvent of methanol (300 mL) / H₂O (30 mL), reacted at room temperature for 72 h, and purified by silica gel column chromatography (DCM:MeOH = 20:1-10:1) to obtain Intermediate A (N-Boc-aminotris(hydroxymethyl)methane) as a white solid (75.0 g). LCMS (ESI) calcd for C₉H₁₉NO₅, [M+H]⁺ m/z 222.13, found 222.25.

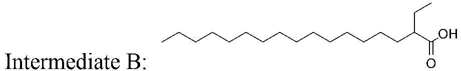

Intermediate B is obtained via the following synthetic route:

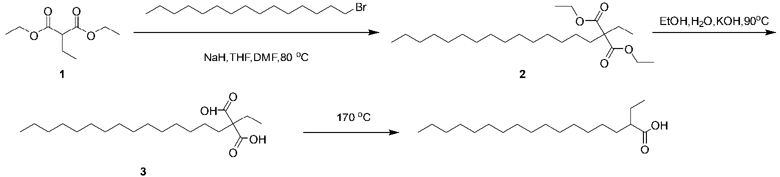

To a single-neck flask were added diethyl 2-ethyl-1,3-propanedioate (29.0 g), THF (90 mL) and DMF (30 mL). NaH (3.7 g) was added under ice bath cooling, and the mixture was stirred at room temperature for 30 min under nitrogen protection; 1-bromopentadecane (15.0 g) was added, and the mixture was reacted at 80°C for 2 h under nitrogen protection. THF was removed by concentration under reduced pressure. The reaction solution was added dropwise into ice water, and ethyl acetate was added with stirring, followed by filtration through celite, and the organic phase was collected for liquid separation. The organic phase was washed with saturated brine and separated, then purified by silica gel column chromatography (PE:EA = 100:1-50:1) to obtain diethyl 2-ethyl-2-pentadecylmalonate (16.5 g). EtOH (50 mL), H₂O (50 mL) and KOH (11.3 g) were added to diethyl 2-ethyl-2-pentadecylmalonate (8.0 g), and the mixture was reacted at 90°C for 12 h. EtOH was removed by concentration under reduced pressure, and dilute hydrochloric acid was added to adjust the pH of the system to 4-5. Water and ethyl acetate were added with stirring for liquid separation. The organic phase was washed with saturated brine and separated, then purified by column chromatography (PE:EA = 5:1-1:1) to obtain 2-ethyl-2-pentadecylmalonic acid (5.2 g). 2-ethyl-2-pentadecylmalonic acid (10.0 g) was reacted at 170°C for 6 h under open conditions, and then cooled to room temperature. Water and ethyl acetate were added with stirring for liquid separation. The organic phase was washed with saturated brine and separated, concentrated, and purified by column chromatography (DCM:MeOH = 20:1-10:1) to obtain Intermediate B (2-ethylheptadecanoic acid) (8.2 g). ¹H NMR (400 MHz, CDCl₃) δ 2.31 (tt,J = 8.6, 5.3 Hz,1H), 1.71-1.46 (m,4H), 1.28 (s,26H), 0.96 (t,J = 7.4 Hz,3H), 0.90 (t,J = 6.7 Hz,3H).

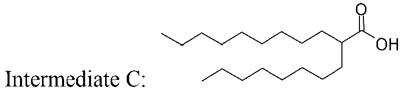

Intermediate C is obtained via the following synthetic route:

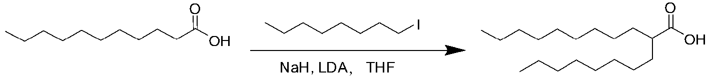

To a three-neck flask were added decanoic acid (50 g) and THF (500 mL). The system was cooled to 0°C, reactant NaH (23.22 g) was then slowly added, and the mixture was stirred at 0°C under nitrogen protection for 1 h. LDA (62.19 g) was slowly added dropwise, and the mixture was stirred at 0°C under nitrogen protection for 1 h. Reactant iodononane (88.52 g) was added dropwise lastly. The mixture was then warmed to room temperature and stirred overnight, then diluted with 1 L DCM, and washed sequentially with saturated NH₄Cl solution and water. The organic phase was dried over anhydrous Na₂SO₄, filtered and concentrated, then purified by silica gel column chromatography (PE:EA = 50:1) to obtain Intermediate C (2-octylundecanoic acid) (11 g). ¹H NMR (400 MHz,Chloroform-d) δ 2.37 (tt,J = 8.7,5.1 Hz,1H),1.71-1.58 (m,2H),1.48 (dt,J = 13.3,6.7 Hz,2H),1.29 (d,J = 9.4 Hz,25H),0.90 (t,J = 6.8 Hz,6H).

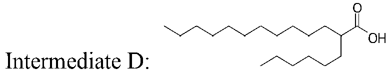

Intermediate D is obtained via the following synthetic route:

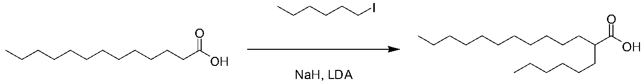

To a three-neck flask were added tridecanoic acid (79.5 g) and solvent THF (800 mL). The system was cooled to 0°C, NaH (22.25 g) was then slowly added, and the reaction solution was stirred at 0°C under nitrogen protection for 1 h. LDA (317.87 g) was slowly added dropwise to the reaction system, and the reaction solution was stirred at 0°C under nitrogen protection for 1 h. Reactant n-hexyl iodide (94.39 g) was added dropwise. The mixture was then warmed to room temperature and stirred overnight, then the reaction solution was diluted with 1 L DCM and washed sequentially with saturated NH₄Cl solution and water. The organic phase was dried over anhydrous Na₂SO₄, filtered and concentrated, then purified by silica gel column chromatography (PE:EA = 50:1) to obtain Intermediate D (2-hexyltridecanoic acid) (26 g). ¹H NMR (400 MHz,Chloroform-d) δ 2.36 (tt,J = 8.7,5.4 Hz,1H),1.63 (ddd,J = 14.3,8.7,5.5 Hz,2H),1.49 (dq,J = 13.5,6.6 Hz,2H),1.29 (d,J = 10.1 Hz,26H),0.90 (t,J = 6.6 Hz,6H)

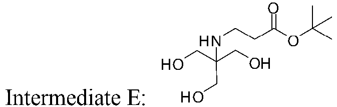

Intermediate E is obtained via the following synthetic route:

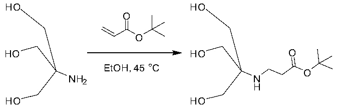

The compound tris(hydroxymethyl)aminomethane (10.0 g) and tert-butyl acrylate (21.1 g) were dissolved in EtOH (150 mL). The mixture was reacted at 45°C under nitrogen protection for 30 h, and EtOH was removed by concentration under reduced pressure. Solvent (PE:EA = 20:1) (100 mL × 3) was added for trituration, followed by filtration to obtain white solid compound Intermediate E (11.5 g). ¹H NMR (400 MHz,CDCl₃) δ 3.60 (s,6H),2.84 (t,J = 5.9 Hz,2H),2.47 (t,J = 5.8 Hz,2H),1.48 (s,9H).

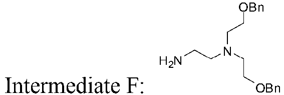

Intermediate F is obtained via the following synthetic route:

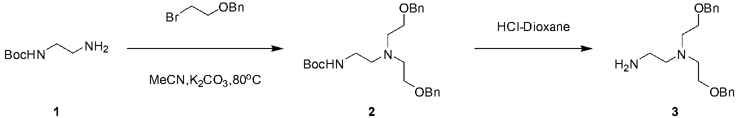

To a single-neck flask were added tert-butyl (2-aminoethyl)carbamate (2.7 g), MeCN (90 mL), benzyl 2-bromoethyl ether (7.99 g) and K₂CO₃ (11.65 g). The mixture was reacted at 80°C overnight. Water and ethyl acetate were then added with stirring for liquid separation. The organic phase was washed with saturated brine, separated and concentrated, then purified by silica gel column chromatography (PE:EA = 10:1-5:1) to obtain Compound 2 (5.5 g). Dioxane (30 mL) and dioxane hydrochloride solution (30 mL) were added to Compound 2, and the mixture was stirred at room temperature for 3 h. The solvent was removed by concentration under reduced pressure to obtain compound 3, namely Intermediate F (6 g). LCMS (ESI) calcd for C₂₀H₂₈N₂O₂, [M+H]⁺ m/z 329.22, found 329.24.

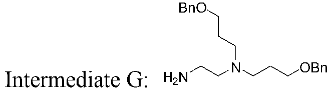

Intermediate G is obtained via the following synthetic route:

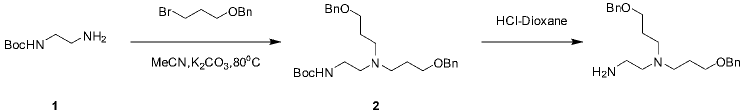

To a single-neck flask were added Compound 1 (3.0 g), MeCN (90 mL), 3-benzyloxypropyl bromide (9.45 g) and K₂CO₃ (12.94 g). The mixture was reacted at 80°C overnight. Water and ethyl acetate were then added with stirring for liquid separation. The organic phase was washed with saturated brine, separated and concentrated, then purified by column chromatography (PE:EA = 10:1-5:1) to obtain Compound 2 (6.9 g). Dioxane (30 mL) and dioxane hydrochloride solution (30 mL) were added to Compound 2 (6.9 g), and the mixture was stirred at room temperature for 3 h. The solvent was removed by concentration under reduced pressure to obtain Intermediate G (7.5 g). LCMS (ESI) calcd for C₂₂H₃₂N₂O₂,[M+H]⁺ m/z 357.25,found 357.51.

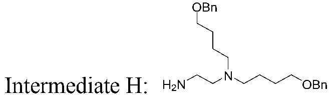

Intermediate H is obtained via the following synthetic route:

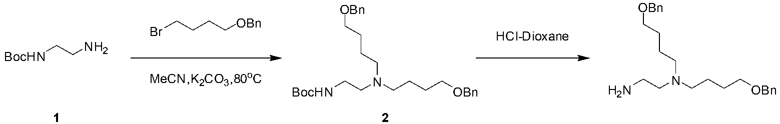

To a single-neck flask were added Compound 1 (1.5 g), MeCN (90 mL), benzyl 4-bromobutyl ether (5.02 g) and K₂CO₃ (6.47 g). The mixture was reacted at 80°C overnight. Water and ethyl acetate were then added with stirring for liquid separation. The organic phase was washed with saturated brine, separated and concentrated, then purified by column chromatography (PE:EA = 20:1-10:1) to obtain Compound 2 (4.1 g). Dioxane (30 mL) and dioxane hydrochloride solution (30 mL) were added to Compound 2 (4.1 g), and the mixture was stirred at room temperature for 3 h. The solvent was removed by concentration under reduced pressure to obtain Intermediate H (4.9 g). LCMS (ESI) calcd for C₂₄H₃₆N₂O₂, [M+H]⁺m/z 385.28, found 385.56.

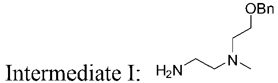

Intermediate I is obtained via the following synthetic route:

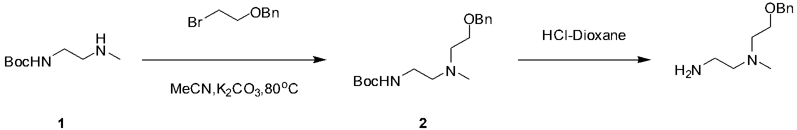

To a single-neck flask were added Compound 1 (3.9 g), MeCN (90 mL), benzyl 2-bromoethyl ether (5.78 g) and K₂CO₃ (15.46 g). The mixture was reacted at 80°C overnight. Water and ethyl acetate were then added with stirring for liquid separation. The organic phase was washed with saturated brine, separated and concentrated, then purified by column chromatography (PE:EA = 20:1-10:1) to obtain Compound 2 (6.8 g). Dioxane (30 mL) and dioxane hydrochloride solution (30 mL) were added to Compound 2 (6.8 g), and the mixture was stirred at room temperature for 3 h. The solvent was removed by concentration under reduced pressure to obtain Intermediate I (6.5 g). LCMS (ESI) calcd for C₁₂H₂₀N₂O, [M+H]⁺m/z 209.16, found 209.31.

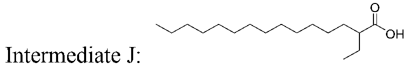

Intermediate J is obtained via the following synthetic route:

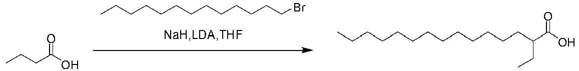

To a single-neck flask were added butyric acid (5.0 g) and THF (100 mL). NaH (2.73 g) was added at 0°C, then LDA (56.8 mL) was slowly added dropwise. The mixture was reacted at room temperature for 30 min, then 1-bromotridecane was added and the mixture was continuously reacted at room temperature overnight. Ice water and ethyl acetate were added with stirring for liquid separation. The organic phase was washed with saturated brine, separated and concentrated, then purified by column chromatography (PE:EA = 10:1-5:1) to obtain Intermediate J (5.0 g). LCMS (ESI) calcd for C₁₇H₃₄O₂, [M+H]⁺m/z 271.26, found 271.46.

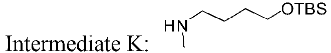

Intermediate K is obtained via the following synthetic route:

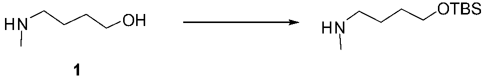

To a three-neck flask were added Reactant 1 (24 g), imidazole (19.00 g) and solvent DCM (200 mL). The system was cooled to 0°C, reactant TBDMSCl (38.57 g) was then slowly added. The mixture was stirred at 25°C for 4 h, and then diluted with DCM (300 mL). The organic phase was washed with 1 L water twice and dried over anhydrous Na₂SO₄. After filtration and concentration, silica gel was added, and column chromatography was performed with DCM:MeOH = 1:20. The collected fractions were concentrated to obtain Intermediate K (28.00 g). ¹H NMR (400 MHz,CDCl₃) δ 4.89 (s,1H),3.58 (t,J = 6.1 Hz,2H),2.66 (t,J = 7.3 Hz,2H),2.45 (s,3H),1.67-1.40 (m,4H),0.84 (s,9H).

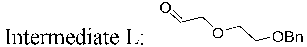

Intermediate L is obtained via the following synthetic route:

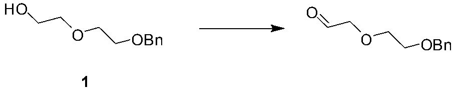

To a three-neck flask were added compound DMSO (3.58 g) and anhydrous solvent DCM (30 mL). The system was cooled to -78°C, and then oxalyl chloride (2.91 g) was slowly added. The reaction solution was stirred at -78°C under N₂ protection for 10 min, and then Compound 1 (3 g) was slowly added dropwise. The mixture was continuously stirred at -78°C under N₂ protection for 1 h, and then TEA (9.28 g) was slowly added dropwise lastly. The reaction solution was continuously stirred at -78°C under N₂ protection for 0.5 h, and the reaction solution was diluted with 100 mL DCM and washed sequentially with saturated NH₄Cl solution and water. The organic layer was dried over anhydrous Na₂SO₄, filtered and concentrated to obtain Intermediate L (3.0 g). LCMS (ESI) calcd for C₁₁H₁₄O₃, [M+H]⁺m/z 195.09, found 195.23.

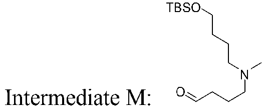

Intermediate M is obtained via the following synthetic route:

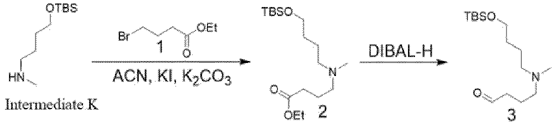

To a sealed tube were added Intermediate K (2.0 g), Compound 1 (1.79 g) and solvent ACN (20 mL), followed by reactant K₂CO₃ (3.81 g) and KI (1.52 g). The mixture was stirred at 70°C overnight under N₂ protection, diluted with 100 mL EA and washed sequentially with saturated NH₄Cl solution and water. The organic layer was dried over anhydrous Na₂SO₄, filtered and concentrated. Silica gel was added for column chromatography with DCM:MeOH = 15:1, and the collected fractions were concentrated to obtain Compound 2 (2.0 g). To a three-neck flask were added Reactant 2 (1.80 g) and solvent DCM (20 mL). The system was purged with nitrogen and cooled to -78°C, and then DIBAL-H (1.00 g) was slowly added dropwise. The mixture was stirred at -78°C for 4 h. Methanol and sodium carbonate solution were slowly added dropwise into the reaction solution to quench the reaction, followed by extraction with DCM (100 mL). The organic layer was dried over anhydrous sodium sulfate, filtered and concentrated to obtain Intermediate M (1.36 g). LCMS (ESI) calcd for C₁₅H₃₃NO₂Si, [M+H]⁺ m/z 288.52, found 288.23.

### C. Compound 1003, synthesized according to General Synthetic Route 1

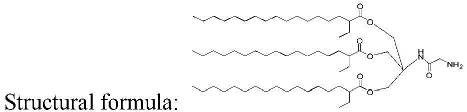

Chemical formula: C₆₃H₁₂₂N₂O₇

Molecular weight: 1019.68

### Step 1: Synthesis of Compound 3 in General Synthetic Route 1

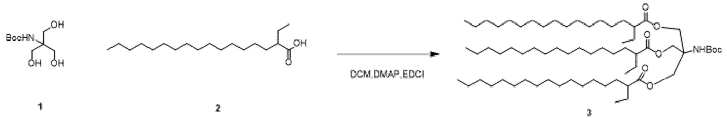

Compound 1 (Intermediate A, 1.0 g), DCM (20 mL), DMAP (2.2 g) and EDCI (3.4 g) were added to Compound 2 (Intermediate B, 4.3 g). The mixture was stirred at room temperature under nitrogen protection for 12 h. Water and dichloromethane were then added with stirring for liquid separation. The organic phase was washed with saturated brine, separated, dried over anhydrous sodium sulfate and concentrated, then purified by silica gel column chromatography (PE:EA = 40:1-30:1) to obtain Compound 3 (2.6 g).

### Step 2: Synthesis of Compound 4 in General Synthetic Route 1

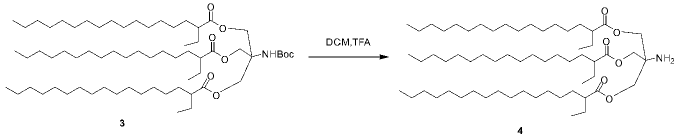

DCM (15 mL) and TFA (5 mL) were added to Compound 3 (2.6 g). The mixture was stirred at room temperature for 3 h and concentrated, then purified by silica gel column chromatography (PE:EA = 10:1-5:1) to obtain Compound 4 (3.2 g).

### Step 3: Synthesis of Compound 6 in General Synthetic Route 1

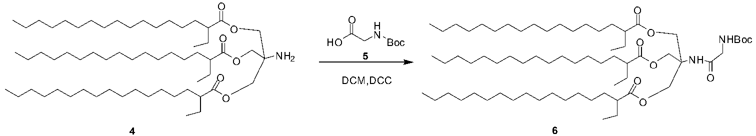

DCM (20 mL), Boc-glycine (Compound 5, 578 mg) and DCC (6.8 g) were added to Compound 4 (3.2 g). The mixture was stirred at room temperature under nitrogen protection for 12 h. Water and ethyl acetate were then added with stirring for liquid separation. The organic phase was washed with saturated brine and separated, dried over anhydrous sodium sulfate and concentrated, then purified by silica gel column chromatography (PE:EA = 5:1-2:1) to obtain Compound 6 (2.2 g).

### Step 4: Compound 1003

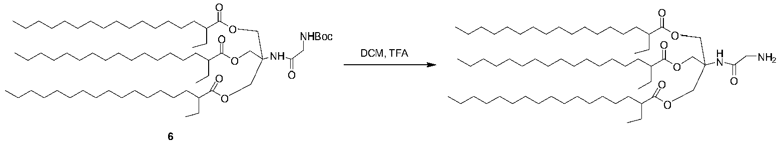

DCM (15 mL) and TFA (5 mL) were added to Compound 6 (2.2 g). The mixture was stirred at room temperature for 12 h and concentrated, then purified by silica gel column chromatography (DCM:MeOH = 30:1-20:1) to obtain Compound 1003 (1.3 g). ¹H NMR (400 MHz,CDCl₃) δ 4.49 (d,J = 2.6 Hz,6H),3.28 (s,2H),2.32 (tt,J = 8.6,5.5 Hz,3H),1.62 (q,J = 7.1 Hz,12H),1.27 (d,J = 3.4 Hz,78H),0.94-0.86 (m,18H).

### D. Compound 1002, synthesized according to General Synthetic Route 1

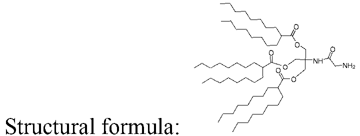

Chemical formula: C₆₀H₁₁₆N₂O₇

Molecular weight: 977.60

Synthesized according to General Synthetic Route 1, with a synthesis process similar to that of Compound 1003. The difference lies in that Intermediate C is used as Compound 2 in General Synthetic Route 1. ¹H NMR (400 MHz,CDCl₃) δ 4.48 (s,6H),3.27 (s,2H),2.37 (tt,J = 8.5,5.5 Hz,3H),1.6 4 (d,J = 6.9 Hz,6H),1.50-1.42 (m,6H),1.27 (s,72H),0.90 (t,J = 6.7 Hz,18H).

### E. Compound 1004, synthesized according to General Synthetic Route 1

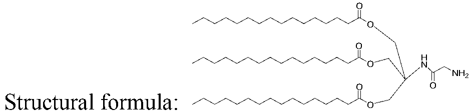

Chemical formula: C₅₄H₁₀₄N₂O₇

Molecular weight: 893.43

Synthesized according to General Synthetic Route 1, with a synthesis process similar to that of Compound 1003. The difference lies in that palmitic acid is used as Compound 2 in General Synthetic Route 1. ¹H NMR (400 MHz,CDCl₃) δ 7.55 (s,1H),4.45 (s,6H),3.74 (s,2H),2.35 (t,J = 7.6 Hz,6H),1.60 (p,J = 6.9 Hz,6H),1.27 (s,72H),0.90 (t,J = 6.7 Hz,9H).

### F. Compound 1001, synthesized according to General Synthetic Route 1

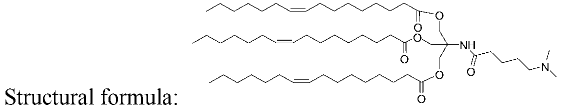

Chemical formula: C₅₉H1₀₈N₂O₇

Molecular weight: 957.52

Synthesized according to General Synthetic Route 1, with a synthesis process similar to that of Compound 1003. The difference lies in that (9Z)-9-hexadecenoic acid is used as Compound 2 in General Synthetic Route 1, and 5-(N,N-dimethylamino)valeric acid is used as Compound 5 in General Synthetic Route 1. ¹H NMR (400 MHz,CDCl₃) δ 5.42-5.30 (m,6H),4.51-4.38 (m,6H),3.19 (dt,J = 11.3,5.6 Hz,2H),2.97 (t,J = 5.9 Hz,6H),2.46 (t,J = 6.6 Hz,1H),2.35 (td,J = 7.6,3.1 Hz,5H),2.20 (t,J = 7.6 Hz,2H),2.03 (d,J = 6.2 Hz,12H),1.89-1.81 (m,3H),1.74 (q,J = 7.8 Hz,2H),1.67-1.51 (m,6H),1.31 (d,J = 7.8 Hz,48H),0.90 (t,J = 6.7 Hz,9H).

### G. Compound 1014, synthesized according to General Synthetic Route 2

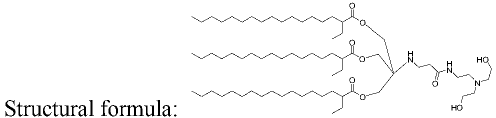

Chemical formula: C₇₀H₁₃₇N₃O₉

Molecular weight: 1164.88

### Step 1: Synthesis of Intermediate 3 in General Synthetic Route 2

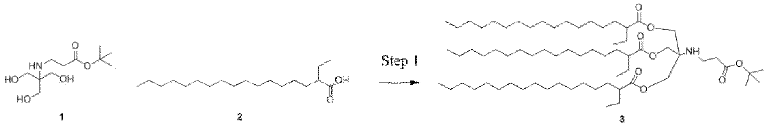

Intermediate 3 is synthesized according to the method illustrated in Step 1 of General Synthetic Route 1. The difference lies in that Intermediate E is used as Compound 1 in General Synthetic Route 1.

### Step 2: Synthesis of Compound 4 in General Synthetic Route 2

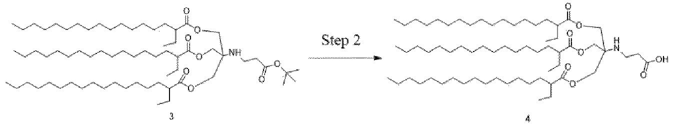

DCM (15 mL), TFA (12 mL) and TiPS (3 mL) were added to Compound 3 (4.8 g). The mixture was reacted at room temperature for 12 h. Water and dichloromethane were then added with stirring for liquid separation. The organic phase was washed with saturated brine, separated, and concentrated, then purified by silica gel column chromatography (DCM:MeOH = 20:1-10:1) to obtain Compound 4 (3.3 g).

### Step 3: Synthesis of Compound 6 in General Synthetic Route 2

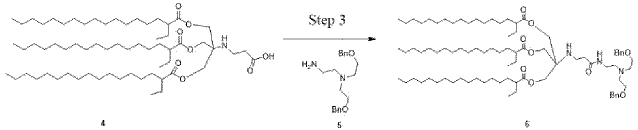

Compound 4 (4.0 g), DCM (80 mL), EDCI (1.1 g), 1-hydroxybenzotriazole (HOBt, 0.78 g) and DIEA (2.5 g) were added to Compound 5 (1.9 g). The mixture was stirred at room temperature for 12 h. Water and dichloromethane were then added with stirring for liquid separation. The organic phase was washed with saturated brine, separated, and concentrated, then purified by silica gel column chromatography (DCM:MeOH = 30:1-20:1) to obtain Compound 6 (4.0 g).

### Step 4: Compound 1014

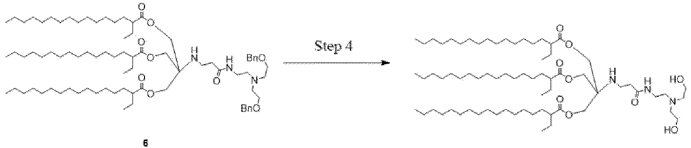

MeOH (30 mL), DCM (10 mL) and Pd/C (4.73 g, 10%) were added to Compound 6 (4.0 g). The mixture was stirred overnight at room temperature under hydrogen atmosphere, filtered through celite and concentrated, then purified by silica gel column chromatography (DCM:MeOH = 30:1-15:1) to obtain Compound 1014 (2 g). ¹H (400 MHz,CDCl₃) δ 7.38 (t,J = 5.8 Hz,1H),4.14-4.05 (m,6H),3.58 (t,J = 4.9 Hz,4H),3.31 (q,J = 5.7 Hz,2H),2.89 (t,J = 6.3 Hz,2H),2.64 (q,J = 5.2 Hz,6H),2.29 (dq,J = 10.8,4.4 Hz,6H),1.66-1.37 (m,12H),1.24 (d,J = 4.3 Hz,78H),0.87 (t,J = 7.1 Hz,18H).

### H. Compound 1015, synthesized according to General Synthetic Route 2

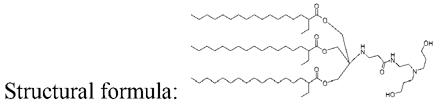

Chemical formula: C₇₂H₁₄₁N₃O₉

Molecular weight: 1192.93

Synthesized according to General Synthetic Route 2, with a synthesis process similar to that of Compound 1014. The difference lies in that Intermediate G is used as Compound 5 in General Synthetic Route 2. ¹H (400 MHz,CDCl₃) δ 7.58 (t,J = 5.7 Hz,1H),4.15-4.01 (m,6H),3.70 (t,J = 5.4 Hz,4H),3.39 (q,J = 5.7 Hz,2H),2.86 (t,J = 6.2 Hz,2H),2.57 (dt,J = 20.5,5.8 Hz,6H),2.36-2.22 (m,5H),1.76-1.67 (m,4H),1.62-1.35 (m,13H),1.23 (d,J = 4.6 Hz,78H),0.92-0.80 (m,18H).

### I. Compound 1020, synthesized according to General Synthetic Route 2

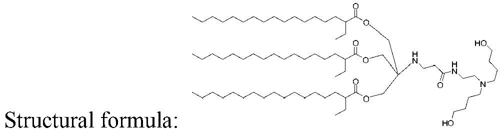

Chemical formula: C₇₄H₁₄₅N₃O₉

Molecular weight: 1220.99

Synthesized according to General Synthetic Route 2, with a synthesis process similar to that of Compound 1014. The difference lies in that Intermediate H is used as Compound 5 in General Synthetic Route 2.

### J. Compound 1025, synthesized according to General Synthetic Route 2

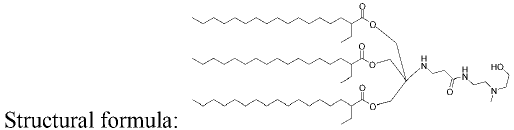

Chemical formula: C₆₉H₁₃₅N₃O₈

Molecular weight: 1134.85

Synthesized according to General Synthetic Route 2, with a synthesis process similar to that of Compound 1014. The difference lies in that Intermediate I is used as Compound 5 in General Synthetic Route 2. ¹H (400 MHz,CDCl₃) δ 6.98 (t,J = 5.7 Hz,1H),4.10 (m,J = 3.8 Hz,6H),3.63 (t,J = 5.2 Hz,2H),3.35 (q,J = 5.8 Hz,2H),2.89 (t,J = 6.1 Hz,2H),2.57 (q,J = 5.3 Hz,4H),2.32 (s,3H),2.28 (q,J = 4.1 Hz,5H),1.65-1.41 (m,13H),1.24 (d,J = 4.4 Hz,78H),0.87 (t,J = 7.1 Hz,18H).

### K. Compound 1005, synthesized according to General Synthetic Route 2

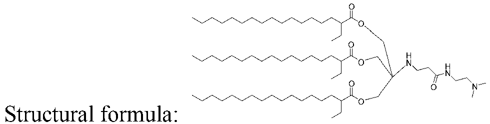

Chemical formula: C₆₈H₁₃₃N₃O₇

Molecular weight: 1104.83

Synthesized according to General Synthetic Route 2, with a synthesis process similar to that of Compound 1014. The difference lies in that N,N-dimethylethylenediamine is used as Compound 5 in General Synthetic Route 2. ¹H NMR (400 MHz,CDCl₃) δ 4.19-4.07 (m,6H),3.40 (d,J = 5.7 Hz,2H),2.95-2.87 (m,2H),2.82 (s,4H),2.64-2.55 (m,2H),2.38 (s,6H),2.33-2.29 (m,3H),1.63-1.55 (m,6H),1.49-1.44 (m,2H),1.26 (d,J = 5.4 Hz,78H),0.89 (td,J = 7.0,2.8 Hz,18H).

### L. Compound 1006, synthesized according to General Synthetic Route 2

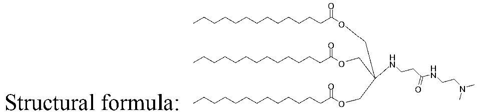

Chemical formula: C₅₃H₁₀₃N₃O₇

Molecular weight: 894.42

Synthesized according to General Synthetic Route 2, with a synthesis process similar to that of Compound 1014. The difference lies in that tetradecanoic acid is used as Compound 2 and N,N-dimethylethylenediamine is used as Compound 5 in General Synthetic Route 2. ¹H NMR (400 MHz,CDCl₃) δ 7.99 (t,J = 5.8 Hz,1H),4.13 (s,6H),3.60 (q,J = 5.3 Hz,2H),3.38-3.34 (m,2H),3.00 (s,6H),2.94 (t,J = 5.8 Hz,2H),2.45 (t,J = 5.8 Hz,2H),2.35 (t,J = 7.6 Hz,6H),1.66-1.56 (m,6H),1.33-1.23 (m,60H),0.90 (t,J = 6.8 Hz,9H).

### M. Compound 1007, synthesized according to General Synthetic Route 2

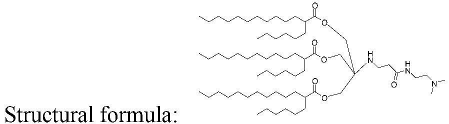

Chemical formula: C₆₈H₁₃₃N₃O₇

Molecular weight: 1104.83

Synthesized according to General Synthetic Route 2, with a synthesis process similar to that of Compound 1014. The difference lies in that Intermediate D is used as Compound 2 and N,N-dimethylethylenediamine is used as Compound 5 in General Synthetic Route 2. ¹H NMR (400 MHz,Chloroform-d) δ 7.13 (t,J = 5.0 Hz,1H),4.13 (s,6H),3.33 (q,J = 5.6 Hz,2H),2.92 (t,J = 6.0 Hz,2H),2.47-2.28 (m,7H),2.24 (s,6H),1.60 (dq,J = 14.7,7.2 Hz,6H),1.47 (dd,J = 14.2,6.7 Hz,6H),1.27 (d,J = 3.4 Hz,78H),0.89 (td,J = 6.7,2.5 Hz,18H).

### N. Compound 1008, synthesized according to General Synthetic Route 2

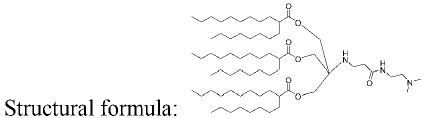

Chemical formula: C₆₈H₁₃₃N₃O₇

Molecular weight: 1104.01

Synthesized according to General Synthetic Route 2, with a synthesis process similar to that of Compound 1014. The difference lies in that Intermediate C is used as Compound 2 and N,N-dimethylethylenediamine is used as Compound 5 in General Synthetic Route 2. ¹H NMR (400 MHz,Chloroform-d) δ 4.13 (s,6H),3.33 (q,J = 5.6 Hz,2H),2.92 (t,J = 5.9 Hz,2H),2.51-2.26 (m,7H),2.24 (s,6H),1.59 (ddt,J = 14.8,10.9,6.4 Hz,6H),1.47 (tq,J = 11.0,5.4 Hz,6H),1.26 (s,77H),0.89 (t,J = 6.7 Hz,18H).

### O. Compound 1009, synthesized according to General Synthetic Route 2

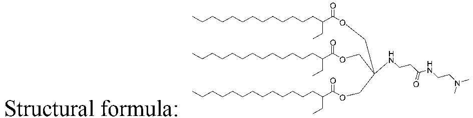

Chemical formula: C₆₂H₁₂₁N₃O₇

Molecular weight: 1020.66

Synthesized according to General Synthetic Route 2, with a synthesis process similar to that of Compound 1014. The difference lies in that Intermediate J is used as Compound 2 and N,N-dimethylethylenediamine is used as Compound 5 in General Synthetic Route 2. ¹H (400 MHz,CDCl₃) δ 7.04 (t,J = 5.0 Hz,1H),4.12 (m,J = 3.3 Hz,6H),3.30 (q,J = 5.6 Hz,2H),2.91 (t,J = 6.0 Hz,2H),2.39 (t,J = 6.0 Hz,2H),2.33-2.25 (m,5H),2.22 (s,6H),1.76-1.54 (m,12H),1.24 (m,J = 3.7 Hz,66H),0.90-0.84 (m,18H).

### P. Compound 1011, synthesized according to General Synthetic Route 2

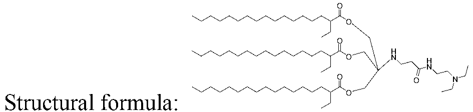

Chemical formula: C₇₀H₁₃₇N₃O₇

Molecular weight: 1132.88

Synthesized according to General Synthetic Route 2, with a synthesis process similar to that of Compound 1014. The difference lies in that N,N-diethylethylenediamine is used as Compound 5 in General Synthetic Route 2. ¹H NMR (400 MHz,CDCl₃) δ 4.19-4.08 (m,6H),3.31 (q,J = 5.8 Hz,2H),2.92 (t,2H),2.57 (t,J = 6.9 Hz,6H),2.37-2.26 (m,5H),1.62 (ddd,J = 18.3,9.0,4.3 Hz,12H),1.27 (d,J = 4.7 Hz,78H),1.04 (t,J = 7.1 Hz,6H),0.94-0.86 (m,18H).

### Q. Compound 1012, synthesized according to General Synthetic Route 2

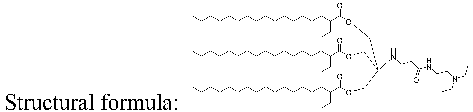

Chemical formula: C₇₂H₁₄₁N₃O₇

Molecular weight: 1160.93

Synthesized according to General Synthetic Route 2, with a synthesis process similar to that of Compound 1014. The difference lies in that N,N-diethylethylenediamine is used as Compound 5 in General Synthetic Route 2. ¹H NMR (400 MHz,CDCl₃) δ 6.59 (s,1H),4.18-4.07 (m,6H),3.29 (q,J = 5.7 Hz,2H),2.91 (t,J = 6.2 Hz,2H),2.54 (t,J = 6.1 Hz,2H),2.40 (t,J = 7.5 Hz,4H),2.35-2.27 (m,5H),1.63-1.42 (m,16H),1.27 (m,J = 4.5 Hz,78H),0.93-0.85 (m,24H).

### R. Compound 1013, synthesized according to General Synthetic Route 2

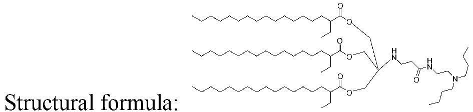

Chemical formula: C₇₄H₁₄₅N₃O₇

Molecular weight: 1188.99

Synthesized according to General Synthetic Route 2, with a synthesis process similar to that of Compound 1014. The difference lies in that N,N-diethylethylenediamine is used as Compound 5 in General Synthetic Route 2. ¹H NMR (400 MHz,CDCl₃) δ 4.10 (d,J = 4.1 Hz,6H),3.26 (q,J = 5.8 Hz,2H),2.89 (t,J = 6.2 Hz,2H),2.52 (d,J = 6.6 Hz,2H),2.41 (t,J = 7.4 Hz,4H),2.33-2.25 (m,5H),1.60 (d,J = 11.8 Hz,16H),1.24 (d,J = 4.5 Hz,82H),0.96-0.79 (m,24H).

### S. Compound 1029, synthesized according to General Synthetic Route 2

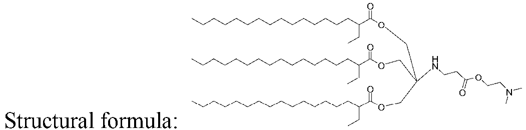

Chemical formula: C₆₈H₁₃₂N₂O₈

Molecular weight: 1105.81

Synthesized according to General Synthetic Route 2, with a synthesis process similar to that of Compound 1014. The difference lies in that N,N-diethyl-2-hydroxyethylamine is used as Compound 5 in General Synthetic Route 2. ¹H NMR (400 MHz,CDCl₃)δ 4.16 (t,J = 5.8 Hz,2H),4.13-4.05 (m,6H),2.86 (t,J = 6.3 Hz,2H),2.55 (t,J = 5.8 Hz,2H),2.44 (t,J = 6.3 Hz,2H),2.35-2.20 (m,9H),1.60-1.39 (m,11H),1.24 (d,J = 4.5 Hz,80H),1.02-0.79 (m,18H).

### T. Compound 1111, synthesized according to General Synthetic Route 2

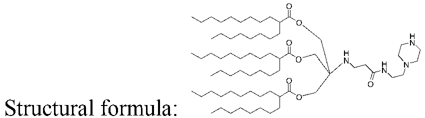

Chemical formula: C₇₀H₁₃₆N₄O₇

Molecular weight: 1145.88

Synthesized according to General Synthetic Route 2, with a synthesis process similar to that of Compound 1014. The difference lies in that Intermediate C is used as Compound 2 and tert-butyl 4-(2-aminoethyl)piperazine-1-carboxylate is used as Compound 5 in General Synthetic Route 2. ¹H NMR (400 MHz,Chloroform-d) δ 4.28 (s,6H),3.41 (s,2H),3.31 (s,4H),3.22 (s,3H),2.97-2.80 (m,5H),2.70 (s,5H),2.46-2.32 (m,4H),1.60 (t,J = 7.1 Hz,6H),1.54-1.40 (m,6H),1.27 (s,78H),0.89 (t,J = 6.7 Hz,18H).

### U. Compound 1118, synthesized according to General Synthetic Route 2

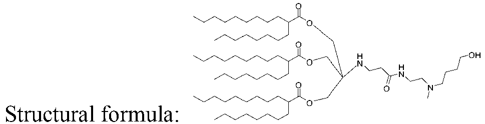

Chemical formula: C₇₁H₁₃₉N₃O₈

Molecular weight: 1162.91

Synthesized according to General Synthetic Route 2, with a synthesis process similar to that of Compound 1014. The difference lies in that Intermediate C is used as Compound 2 and Intermediate K is used as Compound 5 in General Synthetic Route 2. ¹H NMR (400 MHz,Chloroform-d) δ 4.11 (s,6H),3.61 (s,2H),3.42 (d,J = 33.5 Hz,2H),2.91 (t,J = 6.2 Hz,3H),2.36 (dd,J = 9.2,3.6 Hz,6H),1.47 (dd,J = 13.9,6.2 Hz,14H),1.27 (s,71H),0.90 (t,J = 6.7 Hz,18H).

### V. Compound 1010, synthesized according to General Synthetic Route 3

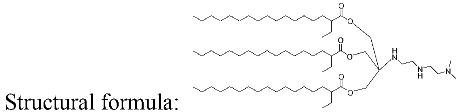

Chemical formula: C₆₇H₁₃₃N₃O₆

Molecular weight: 1076.82

### Step 1 and Step 2: Synthesis of Compound 3 and Compound 4 in General Synthetic Route 3

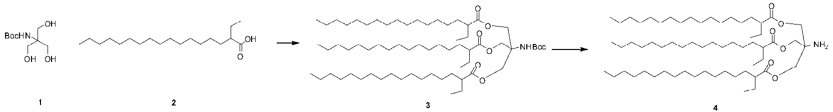

Synthesized according to Step 1 and Step 2 of General Synthetic Route 1, with a synthesis process identical to that of Compound 1003.

### Step 3: Synthesis of Compound 6 in General Synthetic Route 3

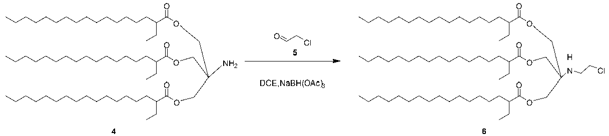

Compound 4 (600 mg), chloroacetaldehyde (Compound 5, 242 mg, 40%) and NaBH(OAc)₃ (394 mg) were added into DCE (60 mL). The mixture was reacted overnight at room temperature. Water and dichloromethane were then added with stirring for liquid separation. The organic phase was washed with saturated brine, separated and concentrated, then purified by silica gel column chromatography (DCM:MeOH = 30:1-15:1) to obtain Compound 6 (270 mg).

### Step 4: Synthesis of Compound 1010

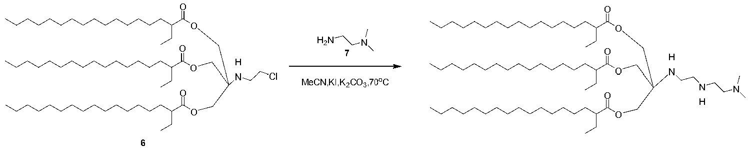

MeCN (20 mL), N,N-dimethylethylenediamine (Compound 7, 229 mg), KI (43 mg) and K₂CO₃ (179 mg) were added to Compound 6 (270 mg). The mixture was reacted overnight at 70°C under nitrogen protection, concentrated, then purified by silica gel column chromatography to obtain Compound 1010 (16 mg). ¹H (400 MHz,CDCl₃) δ 4.10 (m,J = 2.9 Hz,6H),2.76-2.64 (m,6H),2.40 (t,J = 6.2 Hz,2H),2.29 (ddd,J = 8.5,5.6,2.9 Hz,3H),2.22 (s,6H),1.97 (s,6H),1.50-1.41 (m,6H),1.24 (d,J = 4.2 Hz,78H),0.87 (t,J = 7.1 Hz,18H).

### W. Compound 1059, synthesized according to General Synthetic Route 3

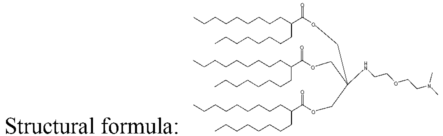

Chemical formula: C₆₇H₁₃₂N₂O₇

Molecular weight: 1077.80

Synthesized according to General Synthetic Route 3, with a synthesis process similar to that of Compound 1010. The difference lies in that Intermediate C and Intermediate L are used as Compound 2 and Compound 5 respectively in General Synthetic Route 3. In addition, Compound 6 in General Synthetic Route 3 needs to undergo the following process steps for functional group transformation:

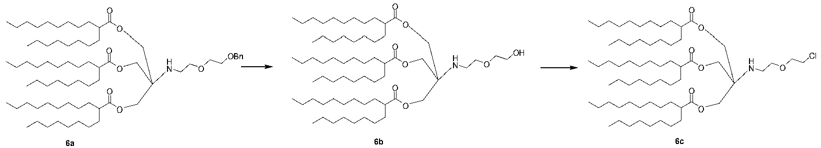

To Intermediate 6a (1.5 g) were added MeOH (5 mL) and THF (5 mL), followed by Pd(OH)₂/C (10%) (0.46 g) and Pd/C (10%) (0.35 g). The mixture was reacted overnight at 25°C under a H₂ atmosphere, filtered through celite and concentrated, then purified by silica gel column chromatography (PE:EA = 30:1-10:1) to obtain Intermediate 6b (520 mg). To Intermediate 6b (470 mg) was added DCM (5 mL), followed by dropwise addition of SOCl₂ (1064.26 mg, 8.946 mmol) at 0°C. The mixture was warmed to room temperature and reacted for 3 hours. The reaction solution was then extracted with water and dichloromethane. The organic phase was concentrated, then purified by silica gel column chromatography (PE:EA = 20:1) to obtain Intermediate 6c (181 mg). To Intermediate 6c (170 mg) were added dimethylamine (143.39 mg) and DMF (2 mL), followed by addition of potassium carbonate (43.96 mg) and potassium iodide (26.39 mg). The mixture was stirred at 70°C, filtered and concentrated, then purified by silica gel column chromatography (DCM:MeOH = 20:1) to obtain Compound 1059 (91 mg, yield 53.22 %). ¹H NMR (400 MHz,Chloroform-d) δ 4.11 (s,6H),3.51 (dt,J = 18.3,5.6 Hz,4H),2.81 (dd,J = 10.3,5.1 Hz,2H),2.50 (t,J = 6.0 Hz,2H),2.42-2.32 (m,3H),2.28 (s,6H),2.08-1.98 (m,1H),1.53-1.38 (m,7H),1.27 (s,77H),0.90 (t,J = 6.7 Hz,18H).

### X. Compound 1112, synthesized according to General Synthetic Route 3

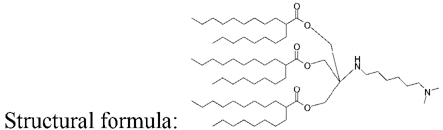

Chemical formula: C₆₉H₁₃₆N₂O₆

Molecular weight: 1089.85

Synthesized according to General Synthetic Route 3, with a synthesis process similar to that of Compound 1010. The difference lies in that Intermediate C and 6-bromohexanal are used as Compound 2 and Compound 5 respectively in General Synthetic Route 3. ¹H NMR (400 MHz,Chloroform-d) δ 4.11 (s,6H),3.11 (s,1H),2.57 (t,J = 6.8 Hz,2H),2.44-2.19 (m,12H),2.04 (q,J = 6.5 Hz,2H),1.53-1.38 (m,13H),1.27 (s,92H),0.90 (t,J = 6.7 Hz,20H).

### Y. Compound 1113, synthesized according to General Synthetic Route 4

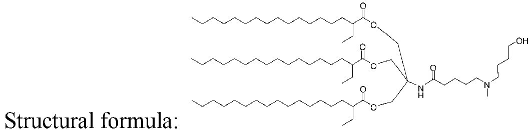

Chemical formula: C₇₁H₁₃₈N₂O₈

Molecular weight: 1147.89

### Step 1 and Step 2: Synthesis of Compound 3 and Compound 4 in General Synthetic Route 4

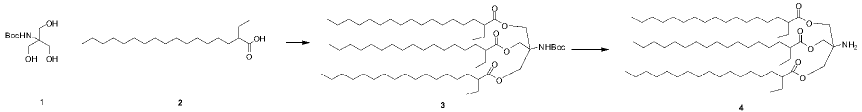

Synthesized according to Step 1 and Step 2 of General Synthetic Route 1, with a synthesis process identical to that of Compound 1003.

### Step 3: Synthesis of Compound 6 in General Synthetic Route 4

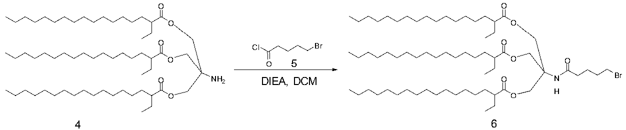

DIEA (0.30 g) and solvent DCM (20 mL) were added to Compound 4. Under nitrogen protection, the system was cooled to 0°C, and then 5-bromopentanoyl chloride (Compound 5, 0.27 g) was added. The mixture was stirred at 25°C for 2 h, diluted with 100 mL DCM, and the organic phase was washed twice with 100 mL water. The organic phase was dried over anhydrous Na₂SO₄, filtered and concentrated, then purified by silica gel column chromatography (PE:EA = 20: 1) to obtain Compound 6 (800 mg).

### Step 4: Synthesis of Compound 8 in General Synthetic Route 4.

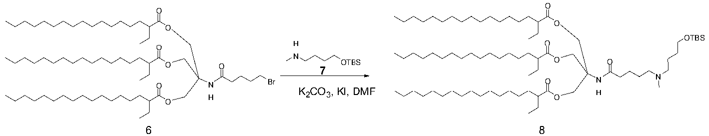

To a three-neck flask were added Compound 6 (750.00 mg), Intermediate K (Compound 7, 217.31 mg) and solvent DMF (20 mL), followed by reactants K₂CO₃ (184.16 mg) and KI (110.60 mg). The mixture was stirred at 25°C for 16 h, then heated to 100°C and stirred for 3 h. The reaction solution was diluted with 200 mL EA, and the organic phase was washed twice with 300 mL saturated brine. The organic phase was dried over anhydrous Na₂SO₄. After filtration and concentration, silica gel was added, and column chromatography was performed with DCM:MeOH = 20:1. The collected fractions were concentrated to obtain Compound 8 (700.00 mg).

### Step 5: Synthesis of Compound 1113

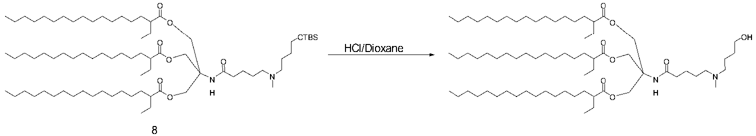

To a three-neck flask were added Compound 8 (700.00 mg) and solvent THF (5 mL), followed by dioxane hydrochloride solution (1 M) (5.55 mL). The mixture was stirred at 25°C for 2 h, diluted with 50 mL EA, and the organic phase was washed with saturated NaHCO₃ solution and water. The organic phase was dried over anhydrous Na₂SO₄, filtered and concentrated, then purified by silica gel column chromatography (MeOH:DCM = 1:20) to obtain Compound 1113 (201.1 mg, yield 31.59 %). ¹H NMR (400 MHz,CDCl₃) δ 4.50-4.39 (m,6H),3.59 (t,J = 4.6 Hz,2H),2.49 (s,3H),2.35-2.26 (m,6H),2.14 (t,J = 7.5 Hz,2H),1.75-1.69 (m,4H),1.66-1.43 (m,17H),1.27 (d,J = 3.7 Hz,78H),0.94-0.87 (m,18H).

### Z. Compound 1115, synthesized according to General Synthetic Route 4

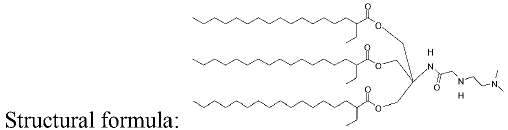

Chemical formula: C₆₇H₁₃₁N₃O₇

Molecular weight: 1090.80

Synthesized according to General Synthetic Route 4, with a synthesis process similar to that of Compound 1010. The difference lies in that 2-bromoacetyl chloride and N,N-dimethylethylenediamine are used as Compound 5 and Compound 7 respectively in General Synthetic Route 4. ¹H NMR (400 MHz,CDCl₃) δ 7.42 (s,1H),4.48 (tt,J = 11.9,5.7 Hz,6H),3.27 (s,2H),3.13 (s,2H),3.01 (s,2H),2.83 (s,6H),2.38-2.25 (m,3H),1.55 (dtt,J = 36.3,14.1,7.3 Hz,12H),1.27 (d,J = 3.3 Hz,78H),0.90 (td,J = 7.2,3.4 Hz,18H)

### AA. Compound 1114, synthesized according to General Synthetic Route 5

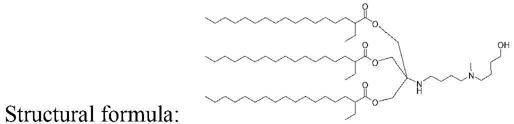

Chemical formula: C₇₀H₁₃₈N₂O₇

Molecular weight: 1119.88

### Step 1 and Step 2: Synthesis of Compound 3 and Compound 4 in General Synthetic Route 5

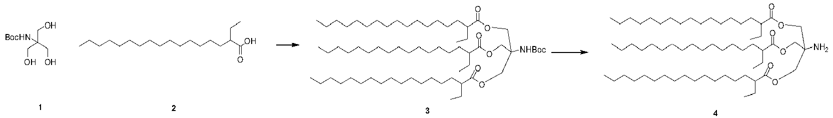

Synthesized according to Step 1 and Step 2 of General Synthetic Route 1, with a synthesis process identical to that of Compound 1003.

### Step 3: Synthesis of Compound 6 in General Synthetic Route 5

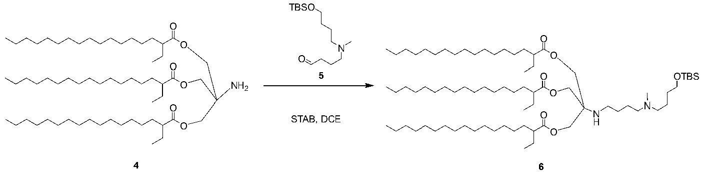

To a three-neck flask, Compound 4 (500 mg) were added Intermediate M (Compound 5, 298.68 mg), reactant STAB (328.68 mg) and DCE (10 mL). The mixture was stirred at 25°C under N₂ protection for 16 h. 100 mL water was then added to the reaction solution, and the mixture was extracted twice with EA (100 mL). The organic phases were combined and concentrated. Silica gel was then added, and column chromatography was performed with DCM:MeOH = 1:20. The collected fractions were concentrated to obtain Compound 6 (500 mg).

### Step 4: Synthesis of Compound 1114

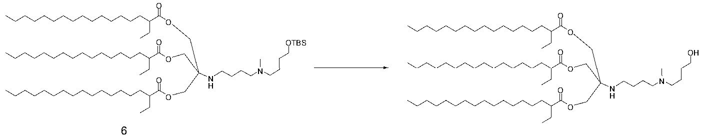

To a three-neck flask were added Compound 6 (450 mg) and solvent THF (5 mL), followed by dioxane hydrochloride solution (1 M) (3.65 mL). The mixture was stirred at 25°C for 2 h and diluted with 50 mL EA. The organic phase was then washed with 50 mL saturated NaHCO₃ solution and water. The organic phase was dried over anhydrous Na₂SO₄, filtered and concentrated, then purified by silica gel column chromatography (MeOH:DCM = 1:20) to obtain Compound 1114 (107 mg, yield 24.84 %). ¹H NMR (400 MHz,CDCl₃) δ 4.16-4.07 (m,6H),3.60 (t,J = 4.8 Hz,2H),2.62 (t,J = 7.0 Hz,2H),2.49 (s,3H),2.31 (tt,J = 8.4,5.5 Hz,6H),1.70 (d,J = 4.6 Hz,3H),1.64-1.39 (m,18H),1.27 (d,J = 4.0 Hz,78H),0.90 (t,J = 7.1 Hz,18H).

### Z. Compound 1120, synthesized according to General Synthetic Route 5

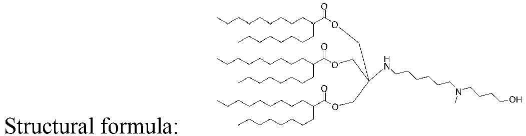

Chemical formula: C₇₂H₁₄₂N₂O₇

Molecular weight: 1147.90

Synthesized according to General Synthetic Route 5, with a synthesis process similar to that of Compound 1114. The difference lies in that Intermediate C and 6-bromohexanal are used as Compound 2 and Compound 5 respectively in General Synthetic Route 5. ¹H NMR (400 MHz,Chloroform-d) δ 4.11 (s,6H),3.63 (s,2H),2.57 (t,J = 6.8 Hz,4H),2.37 (ddd,J = 8.5,7.1,4.3 Hz,5H),1.67-1.41 (m,24H),1.27 (s,79H),0.90 (t,J = 6.7 Hz,18H).

### (2) Preparation Example 2: Synthesis of the Second Ionizable Lipid

### 1. Preparation of Compound 6001

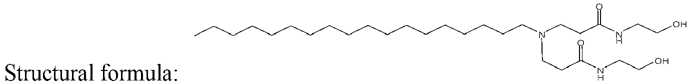

Molecular weight: 499.43

0.8 g oleylamine and 0.8 g hydroxyethyl acrylamide were added to 6.4 mL ethanol, stirred and heated to 70°C. After 3.5 h, 0.8 g hydroxyethylacrylamide was supplemented. After further reaction for 16 h, the mixture was subjected to reverse-phase column chromatography to obtain Compound 6002.

To a reaction flask were added 0.7 g Compound 6002, 0.4 g palladium on carbon and 28 mL tetrahydrofuran, followed by stirring. The system was purged with hydrogen, and a hydrogen balloon was used to maintain pressure. After 16 h, the mixture was filtered and subjected to normal-phase column chromatography (methanol-DCM system) to obtain 200 mg Compound 6001. ¹H NMR (400 MHz, CDCl₃) δ 7.54 (t, *J =* 5.5 Hz, 2H), 3.79-3.68 (m, 4H), 3.42 (dd, *J =* 10.0, 5.4 Hz, 4H), 2.89 (t, *J =* 6.0 Hz, 4H), 2.55 (dt, *J=* 11.8, 6.8 Hz, 6H), 1.54 (s, 2H), 1.27 (d, *J=* 11.2 Hz, 32H), 0.91 (t, *J=* 6.8 Hz, 3H).

### 2. Preparation of Compound 6002

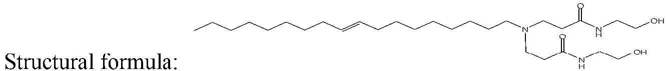

Molecular weight: 497.42

0.8 g oleylamine and 0.8 g hydroxyethyl acrylamide were added to 6.4 mL ethanol, stirred and heated to 70°C. After 3.5 h, 0.8 g hydroxyethyl acrylamide was supplemented. After further reaction for 16 h, the organic solvent was concentrated, followed by reverse-phase column chromatography and lyophilization to obtain a total of 400 mg Compound 6002. ¹H NMR (400 MHz, CDCl₃) δ 7.31 (s, 2H), 5.38 (dd, *J=* 13.0, 7.4 Hz, 2H), 3.79-3.66 (m, 4H), 3.41 (dd, *J=* 10.1, 5.4 Hz, 4H), 2.82-2.72 (m, 4H), 2.44 (dd, *J=* 15.1, 9.2 Hz, 6H), 2.10-1.99 (m, 4H), 1.46 (s, 2H), 1.29 (s, 24H), 0.90 (t, *J* = 6.8 Hz, 3H).

### 3. Preparation of Compound 6003

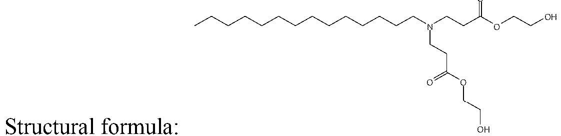

Molecular weight: 445.32

To a reaction flask were added 4.00 g tetradecylamine, 6.53 g hydroxyethyl acrylate and 80 mL tert-butanol, followed by stirring and heating to 70°C. After 29 h, the organic solvent was concentrated, followed by reverse-phase column chromatography and lyophilization to obtain 1.5 g Compound 6003. ¹H NMR (400 MHz, CDCl₃) δ 4.29 (t, *J =* 7.5 Hz, 4H), 3.89-3.75 (m, 4H), 2.81 (t, *J =* 6.2 Hz, 4H), 2.50 (ddd, *J=* 23.2, 14.1, 7.1 Hz, 6H), 1.46 (s, 2H), 1.28 (s, 24H), 0.91 (t, *J =* 6.3 Hz, 3H).

### 4. Preparation of Compound 6004

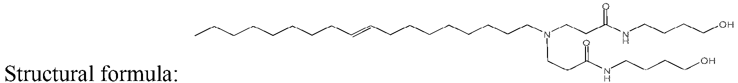

Molecular weight: 553.48

To a reaction flask were added 0.8 g oleylamine, 1.5 g n-butyl acrylate and 5.6 mL n-butanol, followed by stirring and heating to 100°C. After 4 h, 1 mL n-butyl acrylate was supplemented. After further reaction for 1 h, the mixture was subjected to normal-phase column chromatography (petroleum ether-ethyl acetate system). 0.4 g sodium hydroxide, 1 mL water and 10 mL methanol were then added and stirred for hydrolysis over 30 min. 1 mL concentrated hydrochloric acid and 10 mL methanol were added to the mixture to adjust pH to neutral. The solvent was removed by rotary evaporation, the residue was then dissolved in DCM and dried over anhydrous magnesium sulfate, followed by filtration and rotary evaporation to remove solvent. 10 mL DCM, 1.06 g 4-amino-1-butanol, 0.81 g HOBT and 2.3 g EDCI were added, and the mixture was reacted at room temperature for 18 h. The organic solvent was concentrated, followed by reverse-phase column chromatography and lyophilization to obtain 240 mg Compound 6004. ¹H NMR (400 MHz, CDCl₃) δ 7.58 (d, *J=* 16.6 Hz, 2H), 5.44-5.31 (m, 2H), 3.72 (d, *J=* 16.5 Hz, 4H), 3.31 (d, J = 5.6 Hz, 4H), 3.17 (s, 4H), 2.84 (s, 2H), 2.69 (s, 4H), 2.09-2.01 (m, 4H), 1.67 (d, *J= 2.7* Hz, 10H), 1.37-1.26 (m, 24H), 0.91 (t, *J=* 6.8 Hz, 3H).

### 5. Preparation of Compound 6005

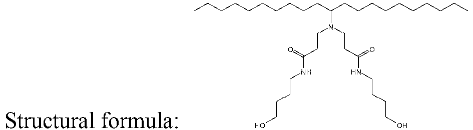

Molecular weight: 597.54

To a reaction flask were added 25.00 g 11-heneicosanone, 62.05 g ammonium acetate and 500 mL methanol, followed by stirring and addition of 6.55 g sodium cyanoborohydride. After reaction for 16 h, 250 mL water and 250 mL DCM were added for liquid separation. The aqueous phase was extracted with 50 mL DCM, and the organic phases were combined, followed by normal-phase column chromatography (methanol-DCM system) to obtain a total of 21.00 g Compound 6005-A.

To a reaction flask were added 21.00 g Compound 6005-A, 58 mL n-butyl acrylate and 100 mL n-butanol, followed by stirring and heating to 100°C. 10 mL n-butyl acrylate was then supplemented, followed by 16 h of reaction and normal-phase column chromatography (petroleum ether-ethyl acetate system) to obtain 31.00 g Compound 6005-B.

To a reaction flask was added a solution prepared from 31.00 g Compound 6005-B, 6.55 g sodium hydroxide, 310 mL methanol and 31 mL water, followed by stirring. After 20 min, 150 mL THF was added, and the mixture was stirred and heated to 50°C. After 30 min, a solution prepared from 16.13 g concentrated hydrochloric acid and 160 mL methanol was added and stirred for 30 min. The solvent was removed by rotary evaporation, followed by dissolution in 300 mL DCM, drying over anhydrous magnesium sulfate, filtration and rotary evaporation to obtain 27.00 g oil Compound 6005-C.

To a reaction flask were added 27.00 g Compound 6005-C, 25.00 g 4-amino-1-butanol, 41.85 g EDCI, 14.74 g HOBT and 310 mL DCM, followed by stirring. After 18 h, the organic solvent was concentrated, followed by normal-phase column chromatography (methanol-DCM system) to remove excess compound of 4-amino-1-butanol and obtain 6.80 g Compound 6005. ¹H NMR (400 MHz, CDCl₃) δ 3.70 (t, *J=* 5.6 Hz, 4H), 3.28 (t, *J=* 5.6 Hz, 4H), 2.74 (t, *J=* 6.0 Hz, 4H), 2.40-2.45 (m, 1H), 2.34 (t, *J =* 6.4 Hz, 4H), 1.63-1.65 (m, 8H), 1.25-1.35 (m, 36H), 0.91 (t, *J=* 6.4 Hz, 6H).

### 6. Preparation of Compound 6006

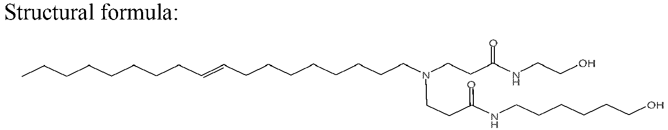

Molecular weight: 553.48

To a reaction flask were added 1.00 g oleylamine, 0.47 g hydroxyethyl acrylamide and 8 mL ethanol, followed by stirring and heating to 70°C. After 13 h, the solvent was removed by rotary evaporation, followed by normal-phase column chromatography (methanol-DCM system) to obtain a total of 800 mg Intermediate 1. 1.5 mL n-butyl acrylate and 5 mL n-butanol were added and stirred, then heated to 100°C. After 3 h, the solvent was removed by rotary evaporation, followed by normal-phase column chromatography (methanol-DCM system). A solution prepared from 0.4 g sodium hydroxide, 10 mL methanol and 1 mL water was added and stirred for 1 h. A solution prepared from 1 mL concentrated hydrochloric acid and 8 mL methanol was added and stirred for 30 min. The solvent was removed by rotary evaporation, followed by dissolution in 100 mL DCM, drying over anhydrous magnesium sulfate, filtration and rotary evaporation. 0.45 g 6-amino-1-hexanol, 0.27 g HOBT, 0.78 g EDCI and 10 mL DCM were added and stirred. After 14 h, the organic solvent was concentrated, followed by normal-phase column chromatography (methanol-DCM system) to obtain a total of 300 mg Compound 6006. ¹H NMR (400 MHz, CDCl₃) δ 7.83 (s, 1H), 6.95 (s, 1H), 5.43-5.28 (m, 2H), 3.74-3.67 (m, 2H), 3.64 (t, *J =* 6.3 Hz, 2H), 3.39 (dd, *J =* 10.0, 5.3 Hz, 2H), 3.24 (dd, *J =* 12.9, 6.7 Hz, 2H), 2.74 (t, *J =* 5.8 Hz, 4H), 2.48-2.42 (m, 2H), 2.41-2.33 (m, 4H), 2.01 (dd, *J =* 13.6, 6.8 Hz, 4H), 1.66-1.17 (m, 34H), 0.89 (t, *J=* 6.7 Hz, 3H).

### 7. Compound 6007

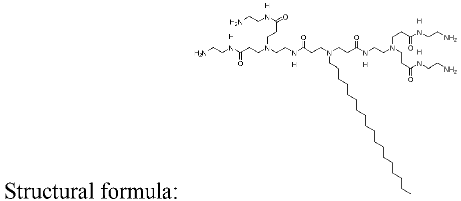

Molecular weight: 953.78

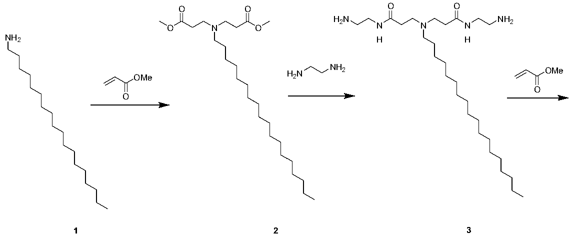

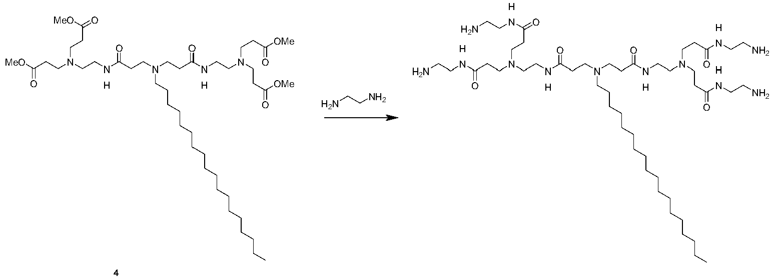

To a three-necked flask were added octadecylamine (50 g) and MeOH (200 mL), followed by mixing and stirring at room temperature. After that, the mixture was cooled to 5°C in an ice-water bath, methyl acrylate (31.5 g) was then slowly added dropwise and mixed with stirring. The system was warmed to room temperature, and the mixture was reacted at room temperature for 4 h. The solvent was concentrated under reduced pressure, and purified by column chromatography purification (PE:EA =20:1-10:1) to obtain Product 2 (68 g). To a single-necked flask were added Product 2 (15.0 g), MeOH (100 mL) and ethylenediamine (1.0 g) at room temperature. The temperature was raised to 60°C and maintained at this temperature for an overnight reaction. The reaction solution was concentrated under reduced pressure, toluene (30 mL) was then added, and the mixture was heated to 60°C for dissolution. After slow cooling down to room temperature, the solvent was evaporated under reduced pressure to obtain Compound 3 (15 g). To a single-necked flask were added Compound 3 (2 g) and MeOH (20 mL), followed by dissolving with stirring at room temperature. After that, methyl acrylate (2.5 g) was then added slowly and mixed with stirring. The mixture was raised to 60°C and maintained at this temperature overnight. After the reaction was terminated, the reaction solution was concentrated under reduced pressure, followed by column chromatography purification (DCM:MeOH =50:1-20:1) to obtain Compound 4 (2.5 g). To a single-necked flask were added Compound 4 (2.3 g) and ethylenediamine (20 mL) at room temperature, and the mixture was maintained at this temperature for an overnight reaction. After the reaction was terminated, the reaction solution was directly concentrated under reduced pressure to obtain Compound 6007 (24 mg). ¹H NMR (400 MHz, Methanol-d4) δ 3.87 (t, J = 6.6 Hz, 2H), 3.55-3.36 (m, 8H), 3.33 (s, 8H), 3.27-3.16 (m, 2H), 3.06-2.90 (m, 4H), 2.77 (t, J = 6.5 Hz,4H), 1.90 (dtdd, J = 21.2, 14.0, 9.5, 6.9 Hz, 4H), 1.73 (p, J = 7.7 Hz, 4H), 1.49 (tdd, J = 9.2, 7.9, 7.1, 4.0 Hz, 4H), 1.31 (s, 28H), 0.92 (t, J = 6.8 Hz, 3H).

### 8. Compound 6008-6018

### Synthesis of Compound 6009

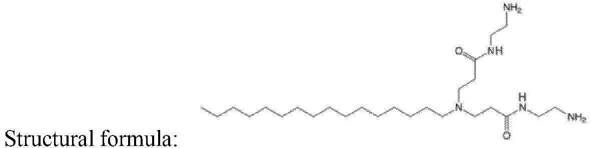

At room temperature and under a nitrogen atmosphere, a methanol (20 mL) solution of freshly recrystallized hexadecylamine (0.03 mol) was added dropwise to a stirred methanol (20 mL) solution containing methyl methacrylate (6 mL), and the mixture was reacted overnight. The reactants were concentrated to dryness by rotary evaporation at room temperature. Then the residue was dissolved in chloroform and washed twice with 0.1 M NaOH solution. The chloroform solution was collected and dried over anhydrous calcium chloride, and separated by column chromatography to obtain a colorless oil. ¹H-NMR (300MHz, CDCl₃): 0.78(t,3H), 1.16(s,30H), 2.38(m,6H), 2.71(t,4H), 3.57(m,6H).

Then a solution of the above colorless oil (11.05 g) in methanol (20 mL) was added to a vigorously stirred methanol (100 mL) solution containing 1,2-ethanediamine (75 g). After complete addition, the mixture was further stirred at room temperature for 24 hours. The solvent was removed under reduced pressure, with the temperature maintained no higher than 40°C. An azeotropic mixture of toluene and methanol (9:1) was used to remove excess 1,2-ethanediamine. Residual toluene was then removed by azeotropic distillation with methanol. A white powder (10.5 g) was finally obtained, which was recrystallized repeatedly with chloroform and cyclohexane to obtain a white solid. ¹H-NMR(300MHz, CDCl₃): 0.88(t,3H), 1.25(s,30H), 1.84(s,4H), 2.38(m,6H), 2.73(m,4H), 2.82(m,4H), 3.29(m,4H), 7.47(s,2H).

The difference between the preparation method of Compound 6008 and that of Compound 6009 lies only in that tetradecylamine in an equimolar amount is used to replace hexadecylamine. Compounds 6010-6017 are prepared with reference to the preparation method of Compound 6009 by replacing hexadecylamine with the corresponding Rₐ-NH₂ in an equimolar amount.

Compound 6018 is prepared with reference to the preparation method of Compound 6007 by replacing octadecylamine with the corresponding Rₐ-NH₂ in an equimolar amount.

### 9. Compound 6019

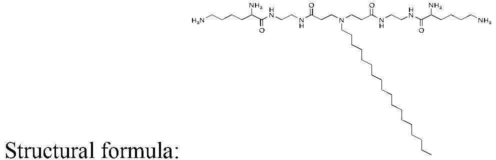

Molecular weight: 753.66

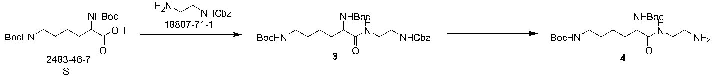

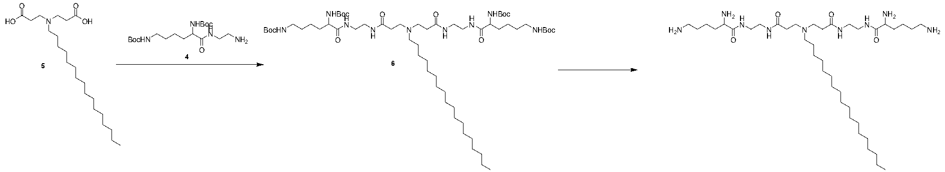

To a single-necked flask were added Compound 2483-46-7 (3 g) and DCM (50 mL), followed by mixing and stirring at room temperature. After that, 18807-71-1 (2.5 g), DCC (2.7 g) and DMAP (1.6 g) were added and mixed with stirring, and the mixture was reacted overnight at room temperature. The mixture was concentrated under reduced pressure and purified by column chromatography (DCM : MeOH = 5:1) to obtain Product 3 (4 g). To a three-necked flask were added Product 3 (5.0 g), MeOH (50 mL) and Pd/C (1.0 g) at room temperature. The system was purged with hydrogen, then the temperature was raised to 50°C and the mixture was stirred for 1 h. After the reaction was terminated, the reaction solution was cooled down to room temperature and filtered. The filtrate was removed by rotary evaporation to obtain Product 4 (3.5 g). To a single-necked flask were added Starting Material 5 (1.2 g) and DMF (50 mL), followed by dissolving with stirring at room temperature. After that, Product 4 (3.3 g), HATU (3.2 g) and TEA (0.9 g) were then added and mixed with stirring, and the mixture was reacted overnight at room temperature. Water (500 mL) and EA (500 mL) were added to conduct liquid separation and extraction. The organic phase was dried over Na₂SO₄ and concentrated under reduced pressure. The crude product was purified by column chromatography (DCM:MeOH = 20:1-10:1) to obtain Solid 6 (380 mg). To a single-necked flask were added Solid 6 (380 mg) and HCl/dioxane (4 M) (1.6 mL) at room temperature. The mixture was reacted for 2 h at room temperature. The mixture was concentrated under reduced pressure and purified to obtain Compound 6019 (220 mg). ¹H NMR (400 MHz, Methanol-d4) δ 3.87 (t, J = 6.6 Hz, 2H), 3.55-3.36 (m, 8H), 3.33 (s, 8H), 3.27-3.16 (m, 2H), 3.06-2.90 (m, 4H), 2.77 (t, J = 6.5 Hz,4H), 1.90 (dtdd, J = 21.2, 14.0, 9.5, 6.9 Hz, 4H), 1.73 (p, J = 7.7 Hz, 4H), 1.49 (tdd, J = 9.2, 7.9, 7.1, 4.0 Hz, 4H), 1.31 (s, 28H), 0.92 (t, J = 6.8 Hz, 3H).

### 10. Compound 6020

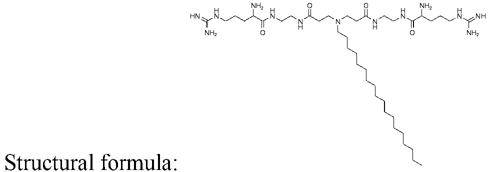

Molecular weight: 810.19

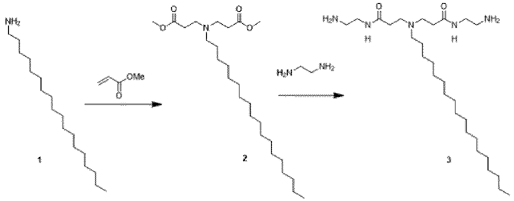

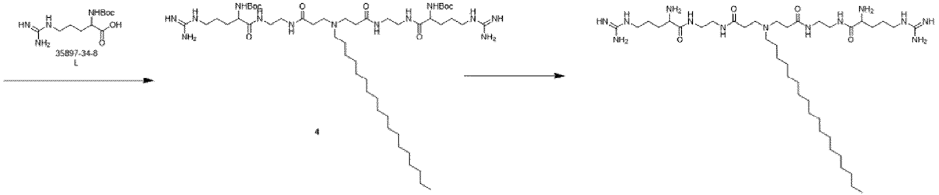

To a three-necked flask were added octadecylamine (50 g) and MeOH (200 mL), followed by mixing with stirring at room temperature. After that, the mixture was cooled down to 5°C in an ice-water bath, methyl acrylate (31.5 g) was then slowly added dropwise and mixed with stirring. The mixture was warmed to room temperature and reacted for 4 h. After the reaction was terminated, the mixture was directly concentrated under reduced pressure and purified by column chromatography (PE:EA = 20:1-10:1) to obtain Product 2 (68 g). To a single-necked flask were added Product 2 (15.0 g), MeOH (100 mL) and ethanediamine (1.0 g) at room temperature. The temperature was raised to 60°C and maintained at this temperature for an overnight reaction. After the reaction was terminated, the reaction solution was concentrated under reduced pressure and toluene (30 mL) was added. The mixture was heated to 60°C for dissolution, then slowly cooled down to room temperature and filtered, followed by evaporation under reduced pressure to obtain Compound 3 (15 g). To a single-necked flask were added Compound 3 (1.3 g) and DMF (20 mL), followed by mixing with stirring at room temperature. After that, 35897-34-8 (3.6 g), EDCI (3.0 g), HOBt (2.1 g) and DIEA (2.0 g) were added and mixed with stirring, and the mixture was reacted overnight at room temperature. Water (200 mL) was added, and the mixture was directly lyophilized under reduced pressure to obtain Compound 4 (4 g). To a single-necked flask were added Compound 4 (100 mg) and HCl/dioxane (4 M) (2 mL) at room temperature, and the mixture was reacted for 1 h at room temperature. After the reaction was terminated, the reaction solution was concentrated under reduced pressure and purified to obtain Compound 6020. ¹H NMR (400 MHz, Methanol-d4) δ 3.90 (t, J = 6.4 Hz, 2H), 3.57-3.34 (m, 10H), 3.24 (dt, J = 16.1, 8.0 Hz, 8H), 2.77 (t, J = 6.5 Hz, 4H), 2.03-1.58 (m, 10H), 1.31 (s, 30H), 1.01-0.82 (m, 3H).

### 11. Compound 6021

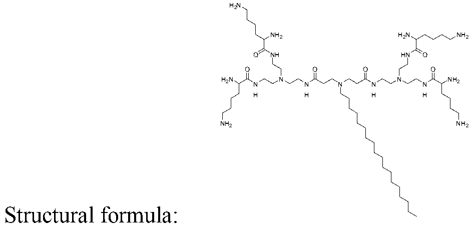

Molecular weight: 1182.79

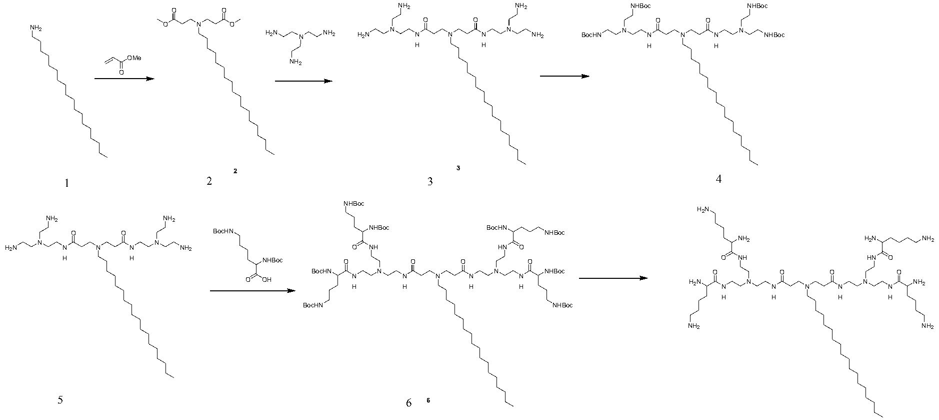

At room temperature, to a single-necked flask were added Compound 2 (3.5 g, prepared by the same synthetic procedure as Product 2 in Compound 6020) and MeOH (20 mL), followed by mixing and stirring. After that, TREN (23.0 g) was added and mixed with stirring. The temperature was raised to 60°C and the mixture was stirred overnight. The reaction solution was concentrated under reduced pressure, and the resulting crude product was lyophilized to obtain Product 3 (25 g). To a single-necked flask were added Product 3 (22.0 g) and DCM (200 mL) at room temperature. The temperature was cooled down to 0°C, then (Boc)₂O (71.7 g) was slowly added dropwise. After dropwise addition, the mixture was warmed to room temperature and reacted for 3 h. Water (200 mL) was added to quench the reaction, followed by DCM (100 mL) for liquid separation and extraction. The organic phase was dried over sodium sulfate and concentrated under reduced pressure, then purified by column chromatography to obtain yellow oil 4 (5.8 g). To a single-necked flask was added yellow oil 4 (1.4 g) at room temperature. Under an ice-water bath, HCl/dioxane (4 M) (15 mL) was slowly added and mixed with stirring. After addition, the mixture was warmed to room temperature and reacted for 0.5 h. The mixture was concentrated under reduced pressure to obtain Compound 5 (800 mg). To a single-necked flask were added Compound 5 (1.0 g), (S)-2,6-bis-tert-butoxycarbonylaminohexanoic acid (2.6 g), EDCI (1.4 g), HOBt (1.0 g), DIEA (1.0 g) and solvent DMF (10 mL) at room temperature, and the mixture was reacted overnight at room temperature. Water (100 mL) and EA (100 mL) were added to conduct liquid separation and extraction. The EA phase was washed once with 100 mL saturated brine and concentrated under reduced pressure, then purified by column chromatography (DCM:MeOH=10:1), to obtain Compound 6 (1.1 g). To a single-necked flask were added Compound 6 (1.2 g), HCl/Dioxane (4M) (10 mL) and solvent DCM (10 mL) at room temperature, and the mixture was reacted overnight at room temperature. The mixture was concentrated under reduced pressure and purified to obtain Compound 6021 (220 mg). ¹H NMR (400 MHz, Methanol-d4) δ 3.96 (t, J = 6.6 Hz, 4H), 3.75 (dt, J = 14.0, 6.6 Hz, 4H), 3.62-3.45 (m, 12H), 3.36-3.33 (m, 14H), 3.25-3.17 (m, 2H), 3.04-2.90 (m, 8H), 2.83 (t, J = 6.9 Hz, 4H), 2.05-1.82 (m, 8H), 1.73 (p, J = 7.7 Hz, 10H), 1.53 (qd, J = 8.3, 7.7, 4.3 Hz, 8H), 1.30 (s, 28H), 0.97-0.87 (m, 3H).

### 12. Compound 6023

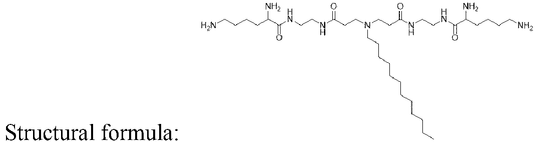

Molecular weight: 670

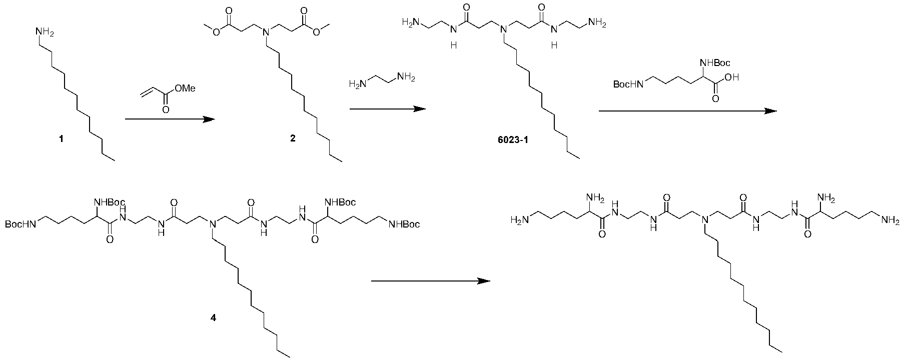

At room temperature, to a three-necked flask were added dodecylamine (10 g) and MeOH (100 mL), followed by mixing and stirring. After that, the temperature was cooled down to 5°C in an ice-water bath, methyl acrylate (10.2 g) was then slowly added dropwise and mixed with stirring. The mixture was warmed to room temperature, and the reaction solution was reacted at room temperature for 4 h. After the reaction was terminated, the mixture was concentrated under reduced pressure and purified by column chromatography (PE:EA =20:1-10:1) to obtain Product 2 (19 g). To a single-necked flask were added Product 2 (19.0 g), MeOH (200 mL) and ethanediamine (127.7 g) at room temperature. The temperature was raised to 60°C, and the mixture was maintained at this temperature for an overnight reaction. The reaction solution was concentrated under reduced pressure to obtain Compound 3 (20 g). A portion of 500 mg was taken for purification and lyophilized to obtain Compound 6023-1 (218 mg). At room temperature, to a single-necked flask were added Starting Material 6023-1 (1.4 g) and DCM (20 mL), followed by dissolving with stirring. After that, (S)-2,6-bis-tert-butoxycarbonylaminohexanoic acid (3.4 g), EDCI (1.9 g) and DMAP (1.2 g) were added and mixed with stirring, and the mixture was maintained at room temperature for an overnight reaction, followed by liquid separation and extraction, drying over sodium sulfate and concentration under reduced pressure to obtain Compound 4 (3.0 g). To a single-necked flask were added Compound 4 (2.8 g) and HCl/1,4-Dioxane (4M) (20 mL) at room temperature, and the mixture was reacted for 2 h at room temperature. The mixture was concentrated under reduced pressure, purified and lyophilized to obtain Compound 6023 (123 mg). ¹H NMR (400 MHz, Methanol-d4) δ 3.88 (t, J = 6.6 Hz, 2H), 3.55-3.36 (m, 8H), 3.36-3.33 (m, 2H), 3.31-3.14 (m, 4H), 3.04-2.93 (m, 4H),2.77 (t, J = 6.5 Hz, 4H), 2.00-1.82 (m, 4H), 1.82-1.66 (m, 6H), 1.50 (qd, J = 8.2, 7.8, 4.2 Hz, 4H), 1.45-1.25 (m, 18H), 0.96-0.87 (m, 3H).

### 13. Compound 6024

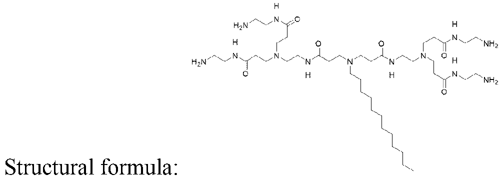

Molecular weight: 870.24

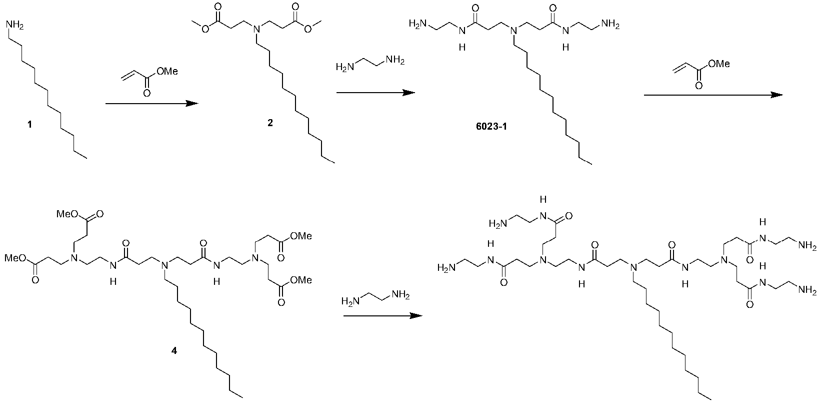

At room temperature, to a single-necked flask were added Product 6023-1 (1.4 g, prepared by the same synthetic method as 6023-1 in Compound 6023) and MeOH (20 mL), followed by dissolving with stirring. After that, methyl acrylate (2.8 g) was slowly added and mixed with stirring, the temperature was raised to 60°C, and the mixture was maintained at this temperature and reacted overnight. The reaction solution was concentrated under reduced pressure to obtain Compound 4 (2.0 g). To a single-necked flask were added Compound 4 (2.4 g) and ethylenediamine (20 mL) at room temperature, and the mixture was reacted overnight at room temperature. The mixture was concentrated under reduced pressure and purified to obtain Compound 6024 (191 mg). ¹H NMR (400 MHz, Methanol-d4) δ 3.69 (t, J = 6.0 Hz, 4H), 3.60-3.48 (m, 20H), 3.42 (t, J = 6.1 Hz, 4H), 3.25-3.19 (m, 2H),3.12 (t, J = 5.8 Hz, 8H), 2.83 (t, J = 6.5 Hz, 12H), 1.78 (tt, J = 11.0, 6.4 Hz, 2H), 1.51-1.18 (m, 20H), 0.91 (t, J = 6.7 Hz, 3H).

### 14. Compound 6026

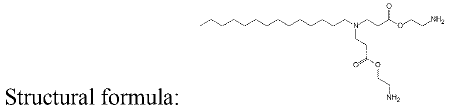

Molecular weight: 870.24

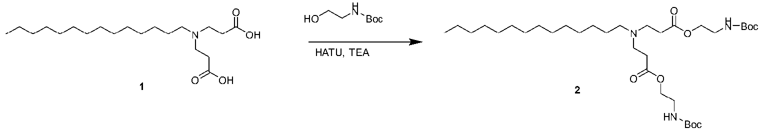

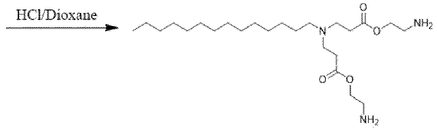

Compound 6026 are synthesized with reference to the synthetic route of Compound 6019, with the only differences as follows: an equimolar amount of Compound 1 is used to replace Starting Material 5 of Compound 6019, and an equimolar amount of N-(tert-butoxycarbonyl)ethanolamine is used to replace Intermediate 4 of Compound 6019. ¹H NMR (300 MHz, DMSO) δ: 4.51 (t, J = 7.3 Hz, 4H), 3.76 (t, J = 6.1 Hz, 4H), 3.18 (t, J = 6.7 Hz, 4H), 3.01 (t, J = 5.8 Hz, 2H), 2.49 (t, J = 7.1 Hz, 4H), 1.36 -1.26 (m, 24H), 0.89 (t, J = 6.2 Hz, 3H).

Compounds 6025-6034 are obtained with reference to the synthetic routes of the compounds described above. The difference lies only in that ethanolamine is used to replace the corresponding ethylenediamine, and the amino group of ethanolamine is first protected with a protecting group such as Fmoc, Boc and the like. The protecting group is removed by a conventional method after the completion of the reaction.

### (3) Exemplary Compounds of First Ionizable Lipids and Properties Thereof

The exemplary compounds of the present application and properties thereof are listed in Table 1, each of which is synthesized according to the process route described in Preparation Example 1.

The calculated c-pKa (molnetwork) and LogP (cLogP driver) values of the exemplary compounds of the present application are generated via the ChemDraw module of ChemOffice.

**Table 1 Properties of Exemplary Compounds of the First Ionizable Lipid**

| No. | Molecular Formula | Molecular Weight | c-pKa | cLog P | No. | Molecular Formula | Molecular Weight | c-pK a | cLog P |
|---|---|---|---|---|---|---|---|---|---|
| 1001 | C₅₉H₁₀₈N₂O₇ | 957.52 | 9.83 | 21.43 | 1201 | C₇₁H₁₄₈N₆O | 1102.01 | 9.01 | 28.48 |
| 1002 | C₆₀H₁₁₆N₂O₇ | 977.60 | 6.97 | 24.49 | 1202 | C₉₂H₁₉₀N₆O | 1396.57 | 9.01 | 39.59 |
| 1003 | C₆₃H₁₂₂N₂O₇ | 1019.68 | 6.97 | 26.07 | 1203 | C₆₅H₁₃₆N₆O | 1017.84 | 9.01 | 25.31 |
| 1004 | C₅₄H₁₀₄N₂O₇ | 893.43 | 6.97 | 21.97 | 1204 | C₆₅H₁₃₆N₆O | 1017.84 | 9.01 | 25.31 |
| 1005 | C₆₈H₁₃₃N₃O₇ | 1104.83 | 9.01 | 26.86 | 1205 | C₉₈H₂₀₂N₆O | 1480.73 | 9.01 | 43.03 |
| 1006 | C₅₃ H₁₀₃N₃O₇ | 894.42 | 9.01 | 19.58 | 1206 | C₉₂H₁₉₀N₆O | 1396.57 | 9.01 | 39.86 |
| 1007 | C₆₈H₁₃₃N₃O₇ | 1104.83 | 9.01 | 26.86 | 1207 | C₈₃H₁₇₂N₆O | 1270.33 | 9.01 | 35.10 |
| 1008 | C₆₈H₁₃₃N₃O₇ | 1104.01 | 9.01 | 26.86 | 1208 | C₆₅H₁₃₀N₆O | 1011.80 | 9.01 | 24.06 |
| 1009 | C₆₂H₁₂₁N₃O₇ | 1020.66 | 9.01 | 23.69 | 1209 | C₆₅H₁₃₀N₆O | 1011.80 | 9.01 | 23.97 |
| 1010 | C₆₇H₁₃₃N₃O₆ | 1076.82 | 9.78 | 27.13 | 1210 | C₄₄H₉₄N₆O | 723.28 | 9.01 | 14.86 |
| 1011 | C₇₀H₁₃₇N₃O₇ | 1132.88 | 9.07 | 27.92 | 1211 | C₅₆H₁₀₆N₆O | 879.50 | 9.01 | 18.30 |
| 1012 | C₇₂H₁₄₁N₃O₇ | 1160.93 | 9.10 | 28.98 | 1212 | C₆₂H₁₁₈N₆O | 963.67 | 9.01 | 20.30 |
| 1013 | C₇₄H₁₄₅N₃O₇ | 1188.99 | 9.12 | 30.03 | 1213 | C₅₀H₁₀₀N₆O | 801.39 | 9.01 | 16.58 |
| 1014 | C₇₀H₁₃₇N₃O₉ | 1164.88 | 7.40 | 26.03 | 1214 | C₆₂H₁₂₄N₆O | 969.71 | 9.01 | 22.93 |
| 1015 | C₇₂H₁₄₁N₃O₉ | 1192.93 | 8.33 | 26.61 | 1215 | C₆₂H₁₃₀N₆O | 975.76 | 9.01 | 24.38 |
| 1016 | C₆₅H₁₂₉N₃O₈ | 1080.80 | 9.11 | 23.70 | 1216 | C₆₈H₁₁₈N₆O | 1035.73 | 9.01 | 25.13 |
| 1017 | C₇₁H₁₄₁N₃O₈ | 1164.90 | 9.10 | 26.87 | 1217 | C₈₃H₁₄₈N₆O | 1246.14 | 9.01 | 32.83 |
| 1018 | C₇₂H₁₄₀N₂O₁₀ | 1193.92 | 8.15 | 27.21 | 1218 | C₅₉H₁₂₁N₃OS₆ | 1081.00 | 9.01 | 27.15 |
| 1019 | C₇₂H₁₄₀N₂O₁₀ | 1193.92 | 8.15 | 27.21 | 1219 | C₅₉H₁₂₁N₃OS₆ | 1081.00 | 9.01 | 27.15 |
| 1020 | C₇₄H₁₄₅N₃O₉ | 1220.99 | 8.74 | 26.06 | 1220 | C₇₁H₁₄₅N₃OS₆ | 1249.32 | 9.01 | 33.50 |
| 1021 | C₇₄H₁₄₅N₃O₉ | 1221.00 | 8.74 | 26.06 | 1221 | C₉₂H₁₈₇N₃OS₆ | 1543.89 | 9.01 | 44.61 |
| 1022 | C₇₃H₁₄₅N₃O₈ | 1193.00 | 9.51 | 26.33 | 1222 | C₆₅H₁₃₃N₃OS₆ | 1165.16 | 9.01 | 30.32 |
| 1023 | C₇₄H₁₄₄N₂O₁₀ | 1221.97 | 8.55 | 26.66 | 1223 | C₁₀₀H₁₉₈N₄O₁₀S₂ | 1680.82 | 8.90 | 43.24 |
| 1024 | C₇₄H₁₄₄N₂O₁₀ | 1221.97 | 8.55 | 26.66 | 1224 | C₉₈H₁₉₉N₃OS₆ | 1628.05 | 9.01 | 48.05 |
| 1025 | C₆₉H₁₃₅N₃O₈ | 1134.85 | 8.20 | 26.44 | 1225 | C₉₂H₁₈₇N₃OS₆ | 1543.89 | 9.01 | 44.88 |
| 1026 | C₆₉H₁₃₄N₂O₉ | 1135.84 | 8.02 | 27.05 | 1226 | C₈₃H₁₆₉N₃OS₆ | 1417.64 | 9.01 | 40.11 |
| 1027 | C₆₉H₁₃₄N₂O₉ | 1135.84 | 8.02 | 27.05 | 1227 | C₆₅H₁₂₇N₃OS₆ | 1159.11 | 9.01 | 29.08 |
| 1028 | C₆₄H₁₂₅N₃O₇ | 1048.70 | 9.07 | 24.74 | 1228 | C₆₅H₁₂₇N₃OS₆ | 1159.11 | 9.01 | 28.99 |
| 1029 | C₆₈H₁₃₂N₂O₃ | 1105.81 | 8.83 | 27.46 | 1229 | C₄₄H₉₁N₃OS₆ | 870.59 | 9.01 | 19.87 |
| 1030 | C₆₆H₁₂₉N₃O₇ | 1076.80 | 9.10 | 25.80 | 1230 | C₅₆H₁₀₃N₃OS₆ | 1026.82 | 9.01 | 23.32 |
| 1031 | C₆₅H₁₂₉N₃O₆ | 1048.80 | 9.86 | 26.07 | 1231 | C₆₂H₁₁₅N₃OS₆ | 1110.98 | 9.01 | 25.32 |
| 1032 | C₆₉H₁₃₅N₃O₇ | 1118.90 | 9.10 | 27.39 | 1232 | C₅₀H₉₇N₃OS₆ | 948.71 | 9.01 | 21.60 |
| 1033 | C₆₆H₁₂₈N₂O₈ | 1077.80 | 8.91 | 26.41 | 1233 | C₆₂H₁₂₁N₃OS₆ | 1117.03 | 9.01 | 27.94 |
| 1034 | C₆₈H₁₃₃N₃O₇ | 1104.83 | 9.12 | 26.86 | 1234 | C₆₂H₁₂₇N₃OS₆ | 1123.08 | 9.01 | 29.40 |
| 1035 | C₇₁H₁₃₉N₃O₇ | 1146.91 | 9.12 | 28.45 | 1235 | C₆₈H₁₁₅N₃OS₆ | 1183.05 | 9.01 | 27.86 |
| 1036 | C₆₈H₁₃₂N₂O₈ | 1105.80 | 8.93 | 27.46 | 1236 | C₈₃H₁₄₅N₃OS₆ | 1393.45 | 9.01 | 35.56 |
| 1037 | C₆₄H₁₂₅N₃O₉ | 1080.70 | 7.40 | 22.85 | 1237 | C₆₂H₁₂₁N₃O₄ | 972.67 | 9.01 | 23.48 |
| 1038 | C₆₇H₁₃₁N₃O₉ | 1122.80 | 7.40 | 24.44 | 1238 | C₇₄H₁₄₅N₃O₄ | 1140.99 | 9.01 | 29.83 |
| 1039 | C₆₄H₁₂₄N₂O₁₀ | 1081.70 | 7.21 | 23.46 | 1239 | C₉₅H₁₈₇N₃O₄ | 1435.56 | 9.01 | 40.94 |
| 1040 | C₆₉H₁₃₅N₃O₉ | 1150.90 | 8.33 | 25.02 | 1240 | C₆₈H₁₃₃N₃O₄ | 1056.83 | 9.01 | 26.65 |
| 1041 | C₆₆H₁₂₈N₂O₁₀ | 1109.80 | 8.15 | 24.04 | 1241 | C₆₈H₁₃₃N₃O₄ | 1056.83 | 9.01 | 26.65 |
| 1042 | C₆₈H₁₃₂N₂O₁₀ | 1137.80 | 8.55 | 23.49 | 1242 | C₁₀₁H₁₉₉N₃O₄ | 1519.72 | 9.01 | 43.72 |
| 1043 | C₇₀H₁₃₈N₄O₈ | 1163.89 | 9.40 | 25.69 | 1243 | C₉₅H₁₈₇N₃O₄ | 1435.56 | 9.01 | 40.55 |
| 1044 | C₆₄H₁₂₆N₄O₈ | 1079.73 | 9.40 | 22.52 | 1244 | C₈₆H₁₆₉N₃O₄ | 1309.32 | 9.01 | 35.79 |
| 1045 | C₇₀H₁₃₈N₄O₈ | 1163.89 | 9.40 | 25.69 | 1245 | C₆₈H₁₂₇N₃O₄ | 1050.78 | 9.01 | 24.75 |
| 1046 | C₇₀H₁₃₈N₄O₈ | 1163.89 | 9.40 | 25.69 | 1246 | C₆₈H₁₂₇N₃O₄ | 1050.78 | 9.01 | 24.66 |
| 1047 | C₆₇H₁₃₁N₃O₈ | 1106.80 | 8.95 | 25.14 | 1247 | C₄₇H₉₁N₃O₄ | 762.26 | 9.01 | 15.54 |
| 1048 | C₆₁H₁₁₉N₃O₈ | 1022.64 | 8.95 | 21.96 | 1248 | C₅₉H₁₀₃N₃O₄ | 918.49 | 9.01 | 18.99 |
| 1049 | C₆₇H₁₃₁N₃O₈ | 1106.80 | 8.95 | 25.14 | 1249 | C₆₅H₁₁₅N₃O₄ | 1002.65 | 9.01 | 20.99 |
| 1050 | C₇₂H₁₃₉NO₉ | 1162.90 | 9.10 | 28.52 | 1250 | C₅₃H₉₇N₃O₄ | 840.38 | 9.01 | 17.27 |
| 1051 | C₇₄H₁₃₉N₃O₁₃ | 1278.90 | 9.01 | 23.74 | 1251 | C₆₆H₁₂₁N₃O₄ | 1008.70 | 9.01 | 23.61 |
| 1052 | C₇₇H₁₅₀N₂O₇ | 1216.10 | 9.83 | 32.01 | 1252 | C₆₅H₁₂₇N₃O₄ | 1014.75 | 9.01 | 25.07 |
| 1053 | C₇₃H₁₃₇N₂O₆P | 1169.88 | 9.68 | 25.77 | 1253 | C₇₁H₁₁₅N₃O₄ | 1074.72 | 9.01 | 25.00 |
| 1054 | C₇₄H₁₃₈N₃O₇P | 1212.91 | 9.95 | 26.18 | 1254 | C₈₆H₁₄₅N₃O₄ | 1285.12 | 9.01 | 32.69 |
| 1055 | C₇₁H₁₃₆N₃O₃P S₉ | 1399.40 | 8.85 | 32.41 | 1255 | C₉₉H₁₉₅N₃O₃S₄ | 1603.91 | 8.68 | 43.96 |
| 1056 | C₆₈H₁₃₈N₂O₅ | 1063.90 | 9.70 | 26.03 | 1256 | C₇₄H₁₄₅N₃O₇ | 1188.99 | 9.01 | 30.03 |
| 1057 | C₇₀H₁₄₀N₄O₆S₂ | 1198.03 | 9.67 | 24.35 | 1257 | C₉₅H₁₈₇N₃O₇ | 1483.55 | 9.01 | 41.14 |
| 1058 | C₆₇H₁₃₂N₂O₇ | 1077.80 | 9.45 | 27.29 | 1258 | C₁₀₄H₂₀₀N₃O₇PS ₄ | 1763.97 | 7.94 | 41.36 |
| 1059 | C₆₇H₁₃₂N₂O₇ | 1077.80 | 9.45 | 27.29 | 1259 | C₁₀₃H₂₀₀N₂OS₇ | 1707.17 | 10.0 9 | 46.14 |
| 1060 | C₆₇H₁₃₃N₃O₈S | 1140.87 | 8.74 | 26.96 | 1260 | C₁₀₁H₁₉₉N₃O₇ | 1567.72 | 9.01 | 44.59 |
| 1061 | C₇₃H₁₂₅N₃O₅ | 1124.82 | 9.77 | 23.41 | 1261 | C₉₅H₁₈₇N₃O₇ | 1483.55 | 9.01 | 41.41 |
| 1062 | C₆₇H₁₁₃N₃O₁₀S ₃ | 1216.83 | 9.73 | 22.69 | 1262 | C₈₆H₁₆₉N₃O₇ | 1357.31 | 9.01 | 36.65 |
| 1063 | C₆₅H₁₀₉N₃O₁₀S ₃ | 1188.78 | 9.01 | 21.48 | 1263 | C₆₈H₁₂₇N₃O₇ | 1098.78 | 9.01 | 25.62 |
| 1064 | C₆₈H₁₃₉N₃OS₆ | 1207.24 | 9.01 | 32.18 | 1264 | C₆₈H₁₂₇N₃O₇ | 1098.78 | 9.01 | 25.53 |
| 1065 | C₆₈H₁₃₉N₃OS₆ | 1207.24 | 9.01 | 32.18 | 1265 | C₄₇H₉₁N₃O₇ | 810.26 | 9.01 | 16.41 |
| 1066 | C₇₄H₁₅₄N₃O₁₃P ₃ | 1386.98 | 9.01 | 26.51 | 1266 | C₅₉H₁₀₃N₃O₇ | 966.49 | 9.01 | 19.85 |
| 1067 | C₇₄H₁₅₄N₃O₁₃P ₃ | 1386.98 | 9.01 | 26.51 | 1267 | C₆₅H₁₁₅N₃O₇ | 1050.65 | 9.01 | 21.86 |
| 1068 | C₇₁H₁₃₁N₃O₇ | 1138.84 | 5.07 | 26.81 | 1268 | C₅₃H₉₇N₃O₇ | 888.37 | 9.01 | 18.13 |
| 1069 | C₇₁H₁₃₁N₃O₇ | 1138.84 | 5.04 | 26.81 | 1269 | C₆₅H₁₂₁N₃O₇ | 1056.70 | 9.01 | 24.48 |
| 1070 | C₆₉H₁₃₀N₄O₇ | 1127.82 | 5.03 | 26.38 | 1270 | C₆₅H₁₂₇N₃O₇ | 1062.74 | 9.01 | 25.93 |
| 1071 | C₆₉H₁₃₀N₄O₇ | 1127.82 | 5.00 | 26.38 | 1271 | C₇₁H₁₁₅N₃O₄ | 1122.71 | 9.01 | 26.17 |
| 1072 | C₇₀H₁₃₅N₃O₈ | 1146.86 | 6.83 | 26.82 | 1272 | C₈₆H₁₄₅N₃O₇ | 1333.12 | 9.01 | 33.87 |
| 1073 | C₇₀H₁₃₅N₃O₈ | 1146.86 | 6.83 | 26.82 | 1273 | C₆₂H₁₂₁N₃O₇ | 1020.66 | 9.01 | 23.69 |
| 1074 | C₇₁H₁₃₈N₄O₈ | 1175.91 | 7.89 | 26.41 | 1274 | C₇₄H₁₄₅N₃O₇ | 1188.99 | 9.01 | 30.03 |
| 1075 | C₇₁H₁₃₈N₄O₈ | 1175.91 | 7.89 | 26.41 | 1275 | C₉₅H₁₈₇N₃O₇ | 1483.55 | 9.01 | 41.14 |
| 1076 | C₇₁H₁₃₈N₄O₉ | 1191.90 | 7.80 | 26.25 | 1276 | C₆₈H₁₃₃N₃O₇ | 1104.83 | 9.01 | 26.86 |
| 1077 | C₇₁H₁₃₈N₄O₉ | 1191.90 | 7.80 | 26.25 | 1277 | C₆₈H₁₃₃N₃O₇ | 1104.83 | 9.01 | 26.86 |
| 1078 | C₇₁H₁₃₈N₄O₉ | 1191.90 | 7.75 | 27.09 | 1278 | C₁₀₁H₁₉₉N₃O₇ | 1567.72 | 9.01 | 44.59 |
| 1079 | C₇₁H₁₃₈N₄O₉ | 1191.90 | 7.75 | 27.09 | 1279 | C₉₅H₁₈₇N₃O₇ | 1483.55 | 9.01 | 41.41 |
| 1080 | C₇₀H₁₃₈N₄O₉S | 1211.95 | 7.61 | 26.56 | 1280 | C₈₆H₁₆₉N₃O₇ | 1357.31 | 9.01 | 36.65 |
| 1081 | C₇₀H₁₃₈N₄O₉S | 1211.95 | 7.61 | 26.56 | 1281 | C₆₈H₁₂₇N₃O₇ | 1098.78 | 9.01 | 25.62 |
| 1082 | C₇₂H₁₃₉N₅O₈ | 1202.93 | 7.82 | 26.46 | 1282 | C₆₈H₁₂₇N₃O₇ | 1098.78 | 9.01 | 25.53 |
| 1083 | C₇₂H₁₃₉N₅O₈ | 1202.93 | 7.82 | 26.46 | 1283 | C₄₇H₉₁N₃O₇ | 810.26 | 9.01 | 16.41 |
| 1084 | C₇₂H₁₃₇N₅O₇S | 1216.98 | 7.95 | 28.08 | 1284 | C₅₉H₁₀₃N₃O₇ | 966.49 | 9.01 | 19.85 |
| 1085 | C₇₂H₁₃₇N₅O₇S | 1216.98 | 7.95 | 28.08 | 1285 | C₆₅H₁₁₅N₃O₇ | 1050.65 | 9.01 | 21.86 |
| 1086 | C₇₄H₁₃₈N₈O₇ | 1251.97 | 5.88 | 25.00 | 1286 | C₅₃H₉₇N₃O₇ | 888.37 | 9.01 | 18.13 |
| 1087 | C₇₄H₁₃₈N₈O₇ | 1251.97 | 5.88 | 25.00 | 1287 | C₆₅H₁₂₁N₃O₇ | 1056.70 | 9.01 | 24.48 |
| 1088 | C₇₄H₁₃₈N₈O₈ | 1267.97 | 7.59 | 25.58 | 1288 | C₆₅H₁₂₇N₃O₇ | 1062.74 | 9.01 | 25.93 |
| 1089 | C₇₄H₁₃₈N₈O₈ | 1267.97 | 7.59 | 25.58 | 1289 | C₇₁H₁₁₅N₃O₄ | 1122.71 | 9.01 | 24.80 |
| 1090 | C₇₃H₁₃₇N₅O₉ | 1228.93 | 7.07 | 26.15 | 1290 | C₈₆H₁₄₅N₃O₇ | 1333.12 | 9.01 | 32.49 |
| 1091 | C₇₃H₁₃₇N₅O₉ | 1228.93 | 7.07 | 26.15 | 1291 | C₆₂H₁₂₁N₃O₁₀ | 1068.66 | 9.01 | 23.51 |
| 1092 | C₇₄H₁₃₉N₅O₈ | 1226.95 | 7.47 | 26.45 | 1292 | C₇₄H₁₄₅N₃O₁₀ | 1236.98 | 9.01 | 29.86 |
| 1093 | C₇₄H₁₃₉N₅O₈ | 1226.95 | 7.47 | 26.45 | 1293 | C₉₅H₁₈₇N₃O₁₀ | 1531.55 | 9.01 | 40.96 |
| 1094 | C₇₀H₁₃₈N₆O₇ | 1175.91 | 8.12 | 25.90 | 1294 | C₆₈H₁₃₃N₃O₁₀ | 1152.82 | 9.01 | 26.68 |
| 1095 | C₇₀H₁₃₈N₆O₇ | 1175.91 | 8.12 | 25.90 | 1295 | C₆₈H₁₃₃N₃O₁₀ | 1152.82 | 9.01 | 26.68 |
| 1096 | C₇₂H₁₄₂N₆O₉S | 1268.02 | 7.63 | 26.40 | 1296 | C₁₀₁H₁₉₉N₃O₁₀ | 1615.71 | 9.01 | 44.41 |
| 1097 | C₇₂H₁₄₂N₆O₉S | 1268.02 | 7.63 | 26.40 | 1297 | C₉₅H₁₈₇N₃O₁₀ | 1531.55 | 9.01 | 41.23 |
| 1098 | C₇₃H₁₄₃N₅O₉S | 1267.03 | 7.51 | 27.09 | 1298 | C₈₆H₁₆₉N₃O₁₀ | 1405.31 | 9.01 | 36.47 |
| 1099 | C₇₃H₁₄₃N₅O₉S | 1267.03 | 7.51 | 27.09 | 1299 | C₆₈H₁₂₇N₃O₁₀ | 1146.77 | 9.01 | 25.44 |
| 1100 | C₇₄H₁₄₅N₅O₉S | 1281.06 | 8.31 | 27.25 | 1300 | C₆₈H₁₂₇N₃O₁₀ | 1146.77 | 9.01 | 25.35 |
| 1101 | C₇₃H₁₄₃N₅O₉S | 1267.03 | 7.51 | 27.09 | 1301 | C₄₇H₉₁N₃O₁₀ | 858.26 | 9.01 | 16.23 |
| 1102 | C₇₂H₁₃₉N₃O₇ | 1158.92 | 10.26 | 27.41 | 1302 | C₅₉H₁₀₃N₃O₁₀ | 1014.48 | 9.01 | 19.68 |
| 1103 | C₇₁H₁₃₈N₄O₇ | 1159.91 | 9.78 | 26.04 | 1303 | C₆₅H₁₁₅N₃O₁₀ | 1098.65 | 9.01 | 21.68 |
| 1104 | C₆₉H₁₃₃N₃O₇ | 1116.84 | 9.32 | 25.52 | 1304 | C₅₃H₉₇N₃O₁₀ | 936.37 | 9.01 | 17.95 |
| 1105 | C₆₉H₁₃₃N₃O₇ | 1116.84 | 9.32 | 25.52 | 1305 | C₆₅H₁₂₁N₃O₁₀ | 1104.69 | 9.01 | 24.30 |
| 1106 | C₆₉H₁₃₆N₆O₇ | 1161.88 | 7.46 | 24.33 | 1306 | C₆₅H₁₂₇N₃O₁₀ | 1110.74 | 9.01 | 25.75 |
| 1107 | C₆₃H₁₂₄N₆O₇ | 1077.72 | 7.46 | 21.15 | 1307 | C₇₁H₁₁₅N₃O₁₀ | 1170.71 | 9.01 | 24.31 |
| 1108 | C₆₉H₁₃₆N₆O₇ | 1161.88 | 7.46 | 24.33 | 1308 | C₈₆H₁₄₅N₃O₁₀ | 1381.12 | 9.01 | 32.00 |
| 1109 | C₆₉H₁₃₆N₆O₇ | 1161.88 | 7.46 | 24.33 | 1309 | C₆₂H₁₂₄N₆O₄ | 1017.71 | 9.01 | 20.89 |
| 1110 | C₇₀H₁₃₆N₄O₇ | 1145.88 | 8.82 | 26.41 | 1310 | C₇₄H₁₄₈N₆O₄ | 1186.04 | 9.01 | 27.24 |
| 1111 | C₇₀H₁₃₆N₄O₇ | 1145.88 | 8.82 | 26.41 | 1311 | C₉₅H₁₉₀N₆O₄ | 1480.60 | 9.01 | 38.35 |
| 1112 | C₆₉H₁₃₆N₂O₆ | 1089.85 | 10.19 | 28.16 | 1312 | C₆₈H₁₃₆N₆O₄ | 1101.87 | 9.01 | 24.06 |
| 1113 | C₇₁H₁₃₈N₂O₈ | 1147.89 | 9.69 | 26.58 | 1313 | C₆₈H₁₃₆N₆O₄ | 1101.87 | 9.01 | 24.06 |
| 1114 | C₇₀H₁₃₈N₂O₇ | 1119.88 | 9.84 | 26.71 | 1314 | C₁₀₁H₂₀₂N₆O₄ | 1564.76 | 9.01 | 41.79 |
| 1115 | C₆₇H₁₃₁N₃O₇ | 1090.80 | 9.50 | 26.91 | 1315 | C₉₅H₁₉₀N₆O₄ | 1480.60 | 9.01 | 38.62 |
| 1116 | C₆₉H₁₃₅N₃O₈ | 1134.85 | 9.52 | 27.58 | 1316 | C₈₆H₁₇₂N₆O₄ | 1354.36 | 9.01 | 33.85 |
| 1117 | C₇₁H₁₄₁N₃O₆ | 1132.92 | 9.88 | 29.03 | 1317 | C₆₈H₁₃₀N₆O₄ | 1095.83 | 9.01 | 22.82 |
| 1118 | C₇₁H₁₃₉N₃O₈ | 1162.91 | 8.87 | 26.46 | 1318 | C₆₈H₁₃₀N₆O₄ | 1095.83 | 9.01 | 22.73 |
| 1119 | C₇₁H₁₃₃NO₉S₅ | 1305.14 | 9.96 | 25.12 | 1319 | C₄₇H₉₄N₆O₄ | 807.31 | 9.01 | 13.61 |
| 1120 | C₇₂H₁₄₂N₂O₇ | 1147.90 | 10.05 | 27.76 | 1320 | C₅₉H₁₀₆N₆O₄ | 963.53 | 9.01 | 17.06 |
| 1121 | C₇₃H₁₃₉N₅O₁₅S ₄ | 1455.17 | 7.80 | 23.62 | 1321 | C₅₉H₁₀₆N₆O₄ | 963.53 | 9.01 | 17.06 |
| 1122 | C₆₈H₁₂₉NO₁₃S₃ | 1264.95 | 8.49 | 25.91 | 1322 | C₅₃H₁₀₀N₆O₄ | 885.42 | 9.01 | 15.34 |
| 1123 | C₇₇H₁₅₂N₂O₇ | 1218.07 | 9.99 | 30.68 | 1323 | C₆₅H₁₂₄N₆O₄ | 1053.74 | 9.01 | 21.68 |
| 1124 | C₆₇H₁₃₃N₃O₈S | 1140.87 | 8.74 | 26.96 | 1324 | C₆₅H₁₃₀N₆O₄ | 1059.79 | 9.01 | 23.14 |
| 1125 | C₆₉H₁₃₆N₄O₇ | 1133.87 | 9.61 | 27.37 | 1325 | C₇₁H₁₁₈N₆O₄ | 1119.76 | 9.01 | 23.40 |
| 1126 | C₆₅H₁₂₁N₃O₇ | 1056.70 | 9.01 | 23.82 | 1326 | C₈₆H₁₄₈N₆O₄ | 1330.17 | 9.01 | 31.09 |
| 1127 | C₆₉H₁₃₆N₂O₉ | 1137.85 | 8.31 | 26.05 | 1327 | C₆₂H₁₂₄N₆O₄ | 1017.71 | 9.01 | 20.89 |
| 1128 | C₇₀H₁₃₉N₂O₁₁P | 1215.86 | 7.77 | 26.53 | 1328 | C₇₄H₁₄₈N₆O₄ | 1186.04 | 9.01 | 27.24 |
| 1129 | C₆₈H₁₃₄N₂O₇S₂ | 1155.95 | 8.84 | 27.80 | 1329 | C₉₅H₁₉₀N₆O₄ | 1480.60 | 9.01 | 38.35 |
| 1130 | C₇₄H₁₅₄N₃O₁₃P ₃ | 1386.98 | 9.01 | 26.51 | 1330 | C₆₈H₁₃₆N₆O₄ | 1101.87 | 9.01 | 24.06 |
| 1131 | C₈₄H₁₅₉N₃O₈ | 1339.21 | 9.46 | 31.70 | 1331 | C₆₈H₁₃₆N₆O₄ | 1101.87 | 9.01 | 24.06 |
| 1132 | C₈₇H₁₄₇N₃O₈ | 1363.15 | 9.46 | 32.11 | 1332 | C₁₀₁H₂₀₁N₅O₅ | 1565.75 | 9.01 | 42.72 |
| 1133 | C₇₁H₁₃₁N₃O₇ | 1138.84 | 5.07 | 26.81 | 1333 | C₉₅H₁₈₉N₅O₅ | 1481.59 | 9.01 | 39.55 |
| 1134 | C₆₉H₁₃₀N₄O₇ | 1127.82 | 5.00 | 26.38 | 1334 | C₈₆H₁₇₁N₅O₅ | 1355.34 | 9.01 | 34.79 |
| 1135 | C₇₁H₁₃₈N₄O₈ | 1175.91 | 7.89 | 26.41 | 1335 | C₆₈H₁₃₀N₆O₄ | 1095.83 | 9.01 | 22.82 |
| 1136 | C₁₀₈H₂₀₄N₂O₉S ₂ | 1738.94 | 8.56 | 46.05 | 1336 | C₆₈H₁₂₉N₅O₅ | 1096.81 | 9.01 | 23.66 |
| 1137 | C₉₉H₁₉₅NO₆S₃ | 1591.83 | 7.68 | 42.68 | 1337 | C₄₇H₉₃N₅O₅ | 808.29 | 9.01 | 14.55 |
| 1138 | C₇₂H₁₃₇N₅O₇S | 1216.98 | 7.95 | 28.08 | 1338 | C₅₉H₁₀₅N₅O₅ | 964.52 | 9.01 | 17.99 |
| 1139 | C₇₄H₁₃₈N₈O₇ | 1251.97 | 5.88 | 25.00 | 1339 | C₆₅H₁₁₇N₅O₅ | 1048.68 | 9.01 | 19.99 |
| 1140 | C₇₀H₁₃₈N₆O₇ | 1175.91 | 8.12 | 25.90 | 1340 | C₅₃H₉₉N₅O₅ | 886.40 | 9.01 | 16.27 |
| 1141 | C₇₃H₁₄₃N₅O₉S | 1267.03 | 7.51 | 27.09 | 1341 | C₆₅H₁₂₃N₅O₅ | 1054.73 | 9.01 | 22.62 |
| 1142 | C₇₀H₁₃₅N₃O₇ | 1130.86 | 9.98 | 26.32 | 1342 | C₆₅H₁₂₉N₅O₅ | 1060.78 | 9.01 | 24.07 |
| 1143 | C₆₅H₁₁₅N₃O₇ | 1050.60 | 9.01 | 21.20 | 1343 | C₇₁H₁₁₇N₅O₅ | 1120.75 | 9.01 | 24.31 |
| 1144 | C₇₅H₁₄₁N₃O₈ | 1212.97 | 9.18 | 28.86 | 1344 | C₈₆H₁₄₈N₆O₄ | 1330.17 | 9.01 | 31.76 |
| 1145 | C₇₀H₁₁₈N₂O₁₁S ₃ | 1259.90 | 9.49 | 22.27 | 1345 | C₆₂H₁₂₄N₆O₇ | 1065.71 | 9.01 | 22.93 |
| 1146 | C₆₈H₁₃₄N₂O₇S₂ | 1155.95 | 9.14 | 27.39 | 1346 | C₇₄H₁₄₈N₆O₇ | 1234.03 | 9.01 | 29.28 |
| 1147 | C₈₆H₁₆₇N₃O₅S₂ | 1387.40 | 6.75 | 35.10 | 1347 | C₉₅H₁₉₀N₆O₇ | 1528.60 | 9.01 | 40.39 |
| 1148 | C₆₉H₁₂₀N₆O₂S₄ | 1194.00 | 8.63 | 24.08 | 1348 | C₆₈H₁₃₆N₆O₇ | 1149.87 | 9.01 | 26.10 |
| 1149 | C₇₆H₁₅₄N₆O₃S | 1232.20 | 7.65 | 30.39 | 1349 | C₆₈H₁₃₆N₆O₇ | 1149.87 | 9.01 | 26.10 |
| 1150 | C₉₁H₁₈₆N₄S₆ | 1528.90 | 9.77 | 43.45 | 1350 | C₁₀₂H₂₀₂N₆O₇ | 1612.76 | 9.01 | 43.83 |
| 1151 | C₇₈H₁₄₂N₆O₅ | 1244.00 | NA | 27.09 | 1351 | C₉₅H₁₉₀N₆O₇ | 1528.60 | 9.01 | 40.66 |
| 1152 | C₁₀₅H₁₈₁N₃O₁₂ | 1677.60 | 9.16 | 40.21 | 1352 | C₈₆H₁₇₂N₆O₇ | 1402.36 | 9.01 | 35.90 |
| 1153 | C₆₉H₁₃₁N₅O₅ | 1110.80 | 7.41 | 22.95 | 1353 | C₆₈H₁₃₀N₆O₇ | 1143.82 | 9.01 | 24.86 |
| 1154 | C₇₆H₁₃₉N₃S₃ | 1191.10 | 8.91 | 30.35 | 1354 | C₆₈H₁₃₀N₆O₇ | 1143.82 | 9.01 | 24.77 |
| 1155 | C₆₆H₁₃₂N₅O₁₀P | 1186.80 | 8.65 | 24.15 | 1355 | C₄₇H₉₄N₆O₇ | 855.30 | 9.01 | 15.66 |
| 1156 | C₇₀H₁₂₀N₇O₈P | 1218.70 | 9.55 | 21.96 | 1356 | C₅₉H₁₀₆N₆O₇ | 1011.53 | 9.01 | 19.10 |
| 1157 | C₇₂H₁₃₅N₅O₁₂S ₃ | 1359.08 | 7.99 | 23.39 | 1357 | C₆₅H₁₁₈N₆O₇ | 1095.69 | 9.01 | 21.10 |
| 1158 | C₇₂H₁₃₀N₂O₉ | 1167.84 | 8.05 | 25.65 | 1358 | C₅₃H₁₀₀N₆O₇ | 933.42 | 9.01 | 17.38 |
| 1159 | C₇₁H₁₃₉N₃O₃S₆ | 1275.30 | 9.04 | 31.51 | 1359 | C₆₅H₁₂₄N₆O₇ | 1101.74 | 9.01 | 23.73 |
| 1160 | C₆₉H₁₃₈N₄O₉S₃ | 1264.10 | 7.48 | 28.35 | 1360 | C₆₅H₁₃₀N₆O₇ | 1107.79 | 9.01 | 25.18 |
| 1161 | C₇₃H₁₄₂N₂O₈ | 1175.90 | 9.39 | 27.24 | 1361 | C₇₁H₁₁₈N₆O₇ | 1167.76 | 9.01 | 24.18 |
| 1162 | C₈₅H₁₆₇N₅O₁₀ | 1419.30 | 9.49 | 27.91 | 1362 | C₈₆H₁₄₈N₆O₇ | 1378.16 | 9.01 | 31.87 |
| 1163 | C₅₉H₁₂₁N₃O₄ | 936.63 | 9.01 | 22.86 | 1363 | C₆₂H₁₂₄N₆O₇ | 1065.71 | 9.01 | 22.93 |
| 1164 | C₇₁H₁₄₅N₃O₄ | 1104.96 | 9.01 | 29.21 | 1364 | C₇₄H₁₄₈N₆O₇ | 1234.03 | 9.01 | 29.28 |
| 1165 | C₉₂H₁₈₇N₃O₄ | 1399.53 | 9.01 | 40.32 | 1365 | C₉₅H₁₉₀N₆O₇ | 1528.60 | 9.01 | 40.39 |
| 1166 | C₆₅H₁₃₃N₃O₄ | 1020.80 | 9.01 | 26.04 | 1366 | C₆₈H₁₃₆N₆O₇ | 1149.87 | 9.01 | 26.10 |
| 1167 | C₆₅H₁₃₃N₃O₄ | 1020.80 | 9.01 | 26.04 | 1367 | C₆₈H₁₃₆N₆O₇ | 1149.87 | 9.01 | 26.10 |
| 1168 | C₉₈H₁₉₉N₃O₄ | 1483.69 | 9.01 | 43.77 | 1368 | C₁₀₁H₂₀₂N₆O₇ | 1612.76 | 9.01 | 43.83 |
| 1169 | C₉₂H₁₈₇N₃O₄ | 1399.53 | 9.01 | 40.59 | 1369 | C₉₅H₁₉₀N₆O₇ | 1528.60 | 9.01 | 40.66 |
| 1170 | C₈₃H₁₆₉N₃O₄ | 1273.28 | 9.01 | 35.83 | 1370 | C₈₆H₁₇₂N₆O₇ | 1402.36 | 9.01 | 35.90 |
| 1171 | C₆₅H₁₂₇N₃O₄ | 1014.75 | 9.01 | 24.80 | 1371 | C₆₈H₁₃₀N₆O₇ | 1143.82 | 9.01 | 24.86 |
| 1172 | C₆₅H₁₂₇N₃O₄ | 1014.75 | 9.01 | 24.71 | 1372 | C₆₈H₁₃₀N₆O₇ | 1143.82 | 9.01 | 24.77 |
| 1173 | C₄₄H₉₁N₃O₄ | 726.23 | 9.01 | 15.59 | 1373 | C₄₇H₉₄N₆O₇ | 855.30 | 9.01 | 15.66 |
| 1174 | C₅₆H₁₀₃N₃O₄ | 882.46 | 9.01 | 19.03 | 1374 | C₅₉H₁₀₆N₆O₇ | 1011.53 | 9.01 | 19.10 |
| 1175 | C₆₂H₁₁₅N₃O₄ | 966.62 | 9.01 | 21.04 | 1375 | C₆₅H₁₁₈N₆O₇ | 1095.69 | 9.01 | 21.10 |
| 1176 | C₅₀H₉₇N₃O₄ | 804.34 | 9.01 | 17.31 | 1376 | C₅₃H₁₀₀N₆O₇ | 933.42 | 9.01 | 17.38 |
| 1177 | C₆₂H₁₂₁N₃O₄ | 972.67 | 9.01 | 23.66 | 1377 | C₆₅H₁₂₄N₆O₇ | 1101.74 | 9.01 | 23.73 |
| 1178 | C₆₂H₁₂₇N₃O₄ | 978.71 | 9.01 | 25.11 | 1378 | C₆₅H₁₃₀N₆O₇ | 1107.79 | 9.01 | 25.18 |
| 1179 | C₆₈H₁₁₅N₃O₄ | 1038.68 | 9.01 | 26.06 | 1379 | C₇₁H₁₁₈N₆O₇ | 1167.76 | 9.01 | 25.06 |
| 1180 | C₈₃H₁₄₅N₃O₄ | 1249.09 | 9.01 | 33.76 | 1380 | C₈₆H₁₄₈N₆O₇ | 1378.16 | 9.01 | 32.76 |
| 1181 | C₅₉H₁₂₁N₃OS₃ | 984.82 | 9.01 | 25.15 | 1381 | C₆₂H₁₂₇N₉O₄ | 1062.76 | 9.01 | 22.06 |
| 1182 | C₇₁H₁₄₅N₃OS₃ | 1153.14 | 9.01 | 31.50 | 1382 | C₇₄H₁₅₁N₉O₄ | 1231.08 | 9.01 | 28.41 |
| 1183 | C₉₂H₁₈₇N₃OS₃ | 1447.71 | 9.01 | 42.61 | 1383 | C₉₅H₁₉₃N₉O₄ | 1525.65 | 9.01 | 39.52 |
| 1184 | C₆₅H₁₃₃N₃OS₃ | 1068.98 | 9.01 | 28.32 | 1384 | C₆₈H₁₃₉N₉O₄ | 1146.92 | 9.01 | 25.24 |
| 1185 | C₆₅H₁₃₃N₃OS₃ | 1068.98 | 9.01 | 28.32 | 1385 | C₆₈H₁₃₉N₉O₄ | 1146.92 | 9.01 | 25.24 |
| 1186 | C₉₈H₁₉₉N₃OS₃ | 1531.87 | 9.01 | 46.05 | 1386 | C₁₀₁H₂₀₅N₉O₄ | 1609.81 | 9.01 | 42.97 |
| 1187 | C₉₂H₁₈₇N₃OS₃ | 1447.71 | 9.01 | 42.88 | 1387 | C₉₅H₁₉₃N₉O₄ | 1525.65 | 9.01 | 39.79 |
| 1188 | C₈₃H₁₆₉N₃OS₃ | 1321.46 | 9.01 | 38.12 | 1388 | C₈₆H₁₇₅N₉O₄ | 1399.41 | 9.01 | 35.03 |
| 1189 | C₆₅H₁₂₇N₃OS₃ | 1062.93 | 9.01 | 27.08 | 1389 | C₆₈H₁₃₃N₉O₄ | 1140.87 | 9.01 | 24.00 |
| 1190 | C₆₅H₁₂₇N₃OS₃ | 1062.93 | 9.01 | 26.99 | 1390 | C₆₈H₁₃₃N₉O₄ | 1140.87 | 9.01 | 23.91 |
| 1191 | C₄₄H₉₁N₃OS₃ | 774.41 | 9.01 | 17.88 | 1391 | C₄₇H₉₇N₉O₄ | 852.35 | 9.01 | 14.79 |
| 1192 | C₅₆H₁₀₃N₃OS₃ | 930.64 | 9.01 | 21.32 | 1392 | C₅₉H₁₀₉N₉O₄ | 1008.58 | 9.01 | 18.23 |
| 1193 | C₆₂H₁₁₅N₃OS₃ | 1014.80 | 9.01 | 23.32 | 1393 | C₆₅H₁₂₁N₉O₄ | 1092.74 | 9.01 | 20.24 |
| 1194 | C₅₀H₉₇N₃OS₃ | 852.53 | 9.01 | 19.60 | 1394 | C₅₃H₁₀₃N₉O₄ | 930.47 | 9.01 | 16.51 |
| 1195 | C₆₂H₁₂₁N₃OS₃ | 1020.85 | 9.01 | 25.95 | 1395 | C₆₅H₁₂₇N₉O₄ | 1098.79 | 9.01 | 22.86 |
| 1196 | C₆₂H₁₂₇N₃OS₃ | 1026.90 | 9.01 | 27.40 | 1396 | C₆₅H₁₃₃N₉O₄ | 1104.84 | 9.01 | 24.31 |
| 1197 | C₆₈H₁₁₅N₃OS₃ | 1086.87 | 9.01 | 27.48 | 1397 | C₇₁H₁₂₁N₉O₄ | 1164.81 | 9.01 | 23.93 |
| 1198 | C₈₃H₁₄₅N₃OS₃ | 1297.27 | 9.01 | 35.18 | 1398 | C₈₆H₁₅₁N₉O₄ | 1375.21 | 9.01 | 31.63 |
| 1199 | C₅₉H₁₂₄N₆O | 933.68 | 9.01 | 22.13 | 1399 | C₆₂H₁₂₁N₃OS₃ | 1020.85 | 9.01 | 24.00 |
| 1200 | C₅₉H₁₂₄N₆O | 933.68 | 9.01 | 22.13 | 1400 | C₇₄H₁₄₅N₃OS₃ | 1189.17 | 9.01 | 30.34 |
| 1401 | C₉₅H₁₈₇N₃OS₃ | 1483.74 | 9.01 | 41.45 | 1601 | C₆₅H₁₃₆N₆O₇S₃ | 1210.04 | 9.01 | 24.53 |
| 1402 | C₆₈H₁₃₃N₃OS₃ | 1105.01 | 9.01 | 27.17 | 1602 | C₆₅H₁₃₆N₆O₇S₃ | 1210.04 | 9.01 | 24.53 |
| 1403 | C₆₈H₁₃₃N₃OS₃ | 1105.01 | 9.01 | 27.17 | 1603 | C₉₈H₂₀₂N₆O₇S₃ | 1672.93 | 9.01 | 42.26 |
| 1404 | C₁₀₁H₁₉₉N₃OS₃ | 1567.90 | 9.01 | 44.90 | 1604 | C₉₂H₁₉₀N₆O₇S₃ | 1588.77 | 9.01 | 39.08 |
| 1405 | C₉₅H₁₈₇N₃OS₃ | 1483.74 | 9.01 | 41.72 | 1605 | C₈₃H₁₇₂N₆O₇S₃ | 1462.52 | 9.01 | 34.32 |
| 1406 | C₈₆H₁₆₉N₃OS₃ | 1357.50 | 9.01 | 36.96 | 1606 | C₆₅H₁₃₀N₆O₇S₃ | 1203.99 | 9.01 | 23.29 |
| 1407 | C₆₈H₁₂₇N₃OS₃ | 1098.96 | 9.01 | 25.93 | 1607 | C₆₅H₁₃₀N₆O₇S₃ | 1203.99 | 9.01 | 23.20 |
| 1408 | C₆₈H₁₂₇N₃OS₃ | 1098.96 | 9.01 | 25.84 | 1608 | C₄₄H₉₄N₆O₇S₃ | 915.47 | 9.01 | 14.08 |
| 1409 | C₄₇H₉₁N₃OS₃ | 810.44 | 9.01 | 16.72 | 1609 | C₅₆H₁₀₆N₆O₇S₃ | 1071.70 | 9.01 | 17.52 |
| 1410 | C₅₉H₁₀₃N₃OS₃ | 966.67 | 9.01 | 20.17 | 1610 | C₆₂H₁₁₈N₆O₇S₃ | 1155.86 | 9.01 | 19.53 |
| 1411 | C₆₅H₁₁₅N₃OS₃ | 1050.83 | 9.01 | 22.17 | 1611 | C₅₀H₁₀₀N₆O₇S₃ | 993.59 | 9.01 | 15.80 |
| 1412 | C₅₃H₉₇N₃OS₃ | 888.56 | 9.01 | 18.44 | 1612 | C₆₂H₁₂₄N₆O₇S₃ | 1161.91 | 9.01 | 22.15 |
| 1413 | C₆₅H₁₂₁N₃OS₃ | 1056.88 | 9.01 | 24.79 | 1613 | C₆₂H₁₃₀N₆O₇S₃ | 1167.96 | 9.01 | 23.60 |
| 1414 | C₆₅H₁₂₇N₃OS₃ | 1062.93 | 9.01 | 26.24 | 1614 | C₆₈H₁₁₈N₆O₇S₃ | 1227.93 | 9.01 | 23.60 |
| 1415 | C₇₁H₁₁₅N₃OS₃ | 1122.90 | 9.01 | 25.38 | 1615 | C₈₃H₁₄₈N₆O₇S₃ | 1438.33 | 9.01 | 31.29 |
| 1416 | C₈₆H₁₄₅N₃OS₃ | 1333.31 | 9.01 | 33.07 | 1616 | C₅₉H₁₂₁N₃O₁₀S₃ | 1128.83 | 9.01 | 22.22 |
| 1417 | C₆₂H₁₂₁N₃OS₆ | 1117.03 | 9.01 | 26.24 | 1617 | C₇₁H₁₄₅N₃O₁₀S₃ | 1297.15 | 9.01 | 28.57 |
| 1418 | C₇₄H₁₄₅N₃OS₆ | 1285.35 | 9.01 | 32.59 | 1618 | C₉₂H₁₈₇N₃O₁₀S₃ | 1591.72 | 9.01 | 39.68 |
| 1419 | C₉₅H₁₈₇N₃OS₆ | 1579.92 | 9.01 | 43.70 | 1619 | C₆₅H₁₃₃N₃O₁₀S₃ | 1212.99 | 9.01 | 25.39 |
| 1420 | C₆₈H₁₃₃N₃OS₆ | 1201.19 | 9.01 | 29.41 | 1620 | C₆₅H₁₃₃N₃O₁₀S₃ | 1212.99 | 9.01 | 25.39 |
| 1421 | C₆₈H₁₃₃N₃OS₆ | 1201.19 | 9.01 | 29.41 | 1621 | C₉₈H₁₉₉N₃O₁₀S₃ | 1675.88 | 9.01 | 43.12 |
| 1422 | C₁₀₁H₁₉₉N₃OS₆ | 1664.08 | 9.01 | 47.14 | 1622 | C₉₂H₁₈₇N₃O₁₀S₃ | 1591.72 | 9.01 | 39.95 |
| 1423 | C₉₅H₁₈₇N₃OS₆ | 1579.92 | 9.01 | 43.97 | 1623 | C₈₃H₁₆₉N₃O₁₀S₃ | 1465.48 | 9.01 | 35.19 |
| 1424 | C₈₆H₁₆₉N₃OS₆ | 1453.68 | 9.01 | 39.20 | 1624 | C₆₅H₁₂₇N₃O₁₀S₃ | 1206.94 | 9.01 | 24.15 |
| 1425 | C₆₈H₁₂₇N₃OS₆ | 1195.14 | 9.01 | 28.17 | 1625 | C₆₅H₁₂₇N₃O₁₀S₃ | 1206.94 | 9.01 | 24.06 |
| 1426 | C₆₈H₁₂₇N₃OS₆ | 1195.14 | 9.01 | 28.08 | 1626 | C₄₄H₉₁N₃O₁₀S₃ | 918.42 | 9.01 | 14.95 |
| 1427 | C₄₇H₉₁N₃OS₆ | 906.62 | 9.01 | 18.96 | 1627 | C₅₆H₁₀₃N₃O₁₀S₃ | 1074.65 | 9.01 | 18.39 |
| 1428 | C₅₉H₁₀₃N₃OS₆ | 1062.85 | 9.01 | 22.41 | 1628 | C₆₂H₁₁₅N₃O₁₀S₃ | 1158.81 | 9.01 | 20.39 |
| 1429 | C₆₅H₁₁₅N₃OS₆ | 1147.01 | 9.01 | 24.41 | 1629 | C₅₀H₉₇N₃O₁₀S₃ | 996.54 | 9.01 | 16.67 |
| 1430 | C₅₃H₉₇N₃OS₆ | 984.74 | 9.01 | 20.69 | 1630 | C₆₂H₁₂₁N₃O₁₀S₃ | 1164.86 | 9.01 | 23.02 |
| 1431 | C₆₅H₁₂₁N₃OS₆ | 1153.06 | 9.01 | 27.03 | 1631 | C₆₂H₁₂₇N₃O₁₀S₃ | 1170.91 | 9.01 | 24.47 |
| 1432 | C₆₅H₁₂₇N₃OS₆ | 1159.11 | 9.01 | 28.49 | 1632 | C₆₈H₁₁₅N₃O₁₀S₃ | 1230.88 | 9.01 | 24.27 |
| 1433 | C₇₁H₁₁₅N₃O₂S₅ | 1203.02 | 9.01 | 26.60 | 1633 | C₈₃H₁₄₅N₃O₁₀S₃ | 1441.28 | 9.01 | 31.96 |
| 1434 | C₈₆H₁₄₅N₃OS₆ | 1429.49 | 9.01 | 34.45 | 1634 | C₅₉H₁₂₁N₃O₁₀S₃ | 1128.83 | 9.01 | 22.22 |
| 1435 | C₆₂H₁₂₁N₃OS₆ | 1117.03 | 9.01 | 26.24 | 1635 | C₇₁H₁₄₅N₃O₁₀S₃ | 1297.15 | 9.01 | 28.57 |
| 1436 | C₇₄H₁₄₅N₃OS₆ | 1285.35 | 9.01 | 32.59 | 1636 | C₉₂H₁₈₇N₃O₁₀S₃ | 1591.72 | 9.01 | 39.68 |
| 1437 | C₉₅H₁₈₇N₃OS₆ | 1579.92 | 9.01 | 43.70 | 1637 | C₆₅H₁₃₃N₃O₁₀S₃ | 1212.99 | 9.01 | 25.39 |
| 1438 | C₆₈H₁₃₃N₃OS₆ | 1201.19 | 9.01 | 29.41 | 1638 | C₆₅H₁₃₃N₃O₁₀S₃ | 1212.99 | 9.01 | 25.39 |
| 1439 | C₆₈H₁₃₃N₃OS₆ | 1201.19 | 9.01 | 29.41 | 1639 | C₉₈H₁₉₉N₃O₁₀S₃ | 1675.88 | 9.01 | 43.12 |
| 1440 | C₁₀₁H₁₉₉N₃OS₆ | 1664.08 | 9.01 | 47.14 | 1640 | C₉₂H₁₈₇N₃O₁₀S₃ | 1591.72 | 9.01 | 39.95 |
| 1441 | C₉₅H₁₈₇N₃OS₆ | 1579.92 | 9.01 | 43.97 | 1641 | C₈₃H₁₆₉N₃O₁₀S₃ | 1465.48 | 9.01 | 35.19 |
| 1442 | C₈₆H₁₆₉N₃OS₆ | 1453.68 | 9.01 | 39.20 | 1642 | C₆₅H₁₂₇N₃O₁₀S₃ | 1206.94 | 9.01 | 24.15 |
| 1443 | C₆₈H₁₂₇N₃OS₆ | 1195.14 | 9.01 | 28.17 | 1643 | C₆₅H₁₂₇N₃O₁₀S₃ | 1206.94 | 9.01 | 24.06 |
| 1444 | C₆₈H₁₂₇N₃OS₆ | 1195.14 | 9.01 | 28.08 | 1644 | C₄₄H₉₁N₃O₁₀S₃ | 918.42 | 9.01 | 14.95 |
| 1445 | C₄₇H₉₁N₃OS₆ | 906.62 | 9.01 | 18.96 | 1645 | C₅₆H₁₀₃N₃O₁₀S₃ | 1074.65 | 9.01 | 18.39 |
| 1446 | C₅₉H₁₀₃N₃OS₆ | 1062.85 | 9.01 | 22.41 | 1646 | C₆₂H₁₁₅N₃O₁₀S₃ | 1158.81 | 9.01 | 20.39 |
| 1447 | C₆₅H₁₁₅N₃OS₆ | 1147.01 | 9.01 | 24.41 | 1647 | C₅₀H₉₇N₃O₁₀S₃ | 996.54 | 9.01 | 16.67 |
| 1448 | C₅₃H₉₇N₃OS₆ | 984.74 | 9.01 | 20.69 | 1648 | C₆₂H₁₂₁N₃O₁₀S₃ | 1164.86 | 9.01 | 23.02 |
| 1449 | C₆₅H₁₂₁N₃OS₆ | 1153.06 | 9.01 | 27.03 | 1649 | C₆₂H₁₂₇N₃O₁₀S₃ | 1170.91 | 9.01 | 24.47 |
| 1450 | C₆₅H₁₂₇N₃OS₆ | 1159.11 | 9.01 | 28.49 | 1650 | C₆₈H₁₁₅N₃O₁₀S₃ | 1230.88 | 9.01 | 23.31 |
| 1451 | C₇₁H₁₁₅N₃OS₆ | 1219.08 | 9.01 | 27.44 | 1651 | C₈₃H₁₄₅N₃O₁₀S₃ | 1441.28 | 9.01 | 31.01 |
| 1452 | C₈₆H₁₄₅N₃OS₆ | 1429.49 | 9.01 | 35.13 | 1652 | C₆₀H₁₂₃N₃O₁₃S₃ | 1190.85 | 9.01 | 23.97 |
| 1453 | C₆₂H₁₂₁N₃O₄S₃ | 1068.85 | 9.01 | 25.13 | 1653 | C₇₂H₁₄₇N₃O₁₃S₃ | 1359.18 | 9.01 | 30.32 |
| 1454 | C₇₄H₁₄₅N₃O₄S₃ | 1237.17 | 9.01 | 31.48 | 1654 | C₉₃H₁₈₉N₃O₁₃S₃ | 1653.74 | 9.01 | 41.43 |
| 1455 | C₉₅H₁₈₇N₃O₄S₃ | 1531.74 | 9.01 | 42.59 | 1655 | C₆₆H₁₃₅N₃O₁₃S₃ | 1275.01 | 9.01 | 27.15 |
| 1456 | C₆₈H₁₃₃N₃O₄S₃ | 1153.01 | 9.01 | 28.30 | 1656 | C₆₆H₁₃₅N₃O₁₃S₃ | 1275.01 | 9.01 | 27.15 |
| 1457 | C₆₈H₁₃₃N₃O₄S₃ | 1153.01 | 9.01 | 28.30 | 1657 | C₉₉H₂₀₁N₃O₁₃S₃ | 1737.91 | 9.01 | 44.78 |
| 1458 | C₁₀₁H₁₉₉N₃O₄S ₃ | 1615.90 | 9.01 | 46.03 | 1658 | C₉₃H₁₈₉N₃O₁₃S₃ | 1653.74 | 9.01 | 41.61 |
| 1459 | C₉₅H₁₈₇N₃O₄S₃ | 1531.74 | 9.01 | 42.86 | 1659 | C₈₄H₁₇₁N₃O₁₃S₃ | 1527.50 | 9.01 | 36.85 |
| 1460 | C₈₆H₁₆₉N₃O₄S₃ | 1405.50 | 9.01 | 38.09 | 1660 | C₆₆H₁₂₇N₃O₁₃S₃ | 1254.94 | 9.01 | 25.28 |
| 1461 | C₆₈H₁₂₇N₃O₄S₃ | 1146.96 | 9.01 | 27.06 | 1661 | C₆₅H₁₂₇N₃O₁₃S₃ | 1254.94 | 9.01 | 25.19 |
| 1462 | C₆₈H₁₂₇N₃O₄S₃ | 1146.96 | 9.01 | 26.97 | 1662 | C₄₅H₉₃N₃O₁₃S₃ | 980.45 | 9.01 | 16.61 |
| 1463 | C₄₇H₉₁N₃O₄S₃ | 858.44 | 9.01 | 17.85 | 1663 | C₅₆H₁₀₃N₃O₁₃S₃ | 1122.65 | 9.01 | 19.52 |
| 1464 | C₅₉H₁₀₃N₃O₄S₃ | 1014.67 | 9.01 | 21.30 | 1664 | C₆₂H₁₁₅N₃O₁₃S₃ | 1206.81 | 9.01 | 21.53 |
| 1465 | C₆₅H₁₁₅N₃O₄S₃ | 1098.83 | 9.01 | 23.30 | 1665 | C₅₀H₉₇N₃O₁₃S₃ | 1044.53 | 9.01 | 17.80 |
| 1466 | C₅₃H₉₇N₃O₄S₃ | 936.56 | 9.01 | 19.58 | 1666 | C₆₂H₁₂₁N₃O₁₃S₃ | 1212.86 | 9.01 | 24.15 |
| 1467 | C₆₅H₁₂₁N₃O₄S₃ | 1104.88 | 9.01 | 25.92 | 1667 | C₆₂H₁₂₇N₃O₁₃S₃ | 1218.91 | 9.01 | 25.60 |
| 1468 | C₆₅H₁₂₇N₃O₄S₃ | 1110.93 | 9.01 | 27.38 | 1668 | C₆₈H₁₁₅N₃O₁₃S₃ | 1278.88 | 9.01 | 25.80 |
| 1469 | C₇₁H₁₁₅N₃O₄S₃ | 1170.90 | 9.01 | 26.31 | 1669 | C₈₃H₁₄₅N₃O₁₃S₃ | 1489.28 | 9.01 | 33.49 |
| 1470 | C₈₆H₁₄₅N₃O₄S₃ | 1381.30 | 9.01 | 34.00 | 1670 | C₅₉H₁₂₁N₃O₇S₃ | 1080.83 | 9.01 | 19.96 |
| 1471 | C₆₂H₁₂₁N₃O₄S₃ | 1068.85 | 9.01 | 25.13 | 1671 | C₅₉H₁₂₁N₃O₇S₃ | 1080.83 | 9.01 | 19.96 |
| 1472 | C₆₂H₁₂₁N₃O₄S₃ | 1068.85 | 9.01 | 25.13 | 1672 | C₇₁H₁₄₅N₃O₇S₃ | 1249.16 | 9.01 | 26.30 |
| 1473 | C₇₄H₁₄₅N₃O₄S₃ | 1237.17 | 9.01 | 31.48 | 1673 | C₉₂H₁₈₇N₃O₇S₃ | 1543.72 | 9.01 | 37.41 |
| 1474 | C₉₅H₁₈₇N₃O₄S₃ | 1531.74 | 9.01 | 42.59 | 1674 | C₆₅H₁₃₃N₃O₇S₃ | 1164.99 | 9.01 | 23.13 |
| 1475 | C₆₈H₁₃₃N₃O₄S₃ | 1153.01 | 9.01 | 28.30 | 1675 | C₆₅H₁₃₃N₃O₇S₃ | 1164.99 | 9.01 | 23.13 |
| 1476 | C₆₈H₁₃₃N₃O₄S₃ | 1153.01 | 9.01 | 28.30 | 1676 | C₉₈H₁₉₉N₃O₇S₃ | 1627.88 | 9.01 | 40.86 |
| 1477 | C₁₀₁H₁₉₉N₃O₄S ₃ | 1615.90 | 9.01 | 46.03 | 1677 | C₉₂H₁₈₇N₃O₇S₃ | 1543.72 | 9.01 | 37.68 |
| 1478 | C₉₅H₁₈₇N₃O₄S₃ | 1531.74 | 9.01 | 42.86 | 1678 | C₈₃H₁₆₉N₃O₇S₃ | 1417.48 | 9.01 | 32.92 |
| 1479 | C₈₆H₁₆₉N₃O₄S₃ | 1405.50 | 9.01 | 38.09 | 1679 | C₆₅H₁₂₇N₃O₇S₃ | 1158.95 | 9.01 | 21.89 |
| 1480 | C₆₈H₁₂₇N₃O₄S₃ | 1146.96 | 9.01 | 27.06 | 1680 | C₆₅H₁₂₇N₃O₇S₃ | 1158.95 | 9.01 | 21.80 |
| 1481 | C₆₈H₁₂₇N₃O₆S | 1114.84 | 9.01 | 26.01 | 1681 | C₄₄H₉₁N₃O₇S₃ | 870.43 | 9.01 | 12.68 |
| 1482 | C₄₇H₉₁N₃O₄S₃ | 858.46 | 9.01 | 17.85 | 1682 | C₅₆H₁₀₃N₃O₇S₃ | 1026.65 | 9.01 | 16.12 |
| 1483 | C₅₉H₁₀₃N₃O₄S₃ | 1014.69 | 9.01 | 21.30 | 1683 | C₆₂H₁₁₅N₃O₇S₃ | 1110.82 | 9.01 | 18.13 |
| 1484 | C₆₅H₁₁₅N₃O₄S₃ | 1098.85 | 9.01 | 23.30 | 1684 | C₅₀H₉₇N₃O₇S₃ | 948.54 | 9.01 | 14.40 |
| 1485 | C₅₃H₉₇N₃O₄S₃ | 936.58 | 9.01 | 19.58 | 1685 | C₆₂H₁₂₁N₃O₇S₃ | 1116.86 | 9.01 | 20.75 |
| 1486 | C₆₅H₁₂₁N₃O₄S₃ | 1104.90 | 9.01 | 25.92 | 1686 | C₆₂H₁₂₇N₃O₇S₃ | 1122.91 | 9.01 | 22.20 |
| 1487 | C₆₅H₁₂₇N₃O₄S₃ | 1110.95 | 9.01 | 27.38 | 1687 | C₆₈H₁₁₅N₃O₇S₃ | 1182.88 | 9.01 | 22.60 |
| 1488 | C₇₁H₁₁₅N₃O₄S₃ | 1170.92 | 9.01 | 26.44 | 1688 | C₈₃H₁₄₅N₃O₇S₃ | 1393.29 | 9.01 | 30.30 |
| 1489 | C₈₆H₁₄₅N₃O₄S₃ | 1381.32 | 9.01 | 34.14 | 1689 | C₆₄H₁₃₂N₂O₄ | 993.77 | 9.45 | 26.47 |
| 1490 | C₆₂H₁₂₁N₃O₇S₃ | 1116.86 | 9.01 | 26.05 | 1690 | C₆₄H₁₃₂N₂O₄ | 993.77 | 9.45 | 26.47 |
| 1491 | C₇₄H₁₄₅N₃O₇S₃ | 1285.19 | 9.01 | 32.39 | 1691 | C₆₇H₁₃₂N₂O₇ | 1077.80 | 9.45 | 27.29 |
| 1492 | C₉₅H₁₈₇N₃O₇S₃ | 1579.76 | 9.01 | 43.50 | 1692 | C₆₇H₁₃₂N₂O₇ | 1077.80 | 9.45 | 27.29 |
| 1493 | C₆₈H₁₃₃N₃O₇S₃ | 1201.03 | 9.01 | 29.22 | 1693 | C₆₇H₁₃₂N₂O₇ | 1077.80 | 9.46 | 27.29 |
| 1494 | C₆₈H₁₃₃N₃O₇S₃ | 1201.03 | 9.01 | 29.22 | 1694 | C₆₇H₁₃₂N₂O₇ | 1077.80 | 9.46 | 27.29 |
| 1495 | C₁₀₁H₁₉₉N₃O₇S ₃ | 1663.92 | 9.01 | 46.95 | 1695 | C₆₄H₁₃₂N₂O₃S | 1009.83 | 9.80 | 26.96 |
| 1496 | C₉₅H₁₈₇N₃O₇S₃ | 1579.76 | 9.01 | 43.77 | 1696 | C₆₄H₁₃₂N₂O₃S | 1009.83 | 9.80 | 26.96 |
| 1497 | C₈₆H₁₆₉N₃O₇S₃ | 1453.51 | 9.01 | 39.01 | 1697 | C₆₇H₁₃₂N₂O₆S | 1093.86 | 9.79 | 27.78 |
| 1498 | C₆₈H₁₂₇N₃O₇S₃ | 1194.98 | 9.01 | 27.98 | 1698 | C₆₇H₁₃₂N₂O₆S | 1093.86 | 9.79 | 27.78 |
| 1499 | C₆₈H₁₂₇N₃O₇S₃ | 1194.98 | 9.01 | 27.89 | 1699 | C₆₇H₁₃₂N₂O₆S | 1093.86 | 9.81 | 27.78 |
| 1500 | C₄₇H₉₁N₃O₇S₃ | 906.46 | 9.01 | 18.77 | 1700 | C₆₇H₁₃₂N₂O₆S | 1093.86 | 9.81 | 27.78 |
| 1501 | C₅₉H₁₀₃N₃O₇S₃ | 1062.69 | 9.01 | 22.21 | 1701 | C₆₄H₁₃₃N₃O₃ | 992.79 | 9.78 | 26.31 |
| 1502 | C₆₅H₁₁₅N₃O₇S₃ | 1146.85 | 9.01 | 24.22 | 1702 | C₆₄H₁₃₃N₃O₃ | 992.79 | 9.78 | 26.31 |
| 1503 | C₅₃H₉₇N₃O₇S₃ | 984.57 | 9.01 | 20.49 | 1703 | C₆₇H₁₃₃N₃O₆ | 1076.82 | 9.78 | 27.13 |
| 1504 | C₆₅H₁₂₁N₃O₇S₃ | 1152.90 | 9.01 | 26.84 | 1704 | C₆₇H₁₃₃N₃O₆ | 1076.82 | 9.78 | 27.13 |
| 1505 | C₆₅H₁₂₇N₃O₇S₃ | 1158.95 | 9.01 | 28.29 | 1705 | C₆₇H₁₃₃N₃O₆ | 1076.82 | 9.79 | 27.13 |
| 1506 | C₇₁H₁₁₅N₃O₇S₃ | 1218.92 | 9.01 | 27.44 | 1706 | C₆₇H₁₃₃N₃O₆ | 1076.82 | 9.79 | 27.13 |
| 1507 | C₈₆H₁₄₅N₃O₇S₃ | 1429.32 | 9.01 | 35.13 | 1707 | C₆₄H₁₃₂N₂O₃S₂ | 1041.89 | 9.65 | 27.39 |
| 1508 | C₆₂H₁₂₁N₃O₇S₃ | 1116.86 | 9.01 | 26.05 | 1708 | C₆₄H₁₃₂N₂O₃S₂ | 1041.89 | 9.65 | 27.39 |
| 1509 | C₇₄H₁₄₅N₃O₇S₃ | 1285.19 | 9.01 | 32.39 | 1709 | C₆₇H₁₃₂N₂O₆S₂ | 1125.92 | 9.65 | 28.21 |
| 1510 | C₉₅H₁₈₇N₃O₇S₃ | 1579.76 | 9.01 | 43.50 | 1710 | C₆₇H₁₃₂N₂O₆S₂ | 1125.92 | 9.65 | 28.21 |
| 1511 | C₆₈H₁₃₃N₃O₇S₃ | 1201.03 | 9.01 | 29.22 | 1711 | C₆₇H₁₃₂N₂O₆S₂ | 1125.92 | 9.65 | 28.21 |
| 1512 | C₆₈H₁₃₃N₃O₇S₃ | 1201.03 | 9.01 | 29.22 | 1712 | C₆₇H₁₃₂N₂O₆S₂ | 1125.92 | 9.65 | 28.21 |
| 1513 | C₁₀₁H₁₉₉N₃O₇S ₃ | 1663.92 | 9.01 | 46.95 | 1713 | C₆₅H₁₃₂N₂O₄ | 1005.78 | 8.95 | 26.46 |
| 1514 | C₉₅H₁₈₇N₃O₇S₃ | 1579.76 | 9.01 | 43.77 | 1714 | C₆₅H₁₃₂N₂O₄ | 1005.78 | 8.95 | 26.46 |
| 1515 | C₈₆H₁₆₉N₃O₇S₃ | 1453.51 | 9.01 | 39.01 | 1715 | C₆₈H₁₃₂N₂O₇ | 1089.81 | 8.95 | 27.28 |
| 1516 | C₆₈H₁₂₇N₃O₇S₃ | 1194.98 | 9.01 | 27.98 | 1716 | C₆₈H₁₃₂N₂O₇ | 1089.81 | 8.95 | 27.28 |
| 1517 | C₆₈H₁₂₇N₃O₇S₃ | 1194.98 | 9.01 | 27.89 | 1717 | C₆₈H₁₃₂N₂O₇ | 1089.81 | 8.96 | 27.28 |
| 1518 | C₄₇H₉₁N₃O₇S₃ | 906.46 | 9.01 | 18.77 | 1718 | C₆₈H₁₃₂N₂O₇ | 1089.81 | 8.96 | 27.28 |
| 1519 | C₅₉H₁₀₃N₃O₇S₃ | 1062.69 | 9.01 | 22.21 | 1719 | C₆₅H₁₃₂N₂O₅ | 1021.78 | 8.83 | 26.64 |
| 1520 | C₆₅H₁₁₅N₃O₇S₃ | 1146.85 | 9.01 | 24.22 | 1720 | C₆₅H₁₃₂N₂O₅ | 1021.78 | 8.83 | 26.64 |
| 1521 | C₅₃H₉₇N₃O₇S₃ | 984.57 | 9.01 | 20.49 | 1721 | C₆₈H₁₃₂N₂O₈ | 1105.81 | 8.83 | 27.46 |
| 1522 | C₆₅H₁₂₁N₃O₇S₃ | 1152.90 | 9.01 | 26.84 | 1722 | C₆₈H₁₃₂N₂O₈ | 1105.81 | 8.83 | 27.46 |
| 1523 | C₆₅H₁₂₇N₃O₇S₃ | 1158.95 | 9.01 | 28.29 | 1723 | C₆₈H₁₃₂N₂O₈ | 1105.81 | 8.83 | 27.46 |
| 1524 | C₇₁H₁₁₅N₃O₇S₃ | 1218.92 | 9.01 | 25.64 | 1724 | C₆₈H₁₃₂N₂O₈ | 1105.81 | 8.83 | 27.46 |
| 1525 | C₈₆H₁₄₅N₃O₇S₃ | 1429.32 | 9.01 | 33.34 | 1725 | C₆₅H₁₃₂N₂O₅ | 1021.78 | 8.67 | 26.64 |
| 1526 | C₆₂H₁₂₁N₃O₄S₆ | 1165.07 | 9.01 | 27.16 | 1726 | C₆₅H₁₃₂N₂O₅ | 1021.78 | 8.67 | 26.64 |
| 1527 | C₇₄H₁₄₅N₃O₄S₆ | 1333.39 | 9.01 | 33.50 | 1727 | C₆₈H₁₃₂N₂O₈ | 1105.81 | 8.67 | 27.46 |
| 1528 | C₉₅H₁₈₇N₃O₄S₆ | 1627.96 | 9.01 | 44.61 | 1728 | C₆₈H₁₃₂N₂O₈ | 1105.81 | 8.67 | 27.46 |
| 1529 | C₆₈H₁₃₃N₃O₄S₆ | 1249.23 | 9.01 | 30.33 | 1729 | C₆₈H₁₃₂N₂O₈ | 1105.81 | 8.67 | 27.46 |
| 1530 | C₆₈H₁₃₃N₃O₄S₆ | 1249.23 | 9.01 | 30.33 | 1730 | C₆₈H₁₃₂N₂O₈ | 1105.81 | 8.67 | 27.46 |
| 1531 | C₁₀₁H₁₉₉N₃O₄S₆ | 1712.12 | 9.01 | 48.06 | 1731 | C₆₅H₁₃₂N₂O₆ | 1037.78 | 8.66 | 26.60 |
| 1532 | C₉₅H₁₈₇N₃O₄S₆ | 1627.96 | 9.01 | 44.88 | 1732 | C₆₅H₁₃₂N₂O₆ | 1037.78 | 8.66 | 26.60 |
| 1533 | C₈₆H₁₆₉N₃O₄S₆ | 1501.72 | 9.01 | 40.12 | 1733 | C₆₈H₁₃₂N₂O₉ | 1121.81 | 8.66 | 27.43 |
| 1534 | C₆₈H₁₂₇N₃O₄S₆ | 1243.18 | 9.01 | 29.09 | 1734 | C₆₈H₁₃₂N₂O₉ | 1121.81 | 8.66 | 27.43 |
| 1535 | C₆₈H₁₂₇N₃O₄S₆ | 1243.18 | 9.01 | 29.00 | 1735 | C₆₈H₁₃₂N₂O₉ | 1121.81 | 8.66 | 27.43 |
| 1536 | C₄₇H₉₁N₃O₄S₆ | 954.66 | 9.01 | 19.88 | 1736 | C₆₈H₁₃₂N₂O₉ | 1121.81 | 8.66 | 27.43 |
| 1537 | C₅₉H₁₀₃N₃O₄S₆ | 1110.89 | 9.01 | 23.32 | 1737 | C₆₅H₁₃₃N₃O₄ | 1020.80 | 8.84 | 26.04 |
| 1538 | C₆₅H₁₁₅N₃O₄S₆ | 1195.05 | 9.01 | 25.33 | 1738 | C₆₅H₁₃₃N₃O₄ | 1020.80 | 8.84 | 26.04 |
| 1539 | C₅₃H₉₇N₃O₄S₆ | 1032.78 | 9.01 | 21.60 | 1739 | C₆₈H₁₃₃N₃O₇ | 1104.83 | 8.83 | 26.86 |
| 1540 | C₆₅H₁₂₁N₃O₄S₆ | 1201.10 | 9.01 | 27.95 | 1740 | C₆₈H₁₃₃N₃O₇ | 1104.83 | 8.84 | 26.86 |
| 1541 | C₆₅H₁₂₇N₃O₄S₆ | 1207.15 | 9.01 | 29.40 | 1741 | C₆₈H₁₃₃N₃O₇ | 1104.83 | 8.84 | 26.86 |
| 1542 | C₇₁H₁₁₅N₃O₄S₆ | 1267.12 | 9.01 | 28.55 | 1742 | C₆₈H₁₃₃N₃O₇ | 1104.83 | 8.84 | 26.86 |
| 1543 | C₈₆H₁₄₅N₃O₄S₆ | 1477.52 | 9.01 | 36.24 | 1743 | C₆₅H₁₃₃N₃O₅ | 1036.79 | 8.85 | 26.48 |
| 1544 | C₆₅H₁₃₆N₃O₁₀P ₃ | 1212.74 | 9.01 | 21.98 | 1744 | C₆₅H₁₃₃N₃O₅ | 1036.79 | 8.85 | 26.48 |
| 1545 | C₇₇H₁₆₀N₃O₁₀P ₃ | 1381.06 | 9.01 | 28.33 | 1745 | C₆₈H₁₃₃N₃O₈ | 1120.82 | 8.85 | 27.30 |
| 1546 | C₉₈H₂₀₂N₃O₁₀P ₃ | 1675.63 | 9.01 | 39.44 | 1746 | C₆₈H₁₃₃N₃O₈ | 1120.82 | 8.85 | 27.30 |
| 1547 | C₇₁H₁₄₈N₃O₁₀P ₃ | 1296.90 | 9.01 | 25.16 | 1747 | C₆₈H₁₃₃N₃O₈ | 1120.82 | 8.85 | 27.30 |
| 1548 | C₇₁H₁₄₈N₃O₁₀P ₃ | 1296.90 | 9.01 | 25.16 | 1748 | C₆₈H₁₃₃N₃O₈ | 1120.82 | 8.85 | 27.30 |
| 1549 | C₁₀₄H₂₁₄N₃O₁₀ P₃ | 1759.79 | 9.01 | 42.88 | 1749 | C₆₅H₁₃₃N₃O₅ | 1036.79 | 8.75 | 26.48 |
| 1550 | C₉₈H₂₀₂N₃O₁₀P ₃ | 1675.63 | 9.01 | 39.71 | 1750 | C₆₅H₁₃₃N₃O₅ | 1036.79 | 8.75 | 26.48 |
| 1551 | C₈₉H₁₈₄N₃O₁₀P ₃ | 1549.38 | 9.01 | 34.95 | 1751 | C₆₈H₁₃₃N₃O₈ | 1120.82 | 8.75 | 27.30 |
| 1552 | C₇₁H₁₄₂N₃O₁₀P ₃ | 1290.85 | 9.01 | 23.91 | 1752 | C₆₈H₁₃₃N₃O₈ | 1120.82 | 8.75 | 27.30 |
| 1553 | C₇₁H₁₄₂N₃O₁₀P ₃ | 1290.85 | 9.01 | 23.82 | 1753 | C₆₈H₁₃₃N₃O₈ | 1120.82 | 8.75 | 27.30 |
| 1554 | C₅₀H₁₀₆N₃O₁₀P ₃ | 1002.33 | 9.01 | 14.71 | 1754 | C₆₈H₁₃₃N₃O₈ | 1120.82 | 8.75 | 27.30 |
| 1555 | C₆₂H₁₁₈N₃O₁₀P ₃ | 1158.56 | 9.01 | 18.15 | 1755 | C₆₅H₁₃₄N₄O₄ | 1035.81 | 8.95 | 26.29 |
| 1556 | C₆₈H₁₃₀N₃O₁₀P ₃ | 1242.72 | 9.01 | 20.16 | 1756 | C₆₅H₁₃₄N₄O₄ | 1035.81 | 8.95 | 26.29 |
| 1557 | C₅₆H₁₁₂N₃O₁₀P ₃ | 1080.44 | 9.01 | 16.43 | 1757 | C₆₈H₁₃₄N₄O₇ | 1119.84 | 8.95 | 27.11 |
| 1558 | C₆₈H₁₃₆N₃O₁₀P ₃ | 1248.77 | 9.01 | 22.78 | 1758 | C₆₈H₁₃₄N₄O₇ | 1119.84 | 8.95 | 27.11 |
| 1559 | C₆₈H₁₄₂N₃O₁₀P ₃ | 1254.82 | 9.01 | 24.23 | 1759 | C₆₈H₁₃₄N₄O₇ | 1119.84 | 8.95 | 27.11 |
| 1560 | C₇₄H₁₃₀N₃O₁₀P ₃ | 1314.79 | 9.01 | 23.74 | 1760 | C₆₈H₁₃₄N₄O₇ | 1119.84 | 8.95 | 27.11 |
| 1561 | C₈₉H₁₆₀N₃O₁₀P ₃ | 1525.19 | 9.01 | 31.43 | 1761 | C₆₅H₁₃₂N₂O₃S | 1021.84 | 9.29 | 26.71 |
| 1562 | C₆₅H₁₃₆N₃O₁₀P ₃ | 1212.74 | 9.01 | 21.98 | 1762 | C₆₅H₁₃₂N₂O₃S | 1021.84 | 9.29 | 26.71 |
| 1563 | C₇₇H₁₆₀N₃O₁₀P ₃ | 1381.06 | 9.01 | 28.33 | 1763 | C₆₈H₁₃₂N₂O₆S | 1105.87 | 9.29 | 27.53 |
| 1564 | C₉₈H₂₀₂N₃O₁₀P ₃ | 1675.63 | 9.01 | 39.44 | 1764 | C₆₈H₁₃₂N₂O₆S | 1105.87 | 9.29 | 27.53 |
| 1565 | C₇₁H₁₄₈N₃O₁₀P ₃ | 1296.90 | 9.01 | 25.16 | 1765 | C₆₈H₁₃₂N₂O₆S | 1105.87 | 9.31 | 27.53 |
| 1566 | C₇₁H₁₄₈N₃O₁₀P ₃ | 1296.90 | 9.01 | 25.16 | 1766 | C₆₈H₁₃₂N₂O₆S | 1105.87 | 9.31 | 27.53 |
| 1567 | C₁₀₄H₂₁₄N₃O₁₀ P₃ | 1759.79 | 9.01 | 42.88 | 1767 | C₆₅H₁₃₂N₂O₃S₂ | 1053.90 | 9.35 | 27.19 |
| 1568 | C₉₈H₂₀₂N₃O₁₀P ₃ | 1675.63 | 9.01 | 39.71 | 1768 | C₆₅H₁₃₂N₂O₃S₂ | 1053.90 | 9.35 | 27.19 |
| 1569 | C₈₉H₁₈₄N₃O₁₀P ₃ | 1549.38 | 9.01 | 34.95 | 1769 | C₆₈H₁₃₂N₂O₆S₂ | 1137.93 | 9.35 | 28.02 |
| 1570 | C₇₁H₁₄₂N₃O₁₀P ₃ | 1290.85 | 9.01 | 23.91 | 1770 | C₆₈H₁₃₂N₂O₆S₂ | 1137.93 | 9.35 | 28.02 |
| 1571 | C₇₁H₁₄₂N₃O₁₀P ₃ | 1290.85 | 9.01 | 23.82 | 1771 | C₆₈H₁₃₂N₂O₆S₂ | 1137.93 | 9.35 | 28.02 |
| 1572 | C₅₀H₁₀₆N₃O₁₀P ₃ | 1002.33 | 9.01 | 14.71 | 1772 | C₆₈H₁₃₂N₂O₆S₂ | 1137.93 | 9.35 | 28.02 |
| 1573 | C₆₂H₁₁₈N₃O₁₀P ₃ | 1158.56 | 9.01 | 18.15 | 1773 | C₆₅H₁₃₂N₂O₃S₂ | 1053.90 | 9.13 | 27.19 |
| 1574 | C₆₈H₁₃₀N₃O₁₀P₃ | 1242.72 | 9.01 | 20.16 | 1774 | C₆₅H₁₃₂N₂O₃S₂ | 1053.90 | 9.13 | 27.19 |
| 1575 | C₅₆H₁₁₂N₃O₁₀P ₃ | 1080.44 | 9.01 | 16.43 | 1775 | C₆₈H₁₃₂N₂O₆S₂ | 1137.93 | 9.13 | 28.02 |
| 1576 | C₆₈H₁₃₆N₃O₁₀P ₃ | 1248.77 | 9.01 | 22.78 | 1776 | C₆₈H₁₃₂N₂O₆S₂ | 1137.93 | 9.13 | 28.02 |
| 1577 | C₆₈H₁₄₂N₃O₁₀P ₃ | 1254.82 | 9.01 | 24.23 | 1777 | C₆₈H₁₃₂N₂O₆S₂ | 1137.93 | 9.14 | 28.02 |
| 1578 | C₇₄H₁₃₀N₃O₁₀P ₃ | 1314.79 | 9.01 | 24.34 | 1778 | C₆₈H₁₃₂N₂O₆S₂ | 1137.93 | 9.14 | 28.02 |
| 1579 | C₈₉H₁₆₀N₃O₁₀P ₃ | 1525.19 | 9.01 | 32.03 | 1779 | C₆₅H₁₃₂N₂O₄S | 1037.84 | 9.20 | 26.95 |
| 1580 | C₅₉H₁₂₄N₆O₇S₃ | 1125.88 | 9.01 | 21.35 | 1780 | C₆₅H₁₃₂N₂O₄S | 1037.84 | 9.20 | 26.95 |
| 1581 | C₇₁H₁₄₈N₆O₇S₃ | 1294.20 | 9.01 | 27.70 | 1781 | C₆₈H₁₃₂N₂O₇S | 1121.87 | 9.20 | 27.78 |
| 1582 | C₉₂H₁₉₀N₆O₇S₃ | 1588.77 | 9.01 | 38.81 | 1782 | C₆₈H₁₃₂N₂O₇S | 1121.87 | 9.20 | 27.78 |
| 1583 | C₆₅H₁₃₆N₆O₇S₃ | 1210.04 | 9.01 | 24.53 | 1783 | C₆₈H₁₃₂N₂O₇S | 1121.87 | 9.20 | 27.78 |
| 1584 | C₆₅H₁₃₆N₆O₇S₃ | 1210.04 | 9.01 | 24.53 | 1784 | C₆₈H₁₃₂N₂O₇S | 1121.87 | 9.20 | 27.78 |
| 1585 | C₉₈H₂₀₂N₆O₇S₃ | 1672.93 | 9.01 | 42.26 | 1785 | C₆₅H₁₃₂N₂O₄S | 1037.84 | 8.80 | 26.95 |
| 1586 | C₉₂H₁₉₀N₆O₇S₃ | 1588.77 | 9.01 | 39.08 | 1786 | C₆₅H₁₃₂N₂O₄S | 1037.84 | 8.80 | 26.95 |
| 1587 | C₈₃H₁₇₂N₆O₇S₃ | 1462.52 | 9.01 | 34.32 | 1787 | C₆₈H₁₃₂N₂O₇S | 1121.87 | 8.80 | 27.78 |
| 1588 | C₆₅H₁₃₀N₆O₇S₃ | 1203.99 | 9.01 | 23.29 | 1788 | C₆₈H₁₃₂N₂O₇S | 1121.87 | 8.80 | 27.78 |
| 1589 | C₆₅H₁₃₀N₆O₇S₃ | 1203.99 | 9.01 | 23.20 | 1789 | C₆₈H₁₃₂N₂O₇S | 1121.87 | 8.80 | 27.78 |
| 1590 | C₄₄H₉₄N₆O₇S₃ | 915.47 | 9.01 | 14.08 | 1790 | C₆₈H₁₃₂N₂O₇S | 1121.87 | 8.80 | 27.78 |
| 1591 | C₅₆H₁₀₆N₆O₇S₃ | 1071.70 | 9.01 | 17.52 | 1791 | C₆₅H₁₃₂N₂O₅S | 1053.84 | 8.70 | 27.15 |
| 1592 | C₆₂H₁₁₈N₆O₇S₃ | 1155.86 | 9.01 | 19.53 | 1792 | C₆₅H₁₃₂N₂O₅S | 1053.84 | 8.70 | 27.15 |
| 1593 | C₅₀H₁₀₀N₆O₇S₃ | 993.59 | 9.01 | 15.80 | 1793 | C₆₈H₁₃₂N₂O₈S | 1137.87 | 8.70 | 27.97 |
| 1594 | C₆₂H₁₂₄N₆O₇S₃ | 1161.91 | 9.01 | 22.15 | 1794 | C₆₈H₁₃₂N₂O₈S | 1137.87 | 8.70 | 27.97 |
| 1595 | C₆₂H₁₃₀N₆O₇S₃ | 1167.96 | 9.01 | 23.60 | 1795 | C₆₈H₁₃₂N₂O₈S | 1137.87 | 8.70 | 27.97 |
| 1596 | C₆₈H₁₁₈N₆O₇S₃ | 1227.93 | 9.01 | 23.53 | 1796 | C₆₈H₁₃₂N₂O₈S | 1137.87 | 8.70 | 27.97 |
| 1597 | C₈₃H₁₄₈N₆O₇S₃ | 1438.33 | 9.01 | 31.23 | 1797 | C₆₅H₁₃₂N₂O₅S | 1053.84 | 9.00 | 27.15 |
| 1598 | C₅₉H₁₂₄N₆O₇S₃ | 1125.88 | 9.01 | 21.35 | 1798 | C₆₅H₁₃₂N₂O₅S | 1053.84 | 9.00 | 27.15 |
| 1599 | C₇₁H₁₄₈N₆O₇S₃ | 1294.20 | 9.01 | 27.70 | 1799 | C₆₈H₁₃₂N₂O₈S | 1137.87 | 9.00 | 27.97 |
| 1600 | C₉₂H₁₉₀N₆O₇S₃ | 1588.77 | 9.01 | 38.81 | 1800 | C₆₈H₁₃₂N₂O₈S | 1137.87 | 9.00 | 27.97 |
| 1801 | C₆₈H₁₃₂N₂O₈S | 1137.87 | 9.00 | 27.97 | 2001 | C₆₉H₁₃₇N₅O₅ | 1116.88 | 7.65 | 24.63 |
| 1802 | C₆₈H₁₃₂N₂O₈S | 1137.87 | 9.00 | 27.97 | 2002 | C₆₉H₁₃₇N₅O₅ | 1116.88 | 7.65 | 24.63 |
| 1803 | C₆₅H₁₃₂N₂O₄S₂ | 1069.90 | 9.06 | 27.39 | 2003 | C₇₂H₁₃₇N₅O₈ | 1200.91 | 7.65 | 25.45 |
| 1804 | C₆₅H₁₃₂N₂O₄S₂ | 1069.90 | 9.06 | 27.39 | 2004 | C₇₂H₁₃₇N₅O₈ | 1200.91 | 7.65 | 25.45 |
| 1805 | C₆₈H₁₃₂N₂O₇S₂ | 1153.93 | 9.06 | 28.21 | 2005 | C₇₂H₁₃₇N₅O₈ | 1200.91 | 7.65 | 25.45 |
| 1806 | C₆₈H₁₃₂N₂O₇S₂ | 1153.93 | 9.06 | 28.21 | 2006 | C₇₂H₁₃₇N₅O₈ | 1200.91 | 7.65 | 25.45 |
| 1807 | C₆₈H₁₃₂N₂O₇S₂ | 1153.93 | 9.06 | 28.21 | 2007 | C₆₉H₁₃₆N₄O₅ | 1101.87 | 7.90 | 25.58 |
| 1808 | C₆₈H₁₃₂N₂O₇S₂ | 1153.93 | 9.06 | 28.21 | 2008 | C₆₉H₁₃₆N₄O₅ | 1101.87 | 7.90 | 25.58 |
| 1809 | C₆₆H₁₃₇N₂O₆P | 1085.80 | 8.79 | 26.39 | 2009 | C₇₂H₁₃₆N₄O₈ | 1185.90 | 7.90 | 26.41 |
| 1810 | C₆₆H₁₃₇N₂O₆P | 1085.80 | 8.79 | 26.39 | 2010 | C₇₂H₁₃₆N₄O₈ | 1185.90 | 7.90 | 26.41 |
| 1811 | C₆₉H₁₃₇N₂O₉P | 1169.83 | 8.79 | 27.21 | 2011 | C₇₂H₁₃₆N₄O₈ | 1185.90 | 7.90 | 26.41 |
| 1812 | C₆₉H₁₃₇N₂O₉P | 1169.83 | 8.79 | 27.21 | 2012 | C₇₂H₁₃₆N₄O₈ | 1185.90 | 7.90 | 26.41 |
| 1813 | C₆₉H₁₃₇N₂O₉P | 1169.83 | 8.79 | 27.21 | 2013 | C₆₉H₁₃₇N₅O₄ | 1100.89 | 8.23 | 25.73 |
| 1814 | C₆₉H₁₃₇N₂O₉P | 1169.83 | 8.79 | 27.21 | 2014 | C₆₉H₁₃₇N₅O₄ | 1100.89 | 8.23 | 25.73 |
| 1815 | C₆₆H₁₃₇N₂O₆P | 1085.80 | 8.41 | 26.39 | 2015 | C₇₂H₁₃₇N₅O₇ | 1184.92 | 8.23 | 26.56 |
| 1816 | C₆₆H₁₃₇N₂O₆P | 1085.80 | 8.41 | 26.39 | 2016 | C₇₂H₁₃₇N₅O₇ | 1184.92 | 8.23 | 26.56 |
| 1817 | C₆₉H₁₃₇N₂O₉P | 1169.83 | 8.41 | 27.21 | 2017 | C₇₂H₁₃₇N₅O₇ | 1184.92 | 8.23 | 26.56 |
| 1818 | C₆₉H₁₃₇N₂O₉P | 1169.83 | 8.41 | 27.21 | 2018 | C₇₂H₁₃₇N₅O₇ | 1184.92 | 8.23 | 26.56 |
| 1819 | C₆₉H₁₃₇N₂O₉P | 1169.83 | 8.41 | 27.21 | 2019 | C₆₉H₁₃₇N₅O₄ | 1100.89 | 7.75 | 26.13 |
| 1820 | C₆₉H₁₃₇N₂O₉P | 1169.83 | 8.41 | 27.21 | 2020 | C₆₉H₁₃₇N₅O₄ | 1100.89 | 7.75 | 26.13 |
| 1821 | C₆₄H₁₃₃N₃O₅S | 1056.84 | 8.74 | 26.14 | 2021 | C₇₂H₁₃₇N₅O₇ | 1184.92 | 7.75 | 26.95 |
| 1822 | C₆₄H₁₃₃N₃O₅S | 1056.84 | 8.74 | 26.14 | 2022 | C₇₂H₁₃₇N₅O₇ | 1184.92 | 7.75 | 26.95 |
| 1823 | C₆₇H₁₃₃N₃O₈S | 1140.87 | 8.74 | 26.96 | 2023 | C₇₂H₁₃₇N₅O₇ | 1184.92 | 7.75 | 26.95 |
| 1824 | C₆₇H₁₃₃N₃O₈S | 1140.87 | 8.74 | 26.96 | 2024 | C₇₂H₁₃₇N₅O₇ | 1184.92 | 7.75 | 26.95 |
| 1825 | C₆₇H₁₃₃N₃O₈S | 1140.87 | 8.74 | 26.96 | 2025 | C₆₈H₁₃₆N₆O₄ | 1101.87 | 7.19 | 25.85 |
| 1826 | C₆₇H₁₃₃N₃O₈S | 1140.87 | 8.74 | 26.96 | 2026 | C₆₈H₁₃₆N₆O₄ | 1101.87 | 7.19 | 25.85 |
| 1827 | C₆₄H₁₃₃N₃O₅S | 1056.84 | 7.78 | 26.14 | 2027 | C₇₁H₁₃₆N₆O₇ | 1185.90 | 7.19 | 26.67 |
| 1828 | C₆₄H₁₃₃N₃O₅S | 1056.84 | 7.78 | 26.14 | 2028 | C₇₁H₁₃₆N₆O₇ | 1185.90 | 7.19 | 26.67 |
| 1829 | C₆₇H₁₃₃N₃O₈S | 1140.87 | 7.78 | 26.96 | 2029 | C₇₁H₁₃₆N₆O₇ | 1185.90 | 7.19 | 26.67 |
| 1830 | C₆₇H₁₃₃N₃O₈S | 1140.87 | 7.78 | 26.96 | 2030 | C₇₁H₁₃₆N₆O₇ | 1185.90 | 7.19 | 26.67 |
| 1831 | C₆₇H₁₃₃N₃O₈S | 1140.87 | 7.78 | 26.96 | 2031 | C₆₉H₁₃₇N₅O₄S | 1132.95 | 7.95 | 27.26 |
| 1832 | C₆₇H₁₃₃N₃O₈S | 1140.87 | 7.78 | 26.96 | 2032 | C₆₉H₁₃₇N₅O₄S | 1132.95 | 7.95 | 27.26 |
| 1833 | C₆₄H₁₃₂N₂O₆S | 1057.83 | 7.60 | 26.33 | 2033 | C₈₉H₁₇₆N₆O₉S | 1506.48 | 7.74 | 33.24 |
| 1834 | C₆₄H₁₃₂N₂O₆S | 1057.83 | 7.60 | 26.33 | 2034 | C₈₈H₁₇₅N₅O₉ | 1447.39 | 8.17 | 35.73 |
| 1835 | C₆₇H₁₃₂N₂O₉S | 1141.86 | 7.60 | 27.15 | 2035 | C₇₂H₁₃₇N₅O₇S | 1216.98 | 7.96 | 28.08 |
| 1836 | C₆₇H₁₃₂N₂O₉S | 1141.86 | 7.60 | 27.15 | 2036 | C₇₂H₁₃₇N₅O₇S | 1216.98 | 7.96 | 28.08 |
| 1837 | C₆₇H₁₃₂N₂O₉S | 1141.86 | 7.60 | 27.15 | 2037 | C₇₁H₁₃₈N₈O₅ | 1183.94 | 7.59 | 24.76 |
| 1838 | C₆₇H₁₃₂N₂O₉S | 1141.86 | 7.60 | 27.15 | 2038 | C₇₁H₁₃₈N₈O₅ | 1183.94 | 7.59 | 24.76 |
| 1839 | C₆₄H₁₃₂N₂O₆S | 1057.83 | 8.32 | 26.33 | 2039 | C₉₃H₁₇₉N₅O₁₁S₃ | 1639.66 | 7.56 | 35.27 |
| 1840 | C₆₄H₁₃₂N₂O₆S | 1057.83 | 8.32 | 26.33 | 2040 | C₉₃H₁₈₀N₅O₁₂PS | 1623.52 | 8.52 | 37.28 |
| 1841 | C₆₇H₁₃₂N₂O₉S | 1141.86 | 8.32 | 27.15 | 2041 | C₇₄H₁₃₈N₈O₈ | 1267.97 | 7.59 | 25.58 |
| 1842 | C₆₇H₁₃₂N₂O₉S | 1141.86 | 8.32 | 27.15 | 2042 | C₇₄H₁₃₈N₈O₈ | 1267.97 | 7.59 | 25.58 |
| 1843 | C₆₇H₁₃₂N₂O₉S | 1141.86 | 8.32 | 27.15 | 2043 | C₆₈H₁₄₀N₆O₄ | 1105.91 | 8.62 | 25.32 |
| 1844 | C₆₇H₁₃₂N₂O₉S | 1141.86 | 8.32 | 27.15 | 2044 | C₆₈H₁₄₀N₆O₄ | 1105.91 | 8.62 | 25.32 |
| 1845 | C₆₄H₁₃₂N₂O₇S | 1073.83 | 8.15 | 26.57 | 2045 | C₇₁H₁₄₀N₆O₇ | 1189.94 | 8.62 | 26.14 |
| 1846 | C₆₄H₁₃₂N₂O₇S | 1073.83 | 8.15 | 26.57 | 2046 | C₇₁H₁₄₀N₆O₇ | 1189.94 | 8.62 | 26.14 |
| 1847 | C₆₇H₁₃₂N₂O₁₀S | 1157.86 | 8.15 | 27.39 | 2047 | C₇₁H₁₄₀N₆O₇ | 1189.94 | 8.62 | 26.14 |
| 1848 | C₆₇H₁₃₂N₂O₁₀S | 1157.86 | 8.15 | 27.39 | 2048 | C₇₁H₁₄₀N₆O₇ | 1189.94 | 8.62 | 26.14 |
| 1849 | C₆₇H₁₃₂N₂O₁₀S | 1157.86 | 8.15 | 27.39 | 2049 | C₆₈H₁₃₉N₅O₄ | 1090.89 | 8.61 | 25.54 |
| 1850 | C₆₇H₁₃₂N₂O₁₀S | 1157.86 | 8.15 | 27.39 | 2050 | C₆₈H₁₃₉N₅O₄ | 1090.89 | 8.61 | 25.54 |
| 1851 | C₆₄H₁₃₂N₂O₅S | 1041.83 | 7.83 | 25.84 | 2051 | C₇₁H₁₃₉N₅O₇ | 1174.92 | 8.61 | 26.36 |
| 1852 | C₆₄H₁₃₂N₂O₅S | 1041.83 | 7.83 | 25.84 | 2052 | C₇₁H₁₃₉N₅O₇ | 1174.92 | 8.61 | 26.36 |
| 1853 | C₆₇H₁₃₂N₂O₈S | 1125.86 | 7.83 | 26.66 | 2053 | C₇₁H₁₃₉N₅O₇ | 1174.92 | 8.61 | 26.36 |
| 1854 | C₆₇H₁₃₂N₂O₈S | 1125.86 | 7.83 | 26.66 | 2054 | C₇₁H₁₃₉N₅O₇ | 1174.92 | 8.61 | 26.36 |
| 1855 | C₆₇H₁₃₂N₂O₈S | 1125.86 | 7.84 | 26.66 | 2055 | C₆₉H₁₃₉N₅O₅ | 1118.90 | 7.82 | 25.64 |
| 1856 | C₆₇H₁₃₂N₂O₈S | 1125.86 | 7.84 | 26.66 | 2056 | C₆₉H₁₃₉N₅O₅ | 1118.90 | 7.82 | 25.64 |
| 1857 | C₆₇H₁₃₇N₃O₄ | 1048.85 | 9.07 | 27.10 | 2057 | C₉₀H₁₇₆N₂OS₈ | 1558.89 | 9.96 | 42.56 |
| 1858 | C₆₇H₁₃₇N₃O₄ | 1048.85 | 9.07 | 7.10 | 2058 | C₈₉H₁₇₄N₂OS₈ | 1544.86 | 8.77 | 40.62 |
| 1859 | C₇₀H₁₃₇N₃O₇ | 1132.88 | 9.07 | 27.92 | 2059 | C₇₂H₁₃₉N₅O₈ | 1202.93 | 7.82 | 26.46 |
| 1860 | C₇₀H₁₃₇N₃O₇ | 1132.88 | 9.07 | 27.92 | 2060 | C₇₂H₁₃₉N₅O₈ | 1202.93 | 7.82 | 26.46 |
| 1861 | C₇₀H₁₃₇N₃O₇ | 1132.88 | 9.07 | 27.92 | 2061 | C₇₀H₁₄₁N₅O₅ | 1132.93 | 7.84 | 26.35 |
| 1862 | C₇₀H₁₃₇N₃O₇ | 1132.88 | 9.07 | 27.92 | 2062 | C₇₀H₁₄₁N₅O₅ | 1132.93 | 7.84 | 26.35 |
| 1863 | C₆₉H₁₄₁N₃O₄ | 1076.90 | 9.10 | 28.15 | 2063 | C₇₃H₁₄₁N₅O₈ | 1216.96 | 7.84 | 27.17 |
| 1864 | C₆₉H₁₄₁N₃O₄ | 1076.90 | 9.09 | 28.15 | 2064 | C₇₃H₁₄₁N₅O₈ | 1216.96 | 7.84 | 27.17 |
| 1865 | C₇₂H₁₄₁N₃O₇ | 1160.93 | 9.10 | 28.98 | 2065 | C₇₃H₁₄₁N₅O₈ | 1216.96 | 7.84 | 27.17 |
| 1866 | C₉₅H₁₉₄N₄O₉S₅ | 1696.92 | 9.86 | 40.86 | 2066 | C₇₃H₁₄₁N₅O₈ | 1216.96 | 7.84 | 27.17 |
| 1867 | C₇₂H₁₄₁N₃O₇ | 1160.93 | 9.10 | 28.98 | 2067 | C₆₉H₁₃₇N₅O₆ | 1132.88 | 7.19 | 25.38 |
| 1868 | C₇₂H₁₄₁N₃O₇ | 1160.93 | 9.10 | 28.98 | 2068 | C₆₉H₁₃₇N₅O₆ | 1132.88 | 7.19 | 25.38 |
| 1869 | C₇₁H₁₄₅N₃O₄ | 1104.96 | 9.12 | 29.21 | 2069 | C₇₂H₁₃₇N₅O₉ | 1216.91 | 7.06 | 26.20 |
| 1870 | C₇₁H₁₄₅N₃O₄ | 1104.96 | 9.12 | 29.21 | 2070 | C₇₂H₁₃₇N₅O₉ | 1216.91 | 7.06 | 26.20 |
| 1871 | C₇₄H₁₄₅N₃O₇ | 1188.99 | 9.12 | 30.03 | 2071 | C₇₂H₁₃₇N₅O₉ | 1216.91 | 7.06 | 26.20 |
| 1872 | C₉₆H₁₉₁NO₁₄S₅ | 1743.88 | 7.67 | 41.25 | 2072 | C₇₂H₁₃₇N₅O₉ | 1216.91 | 7.06 | 26.20 |
| 1873 | C₇₄H₁₄₅N₃O₇ | 1188.99 | 9.12 | 30.03 | 2073 | C₆₉H₁₃₈N₄O₆ | 1119.88 | 7.66 | 26.29 |
| 1874 | C₇₄H₁₄₅N₃O₇ | 1188.99 | 9.12 | 30.03 | 2074 | C₆₉H₁₃₈N₄O₆ | 1119.88 | 7.66 | 26.29 |
| 1875 | C₆₆H₁₃₅N₃O₄ | 1034.82 | 9.04 | 26.57 | 2075 | C₇₂H₁₃₈N₄O₉ | 1203.91 | 7.66 | 27.11 |
| 1876 | C₆₆H₁₃₅N₃O₄ | 1034.82 | 9.04 | 26.57 | 2076 | C₇₂H₁₃₈N₄O₉ | 1203.91 | 7.66 | 27.11 |
| 1877 | C₆₉H₁₃₅N₃O₇ | 1118.85 | 9.04 | 27.39 | 2077 | C₇₂H₁₃₈N₄O₉ | 1203.91 | 7.66 | 27.11 |
| 1878 | C₆₉H₁₃₅N₃O₇ | 1118.85 | 9.04 | 27.39 | 2078 | C₇₂H₁₃₈N₄O₉ | 1203.91 | 7.66 | 27.11 |
| 1879 | C₆₉H₁₃₅N₃O₇ | 1118.85 | 9.04 | 27.39 | 2079 | C₇₀H₁₄₀N₄O₅ | 1117.91 | 7.94 | 26.15 |
| 1880 | C₆₉H₁₃₅N₃O₇ | 1118.85 | 9.04 | 27.39 | 2080 | C₇₀H₁₄₀N₄O₅ | 1117.91 | 7.93 | 26.15 |
| 1881 | C₆₇H₁₃₇N₃O₄ | 1048.85 | 9.05 | 27.10 | 2081 | C₇₃H₁₄₀N₄O₈ | 1201.94 | 7.94 | 26.97 |
| 1882 | C₆₇H₁₃₇N₃O₄ | 1048.85 | 9.05 | 27.10 | 2082 | C₇₃H₁₄₀N₄O₈ | 1201.94 | 7.93 | 26.97 |
| 1883 | C₇₀H₁₃₇N₃O₇ | 1132.88 | 9.05 | 27.92 | 2083 | C₇₃H₁₄₀N₄O₈ | 1201.94 | 7.94 | 26.97 |
| 1884 | C₇₀H₁₃₇N₃O₇ | 1132.88 | 9.05 | 27.92 | 2084 | C₇₃H₁₄₀N₄O₈ | 1201.94 | 7.94 | 26.97 |
| 1885 | C₇₀H₁₃₇N₃O₇ | 1132.88 | 9.05 | 27.92 | 2085 | C₇₀H₁₃₈N₄O₆ | 1131.90 | 7.19 | 25.85 |
| 1886 | C₇₀H₁₃₇N₃O₇ | 1132.88 | 9.05 | 27.92 | 2086 | C₇₀H₁₃₈N₄O₆ | 1131.90 | 7.19 | 25.85 |
| 1887 | C₆₈H₁₃₉N₃O₄ | 1062.88 | 9.06 | 27.63 | 2087 | C₇₃H₁₃₈N₄O₉ | 1215.93 | 7.17 | 26.67 |
| 1888 | C₆₈H₁₃₉N₃O₄ | 1062.88 | 9.06 | 27.63 | 2088 | C₇₃H₁₃₈N₄O₉ | 1215.93 | 7.17 | 26.67 |
| 1889 | C₇₁H₁₃₉N₃O₇ | 1146.91 | 9.06 | 28.45 | 2089 | C₇₃H₁₃₈N₄O₉ | 1215.93 | 7.17 | 26.67 |
| 1890 | C₇₁H₁₃₉N₃O₇ | 1146.91 | 9.06 | 28.45 | 2090 | C₇₃H₁₃₈N₄O₉ | 1215.93 | 7.17 | 26.67 |
| 1891 | C₇₁H₁₃₉N₃O₇ | 1146.91 | 9.06 | 28.45 | 2091 | C₇₀H₁₃₉N₅O₆ | 1146.91 | 7.19 | 25.65 |
| 1892 | C₇₁H₁₃₉N₃O₇ | 1146.91 | 9.06 | 28.45 | 2092 | C₇₀H₁₃₉N₅O₆ | 1146.91 | 7.19 | 25.65 |
| 1893 | C₆₇H₁₃₇N₃O₄ | 1048.85 | 9.11 | 26.88 | 2093 | C₇₃H₁₃₉N₅O₉ | 1230.94 | 7.08 | 26.47 |
| 1894 | C₆₇H₁₃₇N₃O₄ | 1048.85 | 9.11 | 26.88 | 2094 | C₇₃H₁₃₉N₅O₉ | 1230.94 | 7.08 | 26.47 |
| 1895 | C₇₀H₁₃₇N₃O₇ | 1132.88 | 9.11 | 27.70 | 2095 | C₇₃H₁₃₉N₅O₉ | 1230.94 | 7.08 | 26.47 |
| 1896 | C₇₀H₁₃₇N₃O₇ | 1132.88 | 9.11 | 27.70 | 2096 | C₇₃H₁₃₉N₅O₉ | 1230.94 | 7.08 | 26.47 |
| 1897 | C₇₀H₁₃₇N₃O₇ | 1132.88 | 9.11 | 27.70 | 2097 | C₇₀H₁₄₁N₅O₅ | 1132.93 | 7.84 | 26.35 |
| 1898 | C₇₀H₁₃₇N₃O₇ | 1132.88 | 9.11 | 27.70 | 2098 | C₇₀H₁₄₁N₅O₅ | 1132.93 | 7.84 | 26.35 |
| 1899 | C₆₈H₁₃₉N₃O₄ | 1062.88 | 9.12 | 27.41 | 2099 | C₇₃H₁₄₁N₅O₈ | 1216.96 | 7.84 | 27.17 |
| 1900 | C₆₈H₁₃₉N₃O₄ | 1062.88 | 9.12 | 27.41 | 2100 | C₇₃H₁₄₁N₅O₈ | 1216.96 | 7.84 | 27.17 |
| 1901 | C₇₁H₁₃₉N₃O₇ | 1146.91 | 9.12 | 28.23 | 2101 | C₇₃H₁₄₁N₅O₈ | 1216.96 | 7.84 | 27.17 |
| 1902 | C₇₁H₁₃₉N₃O₇ | 1146.91 | 9.12 | 28.23 | 2102 | C₇₃H₁₄₁N₅O₈ | 1216.96 | 7.84 | 27.17 |
| 1903 | C₇₁H₁₃₉N₃O₇ | 1146.91 | 9.12 | 28.23 | 2103 | C₇₀H₁₃₈N₄O₇ | 1147.89 | 7.19 | 26.06 |
| 1904 | C₇₁H₁₃₉N₃O₇ | 1146.91 | 9.12 | 28.23 | 2104 | C₇₀H₁₃₈N₄O₇ | 1147.89 | 7.19 | 26.06 |
| 1905 | C₆₉H₁₄₁N₃O₄ | 1076.90 | 9.20 | 27.71 | 2105 | C₆₈H₁₃₅N₅O₅ | 1102.86 | 7.65 | 24.36 |
| 1906 | C₆₉H₁₄₁N₃O₄ | 1076.90 | 9.20 | 27.71 | 2106 | C₆₈H₁₃₅N₅O₅ | 1102.86 | 7.65 | 24.36 |
| 1907 | C₇₂H₁₄₁N₃O₇ | 1160.93 | 9.20 | 28.54 | 2107 | C₇₁H₁₃₅N₅O₈ | 1186.89 | 7.65 | 25.18 |
| 1908 | C₇₂H₁₄₁N₃O₇ | 1160.93 | 9.20 | 28.54 | 2108 | C₇₁H₁₃₅N₅O₈ | 1186.89 | 7.65 | 25.18 |
| 1909 | C₇₂H₁₄₁N₃O₇ | 1160.93 | 9.20 | 28.54 | 2109 | C₇₁H₁₃₅N₅O₈ | 1186.89 | 7.65 | 25.18 |
| 1910 | C₇₂H₁₄₁N₃O₇ | 1160.93 | 9.20 | 28.54 | 2110 | C₇₁H₁₃₅N₅O₈ | 1186.89 | 7.65 | 25.18 |
| 1911 | C₆₉H₁₄₁N₃O₄ | 1076.90 | 9.15 | 27.93 | 2111 | C₆₉H₁₃₆N₄O₅ | 1101.87 | 7.90 | 25.58 |
| 1912 | C₆₉H₁₄₁N₃O₄ | 1076.90 | 9.15 | 27.93 | 2112 | C₆₉H₁₃₆N₄O₅ | 1101.87 | 7.90 | 25.58 |
| 1913 | C₇₂H₁₄₁N₃O₇ | 1160.93 | 9.15 | 28.76 | 2113 | C₇₂H₁₃₆N₄O₈ | 1185.90 | 7.90 | 26.41 |
| 1914 | C₇₂H₁₄₁N₃O₇ | 1160.93 | 9.15 | 28.76 | 2114 | C₇₂H₁₃₆N₄O₈ | 1185.90 | 7.90 | 26.41 |
| 1915 | C₇₂H₁₄₁N₃O₇ | 1160.93 | 9.15 | 28.76 | 2115 | C₇₂H₁₃₆N₄O₈ | 1185.90 | 7.90 | 26.41 |
| 1916 | C₇₂H₁₄₁N₃O₇ | 1160.93 | 9.15 | 28.76 | 2116 | C₇₂H₁₃₆N₄O₈ | 1185.90 | 7.90 | 26.41 |
| 1917 | C₆₇H₁₃₅N₃O₄ | 1046.83 | 8.42 | 26.62 | 2117 | C₇₀H₁₃₇N₅O₆ | 1144.89 | 7.19 | 25.33 |
| 1918 | C₆₇H₁₃₅N₃O₄ | 1046.83 | 8.42 | 26.62 | 2118 | C₇₀H₁₃₇N₅O₆ | 1144.89 | 7.19 | 25.33 |
| 1919 | C₇₀H₁₃₅N₃O₇ | 1130.86 | 8.42 | 27.44 | 2119 | C₁₀₀H₁₉₅NO₉S₂ | 1619.78 | 9.12 | 42.45 |
| 1920 | C₇₀H₁₃₅N₃O₇ | 1130.86 | 8.42 | 27.44 | 2120 | C₉₉H₁₈₉N₃O₉ | 1565.61 | 8.77 | 41.14 |
| 1921 | C₇₀H₁₃₅N₃O₇ | 1130.86 | 8.42 | 27.44 | 2121 | C₇₃H₁₃₇N₅O₉ | 1228.92 | 7.07 | 26.15 |
| 1922 | C₇₀H₁₃₅N₃O₇ | 1130.86 | 8.42 | 27.44 | 2122 | C₇₃H₁₃₇N₅O₉ | 1228.92 | 7.07 | 26.15 |
| 1923 | C₆₉H₁₃₉N₃O₄ | 1074.89 | 8.99 | 27.51 | 2123 | C₇₁H₁₃₉N₅O₅ | 1142.92 | 7.47 | 25.63 |
| 1924 | C₆₉H₁₃₉N₃O₄ | 1074.89 | 8.99 | 27.51 | 2124 | C₇₁H₁₃₉N₅O₅ | 1142.92 | 7.47 | 25.63 |
| 1925 | C₇₂H₁₃₉N₃O₇ | 1158.92 | 8.99 | 28.33 | 2125 | C₁₀₃H₁₉₄N₂O₁₅S₂ | 1764.80 | 8.09 | 42.63 |
| 1926 | C₇₂H₁₃₉N₃O₇ | 1158.92 | 8.99 | 28.33 | 2126 | C₁₀₁H₁₉₆N₂O₁₄S | 1694.74 | 9.65 | 42.49 |
| 1927 | C₇₂H₁₃₉N₃O₇ | 1158.92 | 9.00 | 28.33 | 2127 | C₇₄H₁₃₉N₅O₈ | 1226.95 | 7.47 | 26.45 |
| 1928 | C₇₂H₁₃₉N₃O₇ | 1158.92 | 9.00 | 28.33 | 2128 | C₇₄H₁₃₉N₅O₈ | 1226.95 | 7.47 | 26.45 |
| 1929 | C₆₇H₁₃₅N₃O₄ | 1046.83 | 8.77 | 26.65 | 2129 | C₇₁H₁₃₈N₈O₄ | 1167.94 | 7.58 | 25.74 |
| 1930 | C₆₇H₁₃₅N₃O₄ | 1046.83 | 8.77 | 26.65 | 2130 | C₇₁H₁₃₈N₈O₄ | 1167.94 | 7.58 | 25.74 |
| 1931 | C₇₀H₁₃₅N₃O₇ | 1130.86 | 8.77 | 27.47 | 2131 | C₈₈H₁₇₂N₄O₇ | 1398.36 | 7.81 | 35.59 |
| 1932 | C₇₀H₁₃₅N₃O₇ | 1130.86 | 8.77 | 27.47 | 2132 | C₈₄H₁₇₁NO₇S | 1339.35 | 10.0 2 | 36.46 |
| 1933 | C₇₀H₁₃₅N₃O₇ | 1130.86 | 8.77 | 27.47 | 2133 | C₇₄H₁₃₈N₈O₇ | 1251.97 | 7.58 | 26.56 |
| 1934 | C₇₀H₁₃₅N₃O₇ | 1130.86 | 8.77 | 27.47 | 2134 | C₇₄H₁₃₈N₈O₇ | 1251.97 | 7.58 | 26.56 |
| 1935 | C₆₈H₁₃₇N₃O₄ | 1060.86 | 9.40 | 26.63 | 2135 | C₆₀H₁₀₀N₂O₈S | 1009.53 | 7.01 | 18.88 |
| 1936 | C₆₈H₁₃₇N₃O₄ | 1060.86 | 9.40 | 26.63 | 2136 | C₆₁H₁₀₅N₃O₆S₃ | 1072.71 | 8.34 | 21.30 |
| 1937 | C₇₁H₁₃₇N₃O₇ | 1144.89 | 9.40 | 27.45 | 2137 | C₆₀H₁₀₅NO₂S₄ | 1000.75 | 8.96 | 22.13 |
| 1938 | C₇₁H₁₃₇N₃O₇ | 1144.89 | 9.40 | 27.45 | 2138 | C₆₃H₁₁₄N₆O₇ | 1067.64 | 10.0 0 | 21.45 |
| 1939 | C₇₁H₁₃₇N₃O₇ | 1144.89 | 9.40 | 27.45 | 2139 | C₅₉H₁₀₁NO₁₀ | 984.45 | 8.35 | 21.09 |
| 1940 | C₇₁H₁₃₇N₃O₇ | 1144.89 | 9.40 | 27.45 | 2140 | C₆₀H₁₀₈N₄O₆S | 1013.61 | 8.40 | 19.22 |
| 1941 | C₆₈H₁₃₇N₃O₄ | 1060.86 | 9.62 | 25.87 | 2141 | C₆₄H₁₁₈N₃O₇P | 1072.64 | 8.79 | 20.87 |
| 1942 | C₆₈H₁₃₇N₃O₄ | 1060.86 | 9.62 | 25.87 | 2142 | C₅₆H₁₀₃NO₉S₄ | 1062.68 | 10.1 3 | 19.46 |
| 1943 | C₇₁H₁₃₇N₃O₇ | 1144.89 | 9.62 | 26.69 | 2143 | C₅₉H₁₀₇NO₉S₄ | 1102.74 | 9.62 | 17.16 |
| 1944 | C₇₁H₁₃₇N₃O₇ | 1144.89 | 9.62 | 26.69 | 2144 | C₆₀H₁₁₀N₂O₁₂S₃ | 1147.72 | 8.95 | 19.15 |
| 1945 | C₇₁H₁₃₇N₃O₇ | 1144.89 | 9.62 | 26.69 | 2145 | C₆₄H₁₁₃N₃O₄S₃ | 1084.80 | 10.1 7 | 23.74 |
| 1946 | C₇₁H₁₃₇N₃O₇ | 1144.89 | 9.62 | 26.69 | 2146 | C₆₀H₁₀₄N₂O₅S₃ | 1029.68 | 9.96 | 22.15 |
| 1947 | C₆₈H₁₃₇N₃O₄ | 1060.86 | 8.87 | 27.21 | 2147 | C₆₃H₁₀₉N₃O₆S₄ | 1132.82 | 9.65 | 22.68 |
| 1948 | C₆₈H₁₃₇N₃O₄ | 1060.86 | 8.87 | 27.21 | 2148 | C₆₂H₁₁₀N₂O₂S₇ | 1139.99 | 10.0 3 | 24.47 |
| 1949 | C₇₁H₁₃₇N₃O₇ | 1144.89 | 8.87 | 28.03 | 2149 | C₅₇H₁₀₂N₆O₃S | 951.54 | 8.64 | 15.85 |
| 1950 | C₇₁H₁₃₇N₃O₇ | 1144.89 | 8.87 | 28.03 | 2150 | C₅₃H₉₇NO₇ | 860.36 | 9.79 | 19.82 |
| 1951 | C₇₁H₁₃₇N₃O₇ | 1144.89 | 8.87 | 28.03 | 2151 | C₅₇H₁₀₃N₃O₂S₃ | 958.65 | 8.96 | 18.52 |
| 1952 | C₇₁H₁₃₇N₃O₇ | 1144.89 | 8.87 | 28.03 | 2152 | C₅₆H₁₀₃NS₄ | 918.69 | 10.2 1 | 21.10 |
| 1953 | C₆₈H₁₃₈N₄O₄ | 1075.88 | 8.77 | 25.07 | 2153 | C₅₆H₁₀₃NO₃S₇ | 1062.86 | 9.96 | 23.06 |
| 1954 | C₆₈H₁₃₈N₄O₄ | 1075.88 | 8.77 | 25.07 | 2154 | C₆₄H₁₁₉NO₈S₄ | 1158.89 | 8.40 | 26.14 |
| 1955 | C₇₁H₁₃₈N₄O₇ | 1159.91 | 8.77 | 25.89 | 2155 | C₆₇H₁₂₂N₄O₅S₄ | 1191.98 | 8.15 | 26.63 |
| 1956 | C₇₁H₁₃₈N₄O₇ | 1159.91 | 8.77 | 25.89 | 2156 | C₇₀H₁₂₈N₂O₅S₅ | 1238.10 | 8.80 | 28.64 |
| 1957 | C₇₁H₁₃₈N₄O₇ | 1159.91 | 8.77 | 25.89 | 2157 | C₆₇H₁₂₂N₂O₅S₄ | 1163.96 | 7.06 | 25.24 |
| 1958 | C₇₁H₁₃₈N₄O₇ | 1159.91 | 8.77 | 25.89 | 2158 | C₆₄H₁₁₇NOS₇ | 1141.07 | 9.43 | 28.02 |
| 1959 | C₆₇H₁₃₅N₃O₅ | 1062.83 | 7.19 | 26.00 | 2159 | C₇₂H₁₄₂N₇O₆P | 1232.94 | 10.1 0 | 26.28 |
| 1960 | C₆₇H₁₃₅N₃O₅ | 1062.83 | 7.19 | 26.00 | 2160 | C₇₂H₁₃₀N₆O₇S₂ | 1255.99 | 8.52 | 24.50 |
| 1961 | C₁₀₄H₁₉₇N₃O₇S ₅ | 1762.03 | 7.97 | 43.84 | 2161 | C₆₇H₁₃₀N₆O₂ | 1051.82 | 8.75 | 23.16 |
| 1962 | C₉₉H₂₀₃N₂O₁₄P ₃S₂ | 1802.75 | 7.87 | 40.39 | 2162 | C₆₉H₁₃₀N₄O₇ | 1127.82 | 9.95 | 26.16 |
| 1963 | C₇₀H₁₃₅N₃O₈ | 1146.86 | 6.84 | 26.82 | 2163 | C₇₂H₁₂₉N₉O₆S | 1248.94 | 8.63 | 24.39 |
| 1964 | C₇₀H₁₃₅N₃O₈ | 1146.86 | 6.83 | 26.82 | 2164 | C₇₀H₁₃₁NO₁₀ | 1146.82 | 10.0 1 | 27.80 |
| 1965 | C₆₉H₁₃₉N₃O₄ | 1074.89 | 9.80 | 26.43 | 2165 | C₆₇H₁₂₁N₃O₈S₃ | 1192.90 | 7.17 | 27.01 |
| 1966 | C₆₉H₁₃₉N₃O₄ | 1074.89 | 9.80 | 26.43 | 2166 | C₆₆H₁₂₃N₃O₇S | 1102.78 | 7.67 | 23.92 |
| 1967 | C₇₂H₁₃₉N₃O₇ | 1158.92 | 9.80 | 27.25 | 2167 | C₇₂H₁₄₀N₂O₁₃P₄ | 1365.81 | 8.13 | 23.27 |
| 1968 | C₇₂H₁₃₉N₃O₇ | 1158.92 | 9.80 | 27.25 | 2168 | C₇₃H₁₃₉N₄O₁₃P₃ | 1373.85 | 7.94 | 22.39 |
| 1969 | C₇₂H₁₃₉N₃O₇₊₊ | 1158.92 | 9.80 | 27.25 | 2169 | C₆₄H₁₂₅N₃O₇S₃ | 1144.90 | 9.63 | 22.96 |
| 1970 | C₇₂H₁₃₉N₃O₇ | 1158.92 | 9.80 | 27.25 | 2170 | C₆₈H₁₂₇N₃O₇S₃ | 1194.96 | 10.1 8 | 28.67 |
| 1971 | C₆₆H₁₃₅N₃O₅ | 1050.82 | 8.20 | 25.62 | 2171 | C₆₉H₁₂₉NO₃S₇ | 1245.21 | 10.0 2 | 29.71 |
| 1972 | C₆₆H₁₃₅N₃O₅ | 1050.82 | 8.20 | 25.62 | 2172 | C₆₄H₁₁₈N₂O₂S₆ | 1140.02 | 7.72 | 28.06 |
| 1973 | C₆₉H₁₃₅N₃O₈ | 1134.85 | 8.20 | 26.44 | 2173 | C₆₅H₁₂₅NO₃S₆ | 1161.08 | 9.59 | 30.57 |
| 1974 | C₆₉H₁₃₅N₃O₈ | 1134.85 | 8.20 | 26.44 | 2174 | C₆₇H₁₂₉NO₂S₇ | 1205.19 | 10.2 5 | 31.99 |
| 1975 | C₆₉H₁₃₅N₃O₈ | 1134.85 | 8.20 | 26.44 | 2175 | C₆₃H₁₂₁N₃OS₈ | 1193.16 | 8.62 | 29.09 |
| 1976 | C₆₉H₁₃₅N₃O₈ | 1134.85 | 8.20 | 26.44 | 2176 | C₅₆H₁₀₄N₄O₃S₂ | 945.59 | 10.0 8 | 17.35 |
| 1977 | C₆₇H₁₃₇N₃O₅ | 1064.85 | 8.67 | 25.91 | 2177 | C₅₅H₁₀₃N₁₁O₄ | 982.50 | 7.63 | 16.96 |
| 1978 | C₆₇H₁₃₇N₃O₅ | 1064.85 | 8.67 | 25.91 | 2178 | C₅₅H₁₀₀N₄O₈S | 977.48 | 9.61 | 18.82 |
| 1979 | C₇₀H₁₃₇N₃O₈ | 1148.88 | 8.67 | 26.73 | 2179 | C₅₇H₁₀₈N₁₀O₈S₄ | 1189.79 | 8.50 | 16.73 |
| 1980 | C₇₀H₁₃₇N₃O₈ | 1148.88 | 8.67 | 26.73 | 2180 | C₅₃H₁₀₂N₆O₁₀S₄ | 1111.67 | 8.18 | 14.64 |
| 1981 | C₇₀H₁₃₇N₃O₈ | 1148.88 | 8.67 | 26.73 | 2181 | C₅₉H₁₀₀N₆O₈ | 1021.48 | 8.42 | 18.23 |
| 1982 | C₇₀H₁₃₇N₃O₈ | 1148.88 | 8.67 | 26.73 | 2182 | C₅₄H₉₈N₂O₈ | 903.38 | 8.85 | 20.07 |
| 1983 | C₆₈H₁₃₉N₃O₅ | 1078.88 | 8.87 | 25.64 | 2183 | C₅₆H₁₀₄N₄O₁₀S | 1025.53 | 9.53 | 19.39 |
| 1984 | C₆₈H₁₃₉N₃O₅ | 1078.88 | 8.87 | 25.64 | 2184 | C₅₃H₉₈N₄O₇S₂ | 967.51 | 8.78 | 17.62 |
| 1985 | C₇₁H₁₃₉N₃O₈ | 1162.91 | 8.87 | 26.46 | 2185 | C₅₄H₁₀₃NO₆S | 894.48 | 9.81 | 18.88 |
| 1986 | C₉₃H₁₈₇NO₈S₆ | 1639.88 | 8.87 | 39.61 | 2186 | C₆₀H₁₁₅N₄O₁₂P₃ S | 1209.58 | 7.38 | 16.63 |
| 1987 | C₇₁H₁₃₉N₃O₈ | 1162.91 | 8.87 | 26.46 | 2187 | C₄₈H₉₁NO₁₄S₄ | 1034.49 | 8.57 | 18.09 |
| 1988 | C₇₁H₁₃₉N₃O₈ | 1162.91 | 8.87 | 26.46 | 2188 | C₅₃H₉₇N₃O₇S₃ | 984.55 | 7.65 | 15.34 |
| 1989 | C₆₈H₁₃₈N₄O₆ | 1107.87 | 7.80 | 25.43 | 2189 | C₅₃H₁₀₀N₄O₁₀S₄ | 1081.64 | 8.02 | 15.11 |
| 1990 | C₆₈H₁₃₈N₄O₆ | 1107.87 | 7.80 | 25.43 | 2190 | C₅₉H₁₀₄N₂O₈S₄ | 1097.73 | 8.07 | 20.70 |
| 1991 | C₉₈H₁₉₅N₅O₅S₃ | 1619.85 | 7.45 | 41.60 | 2191 | C₅₇H₁₀₃NO₆S₄ | 1026.69 | 10.1 6 | 22.82 |
| 1992 | C₉₈H₁₉₃NO₁₀S₃ | 1641.80 | 9.82 | 42.89 | 2192 | C₅₇H₁₀₃NO₆S₆ | 1090.81 | 8.72 | 25.21 |
| 1993 | C₇₁H₁₃₈N₄O₉ | 1191.90 | 7.80 | 26.25 | 2193 | C₅₇H₁₀₃NO₆S₇ | 1122.87 | 9.87 | 23.86 |
| 1994 | C₇₁H₁₃₈N₄O₉ | 1191.90 | 7.80 | 26.25 | 2194 | C₅₇H₉₉N₃O₈S₇ | 1178.85 | 7.78 | 21.15 |
| 1995 | C₆₉H₁₃₇N₅O₅ | 1116.88 | 7.49 | 25.53 | 2195 | C₅₆H₁₀₉N₂O₃PS₆ | 1081.83 | 8.96 | 22.34 |
| 1996 | C₆₉H₁₃₇N₅O₅ | 1116.88 | 7.49 | 25.53 | 2196 | C₆₉H₁₂₁NO₅S | 1076.79 | 9.98 | 23.52 |
| 1997 | C₇₂H₁₃₇N₅O₈ | 1200.91 | 7.49 | 26.35 | 2197 | C₇₀H₁₂₂N₄O₂S | 1147.95 | 8.85 | 22.42 |
| 1998 | C₇₂H₁₃₇N₅O₈ | 1200.91 | 7.49 | 26.35 | 2198 | C₇₂H₁₂₃N₉O₇ | 1226.83 | 8.52 | 18.69 |
| 1999 | C₇₂H₁₃₇N₅O₈ | 1200.91 | 7.49 | 26.35 | 2199 | C₆₇H₁₁₇N₅O₅ | 1072.70 | 8.36 | 19.23 |
| 2000 | C₇₂H₁₃₇N₅O₈ | 1200.91 | 7.49 | 26.35 | 2200 | C₆₈H₁₂₇N₁₁O₄ | 1162.84 | 9.49 | 19.64 |
| 2201 | C₆₈H₁₂₅N₇O₅ | 1120.79 | 9.91 | 22.00 | 2401 | C₇₇H₁₅₇N₃O₁₀S₃ | 1381.31 | 10.2 9 | 31.76 |
| 2202 | C₇₀H₁₂₆N₁₀O₇S | 1251.90 | 7.99 | 20.23 | 2402 | C₇₇H₁₄₈N₄O₇S₇ | 1466.52 | 7.62 | 33.01 |
| 2203 | C₆₇H₁₁₈N₈O₈ | 1163.73 | 7.71 | 21.54 | 2403 | C₇₆H₁₄₇NO₂S₆ | 1299.42 | 10.0 2 | 34.04 |
| 2204 | C₆₇H₁₂₃N₇O₂ | 1058.77 | 8.87 | 20.62 | 2404 | C₇₆H₁₅₃NOS₈ | 1353.60 | 10.0 9 | 38.26 |
| 2205 | C₆₇H₁₂₂N₁₀O | 1083.78 | 8.58 | 19.27 | 2405 | C₇₅H₁₄₉N₃O₂S₈ | 1381.57 | 8.73 | 35.21 |
| 2206 | C₆₃H₁₁₈N₄O₆S₅ | 1187.96 | 9.68 | 21.42 | 2406 | C₉₉H₁₉₃N₇O₆ | 1577.68 | 8.36 | 38.16 |
| 2207 | C₆₅H₁₁₁NO₇S | 1050.66 | 8.10 | 22.75 | 2407 | C₉₉H₁₈₉N₃ O₅S₃ | 1597.82 | 8.77 | 42.25 |
| 2208 | C₇₀H₁₁₉N₃O₉S₅ | 1307.03 | 7.99 | 25.56 | 2408 | C₉₆H₁₈₉NO₃S₅ | 1565.91 | 9.91 | 45.50 |
| 2209 | C₆₇H₁₁₈N₂O₁₂S ₅ | 1303.98 | 8.08 | 20.95 | 2409 | C₁₀₆H₂₀₅NO₅ | 1573.81 | 9.90 | 46.33 |
| 2210 | C₆₄H₁₁₇NO₁₃S₃ | 1204.81 | 9.78 | 23.12 | 2410 | C₁₀₇H₂₀₉N₅O₅ | 1645.88 | 8.96 | 44.62 |
| 2211 | C₆₁H₁₁₂N₂O₁₄S ₃ | 1193.75 | 9.49 | 22.15 | 2411 | C₁₀₅H₂₀₄N₆O₃S₂ | 1662.96 | 8.70 | 42.53 |
| 2212 | C₇₂H₁₃₃N₄O₁₂P ₃ | 1339.79 | 8.12 | 20.87 | 2412 | C₁₀₆H₂₀₅N₃O₉ | 1665.82 | 8.80 | 45.11 |
| 2213 | C₆₈H₁₂₈NO₁₀P₃ S₂ | 1276.81 | 9.04 | 21.27 | 2413 | C₁₀₄H₂₀₄N₂O₃S₃ | 1626.99 | 9.75 | 44.82 |
| 2214 | C₆₅H₁₁₇NO₇S₄ | 1152.89 | 9.86 | 19.82 | 2414 | C₁₀₄H₂₀₁NOS₇ | 1706.23 | 9.86 | 49.18 |
| 2215 | C₆₆H₁₂₀N₆O₈S₃ | 1221.90 | 9.07 | 19.62 | 2415 | C₁₀₅H₂₀₄N₃O₅PS ₆ | 1812.18 | 7.51 | 47.43 |
| 2216 | C₆₈H₁₂₄N₂O₁₀S ₅ | 1290.04 | 9.08 | 22.31 | 2416 | C₁₀₅H₂₀₆N₆O₆S | 1680.91 | 8.82 | 42.03 |
| 2217 | C₆₃H₁₁₂N₄O₂S₆ | 1149.97 | 7.37 | 24.03 | 2417 | C₁₀₃H₂₀₄N₈O₄S₂ | 1682.95 | 8.74 | 43.18 |
| 2218 | C₇₀H₁₃₁N₃O₄S₂ | 1142.95 | 8.36 | 25.63 | 2418 | C₁₀₆H₂₁₁N₇O₂ | 1615.90 | 8.51 | 43.80 |
| 2219 | C₆₇H₁₃₀N₂O₅ | 1043.79 | 9.54 | 27.28 | 2419 | C₁₀₃H₂₁₁N₄O₃P | 1584.82 | 9.91 | 45.36 |
| 2220 | C₆₈H₁₃₁NO₅S₄ | 1171.04 | 8.83 | 28.53 | 2420 | C₁₀₄H₂₀₈N₆O₆ | 1638.84 | 8.96 | 43.83 |
| 2221 | C₈₇H₁₆₉NO₅S | 1341.37 | 9.31 | 36.31 | 2421 | C₁₀₇H₂₀₈N₆O₁₀S | 1770.94 | 8.19 | 44.86 |
| 2222 | C₈₈H₁₇₂N₂O₅ | 1338.35 | 9.01 | 37.81 | 2422 | C₁₀₂H₂₀₂N₄O₈S | 1644.82 | 9.95 | 44.99 |
| 2223 | C₈₇H₁₆₆NO₆S₃ | 1421.49 | 9.94 | 39.01 | 2423 | C₁₀₆H₂₀₈N₄O₇S₃ | 1747.05 | 9.64 | 47.63 |
| 2224 | C₉₂H₁₇₅N₆O₇P S₃ | 1604.60 | 7.83 | 37.76 | 2424 | C₁₀₆H₂₀₇N₄O₉PS ₃ | 1809.02 | 8.18 | 47.75 |
| 2225 | C₈₇H₁₇₀N₄O₂S₆ | 1496.70 | 8.30 | 39.06 | 2425 | C₁₀₂H₂₀₁NO₉S₄ | 1714.00 | 9.33 | 48.93 |
| 2226 | C₈₂H₁₆₈N₄OS | 1258.33 | 9.43 | 35.75 | 2426 | C₁₀₄H₂₀₁NO₇S₄ | 1706.02 | 9.76 | 48.92 |
| 2227 | C₈₉H₁₇₃NO₉S₄ | 1529.60 | 9.78 | 39.84 | 2427 | C₁₀₂H₂₀₃NO₅ | 1523.75 | 9.86 | 45.85 |
| 2228 | C₈₇H₁₇₅NO₁₆S₄ | 1619.59 | 9.94 | 38.90 | 2428 | C₁₀₈H₂₁₄N₇O₁₂P₃ | 1895.86 | 7.23 | 41.61 |
| 2229 | C₈₉H₁₇₇NO₁₃S₄ | 1597.63 | 10.04 | 39.39 | 2429 | C₁₀₀H₁₉₉N₃O₁₅S₅ | 1844.03 | 7.71 | 46.10 |
| 2230 | C₈₅H₁₇₂N₆O₁₀S ₄ | 1566.58 | 8.43 | 34.65 | 2430 | C₉₉H₁₉₉NO₁₄S₅ | 1788.01 | 8.79 | 44.17 |
| 2231 | C₈₅H₁₇₅N₅O₉S₃ | 1507.54 | 9.58 | 34.71 | 2431 | C₁₀₃H₂₀₆N₆O₇S₄ | 1769.08 | 8.96 | 44.54 |
| 2232 | C₈₅H₁₇₄N₂O₁₁S ₄ | 1528.60 | 8.94 | 37.92 | 2432 | C₁₀₃H₂₀₉N₆O₁₀P S₃ | 1819.01 | 8.49 | 41.31 |
| 2233 | C₈₅H₁₇₁N₃O₁₀S ₄ | 1523.58 | 8.67 | 35.43 | 2433 | C₉₉H₂₀₁N₃O₁₀S₃ | 1689.91 | 9.61 | 44.80 |
| 2234 | C₉₀H₁₇₃N₃O₆S₄ | 1521.66 | 8.82 | 38.34 | 2434 | C₁₀₁H₂₀₅NO₁₀S₅ | 1754.08 | 9.99 | 43.99 |
| 2235 | C₉₀H₁₇₃NO₃S₈ | 1573.91 | 9.95 | 43.51 | 2435 | C₁₀₄H₁₉₉N₃O₅S₅ | 1732.09 | 7.64 | 46.37 |
| 2236 | C₇₂H₁₄₀N₆O₅ | 1169.95 | 8.47 | 24.78 | 2436 | C₁₀₃H₁₉₉N₃O₉S₄ | 1752.00 | 7.51 | 47.66 |
| 2237 | C₆₆H₁₃₇N₇O₂ | 1060.87 | 8.33 | 24.92 | 2437 | C₉₇H₁₉₃N₇O₅S | 1569.72 | 9.22 | 38.28 |
| 2238 | C₇₃H₁₄₁N₃O₇S₂ | 1237.08 | 9.74 | 28.32 | 2438 | C₉₃H₁₈₈N₆O₇S₄ | 1630.83 | 7.70 | 37.71 |
| 2239 | C₆₈H₁₃₆N₄O₈ | 1137.86 | 9.16 | 26.01 | 2439 | C₁₀₀H₁₉₆N₄O₈ | 1582.69 | 9.91 | 43.00 |
| 2240 | C₇₀H₁₄₀N₄O₁₀S | 1229.97 | 9.11 | 27.00 | 2440 | C₉₉H₁₉₂N₂O₉S₄ | 1682.90 | 8.81 | 44.24 |
| 2241 | C₆₈H₁₃₃NO₁₁S | 1172.87 | 9.51 | 27.24 | 2441 | C₉₄H₁₉₁NO₃S | 1415.63 | 10.1 0 | 42.86 |
| 2242 | C₆₉H₁₃₁NO₁₀S | 1166.87 | 9.12 | 27.95 | 2442 | C₁₀₀H₂₀₅N₂O₁₁P₃ | 1704.66 | 10.1 4 | 43.84 |
| 2243 | C₆₇H₁₃₅NO₄S | 1050.89 | 9.96 | 27.64 | 2443 | C₉₂H₁₈₆N₂O₁₁S₃ | 1592.70 | 8.75 | 40.86 |
| 2244 | C₇₀H₁₄₅N₂O₁₂P ₃ | 1299.85 | 7.89 | 25.09 | 2444 | C₉₄H₁₈₇N₃O₁₅S₃ | 1695.74 | 7.43 | 40.74 |
| 2245 | C₇₄H₁₅₂NO₁₂P₃ S | 1373.01 | 9.94 | 27.12 | 2445 | C₉₃H₁₉₀N₂O₆S₃ | 1528.75 | 10.0 6 | 38.72 |
| 2246 | C₆₉H₁₃₈N₂O₁₀S ₃ | 1252.07 | 9.92 | 27.38 | 2446 | C₉₄H₁₈₇N₃O₁₂S₄ | 1679.81 | 7.37 | 39.57 |
| 2247 | C₆₉H₁₃₉NO₁₅S₄ | 1351.13 | 9.90 | 28.99 | 2447 | C₁₀₁H₁₉₆N₃O₇PS ₆ | 1788.07 | 8.79 | 44.46 |
| 2248 | C₇₂H₁₃₈N₆O₁₃S ₅ | 1456.26 | 8.64 | 26.43 | 2448 | C₉₄H₁₈₇NOS₆ | 1539.94 | 9.73 | 45.76 |
| 2249 | C₇₀H₁₃₇N₃O₉S₃ | 1261.08 | 8.66 | 22.65 | 2449 | C₇₇H₁₂₀N₆O₃ S₂ | 1241.98 | 8.70 | 26.41 |
| 2250 | C₆₇H₁₃₈N₆O₇S₃ | 1236.08 | 8.87 | 26.36 | 2450 | C₇₅H₁₂₄N₄O₆ | 1177.84 | 10.2 0 | 25.90 |
| 2251 | C₆₉H₁₃₇N₃O₁₃S ₃ | 1313.06 | 8.54 | 25.97 | 2451 | C₇₂H₁₁₅NO₆S₂ | 1154.85 | 10.0 6 | 27.89 |
| 2252 | C₇₂H₁₃₇N₃O₂S₆ | 1269.31 | 8.65 | 29.55 | 2452 | C₇₆H₁₂₅N₅O₄S₃ | 1269.07 | 9.66 | 26.77 |
| 2253 | C₇₄H₁₄₆NO₃PS ₆ | 1321.36 | 10.13 | 32.30 | 2453 | C₇₅H₁₁₇N₃O₂S₈ | 1349.32 | 7.66 | 27.75 |
| 2254 | C₉₀H₁₄₇N₃O₈ | 1399.18 | 7.90 | 32.42 | 2454 | C₇₇H₁₂₃N₅O₆S | 1246.93 | 8.19 | 24.95 |
| 2255 | C₈₉H₁₄₉NO₆S | 1361.24 | 9.32 | 34.59 | 2455 | C₇₆H₁₂₇N₇O₄ | 1202.90 | 9.00 | 24.97 |
| 2256 | C₈₈H₁₄₆N₂O₇S | 1376.21 | 6.19 | 33.17 | 2456 | C₇₅H₁₂₆N₆O₈ | 1239.87 | 9.84 | 25.43 |
| 2257 | C₈₈H₁₅₀N₄O₄S | 1360.26 | 10.02 | 32.47 | 2457 | C₆₈H₁₁₈N₄O₃S | 1071.78 | 9.27 | 26.81 |
| 2258 | C₉₂H₁₅₄N₆O₃S₃ | 1488.48 | 9.03 | 34.03 | 2458 | C₇₄H₁₂₈N₄O₈S₄ | 1330.13 | 10.3 3 | 26.97 |
| 2259 | C₈₉H₁₅₀N₂O₇ | 1360.19 | 10.09 | 35.36 | 2459 | C₇₄H₁₁₉NO₉ | 1166.76 | 10.0 0 | 26.77 |
| 2260 | C₈₈H₁₄₄N₂O₇S | 1374.19 | 8.78 | 34.21 | 2460 | C₇₂H₁₁₈N₂O₁₀ | 1171.74 | 9.32 | 24.67 |
| 2261 | C₉₀H₁₅₅N₇O₅ | 1415.27 | 9.54 | 32.12 | 2461 | C₇₈H₁₂₁N₃O₁₂S₂ | 1356.97 | 8.21 | 25.60 |
| 2262 | C₉₀H₁₅₈N₅O₅P | 1421.26 | 8.10 | 33.64 | 2462 | C₈₀H₁₃₇N₂O₁₁P3 | 1395.90 | 8.95 | 25.51 |
| 2263 | C₉₀H₁₅₃N₇O₉S | 1509.32 | 8.41 | 32.06 | 2463 | C₇₇H₁₃₅N₄O₁₀P₃ S | 1401.93 | 7.50 | 25.37 |
| 2264 | C₉₃H₁₆₄N₃O₁₁P ₃S | 1625.33 | 8.74 | 32.17 | 2464 | C₆₉H₁₁₈N₄O₉S₄ | 1275.99 | 7.50 | 25.37 |
| 2265 | C₉₂H₁₆₄NO₁₄P₃ S | 1633.31 | 7.95 | 33.25 | 2465 | C₇₁H₁₁₉NO₁₃S₄ | 1322.99 | 9.94 | 25.39 |
| 2266 | C₈₈H₁₅₀N₄O₁₂S ₄ | 1584.45 | 8.03 | 31.72 | 2466 | C₇₂H₁₁₅N₃O₃S₄ | 1199.00 | 8.42 | 26.27 |
| 2267 | C₈₈H₁₅₃N₃O₁₃S ₃ | 1557.40 | 9.96 | 35.94 | 2467 | C₇₃H₁₁₈N₂O₂S₇ | 1280.23 | 9.68 | 28.94 |
| 2268 | C₉₃H₁₆₇N₂O₁₁P ₃ | 1582.28 | 10.00 | 33.87 | 2468 | C₇₀H₁₃₃NO₄S₂ | 1116.97 | 9.97 | 28.02 |
| 2269 | C₉₇H₁₆₈N₃O₁₂P ₃S | 1693.41 | 8.60 | 32.71 | 2469 | C₆₇H₁₂₉NO₇ | 1060.77 | 8.51 | 28.62 |
| 2270 | C₆₇H₁₅₁N₃O₉S₃ | 1479.38 | 8.83 | 30.34 | 2470 | C₆₈H₁₂₇N₃O₂S₃ | 1114.98 | 8.46 | 26.40 |
| 2271 | C₈₇H₁₅₄N₆O₇S₅ | 1556.56 | 8.94 | 32.10 | 2471 | C₆₆H₁₂₄N₄S₄ | 1102.01 | 7.24 | 26.38 |
| 2272 | C₈₇H₁₄₃NO₅S₇ | 1507.57 | 9.36 | 38.83 | 2472 | C₆₇H₁₃₅N₇O₃S | 1118.93 | 8.80 | 26.76 |
| 2273 | C₈₈H₁₄₄N₂O₅S₆ | 1502.53 | 8.66 | 36.59 | 2473 | C₆₅H₁₃₃N₅O₂ | 1016.81 | 9.94 | 25.89 |
| 2274 | C₈₉H₁₅₃N₅OS₃ | 1405.44 | 9.38 | 36.17 | 2474 | C₆₂H₁₂₇N₅O₈S₃ | 1166.93 | 8.18 | 24.53 |
| 2275 | C₈₈H₁₅₂N₄O₂S₄ | 1426.48 | 8.67 | 36.49 | 2475 | C₆₇H₁₃₈N₄O₆S₅ | 1256.20 | 9.66 | 27.43 |
| 2276 | C₅₃H₁₀₁N₇O₅ | 916.43 | 9.37 | 14.69 | 2476 | C₆₅H₁₃₄N₆O₇S₅ | 1272.16 | 8.65 | 24.58 |
| 2277 | C₄₈H₉₁NO₉S | 858.32 | 7.45 | 18.24 | 2477 | C₆₉H₁₂₉N₅O₈ | 1156.82 | 7.35 | 25.88 |
| 2278 | C₄₉H₉₃NO₇S | 840.35 | 9.87 | 18.01 | 2478 | C₆₄H₁₂₃NO₁₀S | 1098.75 | 8.66 | 26.59 |
| 2279 | C₅₀H₉₂N₄O₆S₃ | 941.51 | 8.18 | 17.07 | 2479 | C₆₈H₁₃ON₄O₈S₅ | 1292.14 | 8.76 | 29.68 |
| 2280 | C₇₃H₁₃₅NO₅ | 1106.88 | 9.49 | 28.47 | 2480 | C₇₀H₁₂₉N₃O₉S₄ | 1285.08 | 7.88 | 28.40 |
| 2281 | C₇₀H₁₃₂N₄O₃S₂ | 1141.98 | 9.64 | 24.97 | 2481 | C₇₁H₁₄₆NO₁₃P₃S | 1346.93 | 9.92 | 26.28 |
| 2282 | C₇₀H₁₂₆N₄O₅S₃ | 1200.00 | 8.18 | 25.90 | 2482 | C₆₅H₁₃₁NO₇S₅ | 1199.10 | 9.82 | 22.46 |
| 2283 | C₇₆H₁₃₈N₃O₈P S₃ | 1349.13 | 8.02 | 27.79 | 2483 | C₆₂H₁₂₈N₄O₇S₅ | 1202.06 | 9.66 | 24.80 |
| 2284 | C₇₀H₁₂₅N₃O₂S₄ | 1169.06 | 8.09 | 24.80 | 2484 | C₆₇H₁₃₆N₂O₂S₃ | 1098.04 | 9.60 | 31.20 |
| 2285 | C₆₉H₁₃₀Na₄O₅S | 1127.89 | 8.62 | 22.64 | 2485 | C₆₇H₁₃₄N₄O₂S₆ | 1220.24 | 8.81 | 29.65 |
| 2286 | C₆₆H₁₂₉N₇O₄S | 1116.87 | 8.42 | 23.80 | 2486 | C₅₀H₉₆N₄O₅ | 833.34 | 7.85 | 14.83 |
| 2287 | C₆₉H₁₃₁N₅O₈S | 1190.90 | 8.72 | 26.48 | 2487 | C₄₉H₁₀₁N₈O₈P | 961.37 | 7.10 | 14.90 |
| 2288 | C₆₉H₁₃₂N₄O₉S | 1193.90 | 9.26 | 26.76 | 2488 | C₄₈H₁₀₀NO₄S | 765.42 | 10.1 7 | 17.18 |
| 2289 | C₆₇H₁₃₄N₄O₂S | 1059.90 | 9.61 | 25.59 | 2489 | C₅₀H₉₉N₅O₁₂S₃ | 1058.57 | 7.89 | 15.01 |
| 2290 | C₇₁H₁₃₈N₆O₈S₃ | 1300.12 | 8.97 | 23.96 | 2490 | C₅₃H₁₀₀N₆O₉ | 965.42 | 8.13 | 16.59 |
| 2291 | C₆₈H₁₃₆N₄O₁₀S ₄ | 1298.12 | 9.75 | 23.58 | 2491 | C₄₉H₉₆N₆O₇ | 881.34 | 8.08 | 16.13 |
| 2292 | C₇₂H₁₃₀N₄O₉S₂ | 1259.98 | 8.25 | 26.34 | 2492 | C₅₂H₉₆N₄O₇S₄ | 1017.63 | 8.99 | 20.64 |
| 2293 | C₇₀H₁₂₇N₃O₁₁ | 1186.80 | 8.54 | 23.99 | 2493 | C₅₀H₉₁N₃O₇S₅ | 1006.63 | 8.54 | 18.41 |
| 2294 | C₇₂H₁₃₁NO₉S₃ | 1251.04 | 7.85 | 29.49 | 2494 | C₄₆H₉₃NO₅S | 772.32 | 9.48 | 16.00 |
| 2295 | C₇₀H₁₃₆NO₆P | 1118.83 | 9.96 | 26.88 | 2495 | C₄₉H₁₀₂NO₁₀P₃S ₂ | 1022.41 | 9.45 | 15.88 |
| 2296 | C₇₅H₁₅₀NO₁₁P₃ S | 1367.01 | 10.01 | 26.33 | 2496 | C₄₄H₉₀N₂O₁₁S₅ | 983.54 | 7.61 | 14.61 |
| 2297 | C₆₈H₁₂₉N₅O₁₀S ₃ | 1273.01 | 8.02 | 23.99 | 2497 | C₅₀H₉₇N₃O₁₃S₅ | 1108.67 | 9.10 | 18.90 |
| 2298 | C₆₂H₁₂₁NO₇S₃ | 1088.85 | 8.69 | 22.15 | 2498 | C₄₆H₉₆N₄₀O₇S₅ | 977.63 | 9.97 | 15.88 |
| 2299 | C₇₂H₁₃₄N₂O₄S₃ | 1188.07 | 8.69 | 30.53 | 2499 | C₅₁H₉₇NO₈S₃ | 948.54 | 9.74 | 20.24 |
| 2300 | C₇₀H₁₃₀N₂O₈S₄ | 1256.08 | 9.65 | 29.21 | 2500 | C₄₈H₈₉NO₉S₃ | 920.44 | 7.12 | 19.34 |
| 2301 | C₇₀H₁₂₉NO₆S₄ | 1209.07 | 9.97 | 29.90 | 2501 | C₅₀H₉₈N₂O₅S₇ | 1031.81 | 10.0 1 | 21.72 |
| 2302 | C₇₃H₁₃₃N₃O₆S₄ | 1277.15 | 8.91 | 29.86 | 2502 | C₄₉H₈₉N₃O₅S₇ | 1024.74 | 7.38 | 20.15 |
| 2303 | C₇₀H₁₂₇N₃O₅S₇ | 1315.27 | 8.15 | 29.29 | 2503 | C₈₈H₁₇₄N₄O₁₀S | 1480.44 | 8.64 | 37.76 |
| 2304 | C₆₉H₁₃₃NO₂S₄ | 1137.10 | 10.08 | 29.80 | 2504 | C₉₁H₁₈₀NO₁₂P | 1511.41 | 9.64 | 38.49 |
| 2305 | C₇₂H₁₄₂N₂O₉S | 1212.00 | 10.03 | 27.69 | 2505 | C₈₇H₁₇₂N₂O₁₃S₃ | 1550.51 | 7.44 | 36.68 |
| 2306 | C₇₀H₁₃₅NO₂S₃ | 1119.06 | 9.94 | 28.41 | 2506 | C₈₉H₁₇₇NO₁₃S₄ | 1597.63 | 9.89 | 39.95 |
| 2307 | C₇₄H₁₄₃NS₄ | 1175.23 | 10.31 | 30.33 | 2507 | C₈₇H₁₇₉N₅O₈S₄ | 1551.66 | 9.72 | 35.89 |
| 2308 | C₇₃H₁₄₂N₆O₉S | 1280.04 | 8.87 | 27.51 | 2508 | C₉₁H₁₇₃N₃O₅S₇ | 1613.82 | 8.49 | 39.46 |
| 2309 | C₇₁H₁₄₃N₇O₂ | 1126.97 | 8.80 | 25.07 | 2509 | C₈₆H₁₇₃NO₂S₃ | 1349.52 | 8.69 | 40.39 |
| 2310 | C₆₇H₁₃₆N₆O₄ | 1089.86 | 7.38 | 25.24 | 2510 | C₈₇H₁₇₇NO₃S₄ | 1413.62 | 9.80 | 39.79 |
| 2311 | C₆₅H₁₃₆N₄O₂S | 1037.90 | 9.89 | 25.86 | 2511 | C₆₈H₁₃₈N₆OS | 1087.95 | 8.34 | 25.64 |
| 2312 | C₇₀H₁₃₃NO₆S | 1116.90 | 9.78 | 27.91 | 2512 | C₇₃H₁₄₁N₅O₇S | 1233.02 | 8.19 | 27.42 |
| 2313 | C₇₂H₁₃₉NO₈ | 1146.90 | 8.29 | 30.37 | 2513 | C₇₆H₁₄₈N₆O₁₀ | 1306.05 | 8.77 | 28.04 |
| 2314 | C₇₂H₁₃₇N₃O₈S | 1204.97 | 8.10 | 27.01 | 2514 | C₇₇H₁₄₅N₅O₁₂ | 1333.03 | 8.22 | 27.36 |
| 2315 | C₇₀H₁₃₅NO₈S₃ | 1215.05 | 8.87 | 31.23 | 2515 | C₇₂H₁₃₉NO₇S₄ | 1259.14 | 9.28 | 30.05 |
| 2316 | C₆₉H₁₃₄N₂O₉S₃ | 1232.04 | 8.56 | 29.17 | 2516 | C₇₄H₁₄₈N₃O₆P | 1206.99 | 9.19 | 27.09 |
| 2317 | C₆₇H₁₃₁N₃O₆ | 1074.80 | 8.05 | 24.99 | 2517 | C₇₃H₁₄₃N₃O₇S₅ | 1335.26 | 9.00 | 27.79 |
| 2318 | C₇₁H₁₄₁N₃O₆ | 1132.92 | 8.87 | 27.00 | 2518 | C₇₄H₁₄₄N₂O₅S₅ | 1302.27 | 8.94 | 31.06 |
| 2319 | C₆₉H₁₃₈N₄O₁₅S ₃ | 1360.08 | 8.57 | 27.02 | 2519 | C₇₄H₁₄₁NO₈S₄ | 1301.18 | 9.84 | 32.75 |
| 2320 | C₇₆H₁₅₂N₃O₁₁P ₃S₂ | 1441.12 | 8.65 | 25.02 | 2520 | C₇₅H₁₄₃N₅O₆S₆ | 1403.36 | 8.02 | 29.84 |
| 2321 | C₇₀H₁₄₅N₅O₆S₃ | 1249.16 | 10.17 | 27.30 | 2521 | C₇₅H₁₄₃NOS₆ | 1267.34 | 10.0 3 | 31.57 |
| 2322 | C₇₀H₁₃₃NO₅S₇ | 1293.30 | 9.72 | 32.57 | 2522 | C₆₉H₁₃₅N₃OS₈ | 1231.28 | 9.26 | 31.21 |
| 2323 | C₉₈H₁₉₃N₃O₅ | 1493.64 | 9.64 | 41.19 | 2523 | C₆₉H₁₃₅N₃OS₈ | 1279.34 | 8.11 | 32.33 |
| 2324 | C₉₇H₁₉₂N₂O₃ | 1434.61 | 9.81 | 43.28 | 2524 | C₉₁H₁₅₁NO₆ | 1355.21 | 9.47 | 35.21 |
| 2325 | C₁₀₀H₁₉₃N₃O₃S ₅ | 1646.00 | 9.08 | 44.26 | 2525 | C₈₉H₁₅₁NO₈S | 1395.25 | 9.10 | 33.28 |
| 2326 | C₁₀₀H₁₉₄N₂O₂S ₄ | 1584.93 | 8.96 | 42.63 | 2526 | C₉₃H₁₅₆N₆O₅ | 1438.31 | 8.90 | 31.09 |
| 2327 | C₉₇H₁₉₀N₂OS₆ | 1593.00 | 7.95 | 44.89 | 2527 | C₉₀H₁₄₉NO₈S | 1405.24 | 9.77 | 36.24 |
| 2328 | C₁₀₁H₁₉₈N₈O₆S | 1652.81 | 9.03 | 41.03 | 2528 | C₉₁H₁₅₇N₇O₄ | 1413.30 | 9.81 | 33.65 |
| 2329 | C₉₈H₁₉₃N₅O₈ | 1569.65 | 9.94 | 42.17 | 2529 | C₉₃H₁₅₈N₆O₉S | 1536.38 | 9.06 | 33.18 |
| 2330 | C₉₈H₁₉₆N₆O₂S | 1522.75 | 8.97 | 42.54 | 2530 | C₈₈H₁₅₄N₆O₂ | 1328.24 | 9.69 | 33.16 |
| 2331 | C₉₅H₁₈₇NO₈S | 1503.61 | 8.62 | 43.07 | 2531 | C₈₉H₁₅₈N₄O₇S₅ | 1556.56 | 9.19 | 36.70 |
| 2332 | C₁₀₀H₁₉₆N₄O₁₀ | 1614.69 | 8.95 | 42.32 | 2532 | C₉₁H₁₅₅N₅O₈ | 1447.27 | 9.75 | 33.92 |
| 2333 | C₉₆H₁₈₉NO₉S₂ | 1565.70 | 9.90 | 43.36 | 2533 | C₉₁H₁₅₃N₃O₁₀S | 1481.30 | 9.45 | 33.16 |
| 2334 | C₉₈H₁₈₉NO₈S₃ | 1605.79 | 7.84 | 44.98 | 2534 | C₈₈H₁₅₄N₄O₅ | 1348.22 | 9.44 | 33.71 |
| 2335 | C₁₀₄H₂₁₀N₃O₁₂ P₃ | 1787.75 | 8.95 | 40.77 | 2535 | C₈₅H₁₄₉NO₁₁S₄ | 1489.36 | 9.07 | 32.92 |
| 2336 | C₉₅H₁₉₂NO₉PS ₃ | 1619.75 | 9.61 | 38.95 | 2536 | C₈₃H₁₄₅NO₂S₃ | 1285.26 | 8.63 | 34.83 |
| 2337 | C₉₆H₁₉₆N₆O₉S₃ | 1674.86 | 9.53 | 39.50 | 2537 | C₈₉H₁₅₂N₄O₂S₆ | 1502.58 | 8.69 | 33.48 |
| 2338 | C₉₆H₁₉₄N₆O₉S₄ | 1704.91 | 8.66 | 39.31 | 2538 | C₈₇H₁₅₃NO₄S₇ | 1501.60 | 9.11 | 35.87 |
| 2339 | C₉₇H₁₉₇NO₉S₅ | 1681.98 | 10.29 | 43.64 | 2539 | C₉₀H₁₅₃N₃O₂S₇ | 1533.65 | 8.67 | 35.66 |
| 2340 | C₁₀₀H₁₉₃N₅O₉S ₃ | 1705.87 | 8.02 | 43.88 | 2540 | C₉₀H₁₅₅N₃O₃S₇ | 1551.67 | 8.78 | 37.08 |
| 2341 | C₉₃H₁₈₅NO₂S₄ | 1477.78 | 8.83 | 44.69 | 2541 | C₅₀H₉₂N₄O₈ | 877.31 | 7.61 | 14.76 |
| 2342 | C₉₆H₁₉₅NS₅ | 1523.96 | 10.32 | 46.50 | 2542 | C₅₄H₁₀₁N₃O₁₀S | 984.47 | 8.61 | 17.32 |
| 2343 | C₉₆H₁₉₂N₄O₂S₇ | 1659.09 | 9.15 | 45.27 | 2543 | C₅₅H₁₀₇N₃OS₃ | 922.66 | 10.3 5 | 19.69 |
| 2344 | C₇₂H₁₃₇N₇O₆ | 1196.93 | 9.55 | 23.01 | 2544 | C₇₃H₁₃₃N₃O₇ | 1164.88 | 8.78 | 26.27 |
| 2345 | C₇₅H₁₃₆N₆O₆S | 1250.02 | 8.12 | 23.71 | 2545 | C₇₂H₁₃₉N₄O₆P | 1187.90 | 8.50 | 25.02 |
| 2346 | C₇₃H₁₃₃N₃O₃S₄ | 1229.15 | 8.60 | 25.60 | 2546 | C₇₆H₁₄₅N₉O₆ | 1281.05 | 8.95 | 25.90 |
| 2347 | C₇₂H₁₃₄N₂O₂S₆ | 1252.28 | 8.91 | 31.69 | 2547 | C₇₂H₁₃₃N₅O₁₀ | 1228.88 | 7.28 | 26.13 |
| 2348 | C₇₃H₁₃₅NO₂S₇ | 1283.36 | 10.25 | 31.89 | 2548 | C₇₅H₁₄₈N₄O₂ | 1138.04 | 10.25 | 29.53 |
| 2349 | C₇₄H₁₃₈N₆O₈S | 1272.02 | 8.96 | 26.06 | 2549 | C₇₂H₁₃₈N₆O₃ | 1135.93 | 8.47 | 25.31 |
| 2350 | C₇₁H₁₃₂N₆O₈ | 1197.87 | 8.73 | 24.03 | 2550 | C₇₂H₁₃₁N₃O₁₁S | 1246.91 | 7.71 | 26.16 |
| 2351 | C₇₁H₁₃₇N₅O₈S | 1220.97 | 10.21 | 27.15 | 2551 | C₇₆H₁₃₅N₃O₁₃ | 1298.92 | 8.06 | 26.23 |
| 2352 | C₇₀H₁₂₇NO₇S | 1126.85 | 8.28 | 27.40 | 2552 | C₇₃H₁₃₃N₃O₁₃S | 1292.93 | 8.53 | 24.60 |
| 2353 | C₆₆H₁₂₄N₄O₃S₂ | 1085.88 | 7.45 | 24.89 | 2553 | C₇₂H₁₃₅NO₁₃S | 1254.93 | 9.37 | 28.42 |
| 2354 | C₆₉H₁₃₃NO₃S₂ | 1088.96 | 10.01 | 27.96 | 2554 | C₇₅H₁₄₉N₂O₁₁P₃ | 1347.94 | 8.85 | 27.16 |
| 2355 | C₇₃H₁₄₁N₂O₁₃P ₃S | 1379.92 | 7.73 | 24.08 | 2555 | C₇₅H₁₄₈N₃O₁₀P₃ S | 377.00 | 9.09 | 25.62 |
| 2356 | C₇₂H₁₃₆N₄O₁₅S ₄ | 1426.16 | 8.39 | 26.10 | 2556 | C₇₂H₁₃₅N₃O₉S₄ | 1315.12 | 8.02 | 23.79 |
| 2357 | C₆₆H₁₂₇NO₈S₄ | 1191.01 | 9.56 | 23.38 | 2557 | C₆₇H₁₃₃N₅O₇S₃ | 1217.01 | 9.55 | 24.42 |
| 2358 | C₆₉H₁₃₄N₆O₈S₄ | 1304.13 | 8.63 | 23.43 | 2558 | C₆₈H₁₃₇N₇O₈S₄ | 1309.12 | 8.59 | 23.56 |
| 2359 | C₇₀H₁₃₄N₂O₁₁S ₃ | 1276.05 | 9.01 | 25.11 | 2559 | C₆₆H₁₃₀N₄O₉S₄ | 1252.03 | 7.70 | 24.69 |
| 2360 | C₇₁H₁₂₉N₃O₆S₆ | 1313.23 | 7.83 | 29.32 | 2560 | C₆₉H₁₃₁NO₁₀S₄ | 1263.04 | 9.72 | 26.82 |
| 2361 | C₇₅H₁₃₈N₃O₇P S₆ | 1417.32 | 8.79 | 29.20 | 2561 | C₆₉H₁₃₄N₂O₁₁S₄ | 1296.07 | 9.65 | 25.47 |
| 2362 | C₇₃H₁₂₈N₆O₆S₇ | 1410.33 | 8.05 | 29.45 | 2562 | C₇₂H₁₃₇N₃O₁₄S₄ | 1397.13 | 7.96 | 24.84 |
| 2363 | C₆₉H₁₃₃N₃O₄S₃ | 1165.04 | 8.75 | 27.33 | 2563 | C₇₆H₁₃₄N₆O₅S₆ | 1404.30 | 8.44 | 29.07 |
| 2364 | C₇₀H₁₃₃N₂O₄P S₃ | 1194.02 | 8.64 | 27.09 | 2564 | C₇₅H₁₃₈N₄O₄S₇ | 1384.37 | 7.99 | 27.97 |
| 2365 | C₆₈H₁₃₁N₅O₅ | 1098.83 | 8.96 | 24.38 | 2565 | C₇₂H₁₃₃NO₂S₇ | 1269.28 | 9.22 | 29.39 |
| 2366 | C₆₆H₁₃₀N₄O₄ | 1043.79 | 9.95 | 23.32 | 2566 | C₇₆H₁₃₉NS₈ | 1323.43 | 9.99 | 32.54 |
| 2367 | C₆₂H₁₂₄N₄O₅S | 1037.76 | 8.99 | 22.55 | 2567 | C₆₈H₁₃₂N₂OS₆ | 1186.18 | 9.61 | 29.43 |
| 2368 | C₆₇H₁₃₀N₆O₃S₂ | 1131.95 | 9.38 | 22.39 | 2568 | C₇₆H₁₄₆N₆O₈ | 1272.04 | 7.91 | 25.83 |
| 2369 | C₆₇H₁₃₁N₅O₅S₂ | 1150.95 | 8.80 | 22.94 | 2569 | C₇₄H₁₄₂N₂O₆S₃ | 1252.14 | 7.97 | 29.92 |
| 2370 | C₆₅H₁₃₀N₄O₃S₂ | 1079.91 | 10.12 | 23.75 | 2570 | C₇₆H₁₄₇N₃O₇S₄ | 1343.27 | 8.74 | 29.77 |
| 2371 | C₆₄H₁₂₁NO₅S₄ | 1112.94 | 9.72 | 26.66 | 2571 | C₆₈H₁₃₃NO₆S₄ | 1189.05 | 9.29 | 28.76 |
| 2372 | C₆₃H₁₂₇N₇O₆S | 1110.82 | 8.83 | 20.79 | 2572 | C₇₃H₁₄₅N₅O₅ | 1172.99 | 9.61 | 27.70 |
| 2373 | C₆₃H₁₂₆N₆OS₂ | 1047.88 | 8.76 | 23.90 | 2573 | C₇₁H₁₃₉N₅O₁₀S | 1254.98 | 7.51 | 27.38 |
| 2374 | C₆₁H₁₁₉NO₁₄S | 1122.68 | 7.69 | 23.72 | 2574 | C₆₉H₁₄₂N₄O₂ | 1059.92 | 10.0 5 | 27.68 |
| 2375 | C₆₆H₁₂₈N₃O₁₃P | 1202.73 | 7.46 | 24.54 | 2575 | C₇₁H₁₄₄N₆O₃S | 1162.03 | 8.83 | 25.65 |
| 2376 | C₇₃H₁₄₅N₄O₁₁P ₃ | 1347.90 | 8.61 | 22.75 | 2576 | C₇₁H₁₄₈N₄O₁₀S₄ | 1346.22 | 9.78 | 25.99 |
| 2377 | C₇₂H₁₅₀NO₁₃P₃S | 1362.97 | 10.23 | 26.58 | 2577 | C₇₅H₁₄₁N₃O₉S₂ | 1293.09 | 9.29 | 29.12 |
| 2378 | C₅₈H₁₂₁N₅O₈S₃ | 1112.83 | 7.95 | 21.32 | 2578 | C₇₅H₁₄₆N₃O₁₀PS ₃ | 1377.16 | 8.69 | 28.51 |
| 2379 | C₆₁H₁₂₈N₄O₉S₄ | 1189.98 | 9.93 | 23.20 | 2579 | C₆₉H₁₃₈N₄O₅S | 1135.95 | 7.81 | 25.35 |
| 2380 | C₇₉H₁₅₀N₆O₅S₂ | 1328.24 | 8.02 | 27.18 | 2580 | C₈₀H₁₆₁N₃O₁₃P₄ | 1497.07 | 8.34 | 25.40 |
| 2381 | C₈₀H₁₅₆N₆O₇ | 1314.16 | 8.79 | 28.87 | 2581 | C₆₈H₁₃₈N₂O₁₃S₄ | 1320.09 | 7.81 | 24.98 |
| 2382 | C₇₇H₁₅₃N₅O₉S | 1325.16 | 8.63 | 27.52 | 2582 | C₇₈H₁₅₆N₃O₁₁P₃ | 1405.04 | 8.90 | 25.51 |
| 2383 | C₇₉H₁₅₆N₄O₅S | 1274.21 | 9.99 | 30.04 | 2583 | C₇₃H₁₄₃N₉O₇S₅ | 1419.30 | 8.50 | 27.26 |
| 2384 | C₇₈H₁₄₈N₂O₃S₇ | 1386.52 | 8.53 | 34.02 | 2584 | C₇₅H₁₄₆N₄O₄S₃ | 1264.20 | 9.75 | 28.36 |
| 2385 | C₇₇H₁₄₈N₂O₅S₇ | 1406.51 | 8.37 | 33.09 | 2585 | C₇₆H₁₄₁N₇O₅S₃ | 1329.19 | 8.44 | 6.51 |
| 2386 | C₇₈H₁₄₉N₃S₇ | 1353.54 | 9.35 | 33.71 | 2586 | C₇₃H₁₄₃N₃S₈ | 1319.45 | 9.73 | 31.55 |
| 2387 | C₈₀H₁₅₅N₇O₅ | 1295.16 | 9.05 | 28.08 | 2587 | C₆₈H₁₃₇NO₂S₇ | 1225.27 | 9.56 | 32.84 |
| 2388 | C₇₇H₁₅₅N₇O₃S₂ | 1291.27 | 9.72 | 30.97 | 2588 | C₁₀₀H₁₉₂N₂O₄S | 1518.71 | 9.01 | 41.94 |
| 2389 | C₇₇H₁₅₃N₉O₂S | 1269.20 | 8.46 | 28.74 | 2589 | C₉₈H₁₈₉NOS₅ | 1557.90 | 7.61 | 43.84 |
| 2390 | C₇₆H₁₅₆N₄O₇S₃ | 1334.31 | 8.94 | 31.72 | 2590 | C₉₈H₁₉₄NO₃PS₃ | 1561.79 | 8.48 | 42.90 |
| 2391 | C₇₆H₁₅₄N₆O₈S₃ | 1376.30 | 8.64 | 28.57 | 2591 | C₉₉H₁₉₄N₄O₇S | 1584.72 | 9.65 | 43.10 |
| 2392 | C₇₈H₁₄₆N₂O₈ | 1240.03 | 8.71 | 30.11 | 2592 | C₉₉H₁₉₄N₄O₈S₂ | 1632.78 | 7.76 | 43.54 |
| 2393 | C₇₆H₁₄₈NO₉P | 1250.99 | 8.86 | 31.56 | 2593 | C₉₆H₁₉₆N₄O₃S | 1486.71 | 9.59 | 42.11 |
| 2394 | C₇₈H₁₅₀N₆O₉S | 1348.16 | 7.69 | 29.53 | 2594 | C₉₇H₁₉₅N₅O₄S | 1527.72 | 8.65 | 40.06 |
| 2395 | C₇₇H₁₅₀N₄O₇S | 1276.13 | 9.43 | 30.93 | 2595 | C₁₀₂H₁₉₉N₅O₉ | 1639.74 | 8.75 | 41.94 |
| 2396 | C₇₇H₁₄₇NO₇S₄ | 1327.29 | 9.86 | 34.36 | 2596 | C₉₈H₁₉₇N₄O₁₀P | 1622.65 | 8.51 | 41.00 |
| 2397 | C₇₅H₁₄₉NO₁₁S₄ | 1369.28 | 9.91 | 31.06 | 2597 | C₉₉H₁₉₅NO₆S₄ | 1623.89 | 10.0 9 | 47.23 |
| 2398 | C₇₉H₁₆₂NO₁₁P₃ S | 1427.15 | 9.02 | 30.52 | 2598 | C₁₀₅H₂₁₄NO₁₀P₃ | 1743.79 | 10.1 9 | 43.28 |
| 2399 | C₇₆H₁₅₇N₆O₁₀P S₃ | 1442.30 | 8.06 | 28.96 | 2599 | C₉₈H₁₉₃N₃O₁₂S₄ | 1733.87 | 8.00 | 41.24 |
| 2400 | C₇₅H₁₄₉N₃O₁₀S 5 | 1413.36 | 8.76 | 28.82 | 2600 | C₆₃H₁₀₇NO₆S | 1006.62 | 9.80 | 21.64 |
| 2601 | C₆₅H₁₁₂N₃O₄P S₆ | 1223.00 | 7.95 | 21.13 | 2801 | C₉₉H₂₀₀N₆O₁₀S₄ | 1762.96 | 8.40 | 40.16 |
| 2602 | C₆₆H₁₁₉N₇O₅ | 1090.72 | 10.00 | 21.99 | 2802 | C₁₀₀H₁₉₉N₃O₁₁S₃ | 1715.88 | 8.98 | 40.36 |
| 2603 | C₆₁H₁₁₄N₄O₇S₅ | 1175.94 | 9.86 | 19.74 | 2803 | C₉₈H₂₀₁N₄O₁₂PS ₃ | 1754.86 | 9.49 | 39.38 |
| 2604 | C₆₁H₁₁₂N₆O₁₁S ₄ | 1233.87 | 7.61 | 17.67 | 2804 | C₉₉H₁₉₅N₃OS₉ | 1732.21 | 8.95 | 48.29 |
| 2605 | C₅₈H₁₁₀N₄O₉S₄ | 1135.80 | 7.67 | 19.80 | 2805 | C₇₇H₁₂₁N₃O₈S | 1248.89 | 8.03 | 26.31 |
| 2606 | C₆₆H₁₁₆N₆O₈S | 1153.75 | 8.85 | 21.05 | 2806 | C₇₂H₁₁₇NO₃S₅ | 1205.03 | 10.0 7 | 28.49 |
| 2607 | C₆₂H₁₀₆N₂O₁₁ | 1055.53 | 9.60 | 20.49 | 2807 | C₇₄H₁₁₇N₃OS₄ | 1193.01 | 8.45 | 25.19 |
| 2608 | C₆₃H₁₀₉NO₈S₃ | 1104.76 | 9.87 | 23.85 | 2808 | C₇₅H₁₂₁NO₃S₇ | 1309.22 | 9.65 | 27.97 |
| 2609 | C₆₃H₁₀₈N₂O₁₀S ₄ | 1181.83 | 8.42 | 23.27 | 2809 | C₇₉H₁₃₄N₉O₆PS | 1369.03 | 8.95 | 25.87 |
| 2610 | C₆₅H₁₀₉N₃O₇S₅ | 1204.94 | 8.71 | 22.79 | 2810 | C₇₉H₁₃₀N₆O₉ | 1307.94 | 8.95 | 26.58 |
| 2611 | C₆₆H₁₂₂NO₁₀P₃ S | 1214.69 | 9.74 | 20.96 | 2811 | C₇₆H₁₃₅N₄O₄P | 1199.91 | 10.0 1 | 25.75 |
| 2612 | C₆₃H₁₁₁N₃O₉S₅ | 1214.93 | 9.29 | 21.07 | 2812 | C₈₁H₁₂₆N₆O₉ | 1327.93 | 8.71 | 26.39 |
| 2613 | C₆₃H₁₁₃N₃O₁₁ S ₄ | 1216.87 | 8.22 | 20.49 | 2813 | C₇₇H₁₂₆N₄O₆S₂ | 1268.00 | 9.98 | 27.83 |
| 2614 | C₆₅H₁₂₂NO₁₁P₃ S | 1218.68 | 8.62 | 19.71 | 2814 | C₇₅H₁₂₄N₄O₈ | 1209.84 | 8.73 | 27.26 |
| 2615 | C₆₀H₁₁₂NO₉PS ₃ | 1118.73 | 10.00 | 17.50 | 2815 | C₇₁H₁₁₉NO₆ | 1082.73 | 8.65 | 26.27 |
| 2616 | C₆₃H₁₀₈N₆O₁₁S ₄ | 1253.86 | 8.07 | 20.48 | 2816 | C₈₁H₁₃₈N₃O₁₂P₃ S | 1470.99 | 8.02 | 25.79 |
| 2617 | C₆₃H₁₁₃N₂O₅P S₃ | 1105.78 | 7.69 | 21.48 | 2817 | C₇₈H₁₃₅N₄O₁₁P₃ S | 1429.94 | 8.22 | 25.01 |
| 2618 | C₆₁H₁₀₉N₃O₂S₄ | 1044.83 | 8.31 | 22.42 | 2818 | C₇₄H₁₂₈NO₁₁P₃S | 1332.82 | 8.72 | 26.25 |
| 2619 | C₆₀H₁₀₅N₃O₄S₆ | 1124.92 | 7.15 | 23.05 | 2819 | C₇₁H₁₂₁NO₁₀S₃ | 1244.93 | 9.53 | 26.84 |
| 2620 | C₅₆H₁₀₄N₂O₅S | 917.52 | 9.66 | 18.46 | 2820 | C₇₆H₁₃₀NO₁₂PS₃ | 1377.02 | 9.99 | 26.39 |
| 2621 | C₅₇H₁₀₀N₂O₁₀ | 973.43 | 7.44 | 19.35 | 2821 | C₆₉H₁₁₇NO₁₆S₄ | 1344.93 | 8.21 | 27.40 |
| 2622 | C₅₇H₁₀₁NOS₄ | 944.71 | 8.76 | 21.30 | 2822 | C₇₆H₁₃₀N₄O₈S₃ | 1324.08 | 10.0 1 | 27.21 |
| 2623 | C₆₁H₁₀₉N₃O₃S₆ | 1124.96 | 8.89 | 21.53 | 2823 | C₇₁H₁₂₁N₇O₈S₅ | 1361.09 | 7.65 | 25.06 |
| 2624 | C₇₂H₁₃₁N₃O₄S | 1134.92 | 8.90 | 25.07 | 2824 | C₇₄H₁₂₀N₂O₅S₃ | 1213.96 | 9.95 | 27.23 |
| 2625 | C₇₃H₁₃₇N₇O₆ | 1208.94 | 8.96 | 23.56 | 2825 | C₇₇H₁₂₀N₆O₇S₄ | 1370.08 | 7.80 | 26.86 |
| 2626 | C₇₂H₁₂₇N₇O₉ | 1234.85 | 8.10 | 24.19 | 2826 | C₇₃H₁₂₀N₂O₅S₄ | 1234.01 | 9.58 | 27.28 |
| 2627 | C₇₃H₁₃₅N₃O₉S | 1230.96 | 9.09 | 25.63 | 2827 | C₇₆H₁₂₄N₃O₁₀PS ₃ | 1366.99 | 7.57 | 26.82 |
| 2628 | C₆₈H₁₂₇NO₃S₅ | 1167.10 | 9.70 | 29.88 | 2828 | C₇₂H₁₂₃NO₃S₇ | 1275.20 | 9.67 | 28.90 |
| 2629 | C₆₈H₁₂₇N₃S₇ | 1211.25 | 9.60 | 27.84 | 2829 | C₇₄H₁₂₃NO₂S₇ | 1283.22 | 9.86 | 30.94 |
| 2630 | C₇₁H₁₃₄NO₁₀P | 1192.82 | 9.64 | 24.22 | 2830 | C₇₂H₁₄₂N₂O₃ | 1083.94 | 9.36 | 28.29 |
| 2631 | C₆₈H₁₂₈N₄O₆S₂ | 1161.93 | 10.07 | 26.57 | 2831 | C₆₉H₁₃₄N₂O₅S | 1103.90 | 9.68 | 26.54 |
| 2632 | C₇₀H₁₃₀N₂O₈S₃ | 1224.02 | 9.71 | 26.56 | 2832 | C₇₁H₁₃₆N₂O₇S₂ | 1194.00 | 8.99 | 28.63 |
| 2633 | C₇₂H₁₃₁N₃O₉S₃ | 1279.05 | 8.83 | 27.55 | 2833 | C₆₅H₁₂₇NO₆S₂ | 1082.85 | 9.93 | 28.44 |
| 2634 | C₆₈H₁₂₅NO₇S₅ | 1229.08 | 9.96 | 29.51 | 2834 | C₇₂H₁₃₃N₃O₆S₃ | 1233.05 | 7.96 | 27.03 |
| 2635 | C₆₉H₁₃₀N₄O₄ | 1079.82 | 8.27 | 25.32 | 2835 | C₇₃H₁₃₉N₃O₆S₃ | 1251.11 | 8.84 | 28.79 |
| 2636 | C₆₈H₁₃₂N₂O₁₄S ₃ | 1298.00 | 10.19 | 27.01 | 2836 | C₇₃H₁₃₉NO₅S₄ | 1239.16 | 10.1 3 | 31.72 |
| 2637 | C₇₂H₁₄₀N₃O₁₂P ₃ | 1332.84 | 7.62 | 23.57 | 2837 | C₆₉H₁₃₁NOS₅ | 1151.11 | 9.73 | 29.40 |
| 2638 | C₇₁H₁₃₅N₇O₉S₃ | 1327.10 | 8.42 | 23.39 | 2838 | C₆₉H₁₃₆N₂S₆ | 1186.22 | 10.2 0 | 29.92 |
| 2639 | C₆₉H₁₃₁N₃O₁₂S ₄ | 1323.08 | 8.78 | 25.51 | 2839 | C₆₈H₁₃₉N₉O₃S₂ | 1195.04 | 9.53 | 26.63 |
| 2640 | C₇₁H₁₂₇N₃O₇S₄ | 1263.08 | 7.70 | 27.37 | 2840 | C₇₀H₁₃₄N₆O₇S | 1203.94 | 8.24 | 27.19 |
| 2641 | C₇₂H₁₃₀N₄O₉S₃ | 1292.05 | 8.32 | 28.21 | 2841 | C₇₀H₁₃₃N₅O₉S | 1220.92 | 8.39 | 26.98 |
| 2642 | C₆₉H₁₂₉N₃O₆S₇ | 1321.27 | 9.35 | 27.16 | 2842 | C₆₉H₁₄₂N₆O₇S₃ | 1264.11 | 9.97 | 26.55 |
| 2643 | C₇₀H₁₂₉NS₈ | 1241.29 | 10.16 | 30.09 | 2843 | C₆₅H₁₃₇N₅O₉S₄ | 1261.08 | 8.86 | 24.06 |
| 2644 | C₅₈H₁₀₈N₄O₈S | 1021.59 | 10.16 | 30.09 | 2844 | C₇₁H₁₃₇NO₉ | 1148.88 | 10.0 5 | 28.77 |
| 2645 | C₅₆H₁₁₁N₆O₄P | 963.52 | 7.19 | 17.58 | 2845 | C₆₉H₁₃₇N₃O₆ | 1104.87 | 8.83 | 26.42 |
| 2646 | C₅₅H₁₀₆N₄O₆S₅ | 1079.81 | 9.87 | 19.57 | 2846 | C₇₂H₁₄₄N₃O₁₀P₃ | 1304.88 | 7.67 | 25.50 |
| 2647 | C₅₆H₁₀₇N₇O₈S₃ | 1102.71 | 8.21 | 17.61 | 2847 | C₇₁H₁₄₉N₂O₁₁P₃ S | 1331.96 | 8.90 | 26.60 |
| 2648 | C₅₄H₁₀₂N₆O₈S₄ | 1091.71 | 7.81 | 16.98 | 2848 | C₆₆H₁₃₄NO₁₂PS₃ | 1260.95 | 9.07 | 25.89 |
| 2649 | C₅₈H₁₀₇N₇O₈S | 1062.60 | 8.83 | 17.19 | 2849 | C₆₃H₁₃₂N₄O₁₀S₄ | 1234.01 | 8.10 | 24.36 |
| 2650 | C₅₄H₁₀₄N₂O₅S | 893.50 | 9.68 | 18.78 | 2850 | C₇₂H₁₃₇NO₃S₅ | 1225.19 | 9.90 | 31.49 |
| 2651 | C₆₄H₁₂₄N₃O₁₄P ₃S | 1284.69 | 8.20 | 17.97 | 2851 | C₇₀H₁₃₅NO₃S₅ | 1199.15 | 9.97 | 30.96 |
| 2652 | C₅₅H₁₀₃N₃O₁₀S ₅ | 1126.77 | 8.47 | 18.72 | 2852 | C₇₁H₁₃₅N₃OS₆ | 1239.24 | 9.56 | 28.40 |
| 2653 | C₅₅H₁₀₀N₆O₁₃S ₄ | 1181.70 | 8.28 | 18.03 | 2853 | C₅₃H₁₀₀N₆O₅ | 901.42 | 8.32 | 14.69 |
| 2654 | C₅₅H₁₀₆N₄O₇S₄ | 1063.74 | 9.80 | 19.25 | 2854 | C₅₀H₁₀₃N₁₁O₅ | 938.45 | 8.48 | 15.09 |
| 2655 | C₅₉H₁₀₄N₄O₄S₃ | 1029.71 | 9.13 | 19.59 | 2855 | C₅₂H₁₀₀N₆O₉S | 985.46 | 8.31 | 17.51 |
| 2656 | C₅₆H₁₀₃NO₇S₄ | 1030.71 | 9.87 | 21.24 | 2856 | C₅₃H₁₀₁N₃O₇ | 892.40 | 9.95 | 18.39 |
| 2657 | C₅₇H₁₀₄N₂OS₆ | 1025.87 | 10.01 | 20.81 | 2857 | C₅₁H₉₇NO₁₂S | 948.39 | 9.30 | 17.75 |
| 2658 | C₇₂H₁₂₈N₂OS₃ | 1134.03 | 10.16 | 24.30 | 2858 | C₅₀H₉₅NO₁₀S₄ | 998.55 | 8.15 | 20.65 |
| 2659 | C₆₉H₁₂₈N₄OS | 1061.88 | 10.02 | 24.28 | 2859 | C₅₄H₁₁₂NO₁₁P₃S | 1076.47 | 9.21 | 18.64 |
| 2660 | C₇₀H₁₂₉N₅O₇S₅ | 1313.16 | 8.99 | 22.78 | 2860 | C₅₃H₁₀₀N₆O₁₀S₄ | 1109.65 | 8.67 | 15.63 |
| 2661 | C₇₂H₁₃₃N₄O₉P | 1229.85 | 9.96 | 23.06 | 2861 | C₅₀H₁₀₁NO₁₄S₄ | 1068.59 | 10.0 7 | 20.13 |
| 2662 | C₆₈H₁₂₀N₂O₁₁ | 1141.71 | 9.11 | 21.06 | 2862 | C₅₁H₁₀₂N₂O₁₄S₅ | 1127.68 | 8.80 | 20.04 |
| 2663 | C₇₂H₁₂₅N₃O₉S₃ | 1273.00 | 9.10 | 26.58 | 2863 | C₅₁H₁₀₀N₆O₉S₄ | 1069.63 | 8.09 | 16.22 |
| 2664 | C₇₂H₁₂₅NO₈S₃ | 1228.99 | 10.00 | 27.22 | 2864 | C₄₆H₉₆N₄O₆S₅ | 961.60 | 9.69 | 17.23 |
| 2665 | C₇₅H₁₄₁N₂O₁₄P ₃S | 1419.94 | 8.57 | 22.34 | 2865 | C₅₂H₁₀₂N₄O₁₂S₃ | 1071.58 | 8.63 | 16.45 |
| 2666 | C₆₇H₁₂₁N₃O₁₁S ₄ | 1272.95 | 7.36 | 19.18 | 2866 | C₄₇H₉₁NO₃S₅ | 878.55 | 9.65 | 20.22 |
| 2667 | C₆₉H₁₂₆N₆O₇S₄ | 1280.04 | 9.00 | 22.36 | 2867 | C₅₁H₉₆N₄OS₆ | 973.72 | 9.50 | 17.13 |
| 2668 | C₆₉H₁₃₀N₄O₆S₅ | 1272.12 | 10.21 | 24.79 | 2868 | C₄₉H₉₉NO₃S₃ | 846.51 | 9.67 | 20.47 |
| 2669 | C₆₇H₁₁₇NO₆S₄ | 1160.91 | 8.83 | 24.72 | 2869 | C₄₈H₉₃N₃OS₇ | 952.71 | 7.90 | 20.75 |
| 2670 | C₇₃H₁₂₉N₃O₄S₅ | 1273.15 | 8.98 | 26.99 | 2870 | C₆₈H₁₃₂N₂O₆S₃ | 1169.99 | 8.20 | 27.11 |
| 2671 | C₆₇H₁₁₉NO₇S₇ | 1275.11 | 7.43 | 24.74 | 2871 | C₆₉H₁₃₁N₅O₇S₄ | 1271.07 | 8.05 | 28.00 |
| 2672 | C₇₀H₁₂₁N₃O₄S₇ | 1293.17 | 8.47 | 24.89 | 2872 | C₇₄H₁₄₇N₄O₁₃P₃ S | 1425.99 | 8.26 | 21.77 |
| 2673 | C₆₇H₁₁₉N₃O₃S₄ | 1142.95 | 7.75 | 24.17 | 2873 | C₇₈H₁₅₅NO₁₃P₄S | 1471.05 | 9.23 | 26.84 |
| 2674 | C₆₆H₁₁₉NO₂S₇ | 1183.10 | 9.76 | 27.41 | 2874 | C₇₄H₁₄₇N₂O₁₂P₃ S | 1381.97 | 7.99 | 25.04 |
| 2675 | C₇₄H₁₄₂N₄O₉S | 1264.03 | 8.40 | 26.85 | 2875 | C₈₀H₁₄₁N₇O₁₂S | 1425.10 | 8.28 | 27.74 |
| 2676 | C₇₂H₁₃₅N₃O₇S | 1186.95 | 7.50 | 28.39 | 2876 | C₇₅H₁₃₇N₅O₁₂S | 1333.00 | 7.02 | 25.80 |
| 2677 | C₇₄H₁₃₈N₄O₂S₃ | 1212.12 | 7.44 | 26.73 | 2877 | C₇₅H₁₄₆N₅O₈PS | 1309.05 | 9.53 | 24.72 |
| 2678 | C₇₅H₁₃₉N₃O₄S₄ | 1275.19 | 7.91 | 28.49 | 2878 | C₅₂H₁₀₁NO₁₁S₂ | 980.50 | 7.66 | 18.55 |
| 2679 | C₇₂H₁₃₆N₂O₂S₆ | 1254.25 | 8.75 | 29.44 | 2879 | C₆₈H₁₁₅NO₇S₄ | 1186.91 | 9.71 | 24.43 |
| 2680 | C₉₁H₁₇₇NO₄S | 1381.48 | 9.69 | 39.41 | 2880 | C₆₅H₁₁₃NO₁₀S₅ | 1228.92 | 7.12 | 22.76 |
| 2681 | C₈₉H₁₇₂N₂O₇ | 1382.36 | 9.42 | 37.27 | 2881 | C₆₅H₁₂₂NO₁₂P₃S | 1234.67 | 7.76 | 19.16 |
| 2682 | C₉₁H₁₇₆N₆O₇ | 1466.44 | 8.11 | 36.76 | 2882 | C₆₈H₁₁₅N₃O₁₂S | 1198.74 | 8.37 | 22.77 |
| 2683 | C₉₂H₁₇₈N₃O₁₀P | 1517.42 | 8.01 | 36.76 | 2883 | C₇₅H₁₃₀N₃O₆PS₄ | 1329.09 | 8.51 | 25.28 |
| 2684 | C₉₁H₁₇₇N₃O₇S₄ | 1553.67 | 8.56 | 37.79 | 2884 | C₇₆H₁₃₆N₂O₁₀S₅ | 1398.23 | 9.74 | 30.25 |
| 2685 | C₉₃H₁₇₉N₃O₃S₃ | 1483.65 | 8.65 | 37.45 | 2885 | C₇₀H₁₃₀NO₁₂PS₅ | 1369.08 | 9.35 | 22.77 |
| 2686 | C₉₀H₁₇₄N₄O₂S₆ | 1536.77 | 9.13 | 38.77 | 2886 | C₆₈H₁₂₇N₃O₈S₅ | 1275.08 | 7.49 | 18.47 |
| 2687 | C₈₉H₁₇₆N₄O₄ | 1366.41 | 9.65 | 36.39 | 2887 | C₅₆H₁₀₆N₄O₅S₂ | 979.61 | 8.60 | 19.07 |
| 2688 | C₈₈H₁₇₆N₄O₇S | 1434.46 | 7.44 | 34.46 | 2888 | C₅₇H₁₀₄N₄O₇S₄ | 1085.72 | 8.19 | 20.14 |
| 2689 | C₉₀H₁₈₀N₁₀O₃S | 1482.56 | 9.39 | 37.26 | 2889 | C₆₁H₁₀₆N₄O₈S₃ | 1119.72 | 7.67 | 21.03 |
| 2690 | C₉₃H₁₈₅N₉O₆S | 1557.62 | 8.83 | 35.92 | 2890 | C₅₉H₁₁₁N₃O₁₄S₆ | 1278.90 | 8.13 | 18.51 |
| 2691 | C₉₁H₁₈₀N₄O₈ | 1458.46 | 9.68 | 39.20 | 2891 | C₅₇H₁₀₇N₃O₁₂S₅ | 1186.79 | 8.93 | 18.22 |
| 2692 | C₈₄H₁₇₄N₄O₁₀S₄ | 1528.57 | 9.73 | 35.64 | 2892 | C₅₈H₁₀₈N₂O₄S₆ | 1089.87 | 9.71 | 23.62 |
| 2693 | C₉₁H₁₇₇NO₈ | 1413.42 | 9.74 | 39.59 | 2893 | C₇₅H₁₃₃N₃O₁₂S₃ | 1365.08 | 7.62 | 28.16 |
| 2694 | C₉₁H₁₇₇NO₈ | 1413.42 | 9.63 | 39.59 | 2894 | C₇₃H₁₄₄N₅O₇PS | 1267.02 | 8.77 | 23.15 |
| 2695 | C₉₄H₁₉₁NO₁₄S₄ | 1687.80 | 8.92 | 40.68 | 2895 | C₇₀H₁₃₅NO₃S₈ | 1295.33 | 9.28 | 29.89 |
| 2696 | C₉₈H₂₀₀N₄O₉S₃ | 1674.88 | 10.05 | 42.36 | 2896 | C₇₁H₁₄₅N₃OS₄ | 1185.20 | 10.1 3 | 32.12 |
| 2697 | C₁₀₁H₁₉₄N₂O₆S ₄ | 1660.91 | 8.50 | 44.51 | 2897 | C₆₉H₁₃₈NO₁₃PS₅ | 1381.13 | 8.17 | 26.27 |
| 2698 | C₉₅H₁₈₇NO₂S₇ | 1599.97 | 7.87 | 43.73 | 2898 | C₇₆H₁₂₈N₄O₇S | 1241.94 | 9.76 | 26.18 |
| 2699 | C₉₆H₁₉₄N₂O₃S₇ | 1649.04 | 8.91 | 43.81 | 2899 | C₇₇H₁₂₈N₄O₁₀S₃ | 1366.07 | 9.13 | 28.18 |
| 2700 | C₇₃H₁₃₅N₃O₇S | 1198.96 | 8.83 | 27.72 | 2900 | C₇₈H₁₂₉N₇O₁₁S₄ | 1469.17 | 7.08 | 24.13 |
| 2701 | C₇₃H₁₃₂N₄O₂S₃ | 1194.07 | 8.26 | 26.95 | 2901 | C₇₆H₁₃₀N₆O₁₀S₆ | 1480.27 | 8.32 | 26.25 |
| 2702 | C₇₄H₁₄₃N₇O₆ | 1227.00 | 10.05 | 27.15 | 2902 | C₇₉H₁₂₈N₆O₁₂S₄ | 1482.16 | 8.21 | 26.97 |
| 2703 | C₇₂H₁₃₈N₄O₈S | 1219.98 | 9.16 | 26.82 | 2903 | C₇₈H₁₃₅N₅O₄ | 1206.97 | 9.64 | 29.41 |
| 2704 | C₇₀H₁₃₃N₅OS₂ | 1124.99 | 8.67 | 26.08 | 2904 | C₈₀H₁₂₉N₇O₇ | 1300.95 | 8.42 | 24.11 |
| 2705 | C₇₀H₁₃₀N₄O₆S | 1155.89 | 9.03 | 26.72 | 2905 | C₉₂H₁₅₆N₂O₅S₅ | 1530.57 | 8.87 | 38.21 |
| 2706 | C₇₁H₁₃₃N₅O₉S | 1232.93 | 7.56 | 25.84 | 2906 | C₉₅H₁₆₀N₃O₁₂PS ₃ | 1663.49 | 7.57 | 35.94 |
| 2707 | C₇₂H₁₃₄N₄O₁₀S | 1247.94 | 9.32 | 26.54 | 2907 | C₉₁H₁₅₇NO₁₇S₅ | 1697.55 | 9.77 | 37.07 |
| 2708 | C₇₂H₁₃₂N₂O₁₂S | 1249.91 | 8.50 | 26.64 | 2908 | C₉₂H₁₅₉NOS₉ | 1583.83 | 9.44 | 41.50 |
| 2709 | C₆₈H₁₃₁NO₁₅S₃ | 1298.97 | 8.73 | 27.84 | 2909 | C₈₀H₁₅₇N₃O₁₁S₂ | 1401.27 | 7.36 | 30.07 |
| 2710 | C₇₀H₁₃₃N₃O₁₅S ₄ | 1385.08 | 8.44 | 27.06 | 2910 | C₈₀H₁₆₁N₇O₉S | 1397.27 | 9.05 | 31.05 |
| 2711 | C₈₀H₁₅₇N₃O₁₄P ₄ | 1509.04 | 8.07 | 24.70 | 2911 | C₈₆H₁₇₂N₃O₁₄P₃ S₂ | 1629.37 | 7.98 | 30.43 |
| 2712 | C₆₉H₁₂₉N₃O₉S₄ | 1273.04 | 8.14 | 23.02 | 2912 | C₈₂H₁₅₉NO₈S | 1319.23 | 9.17 | 32.07 |
| 2713 | C₇₁H₁₃₉N₃O₁₀S ₃ | 1291.08 | 10.20 | 26.84 | 2913 | C₈₂H₁₅₇NO₉S₂ | 1365.28 | 8.94 | 34.05 |
| 2714 | C₇₁H₁₃₀N₆O₉S₅ | 1372.15 | 7.66 | 25.94 | 2914 | C₈₂H₁₅₅NO₁₀S₃ | 1411.32 | 9.46 | 33.89 |
| 2715 | C₇₁H₁₃₁NO₄S₃ | 1159.01 | 9.05 | 28.83 | 2915 | C₈₂H₁₅₆N₆O₈ | 1354.18 | 7.58 | 27.67 |
| 2716 | C₇₄H₁₃₆N₄O₅S₃ | 1258.11 | 9.28 | 26.98 | 2916 | C₁₀₂H₂₀₀N₄O₆S | 1610.80 | 9.30 | 41.11 |
| 2717 | C₇₄H₁₄₀NO₆PS ₆ | 1363.27 | 10.18 | 30.16 | 2917 | C₉₉H₁₉₉N₅O₁₂S₃ | 1747.88 | 8.74 | 39.52 |
| 2718 | C₇₂H₁₄₁NO₃S₄ | 1197.16 | 10.22 | 30.35 | 2918 | C₉₈H₂₀₁N₅O₁₂S₅ | 1802.01 | 7.78 | 38.29 |
| 2719 | C₇₄H₁₄₀N₂O₃S₆ | 1298.30 | 9.07 | 30.62 | 2919 | C₁₀₄H₂₀₂N₃O₁₃P S₂ | 1797.86 | 8.16 | 42.89 |
| 2720 | C₆₈H₁₃₃N₃O₅S | 1104.89 | 9.56 | 23.55 | 2920 | C₇₂H₁₄₃N₅O₆ | 1174.96 | 7.02 | 24.11 |
| 2721 | C₆₈H₁₃₁NO₂S₃ | 1090.98 | 10.20 | 27.08 | 2921 | C₇₅H₁₄₃NOS₄ | 1203.22 | 9.90 | 30.72 |
| 2722 | C₆₆H₁₂₇N₅O₇ | 1102.77 | 7.40 | 22.13 | 2922 | C₇₆H₁₄₆N₄O₈S₅ | 1404.32 | 7.57 | 29.76 |
| 2723 | C₆₇H₁₃₄N₆O₄S | 1119.91 | 8.03 | 22.12 | 2923 | C₇₆H₁₄₆N₄O₉S₃ | 1356.20 | 8.27 | 30.39 |
| 2724 | C₆₅H₁₃₅N₅O₉S₃ | 1227.00 | 9.81 | 23.41 | 2924 | C₇₈H₁₆₁N₂O₁₁P₃ S | 1428.13 | 9.86 | 28.23 |
| 2725 | C₆₇H₁₂₇N₃O₇ | 1086.77 | 9.14 | 23.56 | 2925 | C₇₇H₁₅₆NO₁₁P₃S ₂ | 1429.13 | 9.80 | 27.74 |
| 2726 | C₆₅H₁₂₆N₂O₁₂S | 1159.78 | 7.67 | 23.67 | 2926 | C₇₂H₁₄₆N₆O₉S₄ | 1368.23 | 7.44 | 25.66 |
| 2727 | C₆₆H₁₂₃N₃O₈S₅ | 1247.02 | 8.30 | 26.43 | 2927 | C₇₅H₁₄₇N₅O₇ | 1231.03 | 8.75 | 27.63 |
| 2728 | C₆₈H₁₄₁N₂O₁₀P ₃ | 1239.80 | 9.68 | 23.81 | 2928 | C₇₁H₁₄₃NO₅S₃ | 1187.11 | 9.63 | 29.70 |
| 2729 | C₆₃H₁₂₅N₃O₁₄S ₄ | 1276.94 | 7.77 | 24.47 | 2929 | C₇₇H₁₄₆N₂O₄S₆ | 1356.38 | 8.92 | 30.90 |
| 2730 | C₆₇H₁₃₃N₃O₇S₄ | 1221.05 | 8.93 | 22.48 | 2930 | C₇₅H₁₄₅N₃O₈S₃ | 1313.18 | 9.69 | 30.82 |
| 2731 | C₆₃H₁₂₃N₃O₃S₆ | 1163.05 | 7.51 | 26.47 | 2931 | C₇₄H₁₄₂N₆O₁₁S₅ | 1452.28 | 7.20 | 26.83 |
| 2732 | C₆₄H₁₃₁NO₃S₇ | 1187.18 | 9.65 | 28.98 | 2932 | C₁₀₇H₂₁₀N₄O₆S | 1680.94 | 9.30 | 44.75 |
| 2733 | C₆₃H₁₂₇NOS₈ | 1171.19 | 10.03 | 29.92 | 2933 | C₁₀₃H₂₀₅N₅OS₇ | 1754.23 | 7.43 | 49.14 |
| 2734 | C₈₂H₁₆₂NO₆P | 1289.17 | 10.20 | 32.78 | 2934 | C₁₀₈H₂₁₂NO₇PS | 1699.92 | 7.96 | 46.71 |
| 2735 | C₇₉H₁₅₃NO₄S₂ | 1245.22 | 9.98 | 34.17 | 2935 | C₉₉H₂₀₀N₄O₂S₃ | 1574.89 | 9.31 | 44.17 |
| 2736 | C₇₉H₁₅₁N₃O₁₀S | 1335.15 | 7.16 | 30.27 | 2936 | C₁₀₀H₁₉₉N₇O₁₁S₄ | 1803.97 | 7.82 | 41.43 |
| 2737 | C₇₉H₁₅₁N₂O₁₀P | 1320.05 | 7.65 | 29.97 | 2937 | C₉₇H₁₉₇NO₁₉S₅ | 1841.93 | 9.29 | 43.58 |
| 2738 | C₇₉H₁₅₂N₄O₅S₃ | 1334.29 | 8.33 | 31.06 | 2938 | C₉₉H₁₉₄N₂O₈S | 1572.71 | 7.31 | 41.98 |
| 2739 | C₇₇H₁₄₆NO₄S₃ | 1249.22 | 9.44 | 32.44 | 2939 | C₉₄H₁₇₈N₄O₅S₃ | 1540.66 | 7.70 | 37.45 |
| 2740 | C₇₈H₁₅₃N₉O₃S₃ | 1361.32 | 8.05 | 31.75 | 2940 | C₇₄H₁₃₇N₃O₄S₅ | 1293.23 | 8.28 | 28.37 |
| 2741 | C₇₅H₁₅₀N₆O₉S₃ | 1376.24 | 7.11 | 28.59 | 2941 | C₇₈H₁₅₁N₂O₁₄P₃ S | 1466.05 | 8.41 | 24.98 |
| 2742 | C₈₀H₁₅₄N₄O₉ | 1316.13 | 8.39 | 31.34 | 2942 | C₇₃H₁₄₁N₇O₈S₃ | 1341.15 | 9.86 | 23.70 |
| 2743 | C₇₈H₁₅₃NO₈S | 1265.14 | 9.73 | 30.75 | 2943 | C₇₆H₁₃₆N₄O₈S | 1266.00 | 7.96 | 27.68 |
| 2744 | C₇₇H₁₅₁NO₆S₂ | 1251.18 | 10.04 | 33.91 | 2944 | C₆₉H₁₃₅N₅O₈S₃ | 1259.05 | 9.93 | 26.72 |
| 2745 | C₇₈H₁₅₆N₄O₉S | 1326.18 | 9.48 | 31.83 | 2945 | C₆₉H₁₃₅NO₃S₆ | 1219.20 | 10.2 6 | 30.26 |
| 2746 | C₈₀H₁₅₂N₂O₁₀S ₄ | 1430.34 | 8.79 | 32.39 | 2946 | C₆₅H₁₂₉NO₃S₆ | 1165.11 | 10.0 3 | 29.30 |
| 2747 | C₇₉H₁₆₀N₄O₄ | 1230.17 | 9.90 | 31.06 | 2947 | C₇₀H₁₃₇N₄O₁₁PS ₄ | 1370.10 | 9.59 | 27.67 |
| 2748 | C₈₂H₁₆₈NO₁₁P₃ S | 1469.22 | 9.35 | 31.16 | 2948 | C₇₃H₁₄₉N₂O₁₃P S | 1387.98 | 9.89 | 25.38 |
| 2749 | C₇₄H₁₄₉NO₉S₄ | 1325.24 | 9.52 | 31.12 | 2949 | C₆₆H₁₃₀N₆O₆S | 1135.86 | 8.58 | 21.52 |
| 2750 | C₇₆H₁₅₁N₃O₉S₃ | 1347.24 | 7.62 | 28.28 | 2950 | C₆₉H₁₃₂N₂O₈S | 1149.88 | 9.31 | 25.41 |
| 2751 | C₈₀H₁₆₃NO₉S₄ | 1411.42 | 10.02 | 31.61 | 2951 | C₆₇H₁₃₀NO₁₁P | 1156.75 | 9.20 | 24.28 |
| 2752 | C₇₇H₁₅₅N₇O₈S₃ | 1403.31 | 8.62 | 28.92 | 2952 | C₇₄H₁₄₁N₄O₇PS | 1262.00 | 10.0 7 | 25.50 |
| 2753 | C₇₇H₁₅₅NO₁₂S₄ | 1415.32 | 9.90 | 31.42 | 2953 | C₇₉H₁₃₉N₇O₂S₈ | 1475.51 | 8.66 | 30.35 |
| 2754 | C₇₉H₁₅₀N₂O₄S₄ | 1320.32 | 8.20 | 32.38 | 2954 | C₈₀H₁₄₆N₃O₇PS₆ | 1485.40 | 8.03 | 29.10 |
| 2755 | C₈₀H₁₅₂N₂O₅S₅ | 1382.40 | 8.23 | 33.60 | 2955 | C₇₂H₁₃₉NO₁₄S₅ | 1403.20 | 10.2 4 | 26.99 |
| 2756 | C₈₂H₁₅₇N₃O₉S₄ | 1457.41 | 8.95 | 34.69 | 2956 | C₆₈H₁₃₄N₂O₁₁S₅ | 1316.12 | 9.97 | 23.02 |
| 2757 | C₇₈H₁₅₃N₂O₈P S₆ | 1470.42 | 7.17 | 32.63 | 2957 | C₇₃H₁₃₈N₄O₅S₇ | 1376.35 | 8.95 | 30.58 |
| 2758 | C₇₈H₁₄₇N₃OS₆ | 1335.41 | 8.18 | 32.47 | 2958 | C₇₆H₁₃₆N₂O₈S | 1237.99 | 8.81 | 25.65 |
| 2759 | C₇₈H₁₅₁N₇O₃S₆ | 1427.47 | 8.10 | 32.55 | 2959 | C₅₅H₁₀₅N₅O₉S | 1012.53 | 9.99 | 19.03 |
| 2760 | C₈₀H₁₅₇N₇OS₆ | 1425.54 | 9.93 | 32.94 | 2960 | C₅₅H₁₀₃N₃O₇S₈ | 1174.92 | 9.06 | 19.72 |
| 2761 | C₁₀₃H₁₉₈N₁₀O₄ | 1640.78 | 8.43 | 38.34 | 2961 | C₅₅H₉₈N₂O₇S₆ | 1091.76 | 8.02 | 19.51 |
| 2762 | C₉₉H₁₉₁NO₈S | 1555.68 | 9.65 | 43.86 | 2962 | C₅₆H₁₀₄N₂O₇S₄ | 1045.70 | 8.13 | 19.65 |
| 2763 | C₉₉H₁₉₁NO₇S₂ | 1571.74 | 9.34 | 44.32 | 2963 | C₅₅H₁₀₃N₃O₁₃S₄ | 1142.68 | 8.95 | 20.08 |
| 2764 | C₁₀₅H₂₀₅NO₅S₃ | 1657.98 | 10.13 | 47.57 | 2964 | C₄₉H₉₇NO₄S₅ | 924.62 | 7.78 | 18.16 |
| 2765 | C₁₀₀H₁₉₈NO₃P S₃ | 1589.84 | 8.58 | 43.46 | 2965 | C₆₀H₁₁₇N₄O₁₅P₃ | 1227.53 | 8.09 | 15.71 |
| 2766 | C₁₀₂H₂₀₀N₂OS₆ | 1663.09 | 10.10 | 45.62 | 2966 | C₅₃H₁₀₅NO₁₂S₅ | 1108.72 | 10.1 3 | 18.40 |
| 2767 | C₁₀₄H₂₀₅NO₆S | 1597.85 | 7.66 | 46.47 | 2967 | C₅₅H₁₀₈N₃O₁₆PS ₅ | 1258.75 | 9.37 | 19.39 |
| 2768 | C₁₀₄H₂₀₉N₇O₃S ₂ | 1669.98 | 9.88 | 46.29 | 2968 | C₅₄H₁₀₄N₄O₁₀S | 1001.50 | 8.74 | 17.69 |
| 2769 | C₁₀₅H₂₀₈N₆O₈ | 1682.85 | 9.03 | 44.74 | 2969 | C₅₄H₁₀₆N₂O₁₀S₂ | 1007.57 | 10.0 2 | 18.69 |
| 2770 | C₁₀₄H₂₀₄N₄O₇S ₂ | 1686.92 | 9.42 | 47.15 | 2970 | C₆₆H₁₁₇N₅O₈S | 1140.75 | 9.75 | 19.41 |
| 2771 | C₁₀₉H₂₀₈N₂O₉ | 1690.87 | 8.92 | 46.22 | 2971 | C₆₈H₁₂₁N₇O₅ | 1116.76 | 8.93 | 17.67 |
| 2772 | C₁₀₅H₂₁₀N₄O₁₀ S | 1720.91 | 9.84 | 46.22 | 2972 | C₆₅H₁₁₄N₂O₅S₅ | 1163.94 | 10.0 2 | 23.50 |
| 2773 | C₁₀₅H₂₀₁N₃O₁₁ S | 1713.83 | 7.46 | 46.28 | 2973 | C₆₈H₁₁₄N₂O₉S₂ | 1167.78 | 8.32 | 21.74 |
| 2774 | C₁₀₂H₂₀₅N₃O₈S | 1633.83 | 8.04 | 44.66 | 2974 | C₇₁H₁₂₆NO₄PS₇ | 1313.19 | 10.09 | 25.85 |
| 2775 | C₁₀₉H₂₂₃N₂O₁₁ P₃S | 1862.97 | 9.95 | 45.08 | 2975 | C₇₄H₁₂₉N₃O₆S₉ | 1445.40 | 8.19 | 27.04 |
| 2776 | C₁₀₂H₂₀₈N₂O₉S ₃ | 1702.97 | 10.14 | 46.00 | 2976 | C₇₄H₁₂₉NO₆S₂ | 1192.97 | 10.3 5 | 24.90 |
| 2777 | C₁₀₇H₂₀₈N₄O₃S ₅ | 1759.17 | 8.34 | 49.21 | 2977 | C₇₃H₁₂₅N₃O₁₁S₂ | 1284.93 | 8.96 | 23.77 |
| 2778 | C₁₀₄H₂₀₃NO₁₀S ₄ | 1756.01 | 9.55 | 48.00 | 2978 | C₇₁H₁₂₅N₅O₁₄S₂ | 1336.92 | 8.57 | 20.70 |
| 2779 | C₁₀₅H₂₀₃NO₇S₄ | 1720.02 | 8.74 | 49.41 | 2979 | C₅₉H₁₀₈N₂O₃S₄ | 1021.76 | 10.2 0 | 20.34 |
| 2780 | C₁₀₈H₂₁₃NO₆S₇ | 1846.31 | 10.05 | 50.72 | 2980 | C₆₁H₁₁₈NO₁₄PS₄ | 1248.82 | 10.2 5 | 22.37 |
| 2781 | C₁₀₃H₂₀₃N₃O₂S ₃ | 1611.96 | 8.66 | 46.73 | 2981 | C₆₆H₁₀₆N₂O₁₁S₄ | 1111.71 | 8.96 | 17.71 |
| 2782 | C₁₀₄H₂₀₈N₂O₂S ₃ | 1615.00 | 9.88 | 47.93 | 2982 | C₅₇H₁₀₈N₂O₁₅S₃ | 1157.67 | 10.0 5 | 20.10 |
| 2783 | C₁₀₁H₂₀₄N₂OS₆ | 1655.12 | 8.86 | 49.01 | 2983 | C₆₀H₁₁₀NO₇P | 988.51 | 10.1 5 | 18.89 |
| 2784 | C₁₀₁H₂₀₅NSs | 1690.24 | 9.98 | 51.49 | 2984 | C₆₁H₁₀₅N₇O₅ | 1016.55 | 8.67 | 17.66 |
| 2785 | C₁₀₂H₂₀₅N₆O₆P | 1642.77 | 9.54 | 39.05 | 2985 | C₆₁H₁₁₂N₂O₉S₂ | 1081.69 | 10.1 1 | 20.51 |
| 2786 | C₁₀₃H₂₀₃N₉O₅S ₂ | 1711.93 | 8.13 | 42.14 | 2986 | C₅₉H₁₀₉N₇O₇S | 1060.62 | 8.74 | 15.29 |
| 2787 | C₉₉H₁₉₈N₈O₅ | 1580.72 | 9.68 | 41.14 | 2987 | C₇₅H₁₃₈N₆O₁₂ | 1315.96 | 8.80 | 26.03 |
| 2788 | C₁₀₀H₂₀₂N₇O₆P | 1629.73 | 9.66 | 40.59 | 2988 | C₇₃H₁₃₄N₃O₁₂PS ₅ | 1437.16 | 8.84 | 29.13 |
| 2789 | C₁₀₃H₂₀₃N₉O₅S | 1679.88 | 8.70 | 41.15 | 2989 | C₇₀H₁₃₄N₄O₄S₃ | 1192.05 | 9.54 | 26.89 |
| 2790 | C₁₀₀H₁₉₄N₁₀O₈ S | 1696.77 | 7.27 | 40.42 | 2990 | C₆₉H₁₂₉N₃O₁₀S₅ | 1321.10 | 9.31 | 24.87 |
| 2791 | C₉₈H₁₉₉N₆O₃P S | 1572.74 | 8.26 | 41.72 | 2991 | C₇₀H₁₄₁NO₄S | 1092.96 | 10.3 7 | 29.21 |
| 2792 | C₉₅H₁₉₄N₄O₈S₃ | 1616.80 | 8.77 | 39.52 | 2992 | C₇₀H₁₄₀N₁₀O₆S | 1250.01 | 9.15 | 24.15 |
| 2793 | C₉₅H₁₉₄N₆O₅S₃ | 1644.81 | 7.43 | 39.83 | 2993 | C₇₂H₁₃₇NO₄S₅ | 1241.19 | 9.95 | 30.25 |
| 2794 | C₁₀₀H₁₉₉N₅O₈ | 1599.72 | 10.02 | 42.80 | 2994 | C₇₁H₁₄₆N₆O₂ | 1115.99 | 10.2 1 | 26.99 |
| 2795 | C₁₀₄H₂₁₃N₄O₉P₃S | 1788.85 | 9.70 | 40.93 | 2995 | C₇₇H₁₃₁N₃O₆S | 1226.97 | 9.99 | 28.80 |
| 2796 | C₉₄H₁₉₂N₂O₁₁S ₄ | 1654.81 | 9.58 | 41.21 | 2996 | C₈₄H₁₄₁N₂O₁₂P₃ S₄ | 1592.22 | 8.25 | 26.39 |
| 2797 | C₁₀₀H₂₀₂NO₁₅P S₃ | 1785.86 | 9.99 | 43.01 | 2997 | C₇₆H₁₂₇N₇O₄ | 1202.90 | 9.28 | 25.07 |
| 2798 | C₉₄H₁₉₁NO₁₇S₄ | 1735.79 | 8.03 | 41.32 | 2998 | C₇₆H₁₂₁N₃O₇S₃ | 1285.00 | 8.58 | 26.20 |
| 2799 | C₉₆H₁₉₃NO₁₄S₅ | 1745.89 | 9.20 | 43.57 | 2999 | C₇₅H₁₁₉NO₁₀S | 1226.83 | 10.0 3 | 27.76 |
| 2800 | C₉₇H₁₉₈N₄O₈S₃ | 1644.85 | 10.17 | 41.87 | 3000 | C₇₇H₁₂₃NO₁₁S₃ | 1335.01 | 9.89 | 27.55 |
| 3001 | C₉₀H₁₅₇N₇O₁₀S ₃ | 1593.47 | 8.81 | 31.48 | 3201 | C₇₂H₁₂₆N₂O₉ | 1163.81 | 9.97 | 23.54 |
| 3002 | C₈₉H₁₅₅NO₆S₈ | 1591.70 | 9.94 | 37.95 | 3202 | C₇₀H₁₂₄N₄O₈ | 1149.78 | 9.97 | 23.36 |
| 3003 | C₈₄H₁₆₈NO₁₀P₃ S₃ | 1541.37 | 9.83 | 31.59 | 3203 | C₇₁H₁₂₅NO₁₄S | 1248.83 | 9.73 | 22.70 |
| 3004 | C₈₀H₁₆₃N₅O₈S₄ | 1451.45 | 10.12 | 31.84 | 3204 | C₇₀H₁₁₇N₃O₁₅ | 1240.71 | 7.26 | 20.93 |
| 3005 | C₈₀H₁₅₂N₄O₇ | 1282.12 | 8.76 | 29.06 | 3205 | C₅₇H₉₉N₃O₃S₈ | 1130.92 | 8.63 | 19.99 |
| 3006 | C₁₀₁H₁₉₈N₄O₅S ₂ | 1612.84 | 9.00 | 42.07 | 3206 | C₆₃H₁₀₉N₆O₅PS₆ | 1253.94 | 8.12 | 21.93 |
| 3007 | C₁₀₃H₁₉₉N₃O₇S ₄ | 1719.98 | 8.96 | 44.17 | 3207 | C₅₈H₁₀₅N₂O₄PS₆ | 1117.82 | 9.26 | 21.79 |
| 3008 | C₁₀₄H₁₉₆N₄O₁₂ S₃ | 1790.91 | 8.40 | 43.89 | 3208 | C₅₉H₁₀₅NO₅S₄ | 1036.73 | 9.99 | 21.27 |
| 3009 | C₁₀₅H₂₀₈N₉O₁₂ PS₄ | 1948.08 | 8.72 | 40.74 | 3209 | C₅₇H₁₀₃N₃O₁₀S₄ | 1118.70 | 9.68 | 21.46 |
| 3010 | C₇₉H₁₅₂N₆O₅S | 1298.18 | 9.08 | 25.22 | 3210 | C₅₉H₁₁₄NO₁₄P₃S ₂ | 1218.60 | 8.06 | 17.66 |
| 3011 | C₇₆H₁₅₂N₆O₁₀S | 1342.14 | 9.91 | 26.73 | 3211 | C₆₀H₁₁₆NO₁₂P₃S ₂ | 1200.62 | 8.76 | 19.01 |
| 3012 | C₇₃H₁₄₂N₂O₃S₆ | 1288.31 | 8.72 | 30.69 | 3212 | C₆₆H₁₂₉N₃O₁₄P₄ | 1312.66 | 8.69 | 16.22 |
| 3013 | C₇₇H₁₄₅N₅O₁₀S ₃ | 1397.21 | 8.79 | 29.01 | 3213 | C₆₂H₁₁₇N₂O₁₃P₃ | 1191.54 | 8.34 | 16.88 |
| 3014 | C₇₇H₁₄₁N₇O₆S₈ | 1517.50 | 8.62 | 30.23 | 3214 | C₅₅H₁₀₄N₆O₉S₃ | 1089.65 | 8.26 | 15.77 |
| 3015 | C₇₅H₁₅₂N₅O₁₂P S₃ | 1443.22 | 8.29 | 25.88 | 3215 | C₅₄H₁₀₂N₂O₁₂S₅ | 1131.71 | 8.76 | 17.82 |
| 3016 | C₇₄H₁₄₅N₃O₁₅S ₄ | 1445.22 | 9.05 | 25.98 | 3216 | C₅₅H₁₀₃NO₁₅S₅ | 1178.72 | 9.92 | 19.53 |
| 3017 | C₇₂H₁₄₂N₂O₉S₆ | 1372.29 | 8.96 | 24.57 | 3217 | C₅₄H₁₀₅N₅O₄S | 920.52 | 9.05 | 18.70 |
| 3018 | C₇₇H₁₅₇N₉O₇S | 1353.22 | 10.26 | 29.33 | 3218 | C₅₃H₁₀₁NO₄S₉ | 1104.93 | 8.98 | 24.27 |
| 3019 | C₇₃H₁₄₅N₃O₅S₅ | 1305.28 | 8.80 | 30.66 | 3219 | C₆₀H₁₀₅N₂O₁₂P | 1077.48 | 8.29 | 18.94 |
| 3020 | C₇₄H₁₄₉N₇O₁₀S ₄ | 1425.28 | 8.95 | 26.66 | 3220 | C₅₈H₁₀₅NO₁₆S | 1104.53 | 9.39 | 18.70 |
| 3021 | C₇₁H₁₃₉N₃O₁₆S ₅ | 1451.20 | 8.48 | 24.73 | 3221 | C₇₀H₁₂₆N₆O₆S | 1179.87 | 7.23 | 23.31 |
| 3022 | C₇₄H₁₄₅N₃O₁₁S ₆ | 1445.34 | 9.07 | 26.30 | 3222 | C₇₄H₁₃₄N₁₀O₁₁S | 1372.00 | 8.45 | 23.42 |
| 3023 | C₇₂H₁₄₅N₄O₁₀P S₃ | 1354.12 | 8.61 | 23.41 | 3223 | C₆₉H₁₂₃NS₇ | 1191.17 | 9.86 | 26.81 |
| 3024 | C₇₃H₁₄₇N₄O₉P S₅ | 1416.27 | 8.33 | 24.01 | 3224 | C₇₁H₁₂₆N₄O₄S₄ | 1228.05 | 7.91 | 25.16 |
| 3025 | C₇₄H₁₄₁NO₁₁S₂ | 1285.06 | 7.98 | 28.99 | 3225 | C₇₁H₁₂₈N₂O₅S₆ | 1282.17 | 9.30 | 28.34 |
| 3026 | C₁₀₉H₂₁₃N₅O₈ | 1721.93 | 8.96 | 42.65 | 3226 | C₇₇H₁₃₇N₆O₇PS₅ | 1450.25 | 8.46 | 25.30 |
| 3027 | C₁₀₄H₂₀₅NOS₇ | 1710.21 | 10.17 | 49.05 | 3227 | C₇₀H₁₂₅N₃O₁₀S₄ | 1297.02 | 7.45 | 26.12 |
| 3028 | C₁₁₁H₂₁₃N₃O₃S ₈ | 1894.42 | 8.11 | 50.12 | 3228 | C₇₂H₁₃₁N₅O₁₁S₃ | 1339.04 | 8.71 | 27.24 |
| 3029 | C₁₁₀H₂₂₂N₃O₁₁ P₃S | 1887.98 | 8.96 | 42.00 | 3229 | C₇₅H₁₄₆N₇O₁₁P₃ | 1414.95 | 7.81 | 22.60 |
| 3030 | C₁₁₀H₂₂₂N₃O₁₁ P₃S | 1887.98 | 8.96 | 42.00 | 3230 | C₇₃H₁₄₀N₃O₁₄P₃ S | 1408.91 | 8.27 | 22.79 |
| 3031 | C₁₀₆H₂₁₁N₃O₁₃ S₂ | 1799.98 | 10.02 | 45.91 | 3231 | C₇₂H₁₄₀N₃O₁₄P₃ | 1364.84 | 8.46 | 23.22 |
| 3032 | C₁₀₃H₂₀₅N₆O₁₀ P | 1718.78 | 8.96 | 40.53 | 3232 | C₇₀H₁₃₁N₅O₁₃S₄ | 1379.08 | 8.01 | 22.50 |
| 3033 | C₁₀₅H₂₁₃N₄O₁₃ P₃S₂ | 1896.92 | 8.94 | 39.79 | 3233 | C₆₇H₁₃₁N₅O₈S₅ | 1295.11 | 9.77 | 25.44 |
| 3034 | C₉₈H₁₉₂N₂O₁₂S ₄ | 1718.86 | 9.02 | 40.76 | 3234 | C₆₅H₁₂₄N₂O₁₄S₅ | 1318.01 | 7.85 | 23.07 |
| 3035 | C₉₃H₁₈₀N₄O₈S | 1514.54 | 9.95 | 38.53 | 3235 | C₇₃H₁₃₉NO₉S₆ | 1367.27 | 10.0 4 | 28.32 |
| 3036 | C₉₆H₁₈₆N₂O₂S₈ | 1657.04 | 10.34 | 44.16 | 3236 | C₇₃H₁₃₀N₄O₈S₃ | 1288.04 | 8.18 | 26.62 |
| 3037 | C₉₄H₁₈₀N₄O₅S₇ | 1670.92 | 8.81 | 39.99 | 3237 | C₇₂H₁₃₂N₄O₁₀S | 1245.93 | 9.51 | 23.80 |
| 3038 | C₈₉H₁₇₇NO₁₅S₃ | 1597.57 | 10.12 | 38.26 | 3238 | C₇₀H₁₃₂NO₁₂PS₂ | 1274.91 | 9.58 | 26.32 |
| 3039 | C₉₂H₁₇₇NO₆S₃ | 1489.61 | 9.88 | 38.99 | 3239 | C₇₃H₁₃₈N₆O₁₀S | 1292.00 | 8.50 | 25.82 |
| 3040 | C₉₃H₁₈₀N₂O₁₂S ₂ | 1582.59 | 8.97 | 38.02 | 3240 | C₇₁H₁₃₇N₇O₁₂S | 1312.97 | 7.95 | 25.01 |
| 3041 | C₇₈H₁₃₉N₃O₆S₅ | 1375.28 | 9.03 | 28.75 | 3241 | C₇₀H₁₃₂N₂O₂S₅ | 1194.14 | 9.86 | 28.40 |
| 3042 | C₇₁H₁₃₈N₆O₁₃S ₅ | 1444.21 | 8.79 | 24.44 | 3242 | C₆₈H₁₂₇N₃S₉ | 1275.32 | 8.31 | 29.94 |
| 3043 | C₇₄H₁₃₇NO₁₀S₃ | 1297.09 | 10.18 | 28.33 | 3243 | C₇₀H₁₃₂N₂O₉S₄ | 1274.07 | 8.35 | 27.50 |
| 3044 | C₅₇H₁₀₉N₅O₈S₅ | 1152.83 | 8.75 | 21.16 | 3244 | C₆₉H₁₃₄N₂O₄S₄ | 1184.08 | 8.84 | 29.64 |
| 3045 | C₅₆H₁₁₂N₆O₃S₂ | 981.67 | 8.79 | 18.08 | 3245 | C₇₁H₁₃₄N₂O₅S₇ | 1320.28 | 7.60 | 30.36 |
| 3046 | C₇₀H₁₂₄N₆O₅S₃ | 1225.98 | 9.62 | 21.57 | 3246 | C₇₀H₁₄₂NO₁₂P₃S ₂ | 1346.94 | 8.97 | 25.79 |
| 3047 | C₇₅H₁₄₀N₈O₅S₃ | 1330.18 | 10.00 | 23.56 | 3247 | C₇₂H₁₄₅N₂O₁₂P₃ S₂ | 1388.00 | 8.91 | 24.47 |
| 3048 | C₇₄H₁₃₉N₅O₁₁ | 1274.95 | 9.94 | 26.43 | 3248 | C₆₅H₁₃₄N₄O₉S₅ | 1276.11 | 8.95 | 25.16 |
| 3049 | C₇₅H₁₃₇N₃O₃S₇ | 1353.36 | 8.51 | 29.95 | 3249 | C₆₈H₁₄₁N₅O₉S₆ | 1365.26 | 9.94 | 24.68 |
| 3050 | C₇₁H₁₄₇N₅O₉S₆ | 1407.34 | 9.97 | 26.49 | 3250 | C₆₅H₁₃₀N₄O₁₆S₄ | 1352.01 | 8.42 | 25.08 |
| 3051 | C₈₁H₁₃₇N₃O₁₀S ₅ | 1473.30 | 9.99 | 30.81 | 3251 | C₆₇H₁₃₅N₅OS₂ | 1090.97 | 9.62 | 24.49 |
| 3052 | C₈₂H₁₂₉N₃O₈S₇ | 1509.37 | 7.82 | 29.90 | 3252 | C₆₉H₁₃₈N₆O₅S | 1163.96 | 8.79 | 24.62 |
| 3053 | C₉₆H₁₆₅N₂O₁₁P S₄ | 1682.59 | 9.47 | 37.52 | 3253 | C₇₀H₁₃₄N₄O₁₁S₂ | 1271.98 | 7.29 | 25.51 |
| 3054 | C₉₃H₁₆₃N₃O₃S₅ | 1531.64 | 8.96 | 37.35 | 3254 | C₇₁H₁₃₇NO₆S₂ | 1165.00 | 10.0 7 | 27.92 |
| 3055 | C₉₈H₁₇₂NO₁₀P₃ S₃ | 1713.55 | 7.92 | 34.58 | 3255 | C₇₄H₁₄₀N₄O₉S₂ | 1294.07 | 8.00 | 27.90 |
| 3056 | C₉₆H₁₆₀N₄O₆S | 1498.42 | 8.16 | 36.02 | 3256 | C₇₃H₁₃₉NO₁₀S₂ | 1255.03 | 10.0 4 | 28.57 |
| 3057 | C₈₇H₁₇₄NO₁₃P₃ | 1535.26 | 9.76 | 31.67 | 3257 | C₇₂H₁₄₁NO₁₃S | 1260.97 | 10.1 1 | 29.32 |
| 3058 | C₇₉H₁₅₃N₂O₇P S₃ | 1370.25 | 9.95 | 30.27 | 3258 | C₇₈H₁₂₆N₈O₉ | 1319.91 | 8.30 | 26.16 |
| 3059 | C₇₅H₁₄₈N₆O₈S₆ | 1454.40 | 8.59 | 27.41 | 3259 | C₇₆H₁₂₅N₅O₁₀S₂ | 1332.98 | 8.90 | 26.29 |
| 3060 | C₁₁₀H₂₂₀N₁₀O₇ | 1795.03 | 9.47 | 43.87 | 3260 | C₇₈H₁₃₄N₁₂O₄S₂ | 1368.13 | 9.28 | 25.15 |
| 3061 | C₁₁₀H₂₁₃N₃O₁₀ S₅ | 1898.22 | 8.79 | 48.01 | 3261 | C₇₇H₁₂₇N₉O₅S₂ | 1323.04 | 8.94 | 26.78 |
| 3062 | C₁₁₀H₂₂₃N₂O₁₁ P₃S₂ | 1907.04 | 9.90 | 44.27 | 3262 | C₇₇H₁₂₅N₂O₆PS₈ | 1462.31 | 8.08 | 28.61 |
| 3063 | C₁₀₉H₂₁₄N₄O₆ | 1676.93 | 9.12 | 44.30 | 3263 | C₇₇H₁₂₃NO₄S₇ | 1351.25 | 9.51 | 29.37 |
| 3064 | C₁₀₇H₂₁₀N₆O_{S} | 1708.89 | 10.02 | 43.55 | 3264 | C₇₇H₁₂₂N₄O₁₁S₄ | 1408.08 | 7.58 | 26.28 |
| 3065 | C₉₃H₁₈₅N₃O₁₅S₄ | 1713.75 | 8.68 | 36.47 | 3265 | C₈₁H₁₄₁N₆O₁₂P₃ | 1483.97 | 7.90 | 24.37 |
| 3066 | C₉₁H₁₈₃NO₁₆S₅ | 1707.76 | 9.33 | 39.98 | 3266 | C₇₃H₁₂₇N₅O₈S₄ | 1331.09 | 9.94 | 26.26 |
| 3067 | C₈₀H₁₄₆N₃O₉P S₃ | 1421.21 | 9.01 | 30.06 | 3267 | C₇₄H₁₃₂N₄O₁₂P₂ S₃ | 1428.01 | 9.34 | 25.27 |
| 3068 | C₇₄H₁₃₈N₂O₁₂S | 1279.98 | 9.95 | 27.25 | 3268 | C₇₆H₁₃₁N₃O₁₃S₄ | 1423.13 | 9.02 | 27.31 |
| 3069 | C₇₃H₁₄₈N₉O₁₁P S | 1391.07 | 8.77 | 22.93 | 3269 | C₇₅H₁₂₇N₅O₉S₃ | 1339.05 | 9.48 | 21.87 |
| 3070 | C₆₉H₁₃₄NO₈PS ₄ | 1265.04 | 9.98 | 27.73 | 3270 | C₇₅H₁₂₂N₂O₈ | 1179.81 | 8.88 | 25.72 |
| 3071 | C₇₃H₁₄₂N₃O₁₁P S₃ | 1365.10 | 9.62 | 27.62 | 3271 | C₇₉H₁₂₈N₂O₁₂S₂ | 1362.01 | 9.85 | 28.34 |
| 3072 | C₅₆H₁₀₄N₄S₅ | 993.78 | 8.81 | 19.28 | 3272 | C₇₆H₁₂₄N₂O₁₁S₂ | 1305.95 | 9.98 | 27.72 |
| 3073 | C₆₈H₁₂₃N₂O₉P | 1143.71 | 8.74 | 20.97 | 3273 | C₉₁H₁₅₄N₆O₈S₂ | 1524.39 | 8.48 | 30.75 |
| 3074 | C₇₇H₁₃₇N₄O₁₂P S₃ | 1438.11 | 8.96 | 25.55 | 3274 | C₉₂H₁₅₅N₉O₈ | 1515.31 | 8.03 | 32.69 |
| 3075 | C₇₄H₁₃₄N₄O₇S | 1223.97 | 10.08 | 21.69 | 3275 | C₉₃H₁₆₁N₄O₉PS | 1542.36 | 9.40 | 34.53 |
| 3076 | C₇₄H₁₃₈N₂O₆ | 1151.93 | 10.06 | 24.52 | 3276 | C₉₀H₁₅₃NO₄S₇ | 1537.64 | 9.51 | 36.88 |
| 3077 | C₉₄H₁₆₃N₅O₇S | 1507.43 | 10.19 | 33.88 | 3277 | C₉₄H₁₅₄N₂O₁₀S₅ | 1632.57 | 8.01 | 35.49 |
| 3078 | C₉₂H₁₆₃N₇O₈S₅ | 1655.66 | 9.65 | 32.84 | 3278 | C₉₄H₁₅₅N₉O₇S₇ | 1747.75 | 8.18 | 34.85 |
| 3079 | C₈₃H₁₅₉NO₇S₅ | 1443.48 | 10.08 | 34.53 | 3279 | C₉₁H₁₆₀N₂O₁₁S₆ | 1650.64 | 9.59 | 34.80 |
| 3080 | C₈₁H₁₆₆N₅O₁₁P S₅ | 1577.52 | 8.86 | 29.78 | 3280 | C₈₈H₁₅₃N₃O₁₄S₃ | 1573.38 | 8.83 | 34.94 |
| 3081 | C₁₀₆H₂₀₅N₃O₅S ₇ | 1826.24 | 9.06 | 44.87 | 3281 | C₈₇H₁₄₇NO₁₄S₄ | 1559.37 | 9.24 | 35.58 |
| 3082 | C₁₀₁H₁₉₉NO₇S₈ | 1796.18 | 10.22 | 47.81 | 3282 | C₉₀H₁₅₀N₄O₉S | 1464.27 | 9.12 | 33.75 |
| 3083 | C₁₀₂H₂₀₅NO₁₁S ₅ | 1782.06 | 10.35 | 44.92 | 3283 | C₉₂H₁₅₈NO₉PS₂ | 1517.37 | 9.60 | 37.32 |
| 3084 | C₁₀₀H₁₉₉NO₂S₈ | 1704.18 | 7.88 | 48.22 | 3284 | C₉₂H₁₅₁NO₆S₄ | 1495.46 | 9.87 | 35.38 |
| 3085 | C₇₁H₁₄₆N₂O₁₃S ₅ | 1396.25 | 9.98 | 26.51 | 3285 | C₉₀H₁₄₈N₄O₁₀S₂ | 1510.31 | 7.57 | 32.53 |
| 3086 | C₇₆H₁₄₈N₄O₁₂S ₅ | 1470.34 | 8.20 | 25.26 | 3286 | C₉₀H₁₅₅N₅O₈S | 1467.32 | 8.94 | 33.22 |
| 3087 | C₁₁₁H₂₁₉N₂O₄P S₆ | 1869.32 | 10.16 | 48.79 | 3287 | C₇₇H₁₅₅N₉O₇ | 1319.14 | 9.48 | 28.87 |
| 3088 | C₁₁₁H₂₁₃N₃O₁₀ S₅ | 1910.24 | 8.95 | 48.91 | 3288 | C₈₁H₁₅₂N₁₀O₁₀S | 1458.23 | 8.16 | 30.05 |
| 3089 | C₁₁₂H₂₁₅N₃O₁₂S₃ | 1892.14 | 8.95 | 48.47 | 3289 | C₇₇H₁₅₃N₉O₆S₃ | 1397.31 | 7.51 | 28.31 |
| 3090 | C₁₀₅H₂₀₁N₃O₁₃ S₄ | 1842.01 | 8.05 | 44.33 | 3290 | C₇₉H₁₅₄N₈O₉ | 1360.15 | 7.29 | 28.84 |
| 3091 | C₉₃H₁₈₅N₃O₈S | 1505.58 | 8.82 | 36.48 | 3291 | C₇₇H₁₄₉NO₃S₈ | 1393.52 | 7.90 | 34.02 |
| 3092 | C₁₀₀H₁₉₉N₄O₁₃ P₃S₂ | 1822.75 | 8.21 | 37.37 | 3292 | C₇₉H₁₅₄N₆O₃S₆ | 1428.50 | 9.20 | 32.59 |
| 3093 | C₈₁H₁₄₆N₃O₁₂P S₃ | 1481.22 | 8.46 | 28.15 | 3293 | C₇₆H₁₄₈N₂O₃S₇ | 1362.45 | 9.46 | 34.16 |
| 3094 | C₆₇H₁₂₆N₆O₅S₂ | 1159.90 | 8.50 | 24.22 | 3294 | C₈₂H₁₅₉N₃O₁₀P₂ S₃ | 1505.31 | 8.51 | 30.25 |
| 3095 | C₆₈H₁₃₃N₆O₉P S | 1241.88 | 7.67 | 19.74 | 3295 | C₇₈H₁₅₂N₂O₇S₄ | 1358.32 | 8.76 | 32.96 |
| 3096 | C₆₉H₁₃₄N₈O₇S₂ | 1252.00 | 8.41 | 22.96 | 3296 | C₇₉H₁₅₄N₄O₁₀S₄ | 1448.36 | 8.70 | 31.46 |
| 3097 | C₇₀H₁₃₁N₃O₆S₈ | 1367.31 | 7.69 | 26.18 | 3297 | C₇₉H₁₆₂NO₃PS₄ | 1333.38 | 9.86 | 33.98 |
| 3098 | C₇₀H₁₃₅NO₄S₇ | 1279.27 | 10.12 | 30.32 | 3298 | C₈₀H₁₅₄N₂O₁₁S₄ | 1448.35 | 9.26 | 33.61 |
| 3099 | C₇₀H₁₃₂N₄O₁₂S ₄ | 1350.08 | 7.46 | 26.35 | 3299 | C₇₆H₁₄₅NO₆S₈ | 1425.48 | 8.81 | 34.83 |
| 3100 | C₆₆H₁₂₅N₃O₁₁S ₄ | 1264.98 | 8.20 | 24.53 | 3300 | C₈₂H₁₇₀N₃O₁₁P₃ | 1467.19 | 9.97 | 30.57 |
| 3101 | C₆₇H₁₂₀NO₁₁S₅ | 1285.06 | 9.67 | 26.84 | 3301 | C₇₇H₁₅₂N₆O₁₁S₅ | 1498.39 | 7.59 | 29.12 |
| 3102 | C₆₇H₁₂₃N₃O₆S₇ | 1291.16 | 8.47 | 27.22 | 3302 | C₇₄H₁₅₃N₇O₁₂S₅ | 1493.38 | 8.28 | 28.08 |
| 3103 | C₆₉H₁₃₈NO₁₂P₃ S₃ | 1362.96 | 9.26 | 24.34 | 3303 | C₇₆H₁₅₂N₆O₁₂S₆ | 1534.44 | 7.73 | 28.03 |
| 3104 | C₆₃H₁₂₅N₇O₉S₆ | 1317.09 | 7.80 | 22.84 | 3304 | C₇₇H₁₅₀N₂O₁₂S₆ | 1488.41 | 7.93 | 28.36 |
| 3105 | C₆₅H₁₂₈N₄O₁₃S ₅ | 1334.05 | 8.14 | 22.01 | 3305 | C₇₇H₁₅₁N₂O₁₀PS ₅ | 1456.33 | 8.39 | 26.01 |
| 3106 | C₆₄H₁₂₈N₃O₁₅P S₄ | 1338.95 | 7.40 | 22.22 | 3306 | C₇₉H₁₅₅N₃O₁₀S₃ | 1403.30 | 8.45 | 27.66 |
| 3107 | C₆₃H₁₂₅N₃O₁₀S ₇ | 1309.12 | 8.27 | 22.61 | 3307 | C₈₀H₁₅₂N₆O₆ | 1294.13 | 8.06 | 30.31 |
| 3108 | C₆₃H₁₂₆N₂O₇S₄ | 1151.95 | 9.53 | 20.16 | 3308 | C₈₁H₁₅₀N₆O₉S | 1384.18 | 8.07 | 31.20 |
| 3109 | C₆₈H₁₃₁N₇O₈S₂ | 1238.96 | 8.67 | 22.69 | 3309 | C₇₈H₁₅₂N₂O₁₁S₂ | 1358.20 | 8.66 | 29.10 |
| 3110 | C₇₁H₁₃₆N₂O₅S | 1129.94 | 9.90 | 27.52 | 3310 | C₈₂H₁₅₂N₈O₁₀ | 1410.16 | 7.54 | 29.00 |
| 3111 | C₇₁H₁₃₈NO₆PS ₂ | 1196.98 | 9.97 | 25.57 | 3311 | C₈₀H₁₅₃N₅O₁₀ | 1345.13 | 8.37 | 30.38 |
| 3112 | C₇₅H₁₄₃N₅O₃S₂ | 1227.12 | 10.12 | 25.60 | 3312 | C₈₂H₁₅₅N₃O₁₁S₂ | 1423.27 | 8.49 | 32.30 |
| 3113 | C₇₂H₁₃₈N₈O₅S | 1228.01 | 9.88 | 25.83 | 3313 | C₉₇H₁₉₇N₂O₆P | 1518.62 | 9.48 | 42.37 |
| 3114 | C₇₆H₁₃₄N₄O₄S₈ | 1424.41 | 8.11 | 29.40 | 3314 | C₁₀₀H₂₀₃NO₆S₃ | 1611.90 | 9.02 | 44.49 |
| 3115 | C₇₁H₁₃₃N₅O₆S₄ | 1281.11 | 7.36 | 26.07 | 3315 | C₁₀₄H₂₀₇N₆O₁₂P S | 1796.86 | 8.77 | 40.97 |
| 3116 | C₇₂H₁₃₂N₄O₅S₃ | 1230.05 | 9.26 | 27.72 | 3316 | C₁₀₀H₁₉₈N₇O₁₂P S | 1753.75 | 7.59 | 39.40 |
| 3117 | C₇₃H₁₃₂N₄O₈S₆ | 1386.24 | 7.72 | 28.90 | 3317 | C₁₀₃H₂₀₄N₉O₉PS | 1775.85 | 7.96 | 38.97 |
| 3118 | C₇₇H₁₅₂N₂O₁₅P ₄S | 1502.02 | 8.23 | 24.37 | 3318 | C₁₀₀H₁₉₀N₄O₅S₆ | 1721.00 | 7.09 | 43.77 |
| 3119 | C₇₇H₁₄₈NO₁₁P₃ S₃ | 1453.13 | 9.91 | 26.96 | 3319 | C₁₀₃H₁₉₉N₄O₄PS ₆ | 1781.08 | 7.23 | 43.88 |
| 3120 | C₇₁H₁₃₇N₅O₈S₅ | 1349.20 | 7.82 | 25.24 | 3320 | C₁₀₀H₁₉₇N₅O₈S₄ | 1725.94 | 9.14 | 42.88 |
| 3121 | C₇₂H₁₃₉N₆O₁₁P S₅ | 1456.21 | 7.67 | 26.24 | 3321 | C₁₀₄H₁₉₈N₄O₉S₆ | 1841.11 | 8.25 | 44.96 |
| 3122 | C₇₃H₁₄₁N₂O₁₂P S₄ | 1398.15 | 8.95 | 24.70 | 3322 | C₉₉H₁₉₂N₂O₁₄S₅ | 1794.92 | 8.35 | 43.66 |
| 3123 | C₇₃H₁₄₀NO₁₃P S₄ | 1399.13 | 9.72 | 26.53 | 3323 | C₁₀₃H₂₀₁N₂O₁₀P S₄ | 1786.96 | 8.46 | 44.74 |
| 3124 | C₆₉H₁₂₇N₃O₁₂S ₅ | 1351.08 | 7.54 | 25.78 | 3324 | C₁₀₂H₁₉₅N₇O₈S₆ | 1840.08 | 8.41 | 43.99 |
| 3125 | C₇₂H₁₃₅N₅O₁₂S ₃ | 1359.08 | 7.71 | 24.04 | 3325 | C₁₀₂H₂₀₄N₃O₁₆P₃ S | 1853.74 | 7.25 | 38.61 |
| 3126 | C₆₈H₁₃₀N₂O₉S₅ | 1280.09 | 9.58 | 25.45 | 3326 | C₁₀₆H₂₁₄N₃O₁₁P₃ S₂ | 1863.93 | 8.94 | 40.89 |
| 3127 | C₇₁H₁₃₃NO₁₃S₄ | 1337.08 | 9.69 | 26.71 | 3327 | C₉₇H₁₉₇N₅O₁₀S₃ | 1689.85 | 9.91 | 40.33 |
| 3128 | C₆₉H₁₃₂N₂O₁₀S ₆ | 1342.18 | 8.71 | 22.55 | 3328 | C₉₇H₁₉₂N₆O₈S₇ | 1795.06 | 7.50 | 39.79 |
| 3129 | C₇₂H₁₃₃N₃O₁₃S | 1280.92 | 7.06 | 25.48 | 3329 | C₉₇H₁₉₇N₇O₆S₅ | 1717.99 | 9.70 | 39.61 |
| 3130 | C₇₈H₁₄₃NO₉S₃ | 1335.18 | 10.04 | 30.97 | 3330 | C₉₉H₁₉₃N₃O₁₀S₇ | 1810.06 | 8.90 | 42.09 |
| 3131 | C₇₆H₁₃₄N₆O₁₂S | 1356.00 | 8.01 | 25.46 | 3331 | C₉₇H₁₉₀N₄O₉S₃ | 1652.79 | 8.14 | 38.15 |
| 3132 | C₇₂H₁₃₆N₄O₈S₂ | 1250.02 | 9.77 | 23.12 | 3332 | C₉₈H₁₉₃N₃O₁₀S₆ | 1765.99 | 8.13 | 37.98 |
| 3133 | C₄₈H₉₇NO₇S₃ | 896.48 | 9.93 | 17.95 | 3333 | C₉₆H₁₉₄N₄OS₂ | 1484.76 | 9.09 | 41.92 |
| 3134 | C₅₅H₁₀₅N₇O₆S | 992.55 | 8.64 | 14.03 | 3334 | C₁₀₅H₂₀₁N₂O₉PS | 1698.80 | 7.87 | 42.52 |
| 3135 | C₅₂H₉₇N₅O₈ | 920.37 | 7.30 | 14.27 | 3335 | C₁₀₂H₁₉₃N₃O₇S₂ | 1637.80 | 7.73 | 42.08 |
| 3136 | C₅₅H₁₀₈N₄O₆S₃ | 1017.67 | 10.20 | 20.03 | 3336 | C₁₀₁H₁₉₂N₄O₁₂ | 1654.66 | 7.87 | 39.92 |
| 3137 | C₅₁H₁₀₁N₄O₁₀P S₂ | 1025.48 | 9.12 | 18.42 | 3337 | C₉₉H₁₉₁NO₁₅S | 1667.67 | 9.69 | 42.63 |
| 3138 | C₅₅H₁₀₁N₃O₄S₄ | 996.67 | 8.27 | 18.05 | 3338 | C₇₅H₁₄₃N₁₃O₆S | 1355.11 | 8.07 | 25.23 |
| 3139 | C₅₂H₁₀₀N₄O₃S₃ | 925.58 | 8.64 | 18.64 | 3339 | C₇₆H₁₄₅N₉O₅S₄ | 1393.29 | 7.57 | 26.41 |
| 3140 | C₄₈H₉₄N₂O₃S₅ | 907.59 | 7.71 | 18.31 | 3340 | C₇₃H₁₅₁N₉O₉ | 1359.12 | 7.94 | 25.79 |
| 3141 | C₅₆H₁₀₆N₂O₉S₅ | 1111.77 | 10.02 | 23.32 | 3341 | C₇₆H₁₃₉N₃O₃S₈ | 1399.45 | 7.63 | 30.64 |
| 3142 | C₅₅H₉₉N₃O₇S₇ | 1138.83 | 9.16 | 21.40 | 3342 | C₇₆H₁₄₅NO₅S₅ | 1313.30 | 9.99 | 31.73 |
| 3143 | C₅₄H₁₀₇N₂ O₁₄P ₃S | 1133.43 | 7.27 | 15.46 | 3343 | C₇₂H₁₃₇N₃O₇S₃ | 1253.08 | 9.28 | 29.58 |
| 3144 | C₅₇H₁₁₆N₃O₁₀P ₃S | 1128.55 | 9.57 | 15.21 | 3344 | C₆₇H₁₃₈N₂O₅S₅ | 1212.15 | 8.77 | 28.81 |
| 3145 | C₅₈H₁₁₉N₃O₁₄P ₄ | 1206.49 | 9.37 | 15.64 | 3345 | C₇₂H₁₃₇NO₄S₉ | 1369.43 | 9.47 | 33.57 |
| 3146 | C₅₀H₁₀₂N₆O₉S₄ | 1059.64 | 8.91 | 14.11 | 3346 | C₇₄H₁₅₂N₃O₁₂P₃ S | 1401.02 | 9.40 | 26.10 |
| 3147 | C₄₆H₉₆N₆O₈S₃ | 957.49 | 9.30 | 14.92 | 3347 | C₆₆H₁₃₈N₄O₉S₅ | 1292.15 | 8.80 | 23.19 |
| 3148 | C₄₉H₉₅NO₁₀S₇ | 1082.72 | 8.89 | 17.71 | 3348 | C₇₁H₁₄₂N₂O₁₁S₅ | 1360.22 | 10.1 0 | 28.27 |
| 3149 | C₅₂H₁₀₆N₂O₈S₄ | 1015.67 | 10.20 | 15.94 | 3349 | C₆₉H₁₃₇N₃O₉S₆ | 1345.23 | 8.87 | 26.02 |
| 3150 | C₅₂H₁₀₂N₆OS₄ | 955.67 | 8.71 | 17.72 | 3350 | C₇₁H₁₃₉N₃O₁₄S₆ | 1451.26 | 7.99 | 27.43 |
| 3151 | C₄₈H₉₉N₅O₅ | 826.35 | 8.78 | 16.86 | 3351 | C₇₁H₁₄₀N₆O₂S₂ | 1174.06 | 7.56 | 26.04 |
| 3152 | C₅₀H₁₀₀N₂O₂S₇ | 985.78 | 8.94 | 20.77 | 3352 | C₇₀H₁₄₃N₅O₂ | 1086.95 | 9.65 | 26.48 |
| 3153 | C₅₀H₉₅NO₉S₂ | 918.43 | 8.74 | 17.41 | 3353 | C₆₉H₁₄₀N₈O₃ | 1129.93 | 7.47 | 24.72 |
| 3154 | C₅₃H₉₇N₃O₁₀ | 936.37 | 8.02 | 17.12 | 3354 | C₇₇H₁₄₁N₃O₈S₂ | 1301.11 | 7.55 | 28.26 |
| 3155 | C₅₂H₉₉N₃O₈S₂ | 958.50 | 9.45 | 17.41 | 3355 | C₇₂H₁₃₅N₃O₁₀S | 1234.94 | 7.57 | 26.66 |
| 3156 | C₅₂H₉₆N₃O₁₁P S | 1002.38 | 8.12 | 16.02 | 3356 | C₇₄H₁₃₈N₂O₁₀S | 1247.98 | 7.46 | 27.52 |
| 3157 | C₅₂H₉₂N₂O₁₅ | 985.31 | 7.52 | 16.32 | 3357 | C₇₀H₁₃₆N₂O₁₀S₂ | 1229.98 | 9.66 | 28.88 |
| 3158 | C₅₃H₁₀₀N₂O₁₃ | 973.38 | 8.88 | 15.78 | 3358 | C₇₅H₁₅₀N₈O₅ | 1244.08 | 9.70 | 26.96 |
| 3159 | C₅₃H₁₀₃N₆O₁₀P S | 1047.47 | 8.46 | 14.49 | 3359 | C₇₄H₁₄₄N₅O₈PS | 1295.03 | 8.00 | 25.17 |
| 3160 | C₆₀H₁₁₀N₂O₇S₂ | 1035.67 | 8.77 | 21.53 | 3360 | C₇₂H₁₄₅N₅O₉S₃ | 1321.16 | 9.35 | 26.34 |
| 3161 | C₆₁H₁₀₉NO₇S₂ | 1032.66 | 9.76 | 22.04 | 3361 | C₇₁H₁₄₄N₈O₇S | 1254.04 | 7.70 | 26.61 |
| 3162 | C₆₈H₁₂₄N₇O₁₁P S | 1278.81 | 9.49 | 18.68 | 3362 | C₇₉H₁₅₁N₉O₇S₂ | 1403.25 | 8.31 | 26.66 |
| 3163 | C₆₈H₁₁₇NO₃S₆ | 1189.05 | 9.07 | 26.17 | 3363 | C₇₂H₁₄₄N₁₀O₇S₃ | 1358.19 | 8.43 | 26.21 |
| 3164 | C₆₅H₁₁₀N₂O₃S₆ | 1159.97 | 9.44 | 24.53 | 3364 | C₇₁H₁₃₅NO₂S₇ | 1259.29 | 9.74 | 30.80 |
| 3165 | C₆₇H₁₁₄N₄O₄S₈ | 1296.15 | 8.05 | 23.10 | 3365 | C₇₃H₁₃₉NO₂S₈ | 1319.40 | 8.81 | 32.79 |
| 3166 | C₆₇H₁₁₉N₃OS₆ | 1175.07 | 9.63 | 26.04 | 3366 | C₇₁H₁₄₄NO₅PS₄ | 1251.15 | 10.0 3 | 31.16 |
| 3167 | C₆₄H₁₀₇NO₂S₆ | 1114.92 | 8.40 | 23.72 | 3367 | C₆₉H₁₃₉NO₃S₆ | 1223.23 | 9.21 | 31.23 |
| 3168 | C₆₅H₁₁₁N₅O₅S₃ | 1138.81 | 8.82 | 21.52 | 3368 | C₇₅H₁₄₁N₂O₁₁PS ₄ | 1406.17 | 8.64 | 28.77 |
| 3169 | C₅₇H₁₀₅N₃O₃S₄ | 1008.72 | 8.60 | 22.28 | 3369 | C₇₄H₁₅₃N₄O₁₁P₃ S₂ | 1432.10 | 8.31 | 25.59 |
| 3170 | C₆₄H₁₁₃N₅O₅S₇ | 1257.06 | 9.42 | 23.90 | 3370 | C₇₇H₁₅₀N₃O₁₄P₃ S | 1467.04 | 7.45 | 24.68 |
| 3171 | C₆₄H₁₁₈NO₁₂P₃ S₄ | 1314.80 | 8.70 | 19.42 | 3371 | C₇₁H₁₄₆N₈O₈S₄ | 1368.24 | 8.39 | 25.23 |
| 3172 | C₅₇H₁₀₇N₅O₈S₄ | 1118.75 | 9.82 | 18.80 | 3372 | C₆₈H₁₃₅NO₁₅S₅ | 1367.12 | 8.51 | 26.84 |
| 3173 | C₆₀H₁₁₃N₅O₈S₄ | 1160.83 | 9.93 | 19.66 | 3373 | C₇₁H₁₄₂N₆O₁₅S₃ | 1416.12 | 7.02 | 26.05 |
| 3174 | C₆₃H₁₁₈N₆O₉S₄ | 1231.91 | 9.47 | 17.86 | 3374 | C₆₈H₁₃₅NO₁₀S₃ | 1223.00 | 8.08 | 24.60 |
| 3175 | C₆₂H₁₁₁N₃O₁₃S ₄ | 1234.82 | 8.51 | 17.77 | 3375 | C₇₃H₁₄₄N₃O₁₀PS ₅ | 1415.24 | 8.25 | 22.65 |
| 3176 | C₆₃H₁₁₄NO₁₃P S₅ | 1284.87 | 9.07 | 21.47 | 3376 | C₇₂H₁₃₇N₃O₁₁S₆ | 1413.26 | 7.91 | 23.79 |
| 3177 | C₆₂H₁₁₇N₂O₁₅P S₄ | 1289.83 | 9.18 | 18.80 | 3377 | C₇₀H₁₄₂N₂O₁₀S₄ | 1300.15 | 9.93 | 24.88 |
| 3178 | C₆₂H₁₁₁N₃O₁₄S ₆ | 1314.94 | 8.47 | 19.91 | 3378 | C₆₈H₁₃₆N₆O₇S₂ | 1213.99 | 7.07 | 26.69 |
| 3179 | C₆₃H₁₁₅N₂O₁₇P S₅ | 1363.88 | 8.72 | 20.79 | 3379 | C₇₂H₁₄₂N₆O₄ | 1155.97 | 8.79 | 26.00 |
| 3180 | C₅₈H₁₀₇N₃O₈S3 | 1070.69 | 9.95 | 16.48 | 3380 | C₇₅H₁₄₆N₃O₇PS | 1265.04 | 9.30 | 28.09 |
| 3181 | C₆₀H₁₀₆N₂O₈S₃ | 1079.69 | 9.71 | 22.06 | 3381 | C₇₄H₁₃₉N₃O₆S₃ | 1263.12 | 8.03 | 27.70 |
| 3182 | C₆₄H₁₁₂N₄O₅S₂ | 1081.74 | 9.13 | 19.93 | 3382 | C₇₅H₁₄₀N₂O₇ | 1181.95 | 8.49 | 27.43 |
| 3183 | C₇₂H₁₂₉N₇O₆S | 1220.93 | 9.47 | 19.86 | 3383 | C₇₄H₁₃₇N₃O₇S₄ | 1309.16 | 8.31 | 28.35 |
| 3184 | C₇₄H₁₃₁N₇O₆S₂ | 1279.03 | 8.74 | 20.89 | 3384 | C₇₄H₁₃₇N₃O₁₄S₂ | 1357.04 | 8.49 | 25.65 |
| 3185 | C₆₉H₁₂₃N₅O₈S | 1182.83 | 9.47 | 23.43 | 3385 | C₁₀₇H₂₀₉N₅O₁₀S | 1757.93 | 9.69 | 46.30 |
| 3186 | C₇₁H₁₂₆N₆O₁₀S ₃ | 1320.00 | 8.10 | 21.56 | 3386 | C₁₀₅H₂₀₈N₆O₁₂S | 1778.91 | 9.08 | 44.20 |
| 3187 | C₇₀H₁₂₉N₄O₁₁P S | 1265.85 | 8.87 | 23.05 | 3387 | C₁₀₇H₂₀₉N₇O₁₀S₂ | 1818.01 | 7.78 | 44.86 |
| 3188 | C₇₁H₁₂₈N₈O₆S | 1221.91 | 9.84 | 21.09 | 3388 | C₁₀₅H₂₀₉N₇O₇S | 1713.93 | 9.76 | 43.29 |
| 3189 | C₆₅H₁₂₂N₈O₉S₂ | 1223.86 | 7.01 | 20.16 | 3389 | C₁₁₀H₂₁₄N₃₀₇PS ₅ | 1882.21 | 8.72 | 46.15 |
| 3190 | C₇₂H₁₂₈N₃O₇P S₄ | 1307.04 | 8.58 | 21.87 | 3390 | C₁₀₄H₂₀₃NO₄S₇ | 1756.19 | 7.73 | 48.33 |
| 3191 | C₆₉H₁₂₀N₂O₂S₇ | 1234.15 | 9.59 | 25.76 | 3391 | C₁₁₀H₂₁₄NO₈PS₄ | 1838.13 | 9.76 | 48.15 |
| 3192 | C₇₂H₁₂₈N₂O₃S₇ | 1294.25 | 9.98 | 27.71 | 3392 | C₁₀₅H₂₀₅NO₈S₅ | 1770.09 | 8.22 | 48.48 |
| 3193 | C₇₁H₁₂₀N₂O₇S₃ | 1209.93 | 8.72 | 23.50 | 3393 | C₁₀₆H₂₀₇N₃O₈S₃ | 1748.01 | 8.86 | 45.55 |
| 3194 | C₆₉H₁₁₉NO₇S₇ | 1299.13 | 9.31 | 26.45 | 3394 | C₁₀₂H₂₀₂N₄O₆S₄ | 1709.00 | 8.10 | 46.12 |
| 3195 | C₆₆H₁₂₀N₂O₅S₄ | 1149.93 | 7.54 | 24.03 | 3395 | C₁₁₁H₂₂₁N₂O₁₃P₃ S₂ | 1949.03 | 7.97 | 44.55 |
| 3196 | C₇₄H₁₃₇N₂O₁₂P ₃S₃ | 1436.01 | 8.52 | 22.06 | 3396 | C₁₀₀H₁₉₉NO₁₁S₇ | 1816.11 | 7.43 | 44.88 |
| 3197 | C₇₄H₁₃₆N₅O₁₂P ₃S₂ | 1444.97 | 7.61 | 19.31 | 3397 | C₁₀₅H₂₁₂N₃O₁₃P S₃ | 1852.01 | 8.82 | 42.85 |
| 3198 | C₆₆H₁₂₃N₅O₆S₅ | 1243.04 | 9.94 | 20.38 | 3398 | C₁₀₂H₂₀₃N₅O₁₂S₅ | 1852.07 | 7.51 | 45.60 |
| 3199 | C₆₈H₁₂₃N₃O₁₂S ₄ | 1302.98 | 8.93 | 19.98 | 3399 | C₁₀₂H₂₁₀N₄OS | 1540.89 | 10.1 3 | 45.50 |
| 3200 | C₆₇H₁₁₇N₃O₉S₅ | 1268.98 | 7.82 | 18.53 | 3400 | C₁₀₆H₂₁₀N₄O₅S₇ | 1845.29 | 8.50 | 47.42 |
| 3401 | C₈₉H₁₇₄NO₇PS ₄ | 1529.58 | 8.39 | 38.72 | 3413 | C₈₈H₁₇₄N₂O₃S₇ | 1532.79 | 8.91 | 42.54 |
| 3402 | C₉₀H₁₇₁N₃O₇S₃ | 1503.55 | 7.71 | 37.84 | 3414 | C₉₁H₁₇₃N₅O₆S₃ | 1529.59 | 7.96 | 34.92 |
| 3403 | C₈₉H₁₇₄N₂O₈S₅ | 1560.68 | 9.46 | 37.71 | 3415 | C₈₇H₁₇₂N₄O₅S | 1386.42 | 8.88 | 34.57 |
| 3404 | C₉₂H₁₇₇NO₆S₈ | 1649.91 | 9.21 | 43.98 | 3416 | C₇₁H₁₃₅N₅O₉S₃ | 1299.07 | 9.96 | 27.23 |
| 3405 | C₉₁H₁₇₇NO₈S₇ | 1637.84 | 8.69 | 40.23 | 3417 | C₇₈H₁₄₉N₄O₁₁P | 1350.04 | 9.88 | 28.11 |
| 3406 | C₈₈H₁₇₁NO₇S₇ | 1579.76 | 9.60 | 39.68 | 3418 | C₇₁H₁₃₁NO₃S₉ | 1335.37 | 9.68 | 29.97 |
| 3407 | C₉₄H₁₉₃N₂O₁₁P ₃S₄ | 1748.74 | 8.86 | 36.61 | 3419 | C₇₅H₁₃₇N₃O₅S₄ | 1289.18 | 9.96 | 29.92 |
| 3408 | C₉₄H₁₉₁N₂O₁₁P ₃S₂ | 1682.61 | 9.39 | 36.62 | 3420 | C₇₁H₁₃₂N₆O₁₀S₆ | 1422.23 | 7.13 | 23.90 |
| 3409 | C₉₇H₁₉₆N₃O₁₅P ₃S | 1769.62 | 9.74 | 34.98 | 3421 | C₆₉H₁₃₃N₅O₈S₅ | 1321.15 | 8.74 | 23.45 |
| 3410 | C₈₈H₁₈₀N₄O₇S₅ | 1566.73 | 9.79 | 37.89 | 3422 | C₇₀H₁₃₉N₄O₁₄PS ₄ | 1420.11 | 7.55 | 24.35 |
| 3411 | C₈₉H₁₈₂NO₁₅P S₄ | 1665.64 | 9.36 | 36.96 | | | | | |
| 3412 | C₈₈H₁₇₆N₂O₁₄S ₄ | 1614.62 | 7.11 | 32.69 | | | | | |

In the present application, the above properties of the exemplary compounds of the first ionizable lipid demonstrate that they can serve as excellent surfactants, and are particularly suitable for preparing lipid nanoparticles to be used as drug delivery carriers.

### (4) Preparation of Blank Lipid Nanoparticle-Based Compositions

### 4.1 Preparation of Blank Lipid Nanoparticles

| Formula No. | Formula Composition | Molar Percentage % of Formula Dosage |
|---|---|---|
| 1 | Compound 1115/DSPC/Chol/ALC-0159 | 40/20/39/1 |
| 2 | Compound 6019/Compound 1114/DSPC/Chol/ALC-0159 | 5/25/15/54.5/0.5 |
| 3 | Compound 6001/Compound 1118/DSPC/Chol/PEG-DMG | 10/30/10/47/3 |
| 4 | Compound 1005/ALC-0315/DSPC/Chol/ALC-0159 | 10/40/10/38.5/1.5 |
| 5 | Compound 6008/Compound 1120/DSPC/Chol/PEG-DMG | 25/25/10/39/1 |
| 6 | Compound 1008/DOPE/Chol/ALC-0159 | 50/10/38.5/1.5 |
| 7 | Compound 1114/DOPC/Chol/PEG-DMG | 45/10/40/5 |
| 8 | Compound 6021/Compound 1009/DOPE/Chol/ALC-0159 | 2.5/30/30/37/0.5 |
| 9 | Compound 6024/Compound 1009/DOPE/Chol/PEG-DMG | 5/45/10/39/1 |
| 10 | Compound 1009/POPC/Chol/PEG-DMG | 40/20/38/2 |
| 11 | Compound 1001/SM102/DOPE/Chol/ALC-0159 | 10/30/15/43.5/1.5 |
| 12 | Compound 1012/cKK-E12/DSPC/Chol/ALC-0159 | 20/25/15/38.5/1.5 |
| 13 | Compound 6018/Compound 1011/DLin-KC2-DMA/DOPE/Chol/ALC-0159 | 2/15/30/10/41/2 |
| 14 | Compound 1013/MVL5/DSPC/Chol/PEG-DMG | 10/40/10/38.5/1.5 |
| 15 | Compound 1005/DOPE/Chol/ALC-0159 | 40/15/43/2 |

Blank lipid nanoparticles were separately prepared according to the above molar ratios, and the specific procedures are as follows:
Lipid starting materials were dissolved in ethanol to obtain a lipid ethanol solution, wherein the total concentration of all lipid starting materials in ethanol was 8 mg/mL. The lipid ethanol solution and 50 mM citrate buffer solution (pH 4.0) were mixed at a volume ratio of 1:3 in a nanoparticle preparation device, followed by ultrafiltration. Samples were collected to obtain blank lipid nanoparticles, and the concentration of lipid in the composition was 2 mg/mL.

### 4.2 Preparation of Blank Lipid Nanoparticle-Based Compositions

| Exampl e No. | Lipid Nanoparticle No. | Nucleic Acid Type | Nucleic Acid Concentration in Delivery System ng/µl | Nucleic Acid Dosage w/w | Lipid Nanoparticle Dosage w/w |
|---|---|---|---|---|---|
| 1 | 1 | eGFP-pDNA | 100 | 36% | 64% |
| 2 | 15 | eGFP-pDNA | 50 | 10% | 90% |
| 3 | 2 | eGFP-mRNA | 300 | 5% | 95% |
| 4 | 3 | eGFP-pDNA | 200 | 2.7% | 97.3% |
| 5 | 4 | Luc-mRNA | 300 | 3.6% | 96.4% |
| 6 | 5 | Epo-mRNA | 100 | 1.8% | 98.2% |
| 7 | 14 | siRNA-cy3 | 150 | 0.5% | 99.5% |
| 8 | 11 | siRNA-cy3 | 300 | 1% | 99% |
| 9 | 6 | eGFP mRNA | 150 | 3.6% | 96.4% |
| 10 | 10 | Luc-mRNA | 300 | 3.6% | 96.4% |
| 11 | 7 | eGFP mRNA | 1000 | 10% | 90% |
| 12 | 8 | Epo-mRNA | 150 | 3.6% | 96.4% |
| 13 | 9 | siRNA-cy3 | 200 | 12% | 88% |
| 14 | 10 | Luc-pDNA | 300 | 7% | 93% |
| 15 | 13 | Luc-pDNA | 500 | 5% | 95% |
| 16 | 3 | Luc-mRNA | 300 | 5% | 95% |
| 17 | 12 | Luc-mRNA | 300 | 1.8% | 98.2% |
| 18 | 6 | Luc-mRNA | 150 | 12% | 88% |

Nucleic acid was used as the active ingredient. Nuclease-free water was used to prepare a nucleic acid solution at twice the concentration specified in each Example, which served as Solution 1. Nuclease-free water was used to dilute blank lipid nanoparticles according to the formula ratio of each Example to form a dilution, which served as Solution 2. Solution 1 and Solution 2 were mixed in equal volumes and vortexed for 2-3 s to obtian blank lipid nanoparticle-based compositions.

### (5) Determination of Particle Size and Zeta Potential of Blank Lipid Nanoparticle-Based Compositions

1. Determination of Particle Size and Polydispersity Index (PDI): the average particle size and PDI of nanoparticle sample solutions prepared in each Example were measured via dynamic light scattering using a Malvern ZetaSizer Nano ZS90 instrument. The detection angle was set to 90°, the refractive index of the dispersant was 1.330, and the test temperature was 25°C.
2. Encapsulation Efficiency Measurement Procedures:
   the encapsulation efficiency of mRNA in blank lipid nanoparticle-based compositions was determined with the Quant-it Ribogreen RNA Quantitation Kit (ThermoFisher Scientific, UK) in accordance with the manufacturer's instructions.
   the encapsulation efficiency of pDNA in blank lipid nanoparticle-based compositions was determined with the dsDNA HS Kit (Vazyme/EQ121) in accordance with the manufacturer's instructions.
   the encapsulation efficiency of siRNA in blank lipid nanoparticle-based compositions was determined with the Qubit^{®} microRNA Reagent Kit in accordance with the manufacturer's instructions.

The data of average particle size, PDI and encapsulation efficiency of blank lipid nanoparticle-based compositions prepared in the Examples are shown in Table 2.

**Table 2 Summary Table of Particle Size, PDI and Encapsulation Efficiency of Blank Lipid Nanoparticle-Based Compositions**

| Example No. | Particle Size Nm | PDI | Encapsulation Efficiency% |
|---|---|---|---|
| 1 | 198.5 | 0.156 | 88.6 |
| 2 | 176.8 | 0.145 | 90.5 |
| 3 | 150.7 | 0.189 | 95.7 |
| 4 | 204.8 | 0.198 | 80.5 |
| 5 | 156.7 | 0.124 | 94.8 |
| 6 | 187.8 | 0.146 | 96.7 |
| 7 | 105.9 | 0.124 | 89.7 |
| 8 | 118.7 | 0.186 | 84.3 |
| 9 | 168.5 | 0.201 | 90.7 |
| 10 | 170.8 | 0.157 | 94.3 |
| 11 | 216.4 | 0.197 | 85.5 |
| 12 | 172.4 | 0.136 | 92.3 |
| 13 | 189.1 | 0.201 | 80.4 |
| 14 | 203.4 | 0.096 | 82.1 |
| 15 | 152.8 | 0.168 | 82.8 |
| 16 | 179.4 | 0.149 | 86.7 |
| 17 | 126.7 | 0.125 | 89.1 |
| 18 | 465.7 | 0.204 | 82.4 |

It can be seen from Table 2 that the blank lipid nanoparticle-based compositions provided by the present application feature small and uniform particle sizes as well as high encapsulation efficiency (>80%).

### (6) Evaluation of In Vivo Delivery Effect of Blank Lipid Nanoparticle-Based Compositions

### 6.1 Evaluation of In Vivo Delivery Effect of Blank Lipid Nanoparticle-mRNA Complexes after Intravenous Administration in Mice

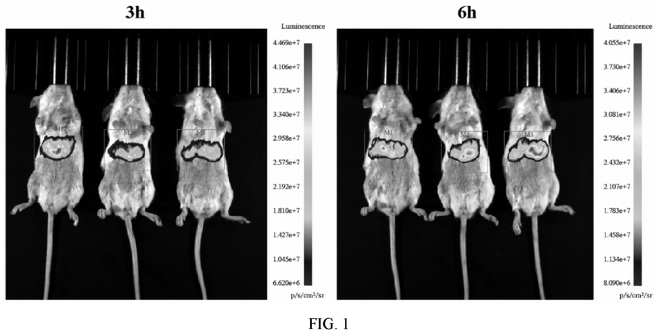

Female Balb/c mice aged 6-8 weeks were used to evaluate the in vivo delivery efficiency of blank lipid nanoparticle-firefly luciferase mRNA (Luciferase mRNA) complexes prepared in Example 5. The blank lipid nanoparticle-Luciferase mRNA complexes were administered via tail vein injection at a single dose of 0.3 mpk. At predetermined time points post-administration (e.g., 3 h and 6 h), the fluorescent imaging substrate was intraperitoneally injected into the mice. In vivo imaging of the animals was performed using a PerkinElmer small animal imaging system to determine bioluminescent signals, and the results are shown in Table 3 and Figure 1.

**Table 3 Evaluation of In Vivo Activity of Blank Lipid Nanoparticle-mRNA Complexes**

| Example | Total whole-body signal of mice [p/s] | |
|---|---|---|
| Example 5 | 3h | 6h |
| | 5.52E+08 | 6.40E+08 |

of Example 5

The results demonstrated that after the blank lipid nanoparticles provided by the present application were complexed with Luciferase mRNA, a high expression level of luciferase protein could be achieved in mice.

Female Balb/c mice aged 6-8 weeks were used to evaluate the in vivo delivery efficiency of blank lipid nanoparticle-human erythropoietin mRNA (hEPO mRNA) complexes prepared in Example 6 and Example 12. The blank lipid nanoparticle-hEPO mRNA complexes were administered via tail vein injection at single doses of 1.5 mpk and 3 mpk, respectively. The hEPO expression level in mice was determined at a predetermined time point post-administration (24 h), and the results are shown in Table 4.

**Table 4 hEPO Expression Level (pg/mL) at 24 h After Administration of Blank Lipid Nanoparticle-human Erythropoietin mRNA Complexes of Examples 6 and 12**

| Batch No. | Example 6 | | Example 12 | |
|---|---|---|---|---|
| Dosage Administered | 1.5mpk | 3mpk | 1.5mpk | 3mpk |
| 24h | 8.02E+06 | 1.53E+07 | 5.10E+06 | 1.62E+07 |

It can be seen that after the blank lipid nanoparticles provided by the present application are complexed with human erythropoietin mRNA, a high hEPO expression level can be achieved in mice via intravenous injection.

### 6.2 Evaluation of In Vivo Delivery Effect of Lipid Nanoparticle-mRNA Complexes via Intramuscular Injection in Mice

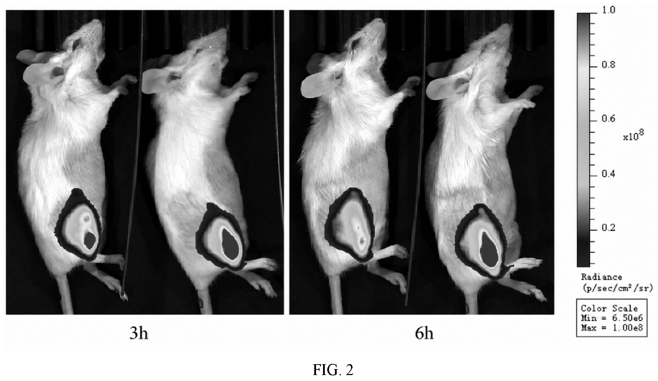

Female Balb/c mice aged 6-8 weeks were used to evaluate the in vivo delivery efficiency of lipid nanoparticle-luciferase mRNA complexes prepared in Example 10. The blank lipid nanoparticle-luciferase mRNA complexes were administered via intramuscular injection at a single dose of 0.3 mpk. At predetermined time points post-administration (3 h and 6 h), the bioluminescent substrate was intraperitoneally injected into the mice. In vivo imaging of the animals was performed using a PerkinElmer small animal imaging system to determine bioluminescent signals, and the results are shown in Table 5 and Figure 2.

**Table 5 Evaluation of In Vivo Activity 3/6 h After Administration of Blank Lipid Nanoparticle-mRNA Complexes of Example 10**

| Example | Administration Route | Total Signal Value [p/s] | |
|---|---|---|---|
| | Time | 3h | 6h |
| Example 10 | Intramuscular Injection | 2.51E+09 | 2.76E+09 |

The results demonstrate that after the blank lipid nanoparticles provided by the present application are complexed with luciferase mRNA, a high expression level of luciferase protein can be achieved in mice via intramuscular injection.

### 6.3 Intraperitoneal Administration (IP) and Subcutaneous Administration (SC)

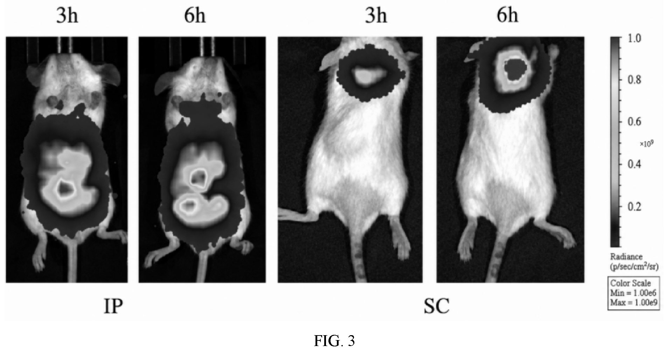

Female Balb/c mice aged 6-8 weeks were used to evaluate the in vivo delivery efficiency of lipid nanoparticle-firefly luciferase mRNA (Luciferase mRNA) complexes prepared in Example 16. The blank lipid nanoparticle-Luciferase mRNA complexes were administered via intraperitoneal injection and subcutaneous injection respectively at a single dose of 0.3 mpk. At predetermined time points post-administration (3 h and 6 h), the bioluminescent substrate was intraperitoneally injected into the mice. In vivo imaging of the animals was performed using a PerkinElmer small animal imaging system to determine bioluminescent signals, and the results are shown in Table 6 and Figure 3.

**Table 6 Evaluation of In Vivo Activity of Blank Lipid Nanoparticle-mRNA Complexes of Example 16**

| Example | Administration Route | Total Whole-Body Signal of Mice [p/s] | |
|---|---|---|---|
| | Time | 3h | 6h |
| Example 16 | IP | 7.65E+09 | 8.42E+09 |
| | SC | 3.52E+09 | 1.80E+10 |

The results demonstrate that after the blank lipid nanoparticles provided by the present application are complexed with Luciferase mRNA, a high expression level of luciferase protein can be achieved in mice via both intraperitoneal injection and subcutaneous injection.

The technical solutions of the present application are not limited to those disclosed in the above embodiments, and further include any technical solutions formed by any combination of the above technical features. The foregoing describes specific embodiments of the present application. It should be pointed out that those skilled in the art of the present application may make several improvements and modifications without departing from the principles of the present application, and such improvements and modifications shall also fall within the protection scope of the present application.

## Claims

1. Use of blank lipid nanoparticles in the preparation of an in-vivo delivery product, wherein the use comprises the step of mixing the blank lipid nanoparticles with a biologically active substance in a solvent to obtain a blank lipid nanoparticle-based composition; wherein the blank lipid nanoparticles comprise: 10-60 mol% of a first ionizable lipid, 0-30 mol% of a second ionizable lipid, 5-30 mol% of a phospholipid, 15-70 mol% of a steroid or a derivative thereof, and 0-10 mol% of a polyethylene glycol-lipid conjugate.

2. The use according to claim 1, wherein the use further comprises the step of introducing the blank lipid nanoparticle-based composition into an animal body.

3. The use according to claim 2, wherein routes for introducing the blank lipid nanoparticle-based composition into an animal body comprise oral administration, intranasal administration, intravenous administration, intraperitoneal administration, intramuscular administration, intra-articular administration, intralesional administration, intratracheal administration, subcutaneous administration or intradermal administration.

4. The use according to claim 2, wherein the animal is a mammal or a non-mammal.

5. The use according to claim 1, wherein the biologically active substance comprises nucleic acids, proteins, polypeptides and small-molecule compounds; wherein the nucleic acids are DNA and/or RNA.

6. The use according to claim 5, wherein the nucleic acid comprises at least one of siRNA, miRNA, saRNA, sgRNA, dsRNA, shRNA, smRNA, ssRNA, mRNA, circRNA, snRNA, crRNA, IncRNA, snoRNA, piRNA, pDNA, ssDNA, circular or linear DNA, DNA minicircles and msDNA.

7. The use according to claim 1, wherein the amount of the nucleic acid accounts for 0.5%-50% (w/w) of the total amount of the blank lipid nanoparticles and the nucleic acid; and wherein, in the blank lipid nanoparticle-based composition, the concentration of the biologically active substance ranges from 50-5000 ng/µl.

8. The use according to any one of claims 1-7, wherein the first ionizable lipid is selected from a compound having a general formula (1), or a pharmaceutically acceptable salt, stereoisomer and tautomer thereof; 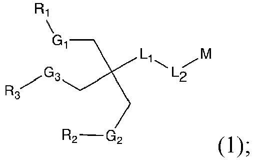
wherein R₁, R₂ and R₃ are each independently H, linear or branched C₅₋₄₀ alkyl, linear or branched C₅₋₄₀ alkenyl, linear or branched C₅₋₄₀ alkynyl, 3- to 6-membered saturated or partially unsaturated cycloalkyl having 1-3 side chains, or 6- to 10-membered aromatic group having 1-3 side chains; wherein the side chains are each independently selected from linear or branched C₁₀₋₃₀ alkyl, linear or branched C₁₀₋₃₀ alkenyl, and linear or branched C₁₀₋₃₀ alkynyl, provided that at most one of R₁, R₂ and R₃ is H;
M is selected from -NR₄R₅, 3- to 6-membered saturated or partially unsaturated heterocyclic group containing at least one nitrogen atom, and 6- to 10-membered heteroaromatic group containing at least one nitrogen atom; wherein the heterocyclic group and the heteroaromatic group are unsubstituted or substituted with one or more -OH, carboxy, amino, oxo or halogen;
R₄ and R₅ are each independently H, linear or branched C₁₋₆ alkyl, linear or branched C₂₋₆ alkenyl, or linear or branched C₂₋₆ alkynyl; wherein the linear or branched C₁₋₆ alkyl, linear or branched C₂₋₆ alkenyl, or linear or branched C₂₋₆ alkynyl is unsubstituted or substituted with one or more -OH, carboxy, aminoamide group, amidino, guanidino, or halogen;
G₁, G₂ and G₃ are each independently -O-, -S-, -NR₆-, -S-S-, -C(=O)-, -C(=S)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₆-, -NR₆-S(=O)₂-, -P(=O)(OR₆)O-, -OP(=O)(OR₆)- or -OP(=O)(OR₆)O-; wherein each R₆ and R₁₃ are each independently selected from H, hydroxy, linear or branched C₁₋₃₀ alkyl or cycloalkyl, and linear or branched C₂₋₃₀ alkenyl;
L₁ is selected from -X₁- or -(CR₇R₈)m-X₁-; wherein each X₁ is independently selected from -O-, -S-, -NR₁₄-, -S-S-, -C(=O)-, -C(=S)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₄-, -NR₁₄-S(=O)₂-, -P(=O)(OR₁₄)O-, -OP(=O)(OR₁₄)- or -OP(=O)(OR₁₄)O-; wherein m is an integer of 2-6, R₇ and R₈ are each independently H, hydroxy, halogen, linear or branched C₁₋₆ alkyl or cycloalkyl, linear or branched C₂₋₆ alkenyl, and each R₁₄ and R₁₅ are each independently selected from H, linear or branched C₁₋₃₀ alkyl or cycloalkyl, linear or branched C₂₋₃₀ alkenyl;
L₂ is -(CR₉R₁₀)ₙ- or -(CR₉R₁₀)ₙ-X₂-(CR₁₁R₁₂)ₖ-; wherein X₂ is selected from -O-, -S-, -NR₁₆-, -S-S-, -C(=O)-, -C(=S)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₆-, -NR₁₆-S(=O)₂-, -P(=O)(OR₁₆)O-, -OP(=O)(OR₁₆)- or -OP(=O)(OR₁₆)O-; wherein n is an integer of 1-6; k is an integer of 1-6; R₉, R₁₀, R₁₁ and R₁₂ are each independently H, hydroxy, halogen, linear or branched C₁₋₆ alkyl or cycloalkyl, linear or branched C₂₋₆ alkenyl, and each R₁₆ and R₁₇ are each independently selected from H, linear or branched C₁₋₃₀ alkyl or cycloalkyl, linear or branched C₂₋₃₀ alkenyl;
wherein the alkyl, cycloalkyl and alkenyl in R₄ to R₁₇ are unsubstituted or substituted with one or more groups selected from hydroxy, mercapto, amino, substituted amino or halogen;
wherein the salts exclude quaternary ammonium salts.

9. The use according to claim 8, wherein R₁, R₂ and R₃ are each independently the following groups: 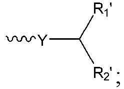 wherein Y is absent, or linear or branched C₁₋₃₀ alkyl or cycloalkyl, linear or branched C₂₋₂₀ alkenyl, linear or branched C₂₋₂₀ alkynyl; R₁' and R₂' are each independently H, linear or branched C₁₋₃₀ alkyl, linear or branched C₂₋₃₀ alkenyl, linear or branched C₂₋₃₀ alkynyl, and the total carbon chain length of Y, R₁' and R₂' is 8-40.

10. The use according to claim 8, wherein R₁, R₂ and R₃ are each independently selected from the following groups: 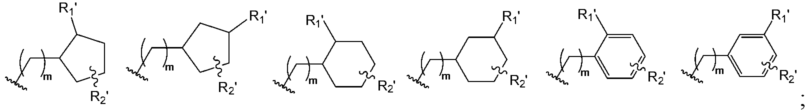 wherein R₁' and R₂' are each independently H, linear or branched C₁₋₃₀ alkyl, linear or branched C₂₋₃₀ alkenyl, linear or branched C₂₋₃₀ alkynyl, and the total carbon chain length of R₁' and R₂' is 8-30.

11. The use according to claim 8, wherein R₁, R₂ and R₃ are each independently selected from any one of the following groups:

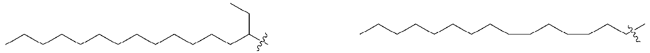

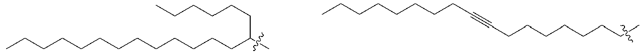

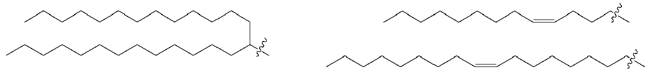

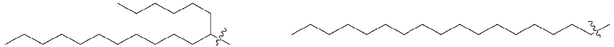

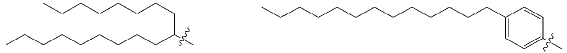

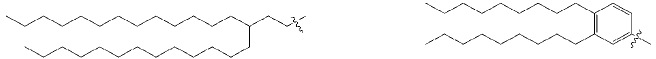

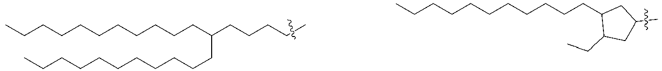

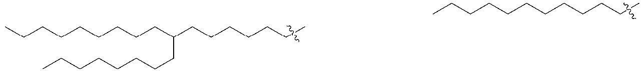

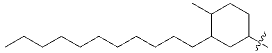

12. The use according to claim 8, wherein G₁, G₂ and G₃ are each independently -O-, -S-, -NR₆-, -S-S-, -C(=O)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -P(=O)(OR₆)O-, -OP(=O)(OR₆)- or -OP(=O)(OR₆)O-.

13. The use according to claim 8, wherein L₁ is selected from -(CR₇R₈)ₘ-X₁-, wherein X₁ is selected from -O-, -S-, -NR₁₄-, -S-S-, -C(=O)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -P(=O)(OR₁₄)O-, -OP(=O)(OR₁₄)- or -OP(=O)(OR₁₄)O-.

14. The use according to claim 8, wherein L₂ is -(CR₉R₁₀)ₙ-X₂-(CR₁₁R₁₂)ₖ-, and wherein X₂ is selected from -O-, -S-, -NR₁₆-, -S-S-, -C(=O)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -P(=O)(OR₁₆)O-, -OP(=O)(OR₁₆)- or -OP(=O)(OR₁₆)O-.

15. The use according to claim 8, wherein M is selected from the following structures: 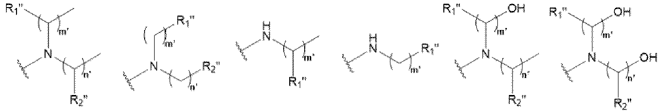 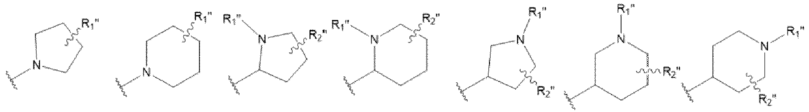 wherein m' and n' are each independently an integer of 0-6, R1" and R2" are each independently H, C₁₋₆ alkyl, C₂₋₆ alkenyl, guanidino, amidino, amido, aliphatic amino, 3- to 10-membered nitrogen-containing heterocycles; wherein the nitrogen-containing heterocycles are selected from pyrrole, imidazole, pyridine, pyrazole, triazole, oxazole, isoxazole, thiophene, isothiazole, pyridazine, pyrazine, piperazine, indole, benzimidazole, carbazole, quinoline, isoquinoline, purine and pyrimidine, and tautomeric forms thereof, which are unsubstituted or optionally substituted with one or more organic groups selected from hydroxy, mercapto, amino, substituted amino, halogen, C₁₋₆ alkyl, C₂₋₆ alkenyl, C₂₋₆ alkynyl and C₆₋₁₄ aryl.

16. The use according to claim 8, wherein the compound of formula (1) is selected from the compound shown in formula (1A):

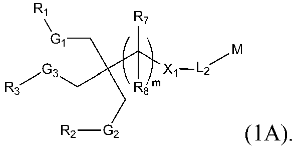

17. The use according to claim 8, wherein the compound of formula (1) is selected from the compound shown in formula (1B):

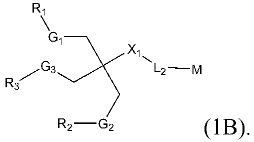

18. The use according to claim 8, wherein the compound of formula (1) is selected from the compound shown in formula (1C):

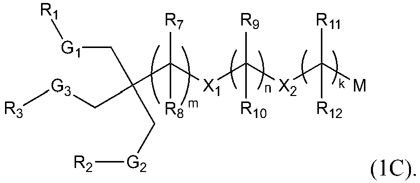

19. The use according to claim 17, wherein the compound of formula (1) is selected from the compound shown in formula (1D):

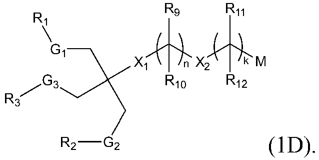

20. The use according to claim 19, wherein the compound of formula (1) is selected from the compound shown in formula (1E):

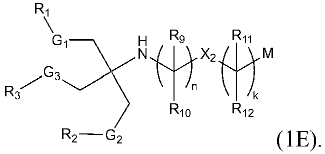

21. The use according to claim 20, wherein the compound of formula (1) is selected from the compound shown in formula (1F):

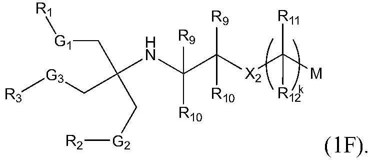

22. The use according to claim 21, wherein the compound of formula (1) is selected from the compound shown in formula (1G):

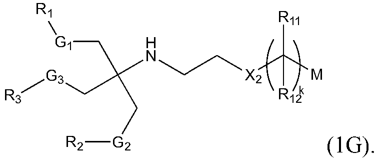

23. The use according to claim 21, wherein the compound of formula (1) is selected from the compound shown in formula (1H):

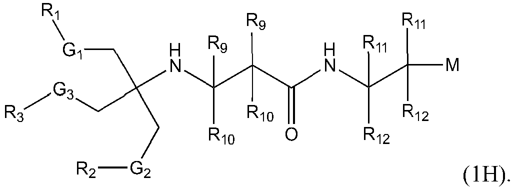

24. The use according to claim 8, wherein the compound of formula (1) is selected from the compound shown in formula (11):

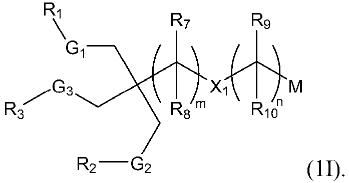

25. The use according to claim 17, wherein the compound of formula (1) is selected from the compound shown in formula (1J):

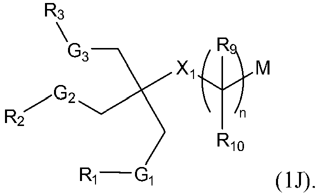

26. The use according to claim 24, wherein the compound of formula (1) is selected from the compound shown in formula (1K):

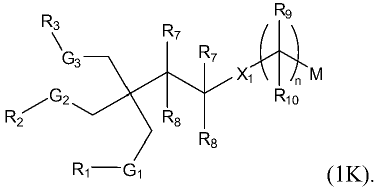

27. The use according to claim 9, wherein Y is absent, and the compound of formula (1) is selected from the compound shown in formula (1L): 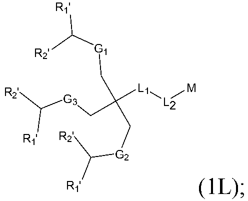 wherein R₁' and R₂' are each independently selected from H, linear or branched C₁₋₃₀ alkyl, linear or branched C₂₋₃₀ alkenyl, linear or branched C₂₋₃₀ alkynyl, and the total carbon chain length of R₁' and R₂' is 8-40.

28. The use according to claim 9, wherein the compound of formula (1) is selected from the compound shown in formula (1M):

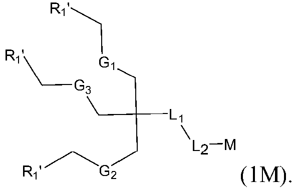

29. The use according to claim 8, wherein the compound of formula (1) is selected from:

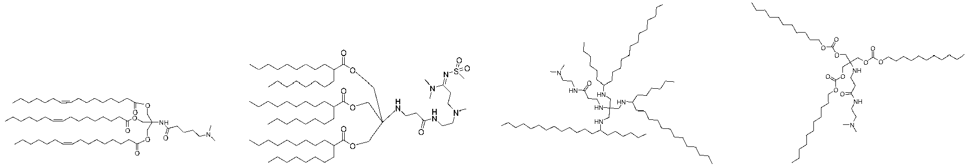

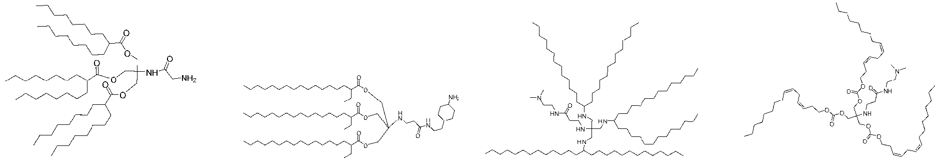

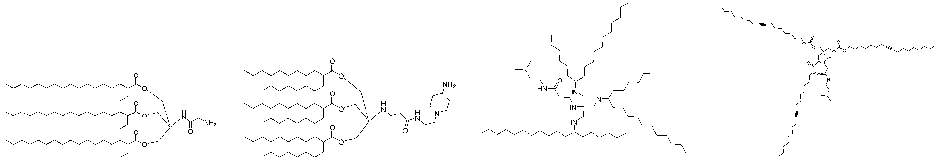

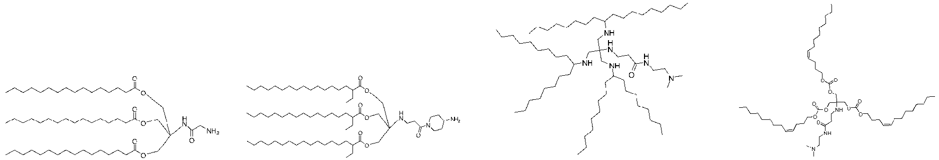

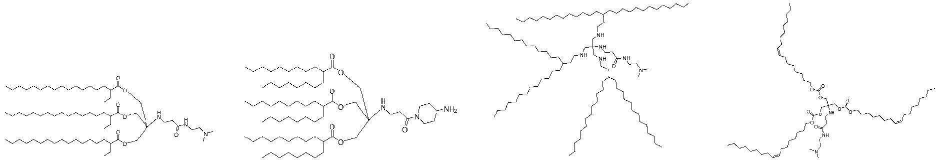

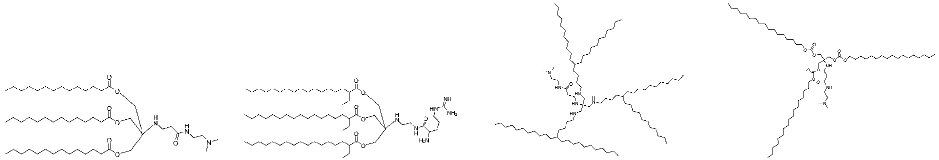

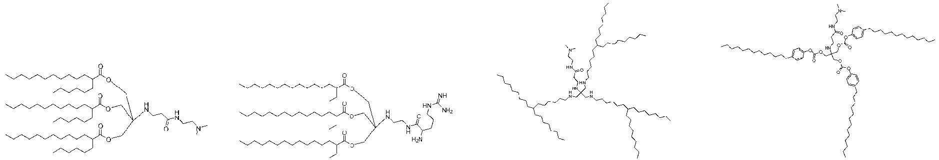

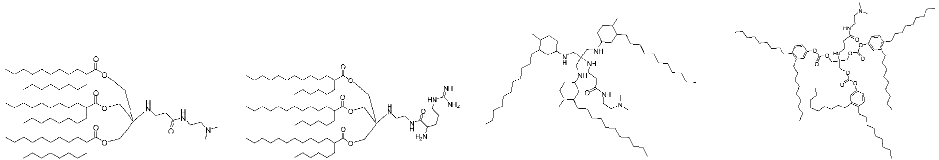

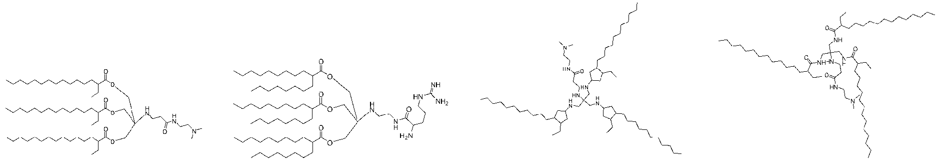

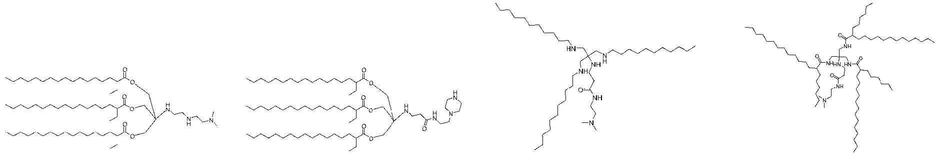

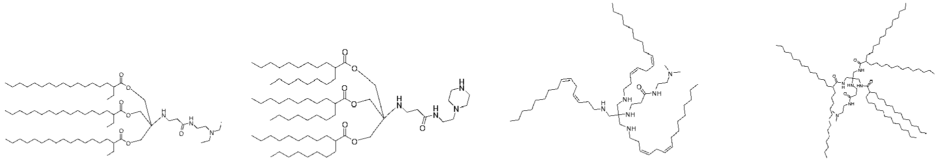

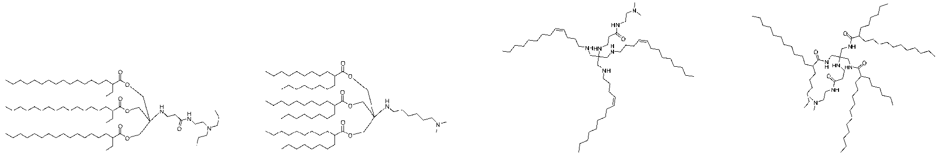

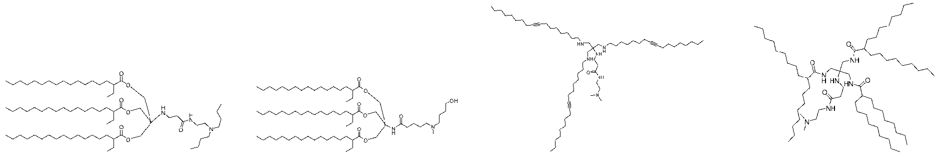

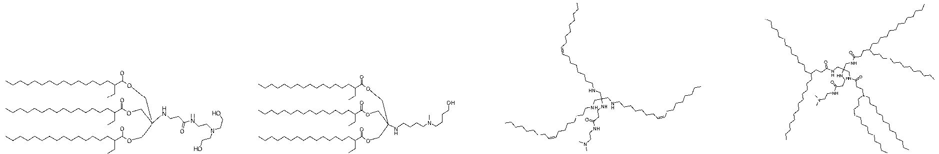

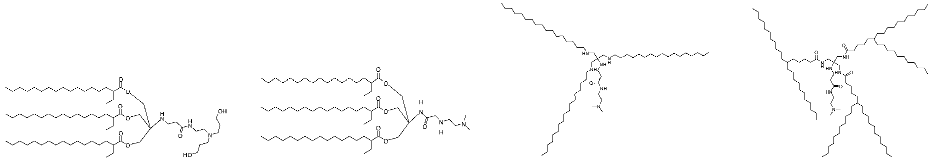

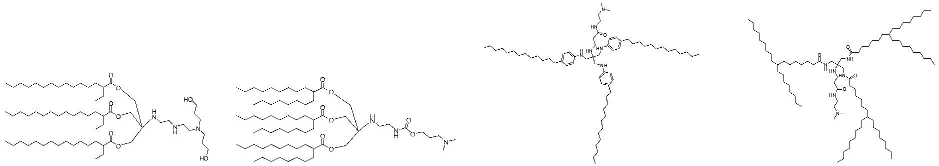

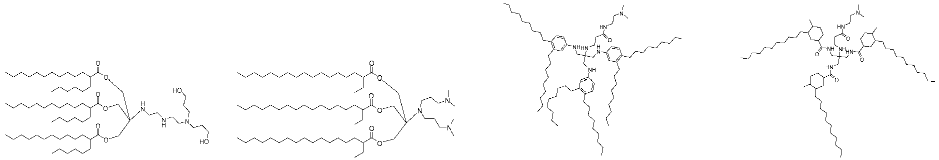

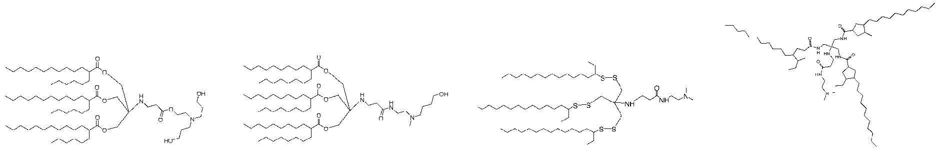

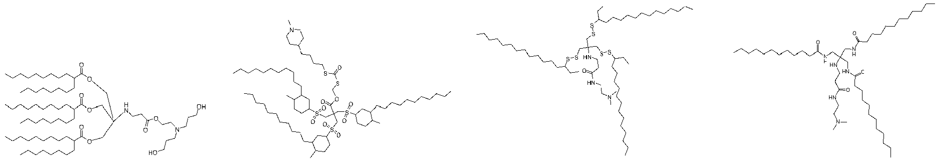

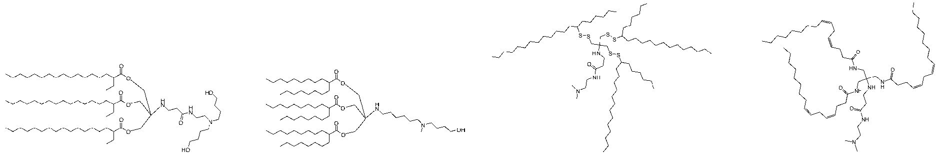

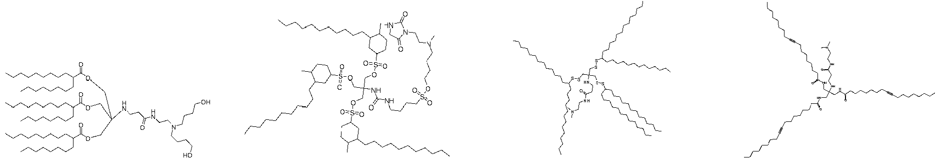

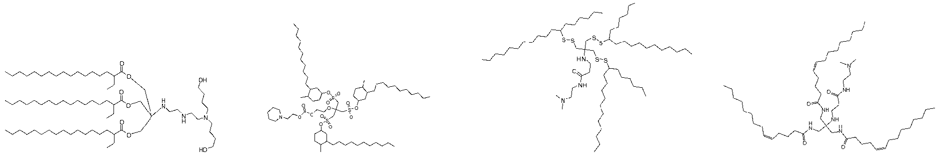

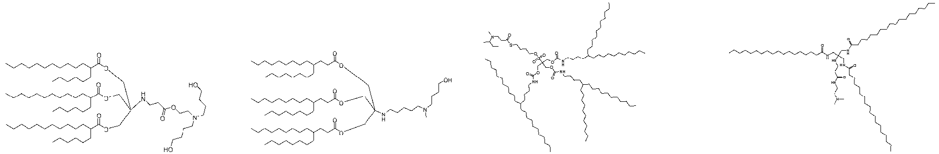

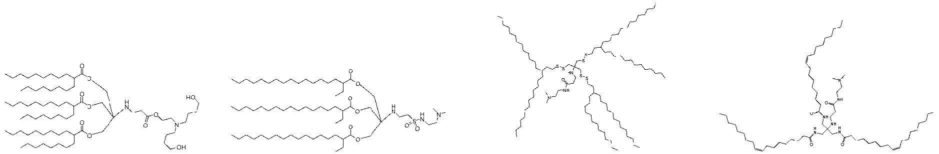

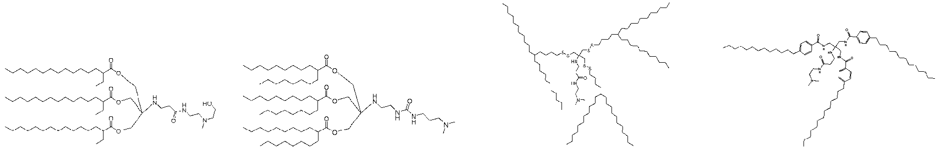

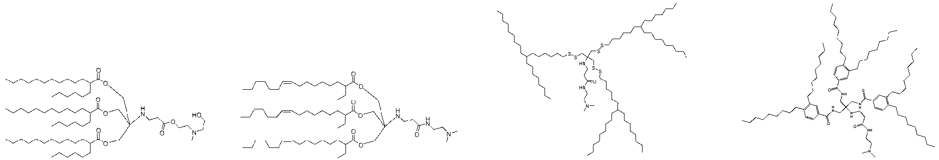

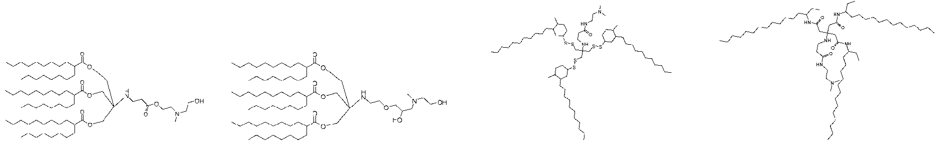

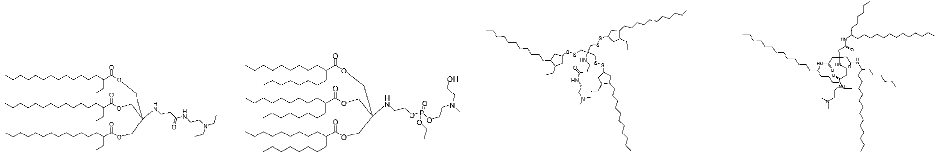

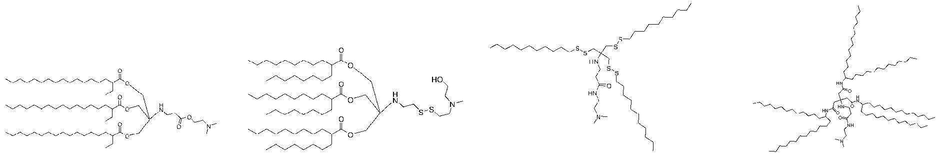

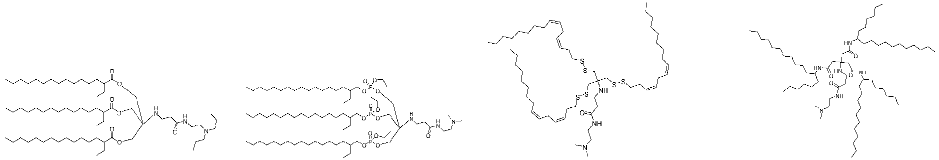

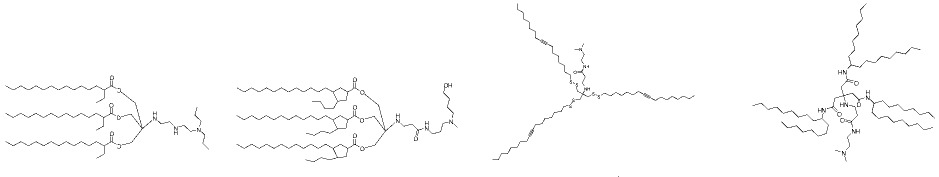

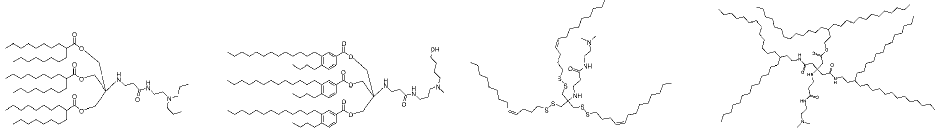

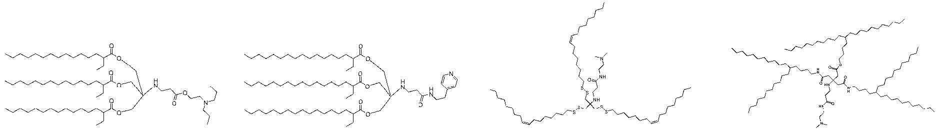

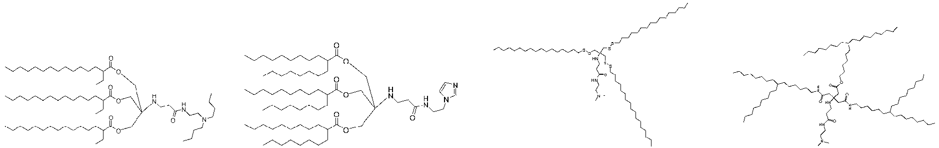

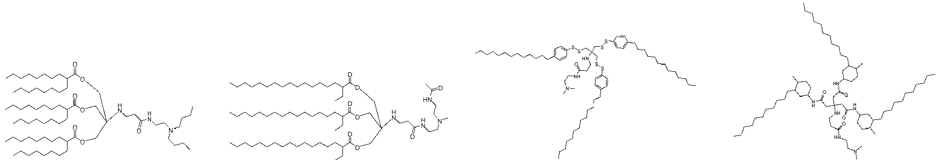

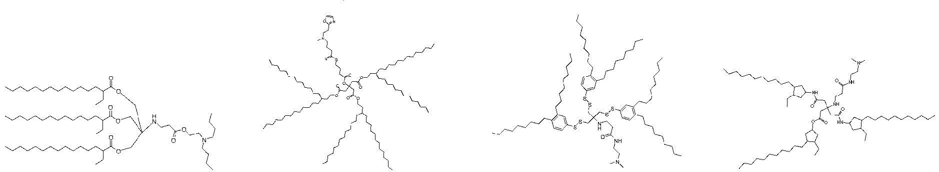

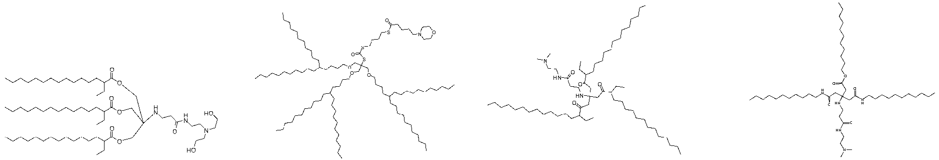

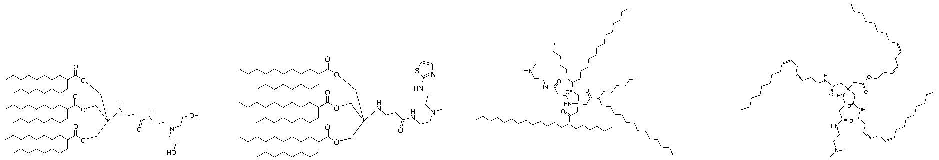

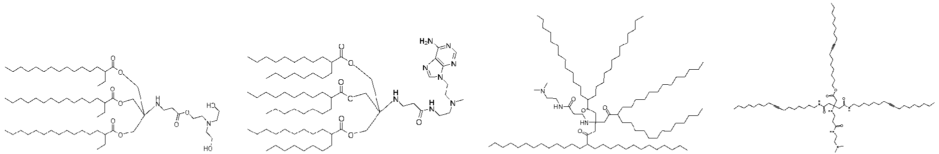

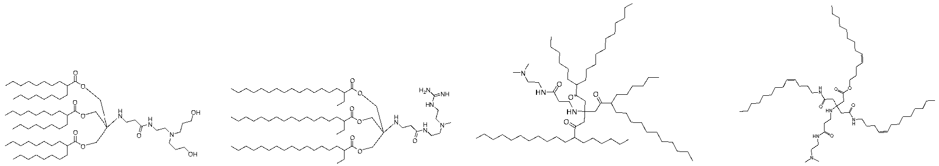

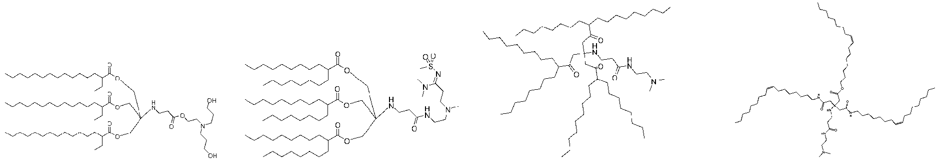

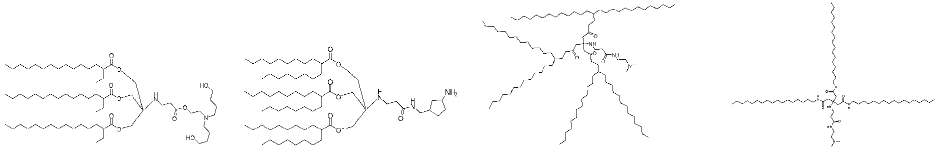

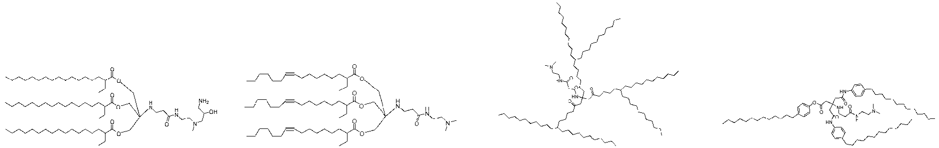

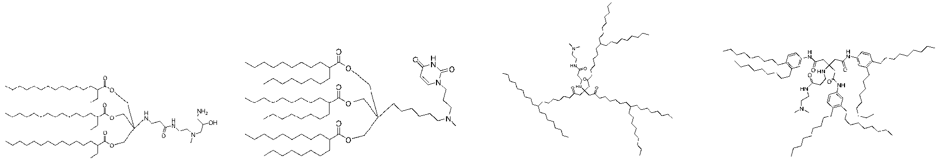

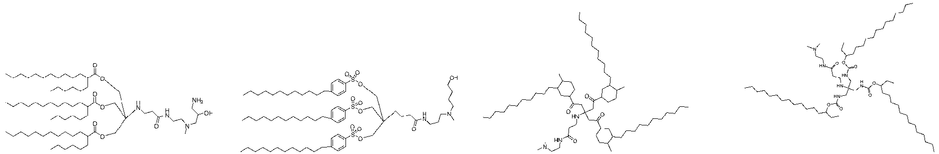

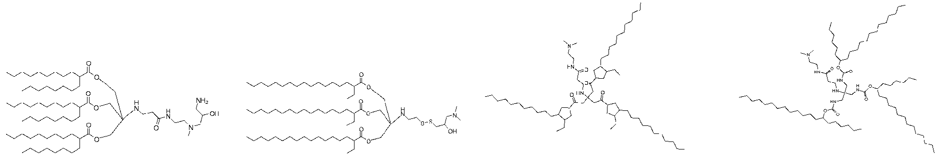

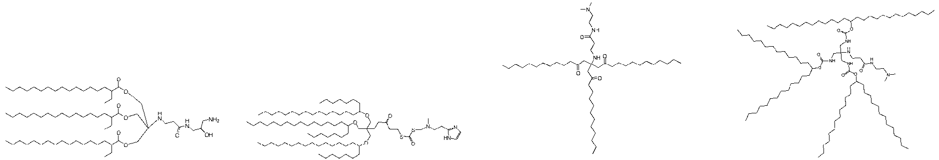

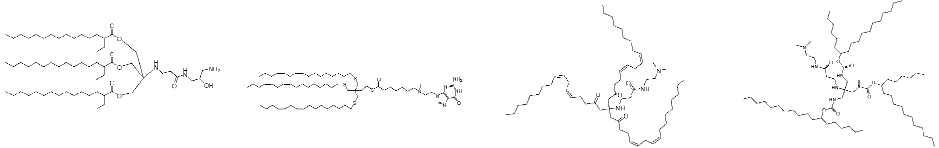

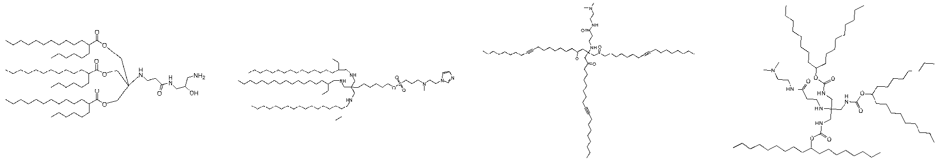

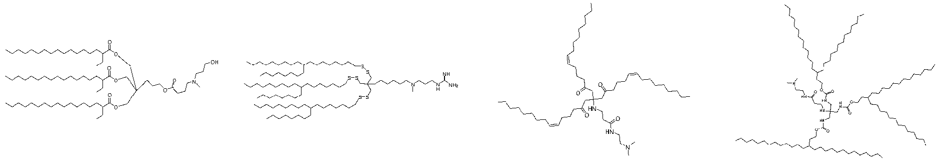

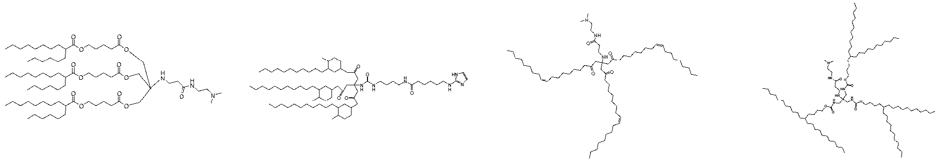

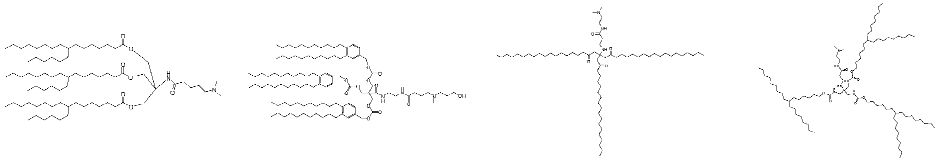

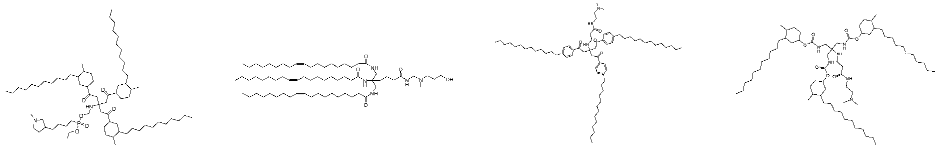

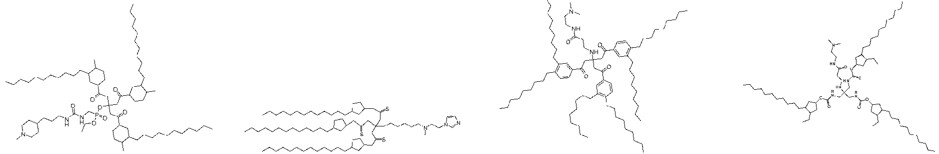

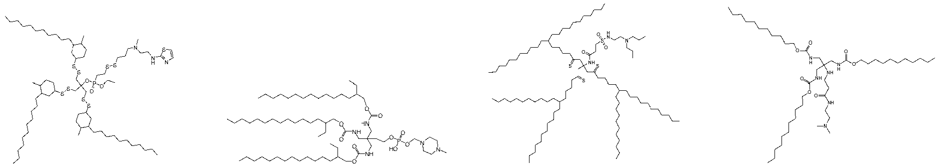

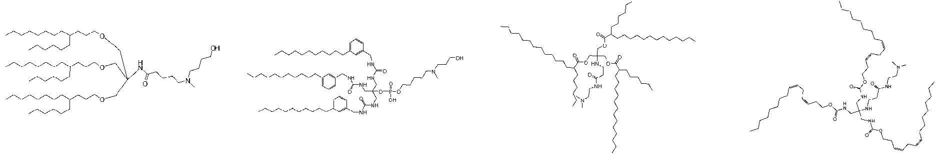

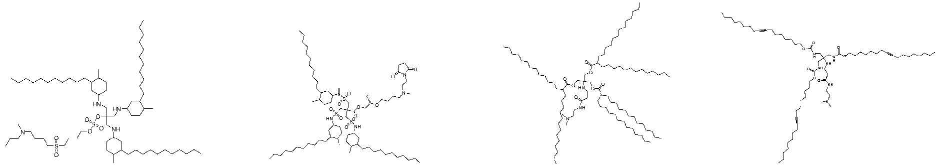

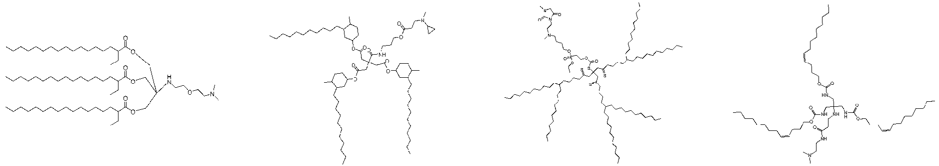

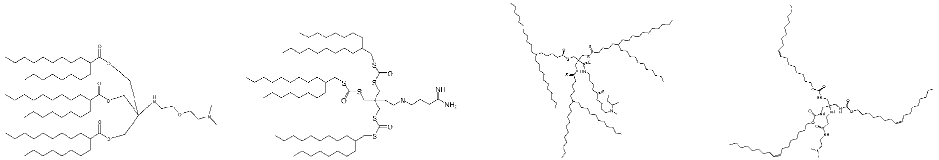

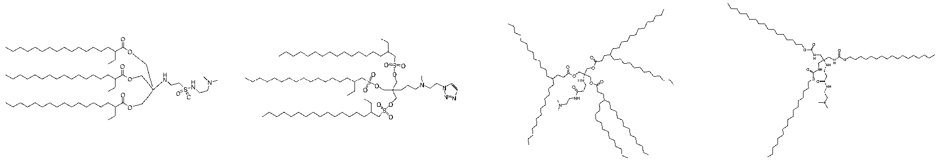

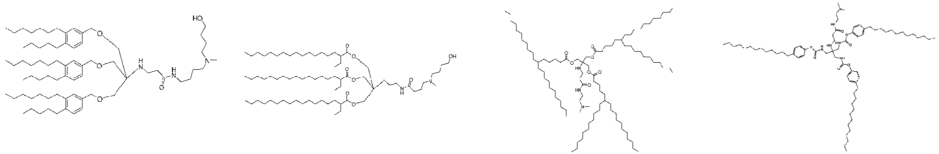

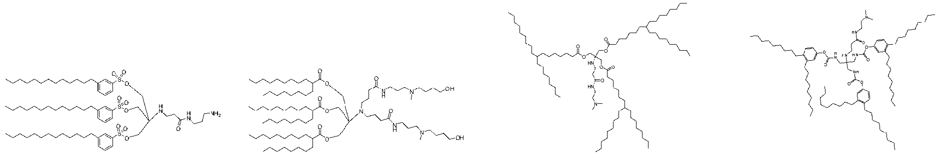

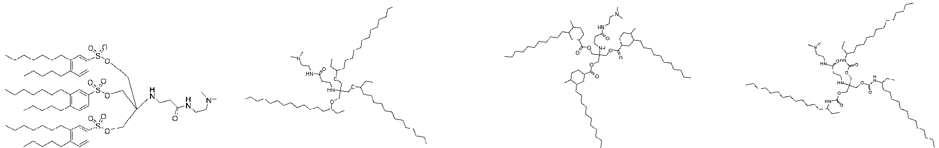

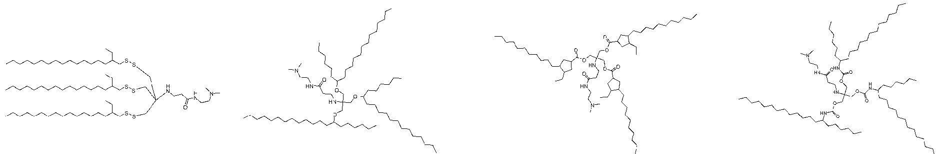

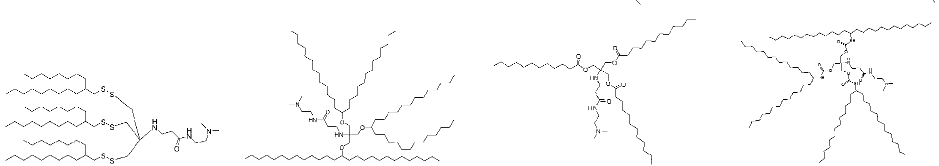

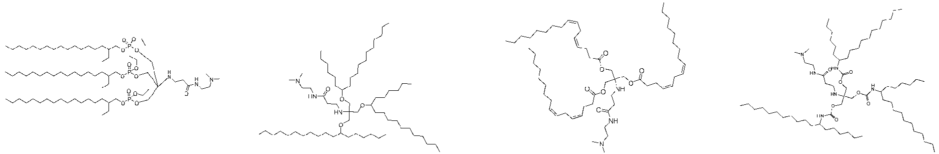

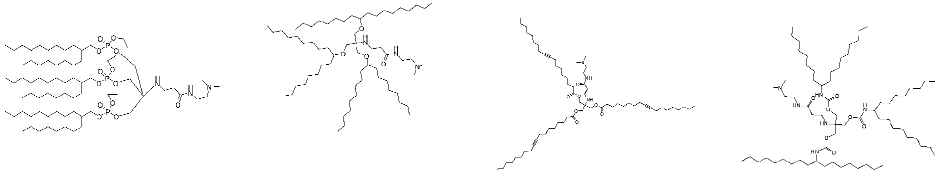

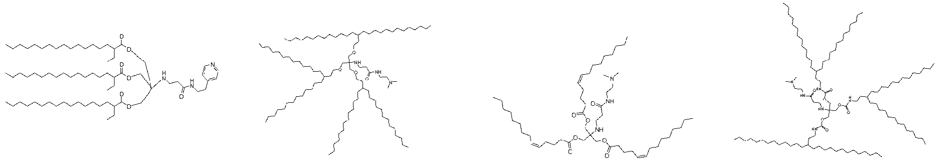

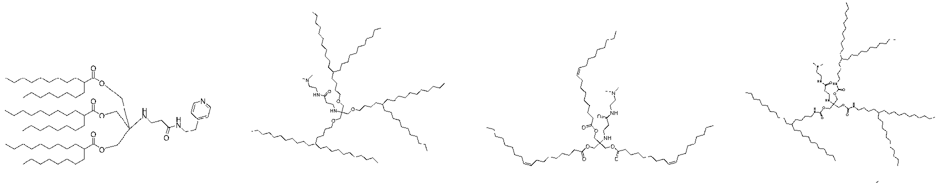

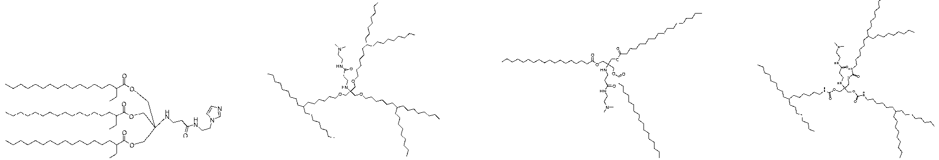

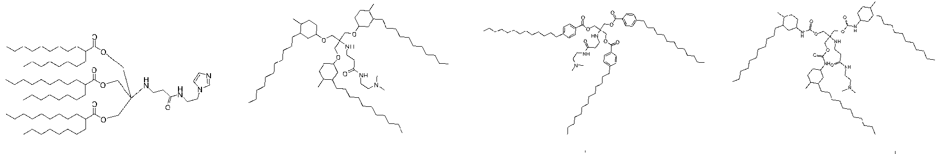

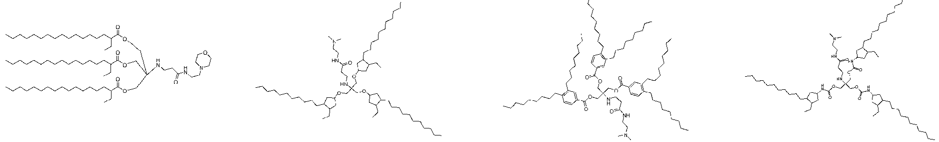

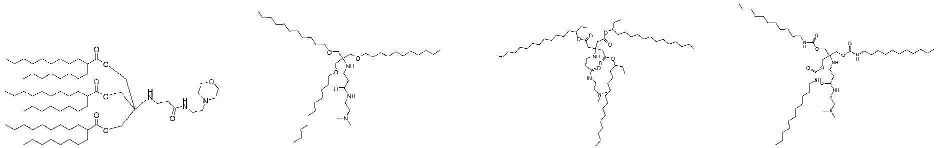

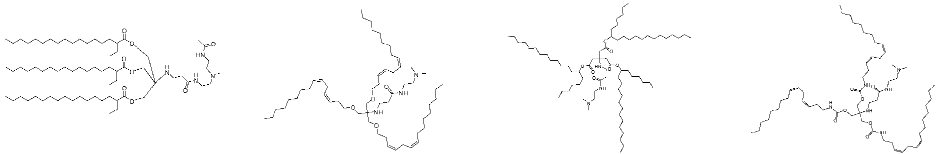

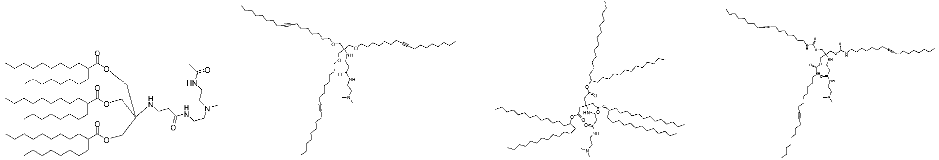

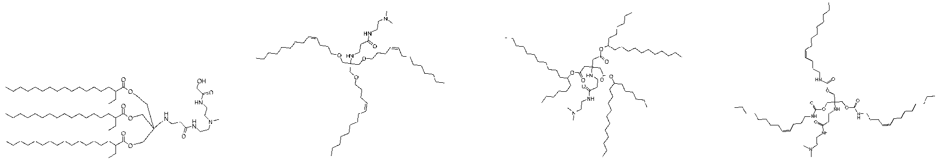

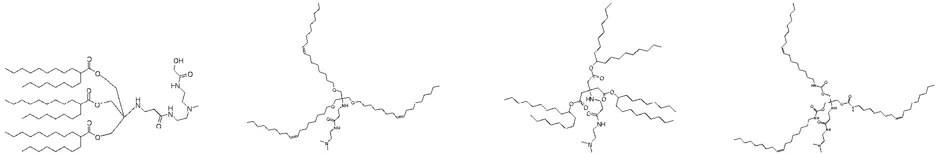

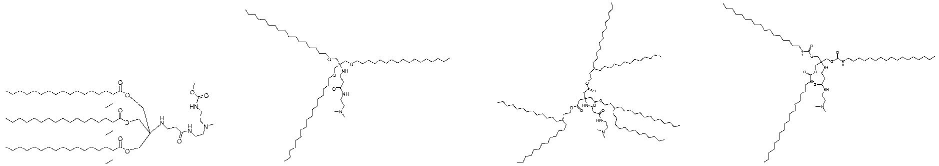

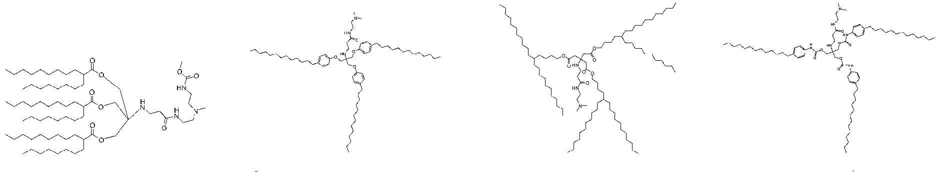

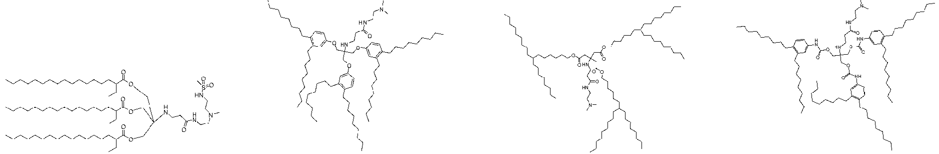

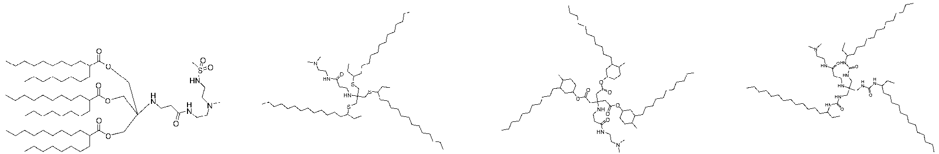

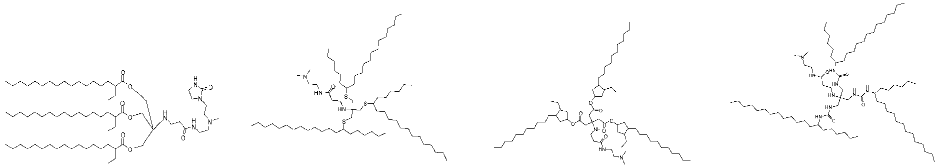

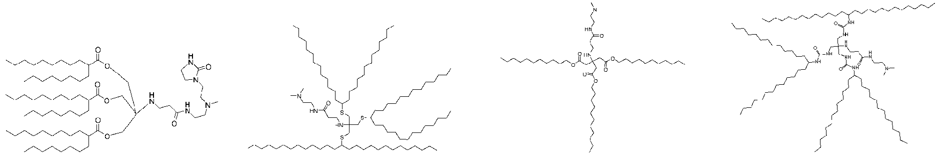

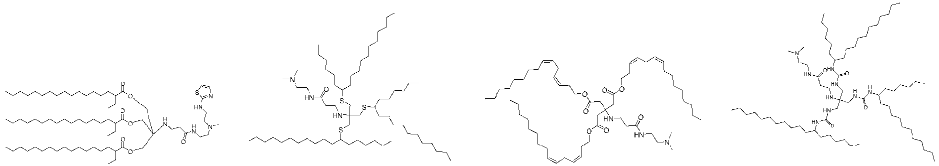

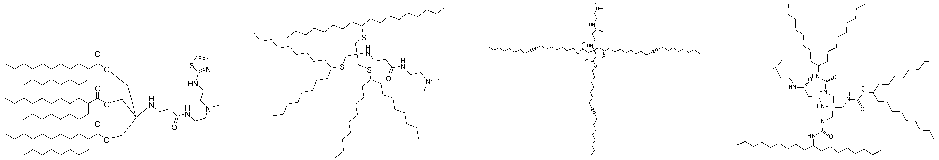

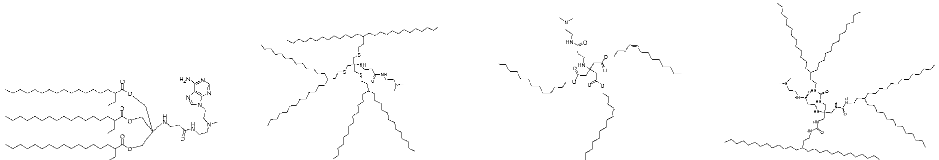

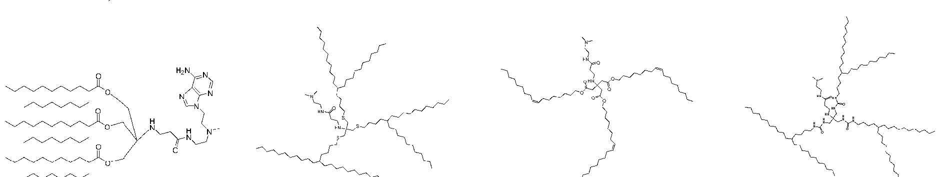

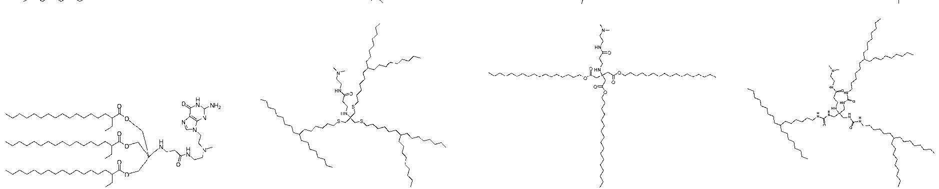

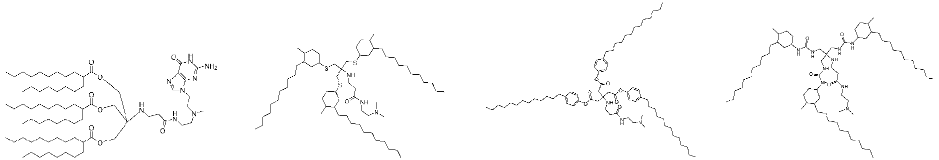

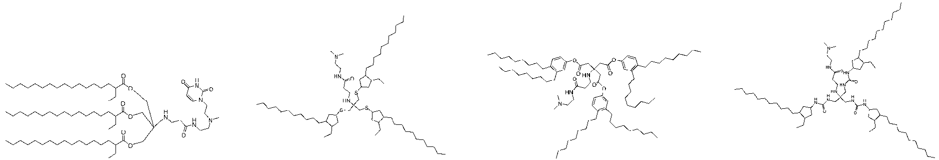

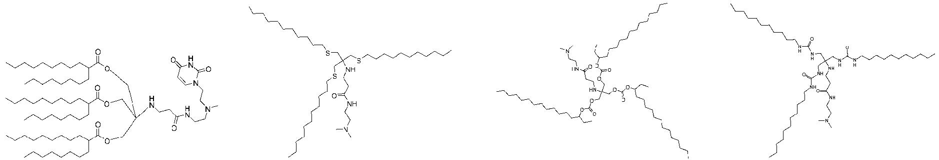

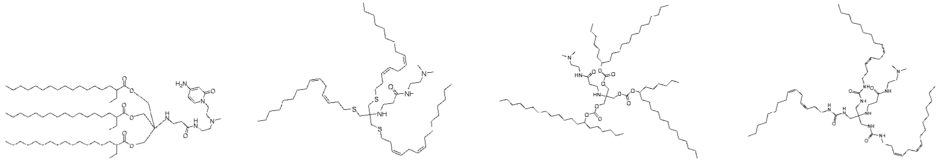

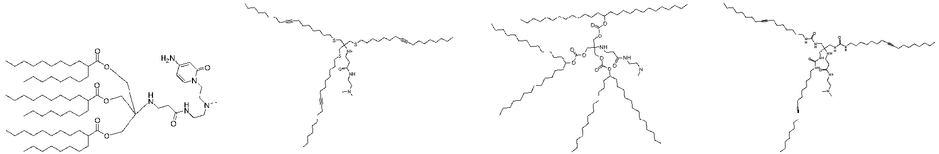

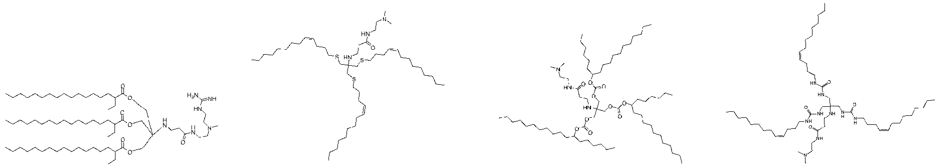

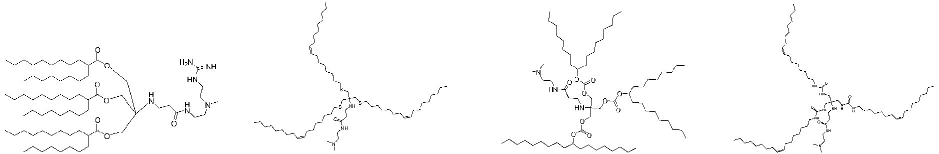

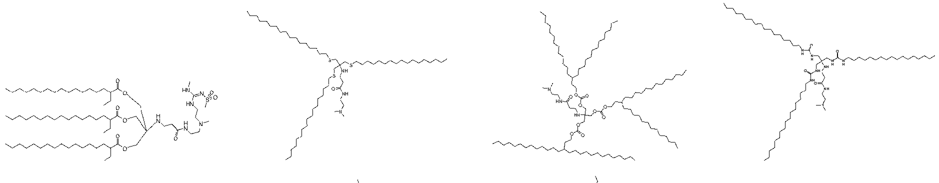

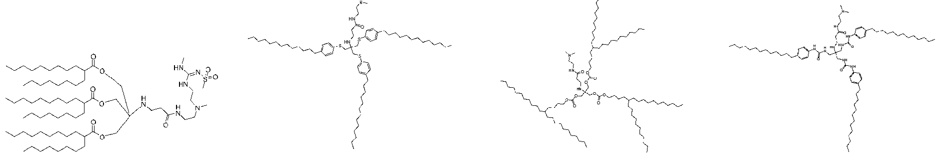

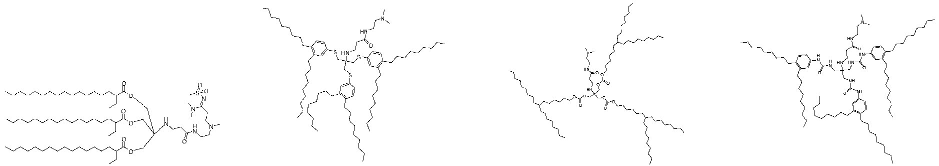

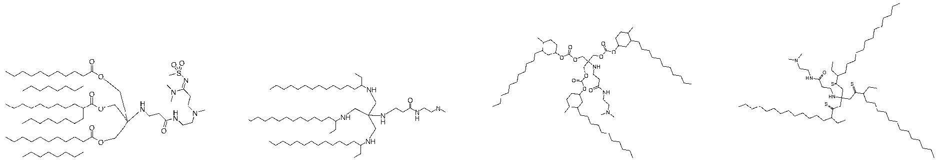

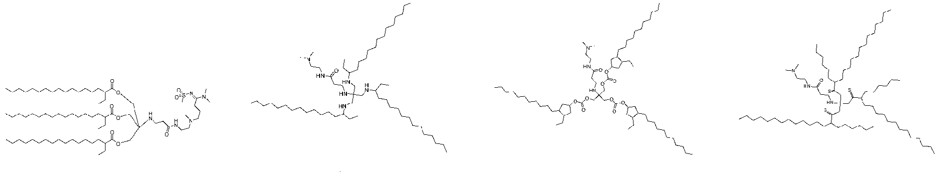

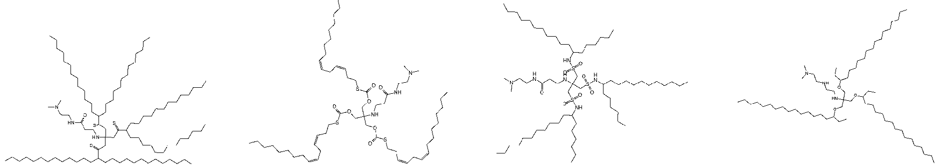

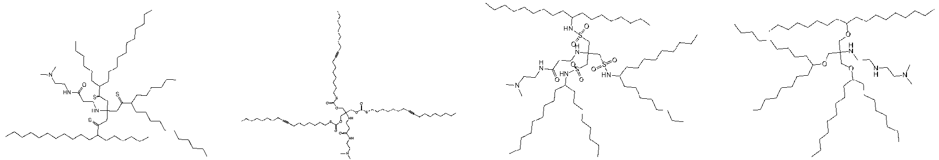

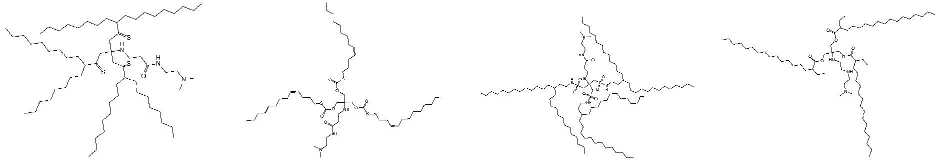

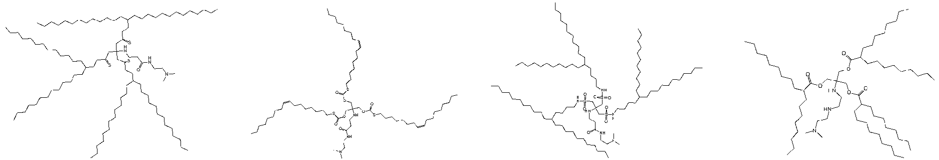

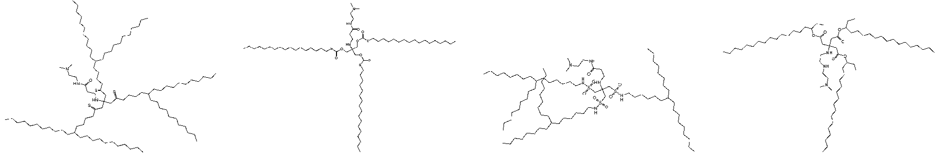

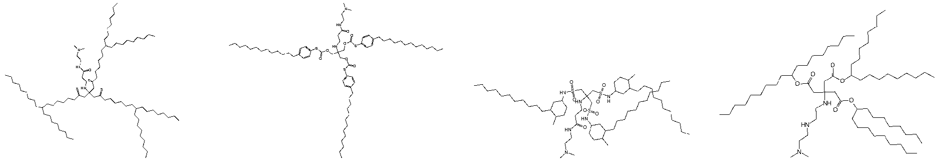

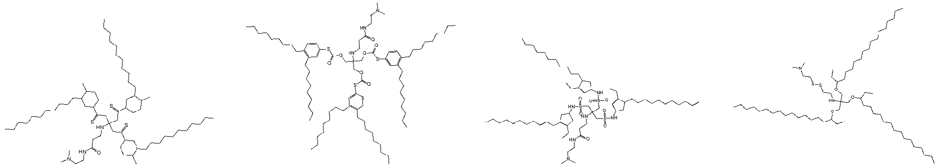

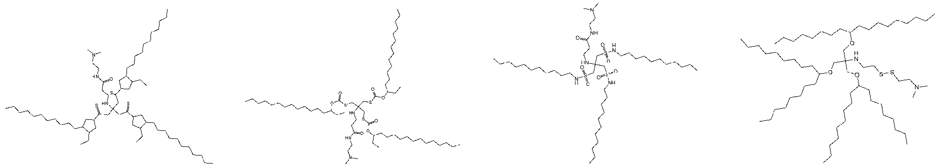

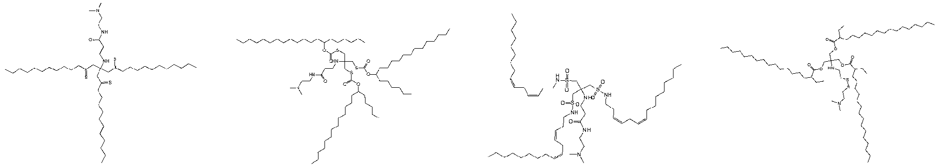

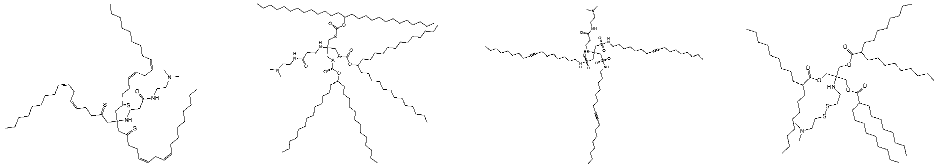

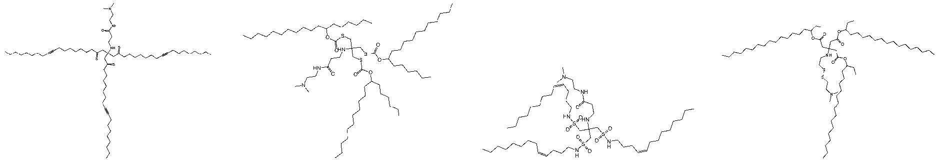

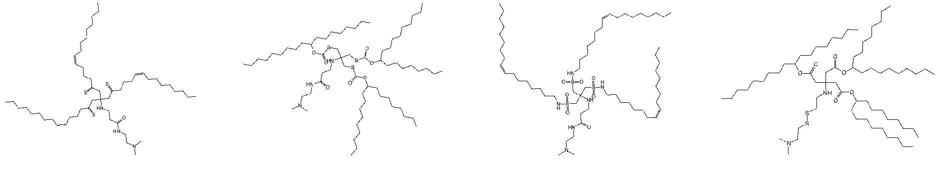

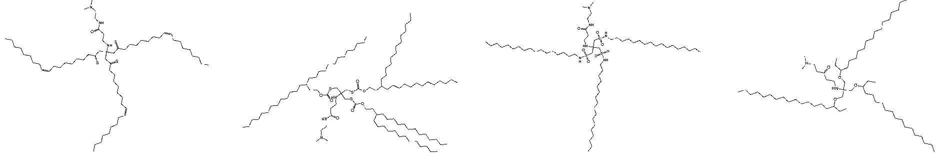

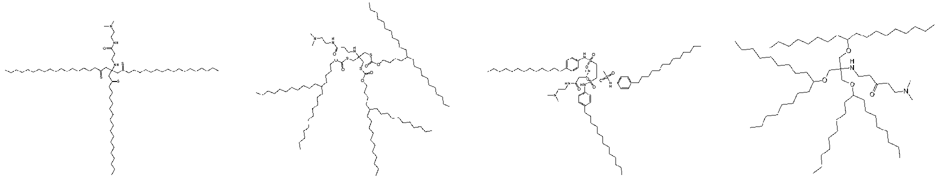

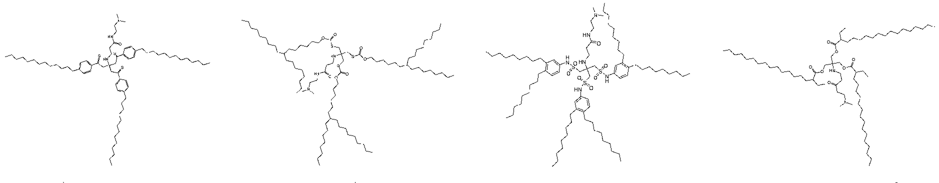

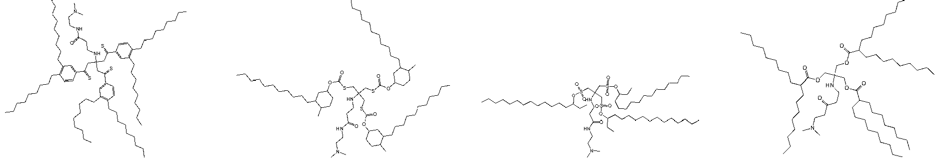

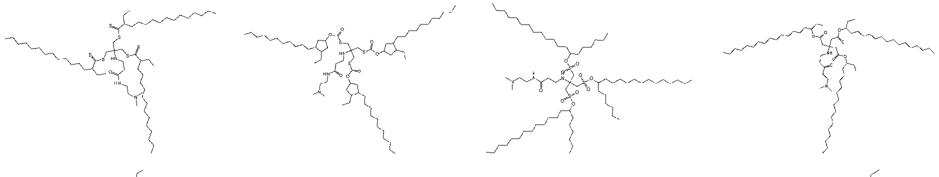

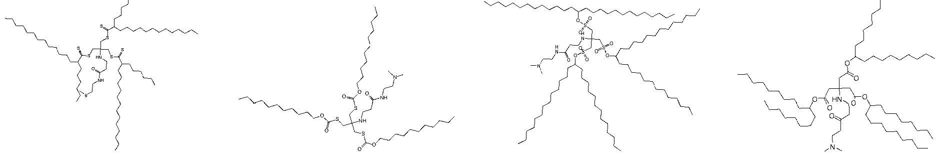

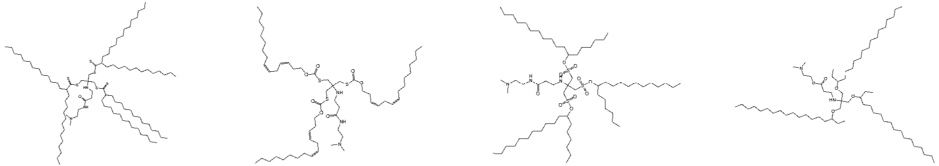

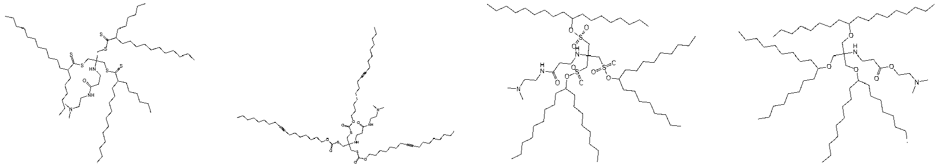

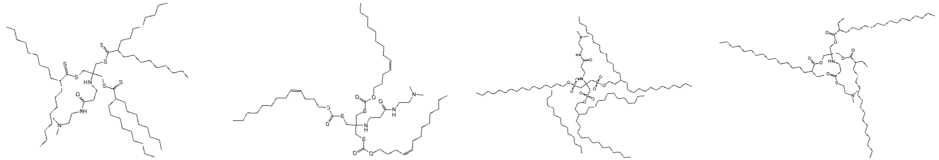

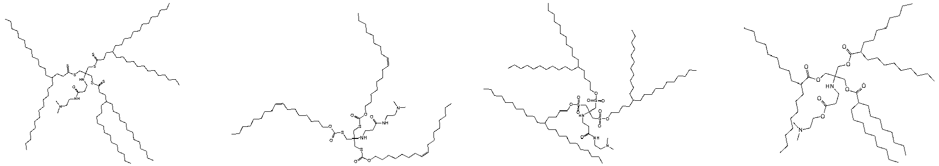

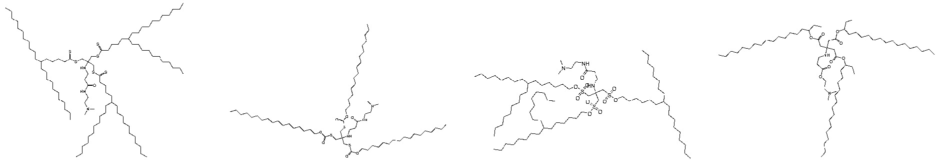

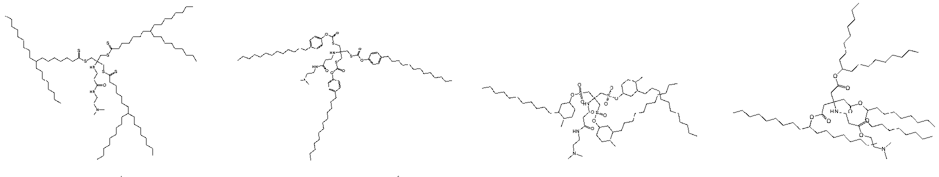

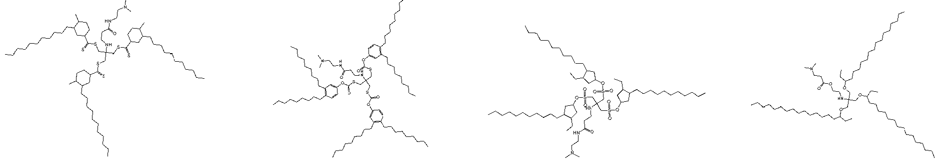

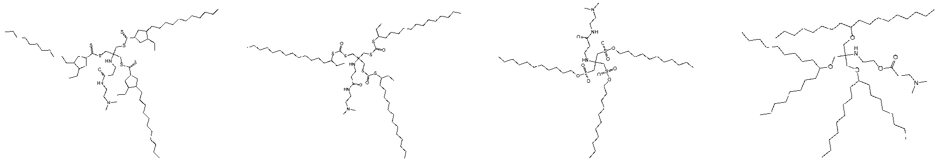

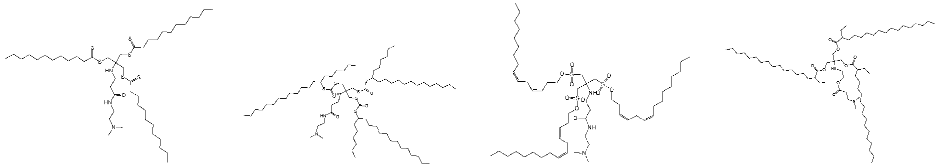

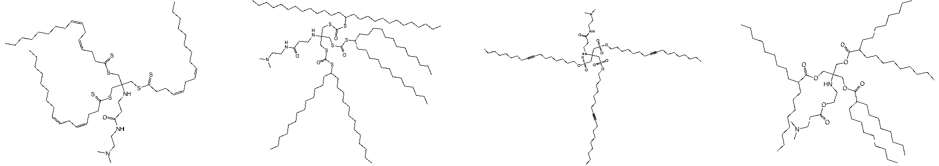

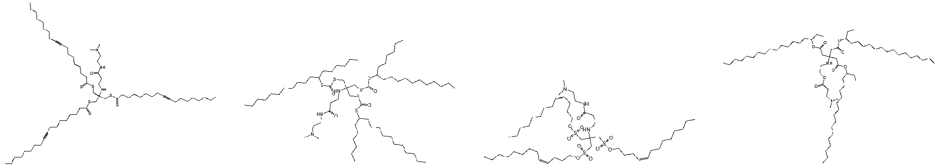

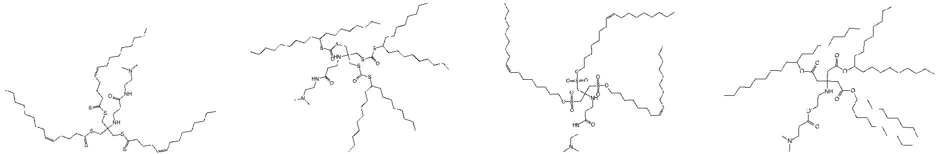

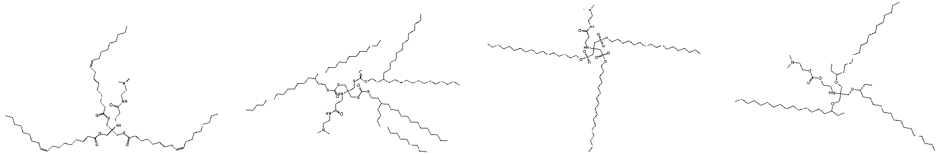

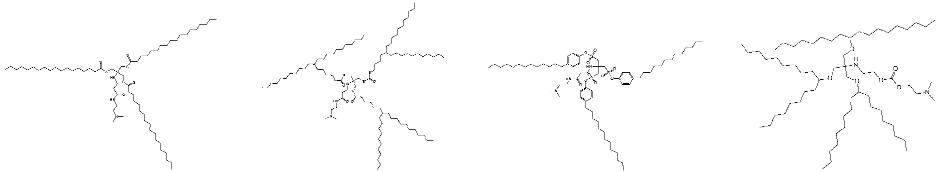

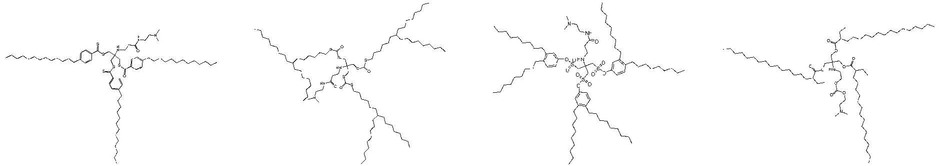

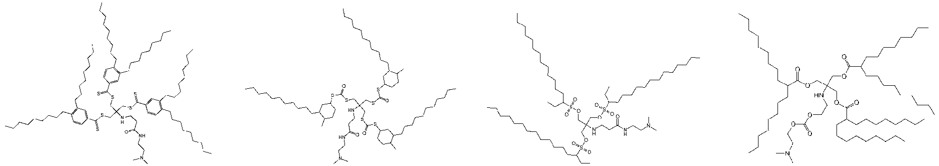

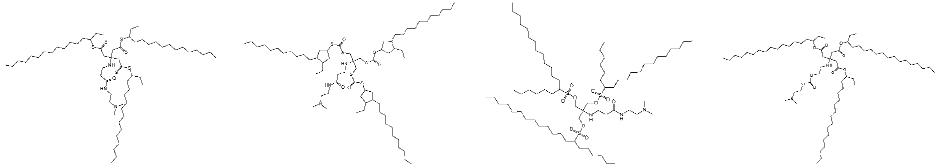

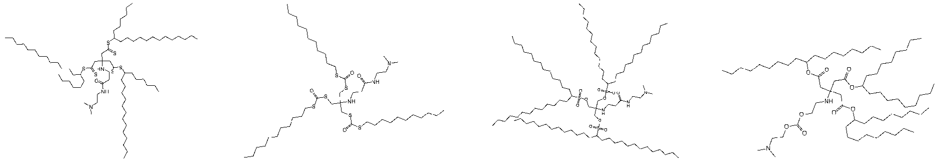

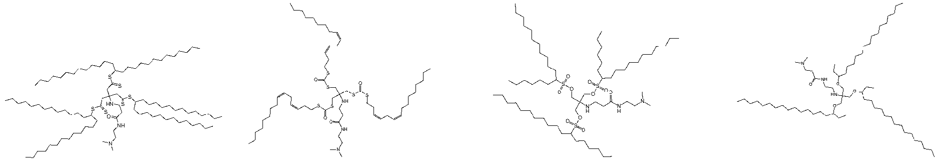

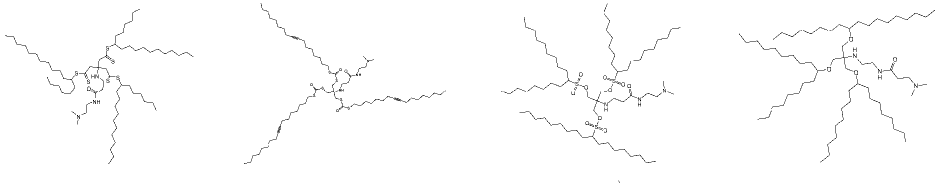

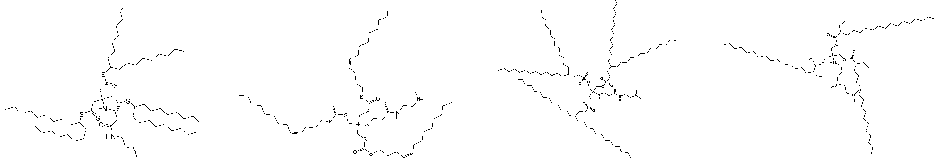

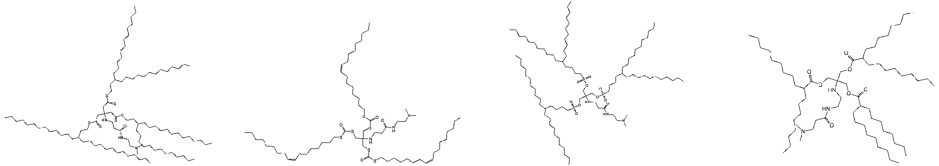

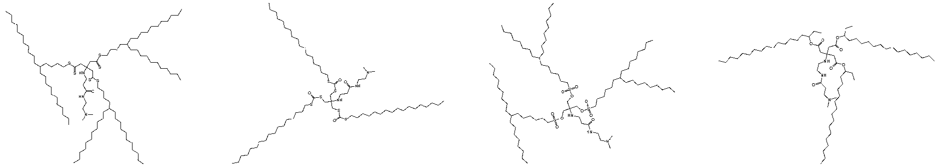

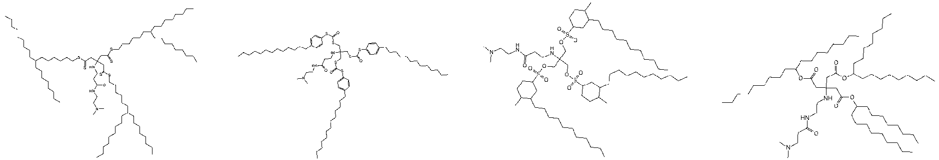

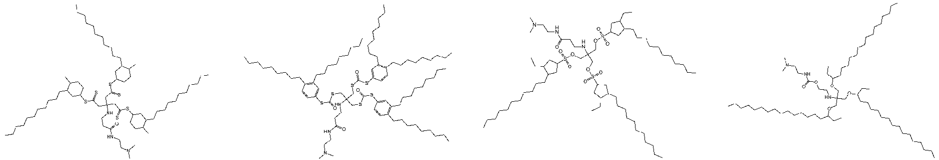

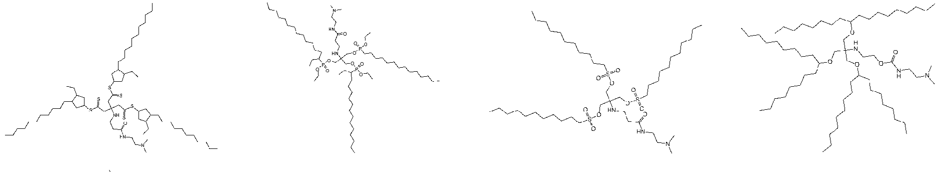

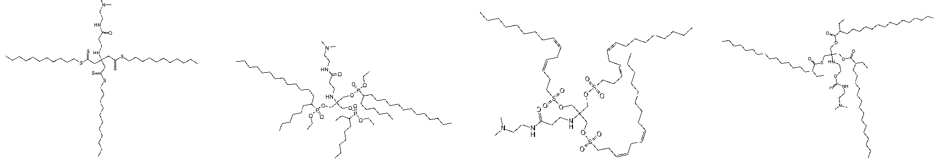

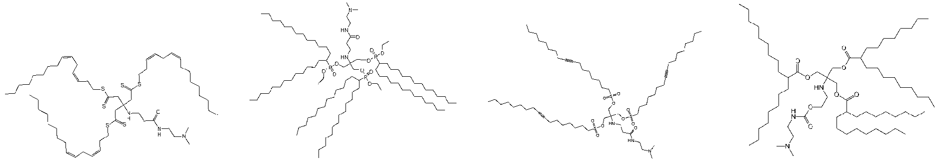

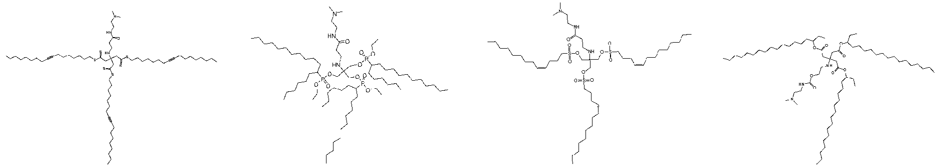

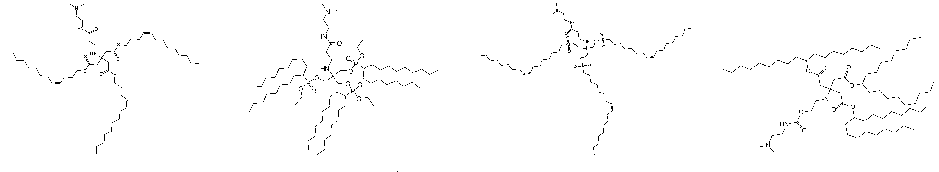

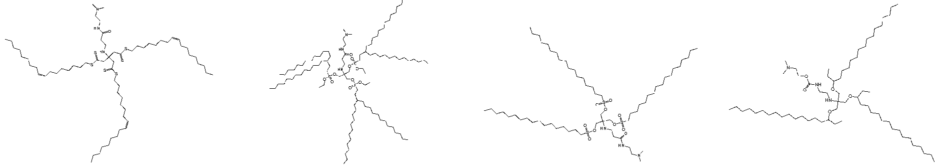

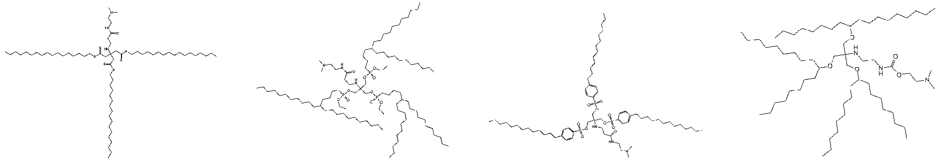

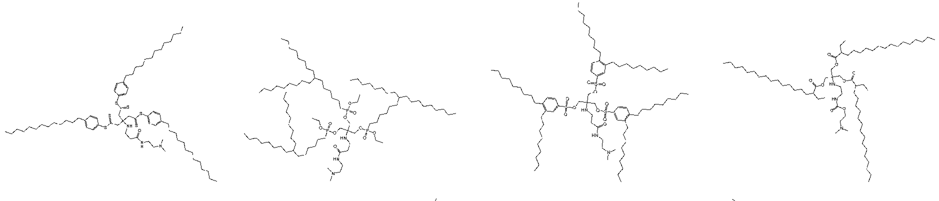

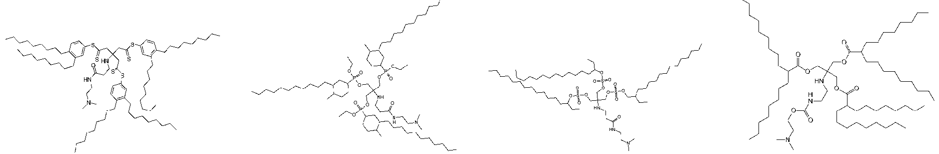

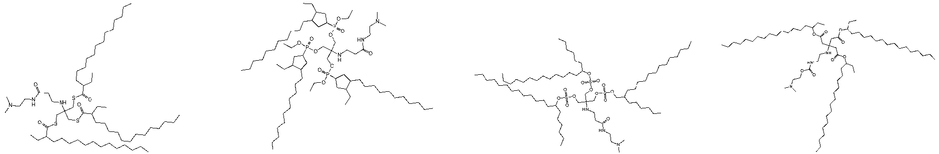

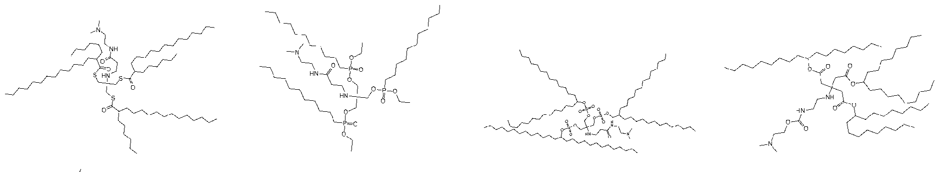

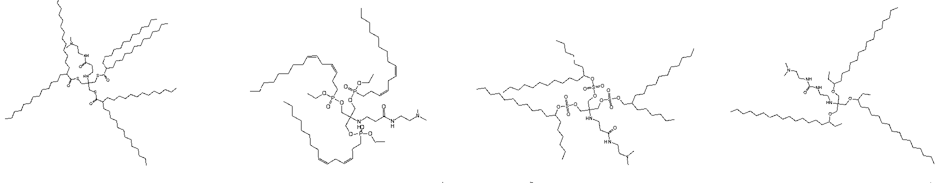

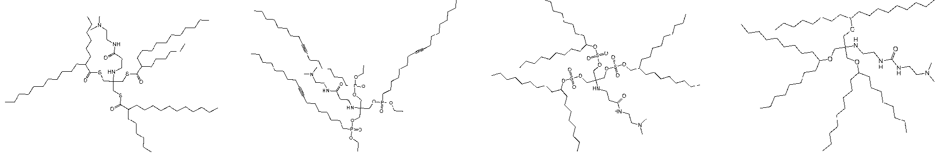

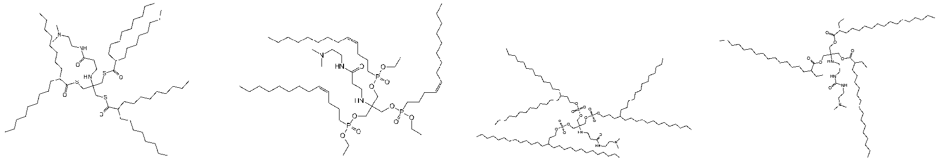

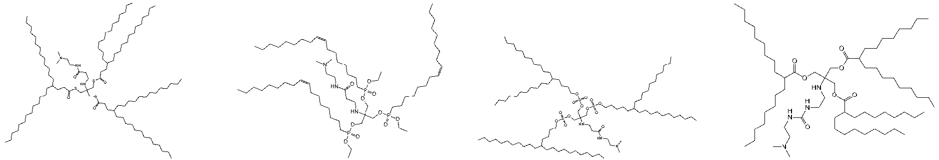

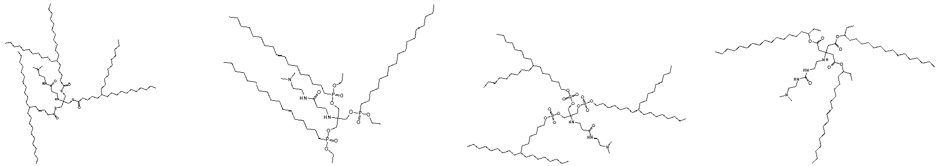

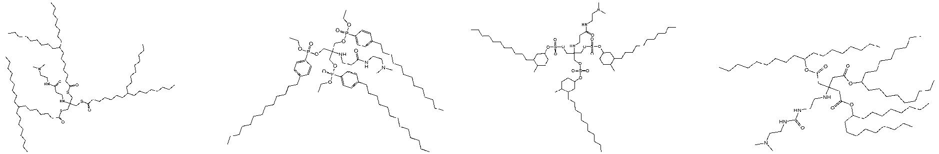

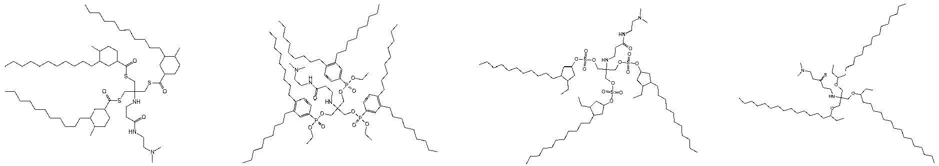

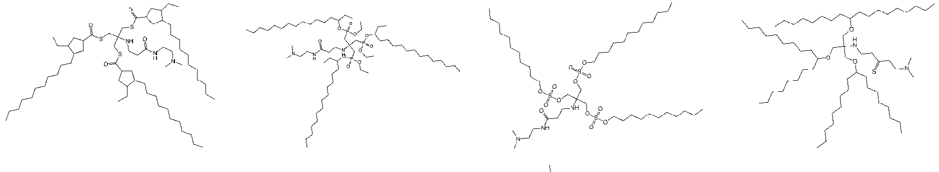

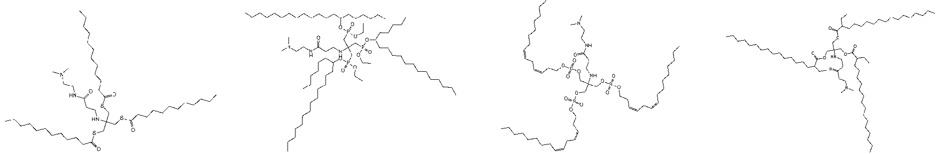

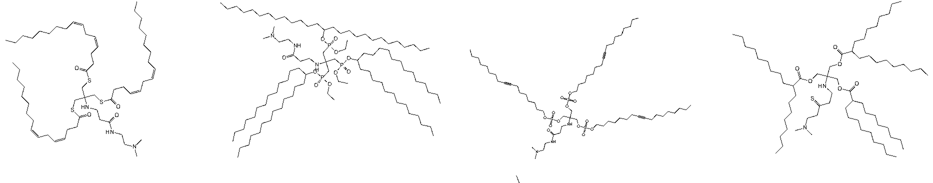

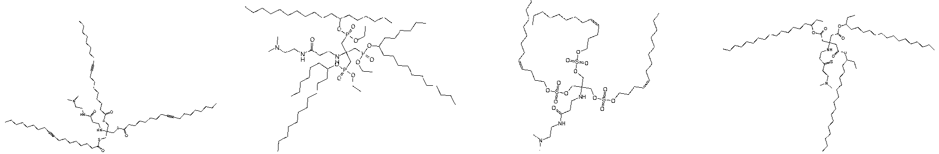

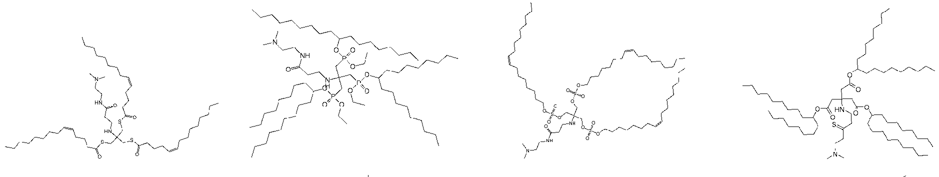

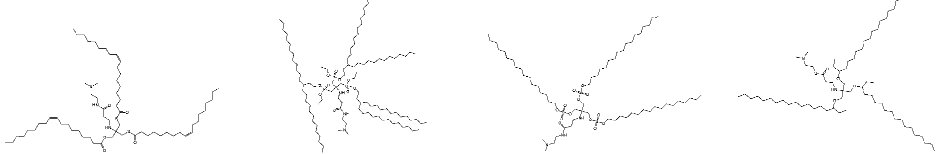

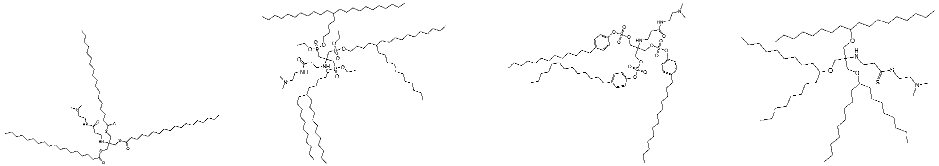

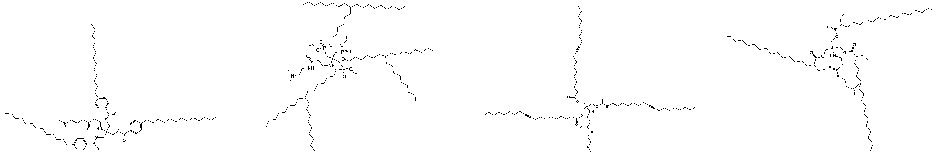

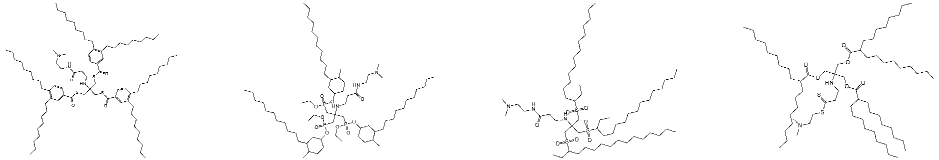

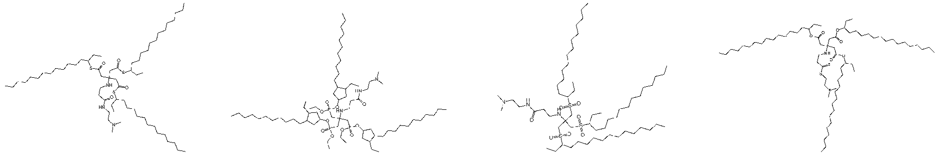

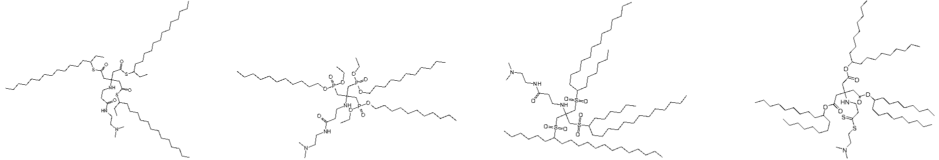

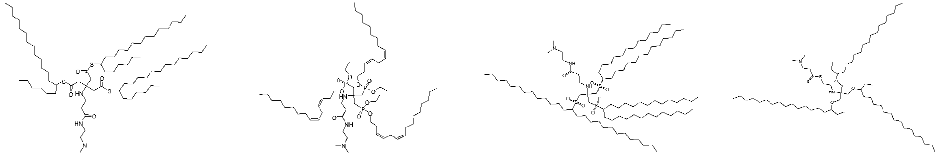

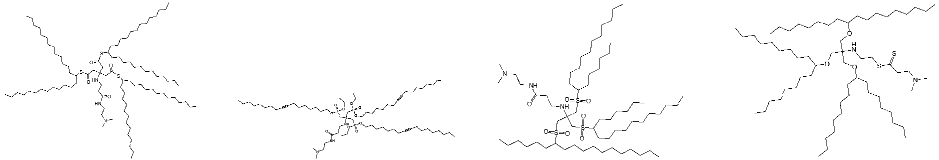

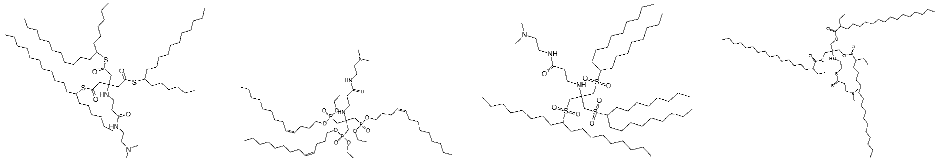

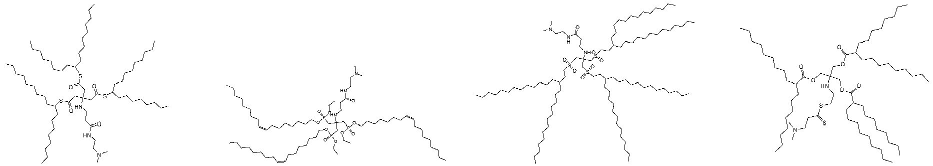

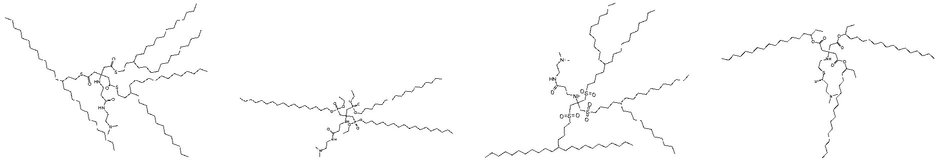

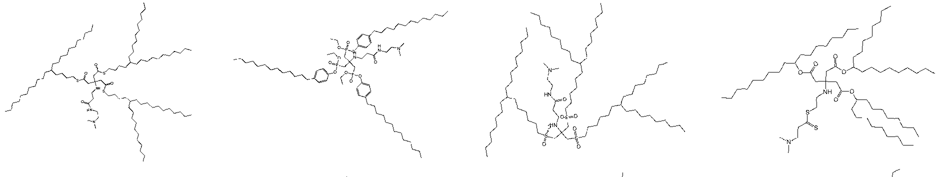

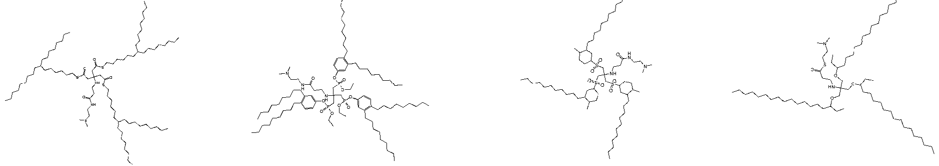

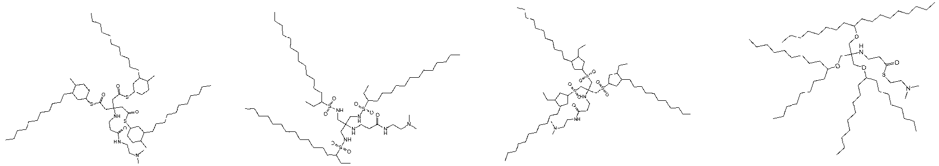

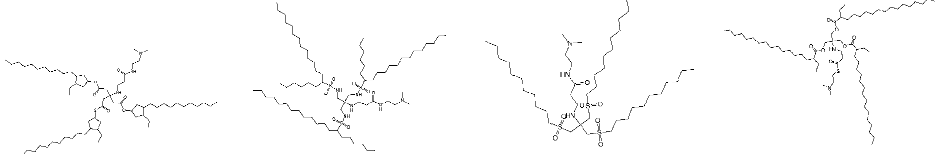

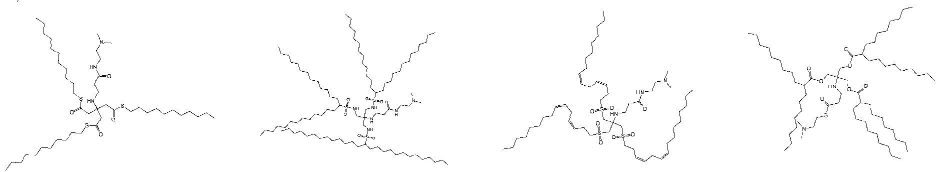

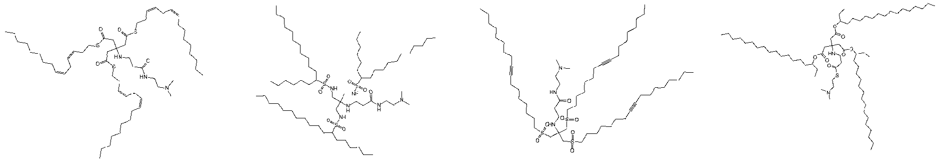

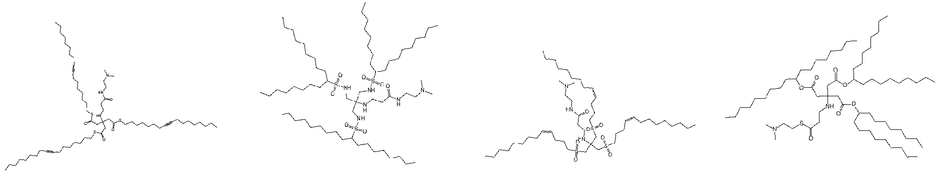

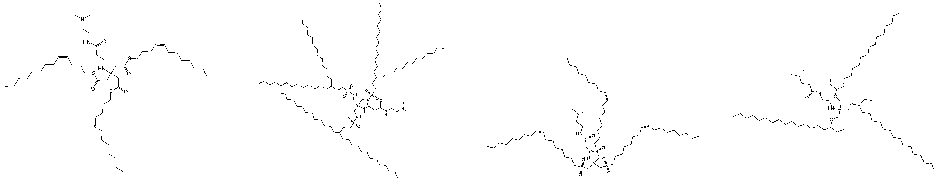

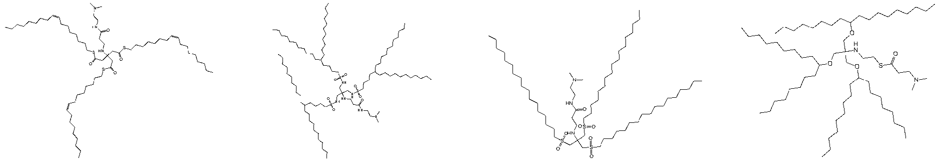

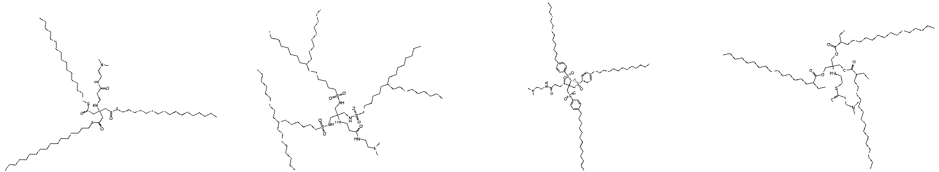

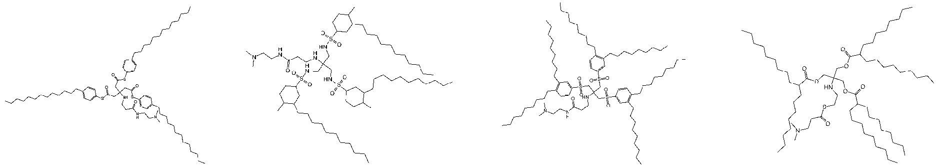

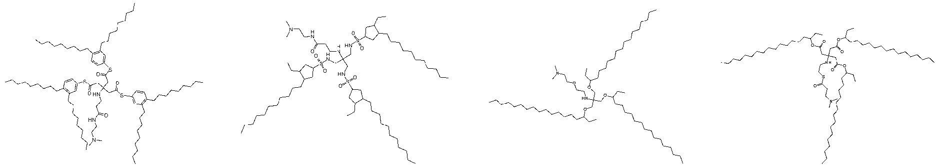

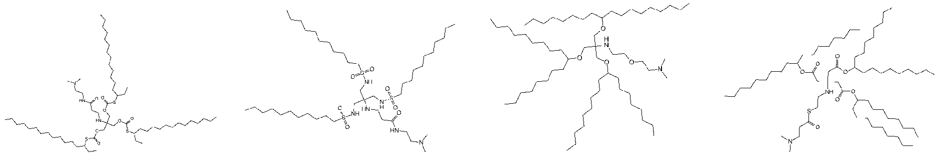

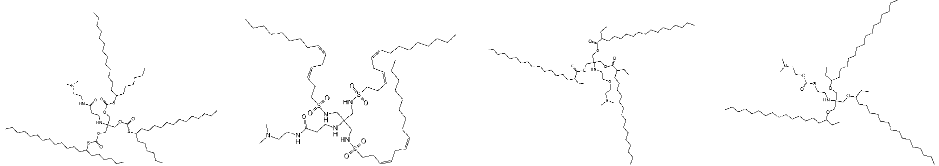

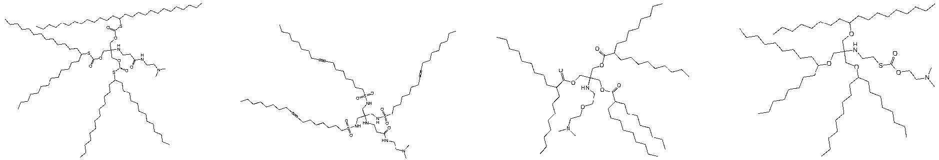

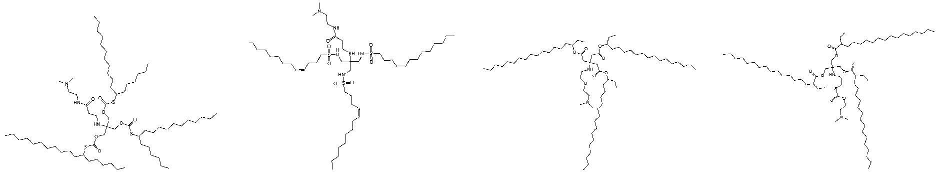

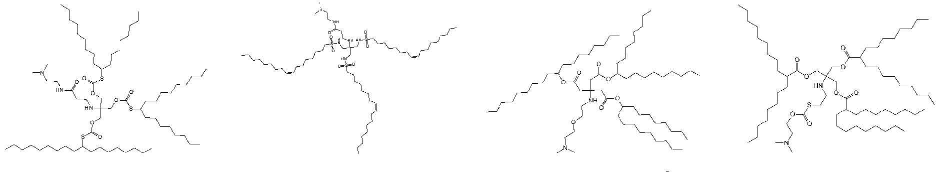

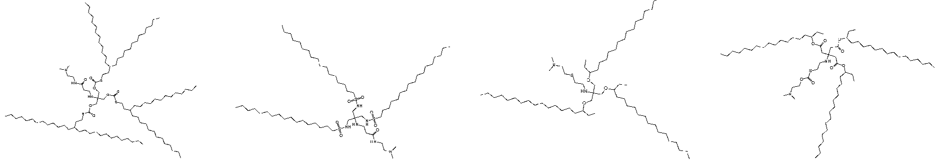

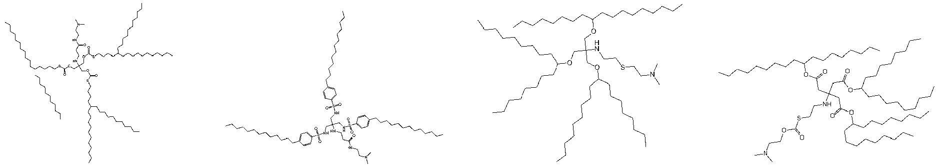

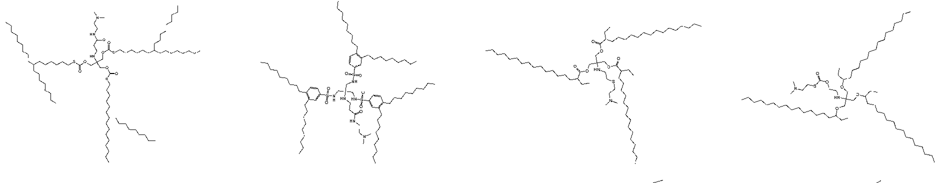

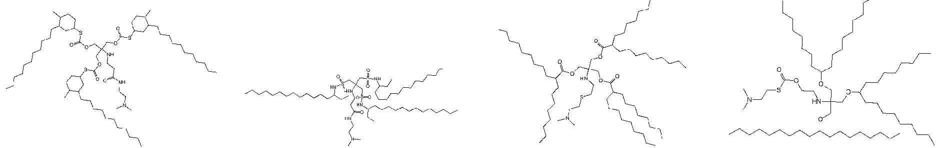

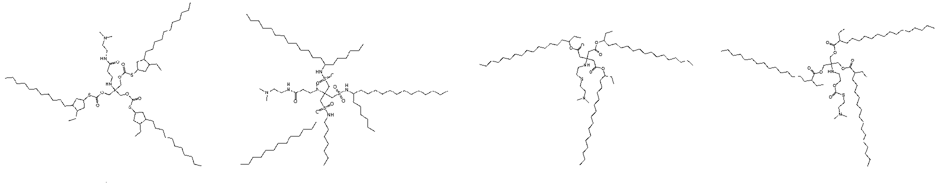

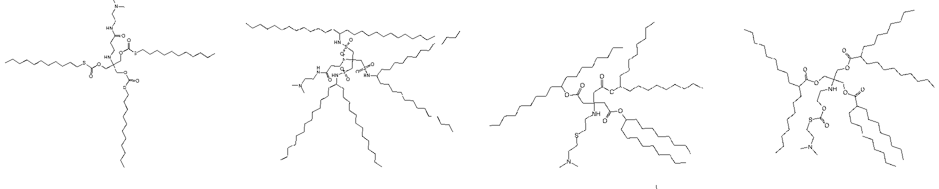

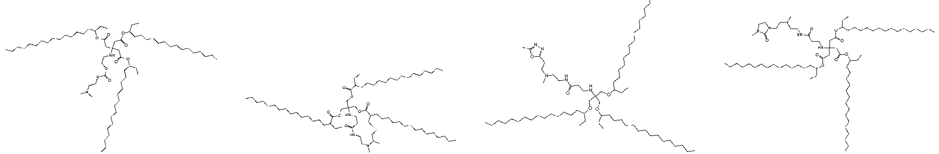

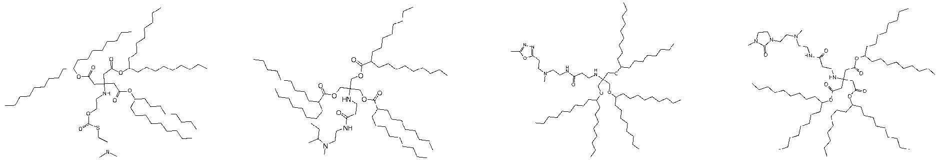

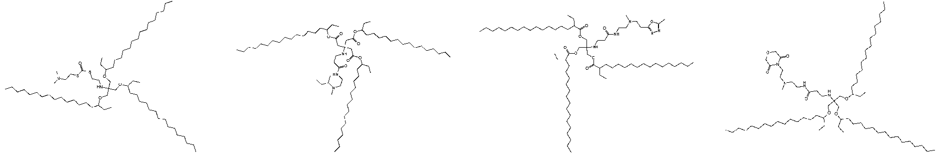

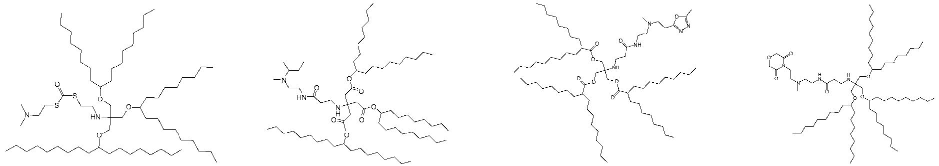

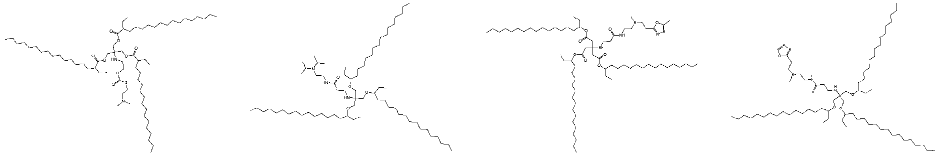

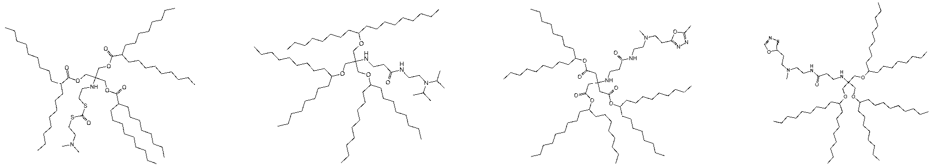

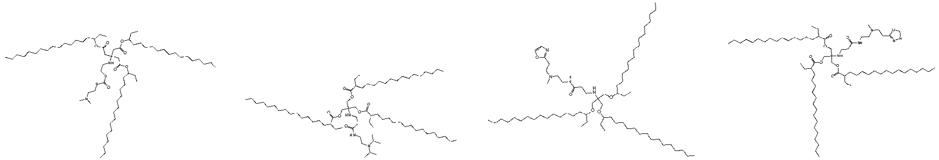

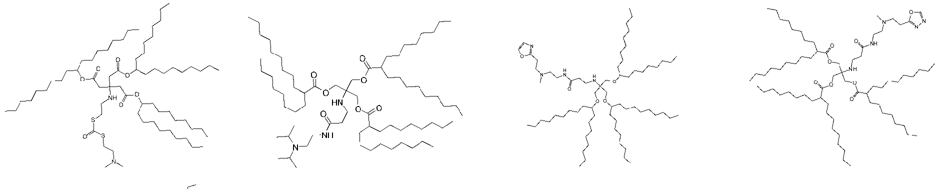

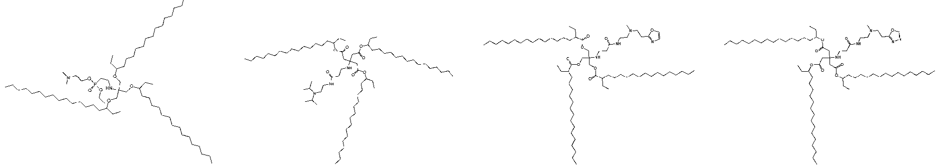

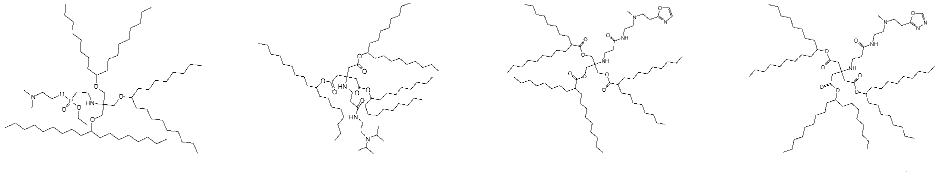

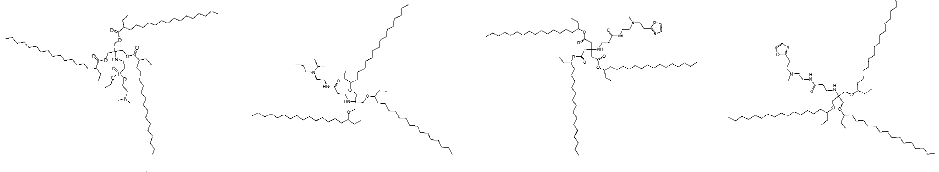

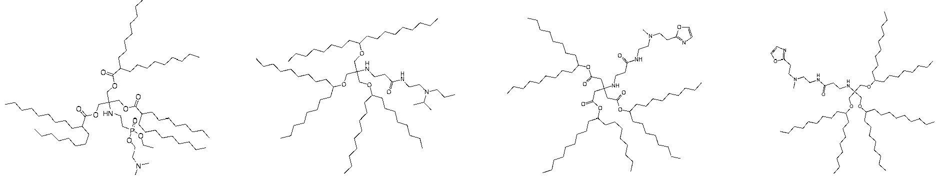

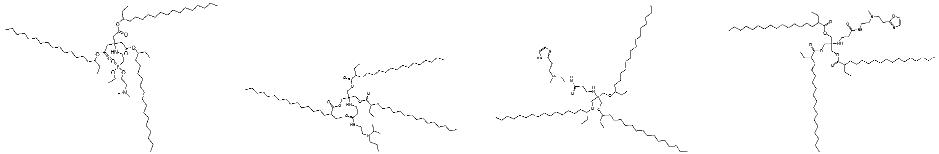

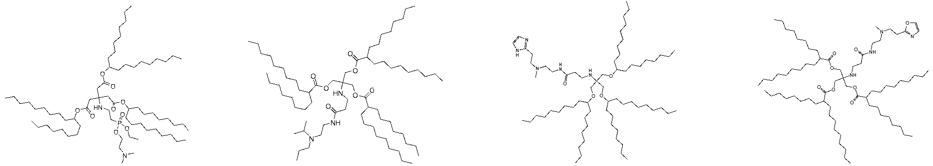

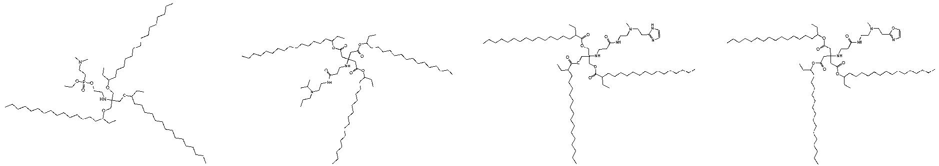

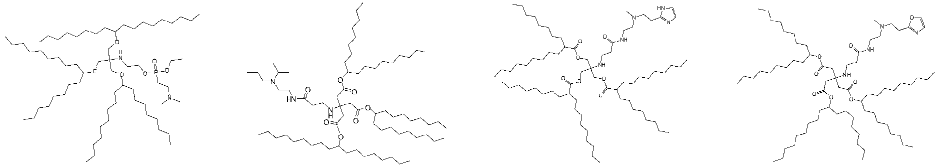

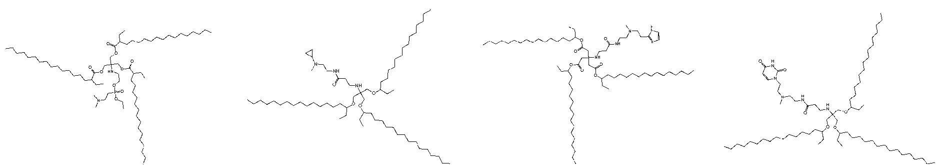

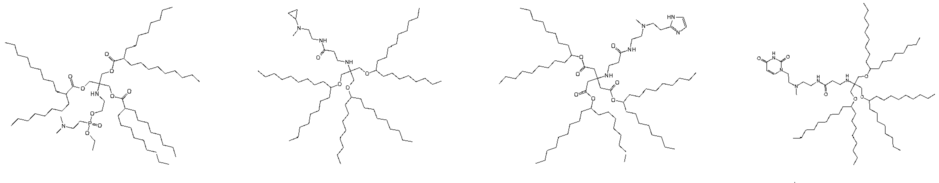

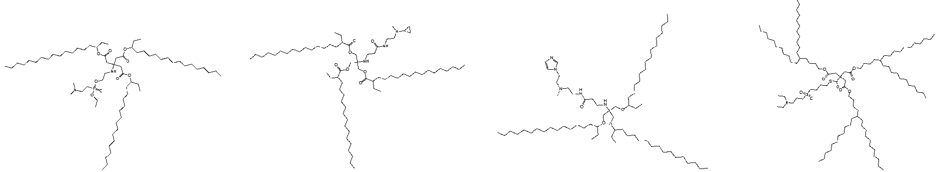

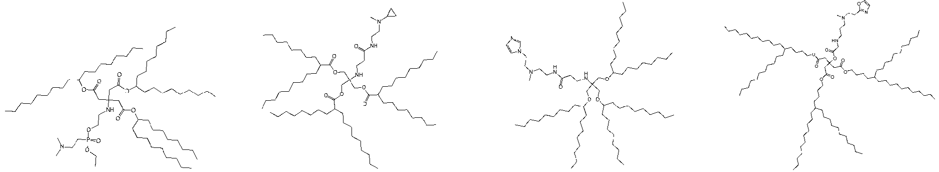

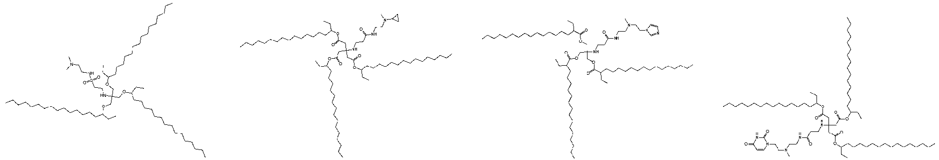

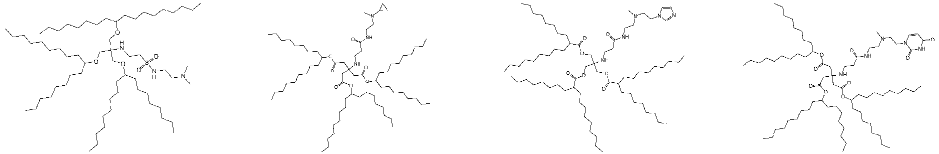

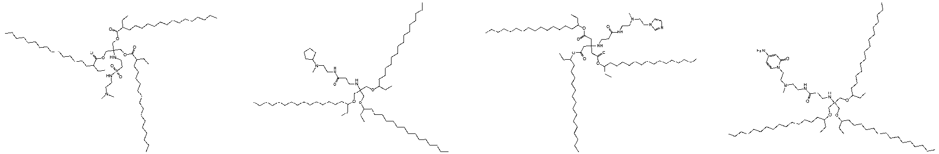

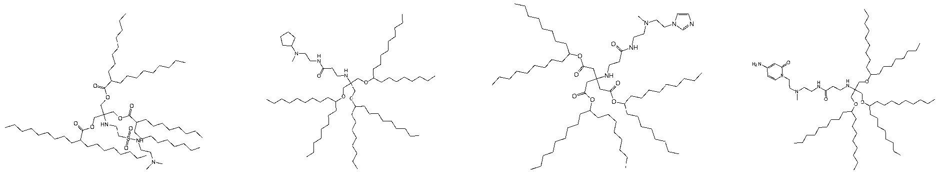

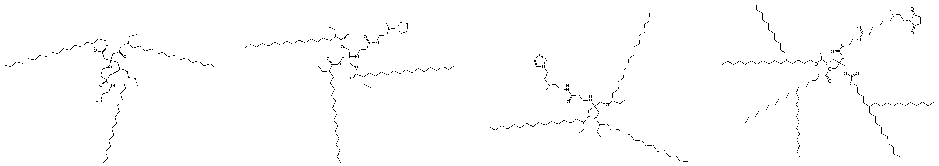

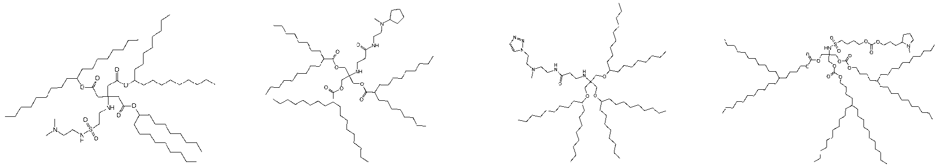

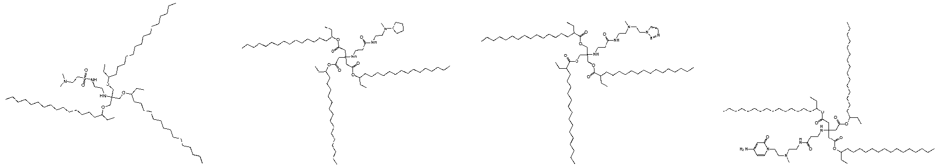

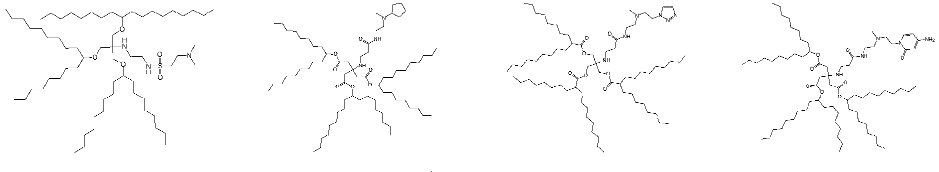

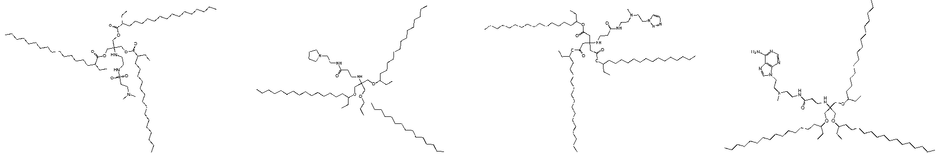

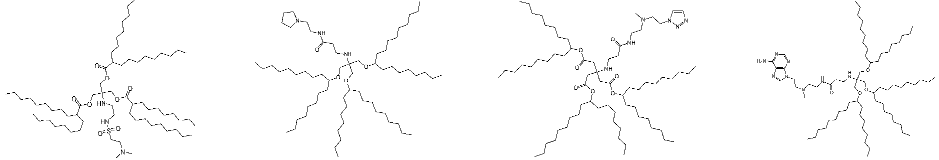

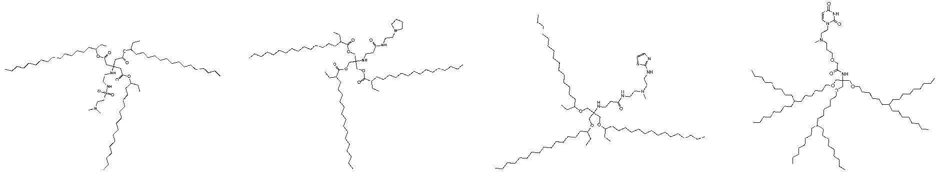

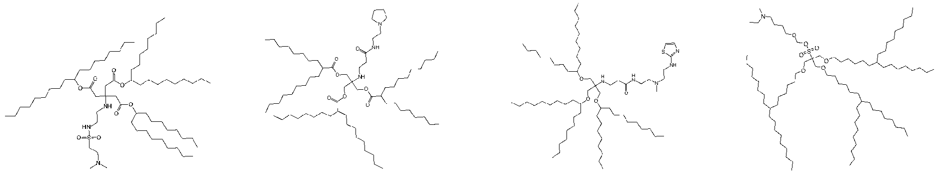

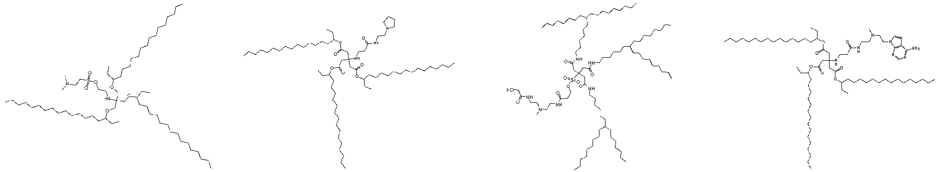

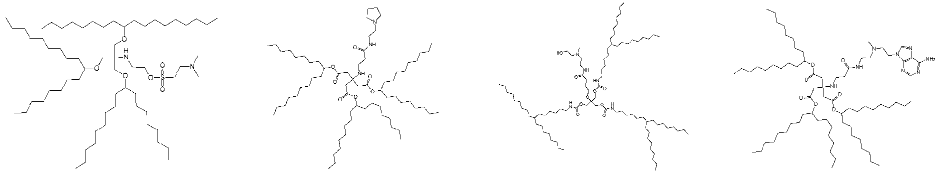

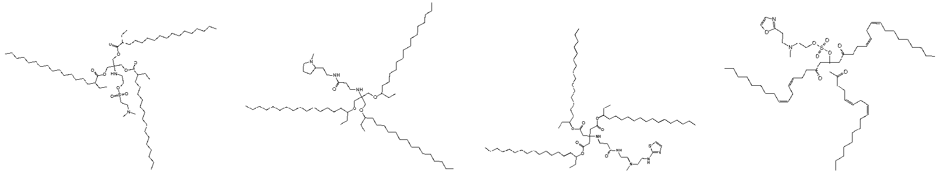

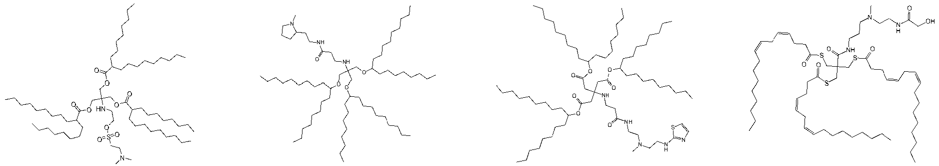

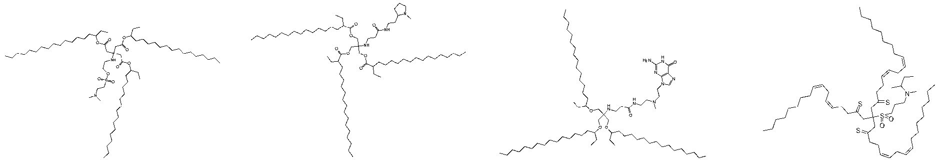

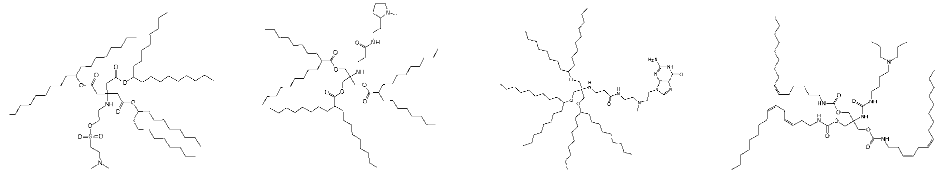

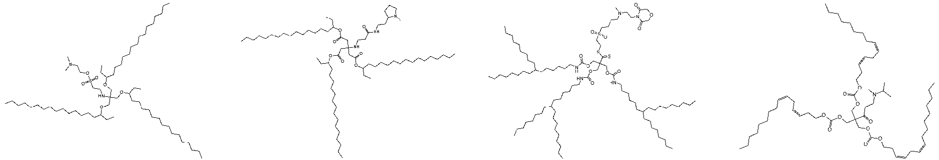

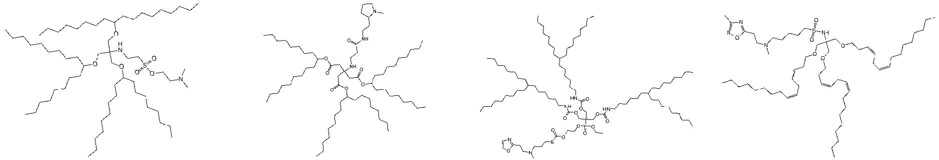

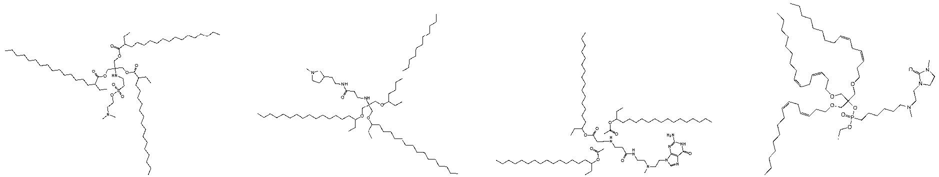

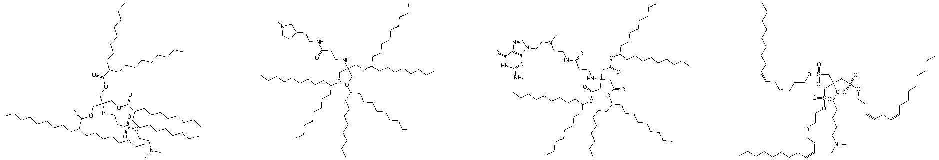

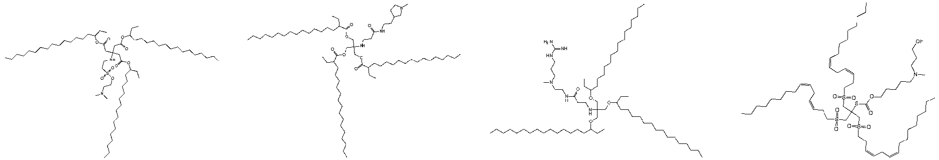

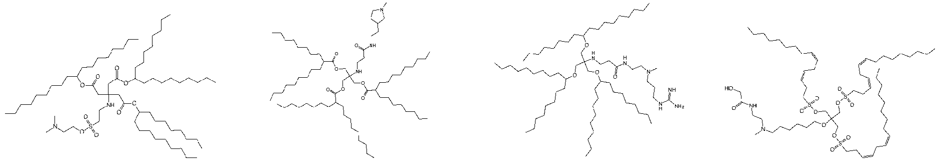

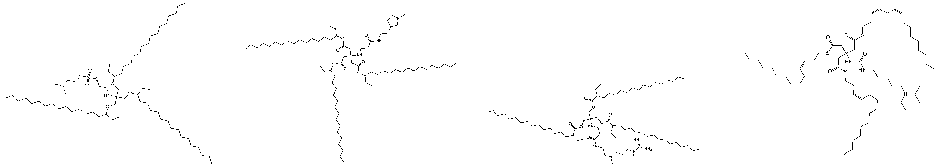

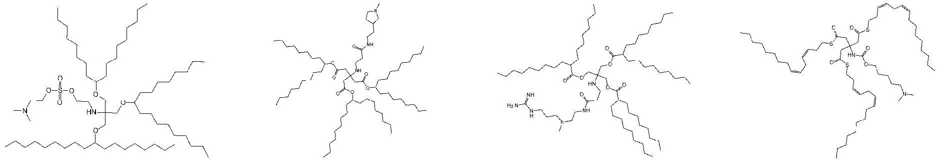

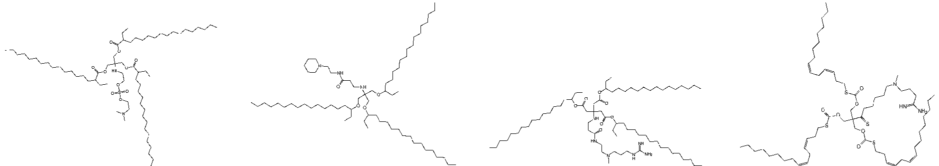

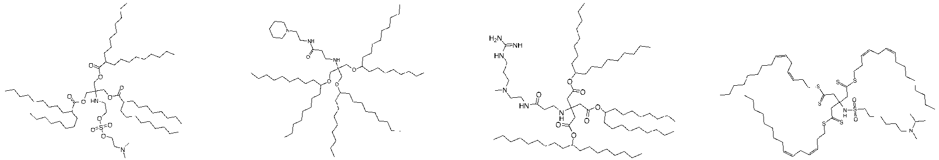

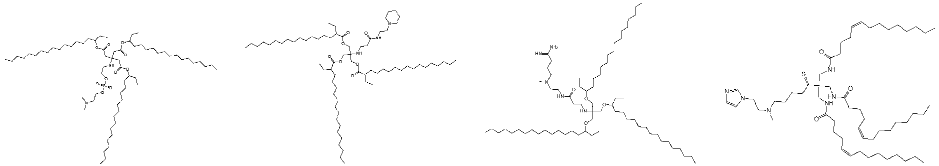

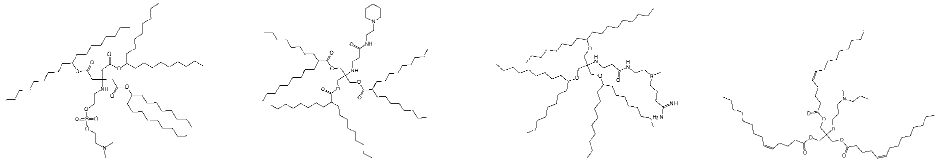

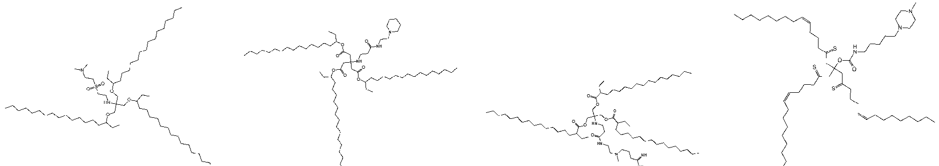

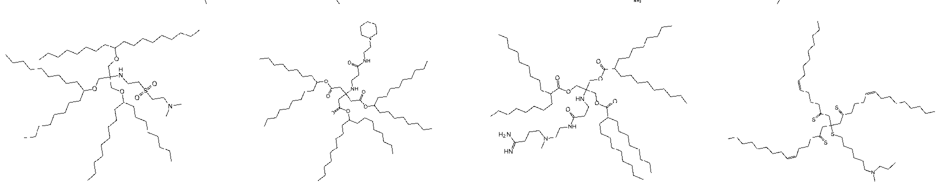

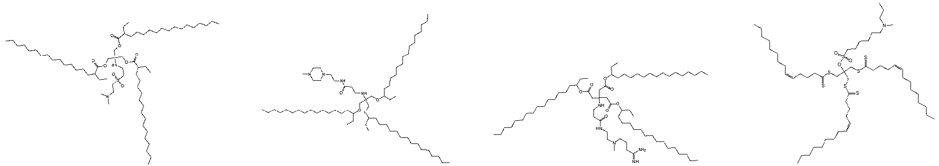

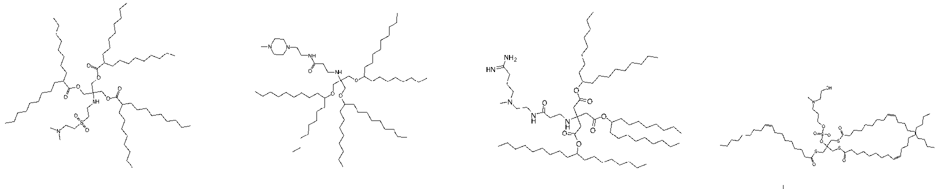

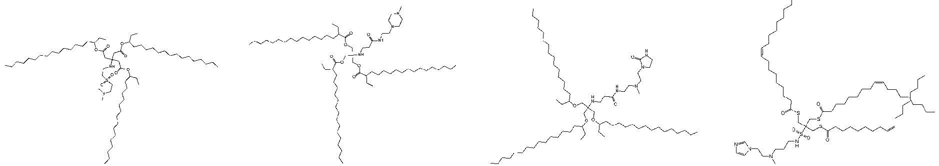

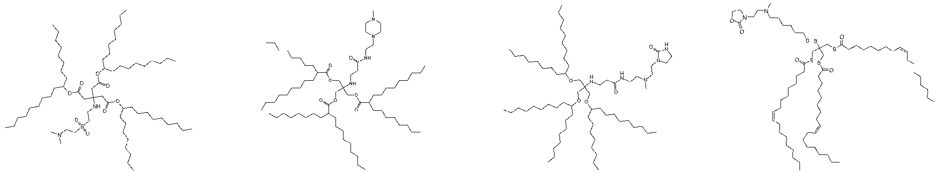

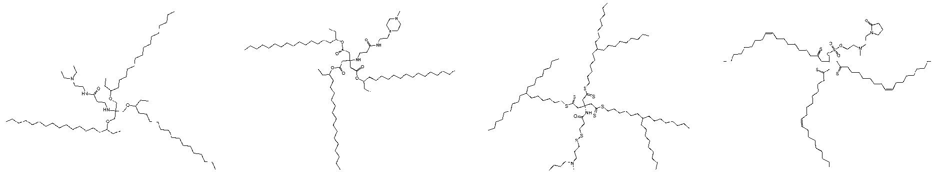

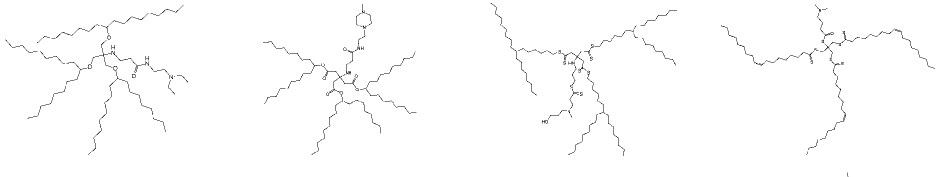

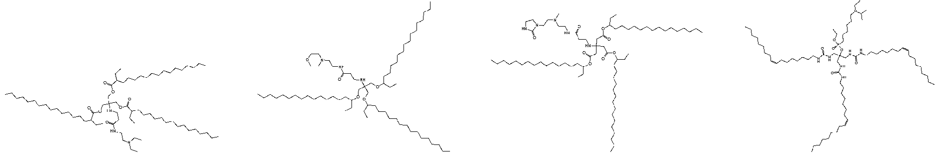

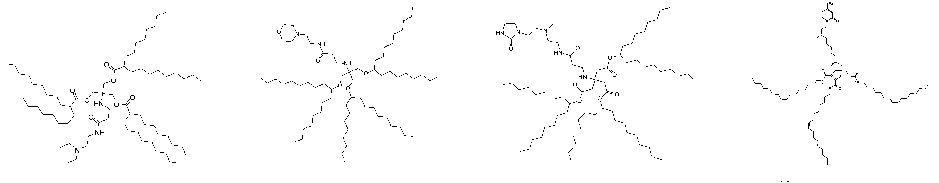

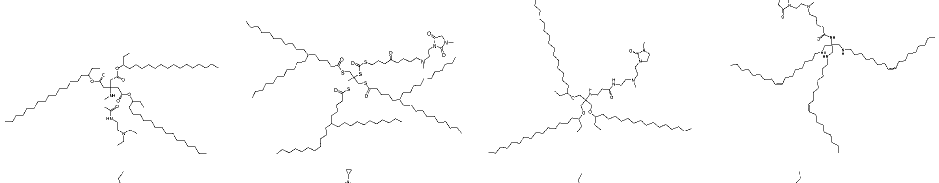

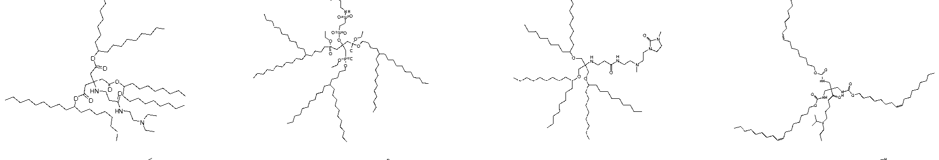

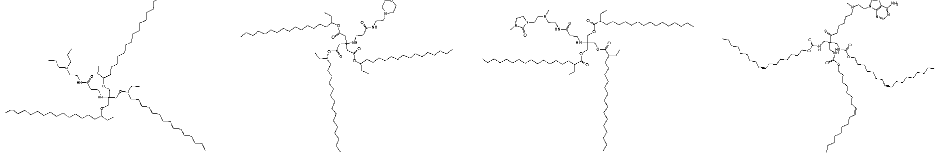

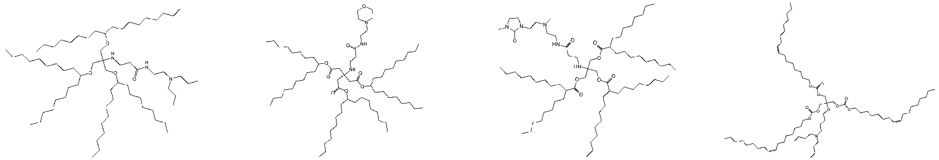

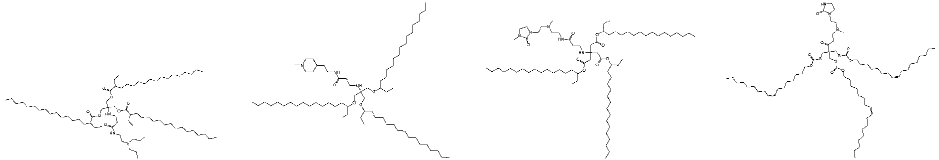

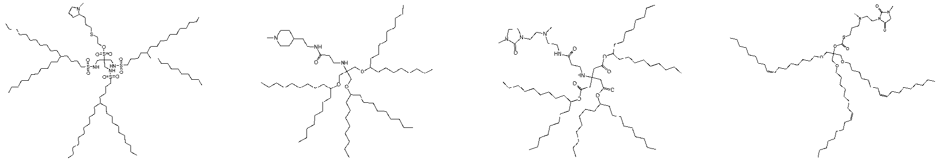

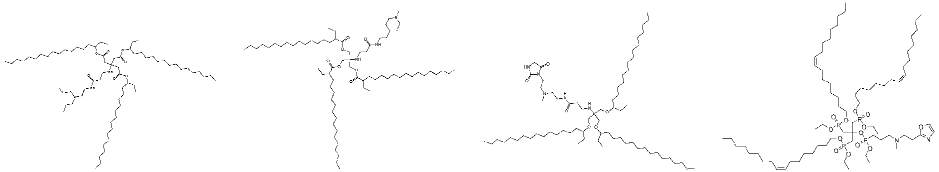

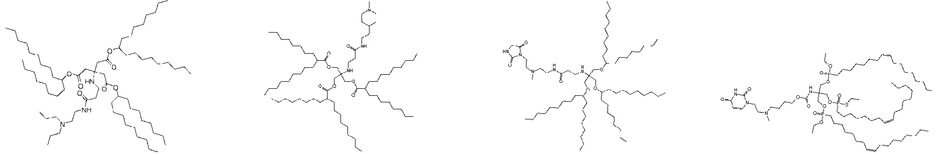

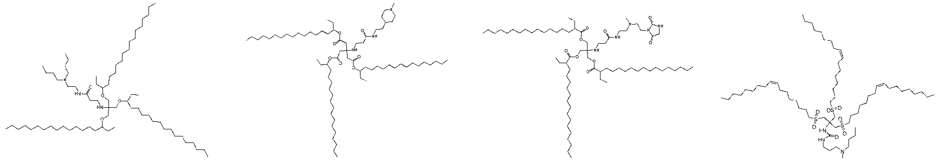

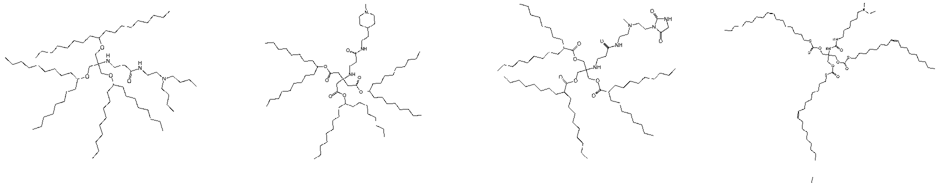

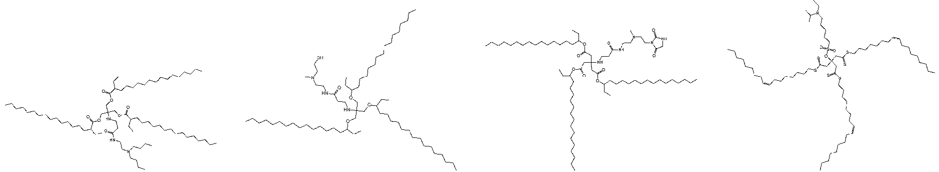

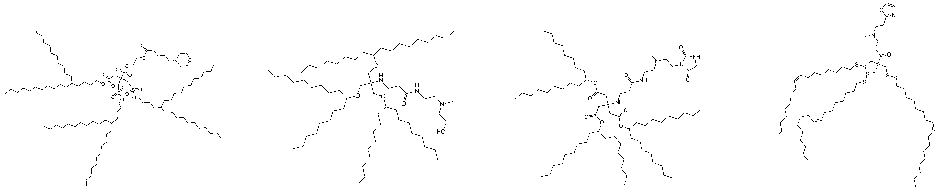

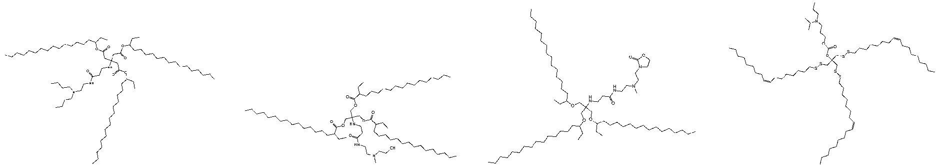

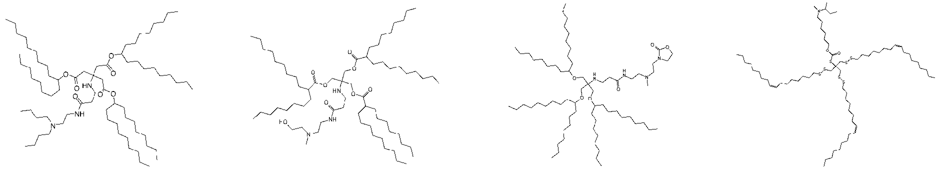

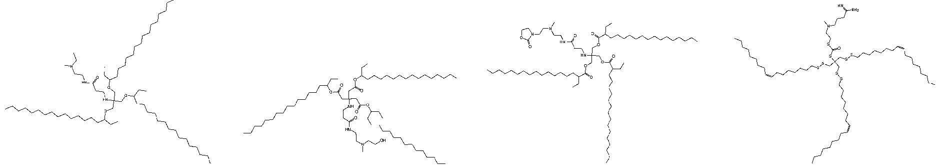

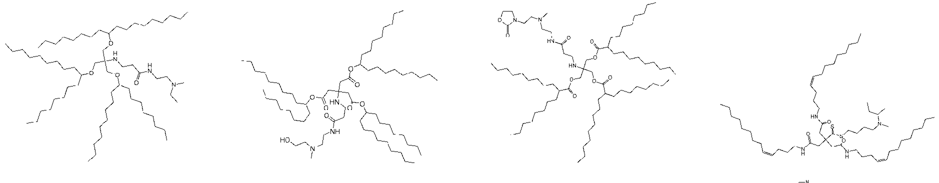

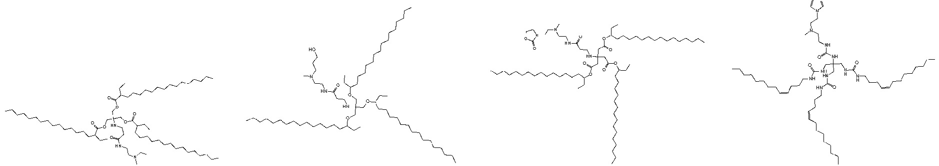

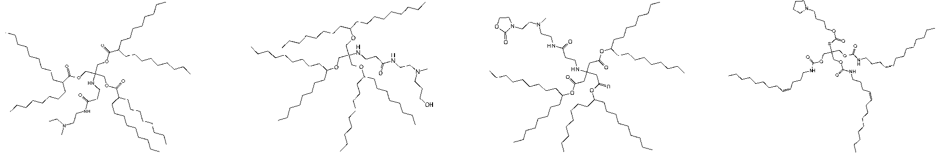

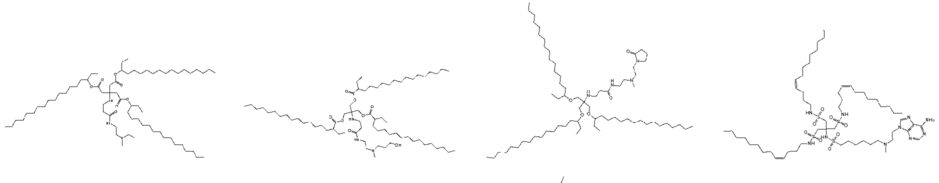

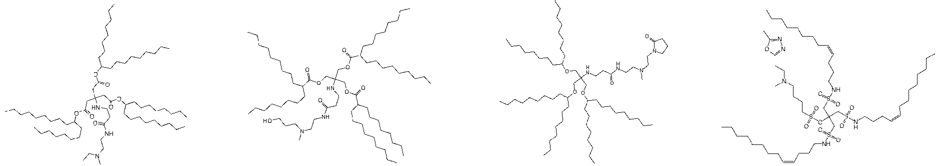

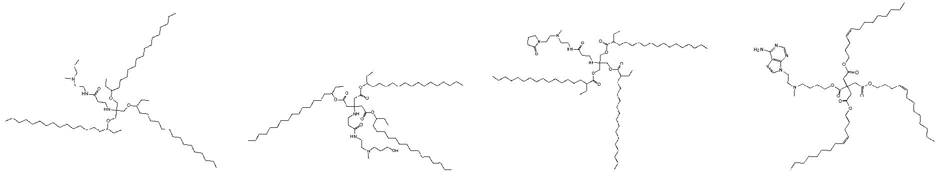

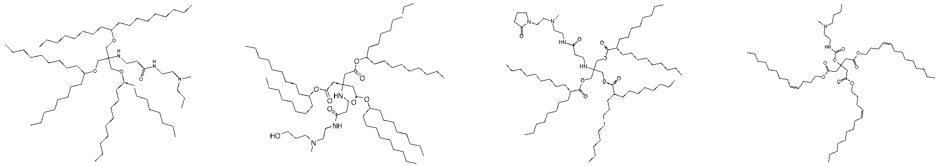

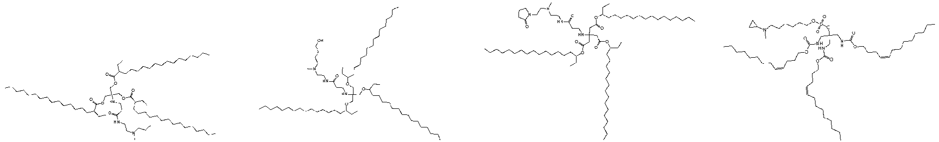

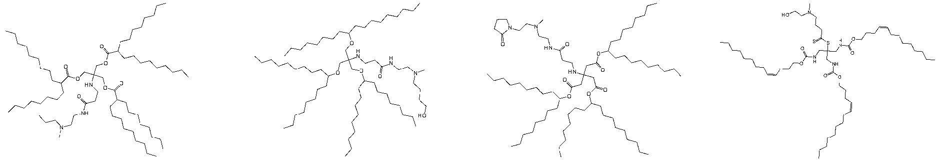

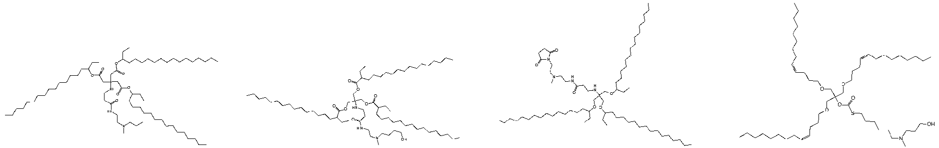

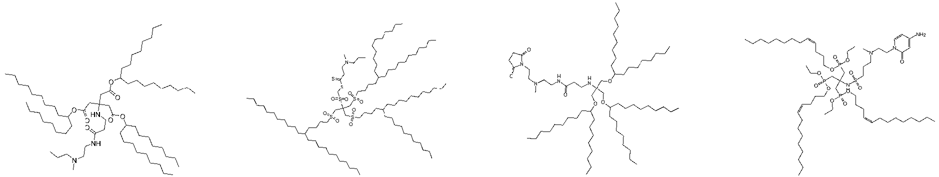

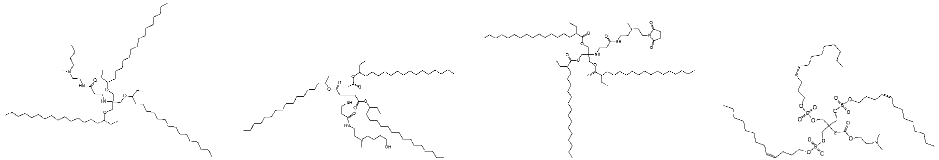

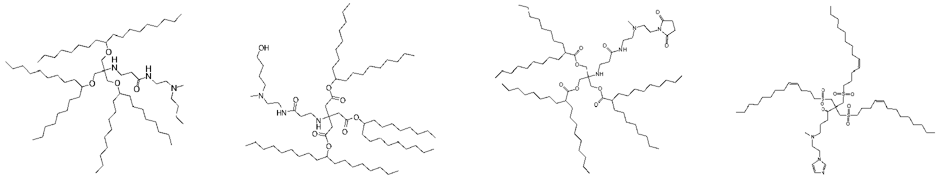

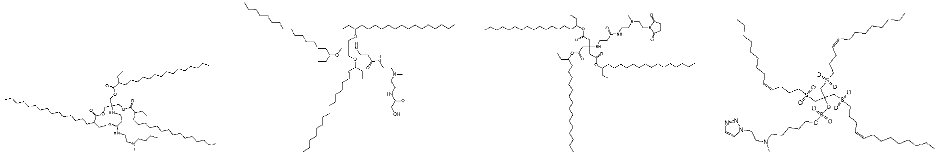

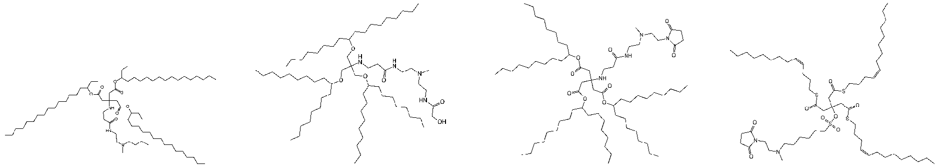

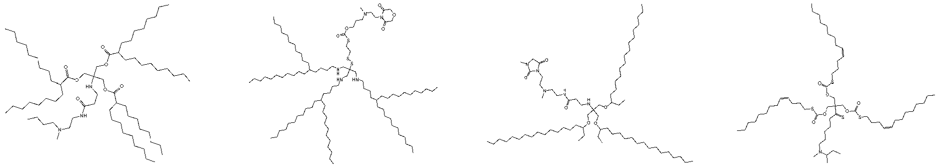

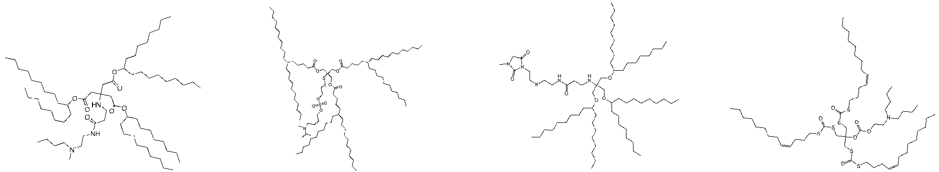

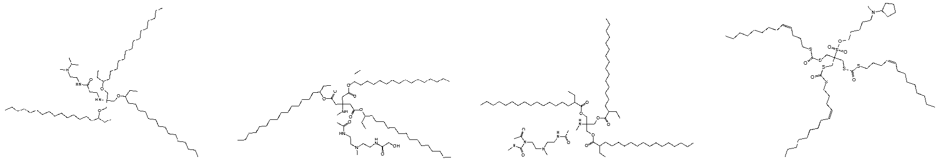

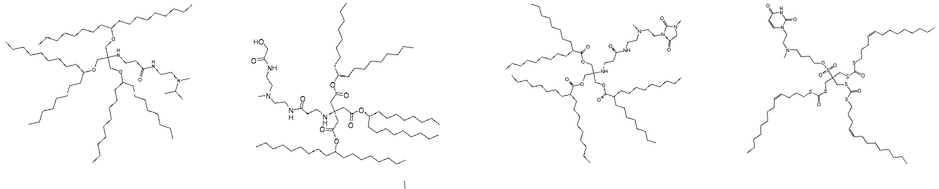

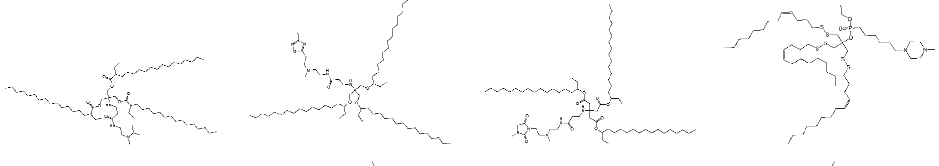

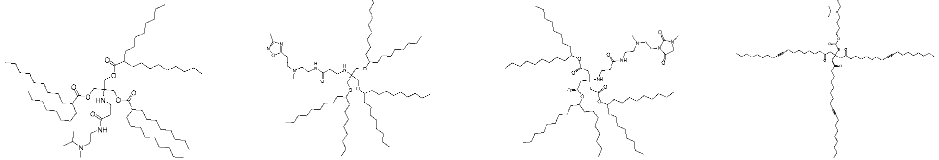

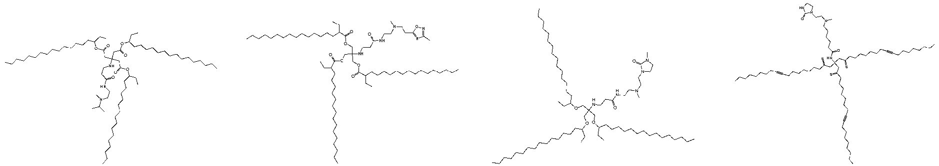

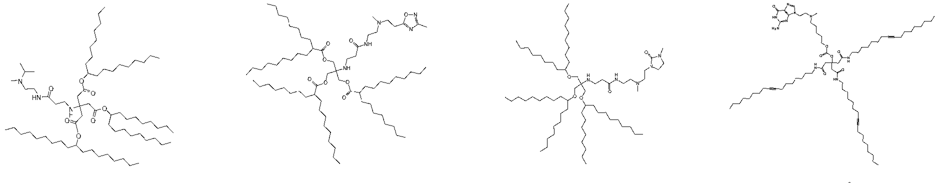

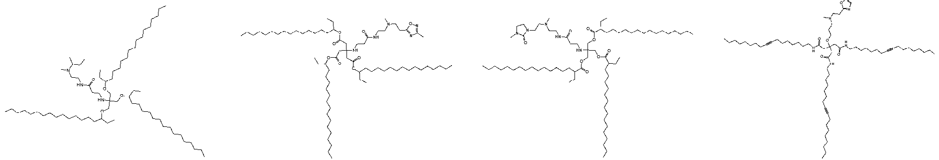

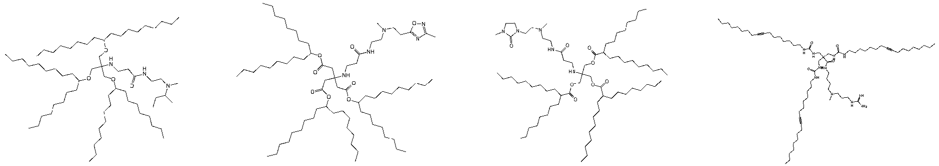

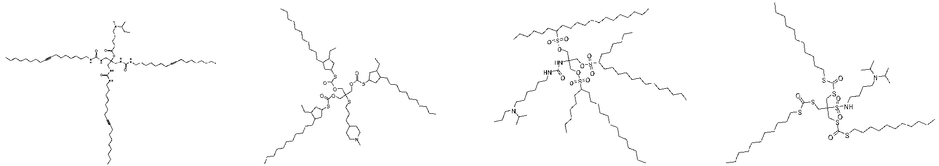

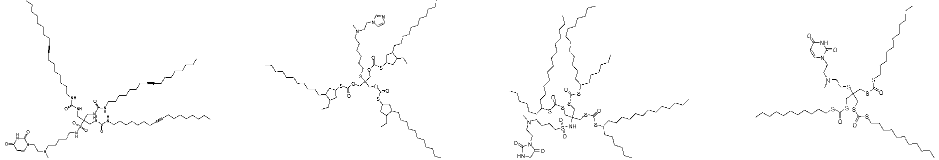

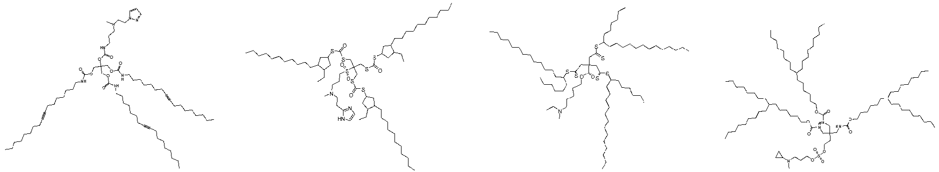

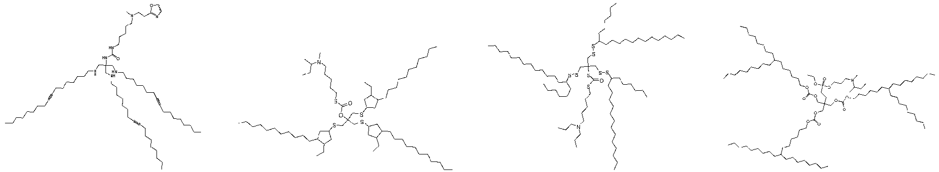

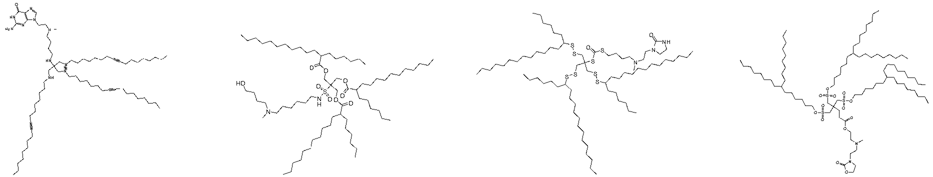

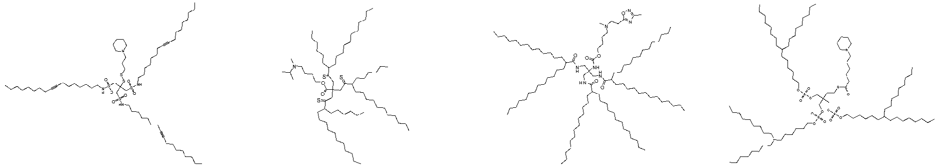

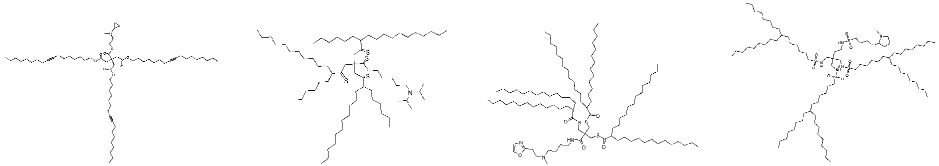

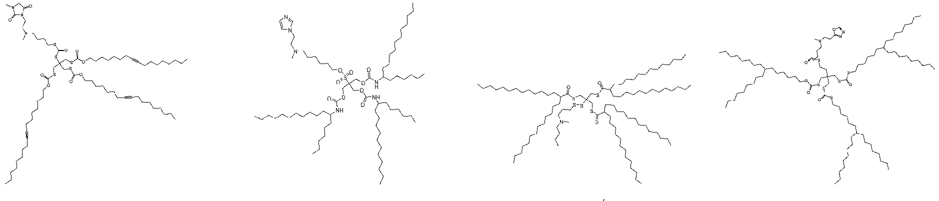

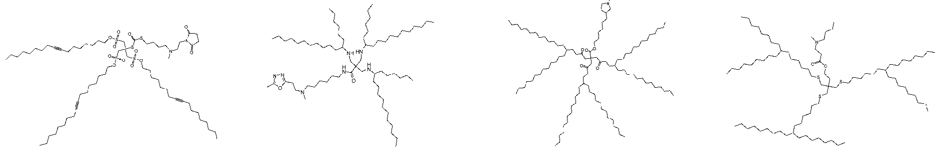

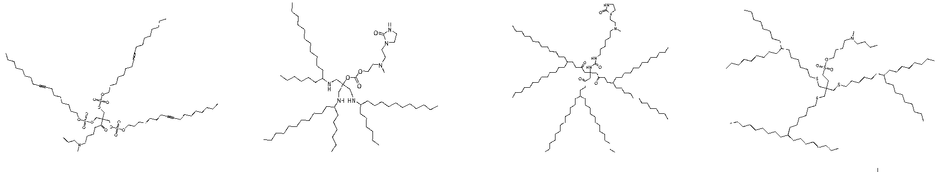

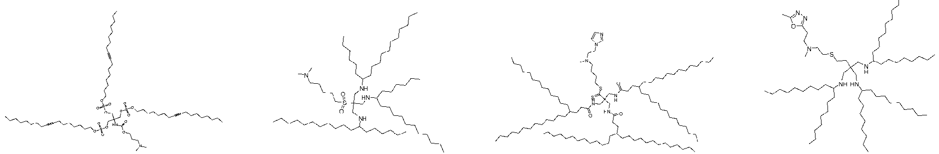

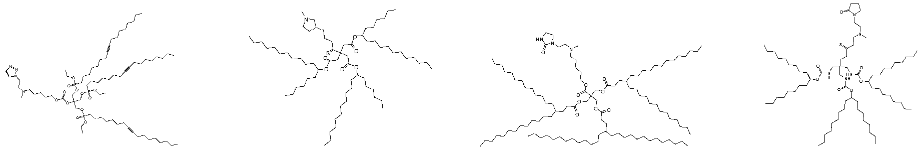

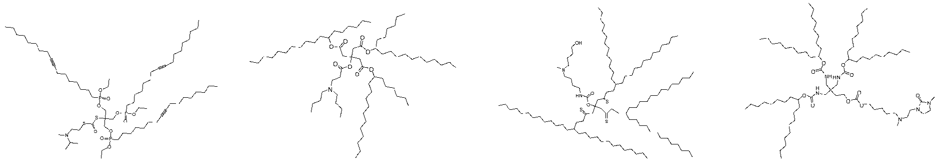

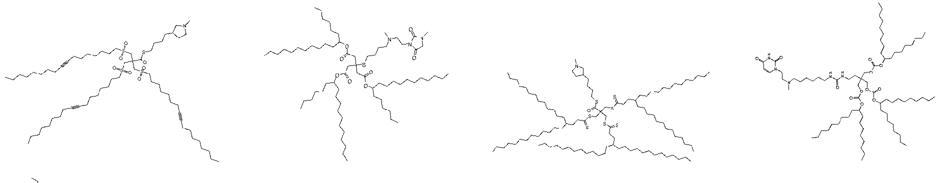

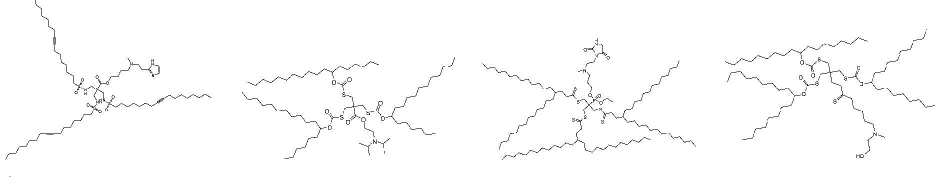

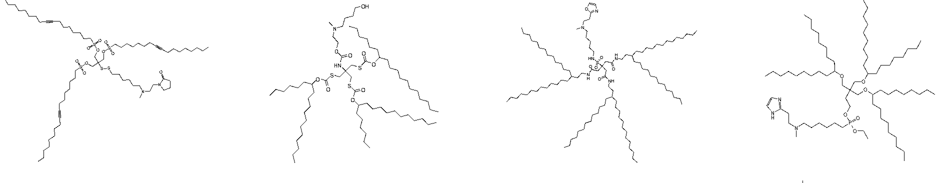

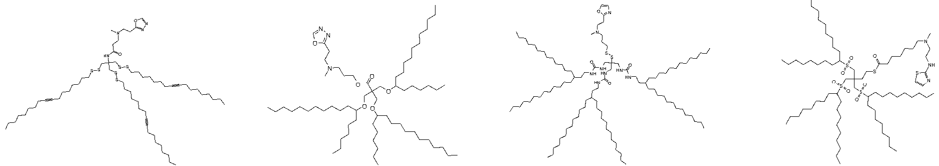

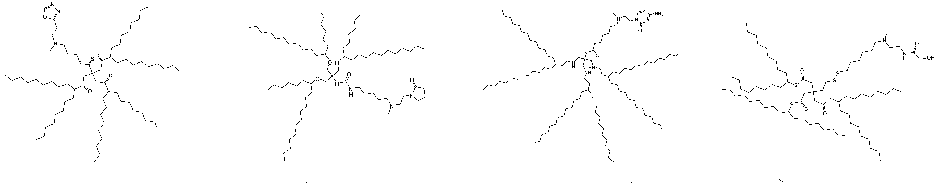

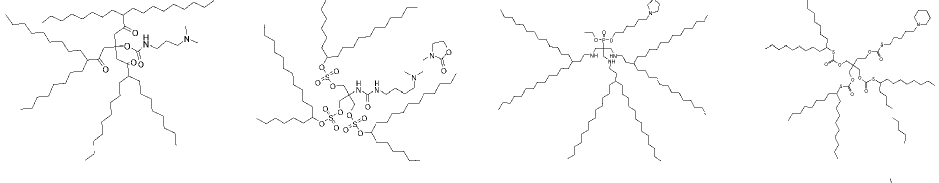

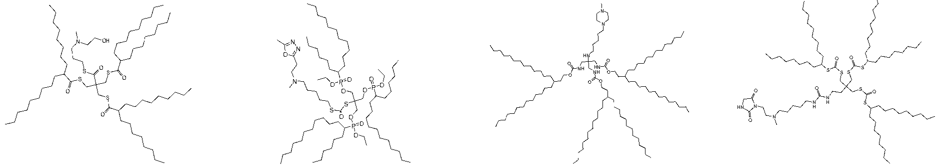

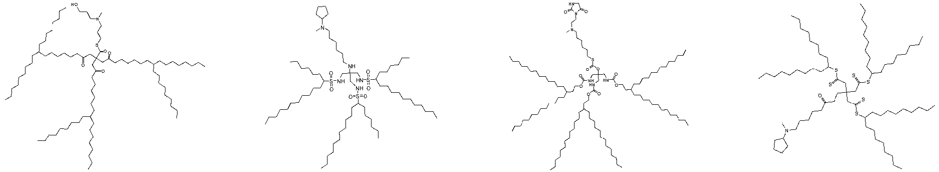

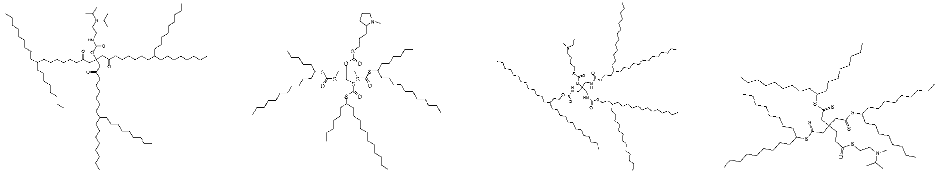

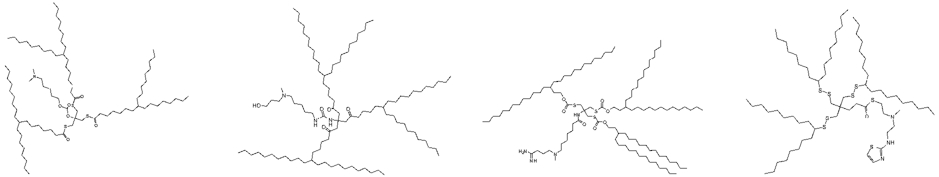

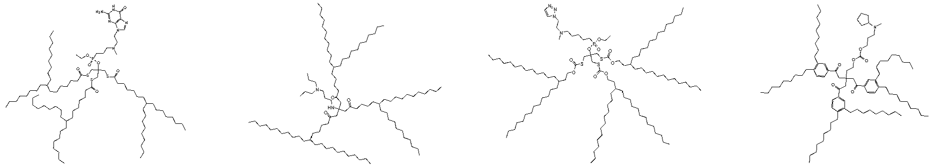

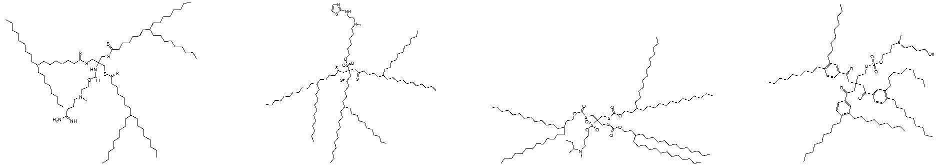

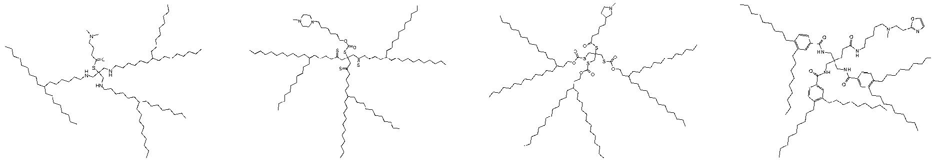

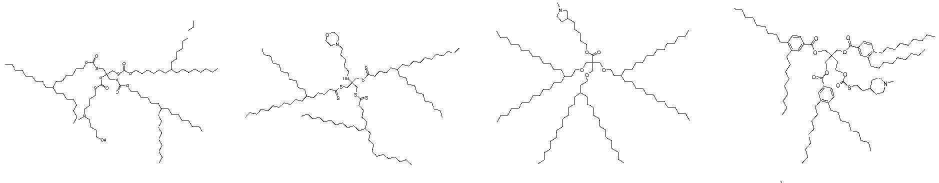

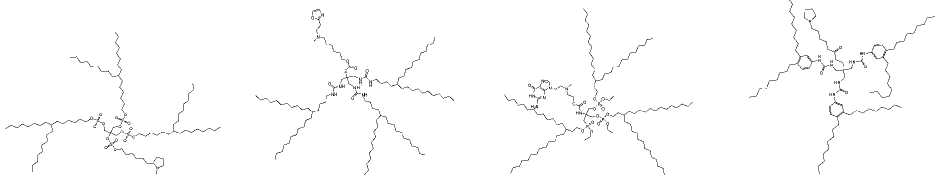

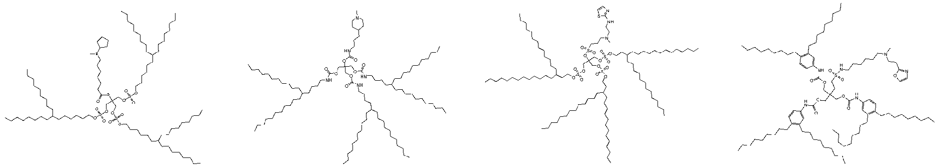

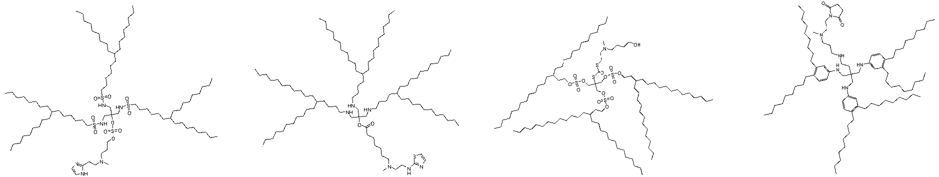

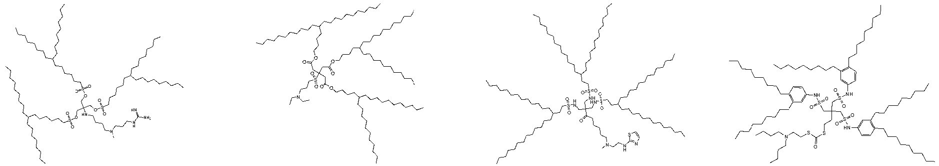

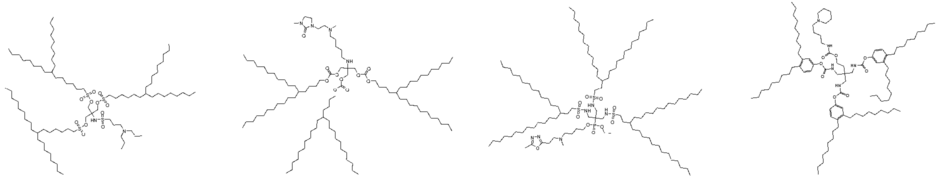

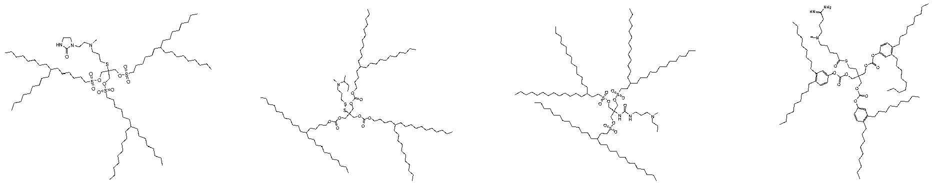

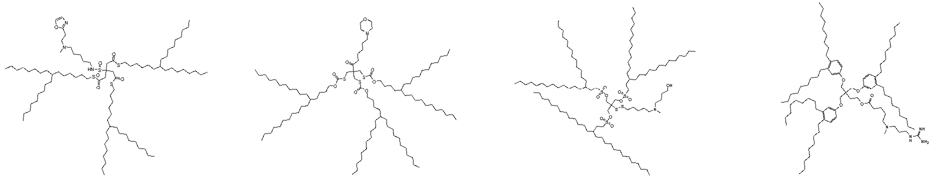

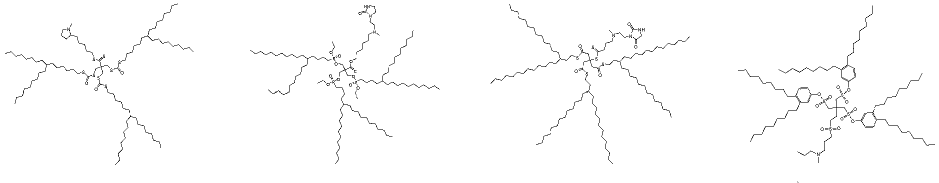

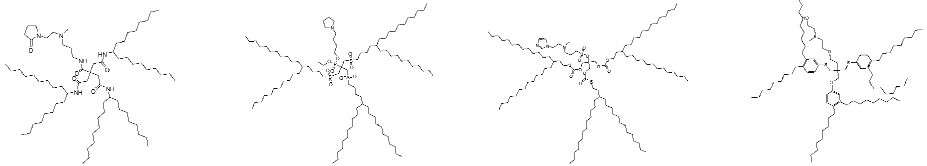

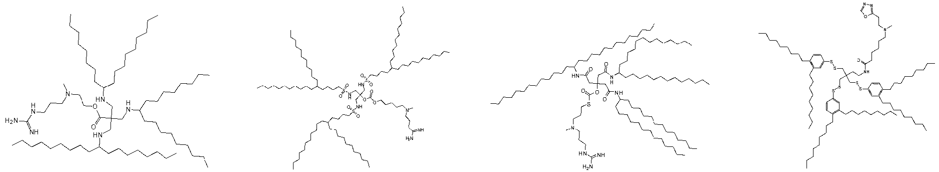

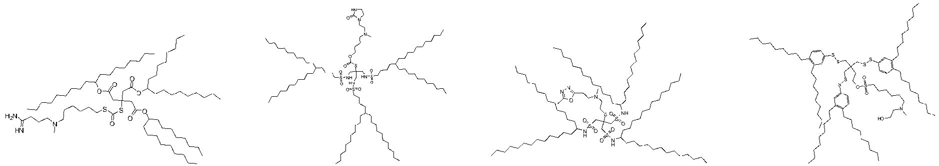

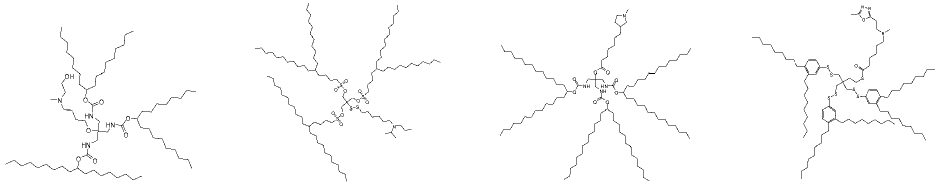

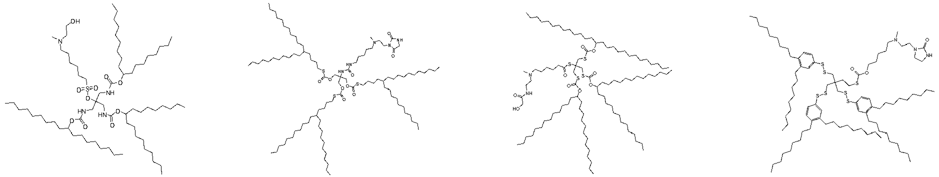

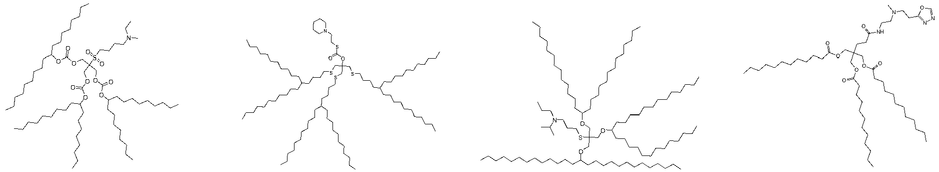

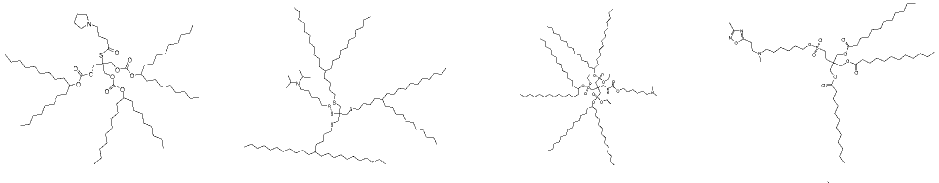

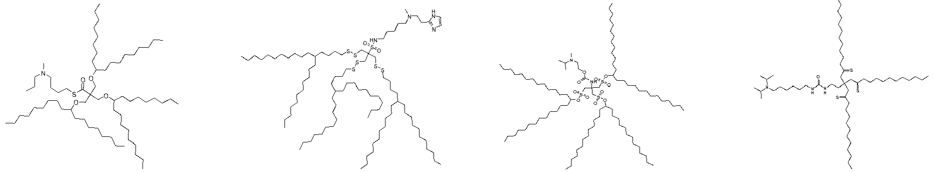

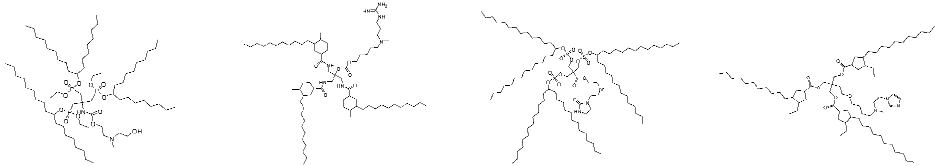

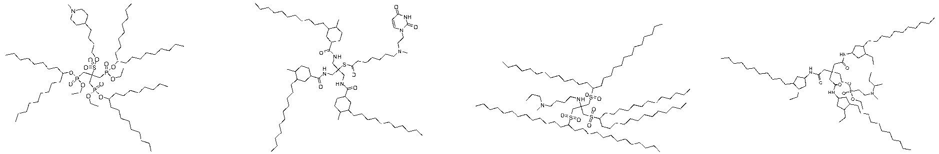

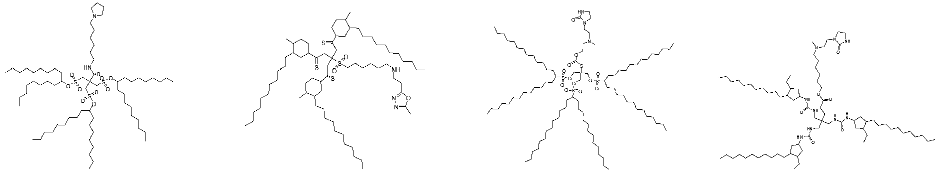

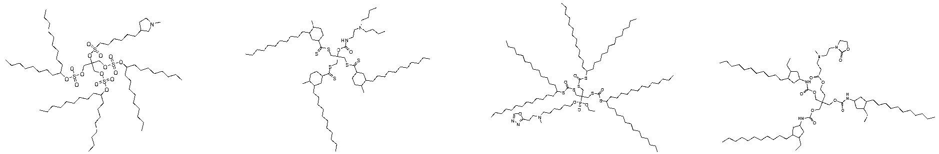

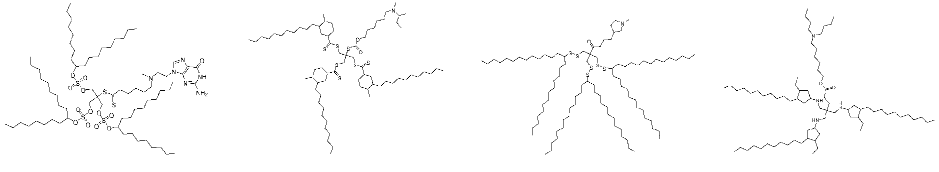

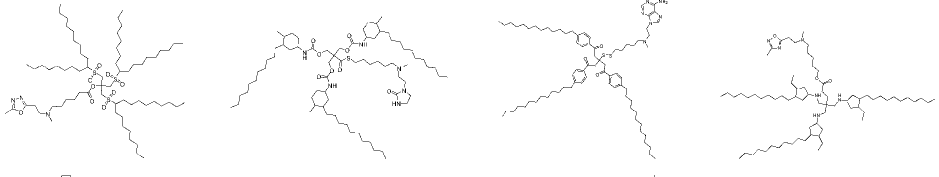

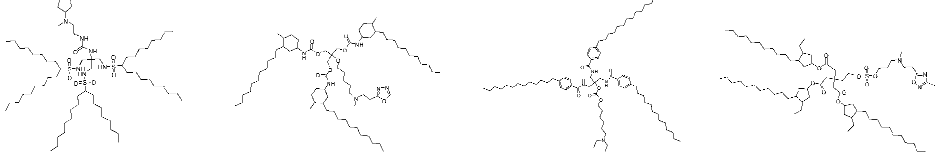

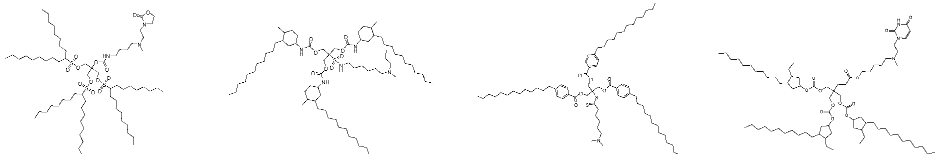

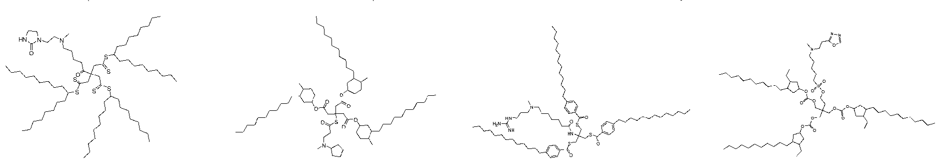

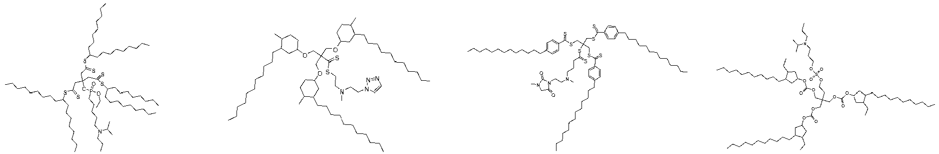

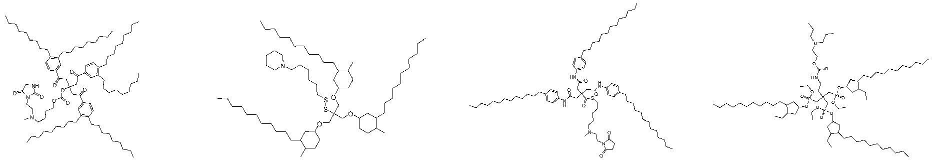

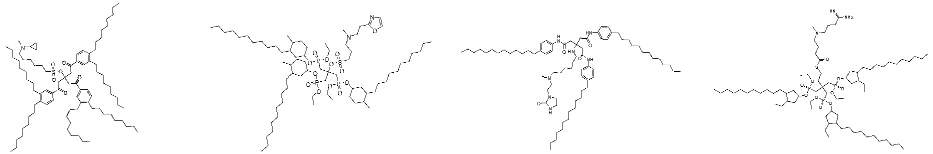

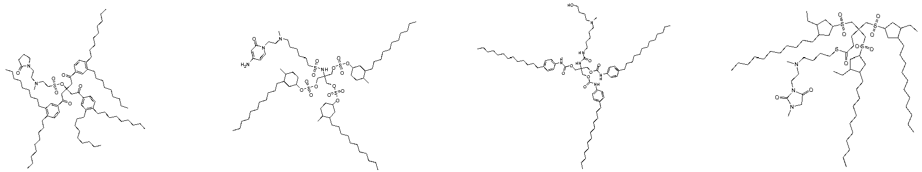

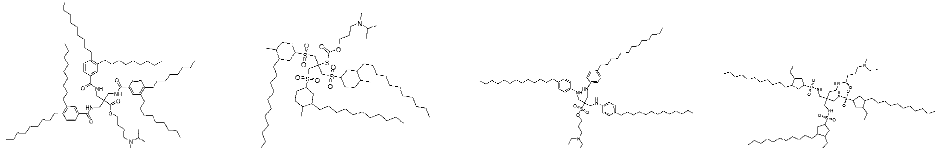

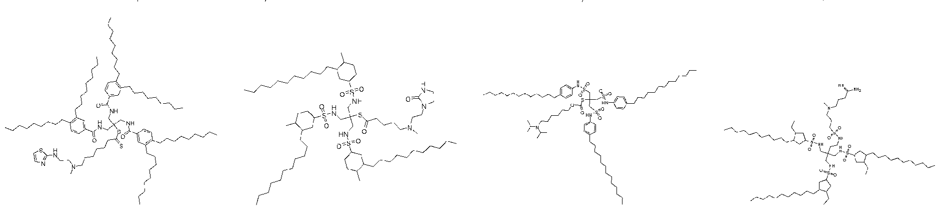

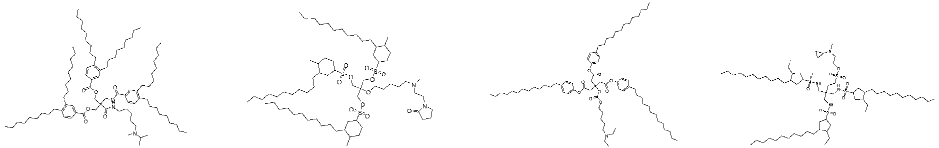

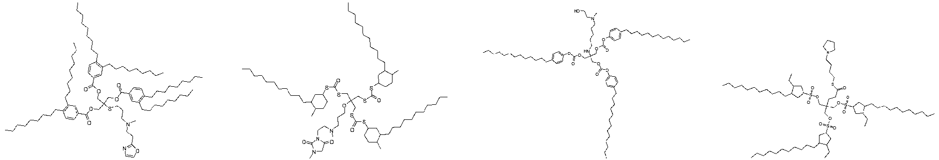

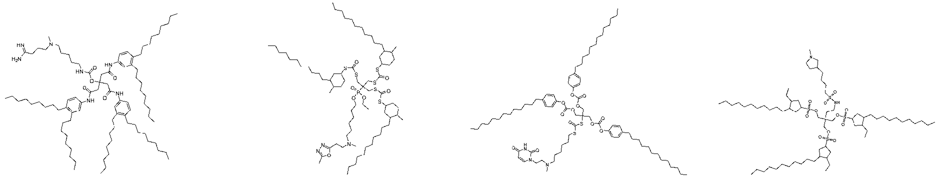

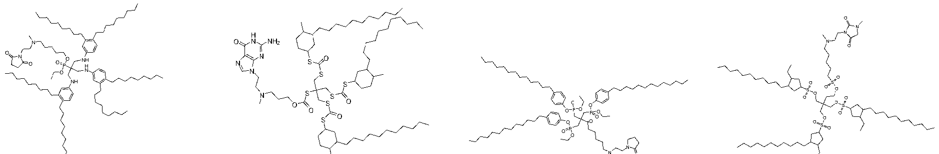

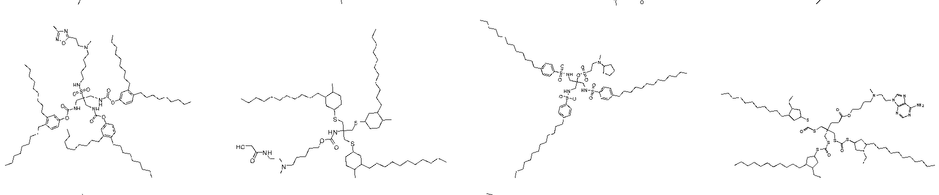

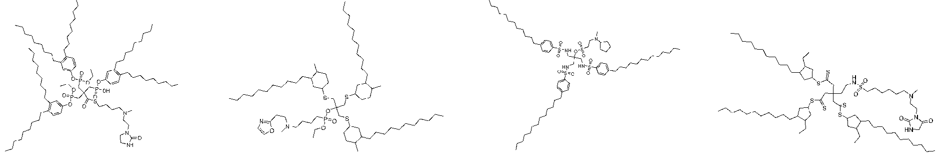

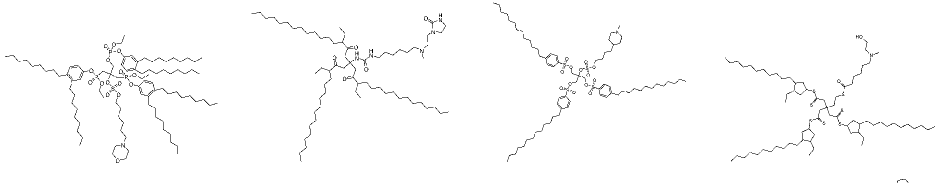

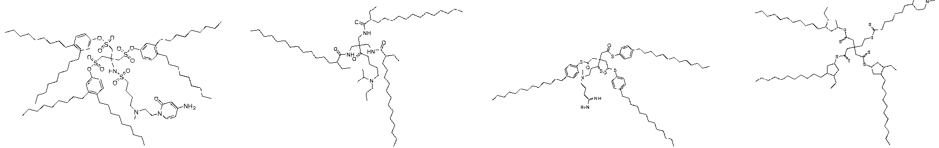

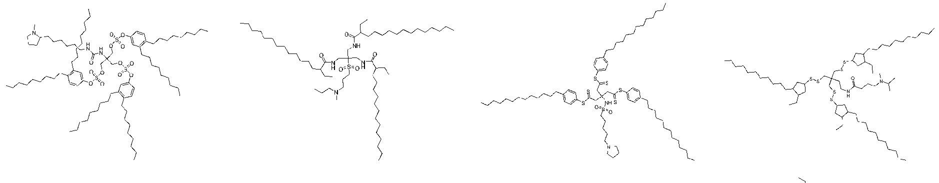

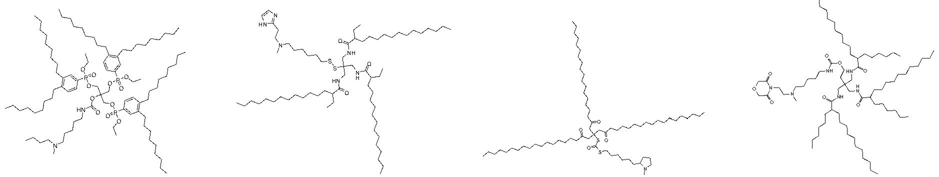

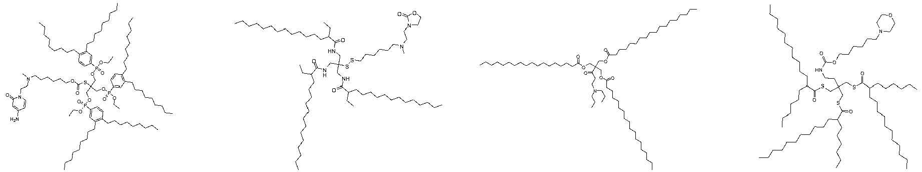

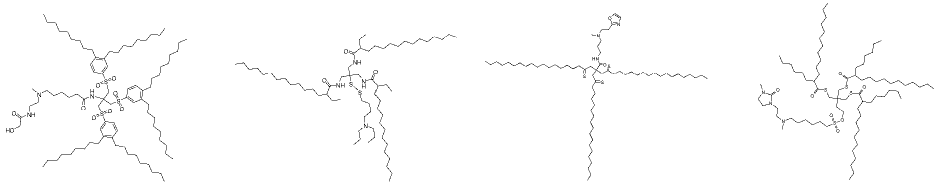

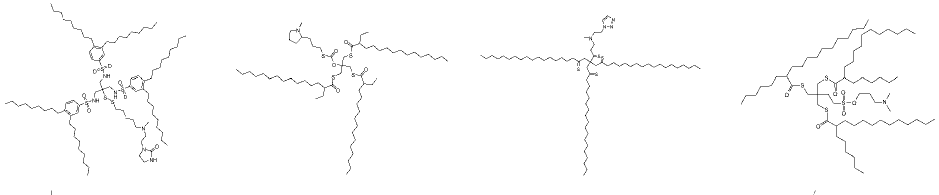

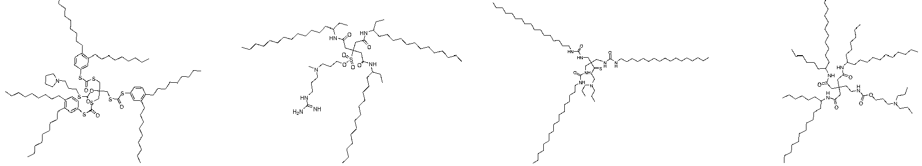

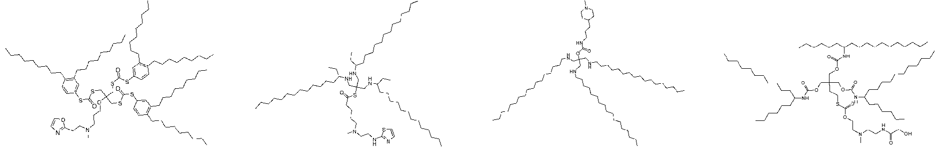

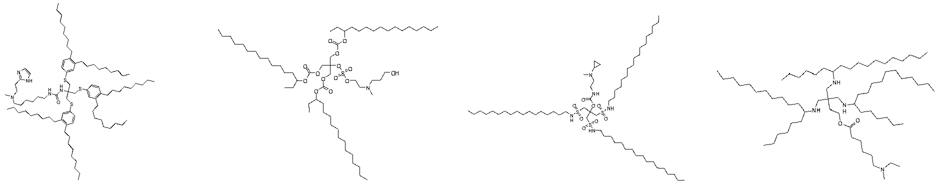

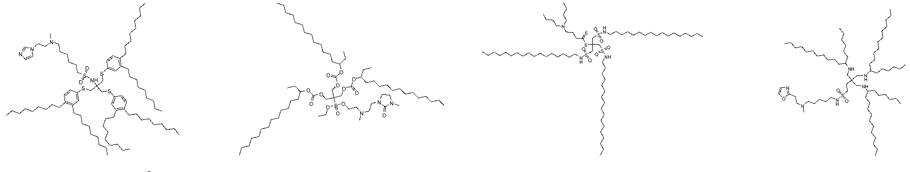

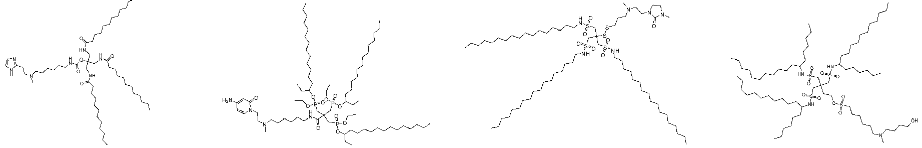

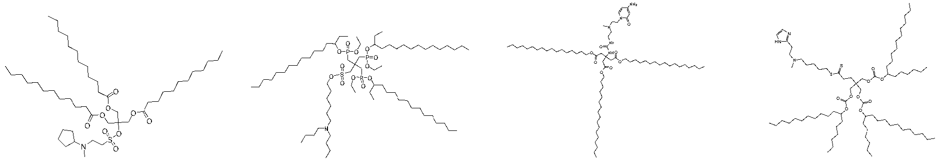

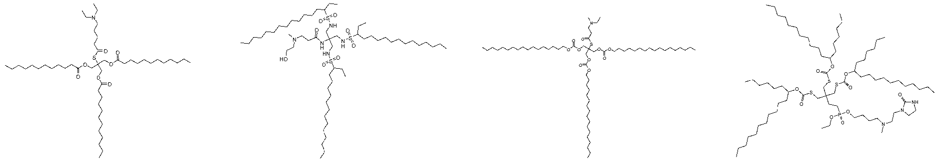

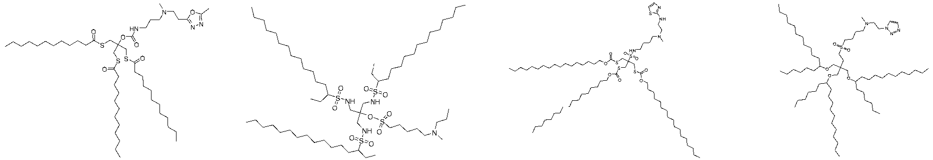

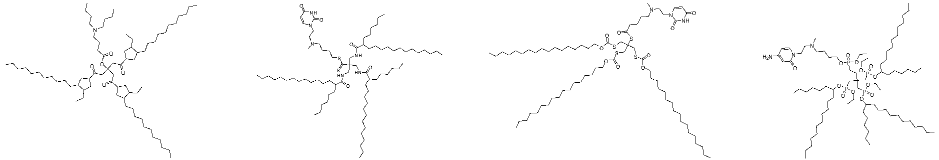

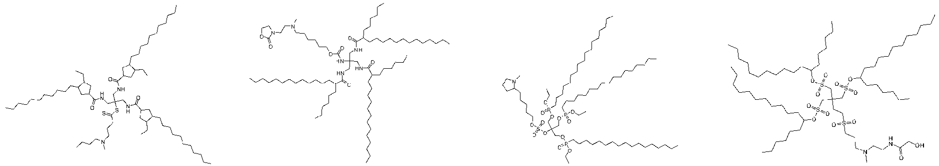

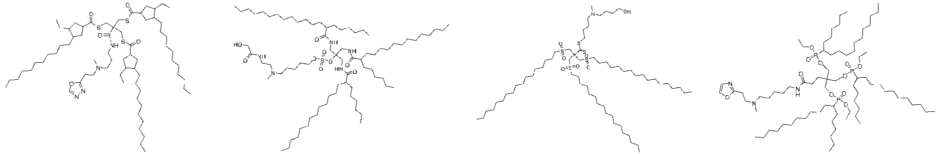

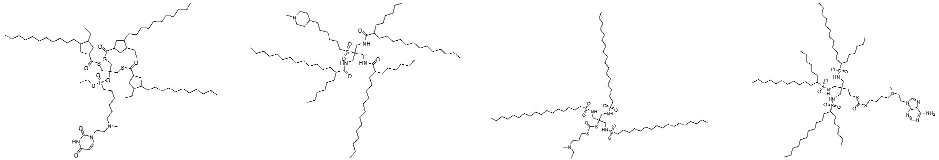

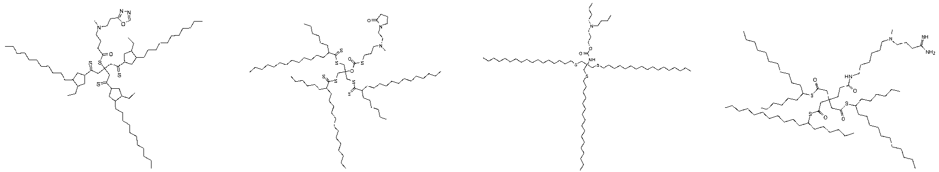

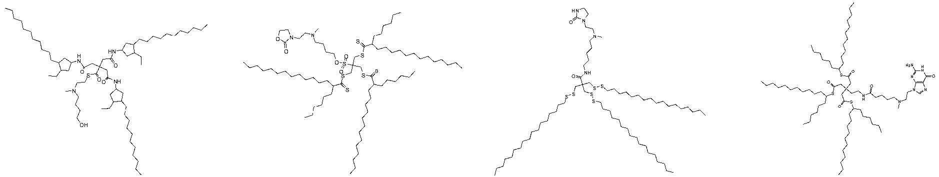

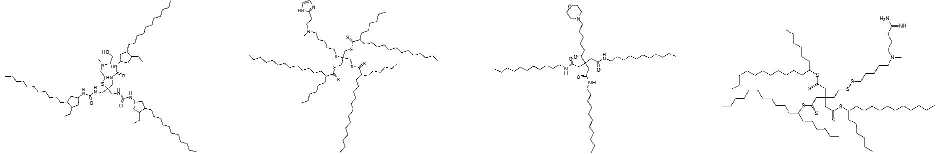

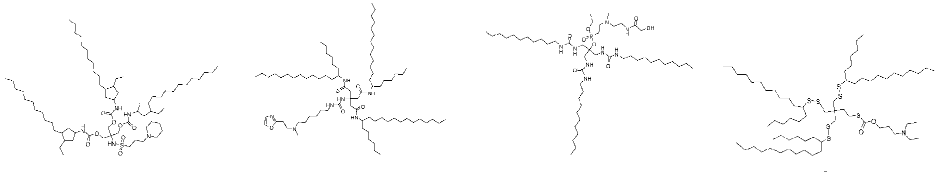

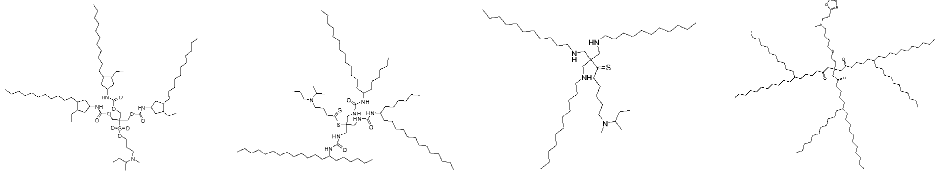

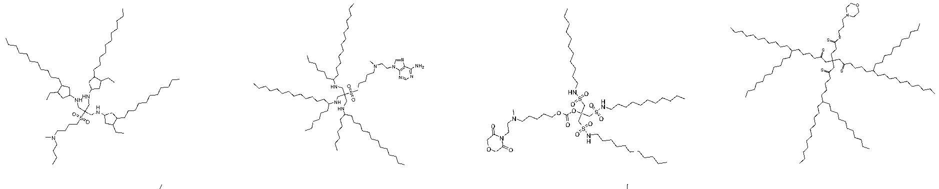

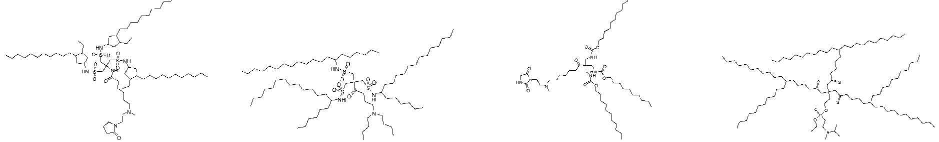

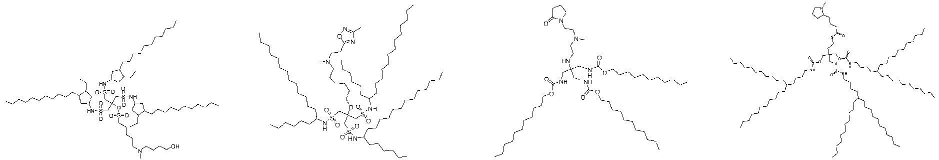

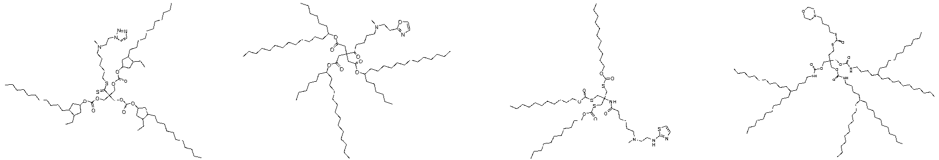

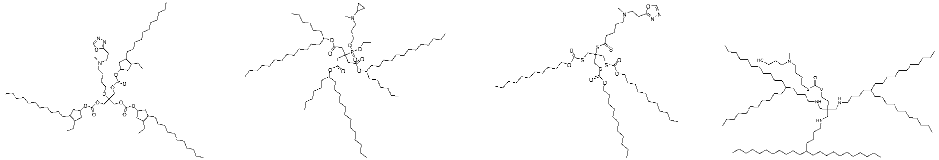

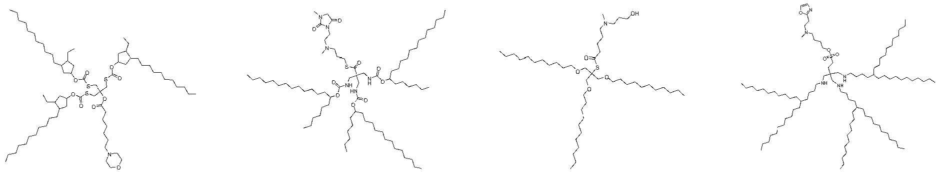

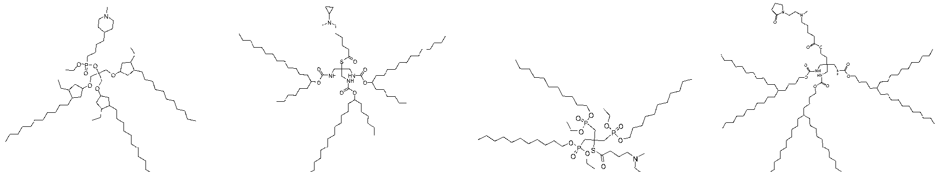

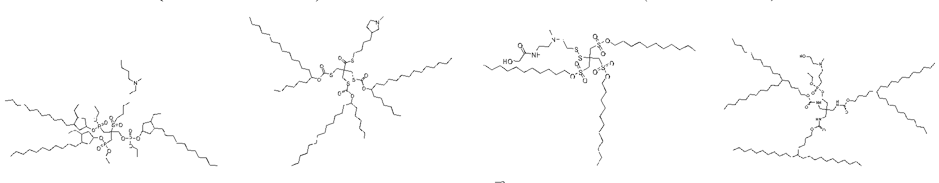

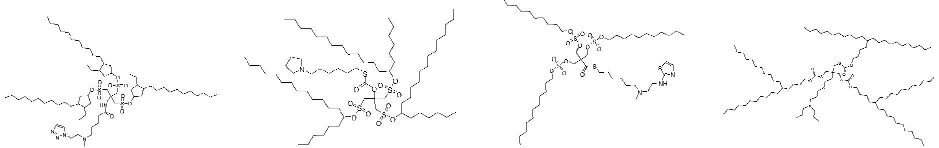

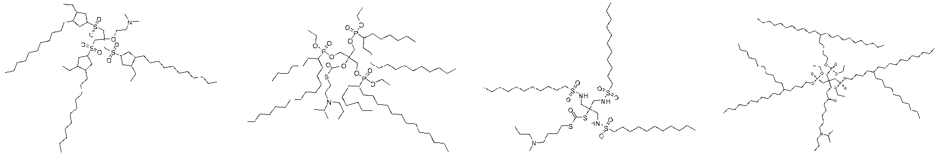

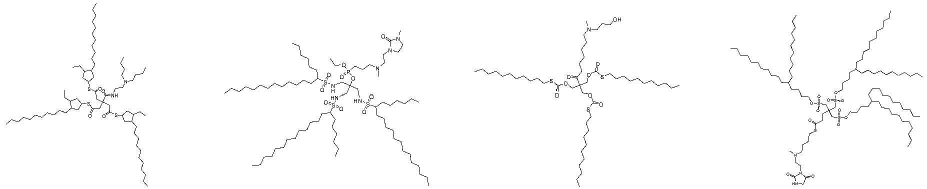

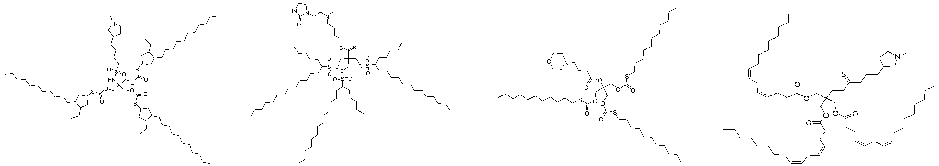

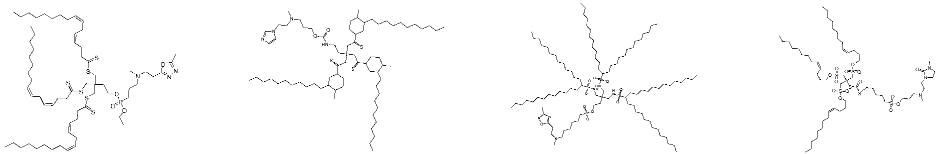

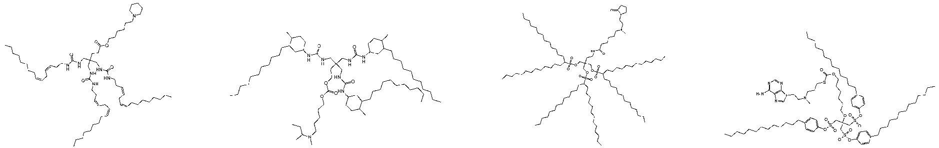

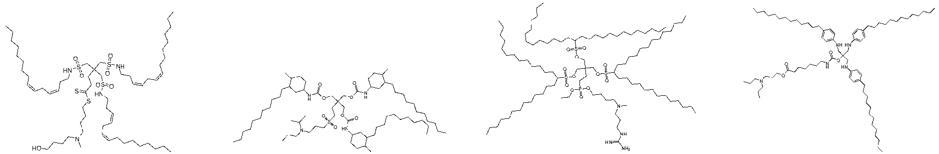

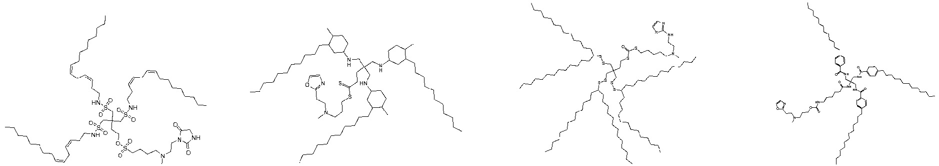

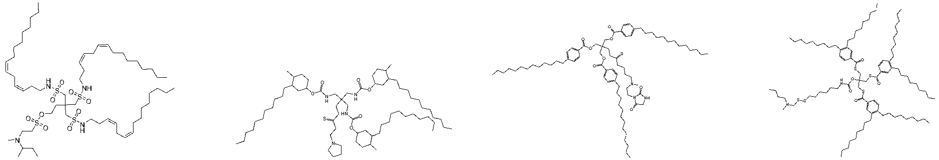

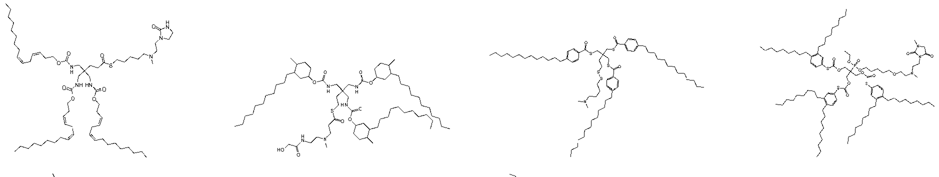

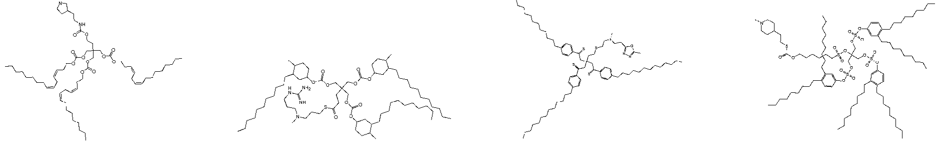

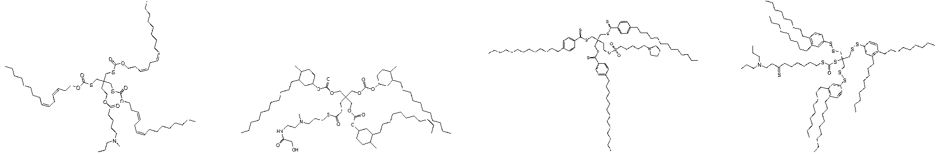

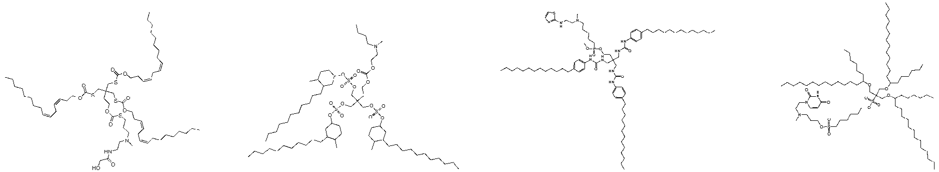

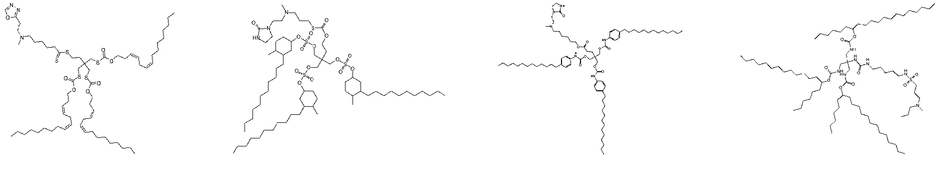

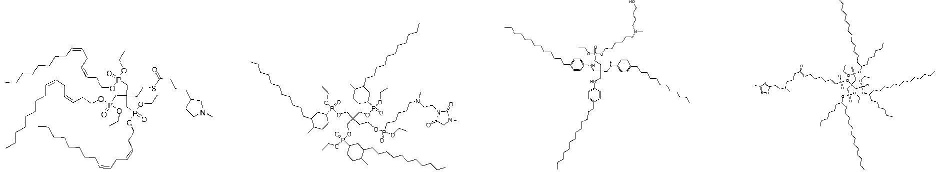

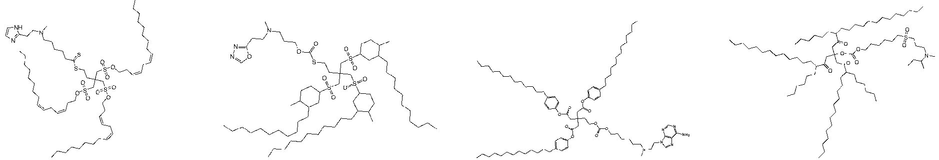

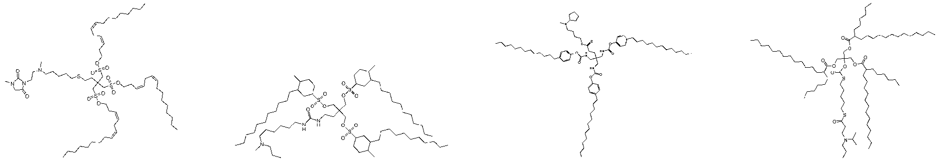

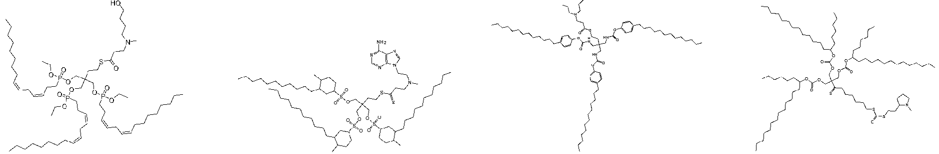

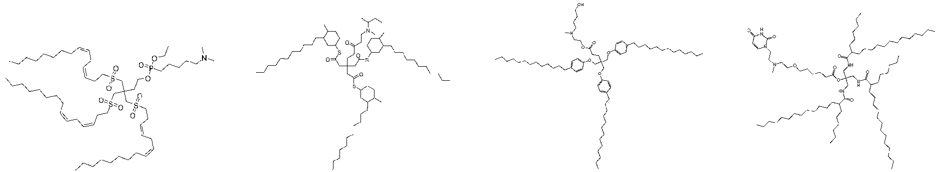

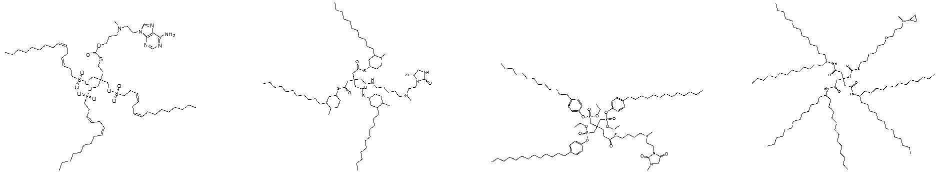

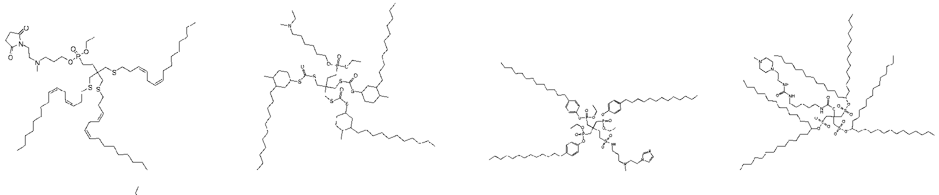

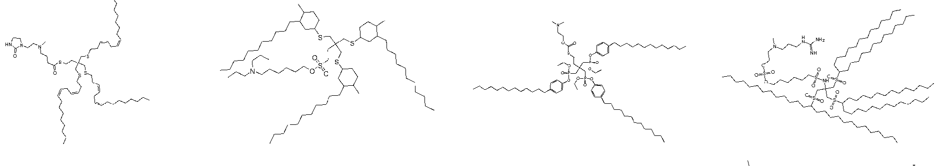

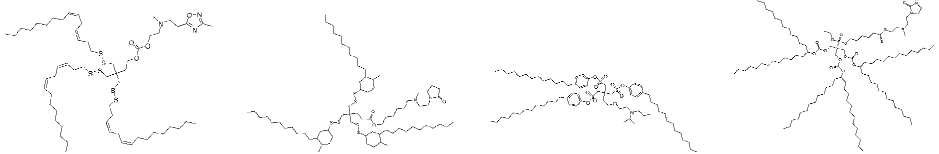

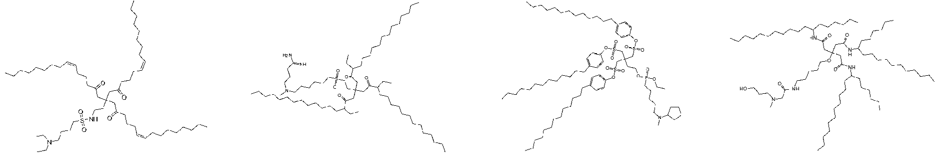

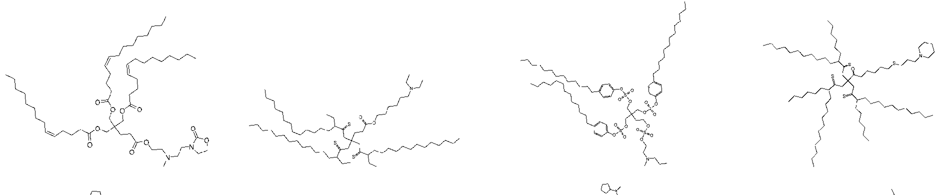

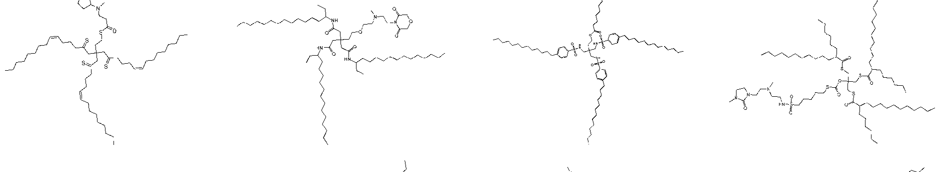

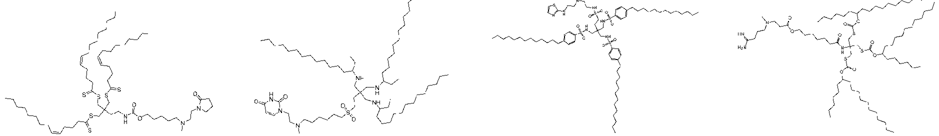

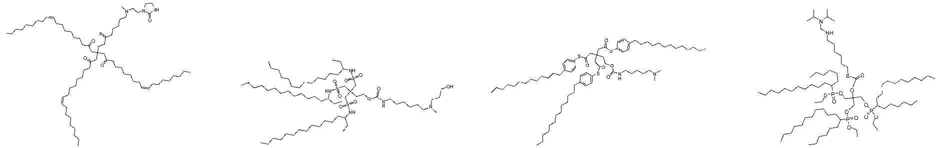

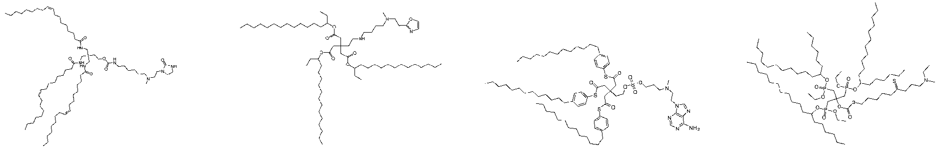

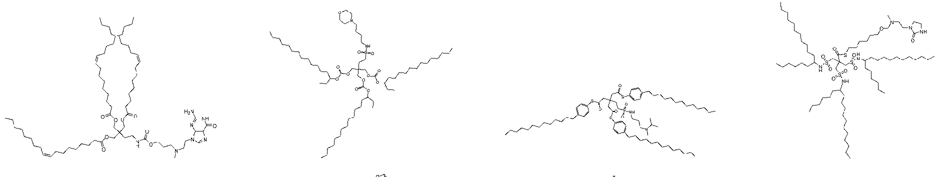

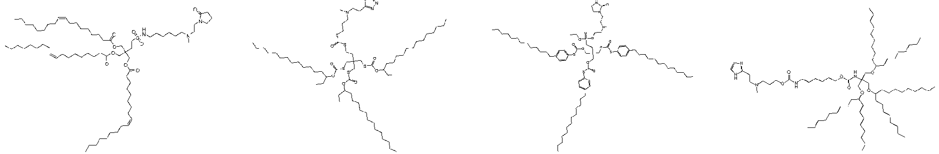

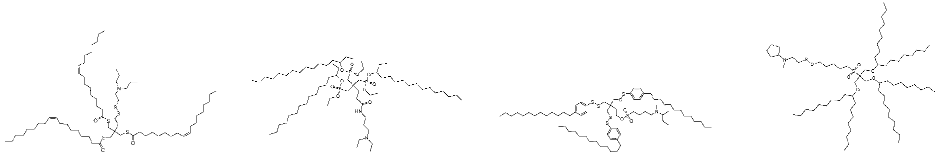

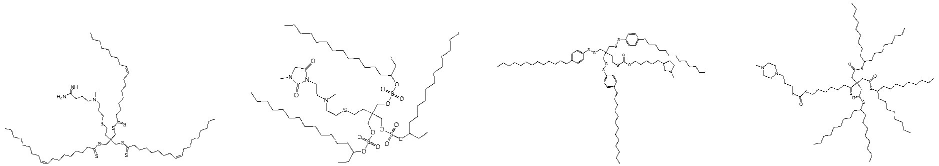

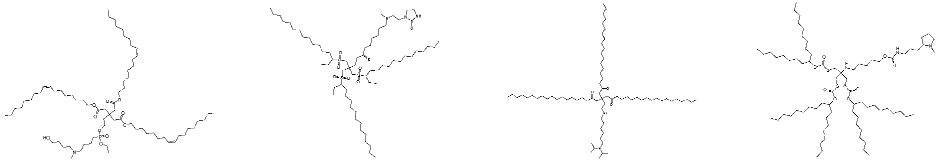

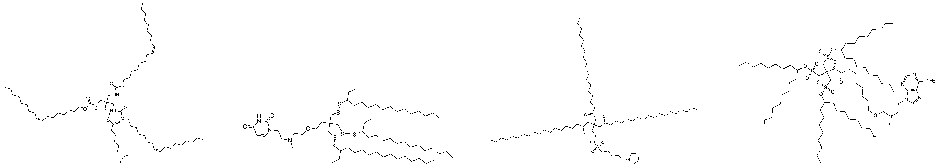

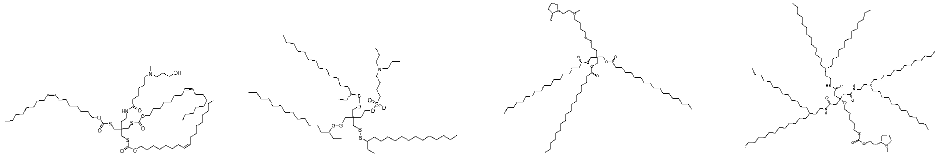

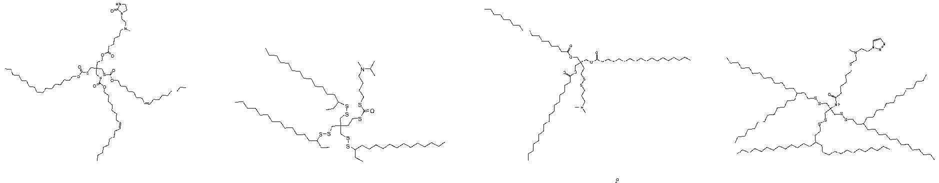

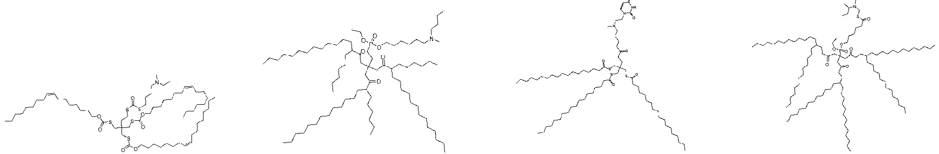

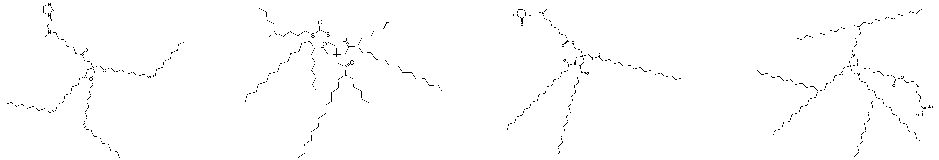

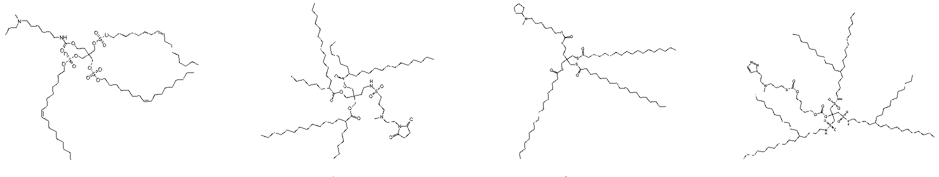

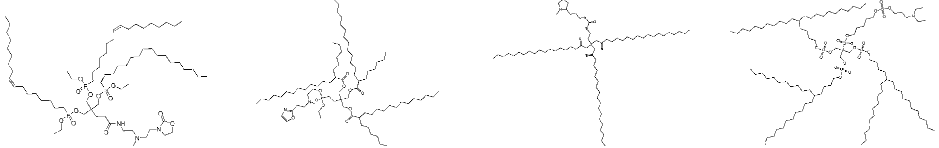

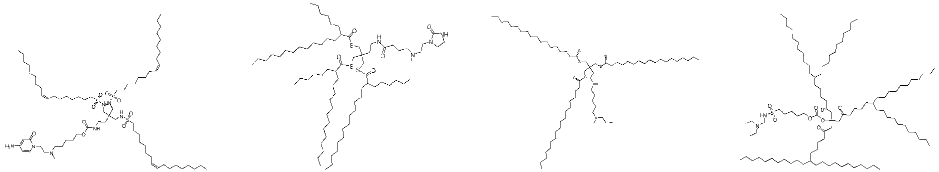

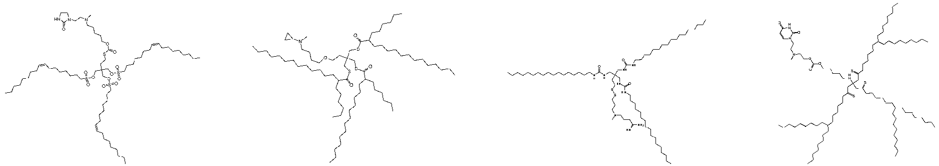

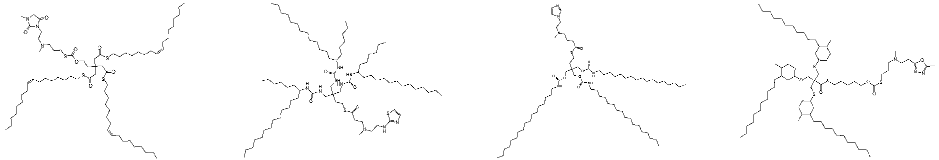

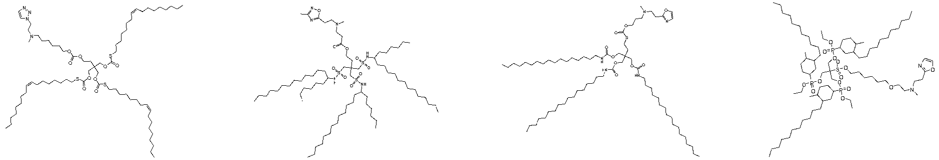

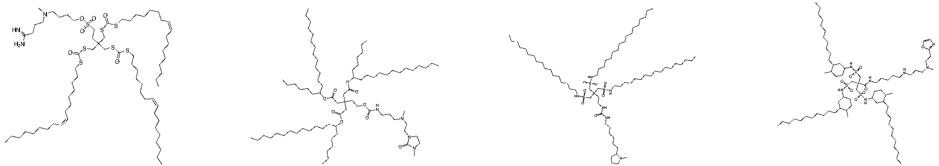

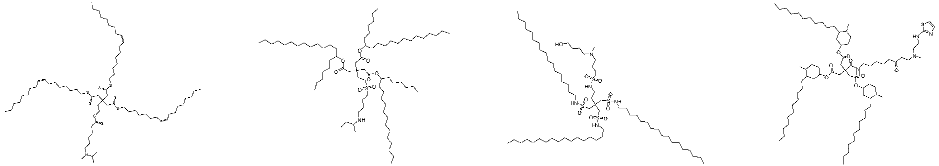

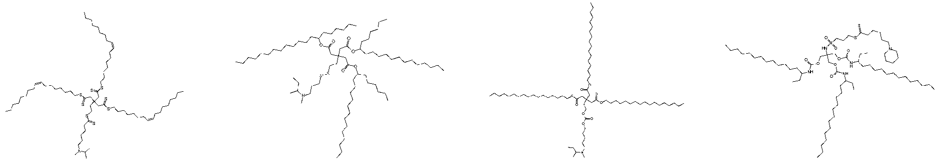

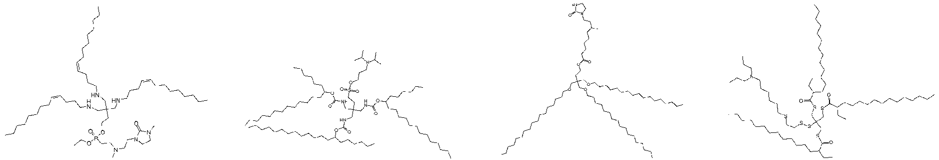

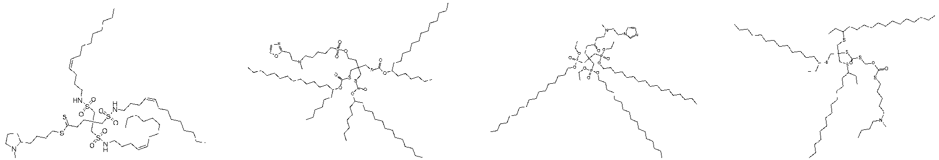

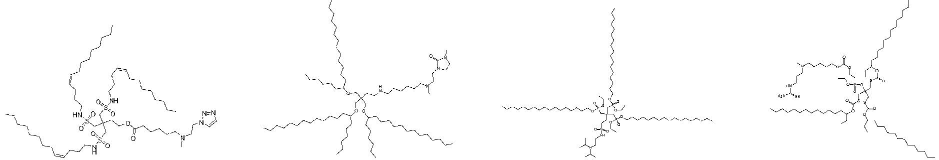

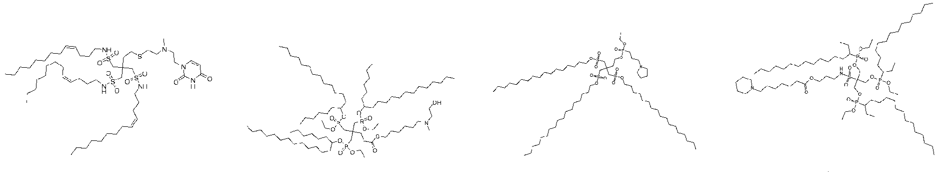

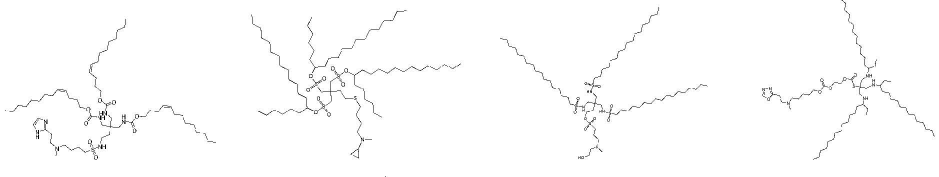

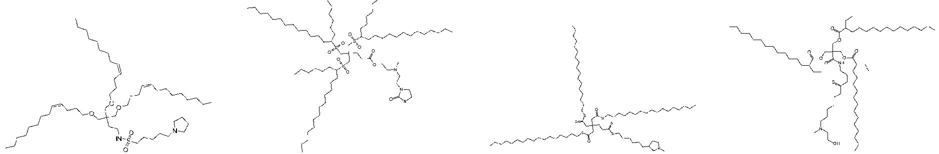

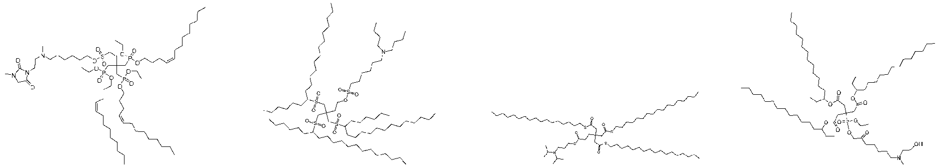

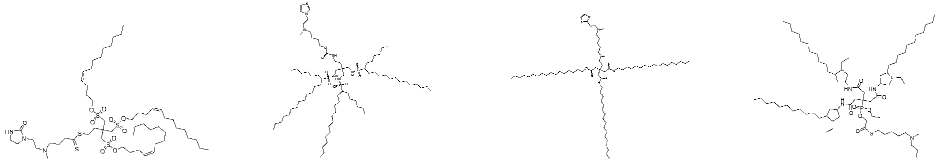

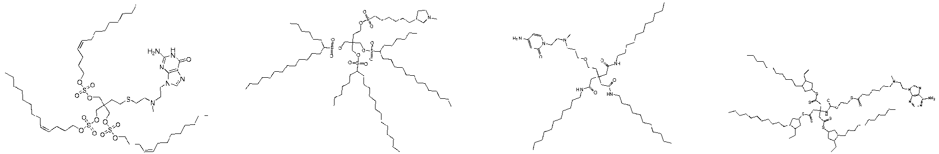

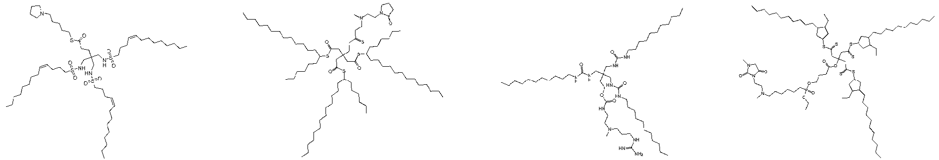

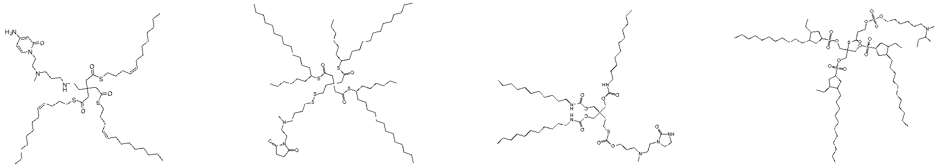

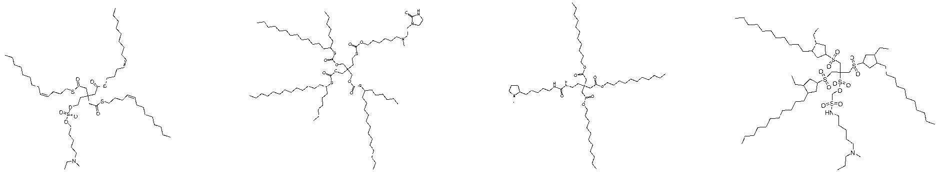

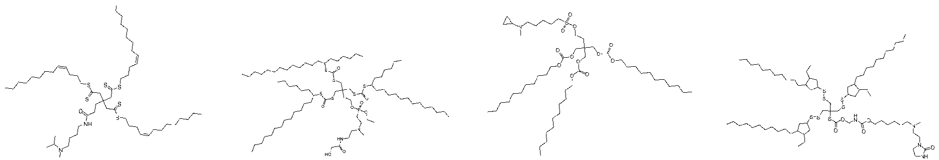

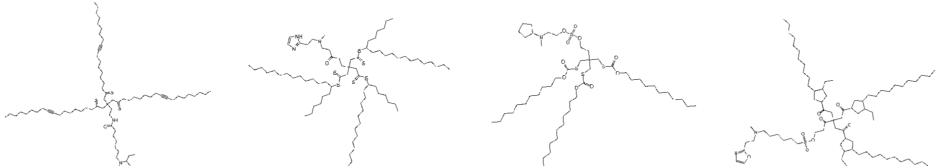

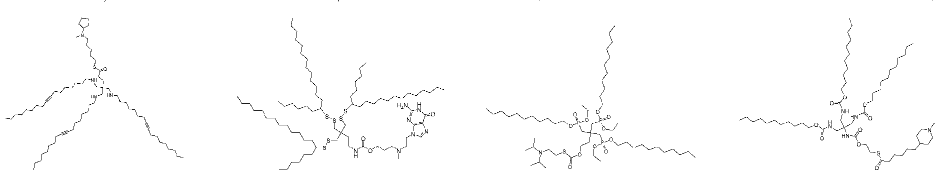

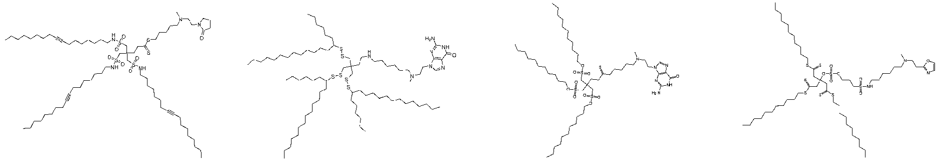

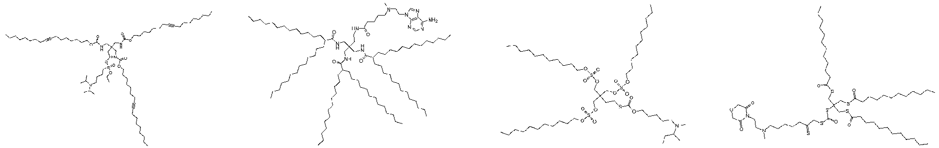

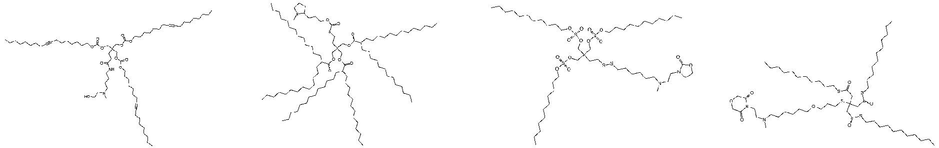

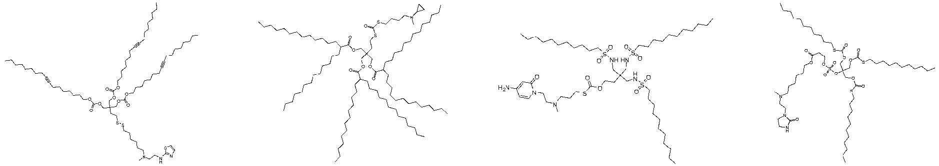

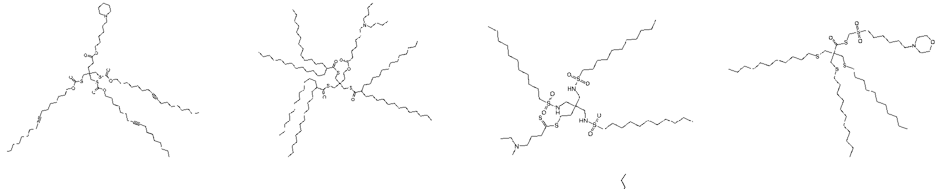

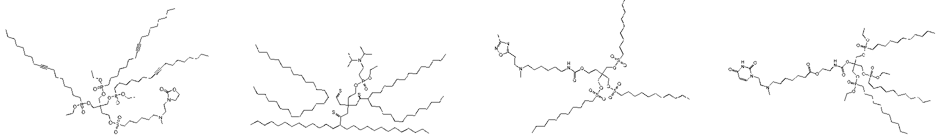

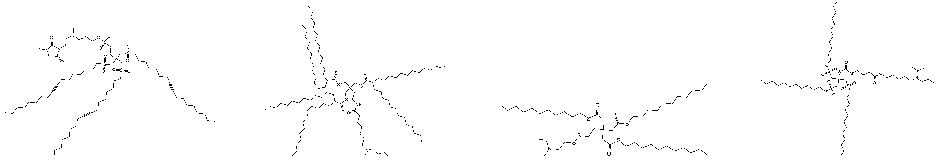

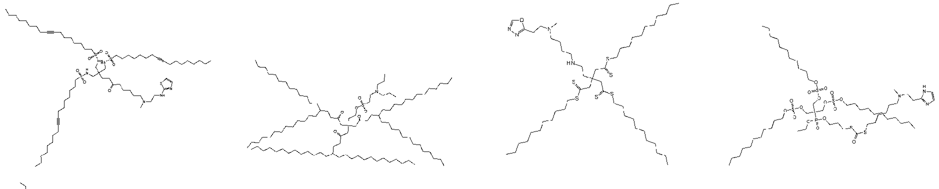

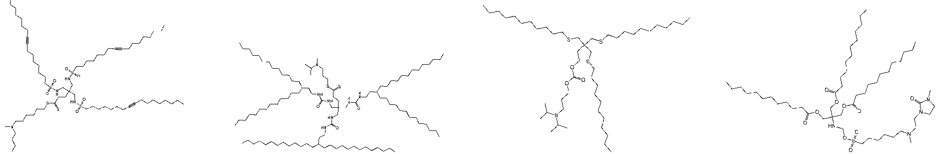

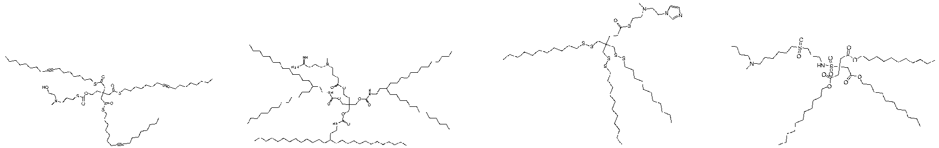

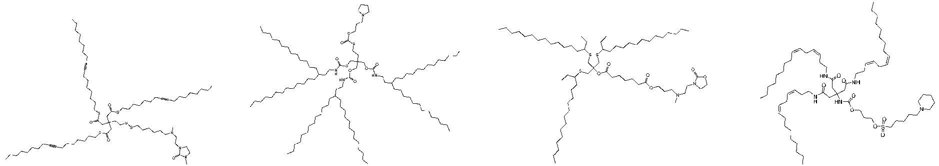

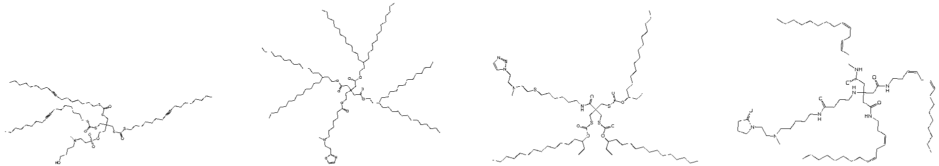

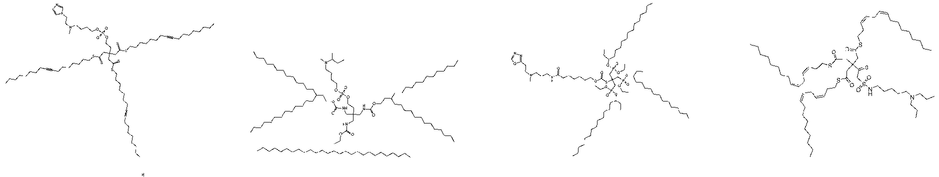

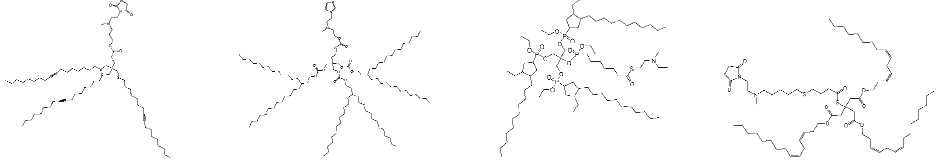

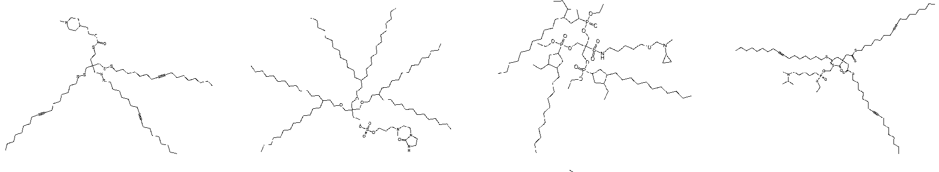

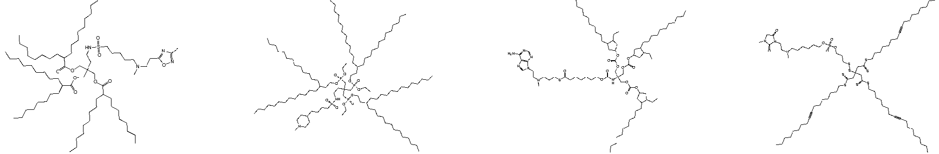

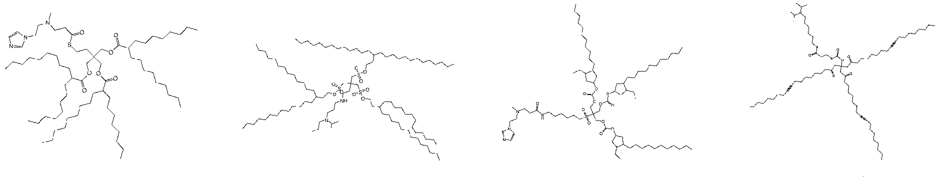

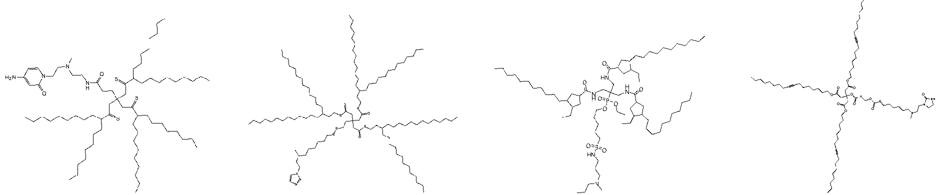

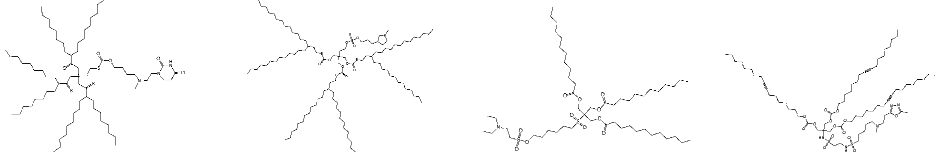

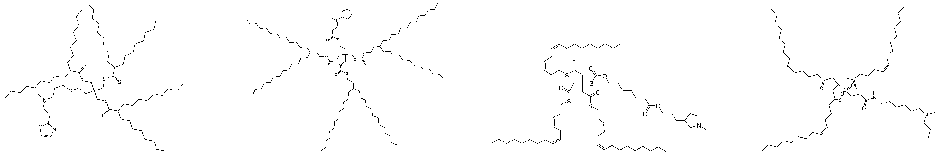

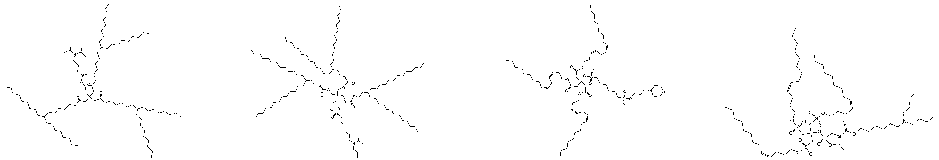

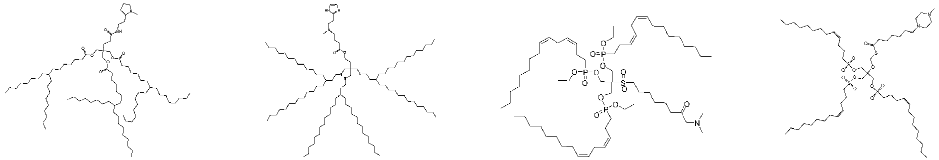

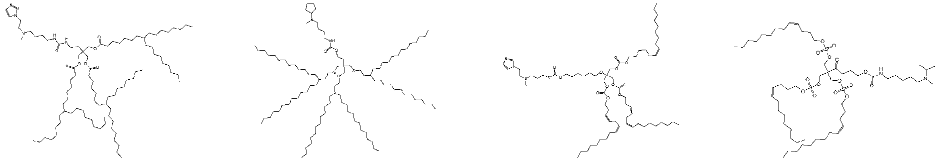

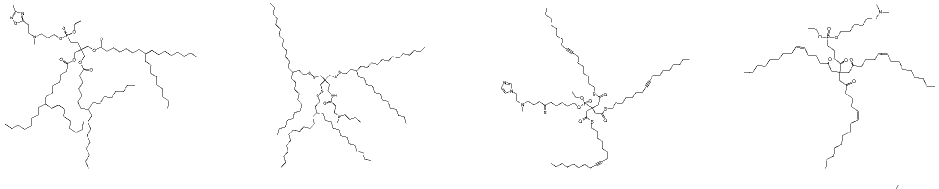

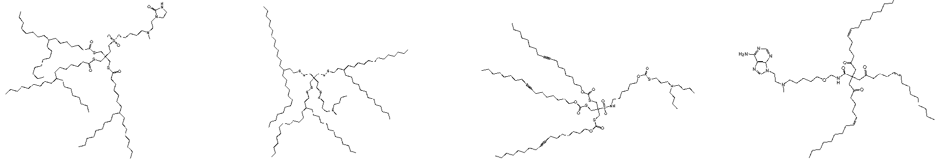

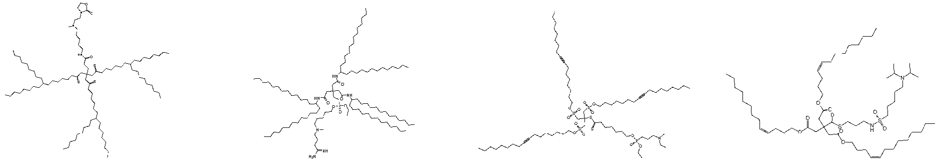

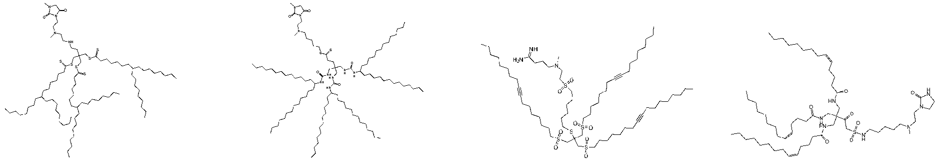

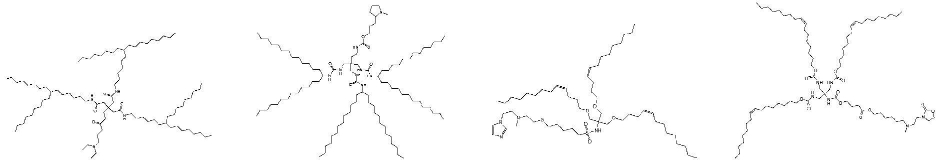

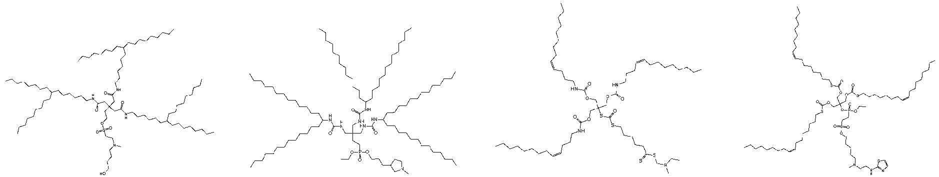

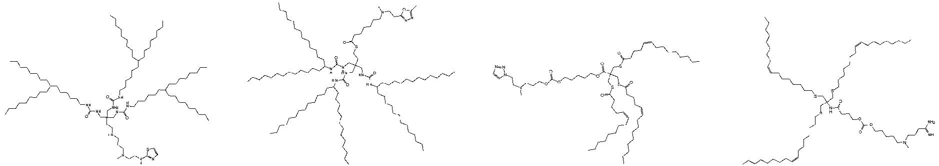

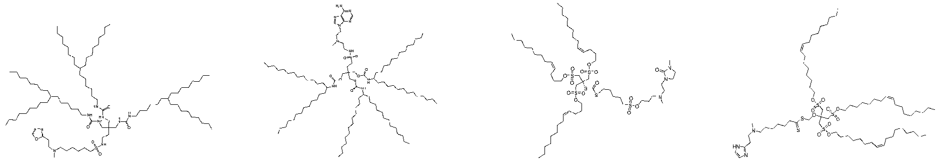

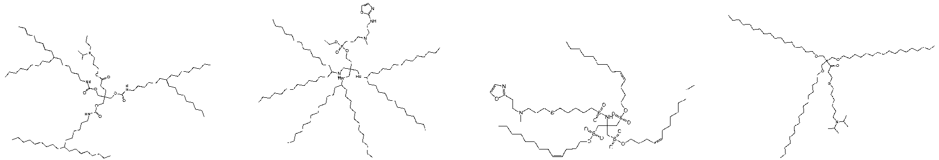

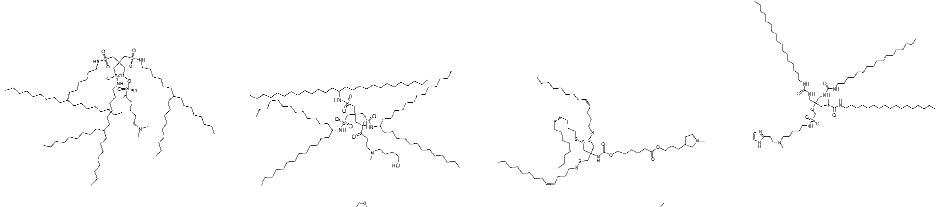

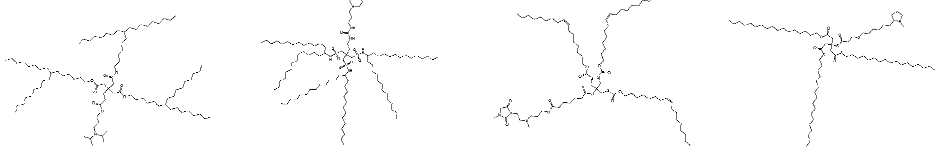

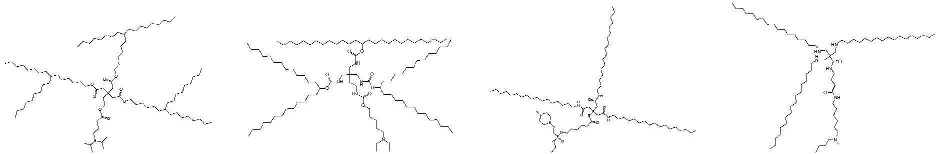

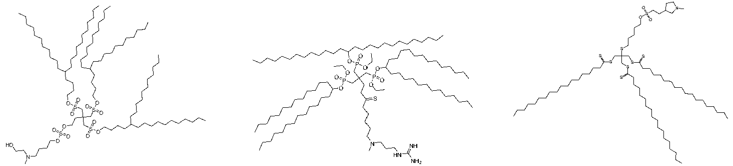

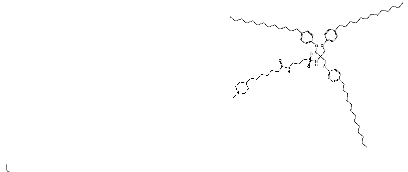

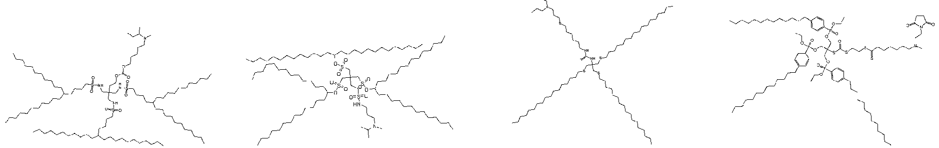

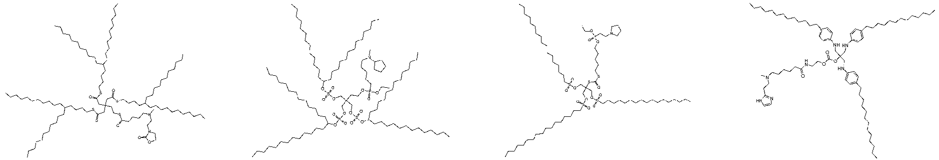

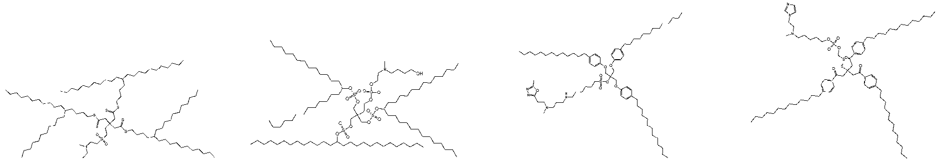

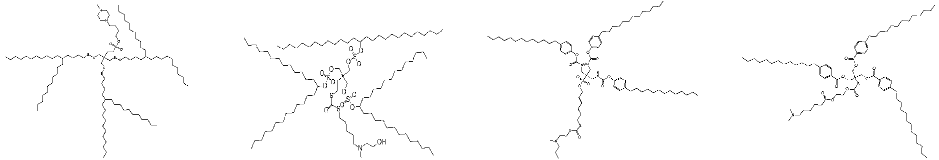

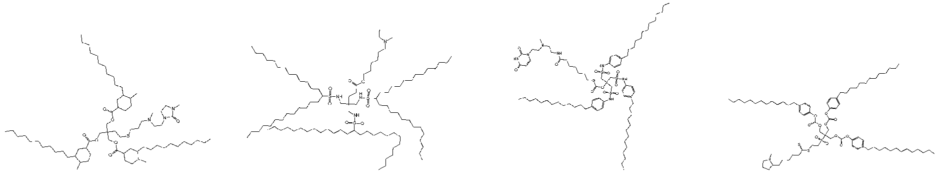

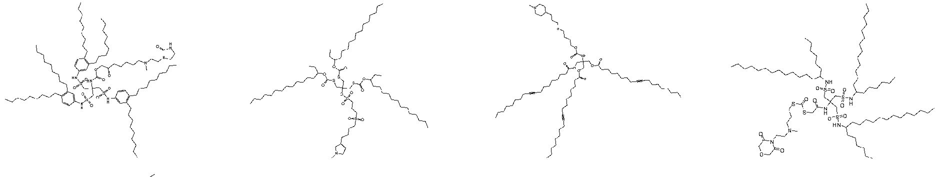

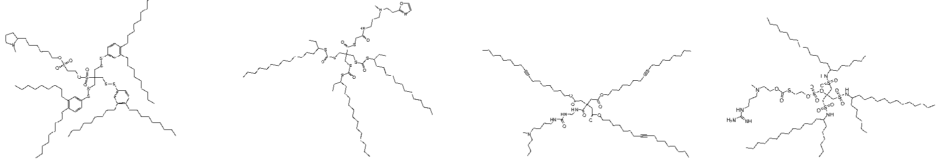

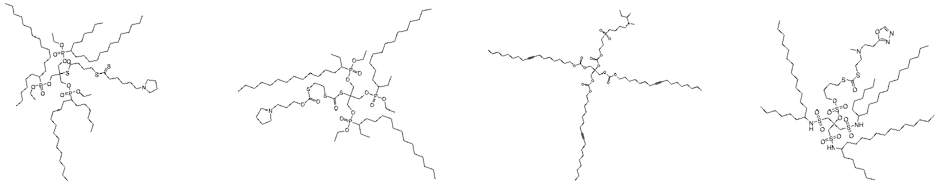

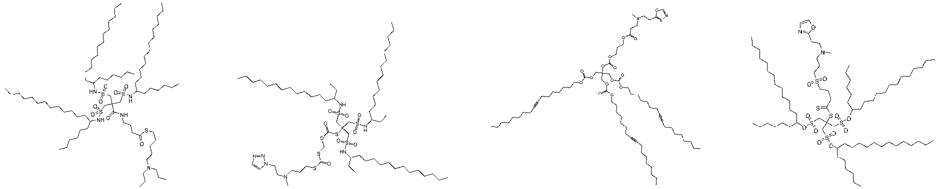

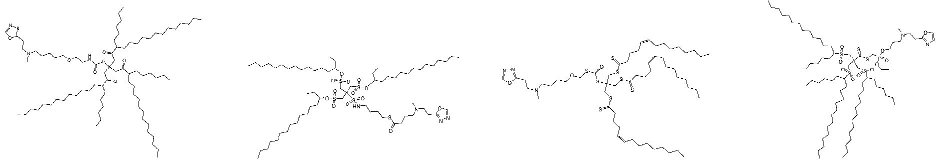

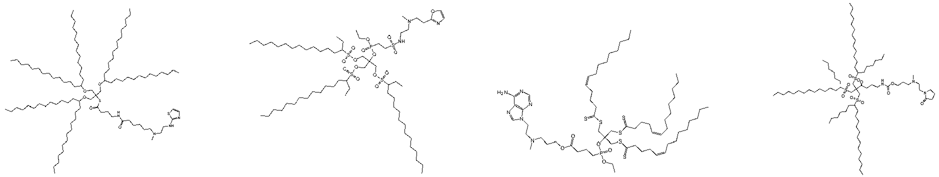

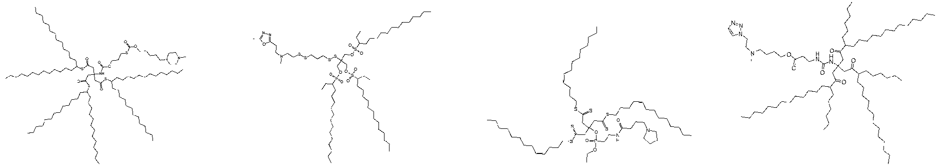

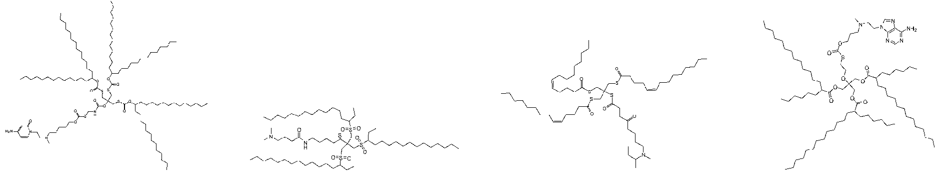

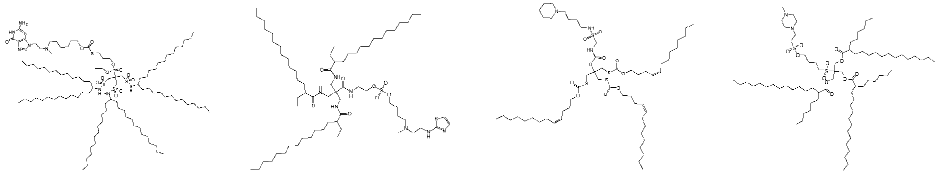

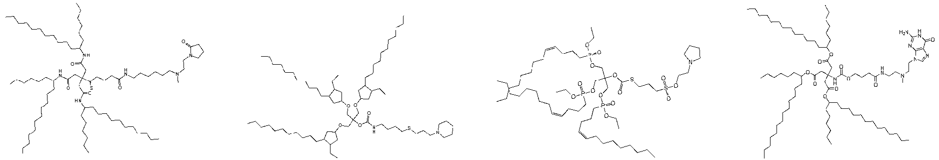

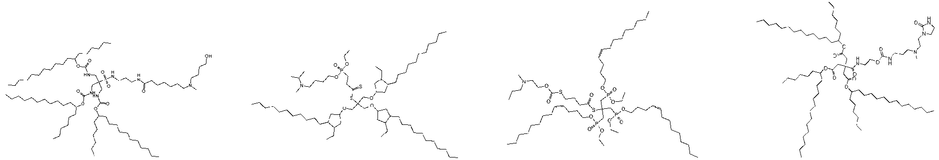

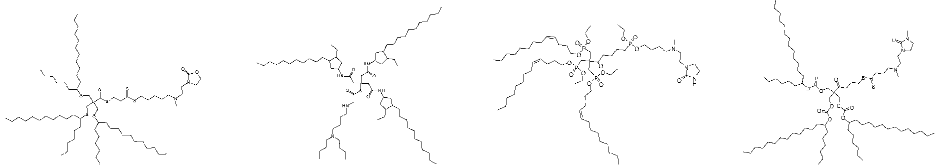

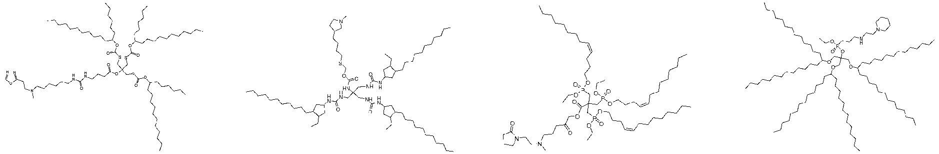

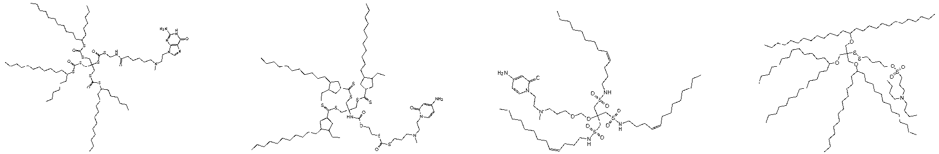

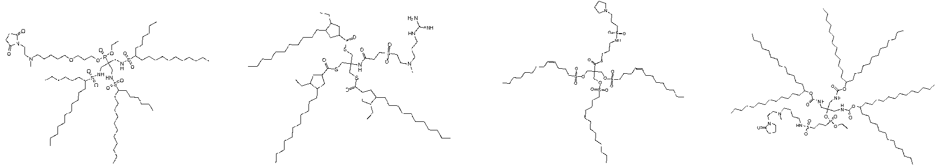

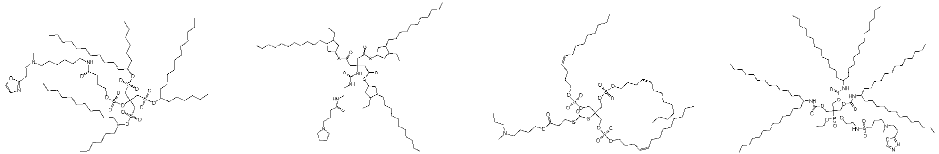

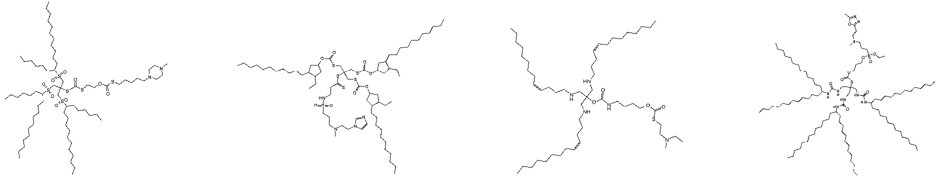

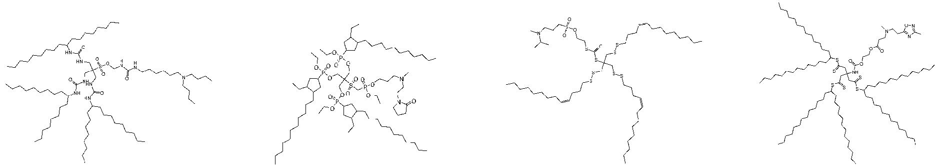

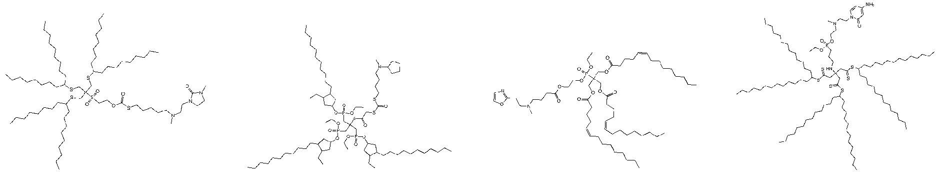

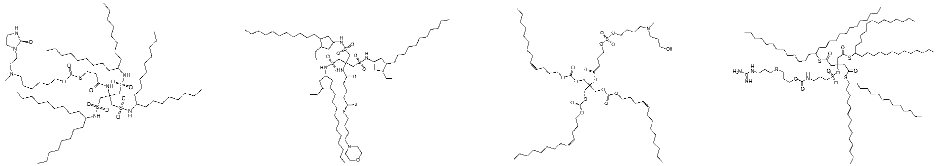

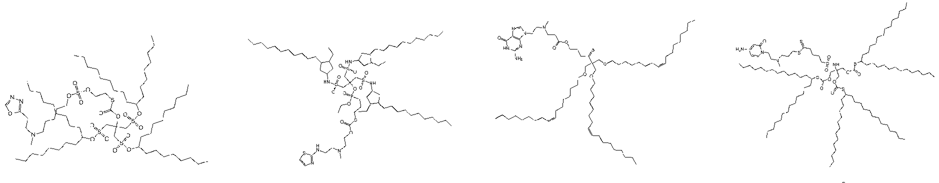

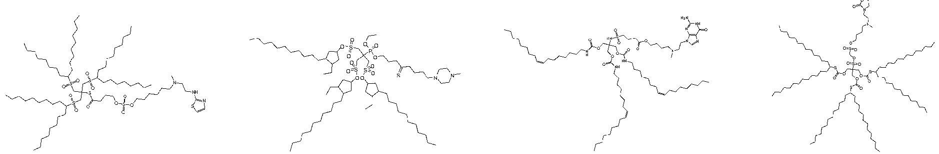

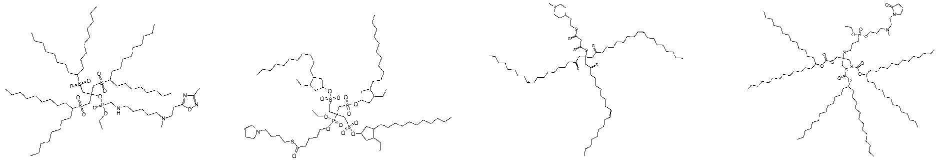

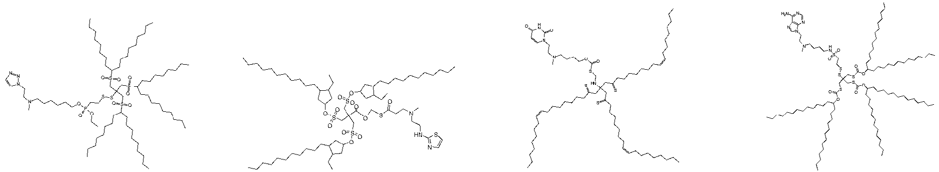

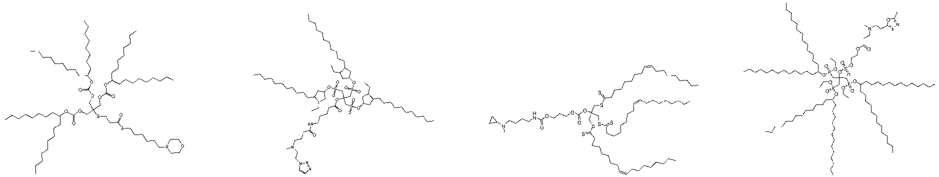

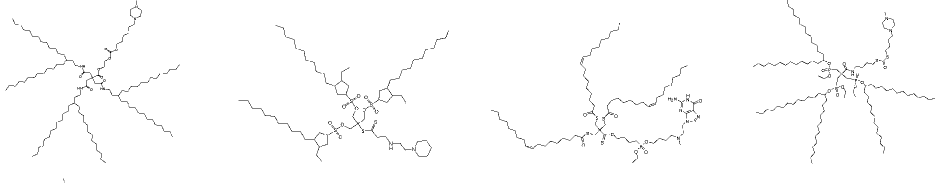

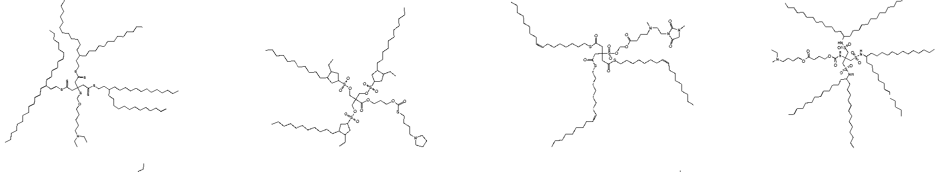

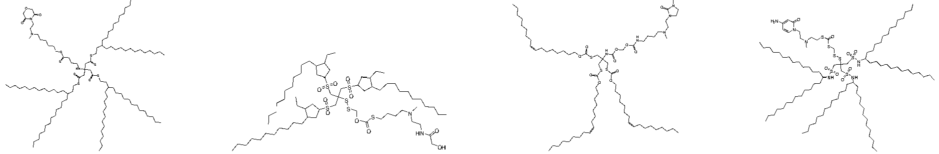

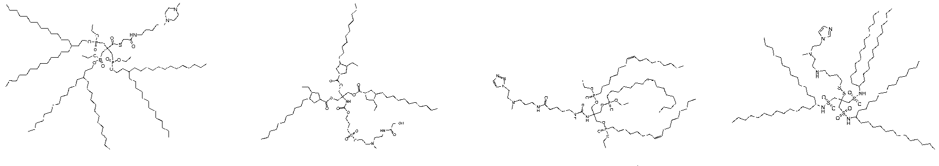

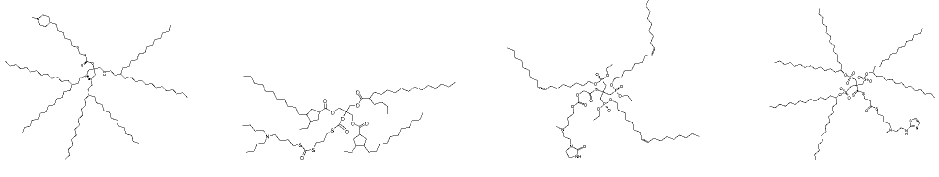

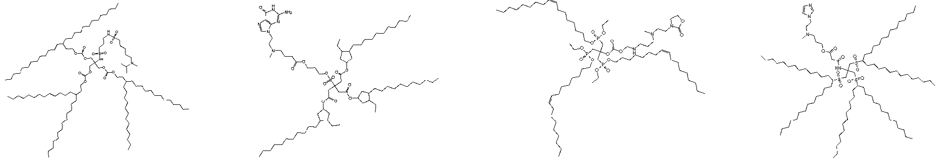

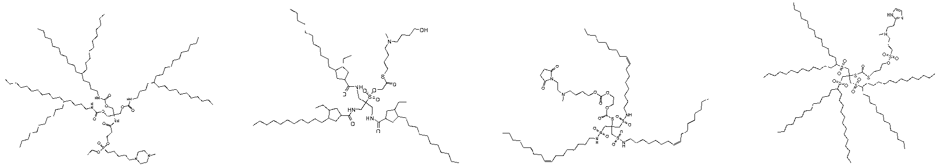

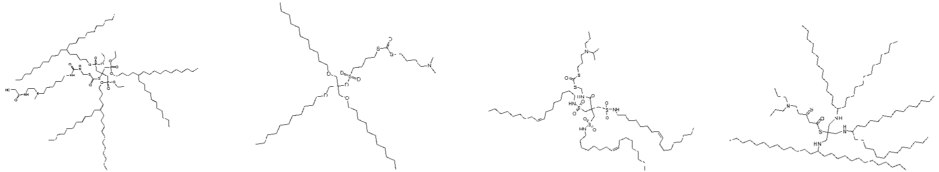

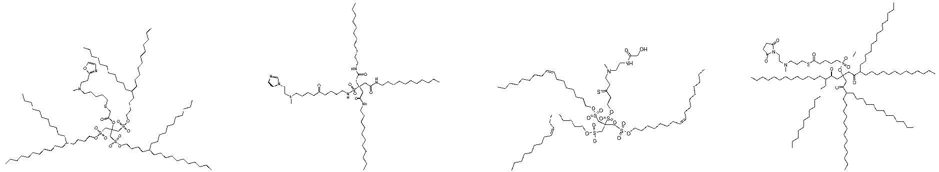

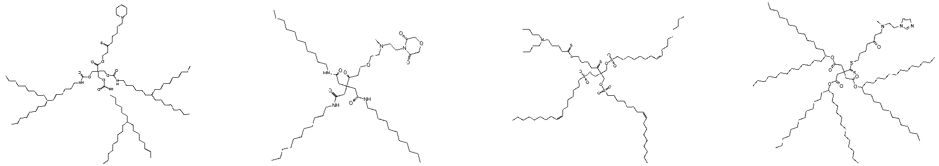

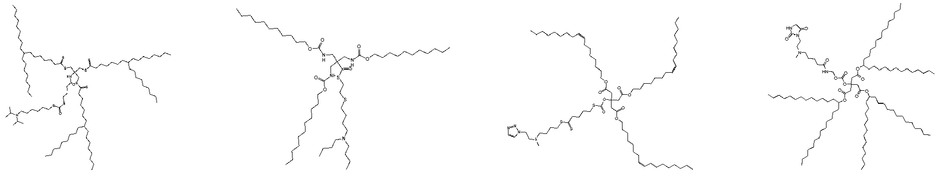

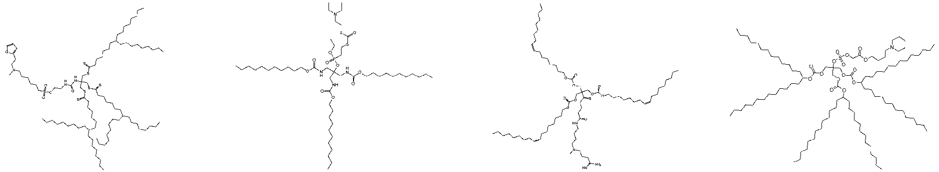

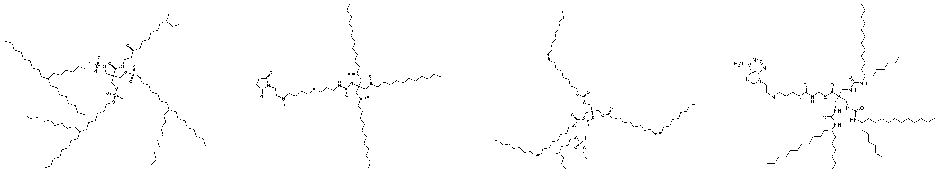

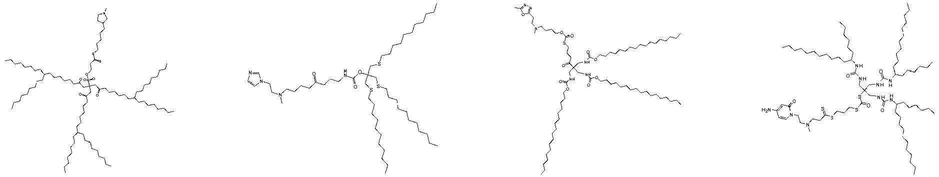

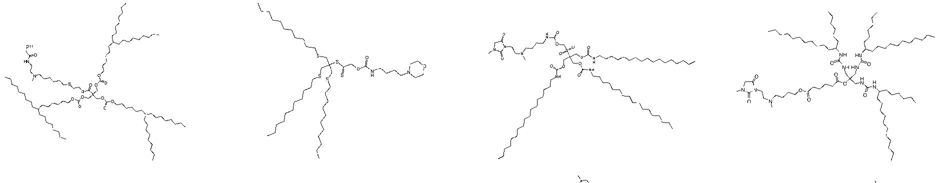

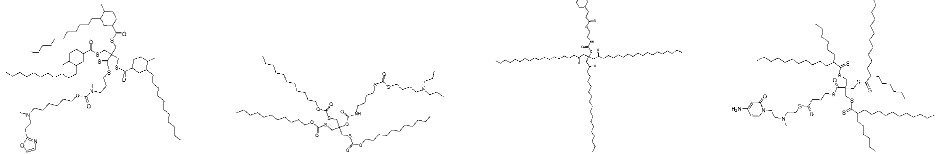

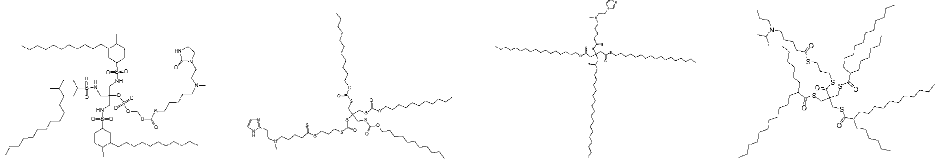

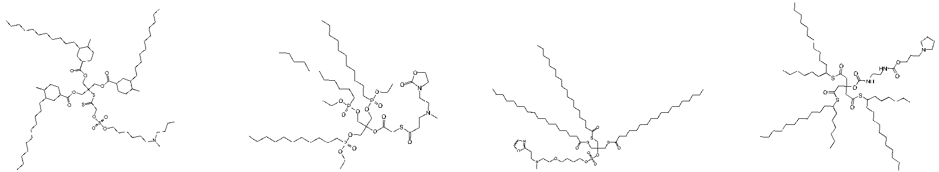

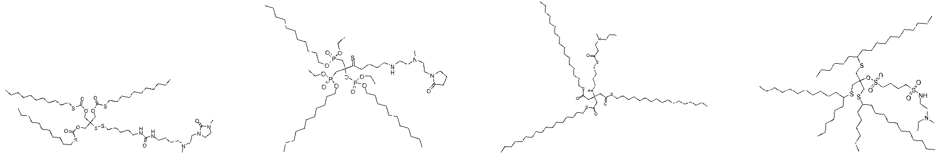

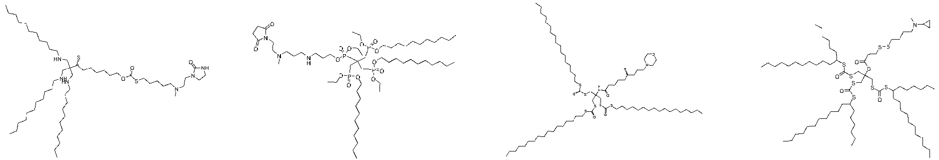

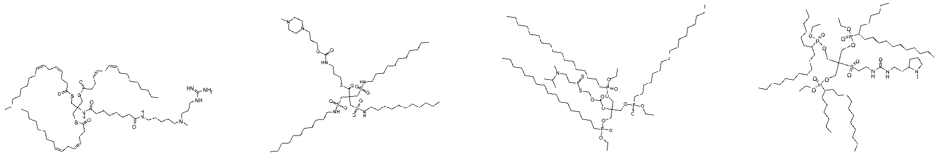

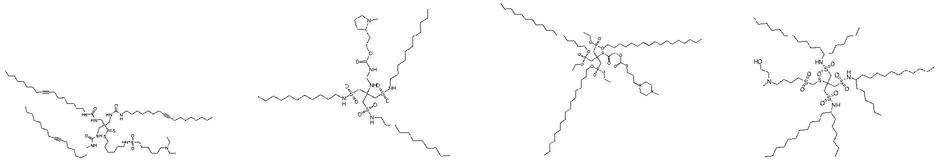

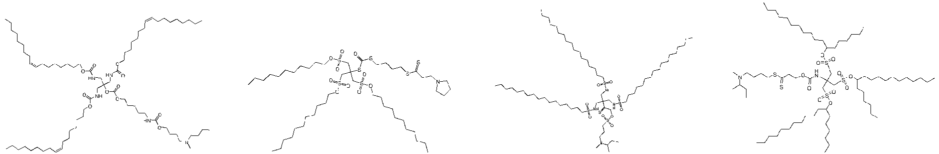

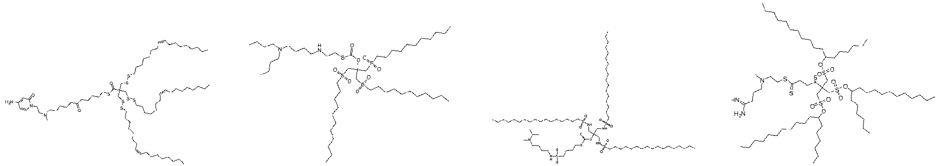

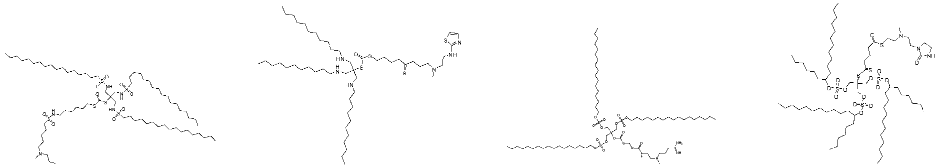

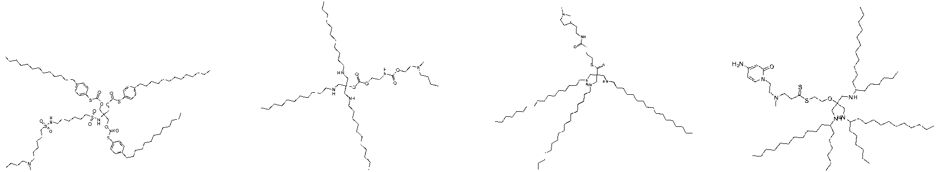

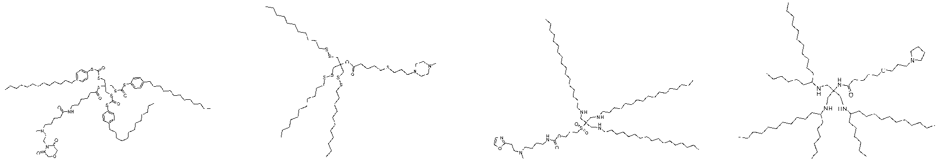

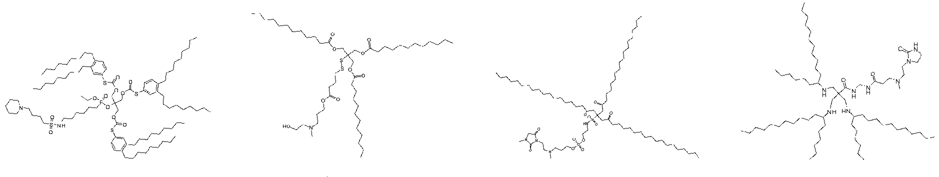

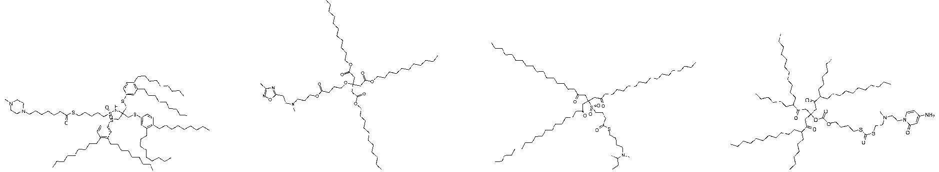

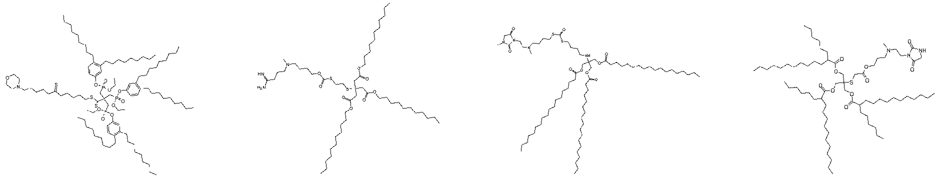

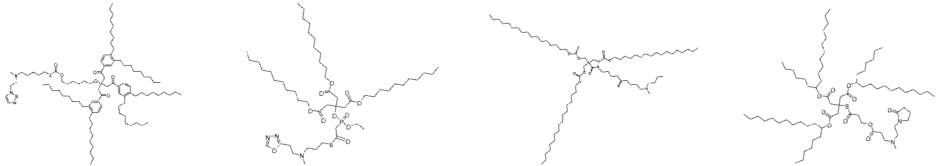

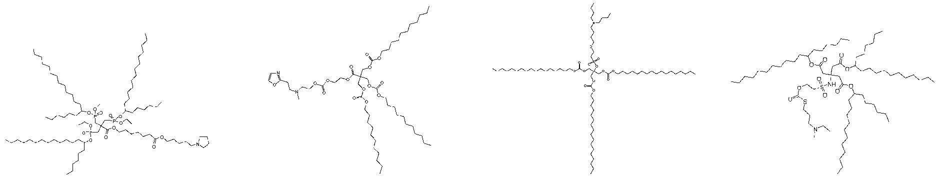

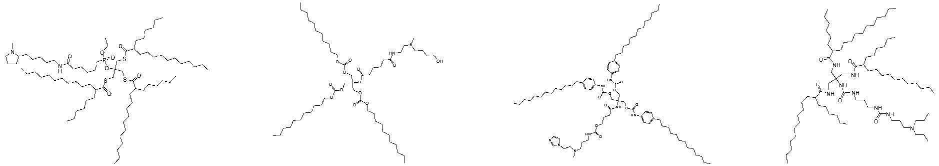

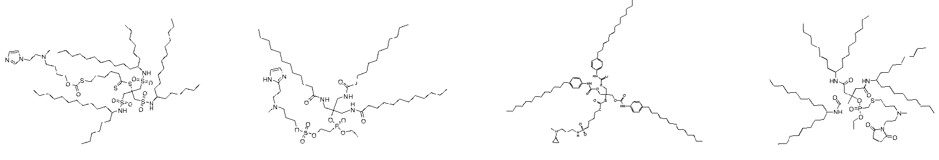

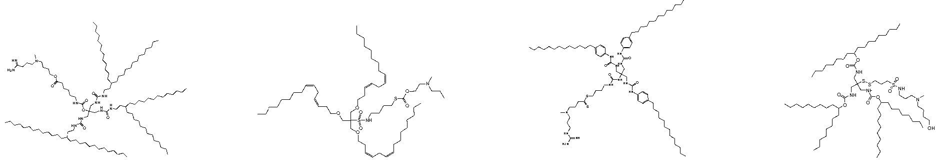

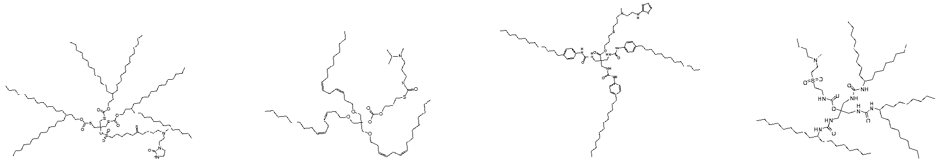

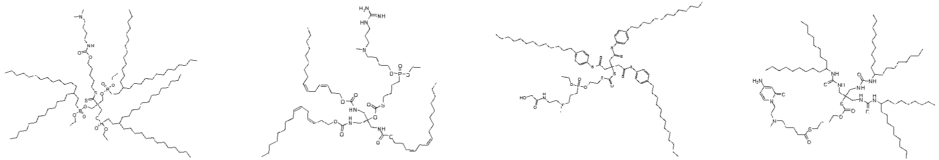

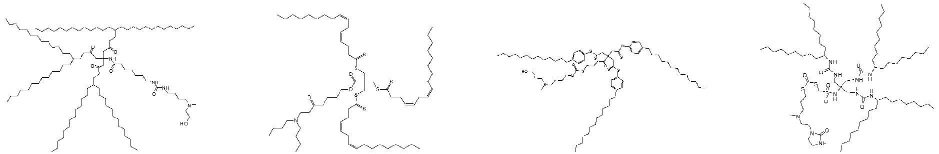

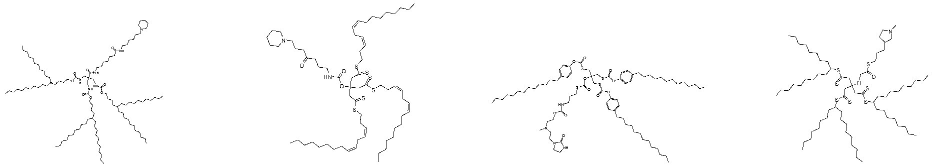

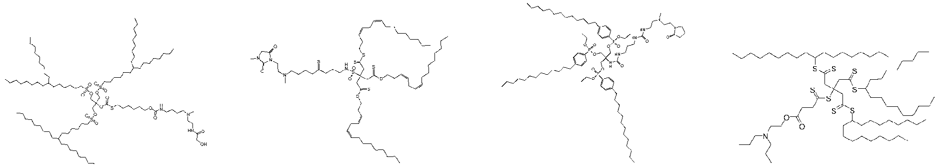

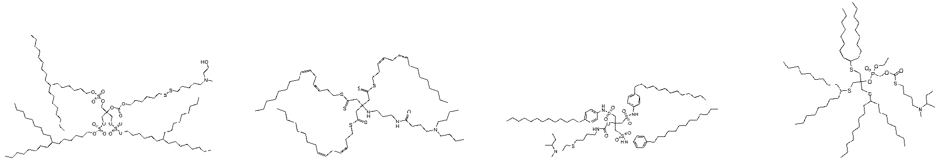

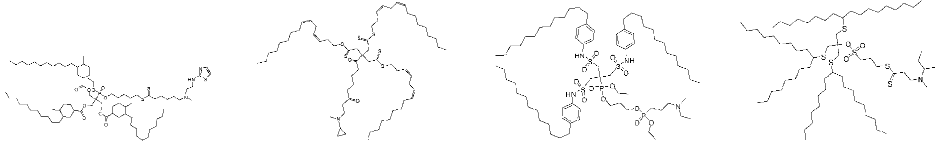

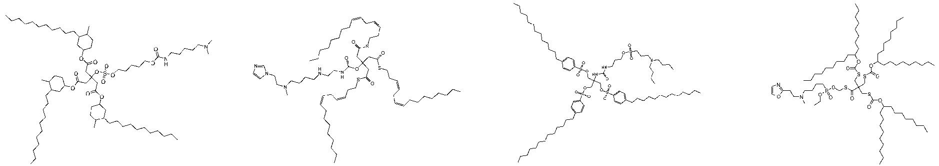

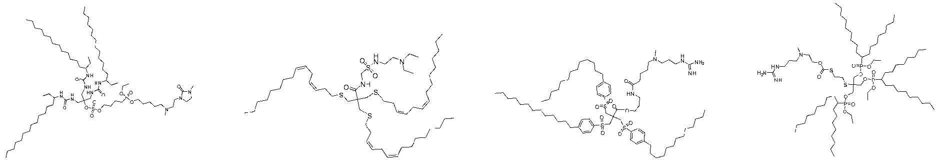

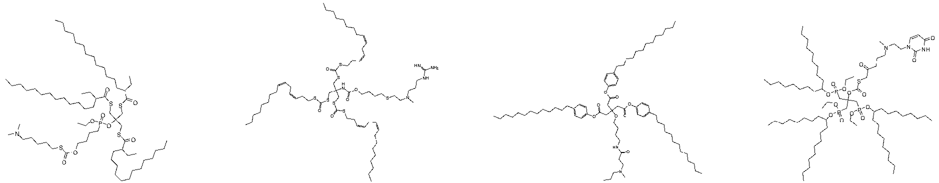

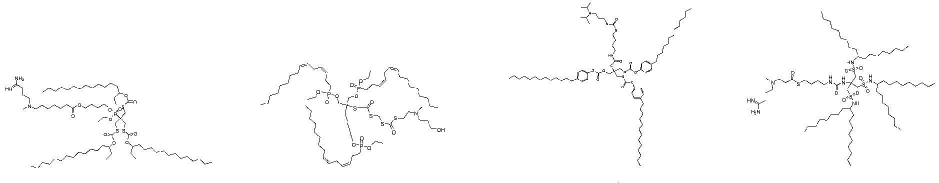

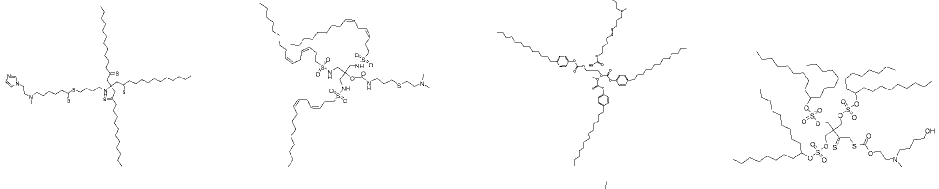

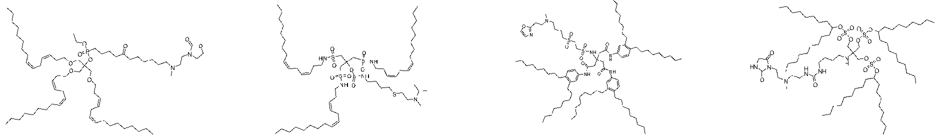

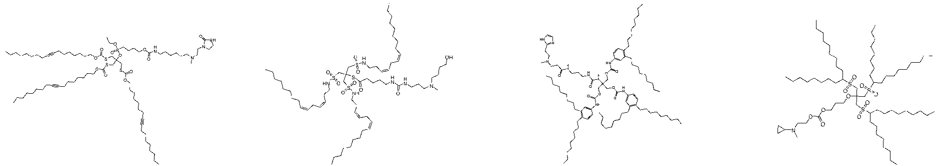

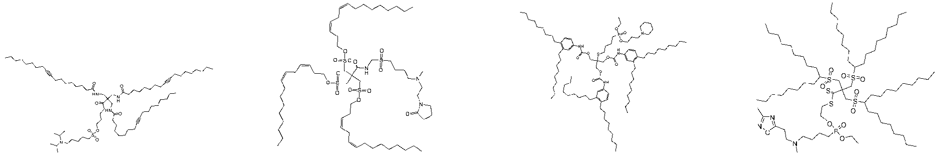

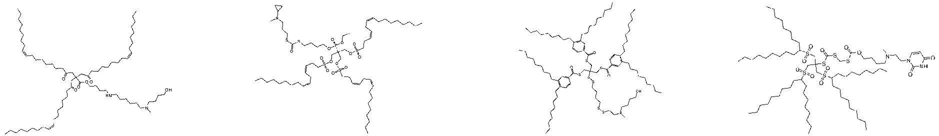

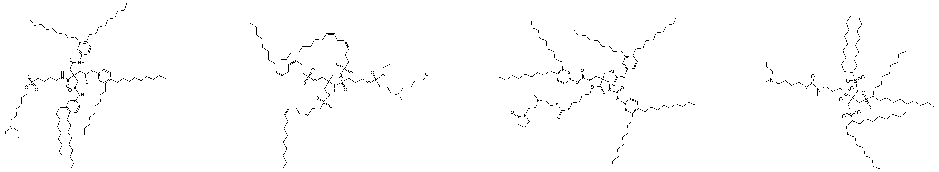

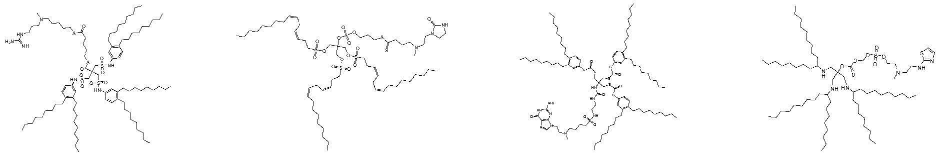

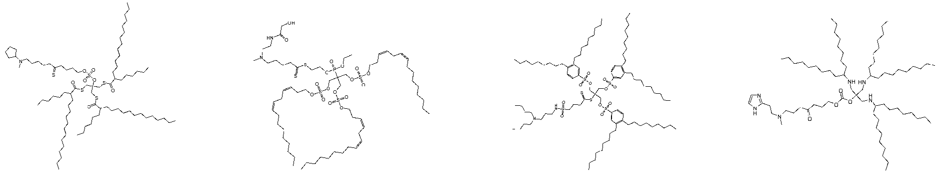

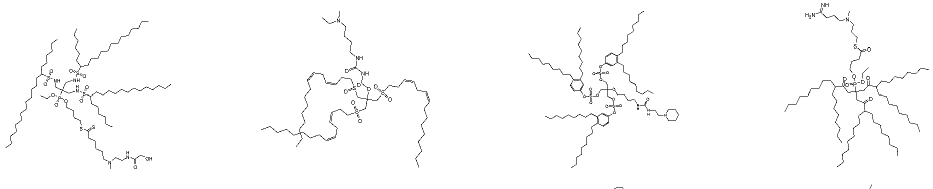

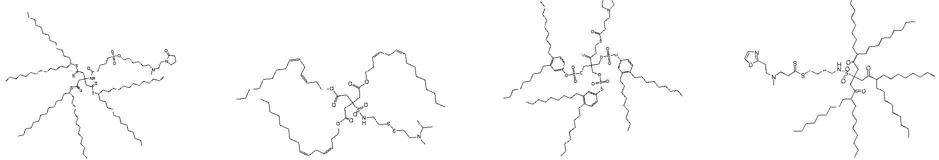

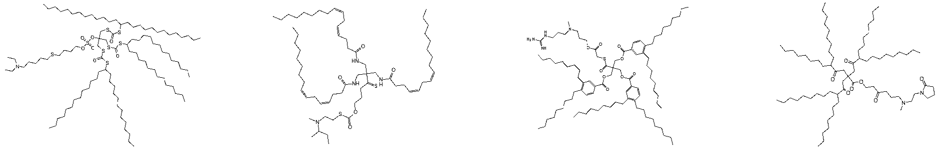

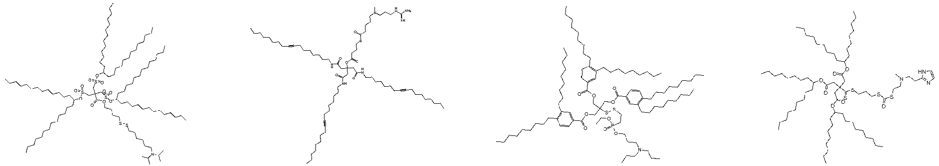

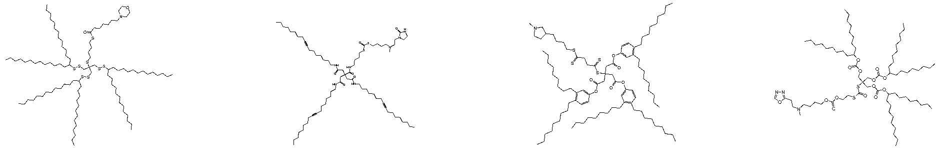

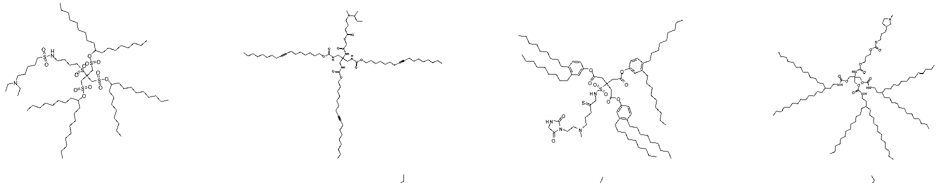

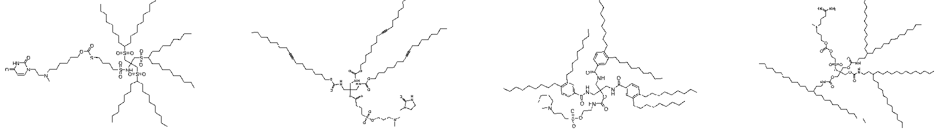

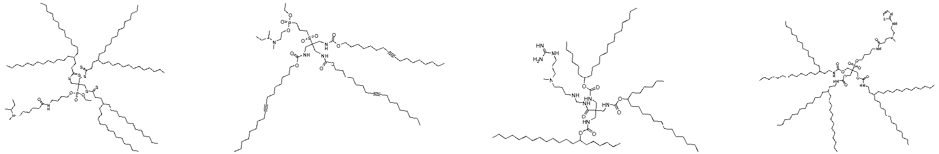

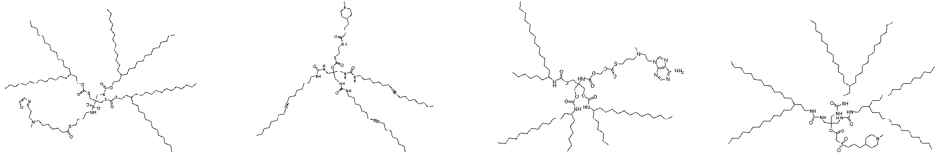

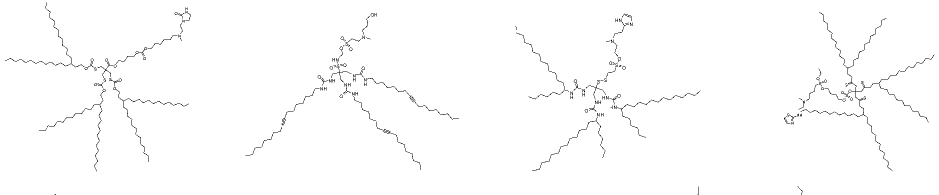

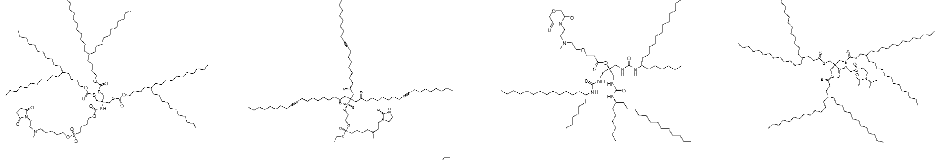

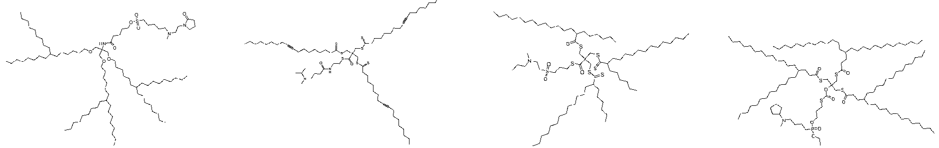

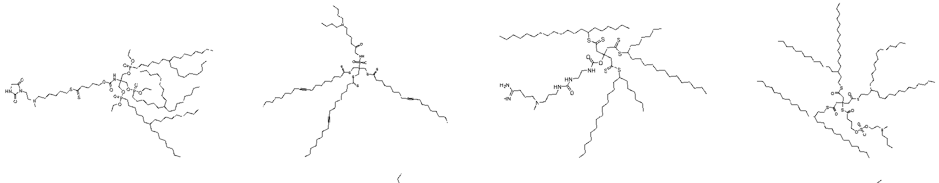

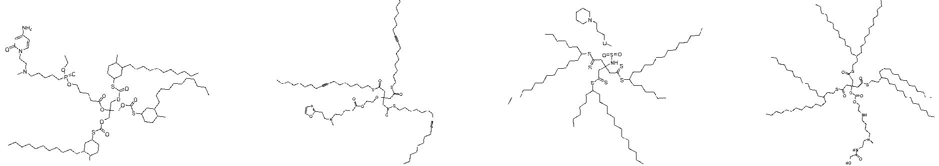

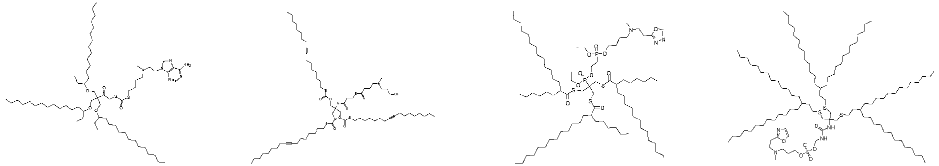

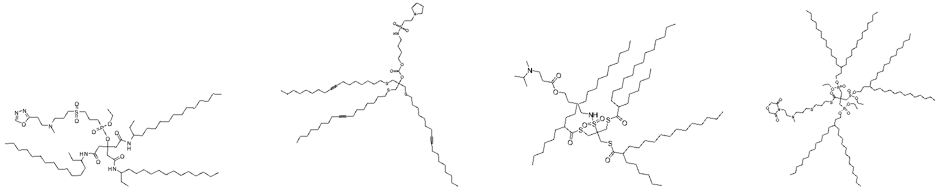

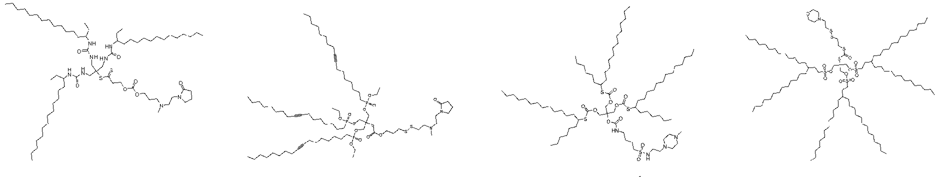

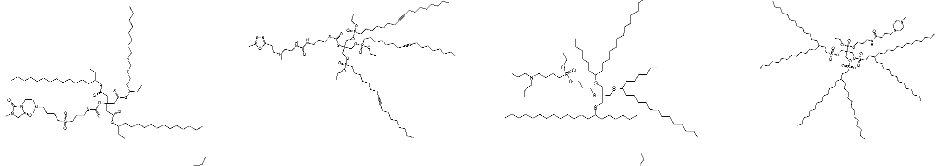

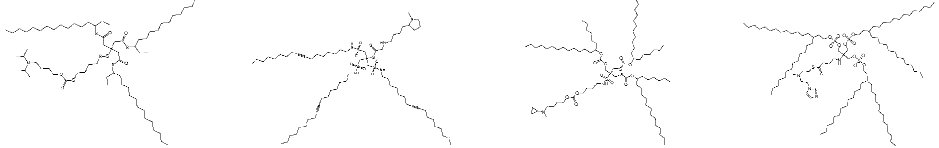

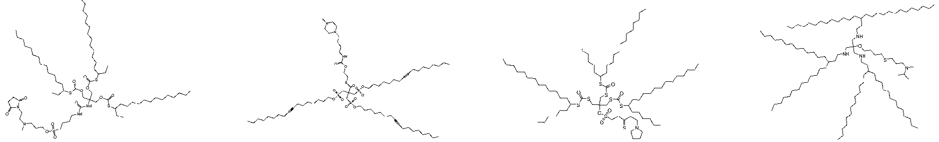

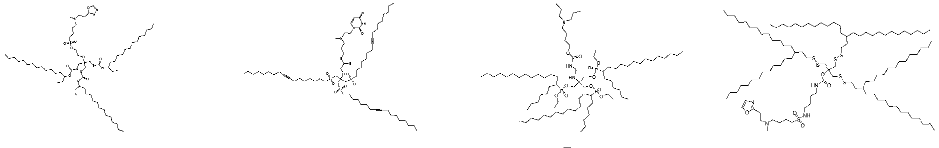

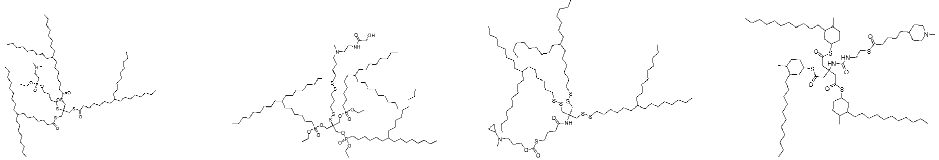

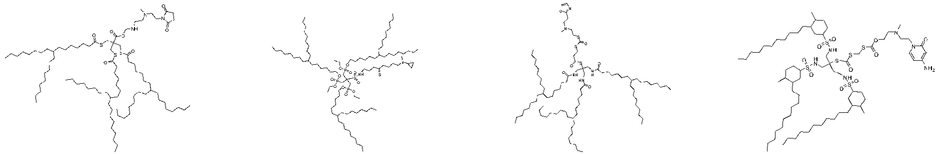

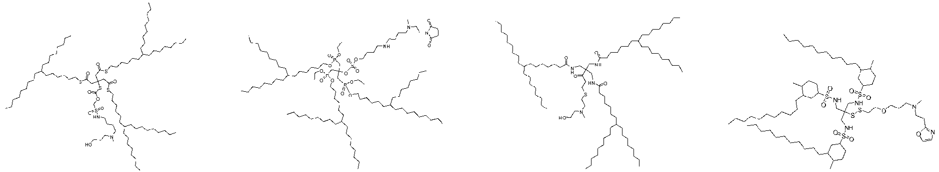

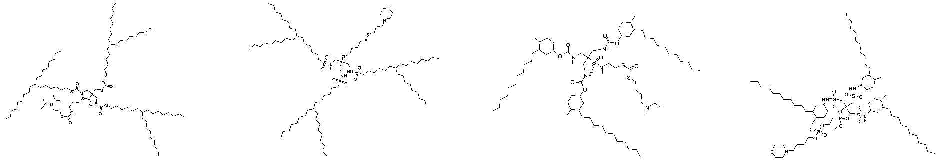

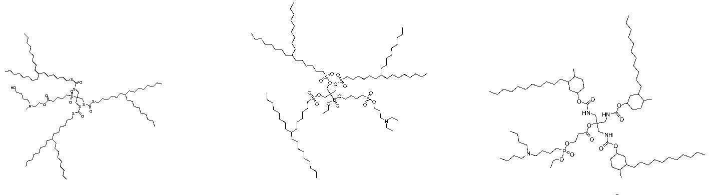

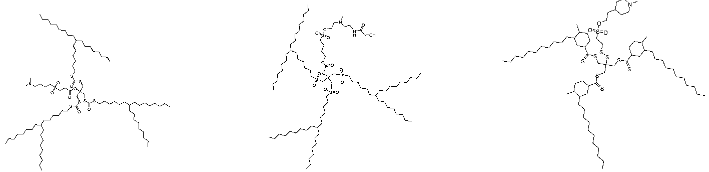

30. The use according to claim 8, wherein the second ionizable lipid is a compound of formula (2), or a salt, stereoisomer or tautomer thereof:
wherein A₁ is NH or O; 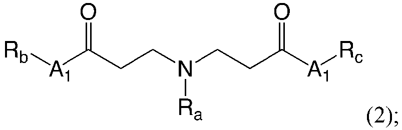
Ra is selected from C₆-C₂₄ alkyl, C₆-C₂₄ alkenyl, C₆-C₂₄ cycloalkyl, C₆-C₂₄ alcohol and short-chain C₆-C₂₄ polyoxyethylene; wherein the C₆-C₂₄ alkyl, C₆-C₂₄ alkenyl, C₆-C₂₄ cycloalkyl, C₆-C₂₄ alcohol and short-chain C₆-C₂₄ polyoxyethylene each have a linear or branched structure; R_{b} and R_{c} are each independently selected from C₁-C₁₂ alkyl, C₂-C₁₂ alkenyl, C₂-C₁₂ alkynyl, C₃-C₁₂ cycloalkyl, C₆-C₁₂ aryl, C₁-C₁₂ alkyl alcohol, C₁-C₁₂ heterocyclyl and alkylamine;
the alkylamine is 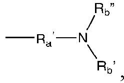 wherein Ra' is C₁-C₁₂ alkyl, and R_{b}' and R_{b}" are each independently selected from H, C₁-C₆ alkylamine, 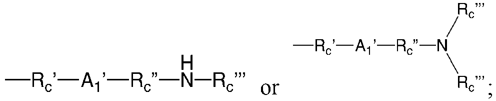 Rc" is selected from C₁-C₆ alkyl unsubstituted or substituted with amino groups, and Rc‴ is H, 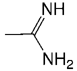 or -R_{c}'-A₁'-R_{c}"-NH₂;
provided that, when A₁' is -CO-NH-, -NH-CO- or -CO-O-, R_{c}' is C₁-C₆ alkyl; when A₁' is -CO-, R_{c}' is absent.

31. The use according to claim 30, wherein the compound of formula (2) is selected from at least one of the following compounds:

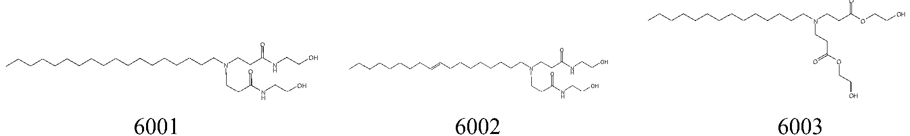

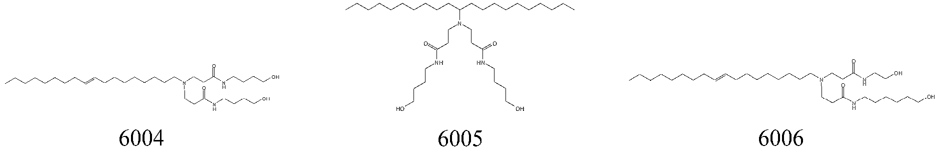

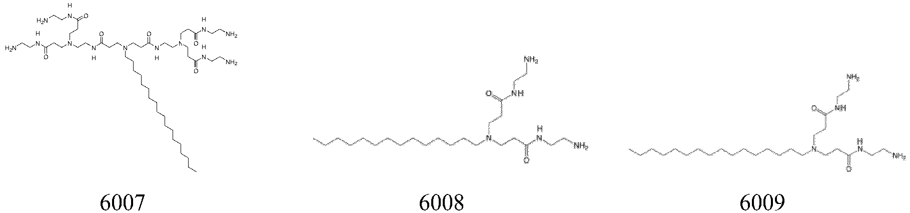

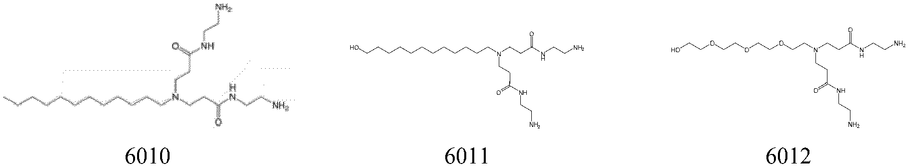

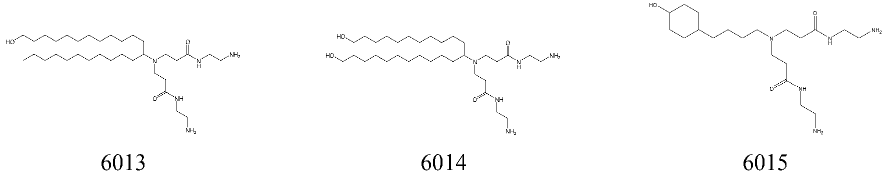

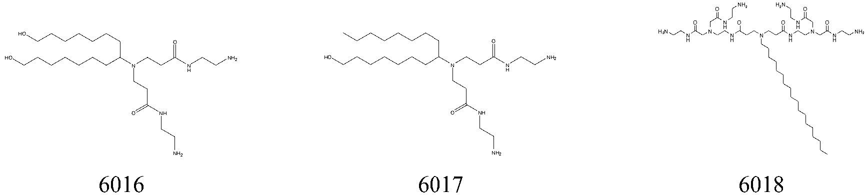

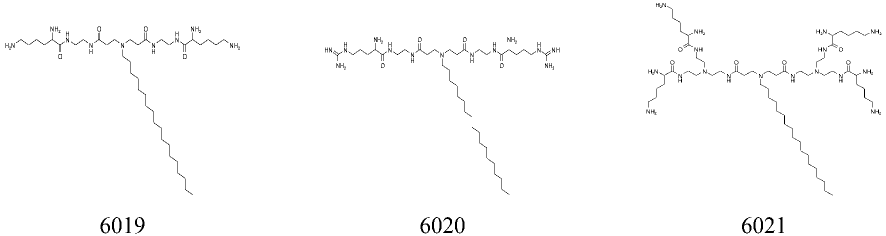

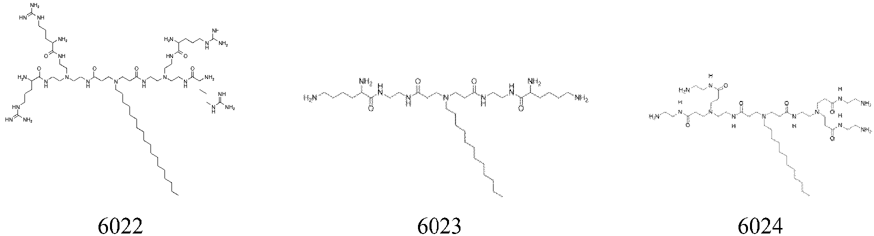

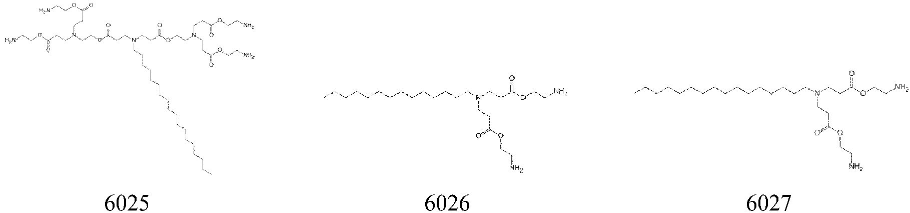

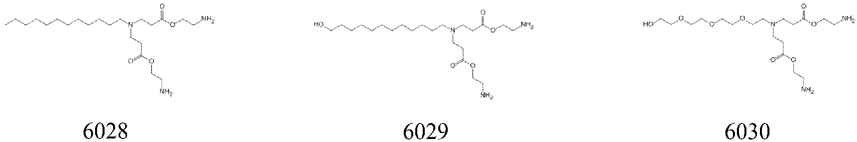

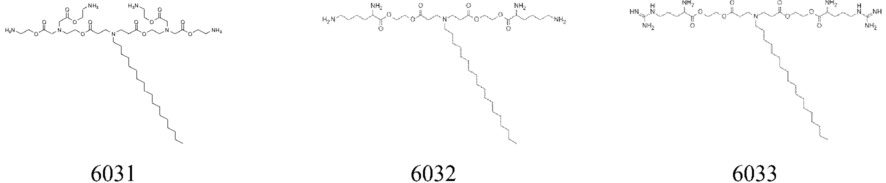

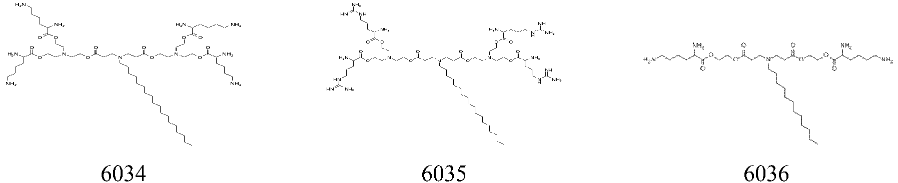

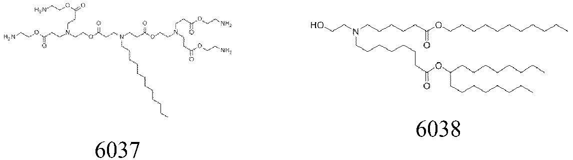

32. The use according to claim 8, wherein the blank lipid nanoparticles further comprise 0-60 mol% of other ionizable lipids, and the other ionizable lipids are selected from at least one of the following compounds:

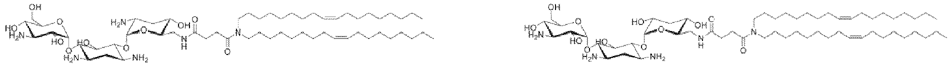

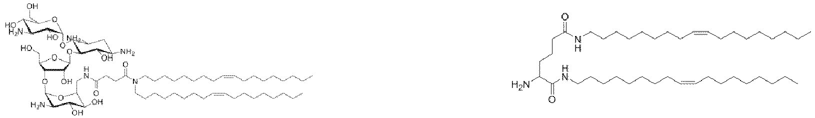

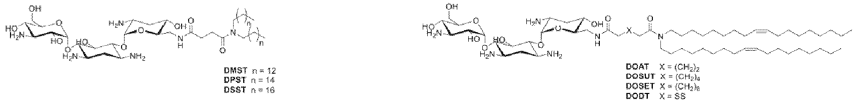

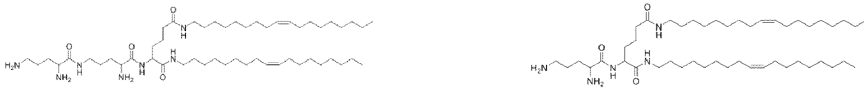

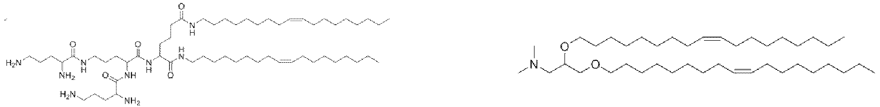

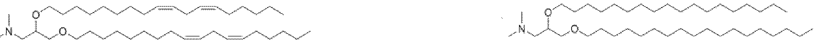

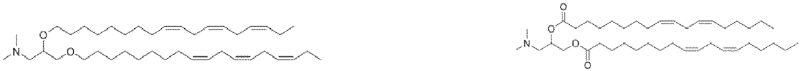

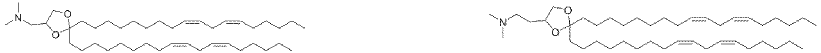

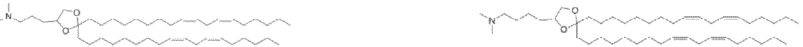

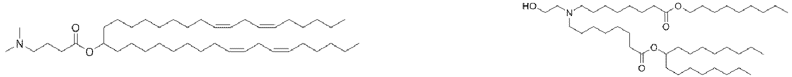

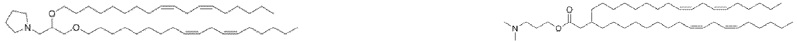

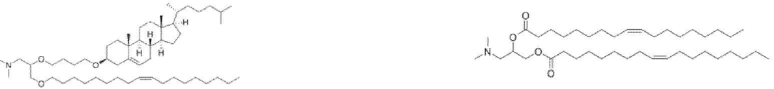

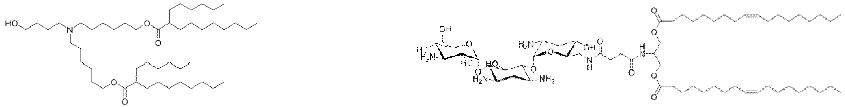

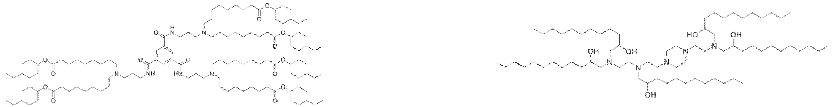

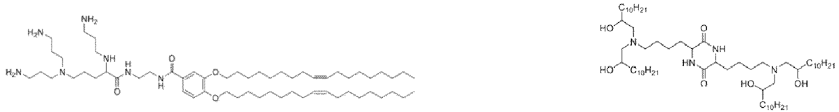

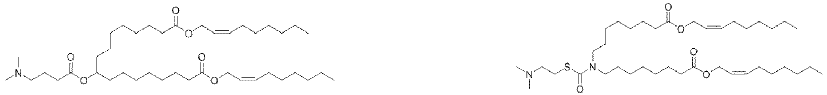

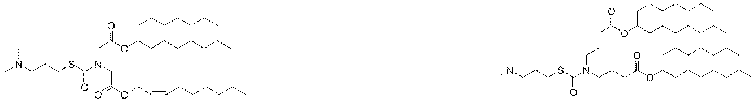

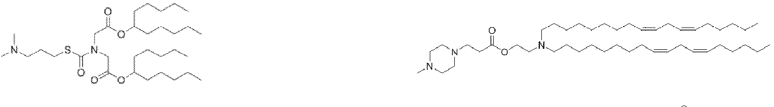

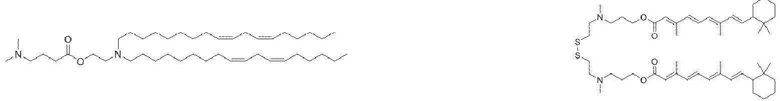

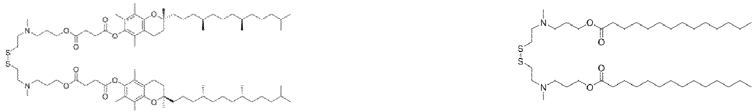

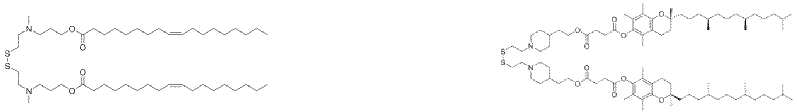

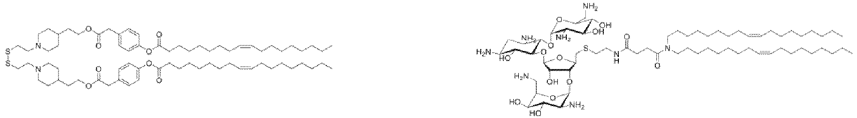

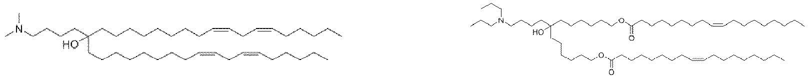

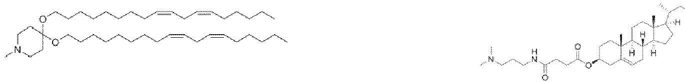

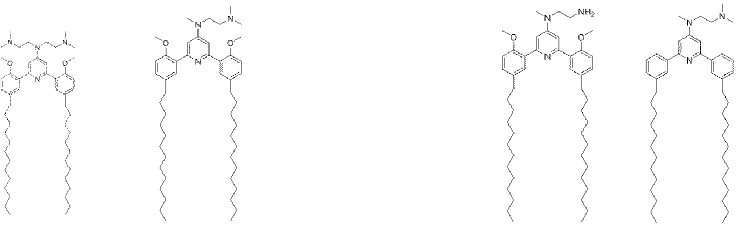

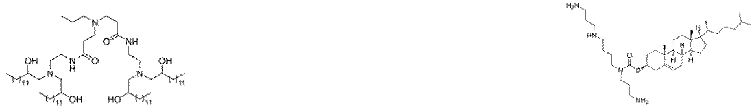

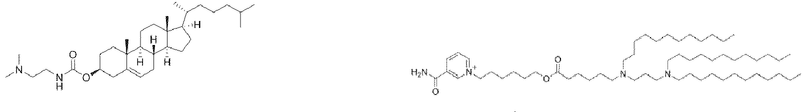

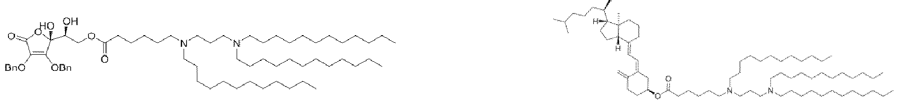

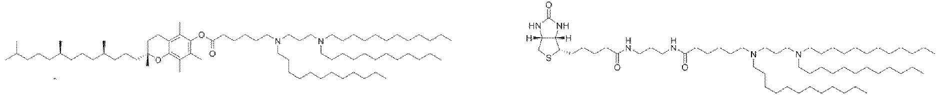

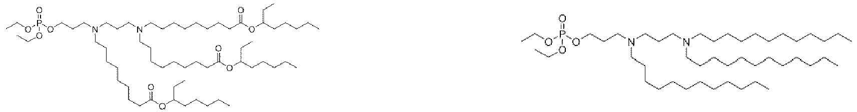

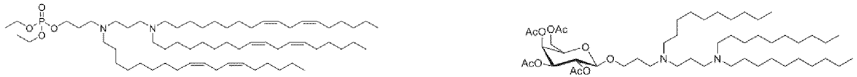

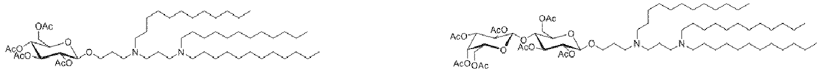

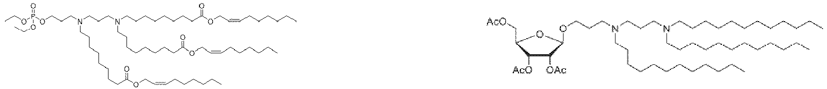

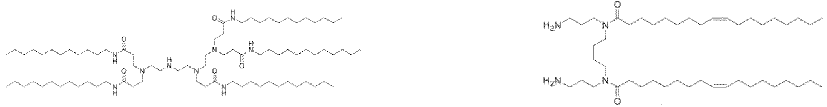

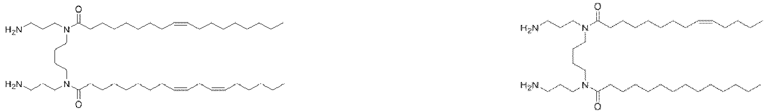

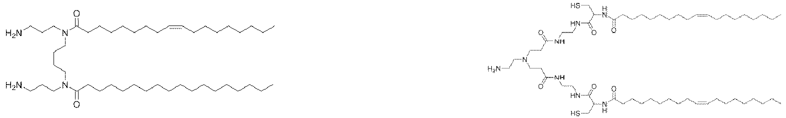

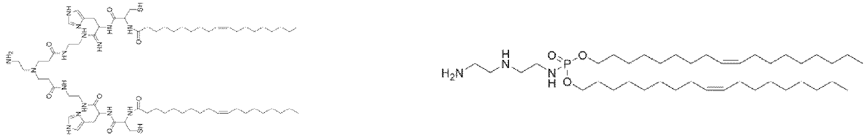

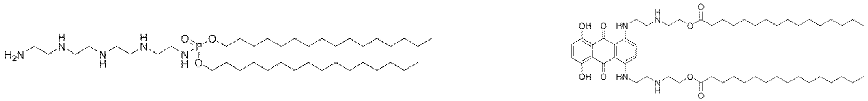

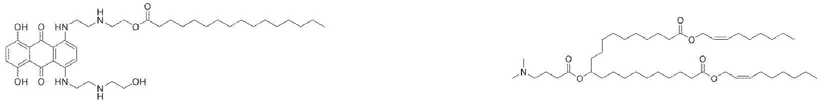

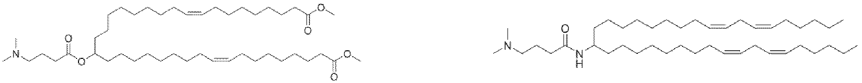

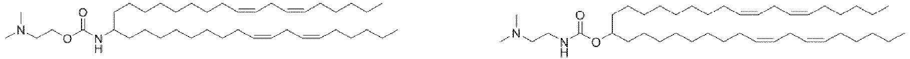

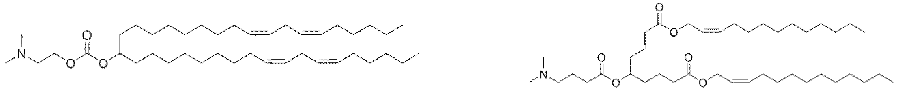

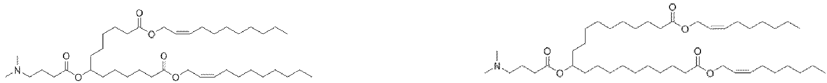

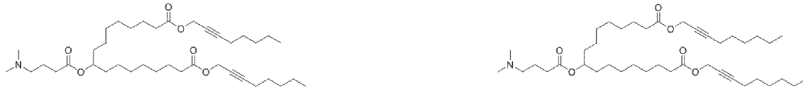

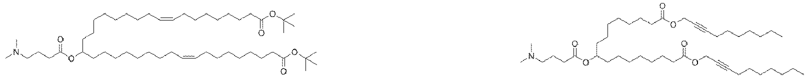

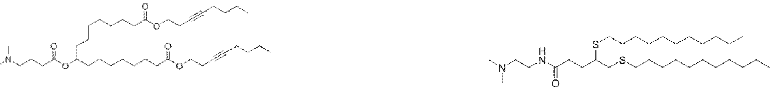

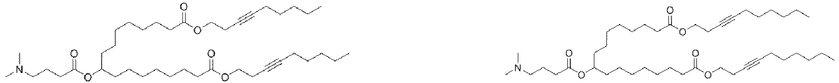

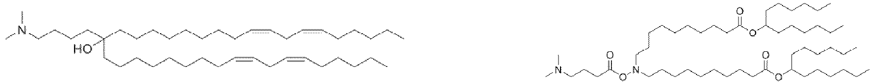

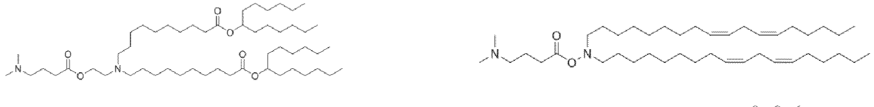

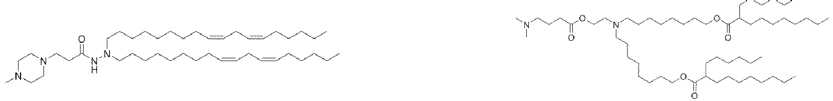

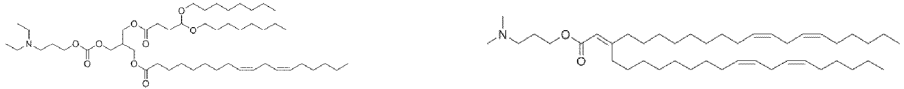

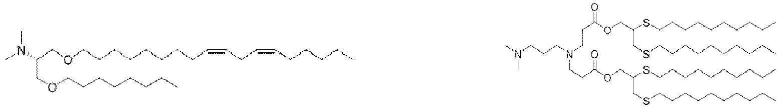

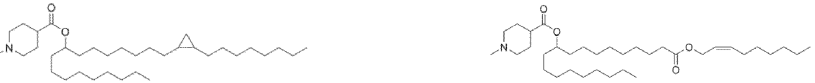

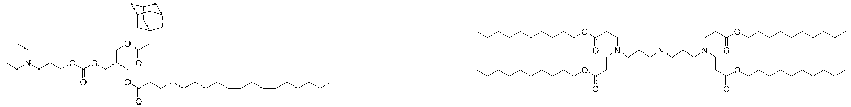

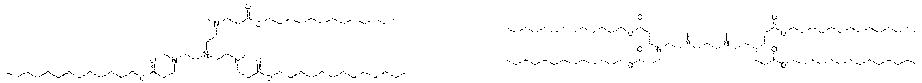

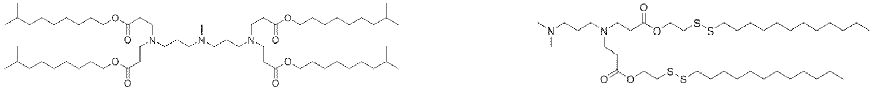

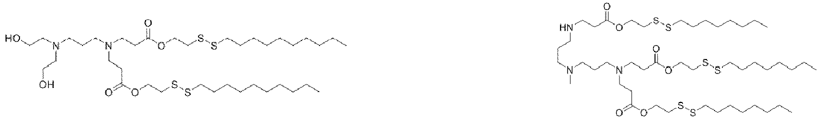

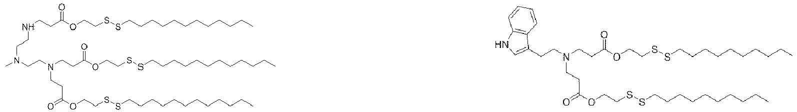

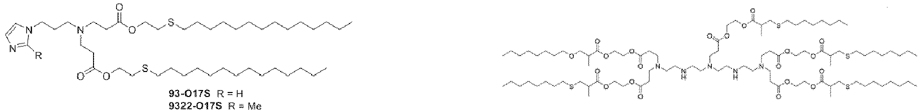

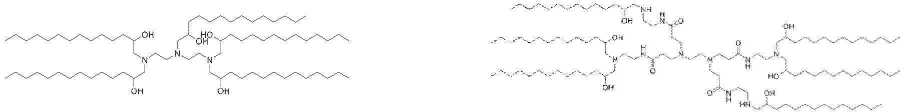

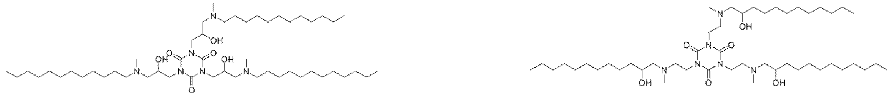

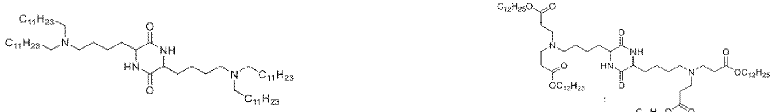

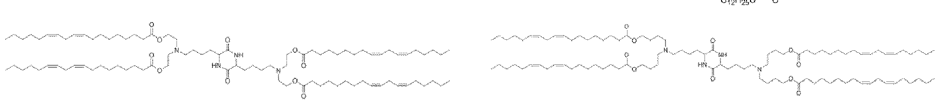

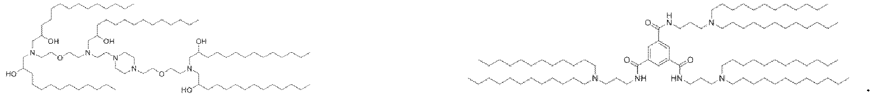

33. The use according to claim 1, wherein the phospholipid comprises at least one of 1,2-dilinoleoyl-sn-glycero-3-phosphocholine, 1,2-dimyristoyl-sn-glycero-3-phosphocholine, 1,2-dioleoyl-sn-glycero-3-phosphocholine, 1,2-dipalmitoyl-sn-glycero-3-phosphocholine, 1,2-distearoyl-sn-glycero-3-phosphocholine, 1,2-diundecanoyl-sn-glycero-3-phosphocholine, 1-palmitoyl-2-oleoyl-sn-glycero-3-phosphocholine, 1,2-di-O-octadecenyl-sn-glycero-3-phosphocholine, 1-oleoyl-2-cholesterylhemisuccinoyl-sn-glycero-3-phosphocholine, 1-hexadecyl-sn-glycero-3-phosphocholine, 1,2-dilinolenoyl-sn-glycero-3-phosphocholine, 1,2-diarachidonoyl-sn-glycero-3-phosphocholine, 1,2-docosahexaenoyl-sn-glycero-3-phosphocholine, 1,2-dioleoyl-sn-glycero-3-phosphoethanolamine, 1,2-diphytanoyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-docosahexaenoyl-sn-glycero-3-phosphoethanolamine, 1,2-dioleoyl-sn-glycero-3-phospho-rac-(1-glycerol) sodium salt, dipalmitoylphosphatidylglycerol, palmitoyloleoylphosphatidylethanolamine, distearoylphosphatidylethanolamine, dipalmitoylphosphatidylethanolamine, dimyristoylphosphoethanolamine, 1-stearoyl-2-oleoyl-phosphatidylethanolamine, 1-stearoyl-2-oleoyl-phosphatidylcholine, sphingomyelin, phosphatidylcholine, phosphatidylethanolamine, phosphatidylserine, phosphatidylinositol, phosphatidic acid, palmitoyloleoylphosphatidylcholine, lysophosphatidylcholine and lysophosphatidylethanolamine.

34. The use according to claim 1, wherein the steroid or a derivative thereof comprises at least one of cholesterol, cholesteryl stearate, coprostanol, sitosterol, ergosterol, campesterol, stigmasterol, brassicasterol, fucosterol, tomatine, ursolic acid and α-tocopherol.

35. The use according to claim 1, wherein the polyethylene glycol-lipid conjugate comprises at least one of PEG-modified phosphatidylethanolamine, PEG-modified phosphatidic acid, PEG-modified ceramide, PEG-modified dialkylamine, PEG-modified diacylglycerol and PEG-modified dialkylglycerol.

36. The use according to claim 35, wherein the polyethylene glycol-lipid conjugate comprises at least one of PEG-distearoxypropyl, PEG-c-DOMG, PEG-DPPC, polyethylene glycol dimethacrylate, 1,2-dimyristoyl-rac-glycero-3-methoxypolyethylene glycol, dipalmitoylglycerol-polyethylene glycol, 1,2-distearoyl-rac-glycero-3-methoxypolyethylene glycol, 2-[(polyethylene glycol)-2000]-N,N-ditetradecylacetamide, dipalmitoylphosphatidylethanolamine-polyethylene glycol, distearoylphosphatidylethanolamine-polyethylene glycol, dilauroylphosphatidylethanolamine-polyethylene glycol and dimyristoylphosphatidylethanolamine-polyethylene glycol lipids.

37. The use according to claim 1, wherein in the blank lipid nanoparticle-based composition, the biologically active substance is encapsulated inside the blank lipid nanoparticles and/or adsorbed onto the surface of the blank lipid nanoparticles to form a complex.

38. The use according to claim 37, wherein the solvent is selected from at least one of water, aqueous organic solvent solutions and buffered salt solutions.

39. The use according to claim 38, wherein the buffered salt solution has a pH value of 1-9, a concentration of the buffered salt is 0.1-200 mM, and the aqueous organic solvent solution is an alcohol solution with a volume concentration of less than 50%.

40. The use according to claim 40, wherein the buffered salt solution is selected from at least one of citrate solution, acetate solution, tartrate solution, phosphate solution, carbonate solution, Tris-HCl solution and sodium chloride solution.

41. The use according to claim 1, wherein the blank lipid nanoparticle-based composition is further added with at least one of sugar, glycerol, DMSO, salt, antibiotics and surfactants.

42. An in-vitro gene delivery method, wherein the in-vitro gene delivery method comprises the step of mixing the blank lipid nanoparticles with a biologically active substance in a solvent to obtain a blank lipid nanoparticle-based composition; wherein the blank lipid nanoparticle comprises the following components: 10-60 mol% first ionizable lipid, 0-30 mol% second ionizable lipid, 5-30 mol% phospholipid, 15-70 mol% cholesterol, and 0-10 mol% polyethylene glycol-lipid conjugate.
